# EUROPEAN PATENT APPLICATION

(11) **EP 4 840 443 A1**
(43) Date of publication of application: **18.11.2026**
(21) Application number: 25741656.0
(22) Date of filing: 20.01.2025
(51) Int. Cl.: A61K 9/51, A61K 47/18, A61K 47/22, A61K 47/26, A61K 47/24

(54) **LIPID COMPOSITION AND PREPARATION METHOD THEREFOR AND USE THEREOF**

(30) Priority: 19.01.2024 CN 202410080713
(71) Applicant: Scindy Pharmaceutical Co., Ltd, Suzhou, Jiangsu 215124 (CN)
(72) Inventor: CHEN, Xiaobao, Suzhou, Jiangsu 215124 (CN)
(74) Representative: Elzaburu S.L.P.
(86) International application number: PCT/CN2025/073384
(87) International publication number: WO 2025/153095

(57) **Abstract**

Disclosed are a lipid composition and a preparation method therefor and use thereof. The lipid composition includes the following components: an ionizable lipid and an auxiliary lipid, and does not contain a component having a preventive or therapeutic effect. The lipid composition can be used for delivering nucleic acids, macromolecular substances and small molecular substances, has a good stability, can be stored at room temperature for a long time, and can be used as a carrier in multiple fields such as cell and disease treatment and prevention.

## Description

### CROSS-REFERENCE TO RELATED APPLICATIONS

This application claims priority to Chinese Patent Application No. 202410080713.7, filed on January 19, 2024, the entire contents of which are incorporated herein by reference.

### TECHNICAL FIELD

The present application relates to the technical field of biomedicine, and more particularly, to a lipid composition and a preparation method therefor and use thereof.

### BACKGROUND

From the perspective of cell biology, drug delivery is a process of introducing active substances into cells to change the functions of host cells. The aforementioned active substances include deoxyribonucleic acid (DNA), ribonucleic acid (RNA), and small non-coding RNAs such as siRNA, shRNA, and miRNA, as well as active macromolecules and small molecules.

Up to now, a variety of biologically derived and chemically derived materials have been developed for delivering the aforementioned active substances, some of which have demonstrated favorable performance. Biologically derived carriers, including exosomes, bacterial outer membrane vesicles, and virus-like particles (such as PEG10 VLPs), have excellent applications in certain scenarios. In delivery systems based on chemical substances, polymers and lipid nanoparticles have been most widely applied due to their high efficiency and versatility. Polymers, including polyamine-based compounds (such as polyethyleneimine), poly(amine-co-ester)s (PACE), poly(glycidyl butylamine) (PGBA), poly(omithine) (PLO), poly(beta-amino esters) (PBAEs), poly(trimethylolpropane allyl ether-co-sebacoyl chloride), and charge-altering releasable transporters (CARTs), have been well developed due to their controllable synthetic routes and ease of scale-up. With respect to lipid carriers, lipid nanoparticles (LNPs) are considered the preferred choice for delivering the aforementioned biologically active substances, such as COVID-19 mRNA vaccines (Comimaty and Spikevax). Currently, more than ten nucleic acid drugs in clinical trials use LNPs as delivery carriers. These cases encompass infectious disease and cancer vaccines as well as genetic disease treatment. Some of these drugs have achieved significant progress, demonstrating the bright prospects of this platform. Recently disclosed data showed that a Phase 3 trial of the respiratory syncytial virus (RSV) vaccine (Modema's mRNA-1345) met its primary efficacy endpoint in adults aged 60 years and older, with a vaccine efficacy of 83.7% against RSV lower respiratory tract disease. In another case, a personalized nucleic acid cancer vaccine (Modema and Merck's mRNA-4157/V940) also achieved positive results when used in adjuvant therapy in high-risk melanoma patients following complete resection. Compared with administration of pembrolizumab alone, the combination therapy reduced the risk of recurrence or death by 44%. Recently, the FDA granted "Breakthrough Therapy" designation to both of the aforementioned mRNA-LNP vaccines, indicating the promising prospects of this field.

Currently, commonly used lipid nanotechnology consists of four components: ionizable cationic lipids, phospholipids, cholesterol, and PEGylated lipids. With regard to the formation of nucleic acid LNPs, different lipids and nucleic acids are dissolved in ethanol and an acidic aqueous phase (such as a citrate buffer with a pH value of 4.0), respectively. After this, the ethanol phase and aqueous phase are then mixed at a volume ratio of 1:3 via a microfluidic device to complete the self-assembly process. During the formation process, the ionizable cationic lipids are protonated to carry positive charges, then bound to negatively charged nucleic acids through electrostatic interactions, thereby encapsulating the nucleic acids within the lipid nanoparticles. Meanwhile, other auxiliary lipids (including phospholipids, cholesterol, and polymer lipids) self-assemble thereon to stabilize the formed nucleic acid LNPs. Subsequently, the mRNA-LNP solution is adjusted to neutral pH through buffer exchange, during which the ionizable lipids become uncharged, rendering them stable and less toxic at physiological pH. This preparation method is cumbersome, requires expensive equipment such as microfluidics, and the encapsulation process of biologically active substances must be performed simultaneously with the preparation process of nanoparticles. Due to material and formulation reasons, lipid nanoparticles can only be stored long-term at extremely low temperatures, thereby limiting the widespread application of lipid nanoparticle technology.

Based on this, the present application provides a lipid composition including an ionizable lipid and an auxiliary lipid, free of cationic polymers and free of components having a preventive or therapeutic effect, which can serve as a drug carrier. The lipid composition provided by the present application exhibits good stability, has a simple preparation method, and can be stored for a long period at room temperature or low temperature, and can be used as a carrier in various fields including cells as well as disease treatment and prevention.

### SUMMARY

To address the problems existing in the prior art, the present application provides a lipid composition and a preparation method therefor and use thereof. The lipid composition provided in the present application includes the following components: an ionizable lipid and an auxiliary lipid, and does not contain a component having a preventive or therapeutic effect. The lipid composition provided in the present application can be used for delivering nucleic acids, small molecular substances or macromolecular substances. The lipid composition provided in the present application has good stability, and can be stored at room temperature for a long time. It can realize good encapsulation of active ingredients through simple mixing with active ingredients, and can be used as a carrier in multiple fields such as cell and disease treatment and prevention.

To achieve the above objective, in a first aspect, the present application provides a lipid composition including the following components: an ionizable lipid and an auxiliary lipid, where the lipid composition does not contain a component having a preventive or therapeutic effect; the ionizable lipid includes a first ionizable lipid, and the first ionizable lipid is selected from a compound of formula (1), or a salt, a stereoisomer, or a tautomer thereof: 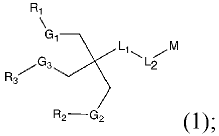 where R₁, R₂, and R₃ are each independently H, a C₅₋₄₀ linear or branched alkyl , a C₅₋₄₀ linear or branched alkenyl, a C₅₋₄₀ linear or branched alkynyl, a 3- to 6-membered saturated or partially unsaturated cyclic hydrocarbon group containing 1-3 side chains, or a 6- to 10-membered aromatic group containing 1-3 side chains; the side chains are each independently selected from a C₁₀₋₃₀ linear or branched alkyl, a C₁₀₋₃₀ linear or branched alkenyl, and a C₁₀₋₃₀ linear or branched alkynyl; with the proviso that at most one of R₁, R₂, and R₃ is H;

M is selected from -NR₄R₅, a saturated or partially unsaturated 3- to 6-membered heterocyclyl containing at least one nitrogen atom, and a 6- to 10-membered heteroaryl containing at least one nitrogen atom, where the heterocyclyl or heteroaryl is unsubstituted or substituted by one or more -OH, carboxyl, amino, oxo, or halogen groups;

R₄ and R₅ are each independently H, a C₁₋₆ linear or branched alkyl, a C₂₋₆ linear or branched alkenyl, or a C₂₋₆ linear or branched alkynyl, where the C₁₋₆ linear or branched alkyl, C₂₋₆ linear or branched alkenyl, or C₂₋₆ linear or branched alkynyl is unsubstituted or substituted by one or more -OH, carboxyl, aminoamide group, amidino, guanidino, or halogen groups;

G₁, G₂, and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)- or -OP(=O)(OR₆)O-; where R₆ and R₁₃ are each independently selected from H, hydroxyl, a C₁₋₃₀ linear or branched alkyl or cycloalkyl, and a -₃₀ linear or branched alkenyl;

L₁ is selected from -X₁- or -(CR₇R₈)ₘ-X₁-, where each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)- or -OP(=O)(OR₁₄)O-; where m is an integer of 2-6; R₇ and R₈ are each independently selected from H, hydroxyl, halogen, a C₁₋₆ linear or branched alkyl or cycloalkyl, and a C₂₋₆ linear or branched alkenyl; and R₁₄ and R₁₅ are each independently selected from H, a C₁₋₃₀ linear or branched alkyl or cycloalkyl, and a C₂₋₃₀ linear or branched alkenyl;

L₂ is -(CR₉R₁₀)ₙ- or -(CR₉R₁₀)ₙ-X₂-(CR₁₁R₁₂)ₖ-, where X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, -NR₁₆-S(=O)₂-, -P(=O)(OR₁₆)O-, -OP(=O)(OR₁₆)-, or -OP(=O)(OR₁₆)O-; n is an integer from 1 to 6; k is an integer from 1 to 6; R₉, R₁₀, R₁₁, and R₁₂ are each independently H, hydroxyl, halogen, a C₁₋₆ linear or branched alkyl or cycloalkyl, or a C₂₋₆ linear or branched alkenyl; and R₁₆ and R₁₇ are each independently selected from H, a C₁₋₃₀ linear or branched alkyl or cycloalkyl, and a C₂₋₃₀ linear or branched alkenyl;
where the alkyl, cycloalkyl, and alkenyl mentioned in R₄ to R₁₇ are unsubstituted or substituted by one or more groups selected from hydroxyl, mercapto, amino, substituted amino, and halogen; and
the salt is not a quaternary ammonium salt.

In one embodiment, the component having a preventive or therapeutic effect is a pharmaceutically active ingredient.

In one embodiment, the R₁, R₂, and R₃ are each independently selected from the following groups: 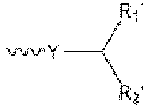 where Y is absent, or is a C₁₋₃₀ linear or branched alkyl or cycloalkyl, a C₂₋₂₀ linear or branched alkenyl, or a C₂₋₂₀ linear or branched alkynyl; R₁' and R₂' are each independently H, a C₁₋₃₀ linear or branched alkyl, a C₂₋₃₀ linear or branched alkenyl, or a C₂₋₃₀ linear or branched alkynyl; and the total carbon chain length of Y, R₁', and R₂' is 8-40.

In one embodiment, the R₁, R₂, and R₃ are each independently selected from the following groups: 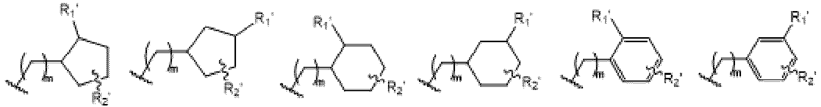 where R₁' and R₂' are each independently selected from H, a C₁₋₃₀ linear or branched alkyl , a C₂₋₃₀ linear or branched alkenyl, or a C₂₋₃₀ linear or branched alkynyl, and the total carbon chain length of R₁' and R₂' is 8-30.

In one embodiment, the R₁, R₂, and R₃ are each independently selected from any one of the following groups:

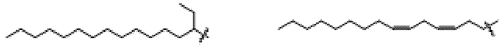

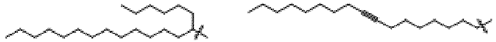

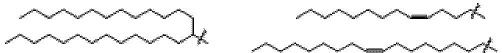

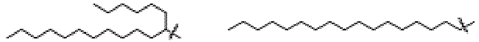

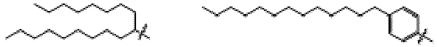

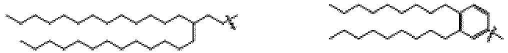

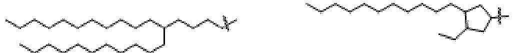

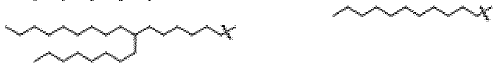

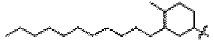

In one embodiment, G₁, G₂, and G₃ are each independently selected from -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)-, or -OP(=O)(OR₆)O-.

In one embodiment, L₁ is selected from -(CR₇R₈)ₘ-X₁-, where X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)-, or -OP(=O)(OR₁₄)O-.

In one embodiment, L₂ is -(CR₉R₁₀)ₙ-X₂-(CR₁₁R₁₂)ₖ-, where X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)-, or -OP(=O)(OR₁₆)O-.

In one embodiment, M is selected from the following structures: 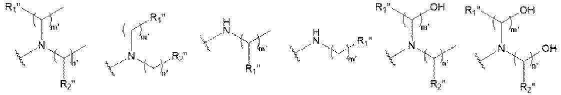 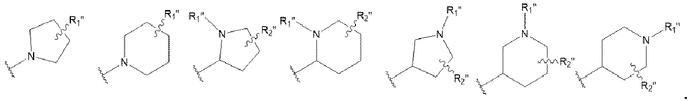 where m' and n' are each independently an integer from 0 to 6; R₁" and R₂'' are each independently H, a C₁₋₆ alkyl, a C₂₋₆ alkenyl, guanidino, amidino, amido, aliphatic amino, or a 3- to 10-membered nitrogen-containing heterocycle; the nitrogen-containing heterocycle is selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine, and pyrimidine, and tautomeric forms thereof, which are unsubstituted or optionally substituted with one or more organic groups selected from hydroxy, mercapto, amino, substituted amino, halogen, a C₁₋₆ alkyl, a C₂₋₆ alkenyl, a C₂₋₆ alkynyl, and a C₆₋₁₄ aryl.

In one embodiment, the compound of formula (1) is selected from a compound represented by formula (1A):

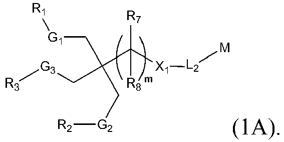

In one embodiment, the compound of formula (1) is selected from a compound represented by formula (1B):

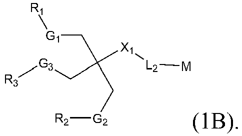

In one embodiment, the compound of formula (1) is selected from a compound represented by formula (1C):

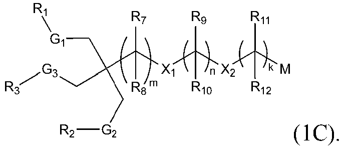

In one embodiment, the compound of formula (1) is selected from a compound represented by formula (1D):

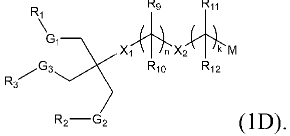

In one embodiment, the compound of formula (1) is selected from a compound represented by formula (1E):

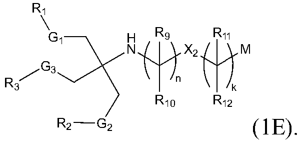

In one embodiment, the compound of formula (1) is selected from a compound represented by formula (1F):

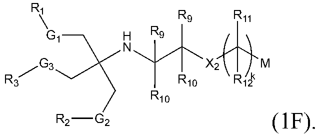

In one embodiment, the compound of formula (1) is selected from a compound represented by formula (1G):

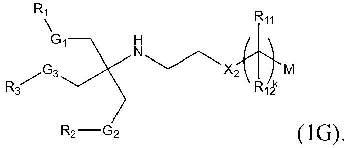

In one embodiment, the compound of formula (1) is selected from a compound represented by formula (1H):

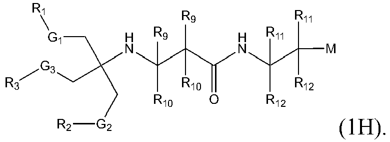

In one embodiment, M is selected from any one of the following groups:

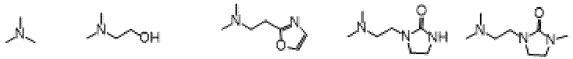

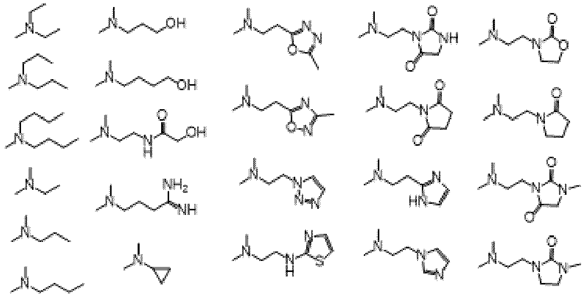

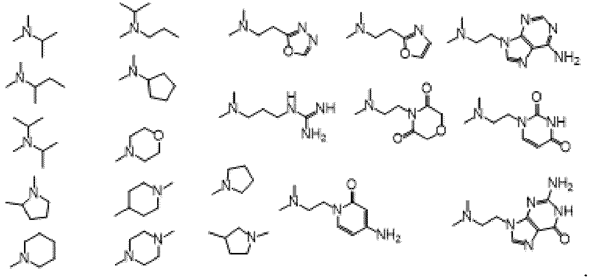

In one embodiment, the compound of formula (1) is selected from a compound represented by formula (1I):

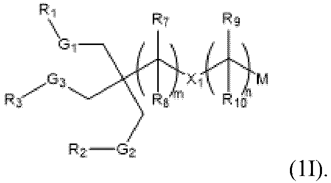

In one embodiment, the compound of formula (1) is selected from a compound represented by formula (1J):

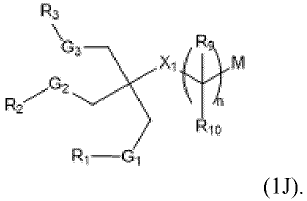

In one embodiment, the compound of formula (1) is selected from a compound represented by formula (1K):

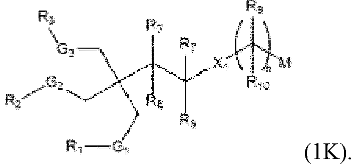

In one embodiment, Y is absent, and the compound of formula (1) is selected from a compound represented by formula (1L):

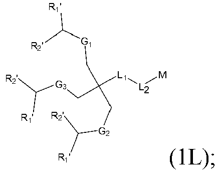

wherein R₁' and R₂' are independently selected from H, a C₁₋₃₀ linear or branched alkyl, a C₂₋₃₀ linear or branched alkenyl, and a C₂₋₃₀ linear or branched alkynyl, and the total carbon chain length of R₁' and R₂' is 8-40.

In one embodiment, the compound of formula (1) is selected from a compound represented by formula (1M):

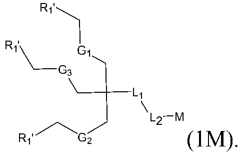

In one embodiment, the compound of formula (1) is selected from:

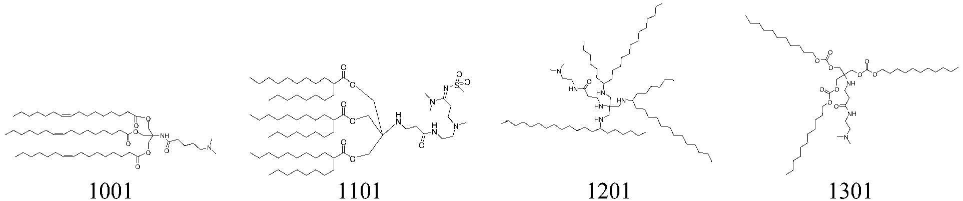

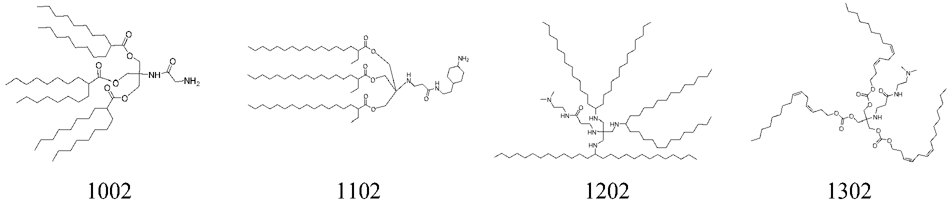

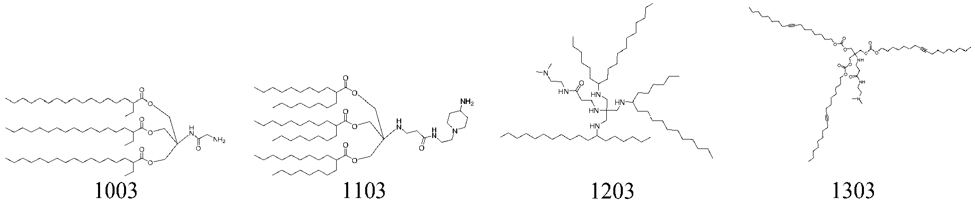

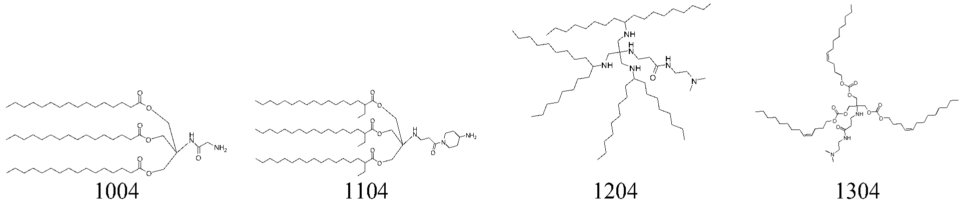

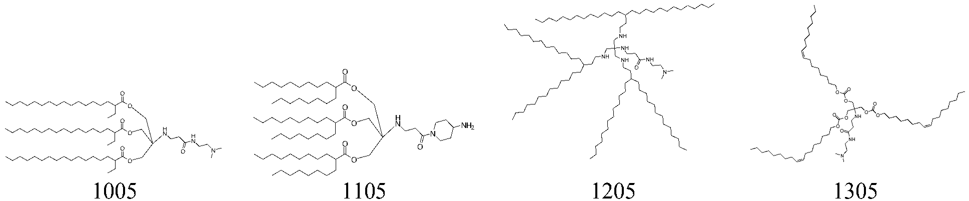

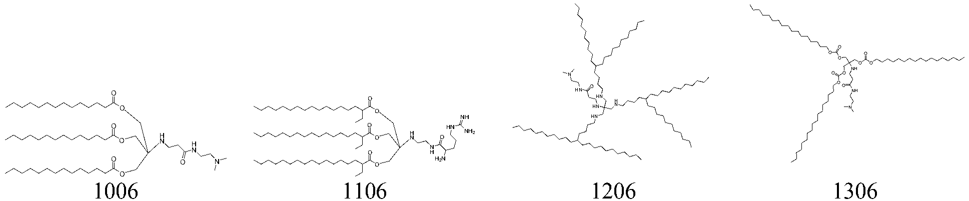

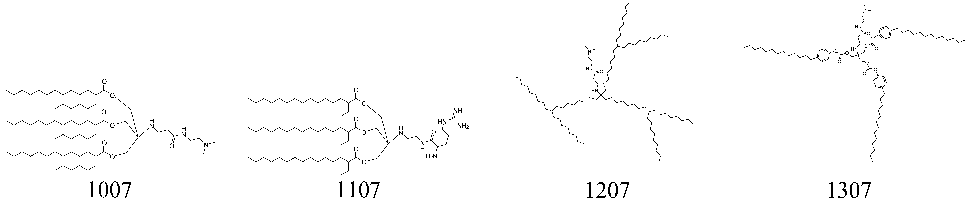

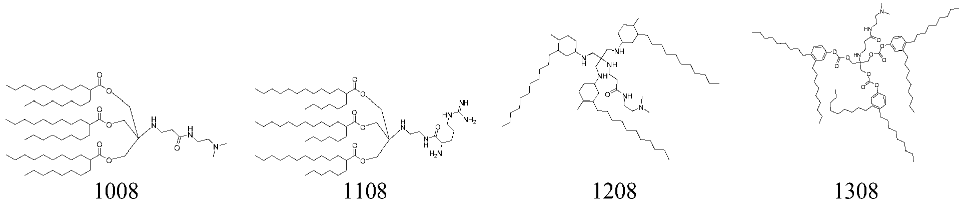

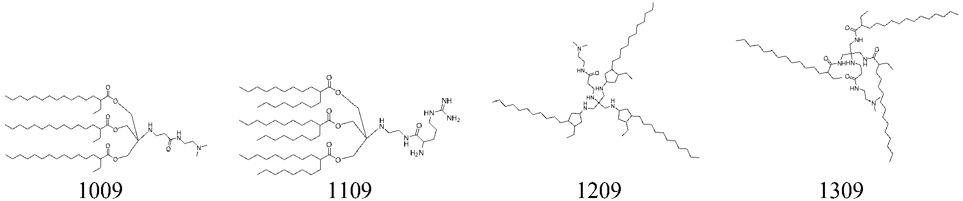

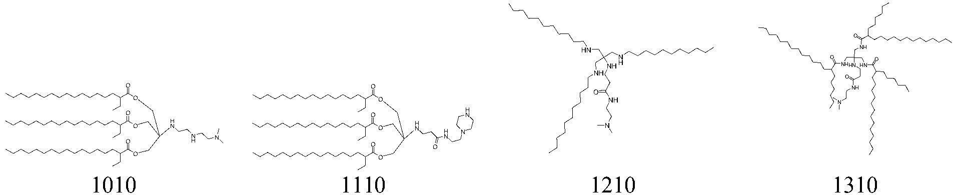

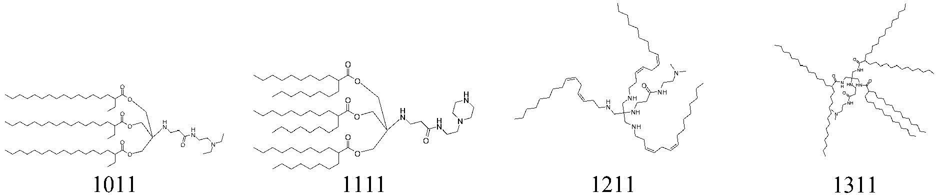

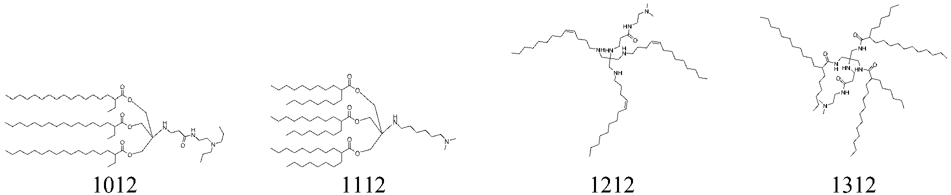

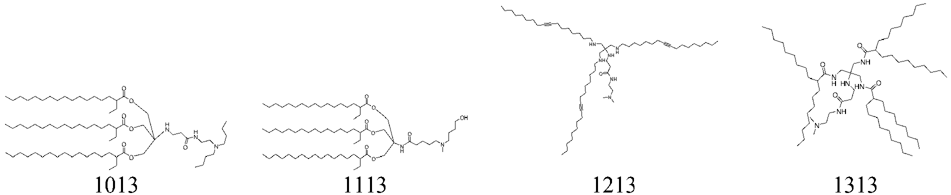

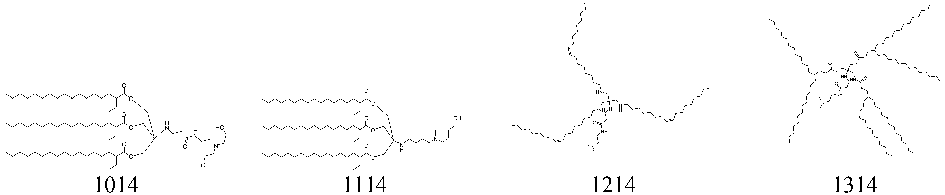

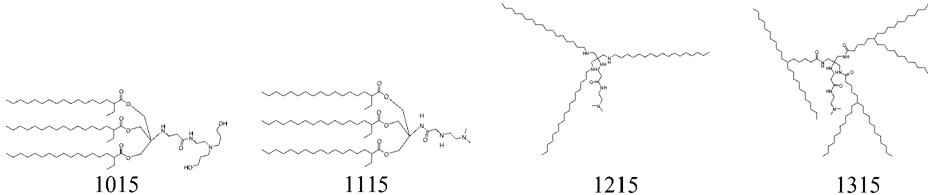

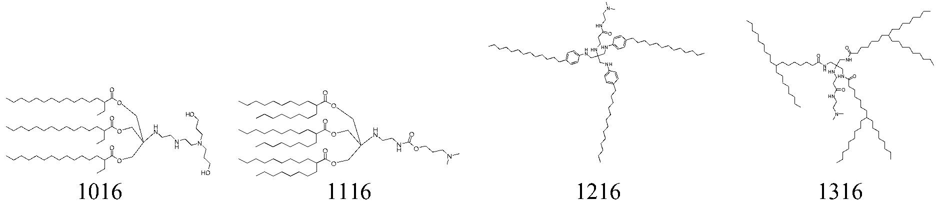

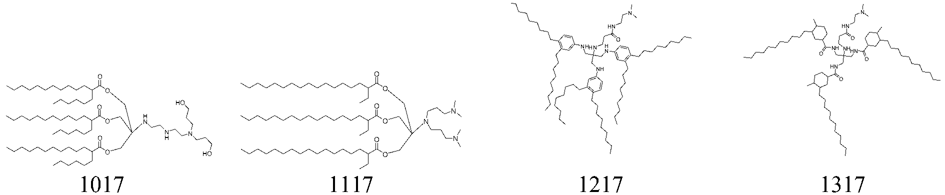

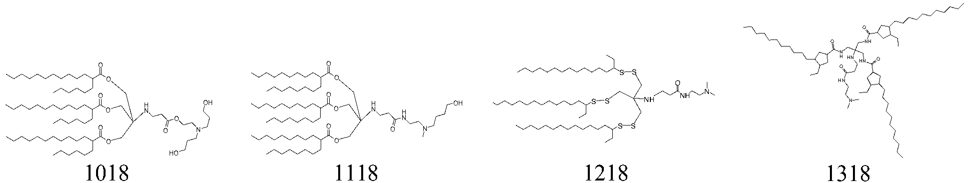

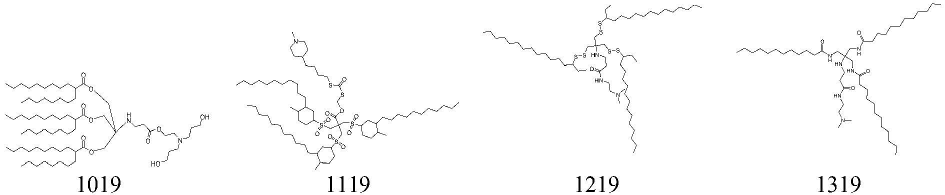

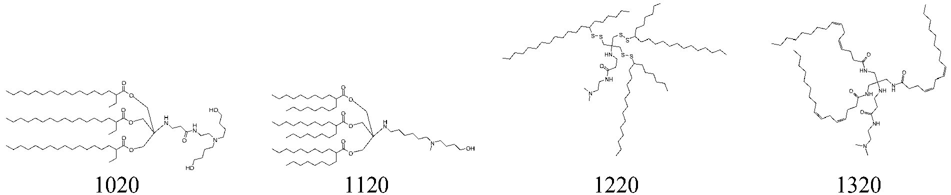

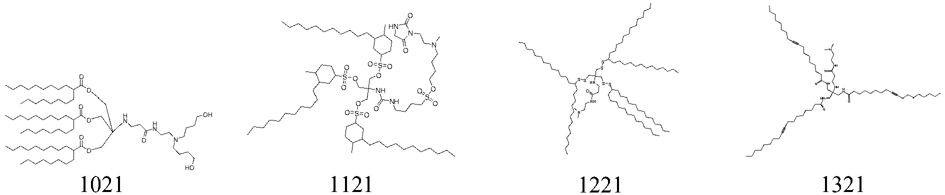

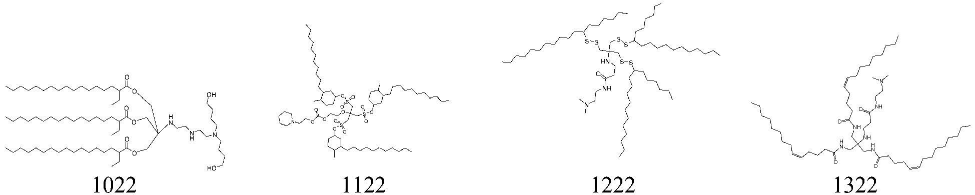

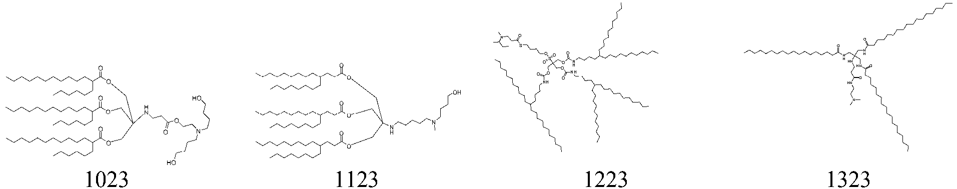

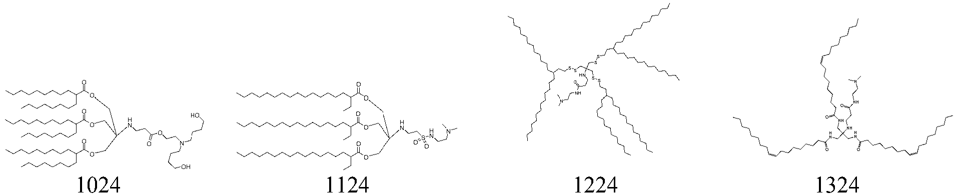

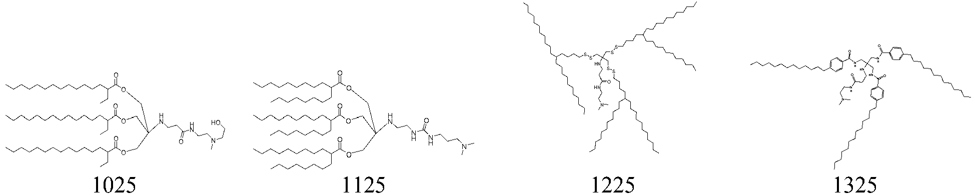

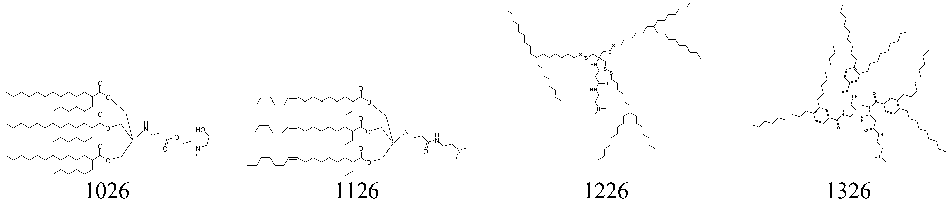

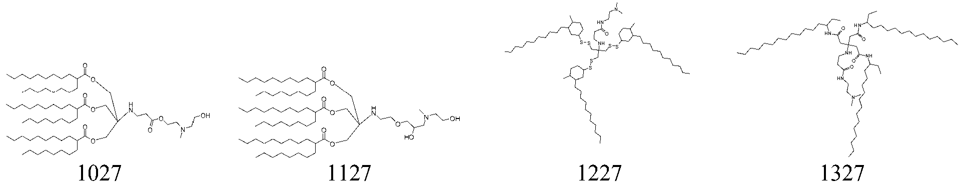

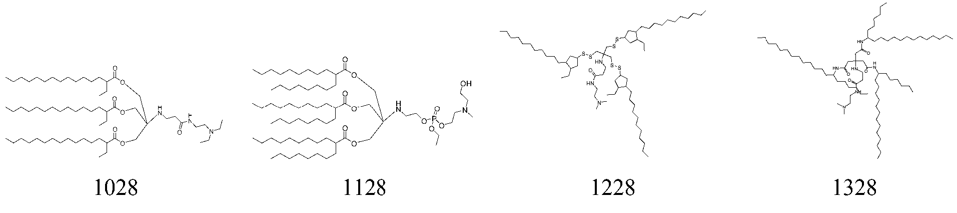

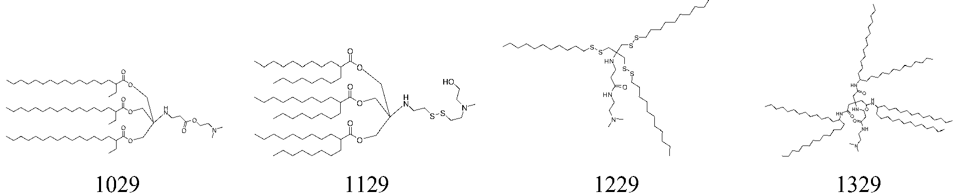

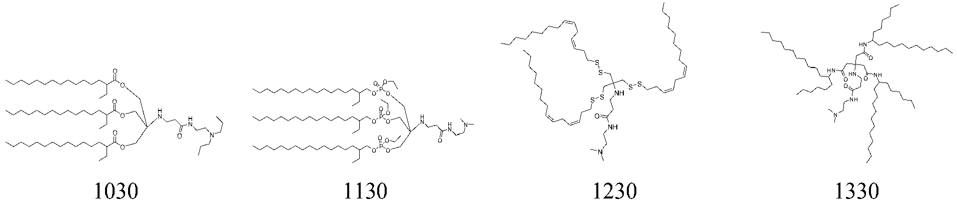

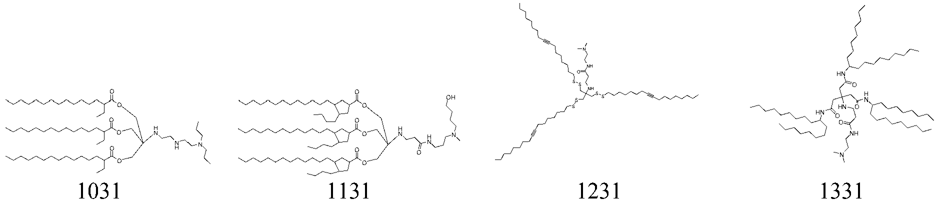

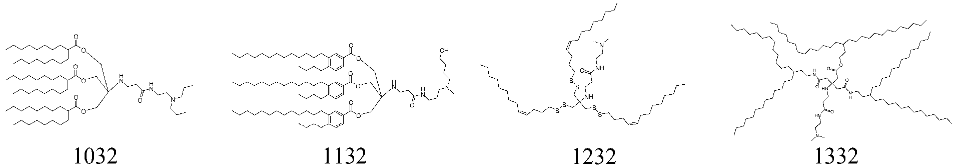

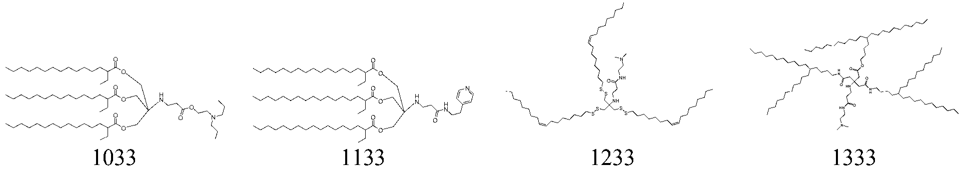

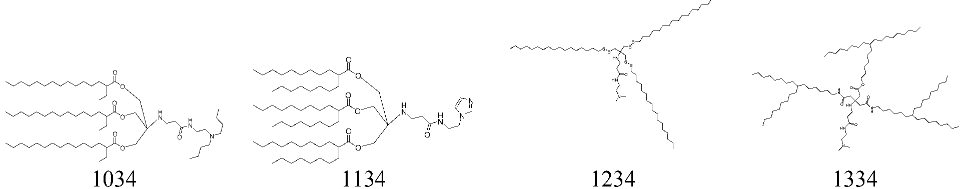

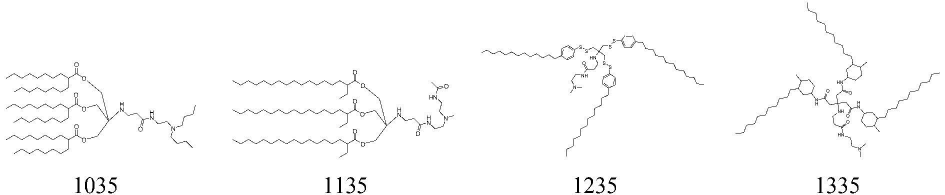

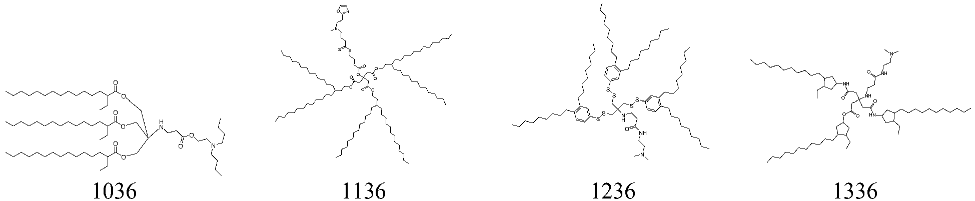

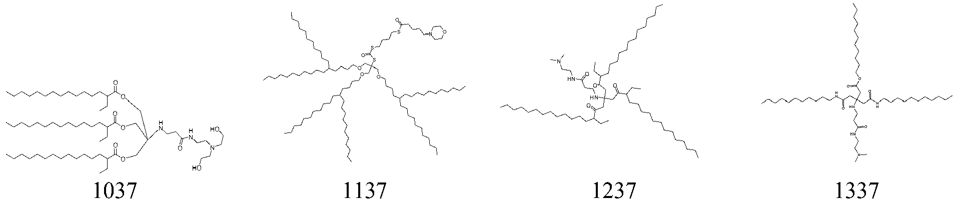

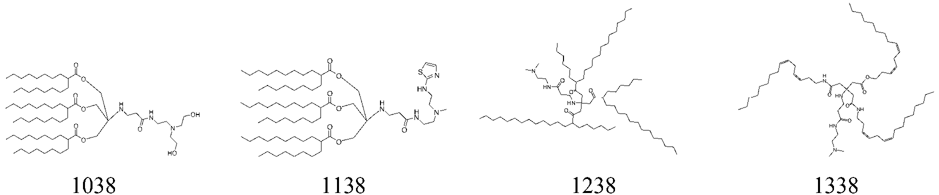

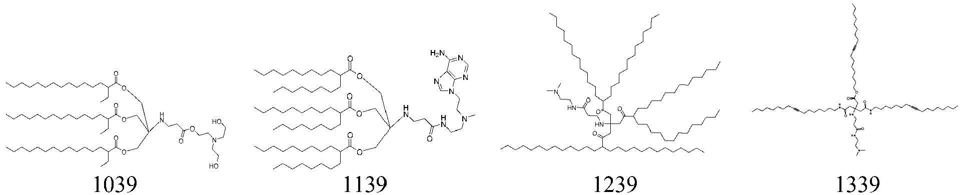

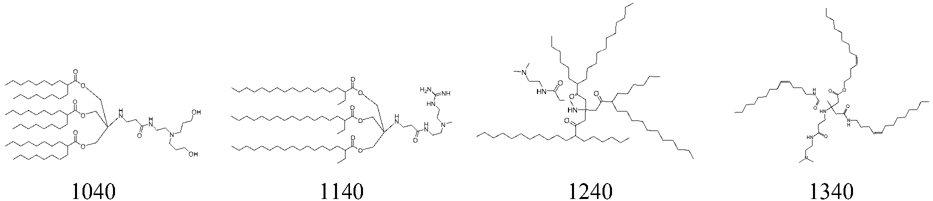

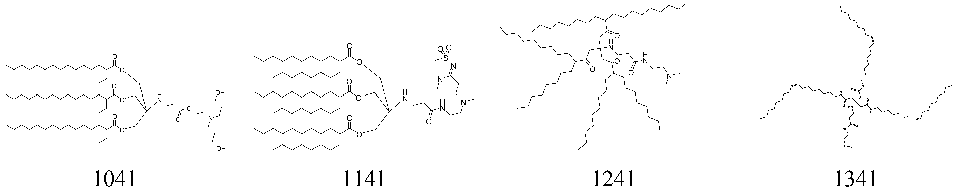

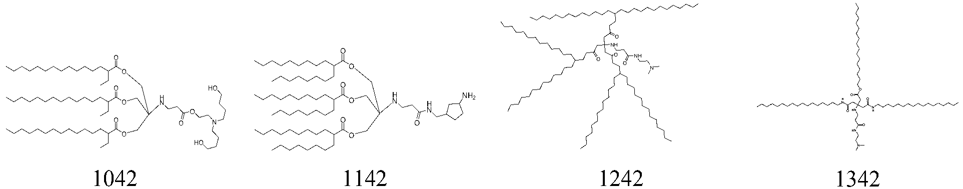

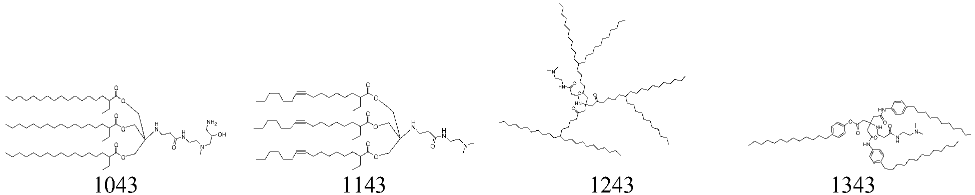

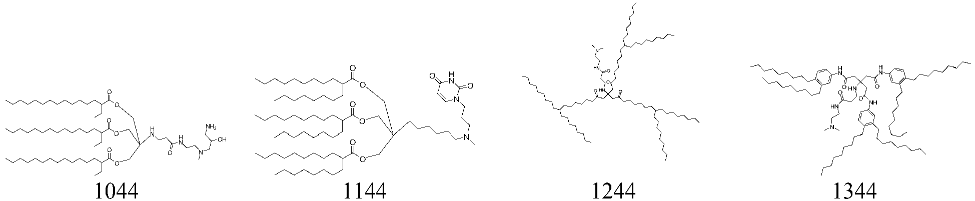

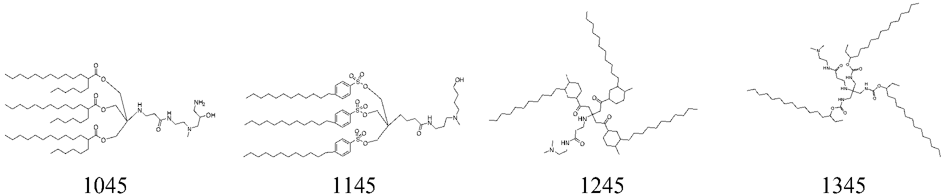

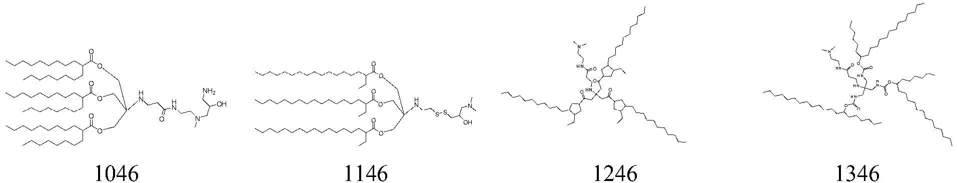

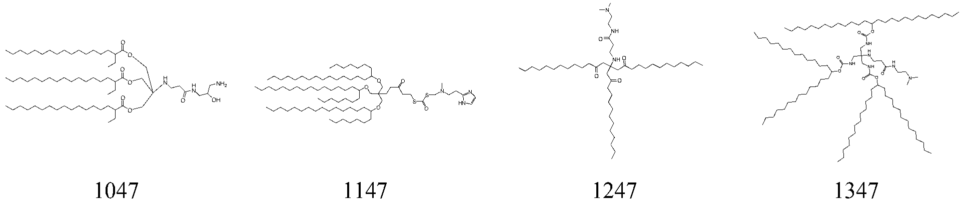

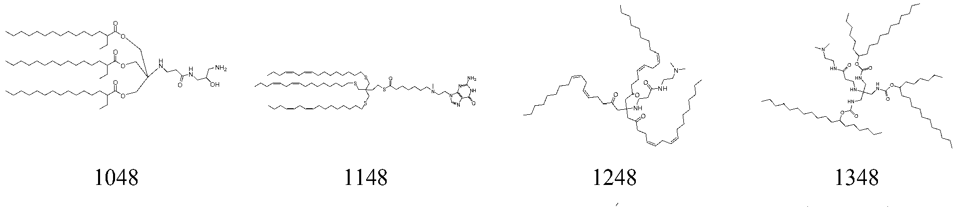

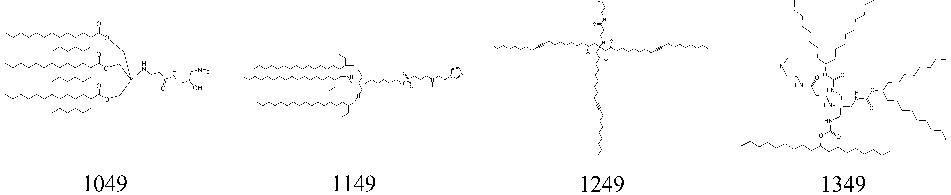

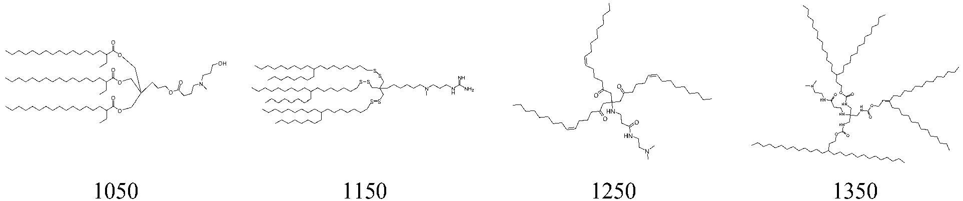

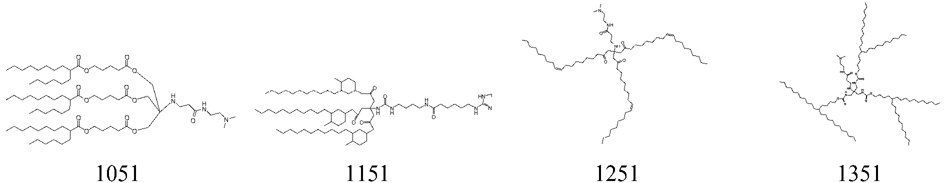

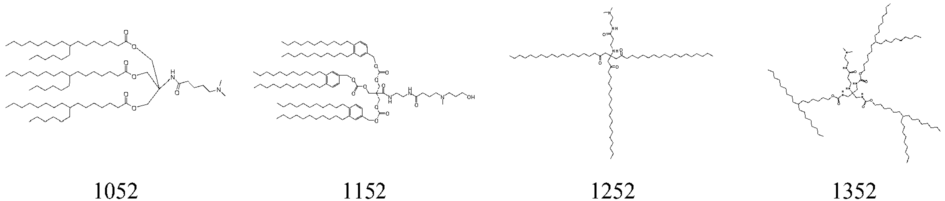

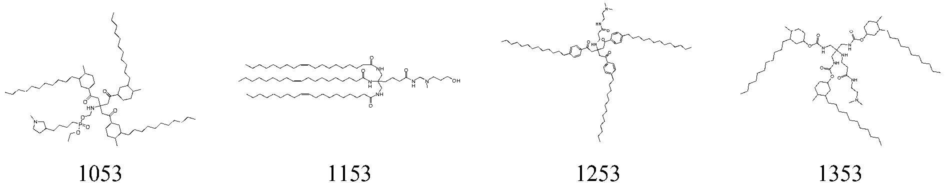

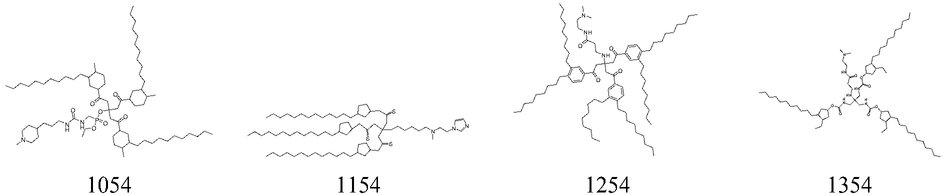

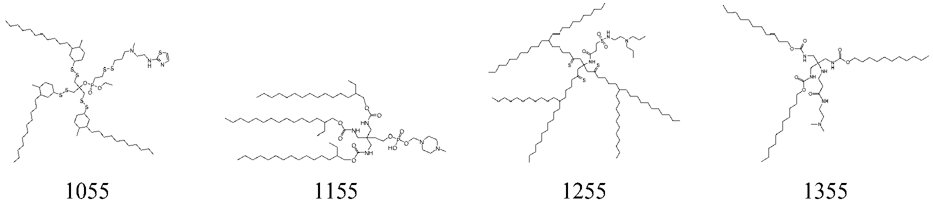

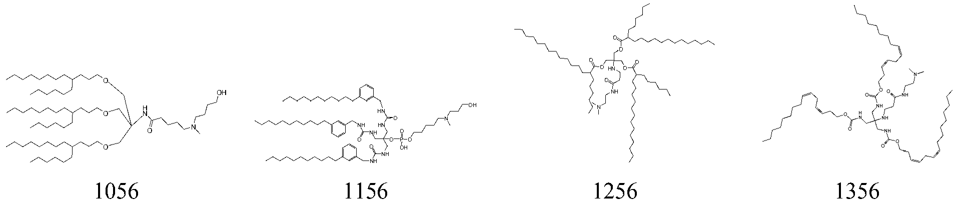

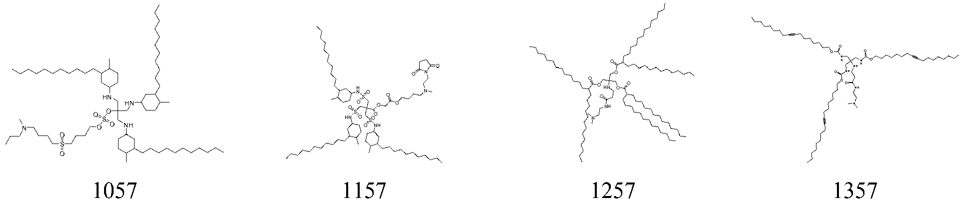

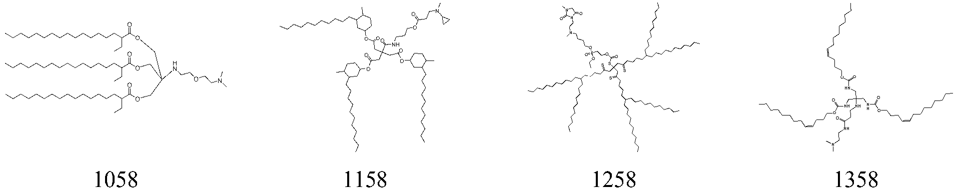

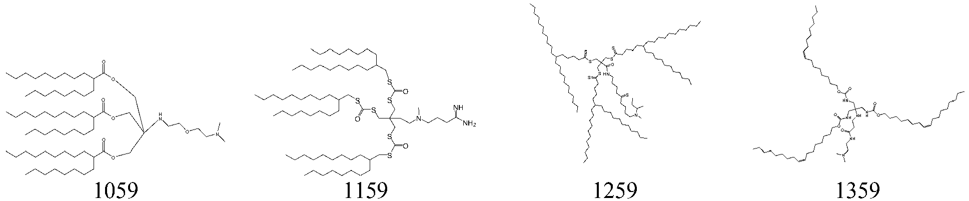

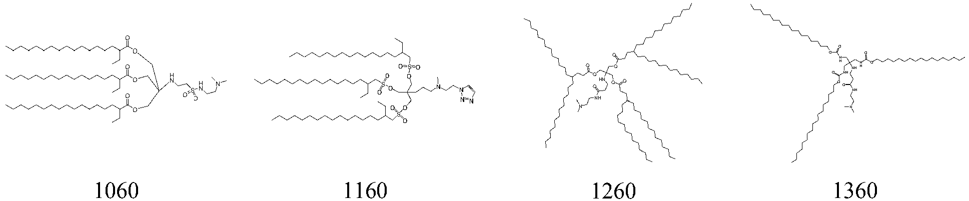

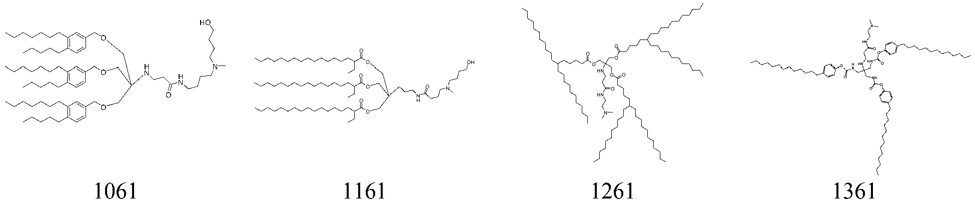

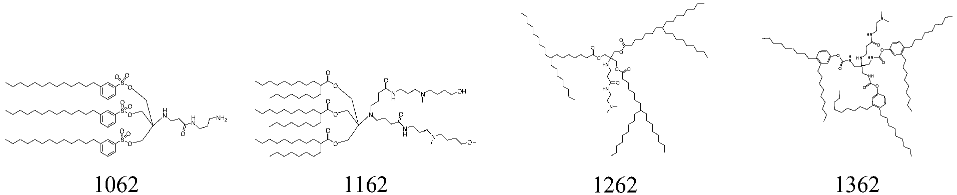

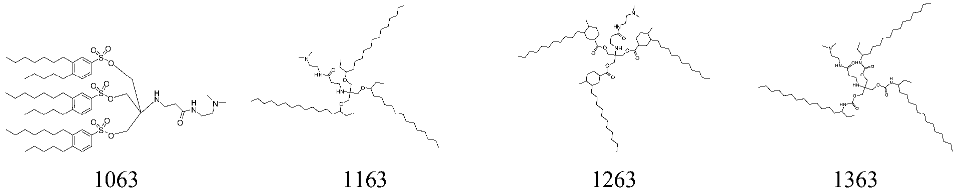

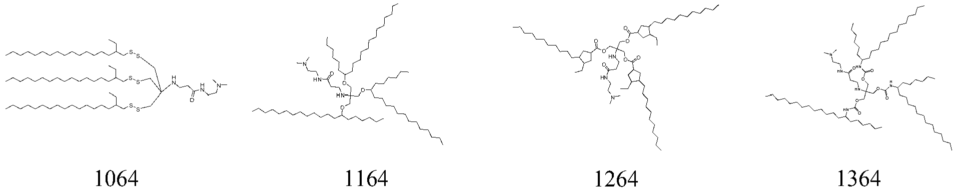

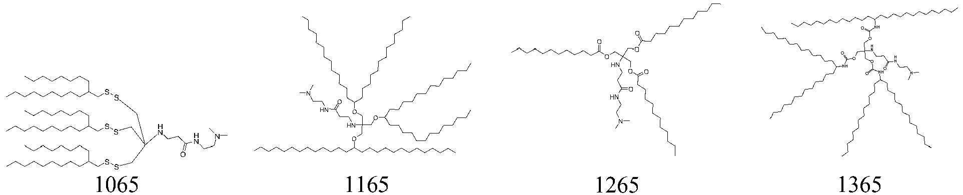

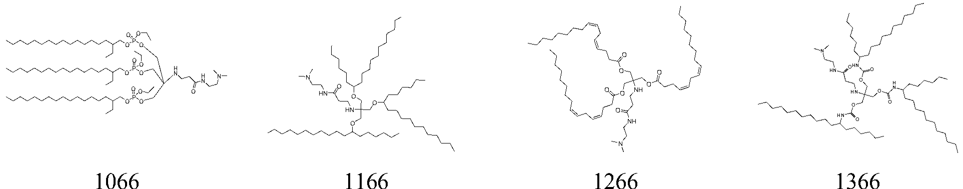

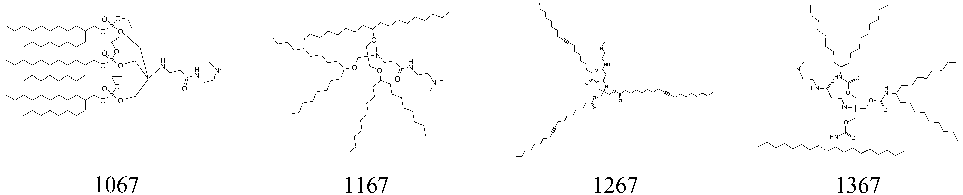

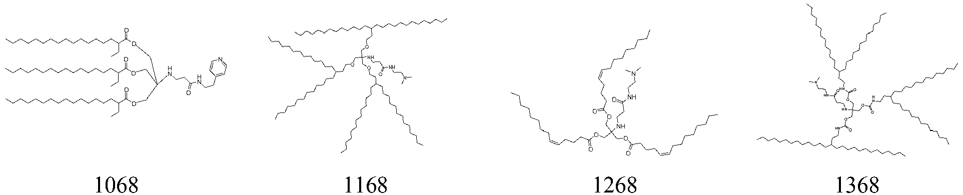

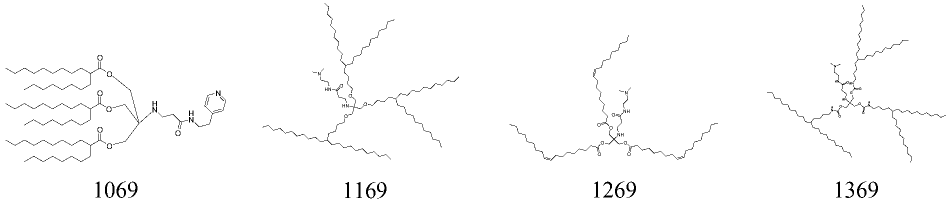

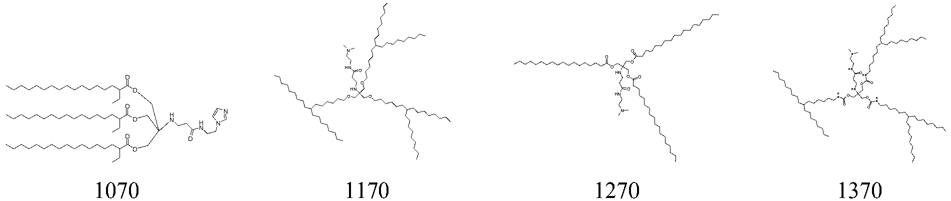

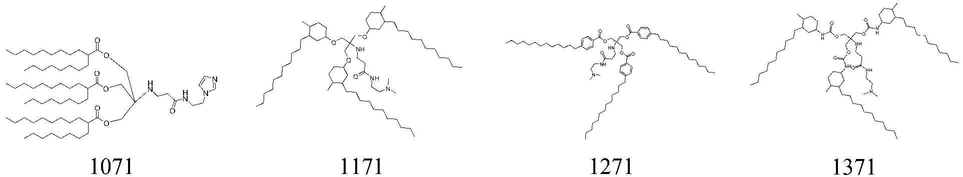

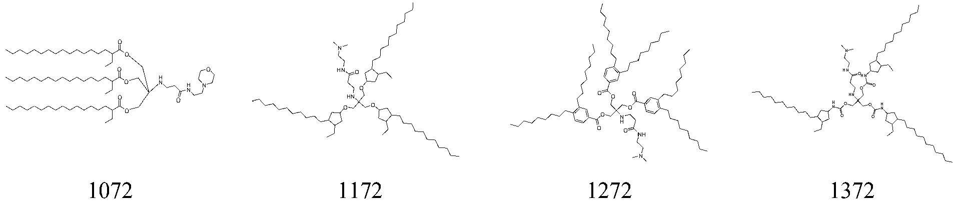

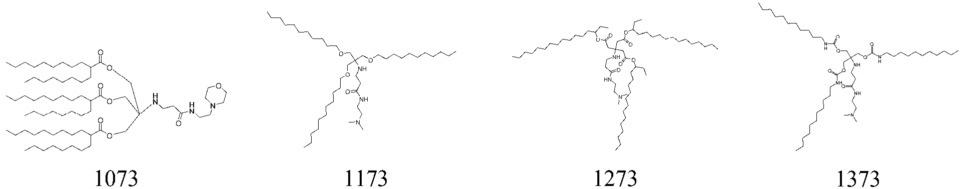

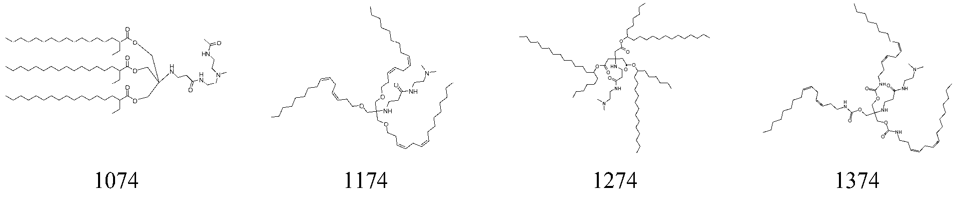

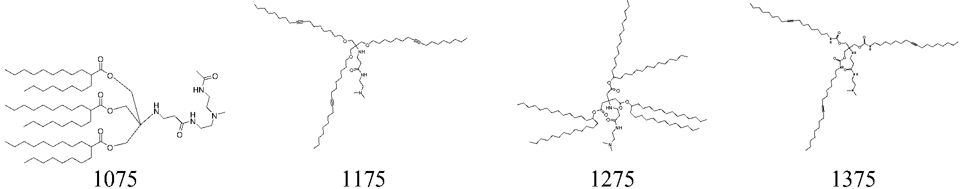

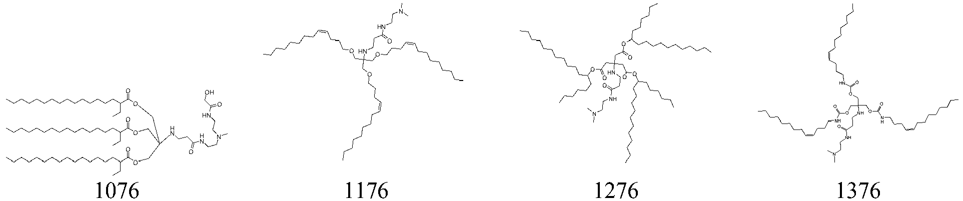

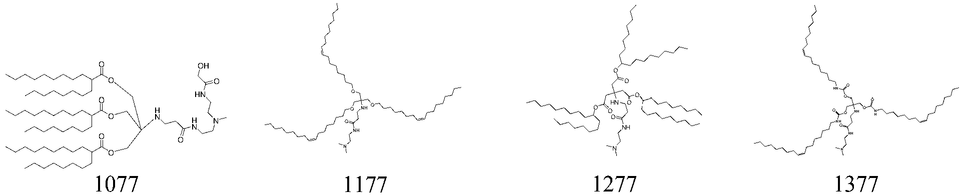

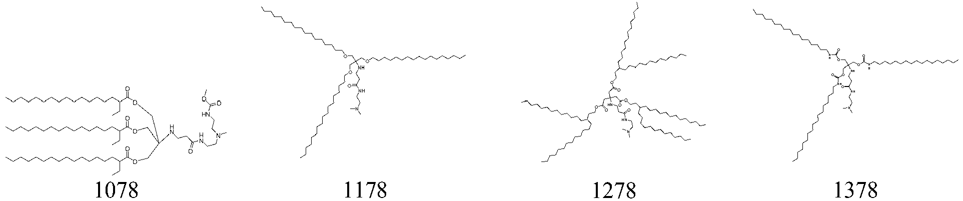

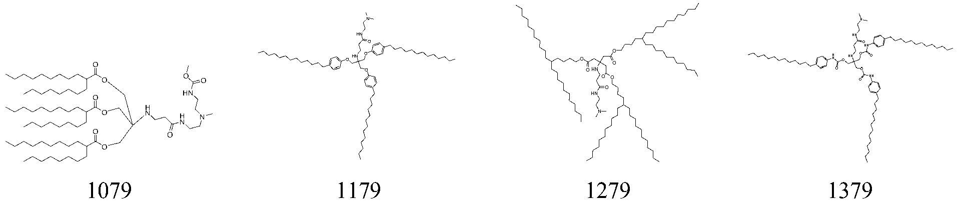

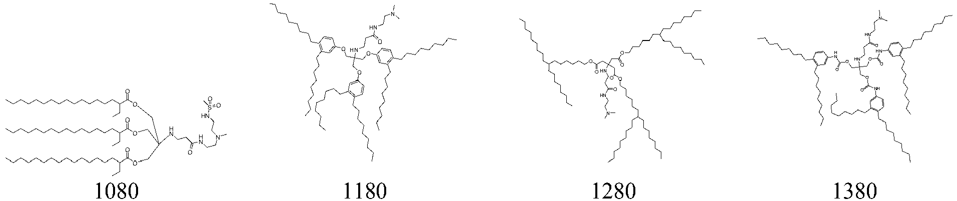

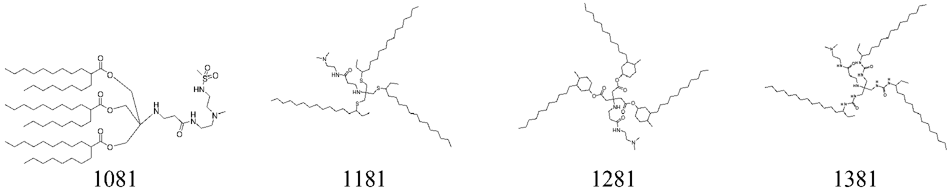

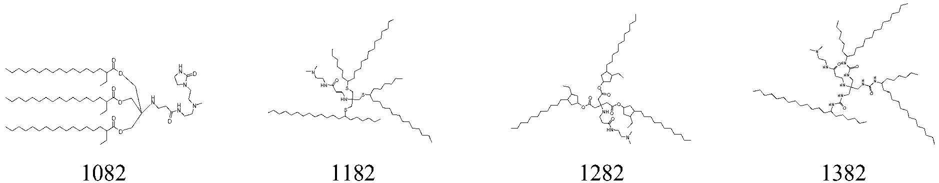

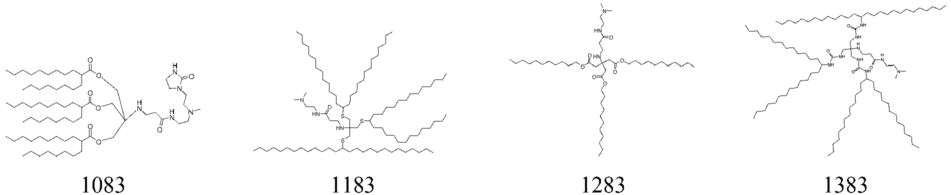

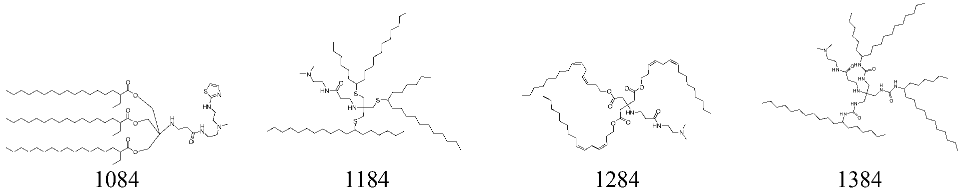

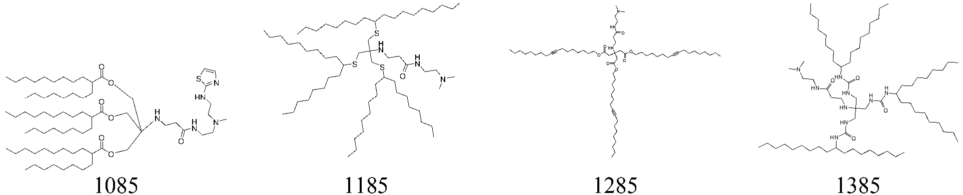

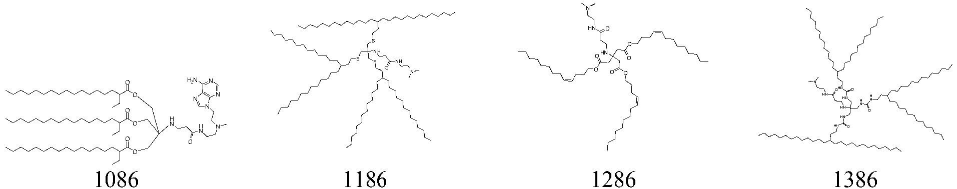

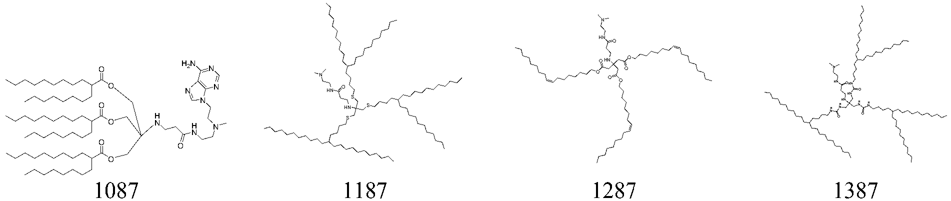

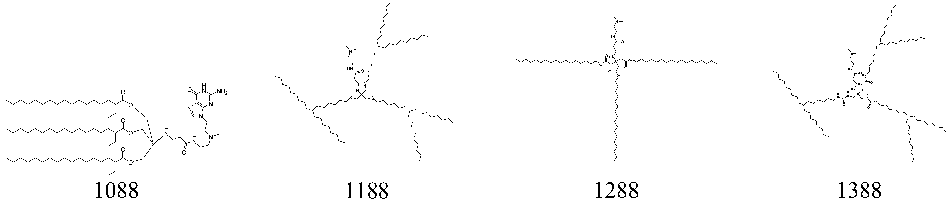

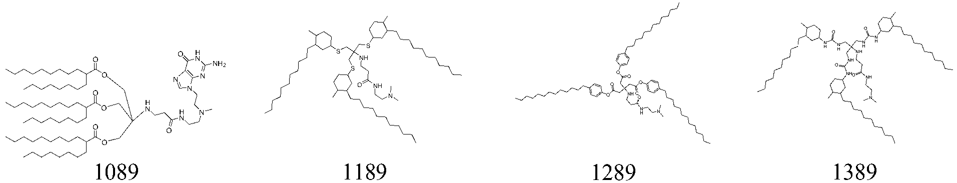

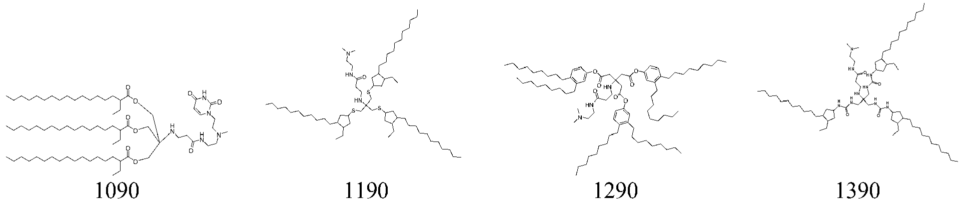

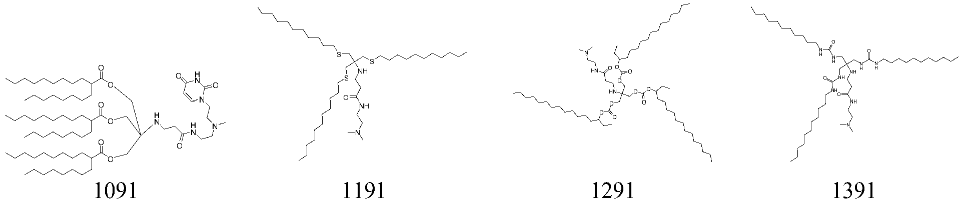

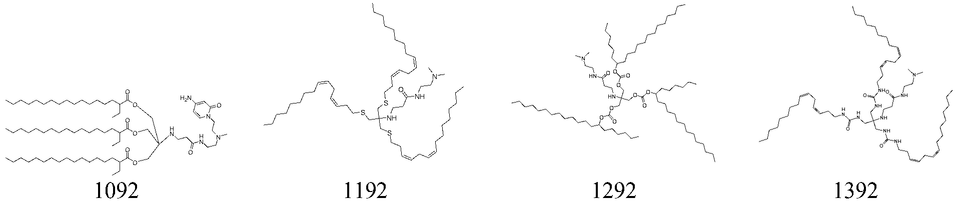

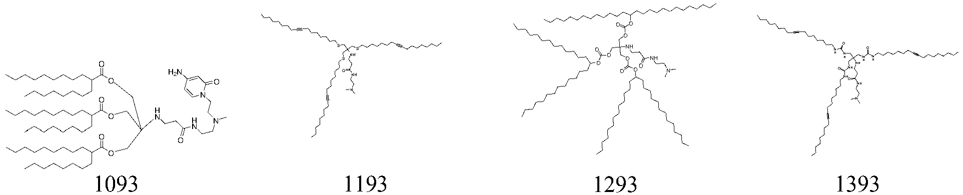

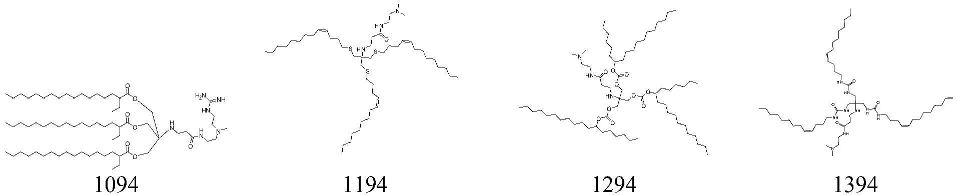

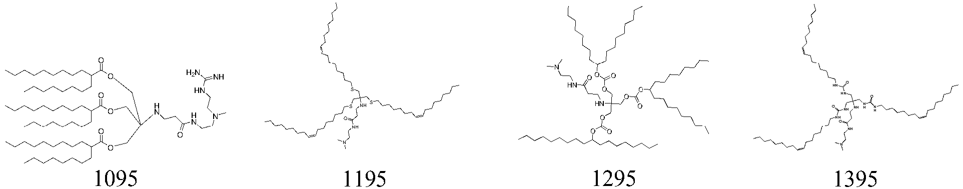

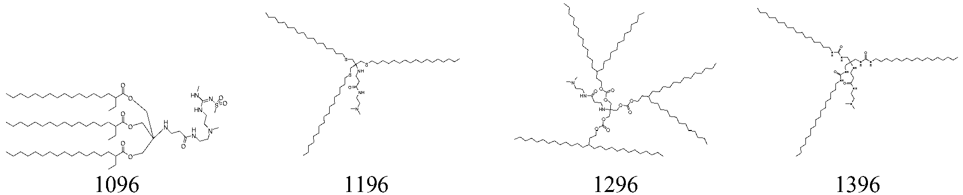

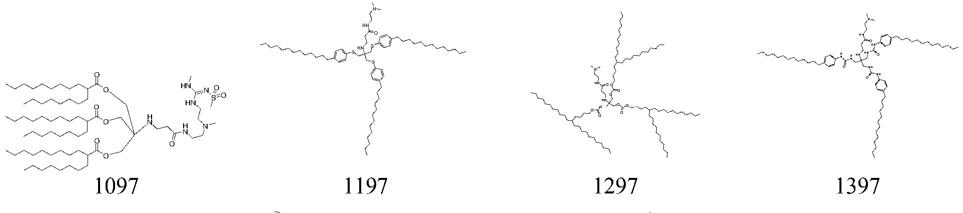

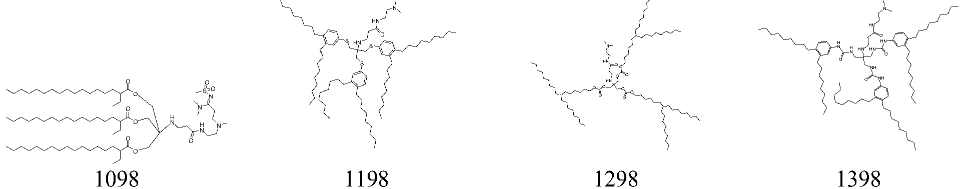

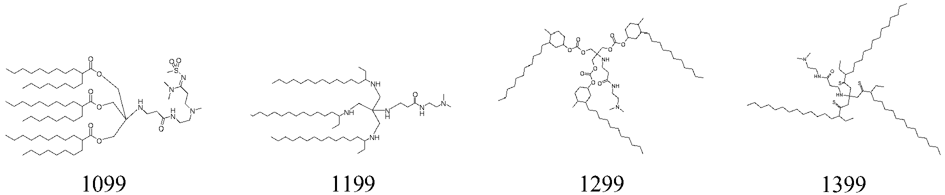

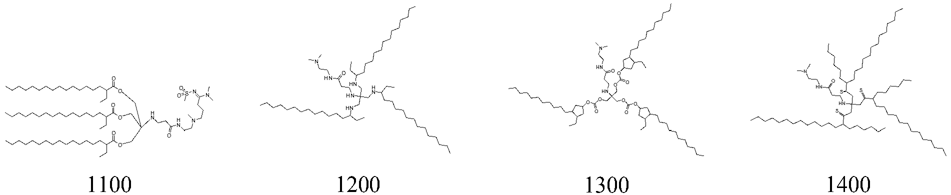

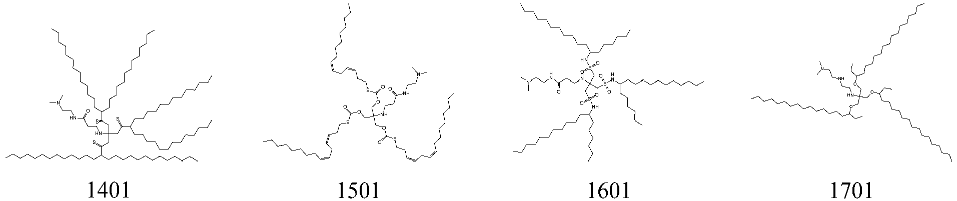

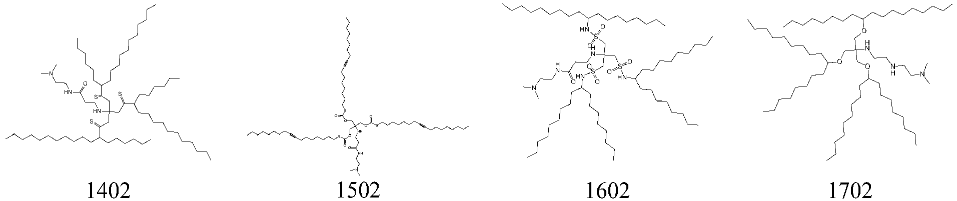

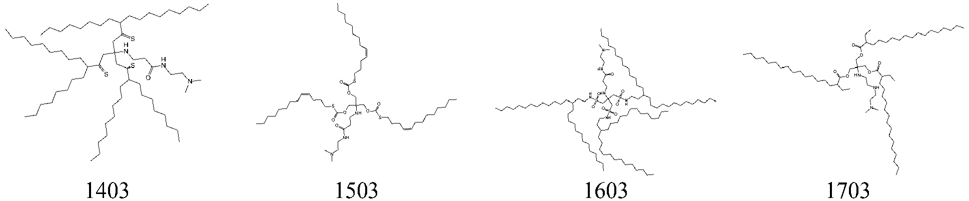

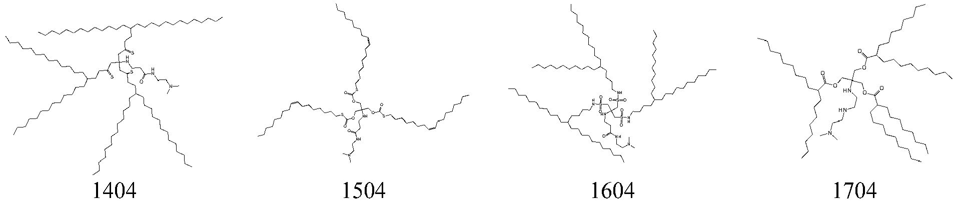

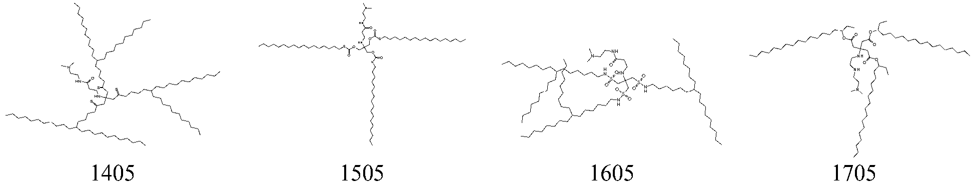

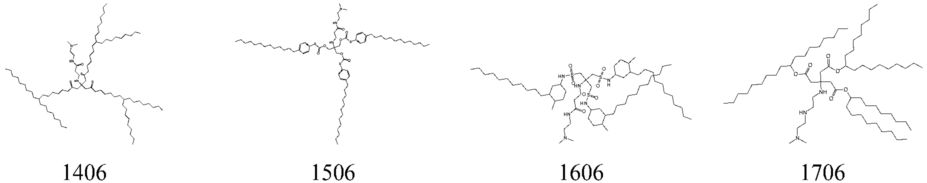

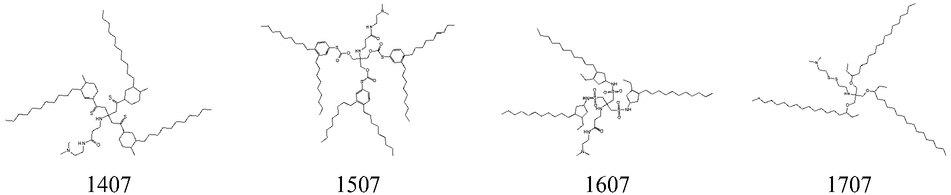

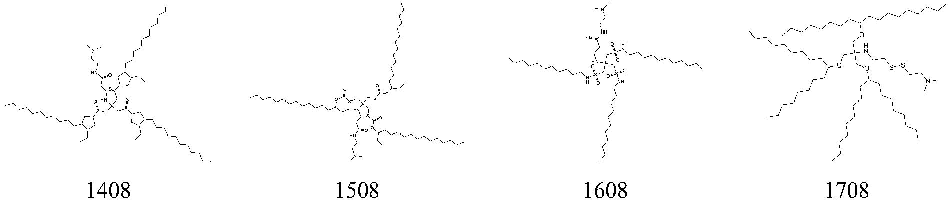

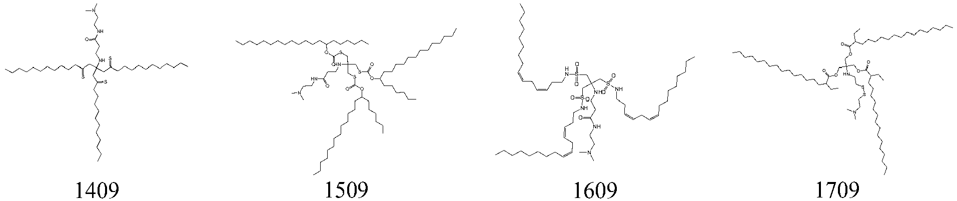

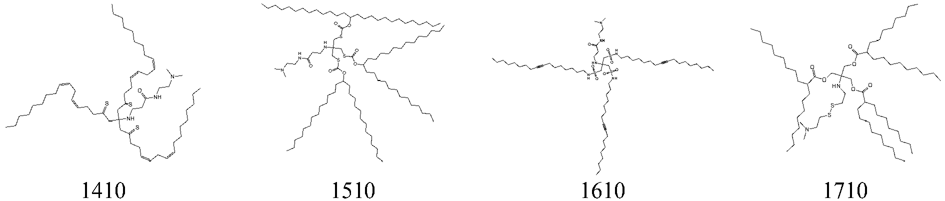

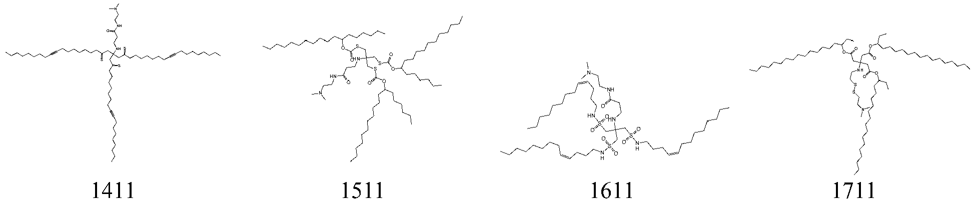

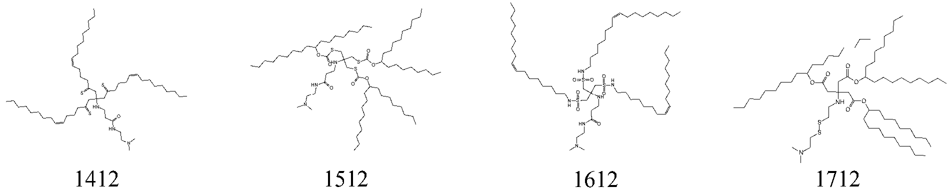

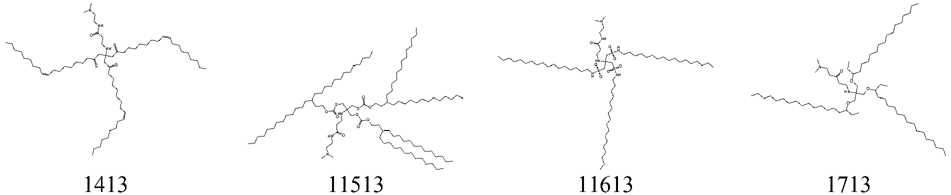

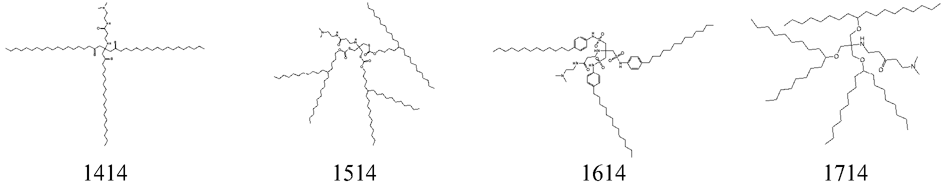

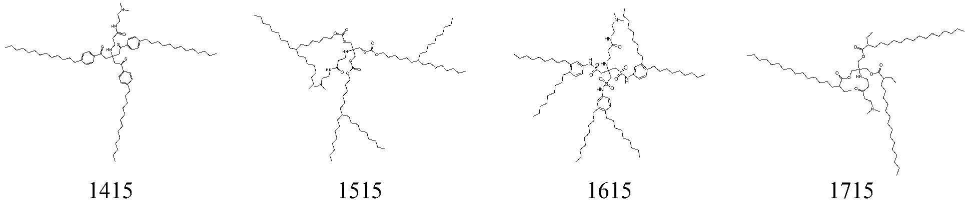

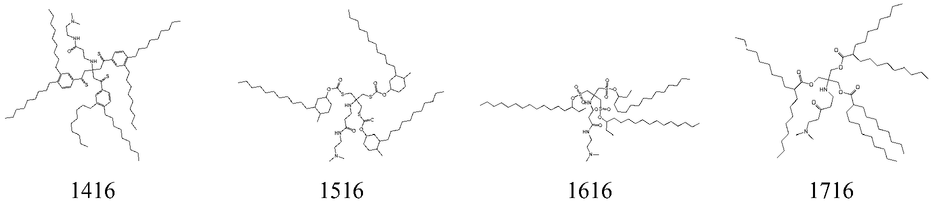

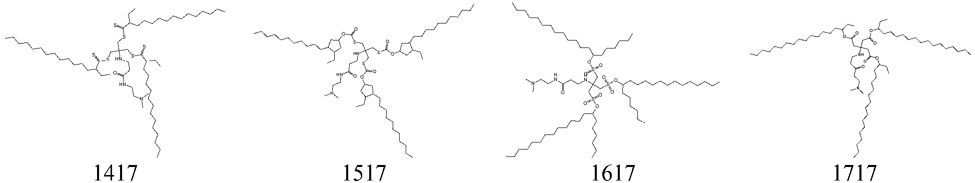

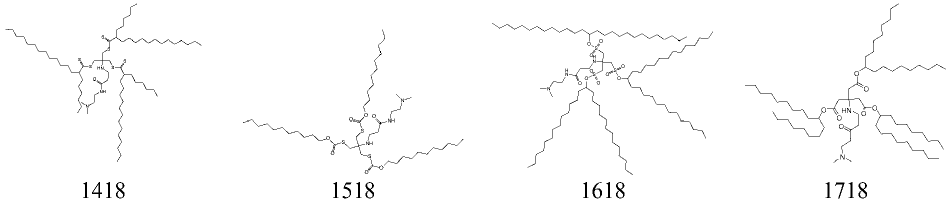

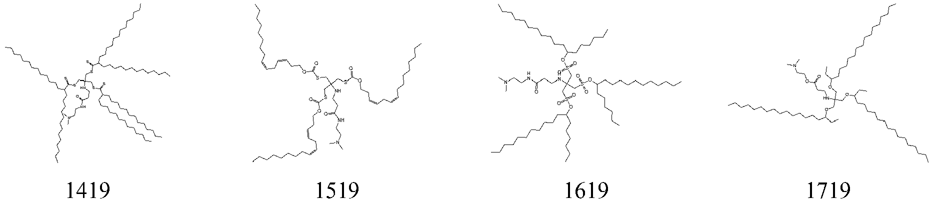

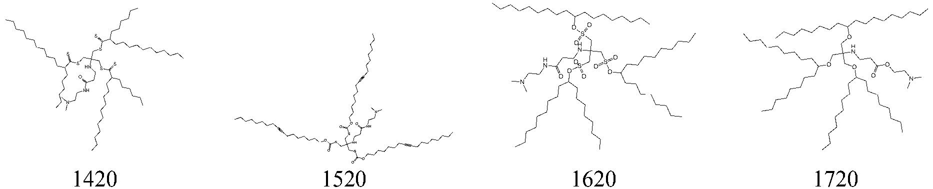

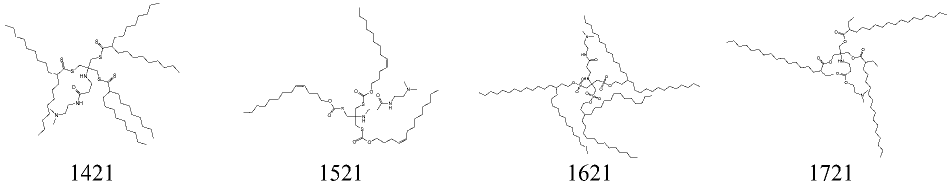

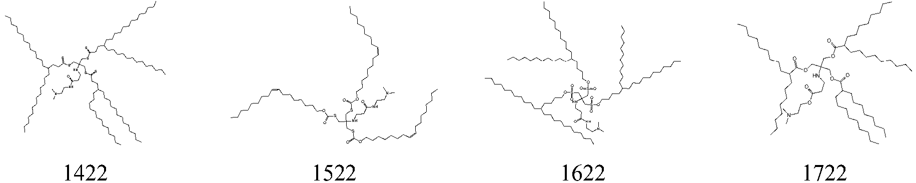

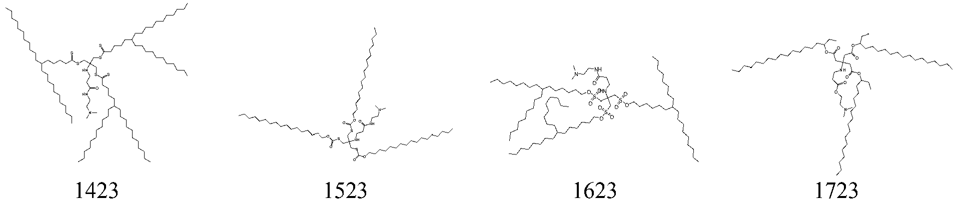

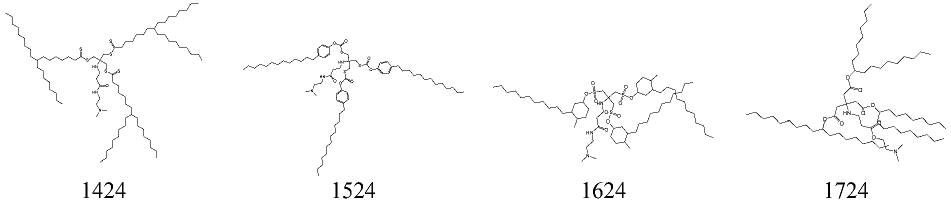

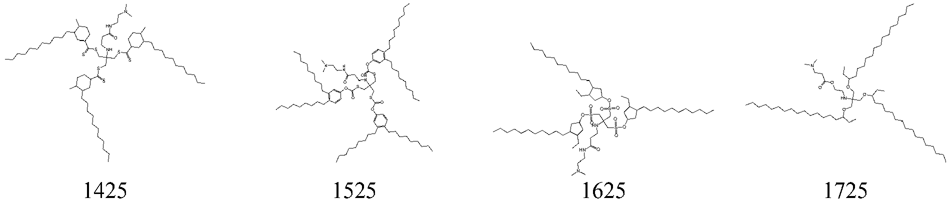

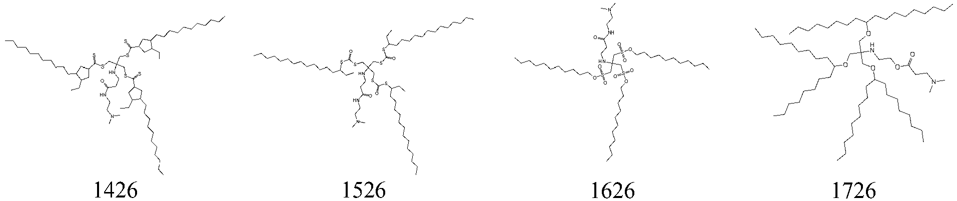

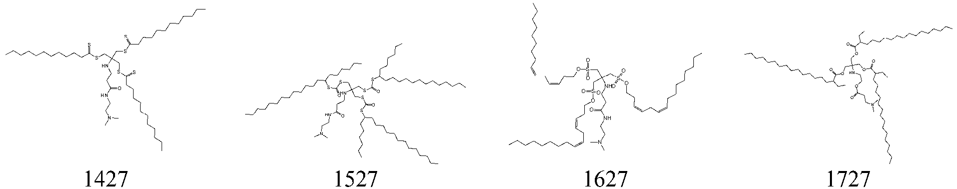

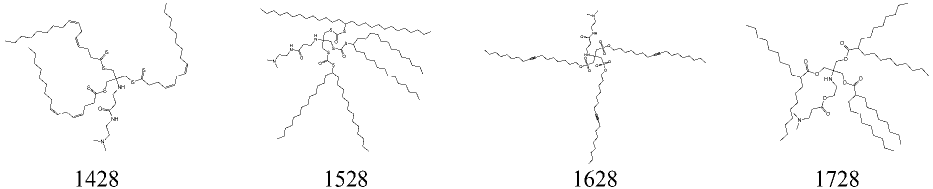

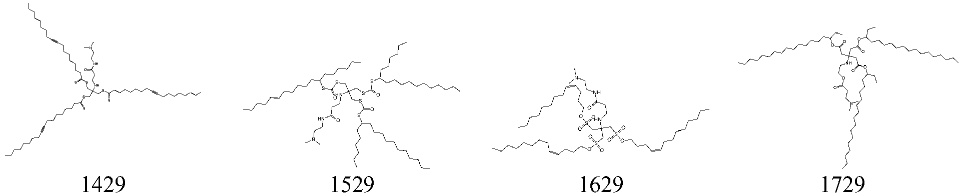

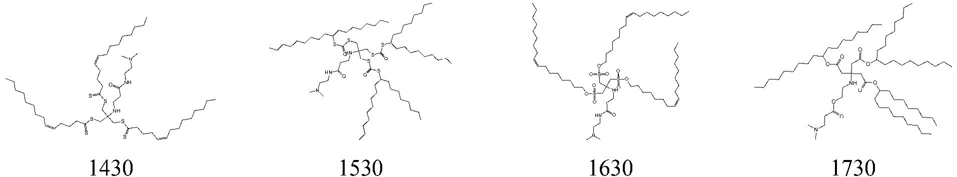

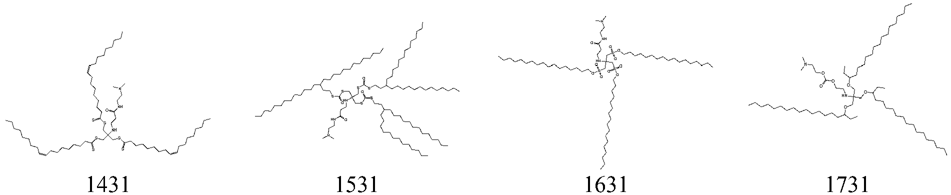

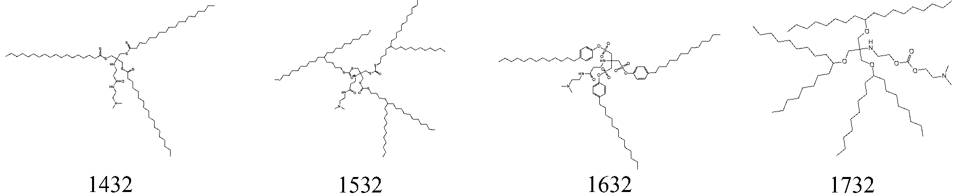

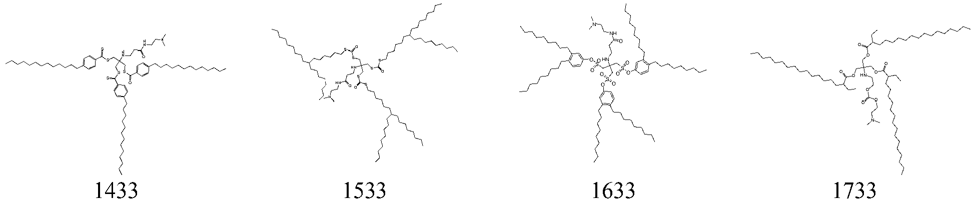

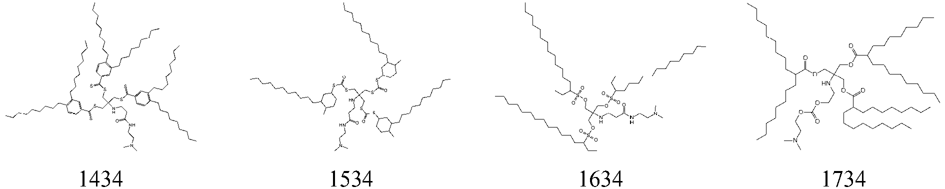

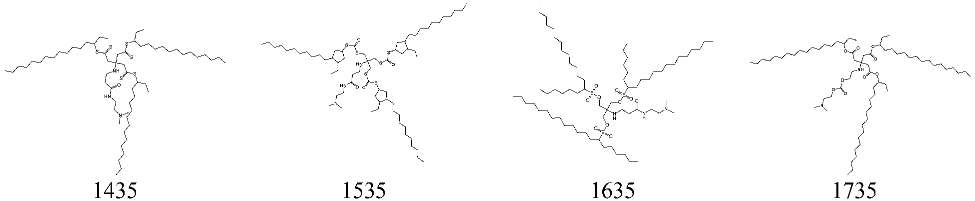

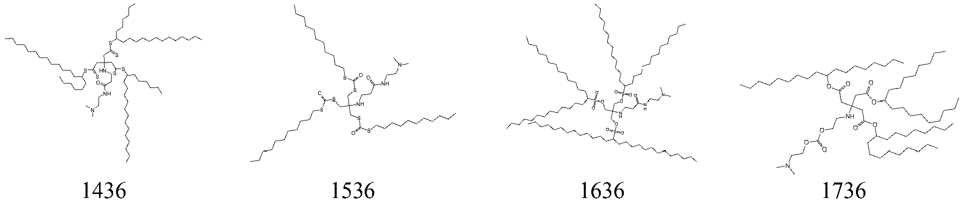

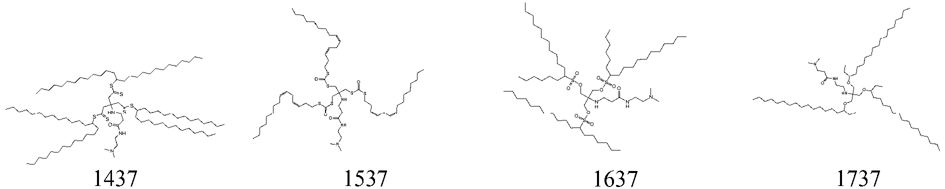

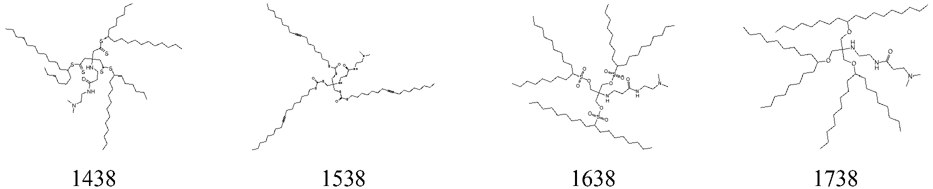

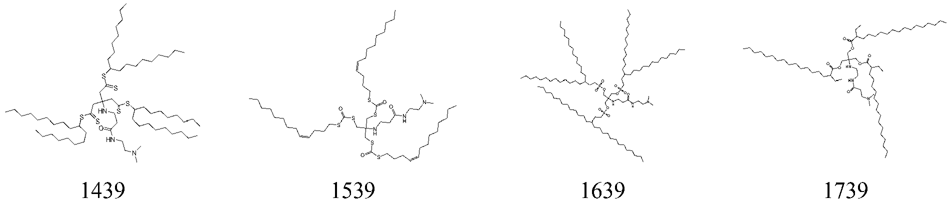

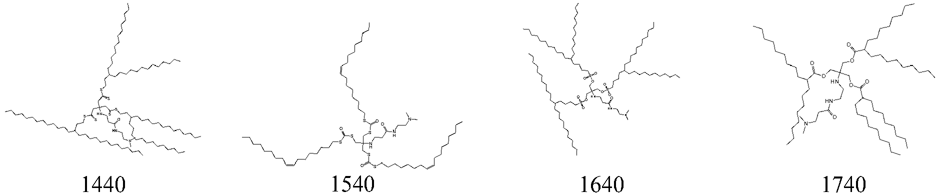

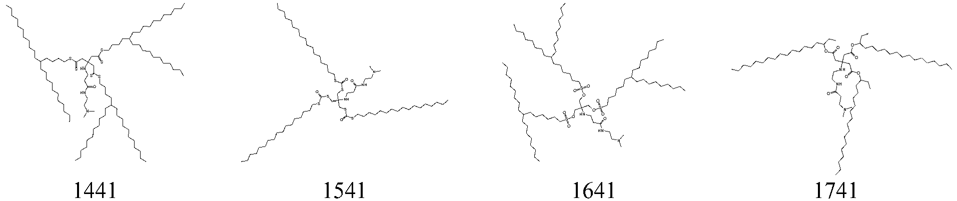

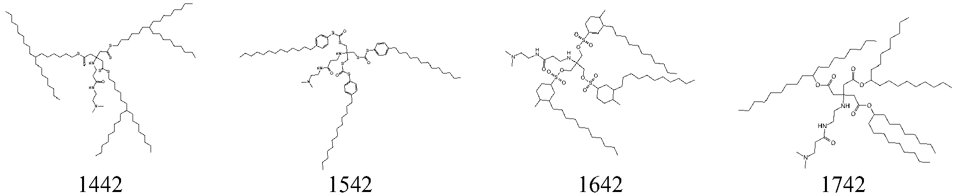

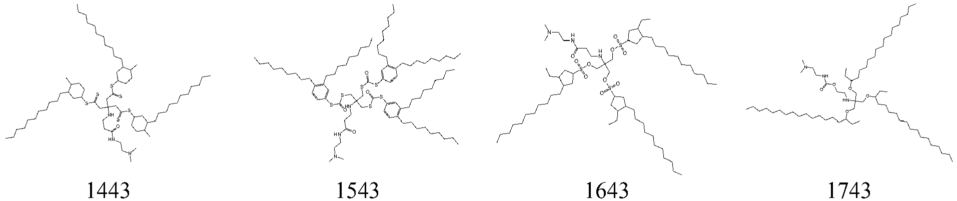

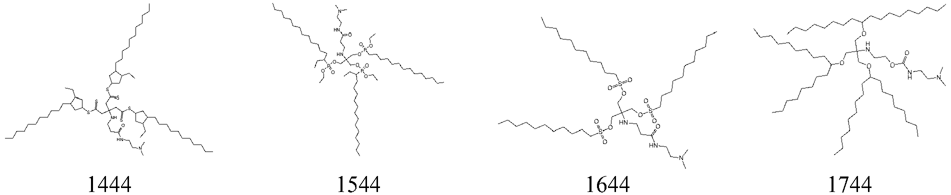

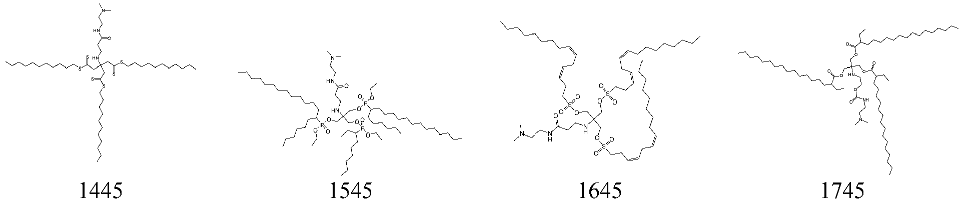

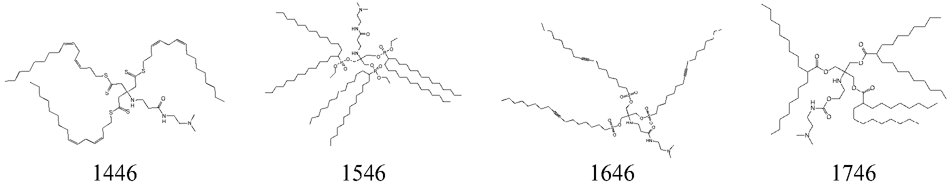

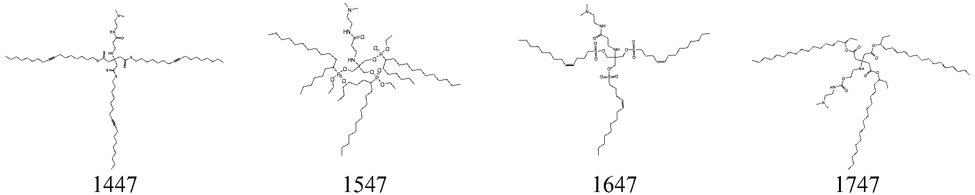

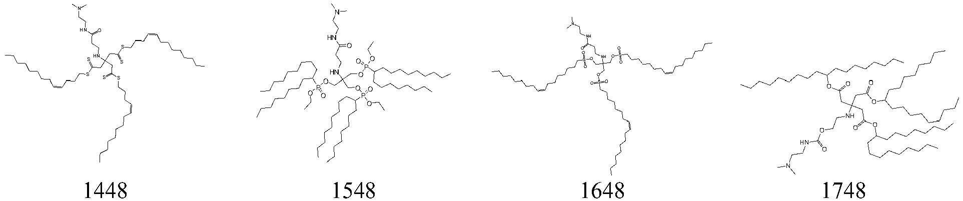

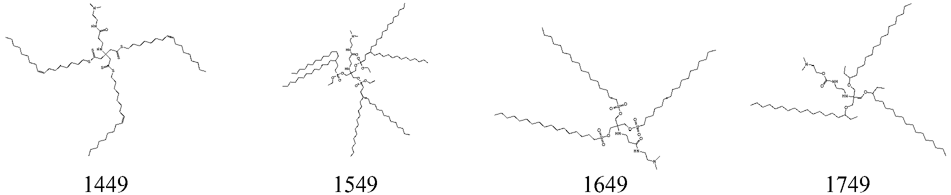

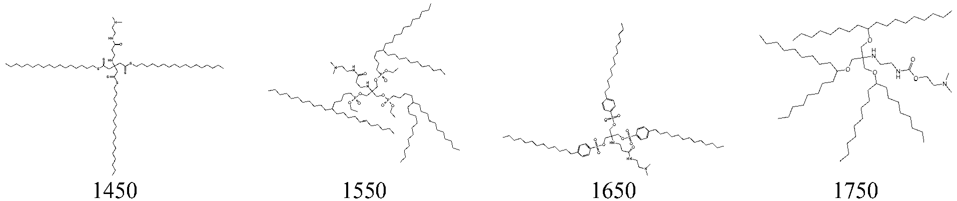

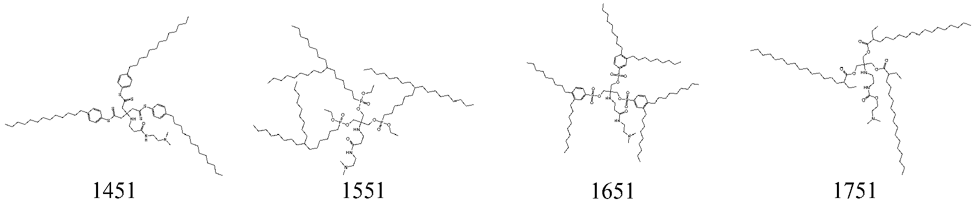

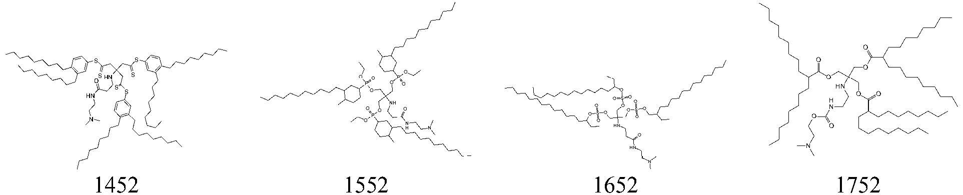

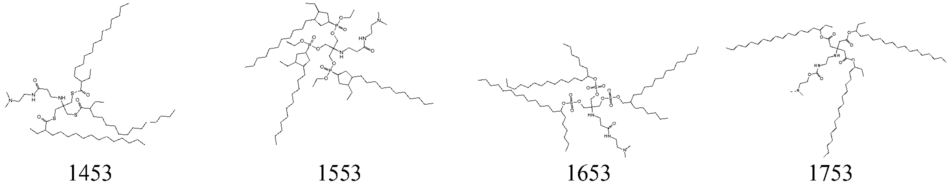

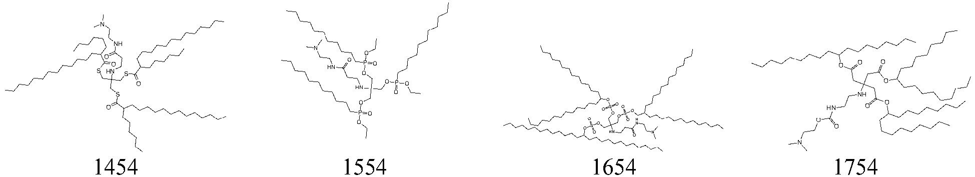

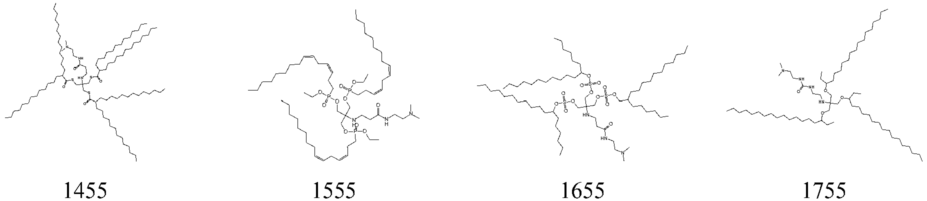

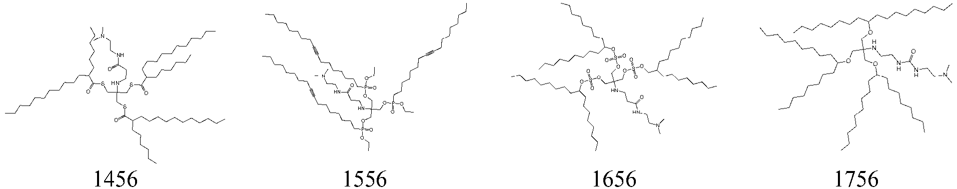

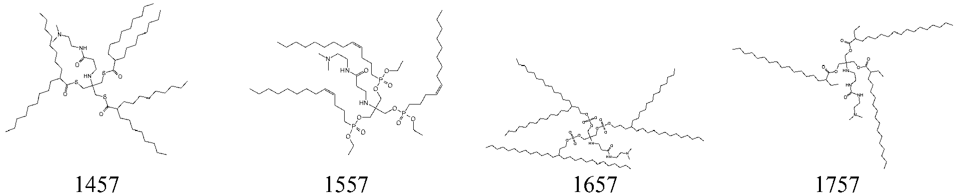

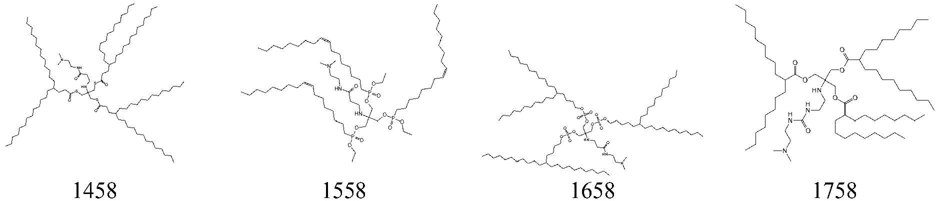

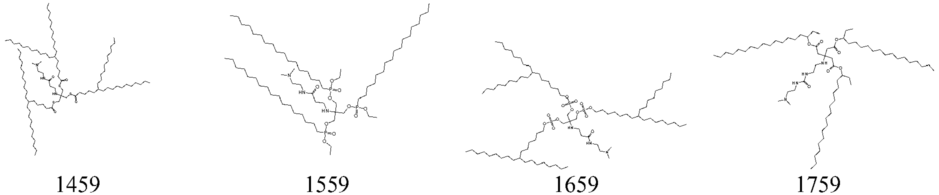

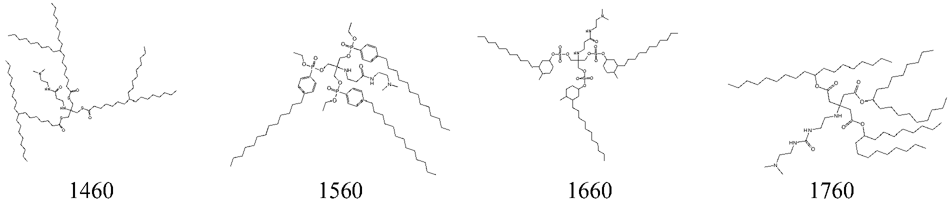

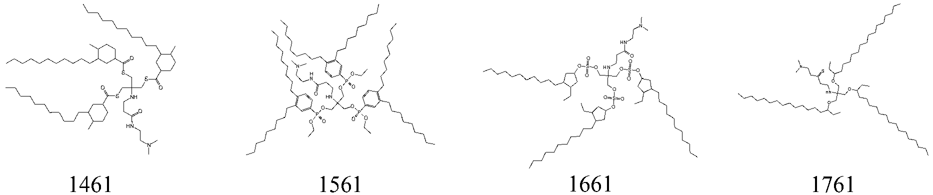

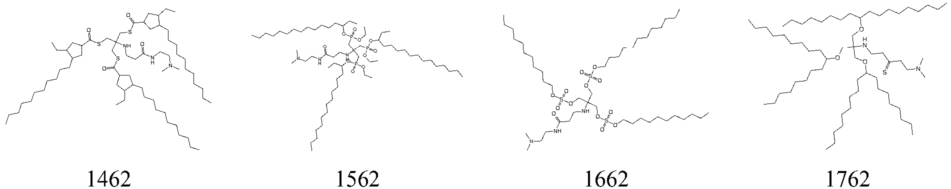

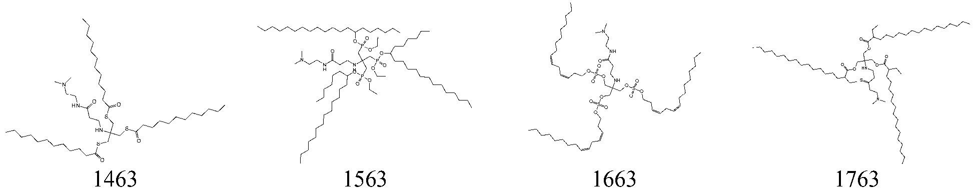

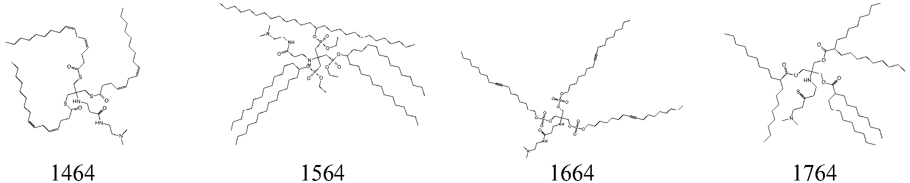

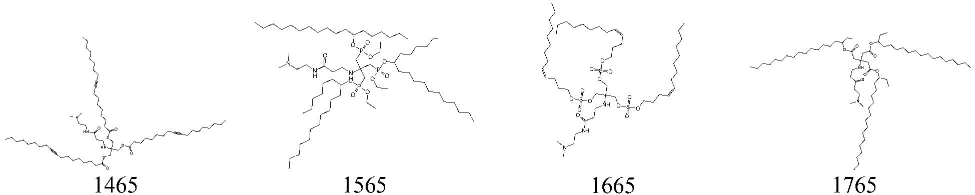

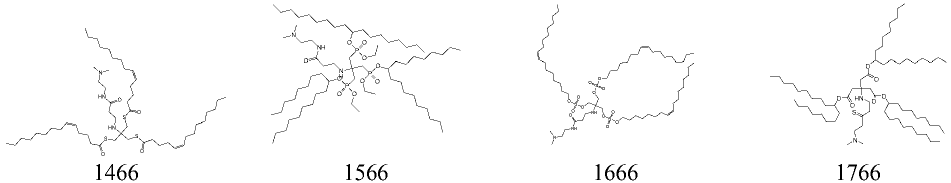

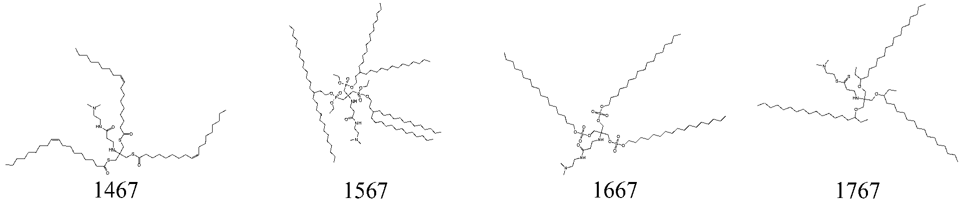

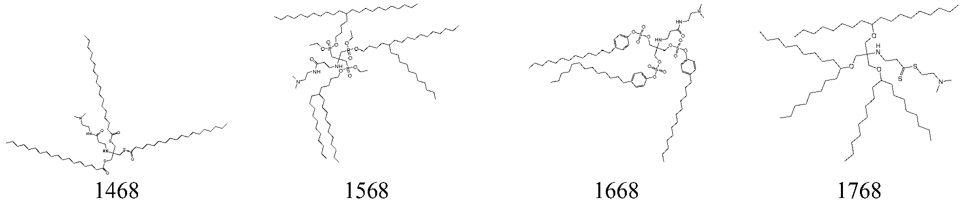

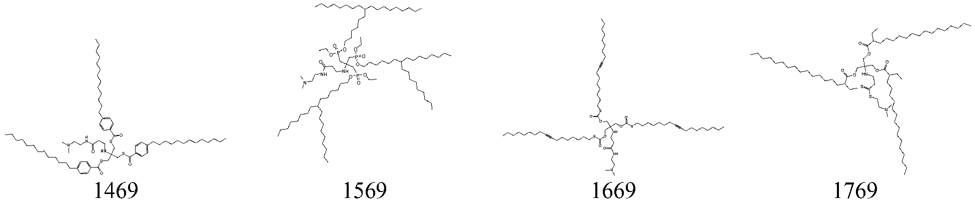

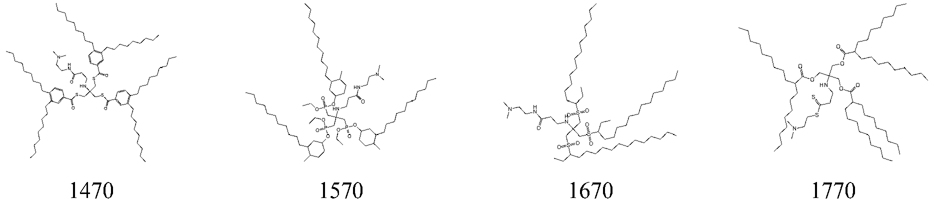

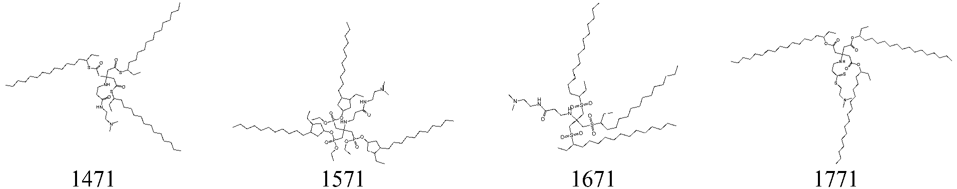

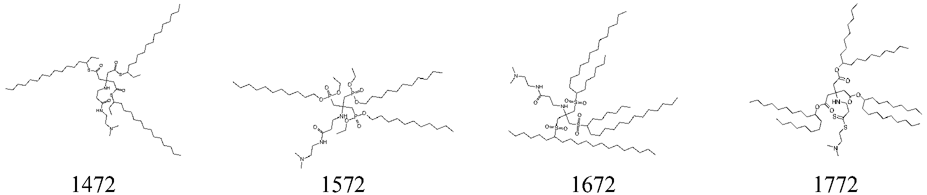

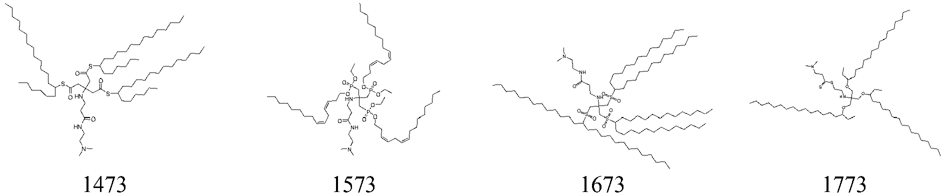

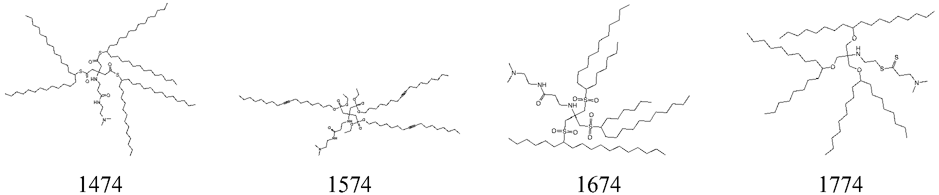

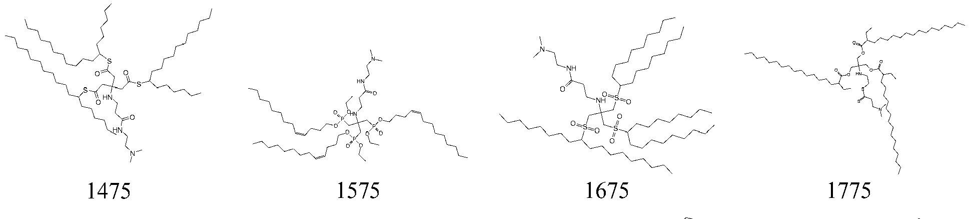

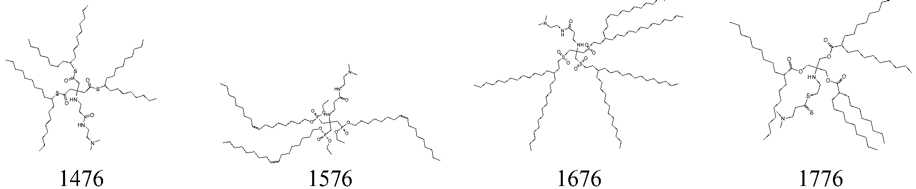

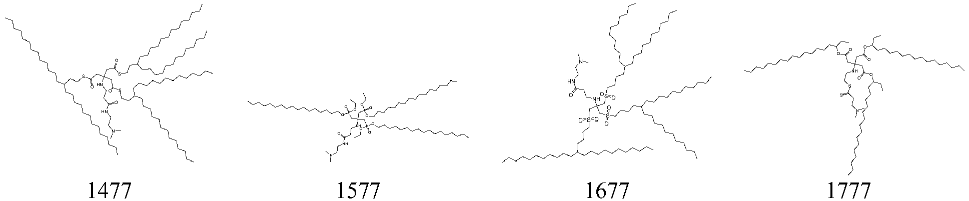

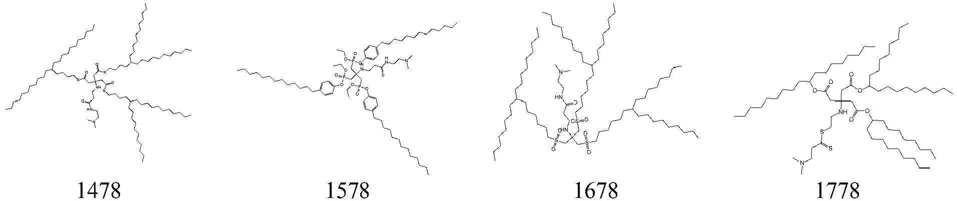

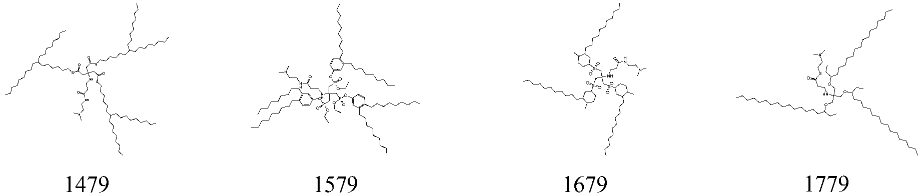

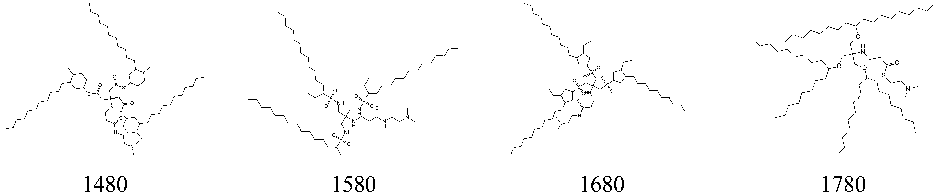

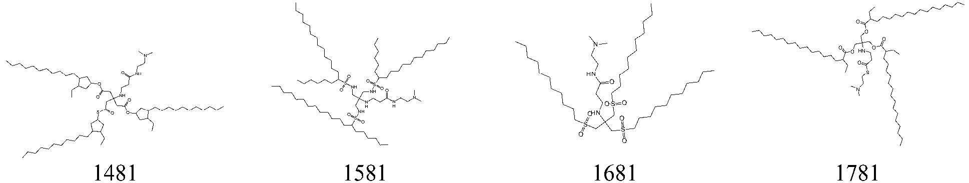

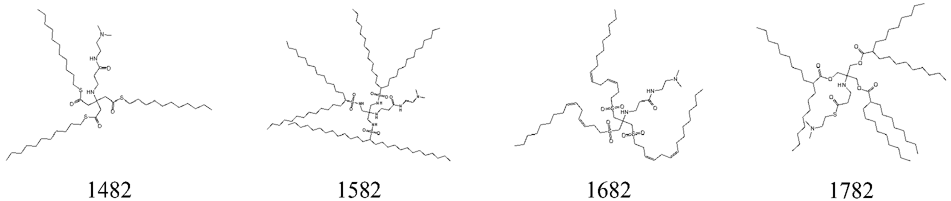

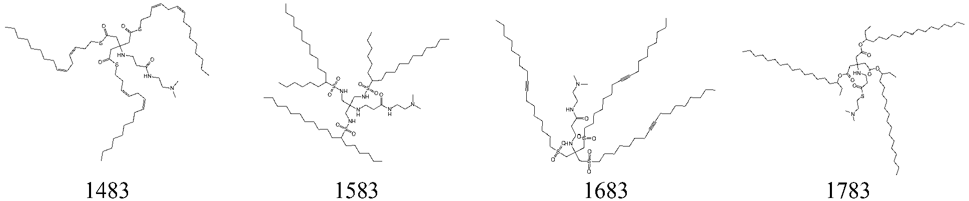

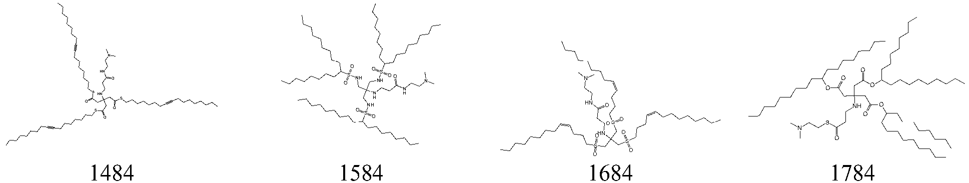

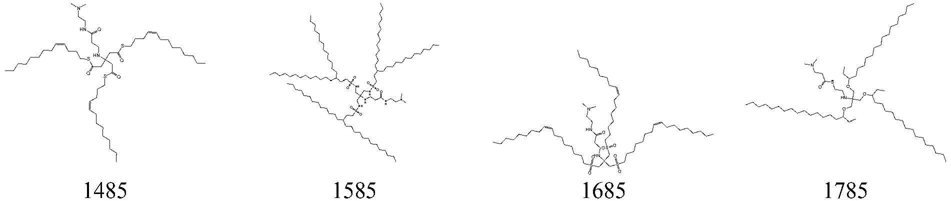

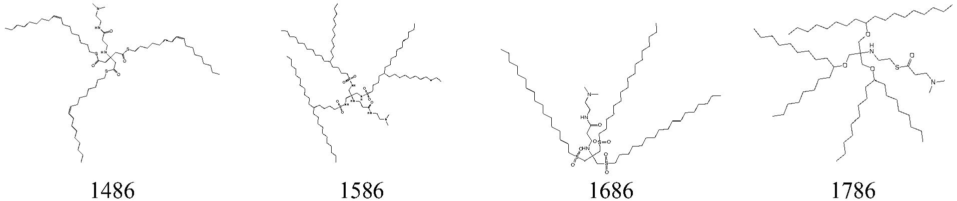

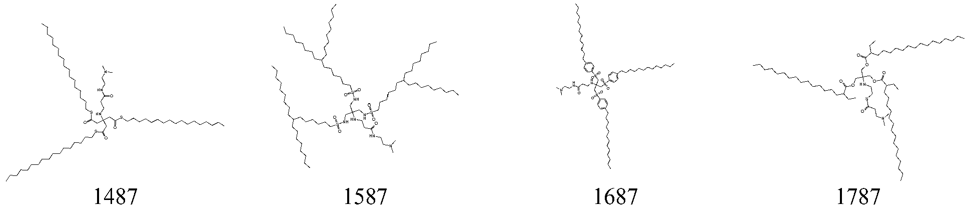

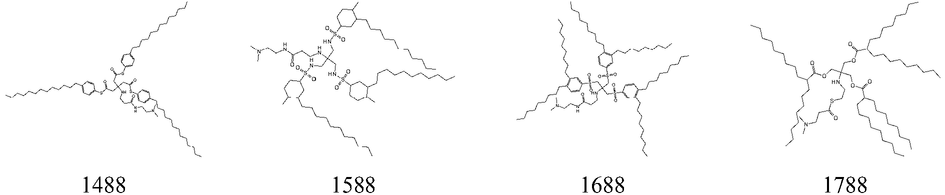

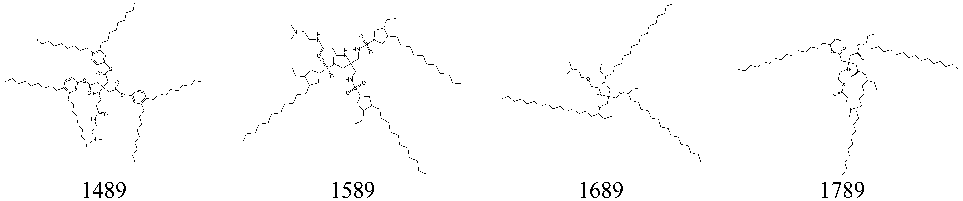

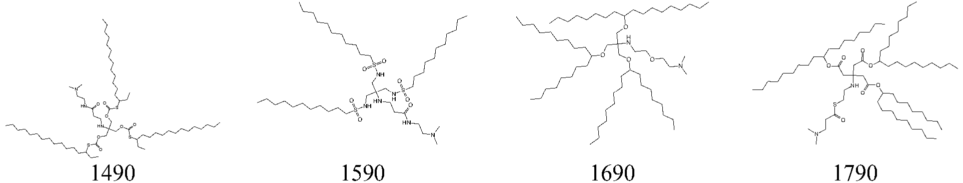

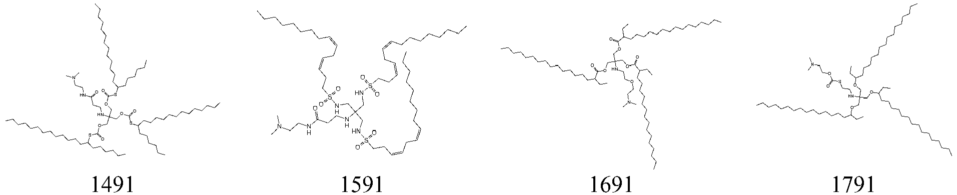

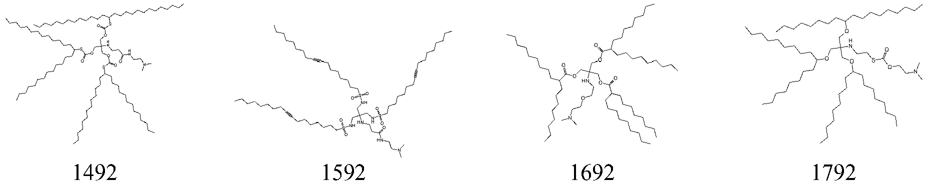

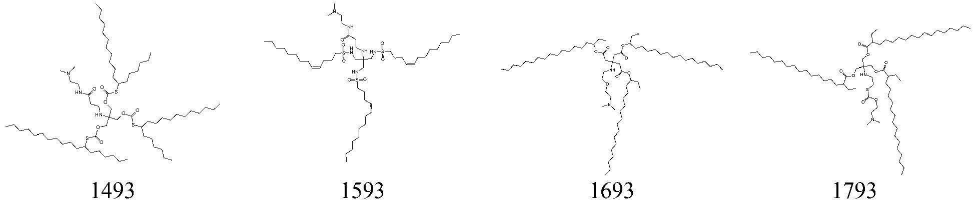

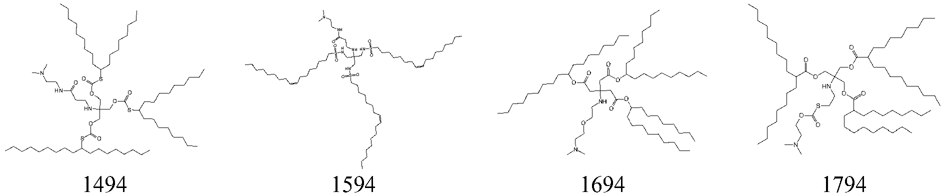

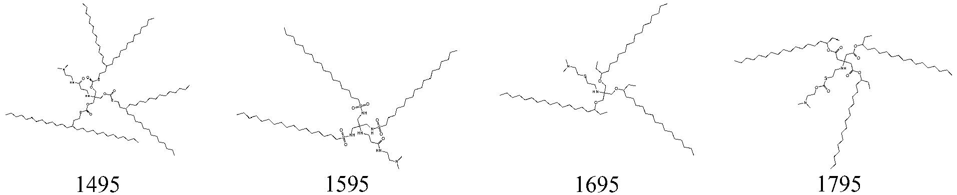

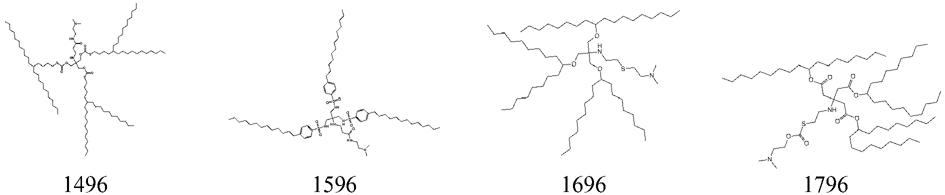

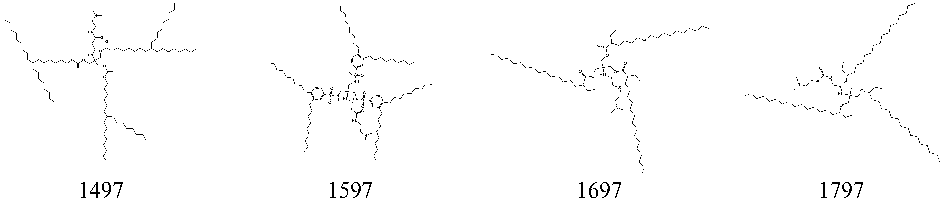

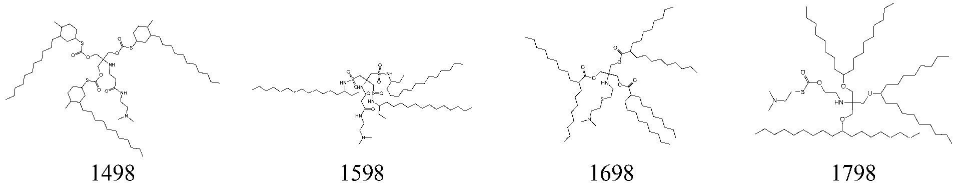

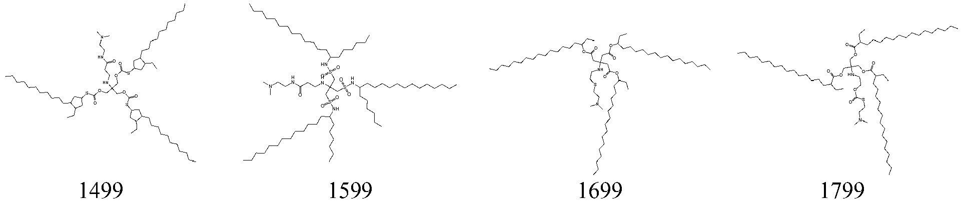

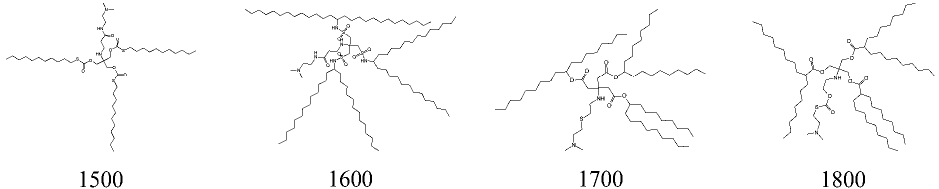

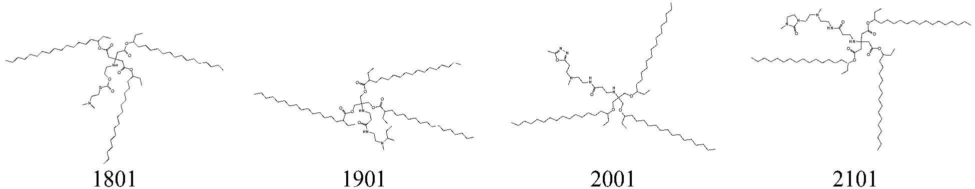

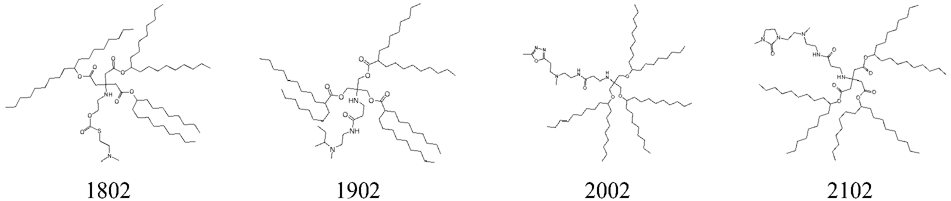

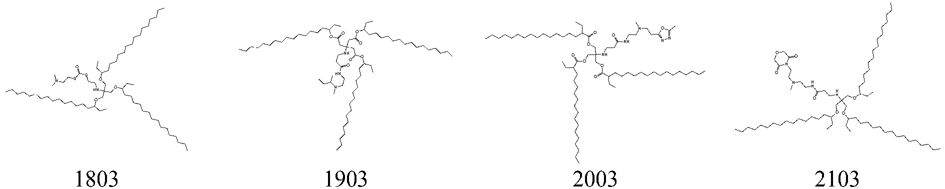

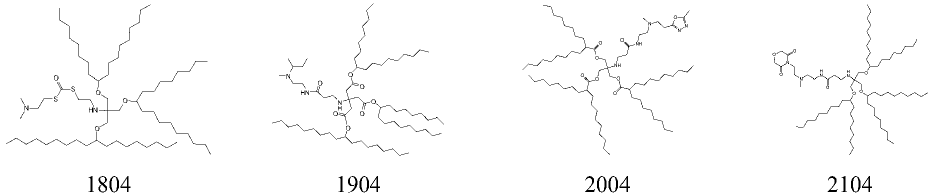

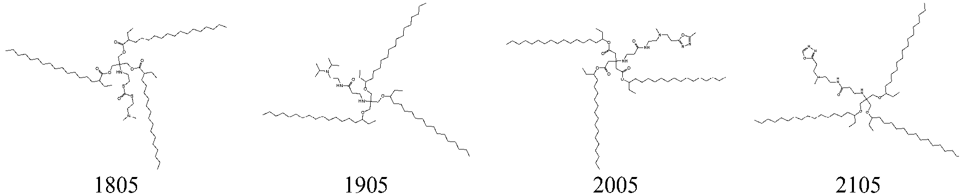

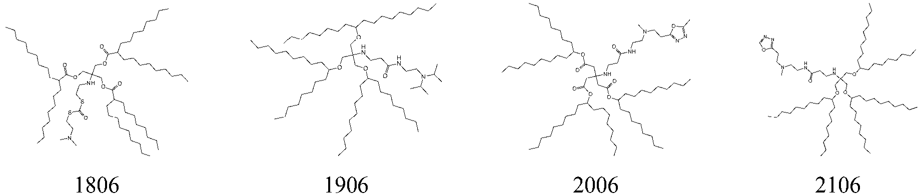

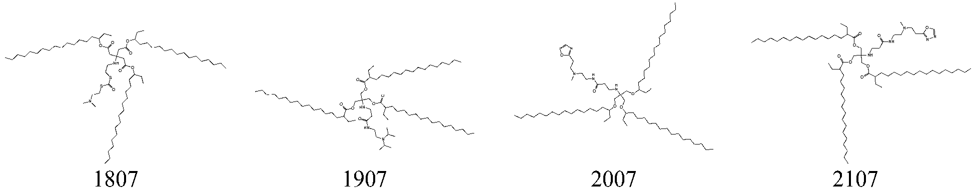

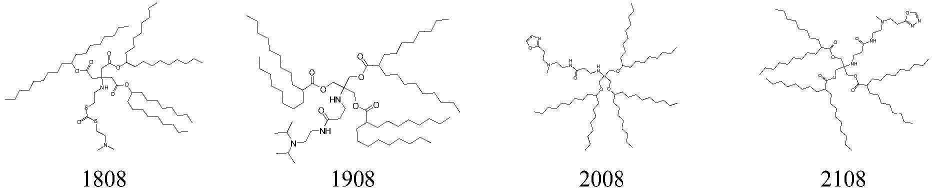

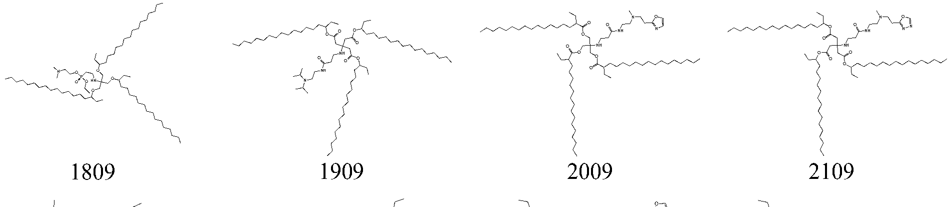

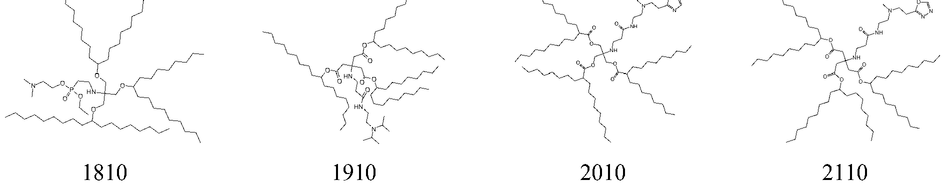

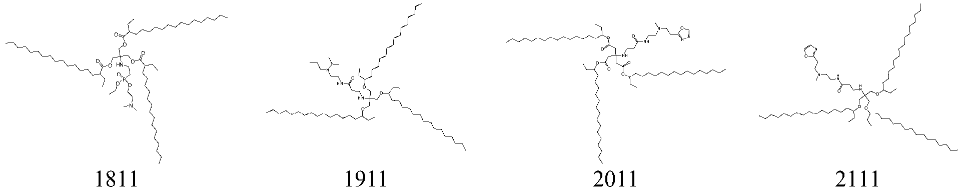

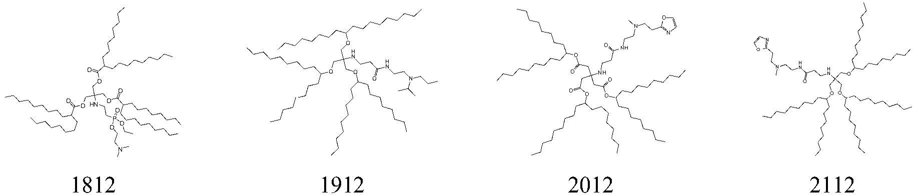

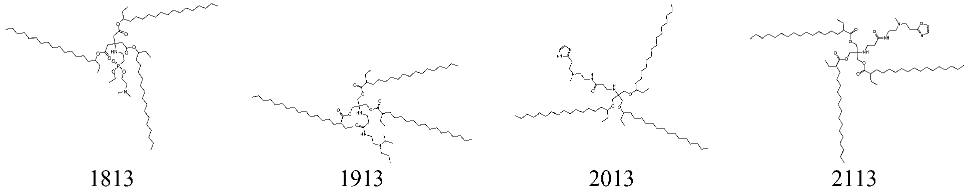

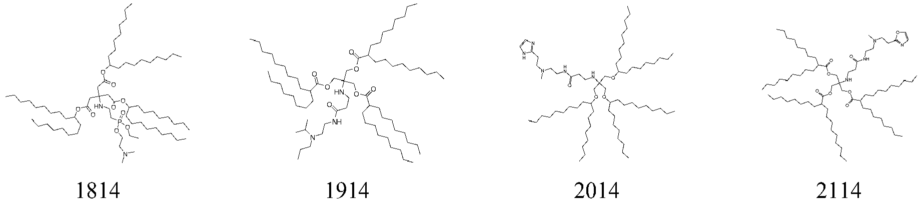

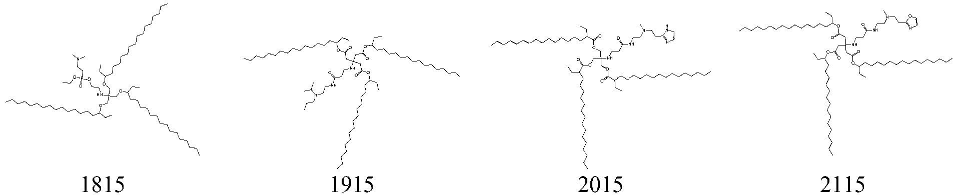

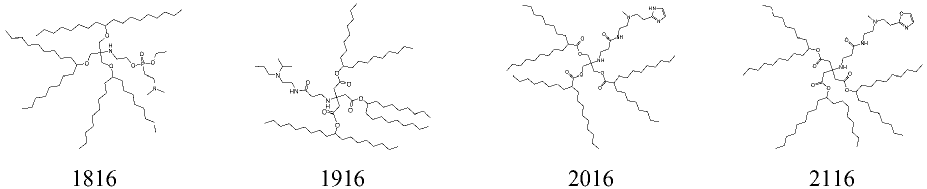

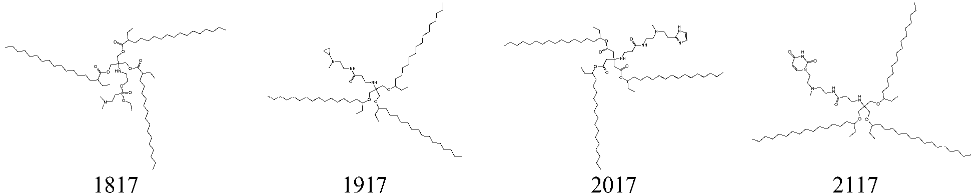

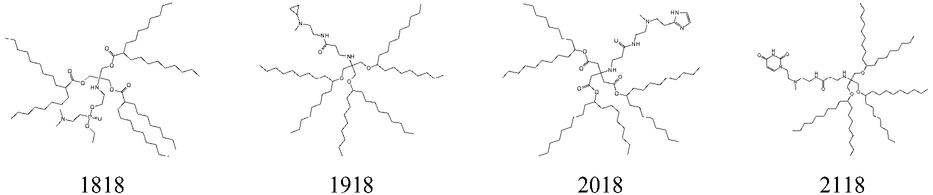

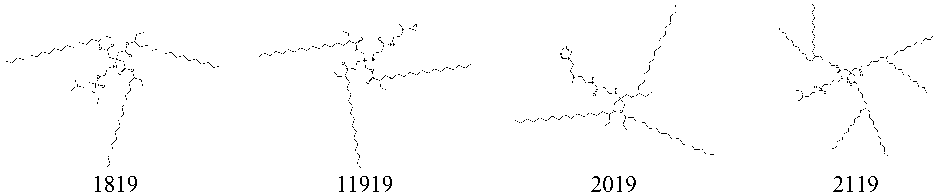

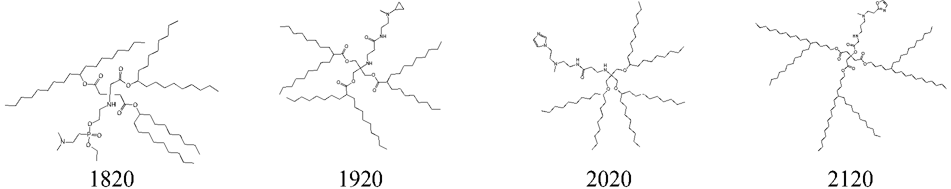

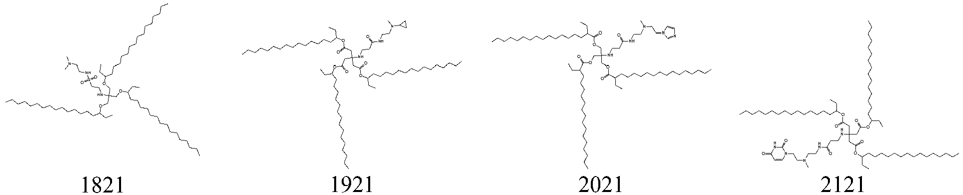

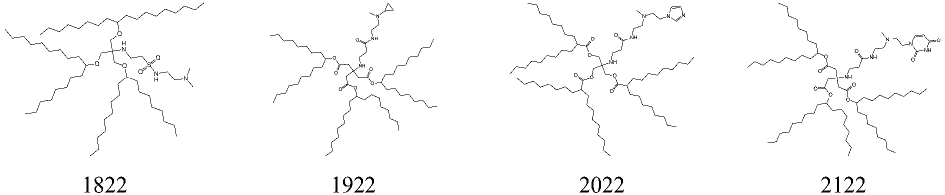

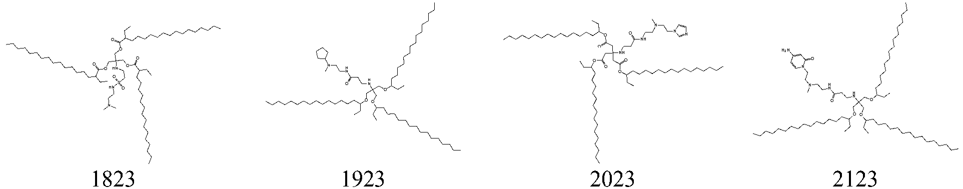

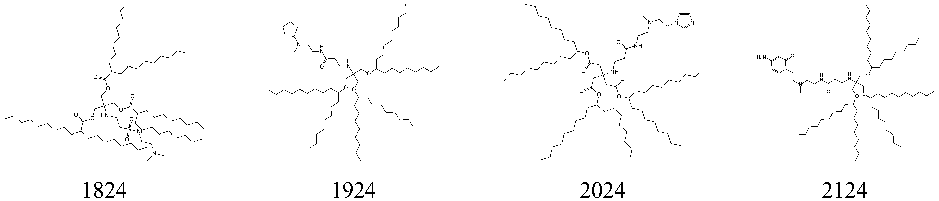

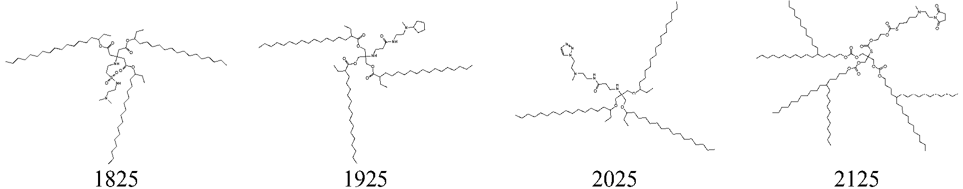

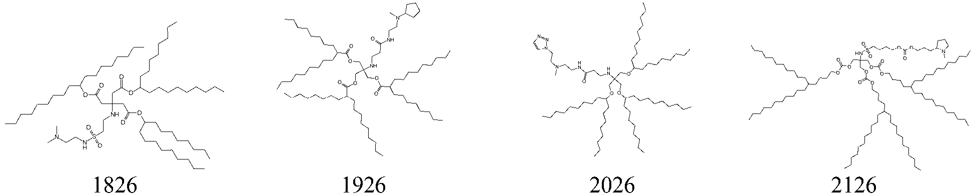

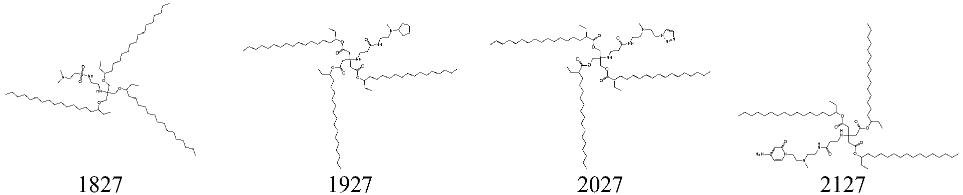

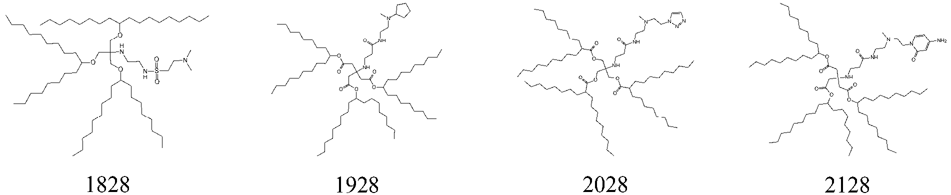

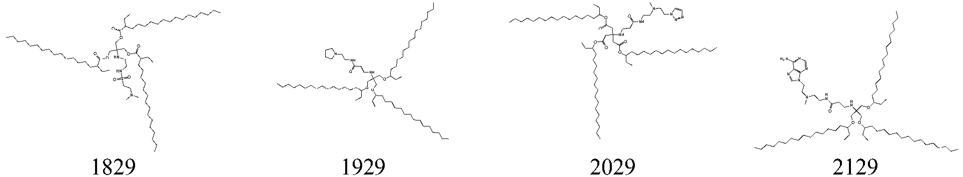

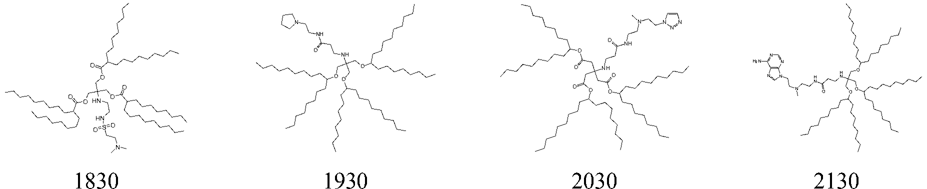

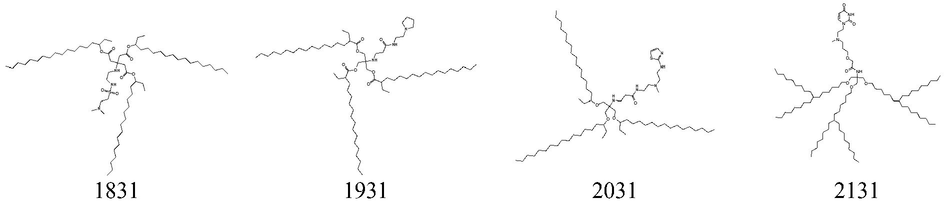

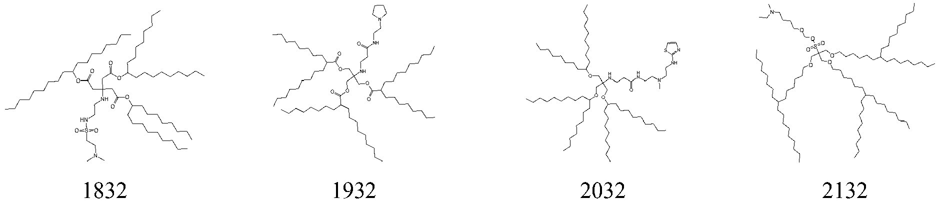

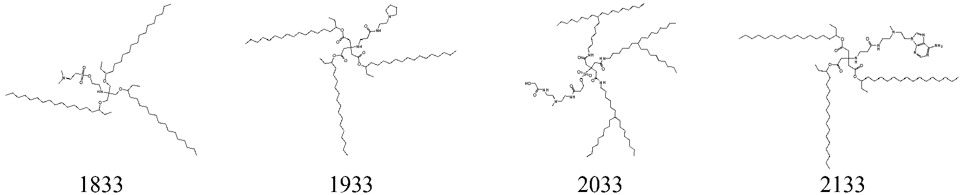

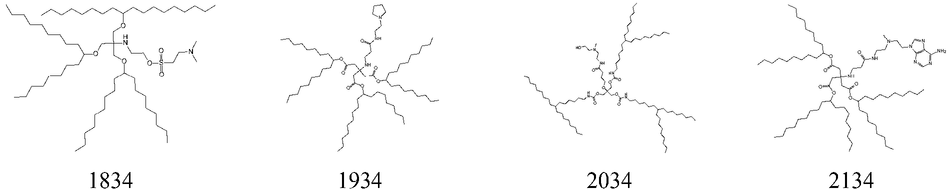

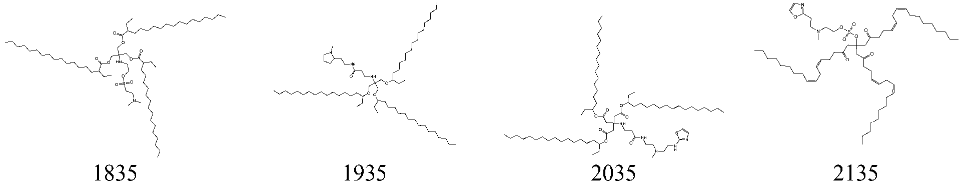

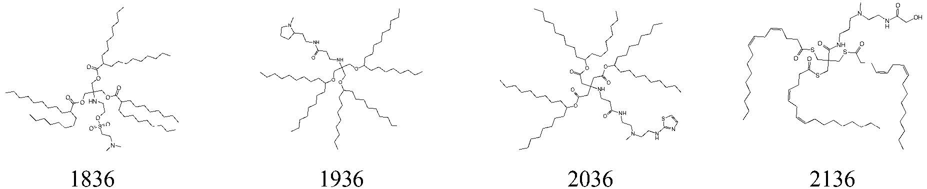

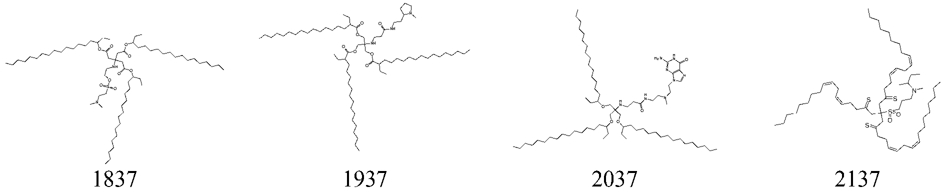

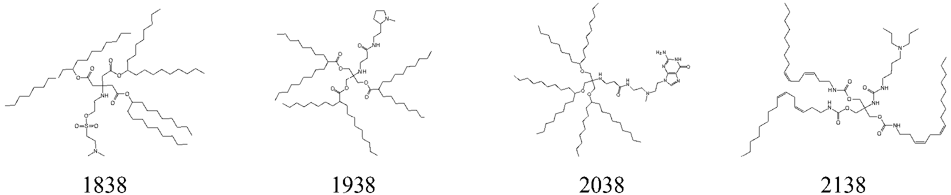

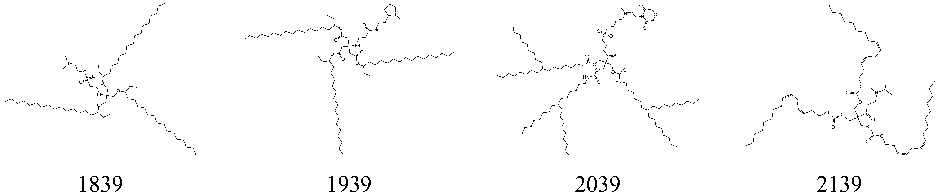

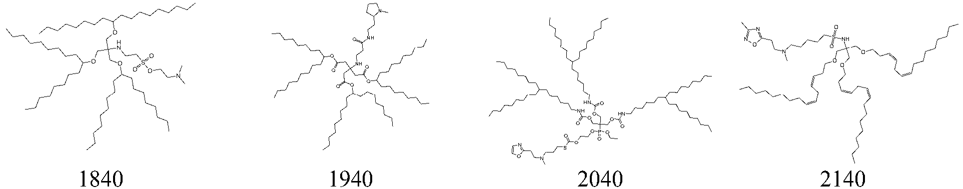

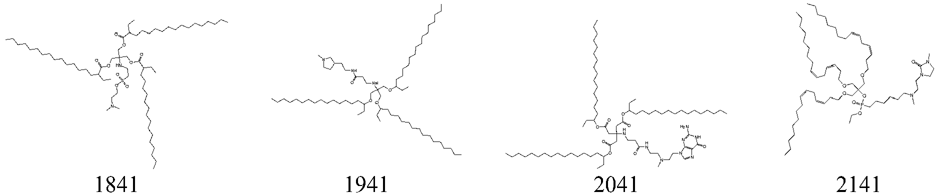

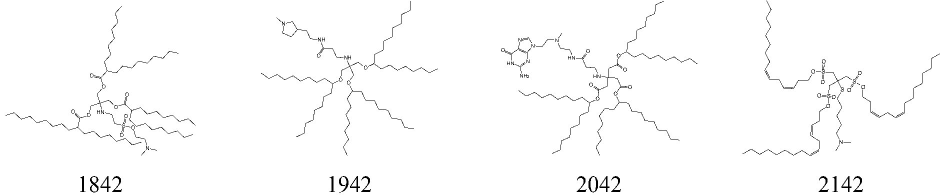

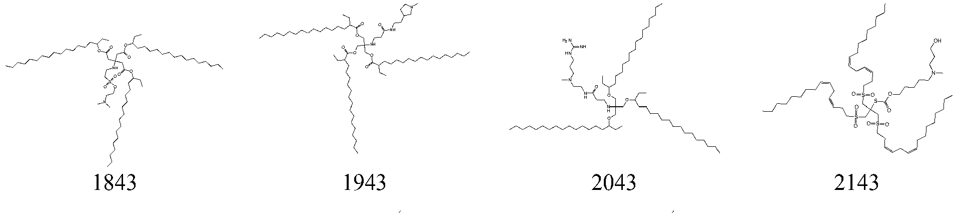

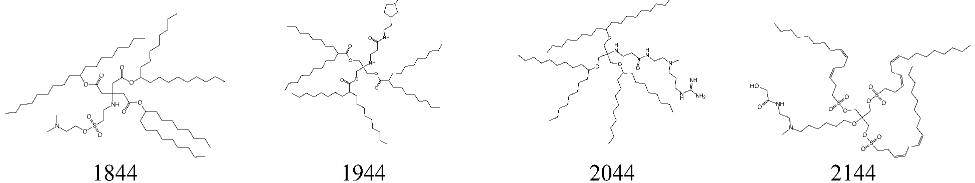

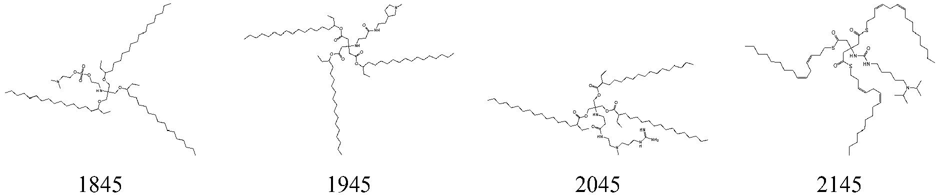

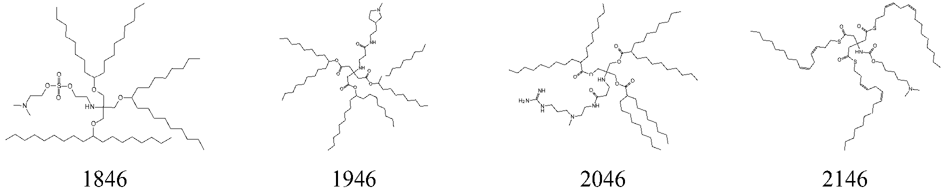

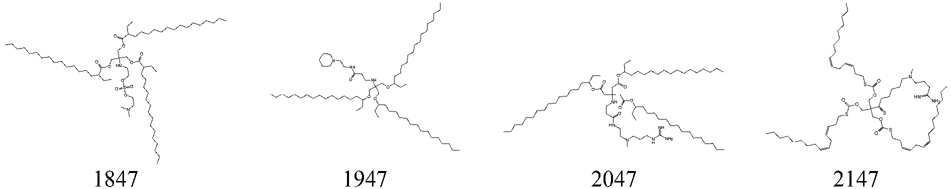

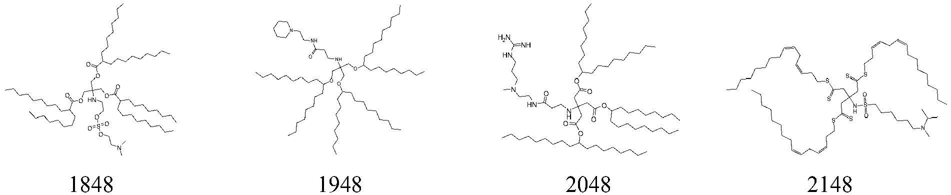

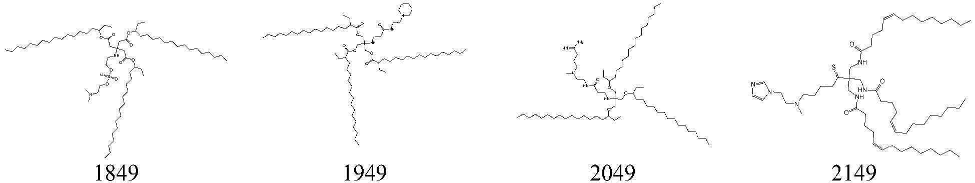

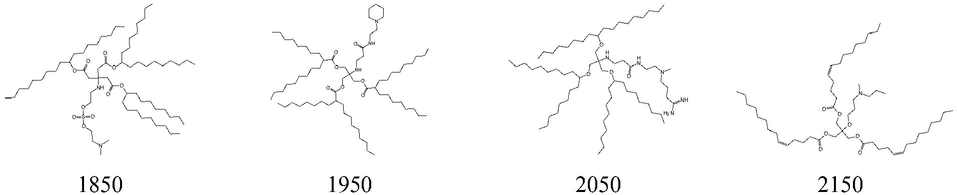

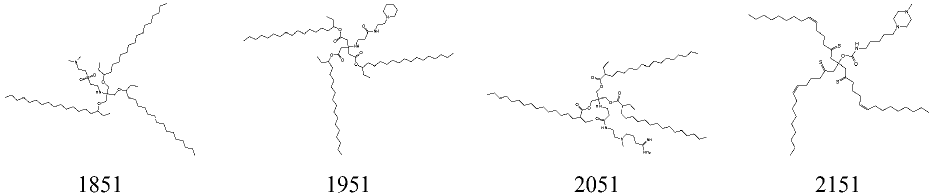

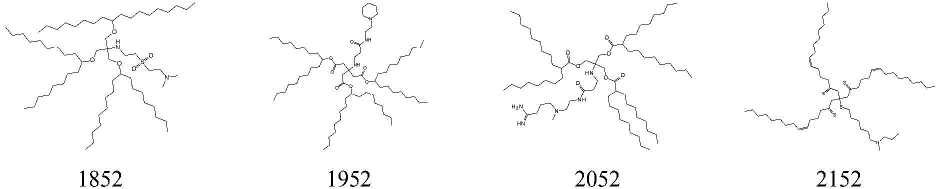

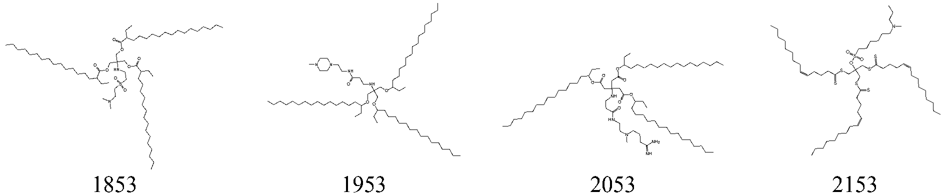

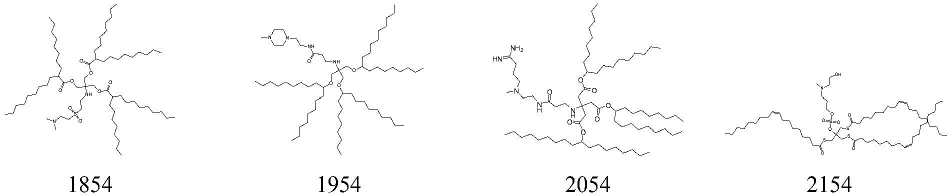

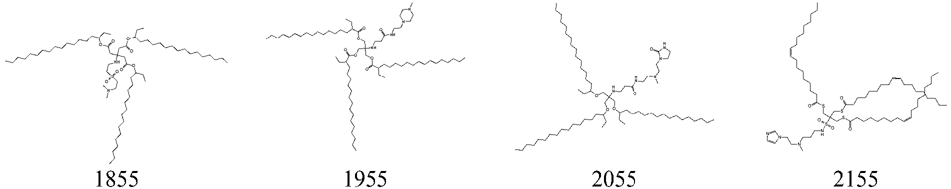

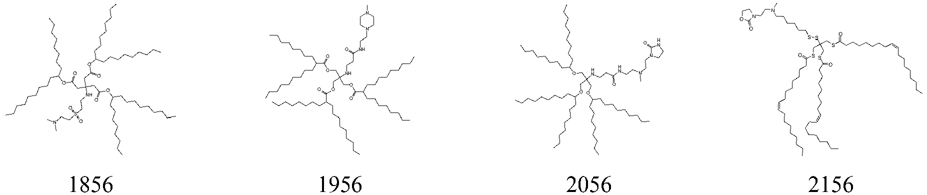

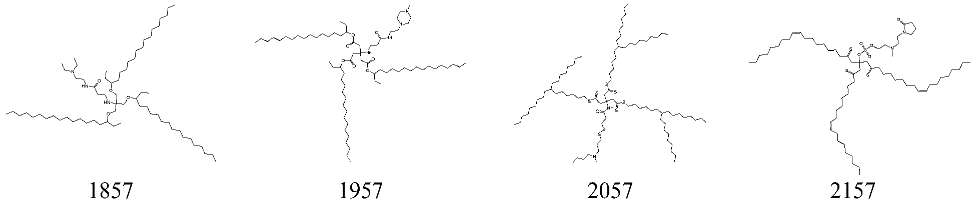

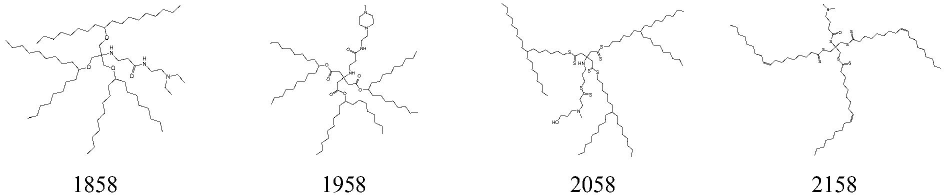

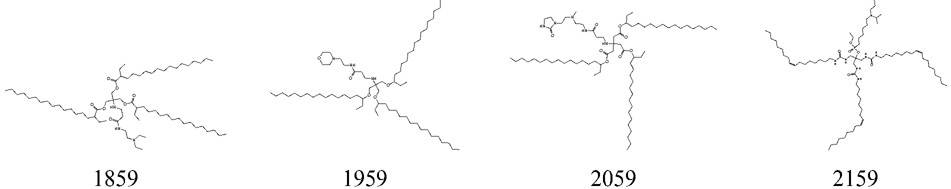

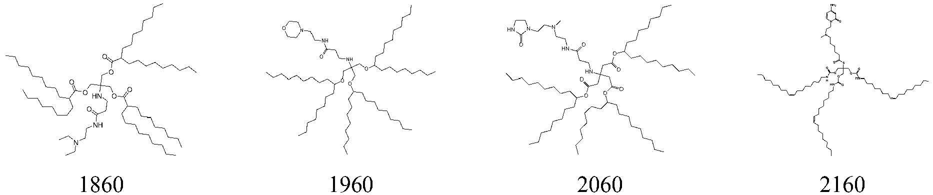

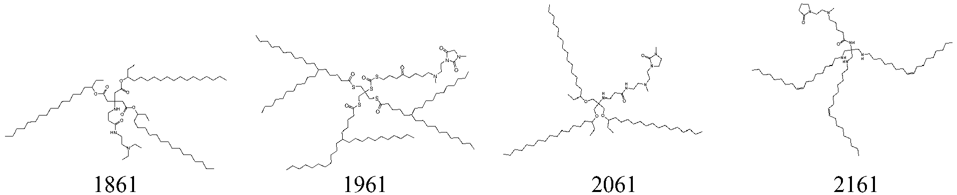

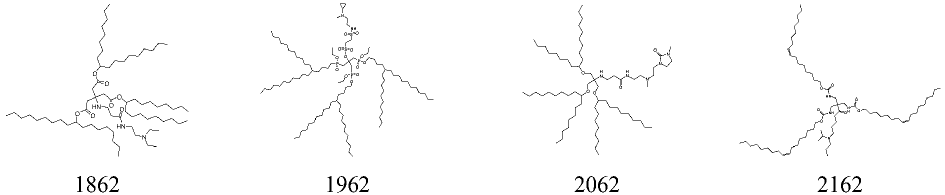

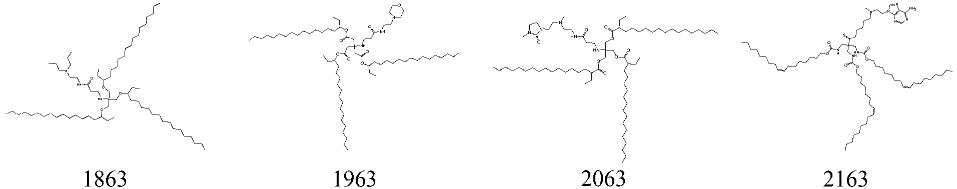

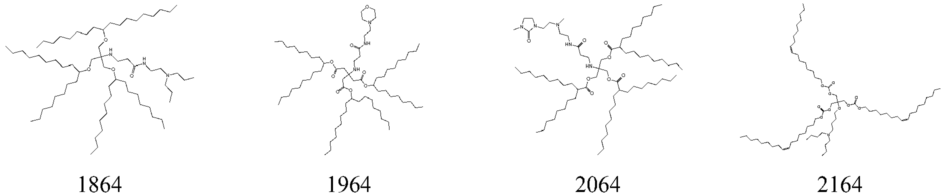

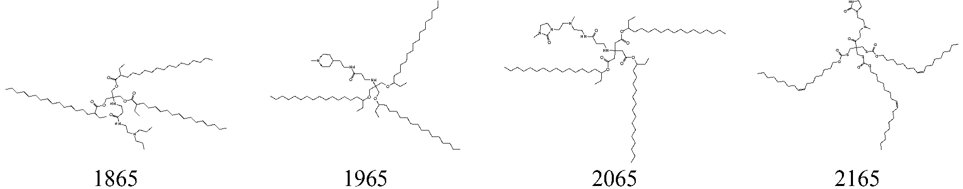

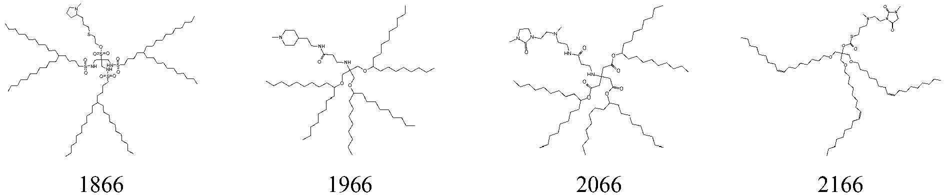

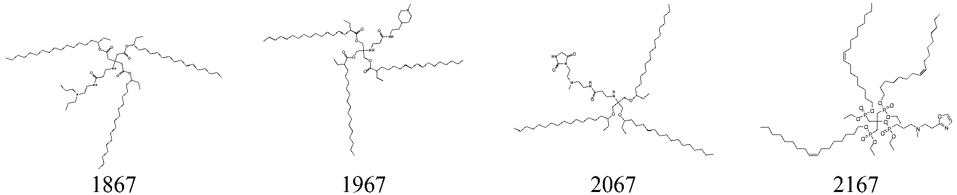

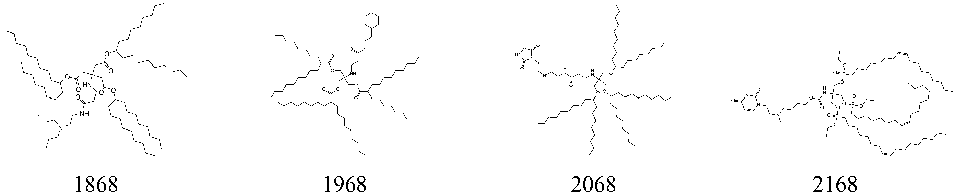

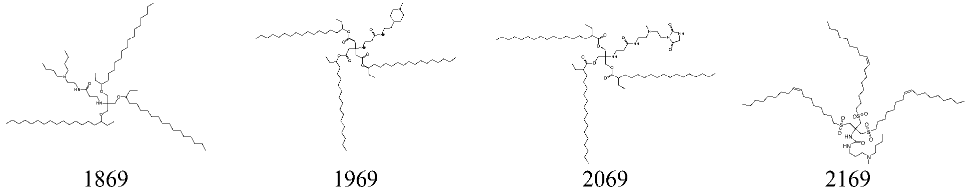

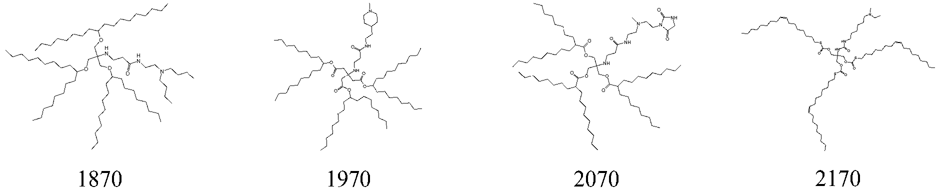

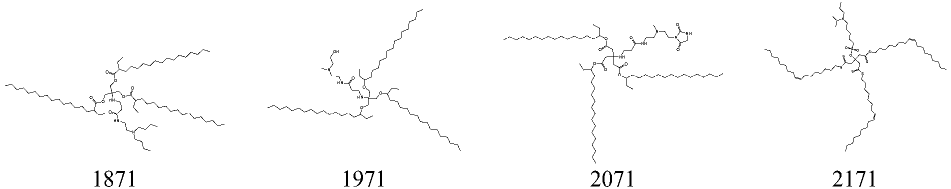

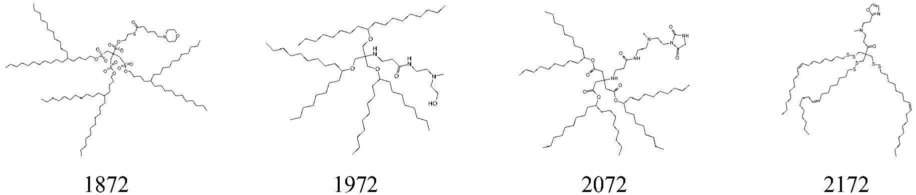

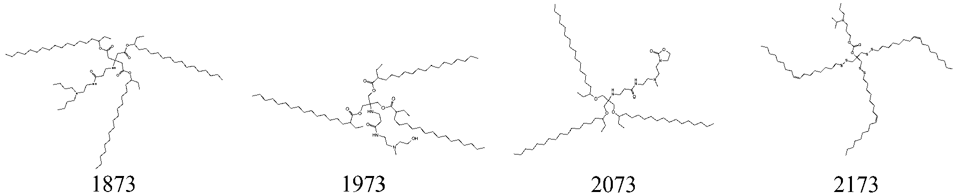

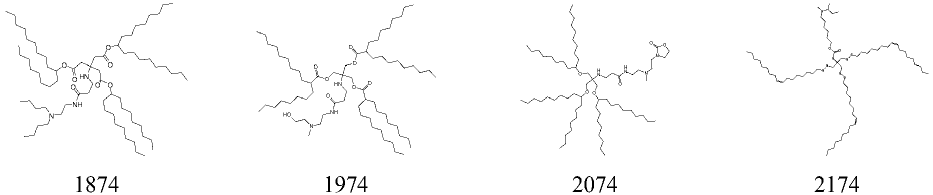

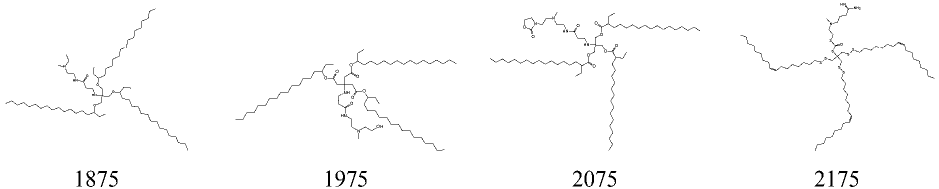

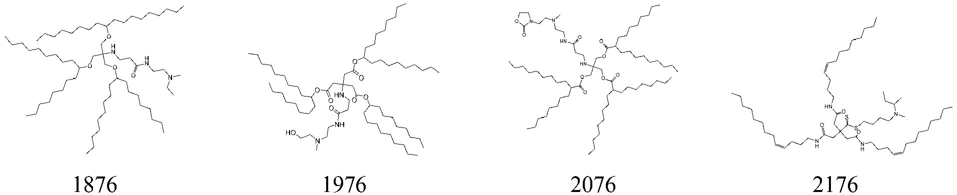

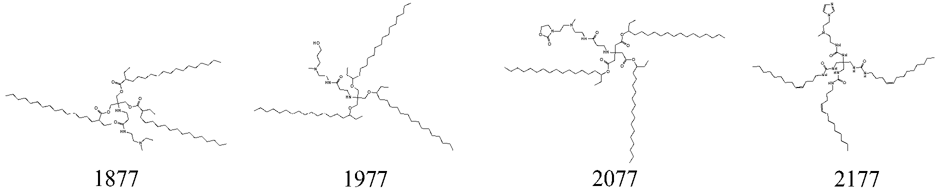

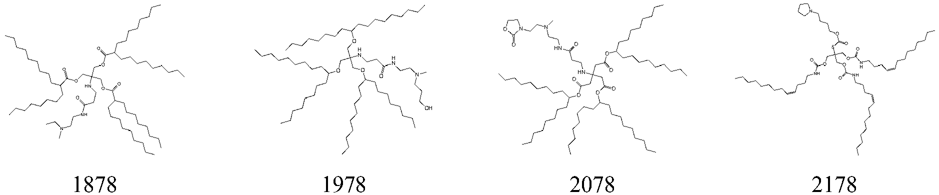

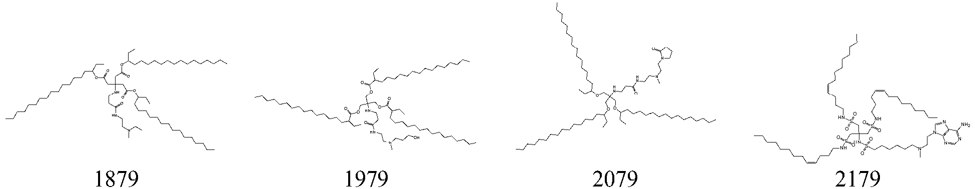

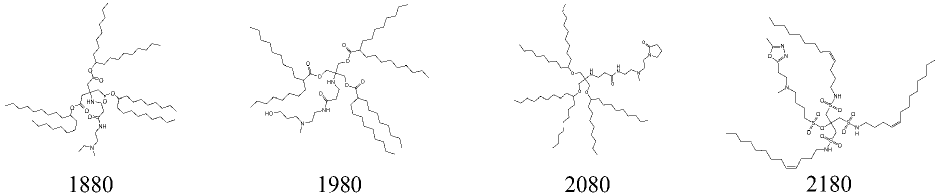

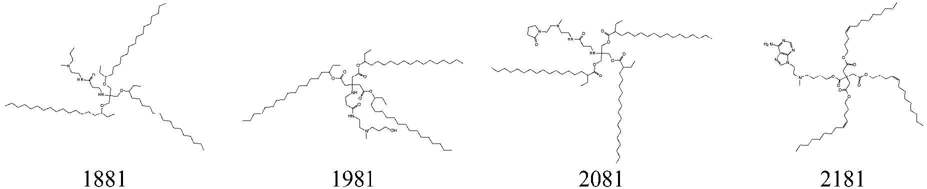

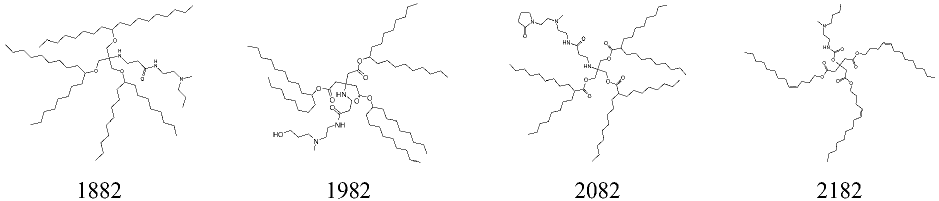

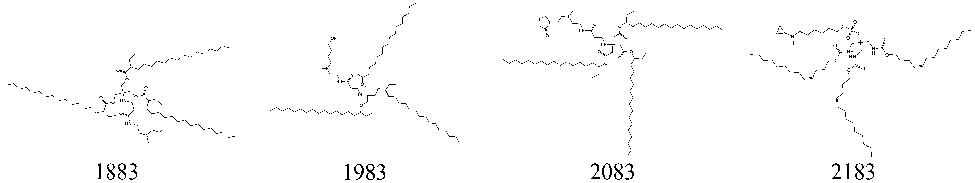

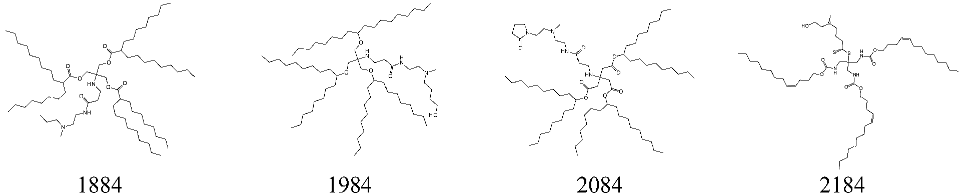

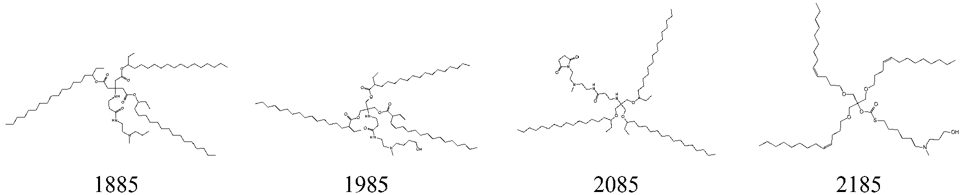

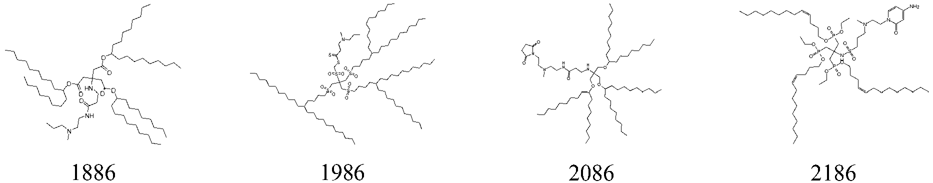

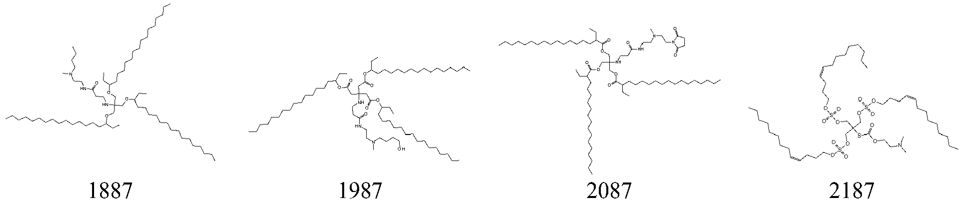

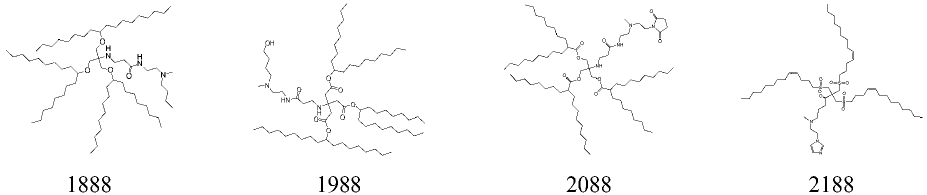

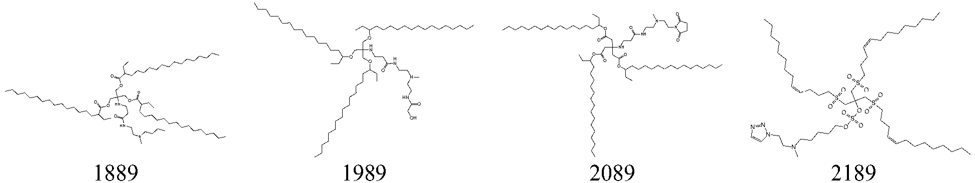

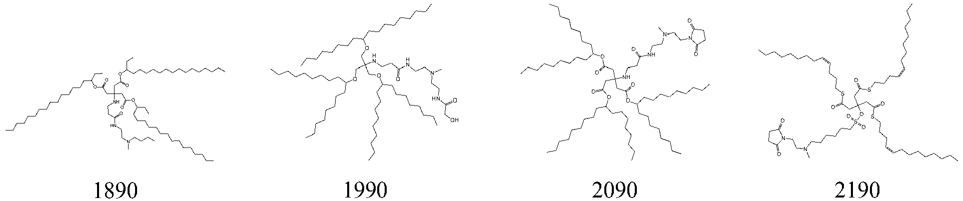

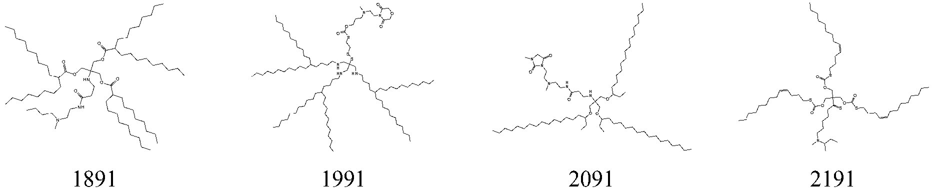

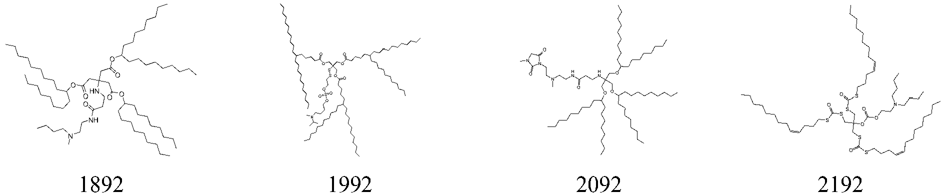

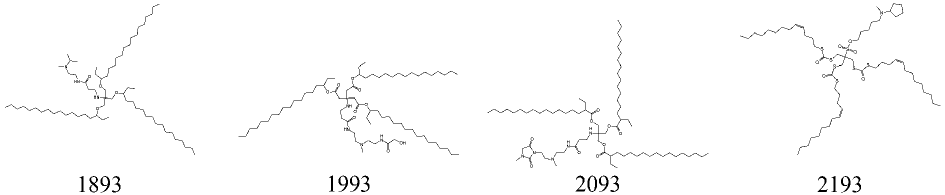

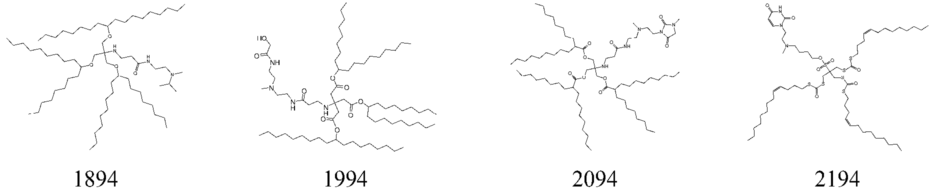

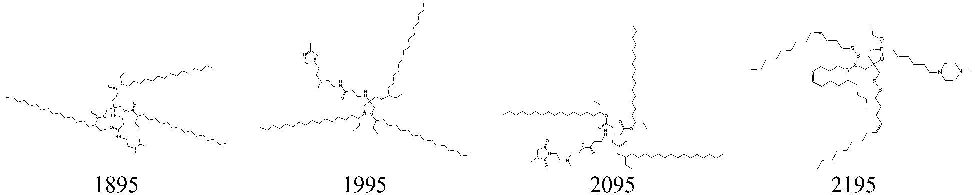

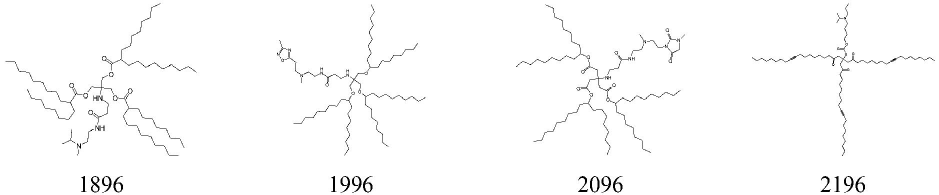

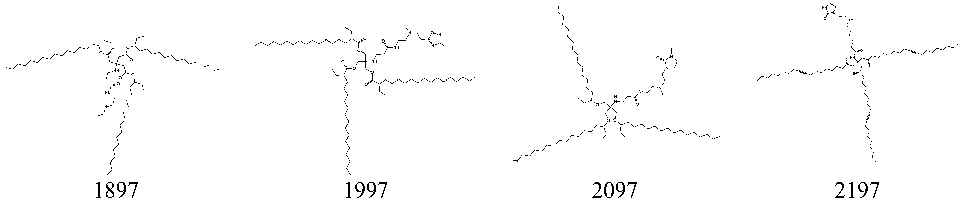

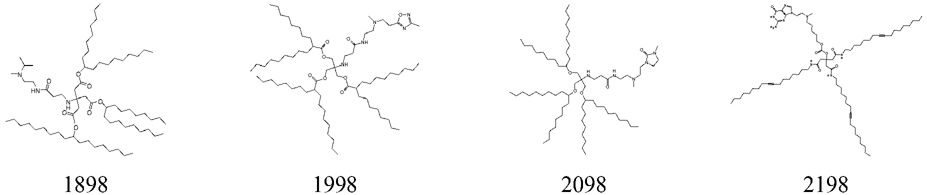

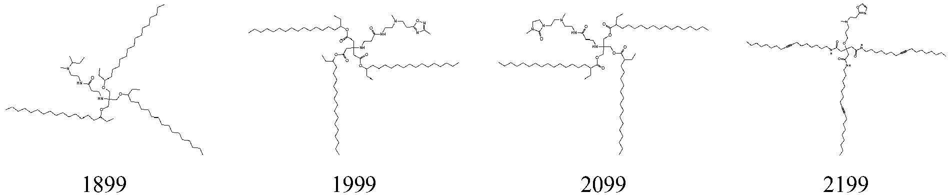

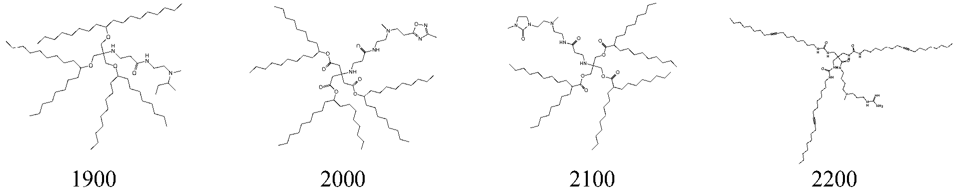

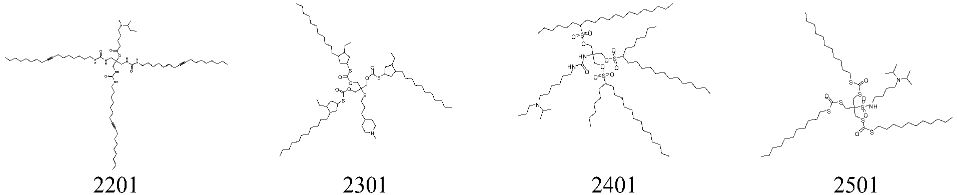

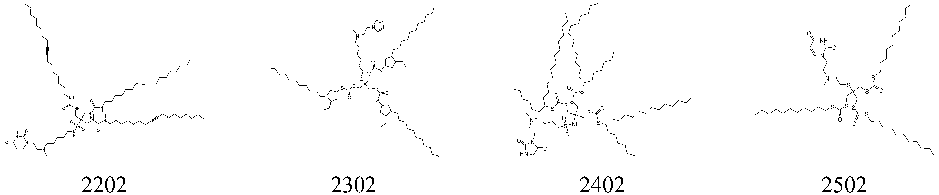

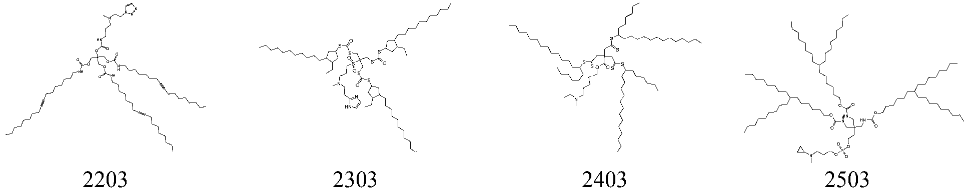

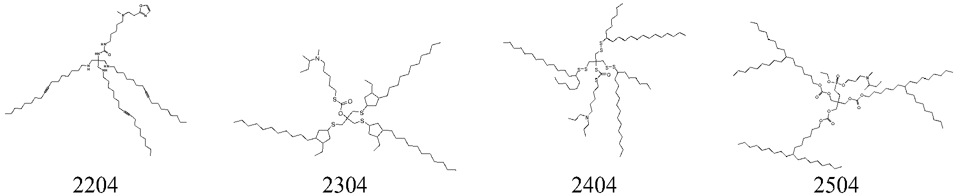

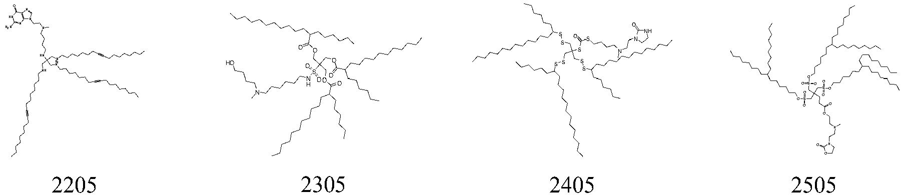

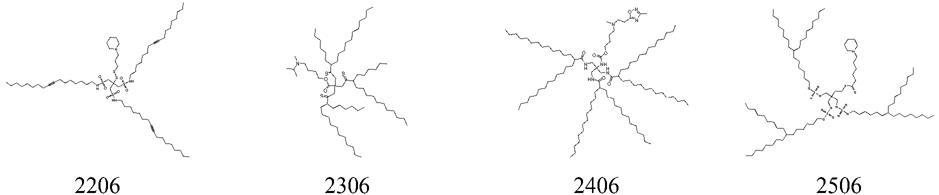

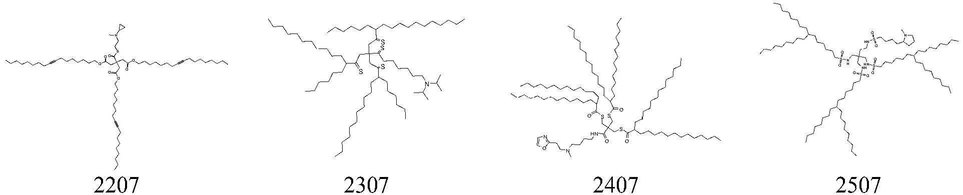

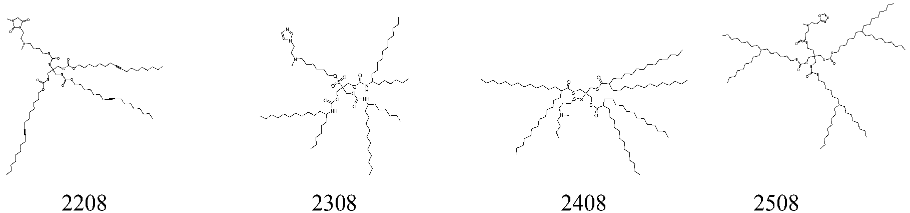

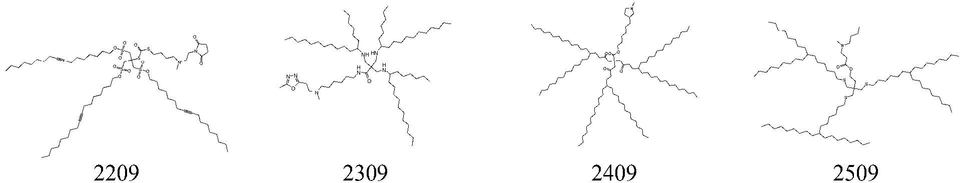

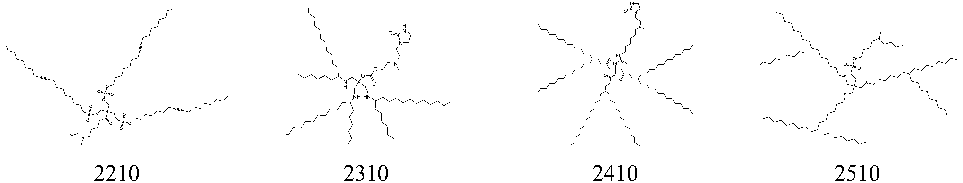

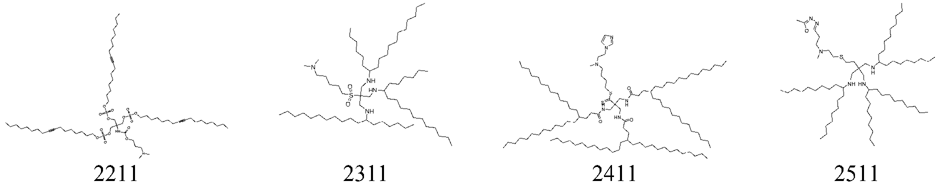

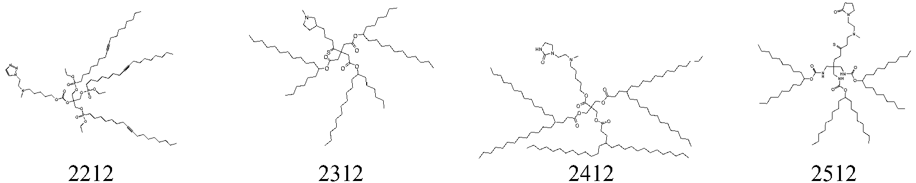

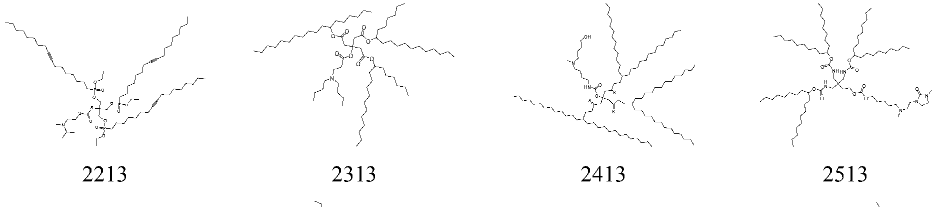

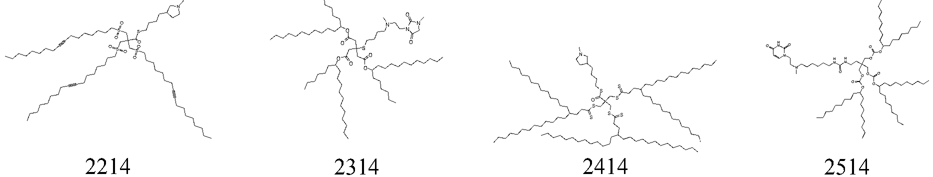

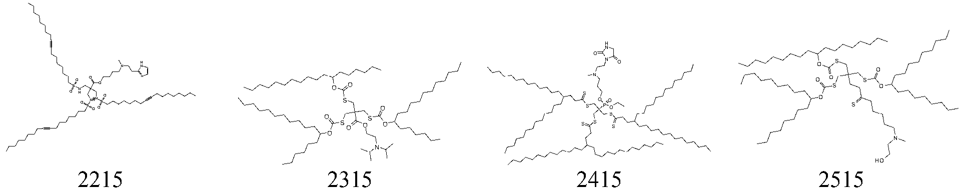

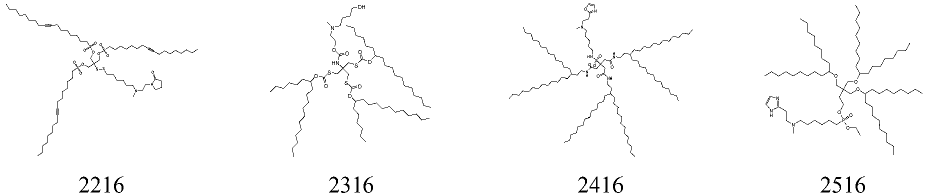

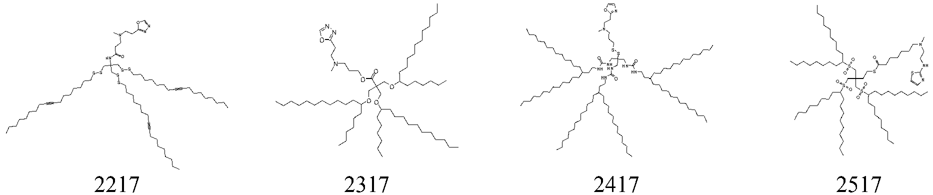

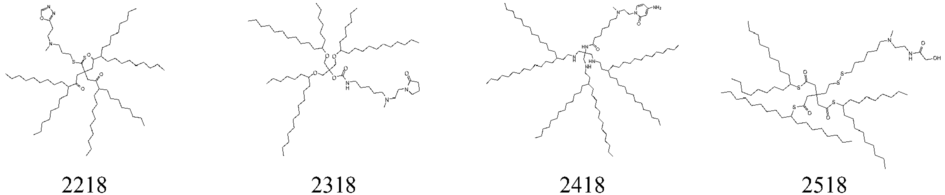

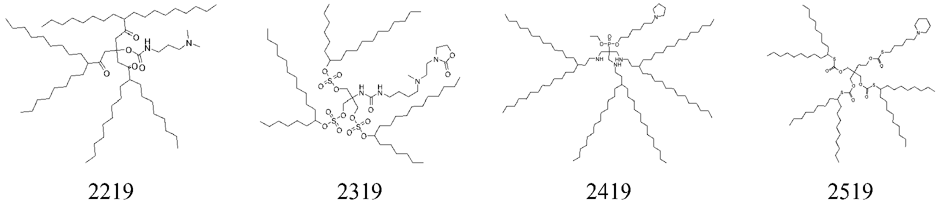

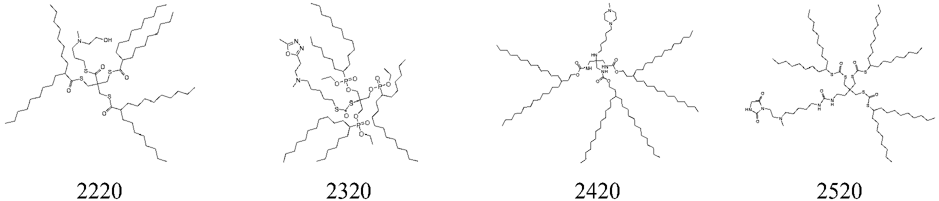

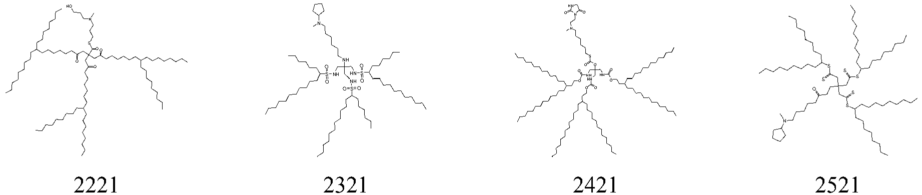

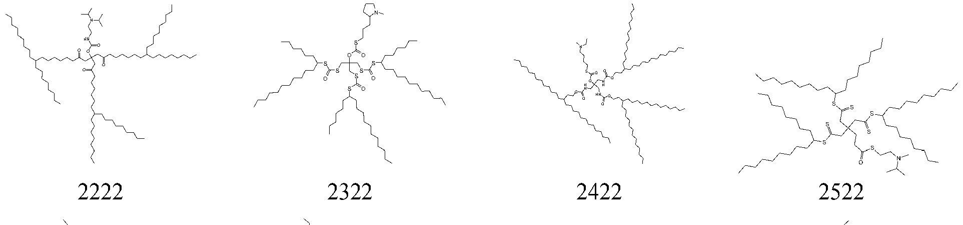

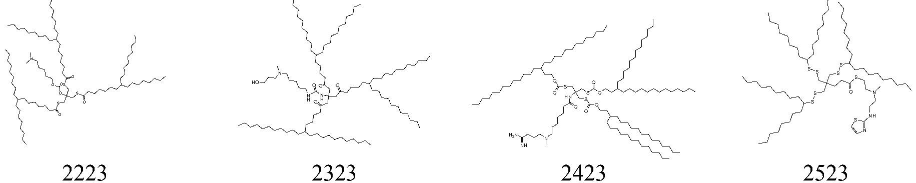

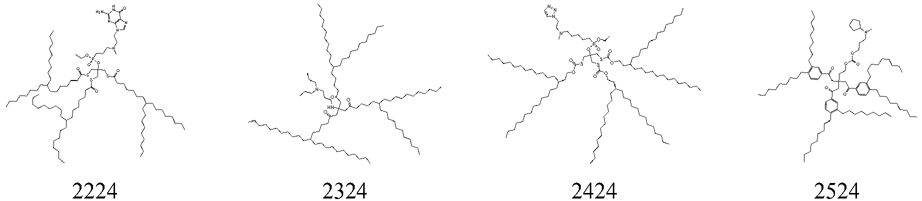

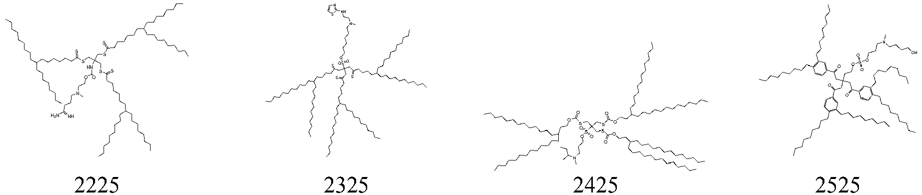

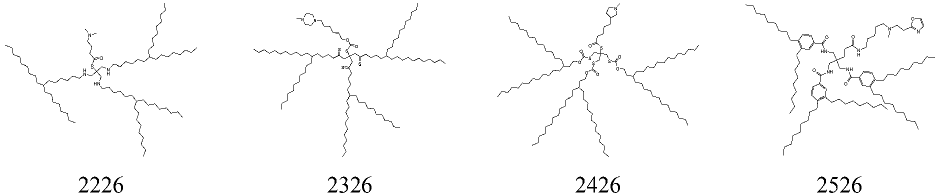

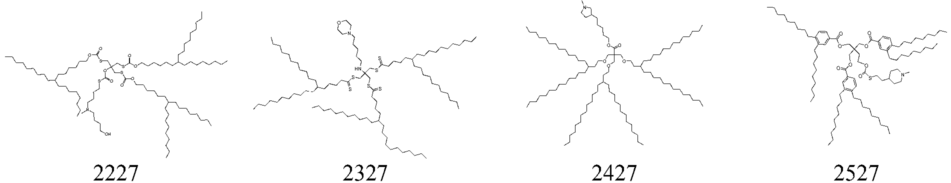

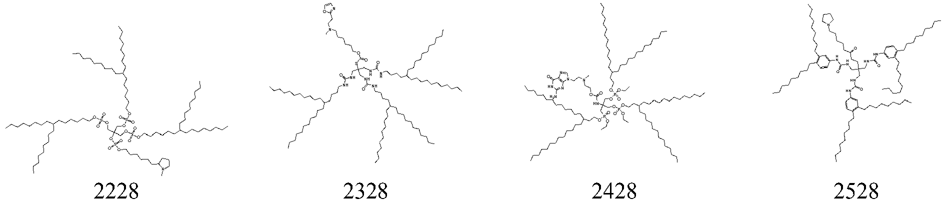

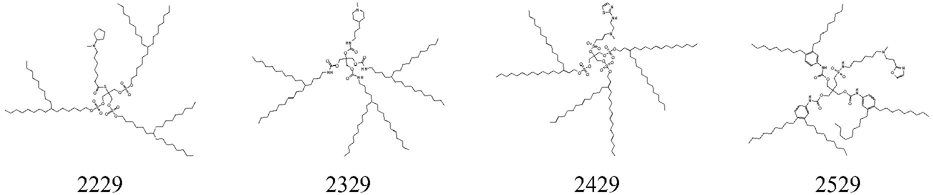

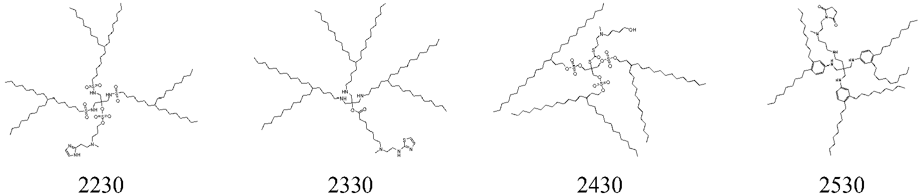

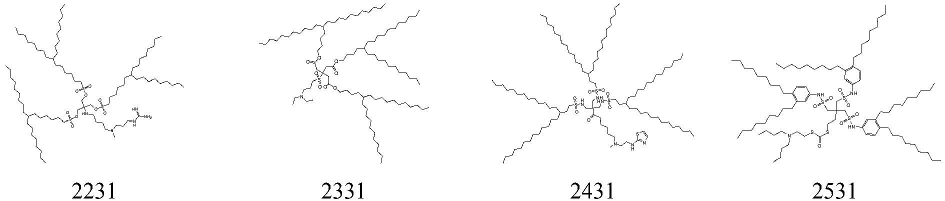

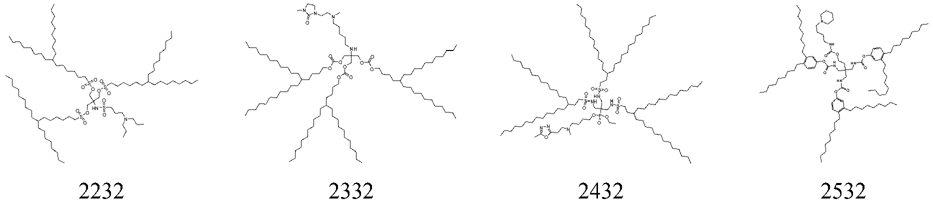

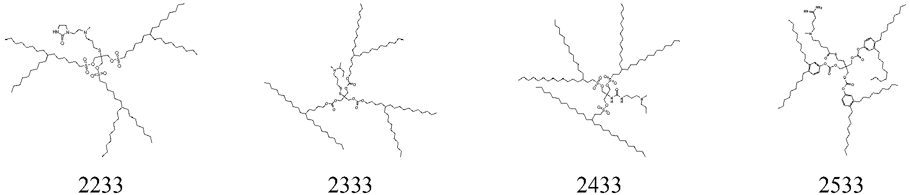

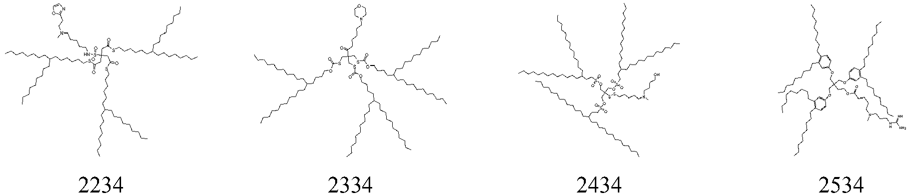

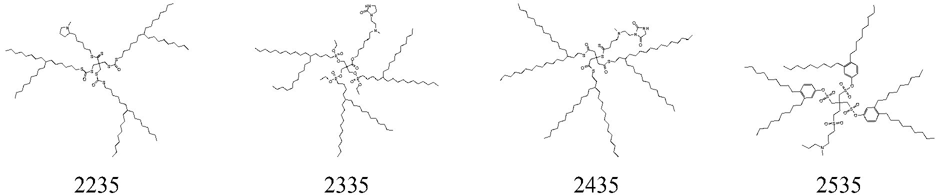

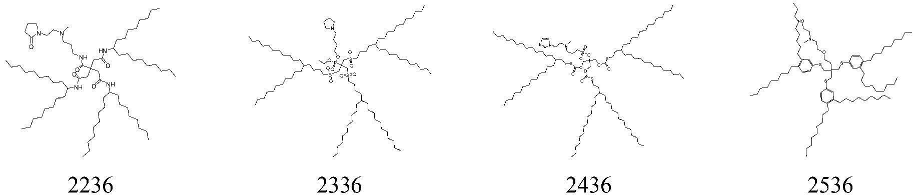

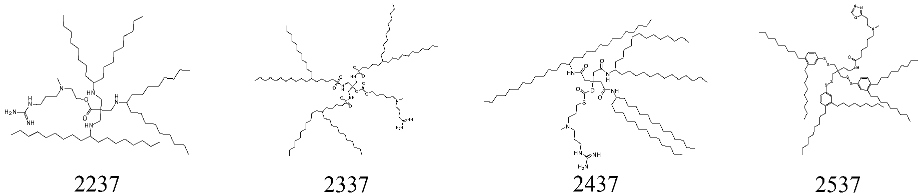

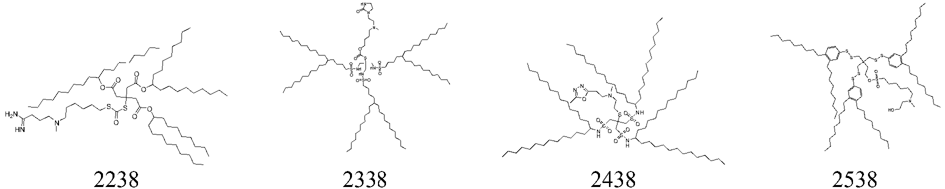

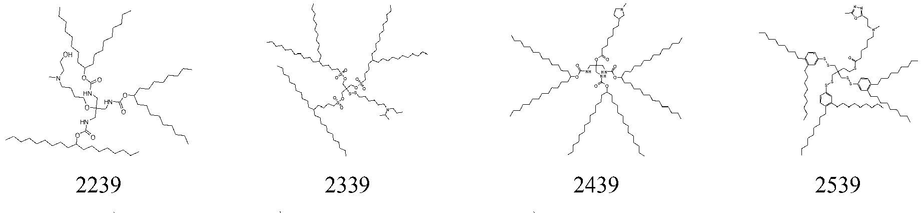

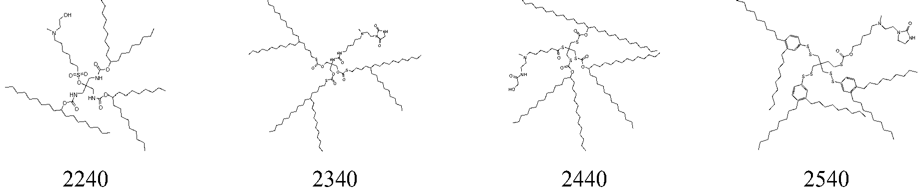

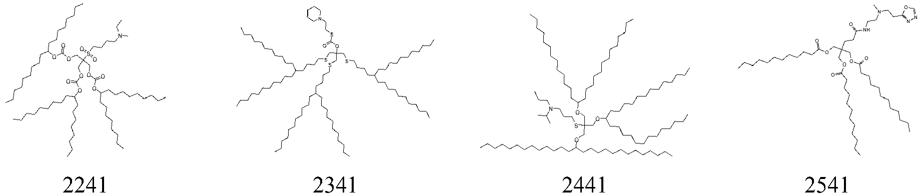

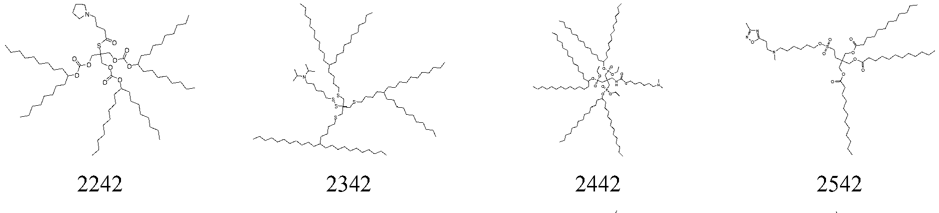

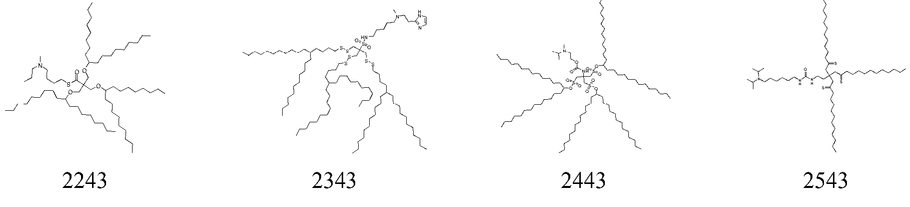

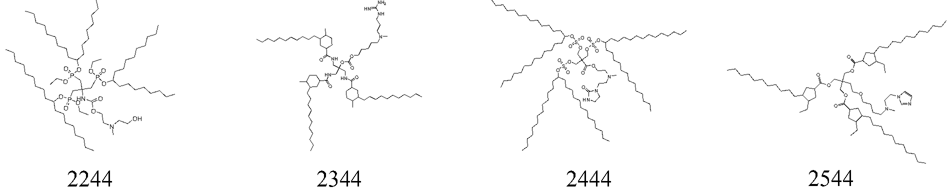

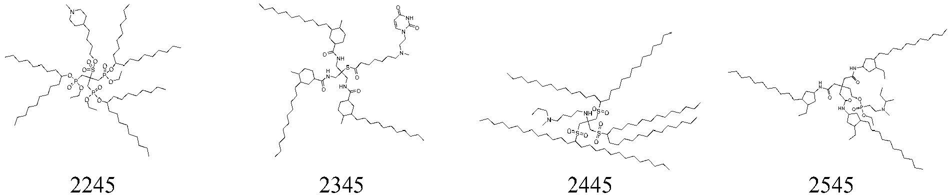

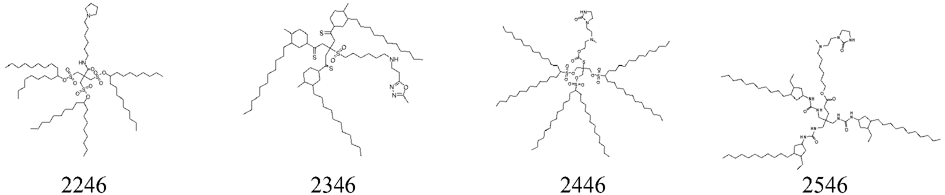

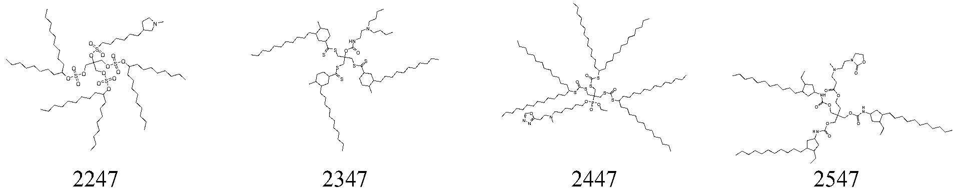

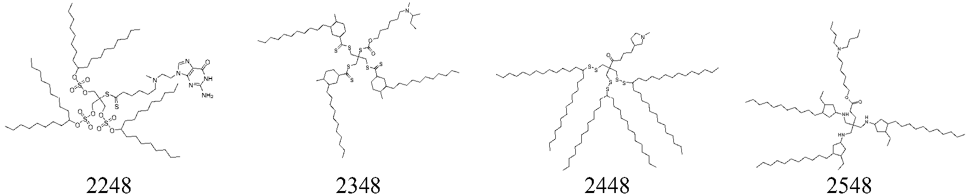

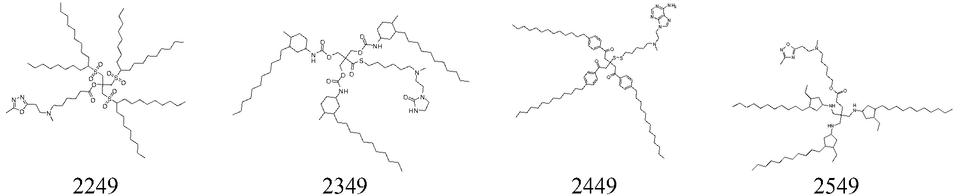

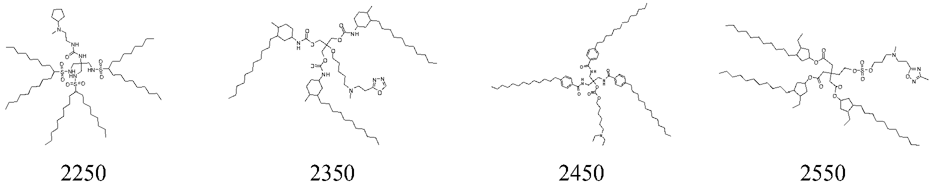

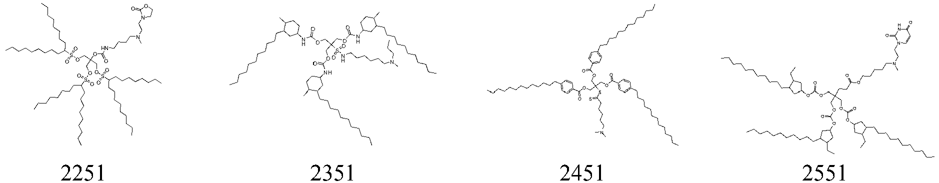

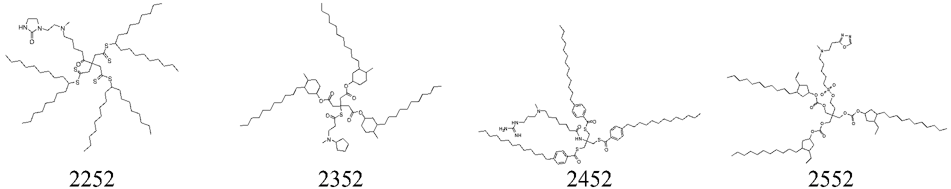

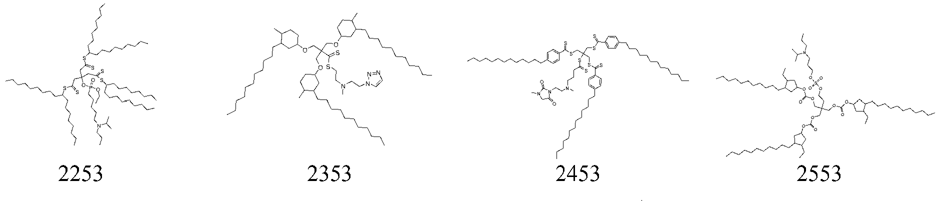

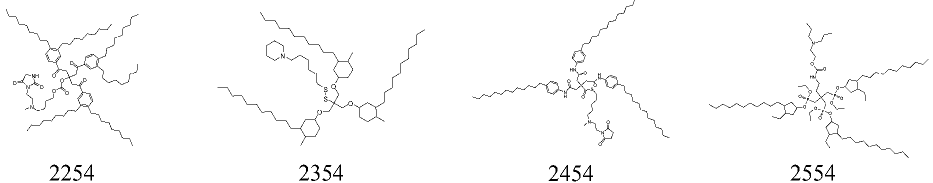

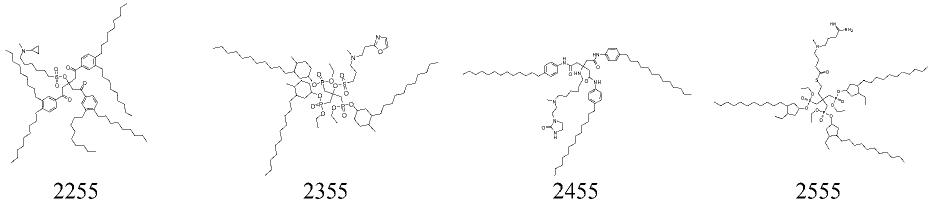

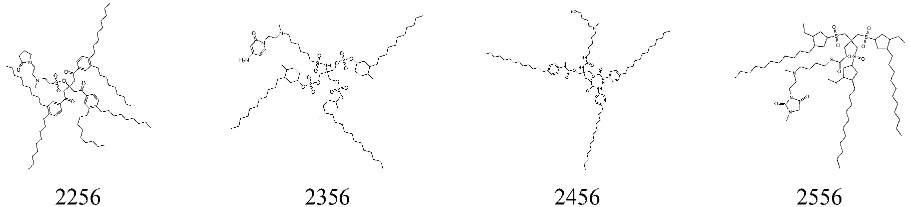

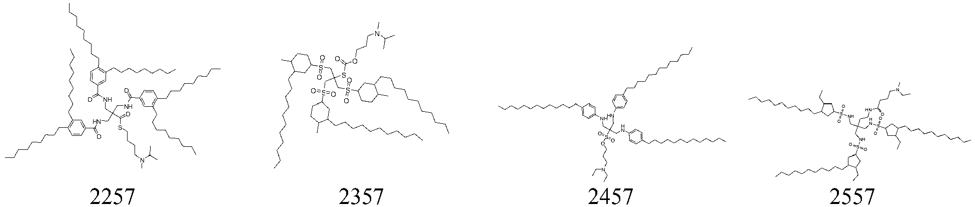

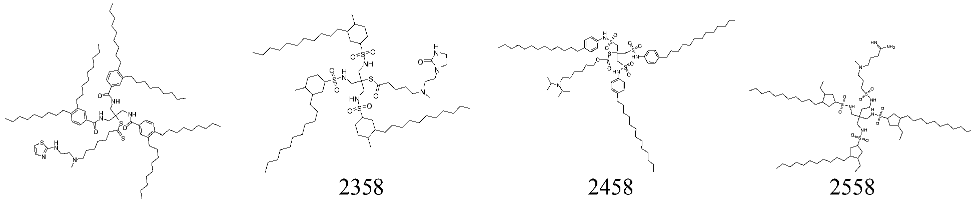

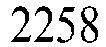

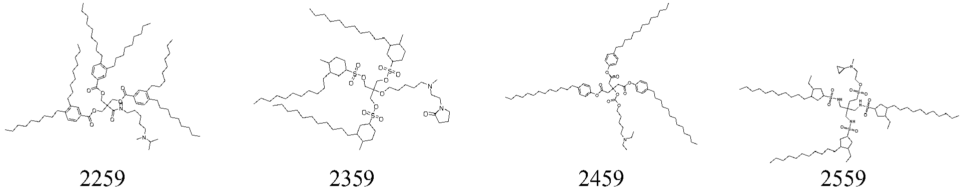

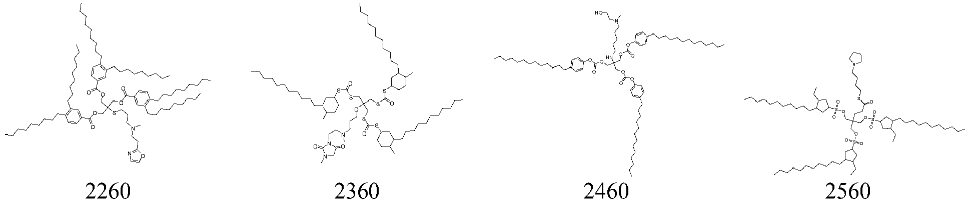

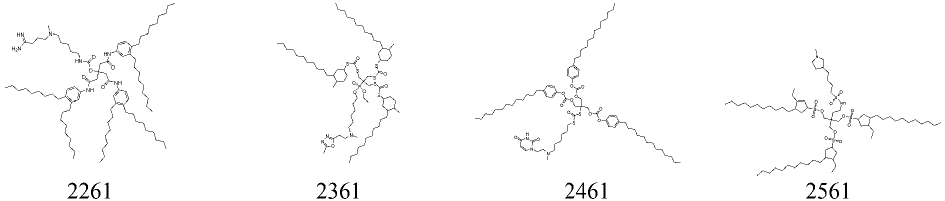

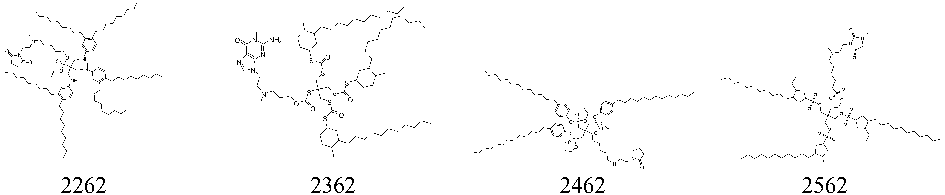

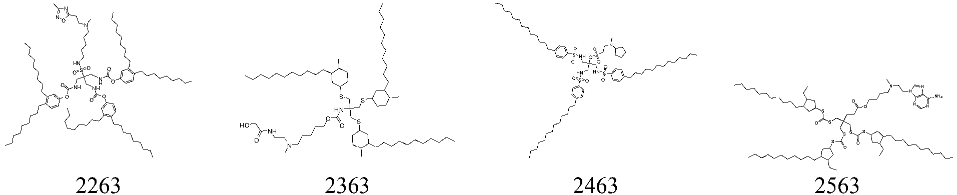

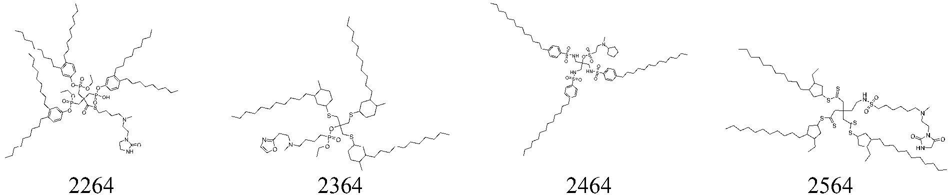

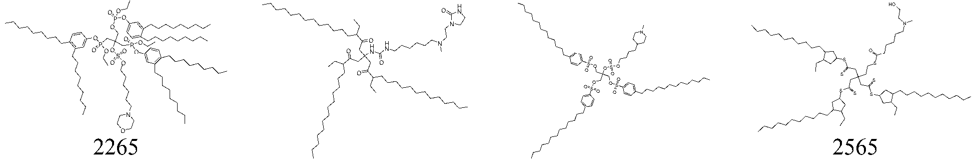

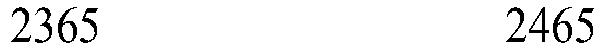

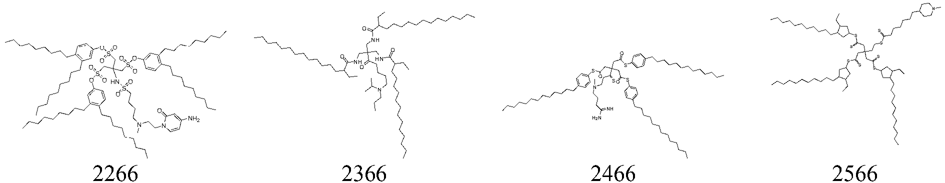

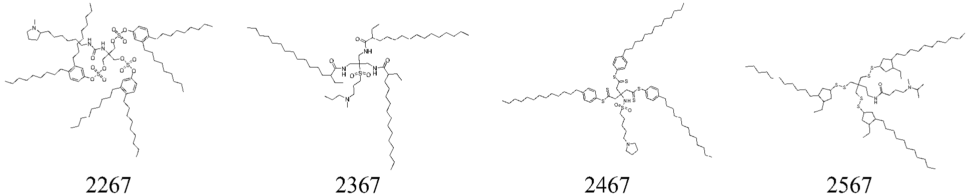

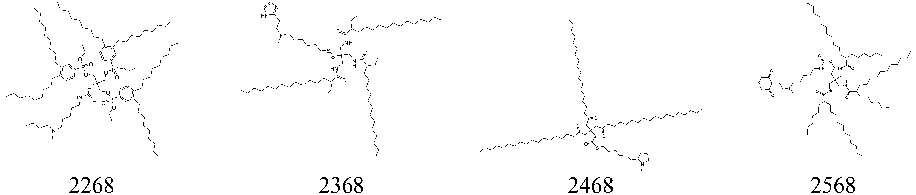

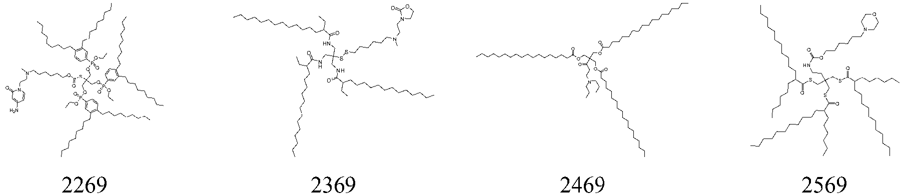

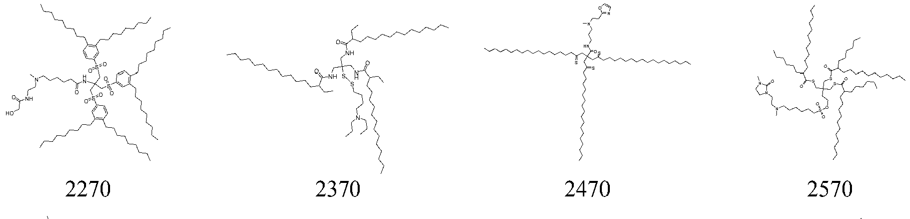

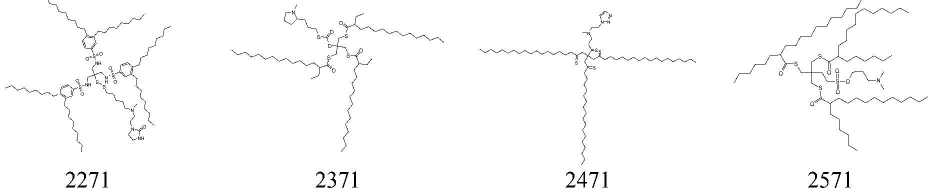

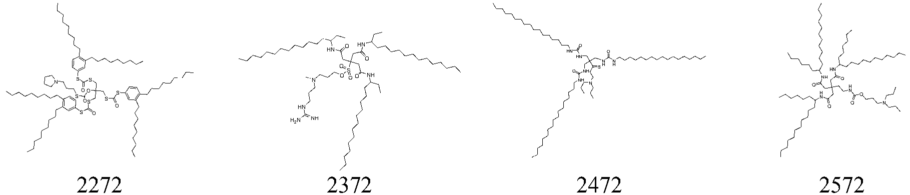

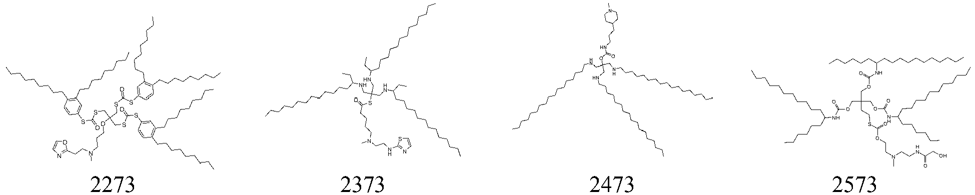

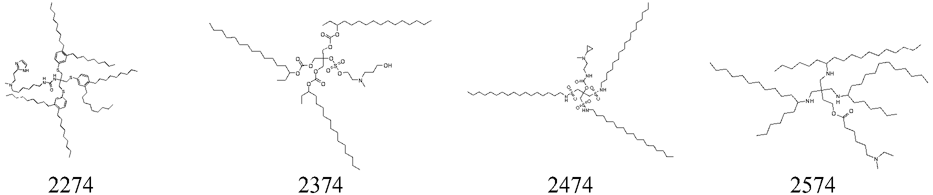

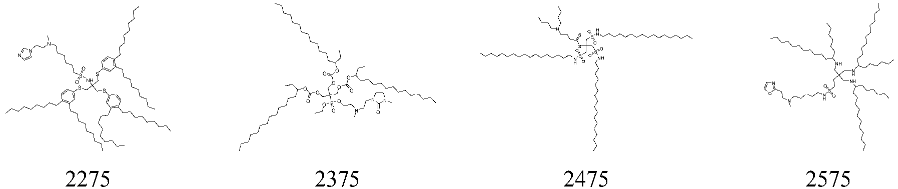

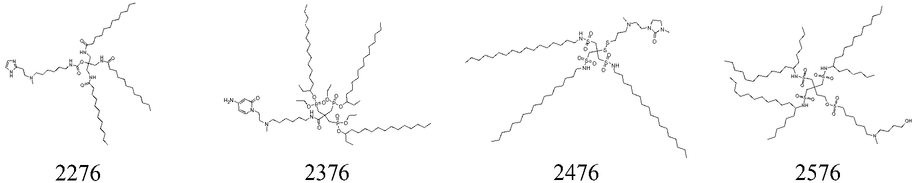

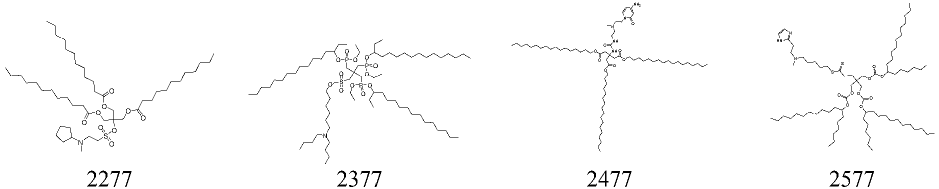

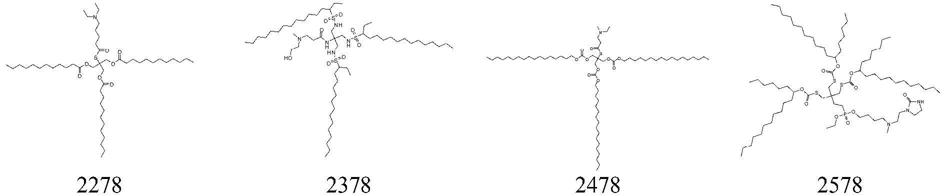

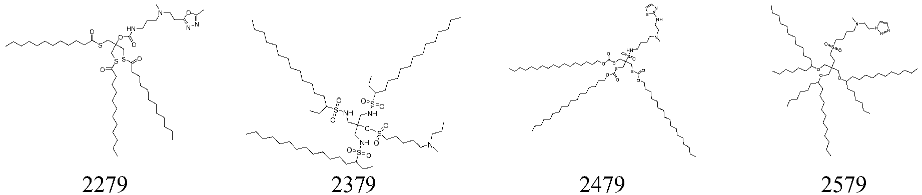

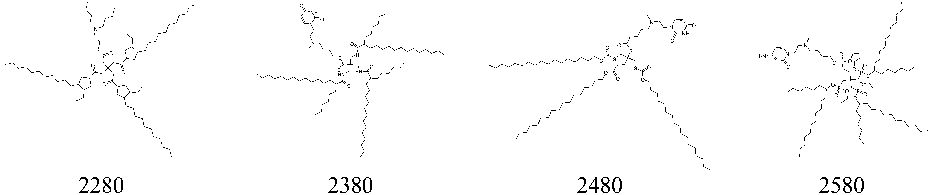

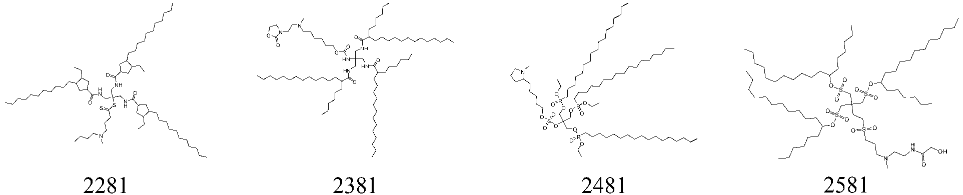

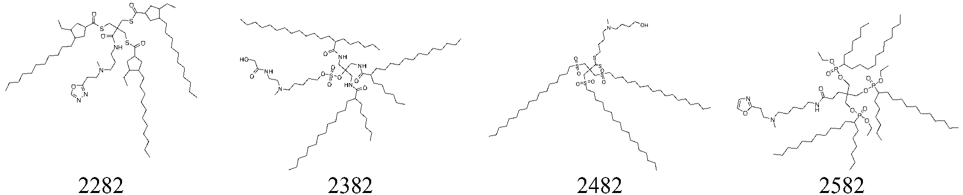

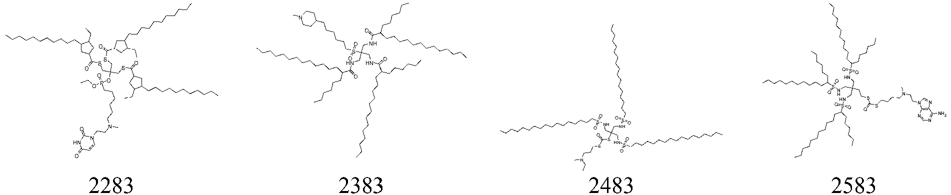

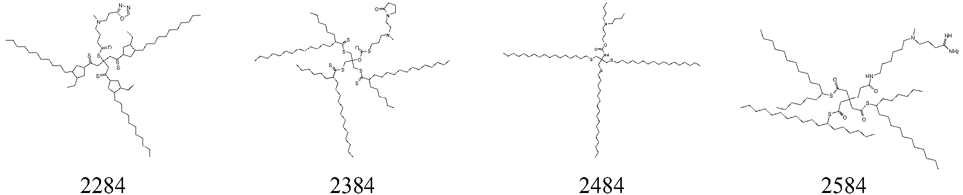

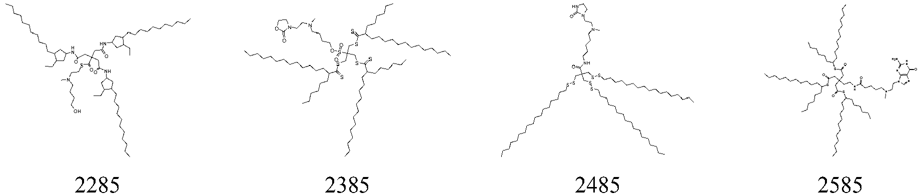

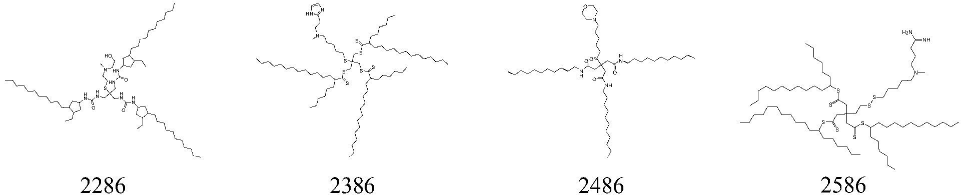

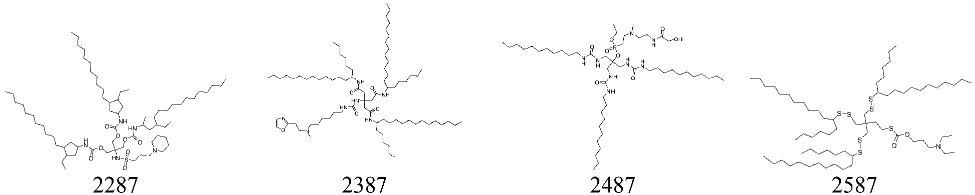

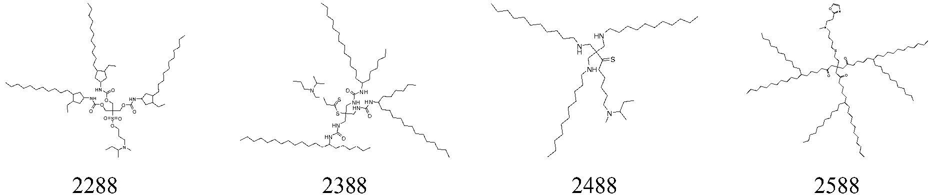

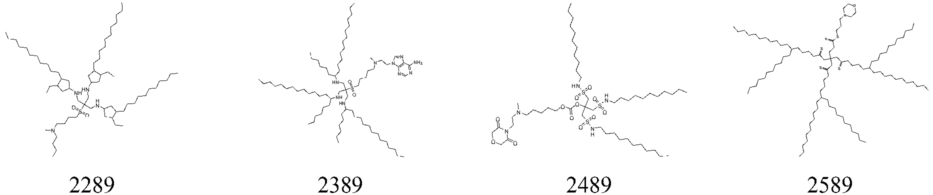

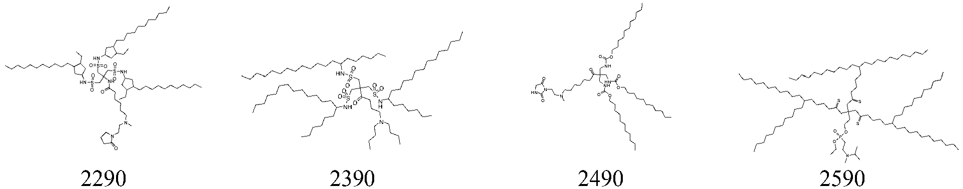

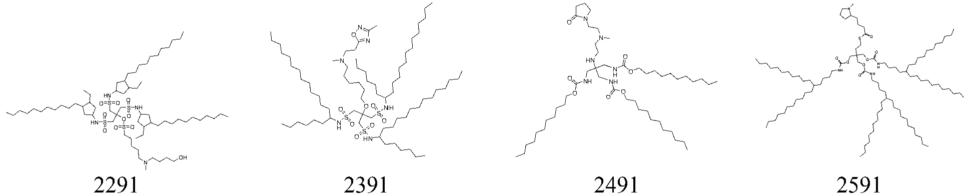

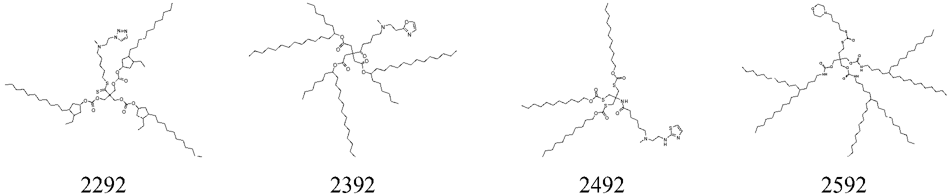

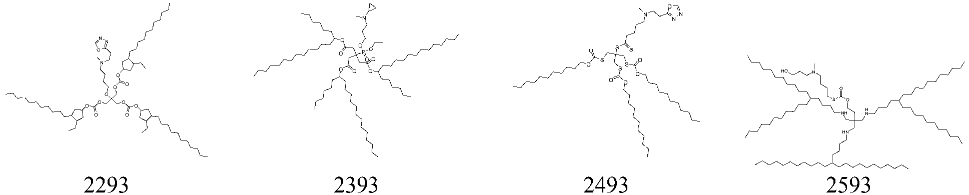

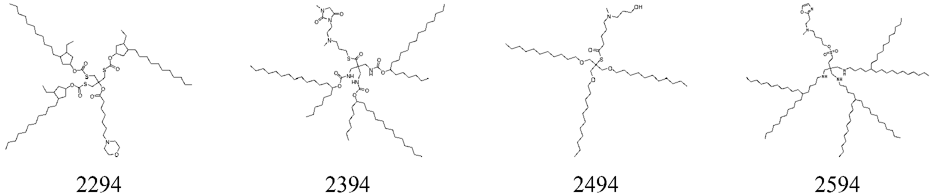

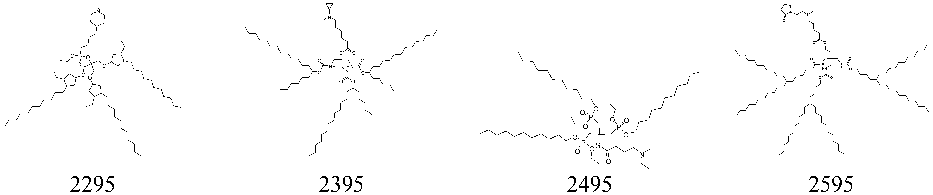

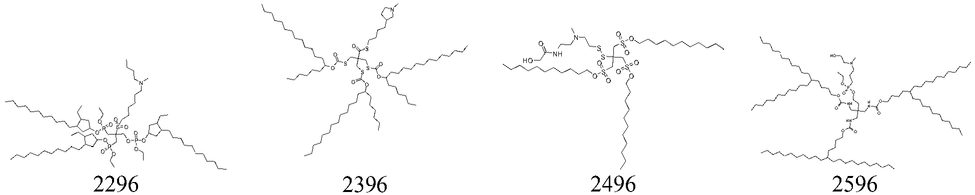

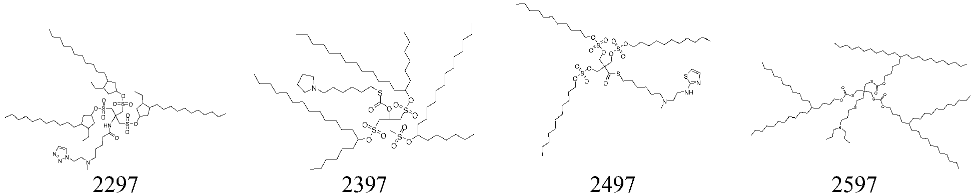

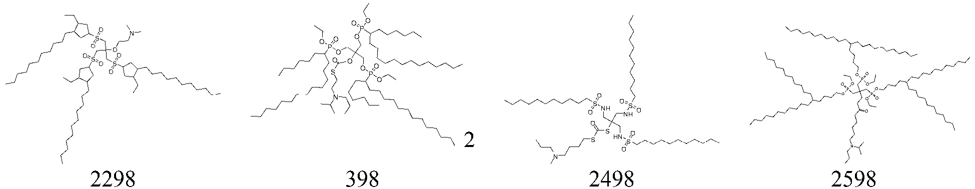

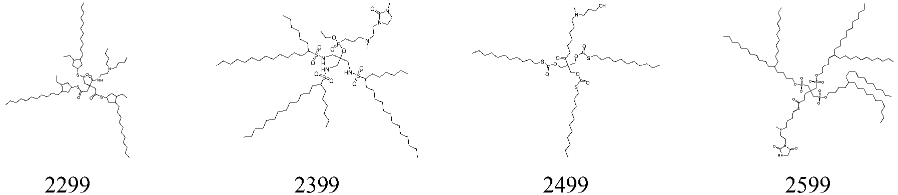

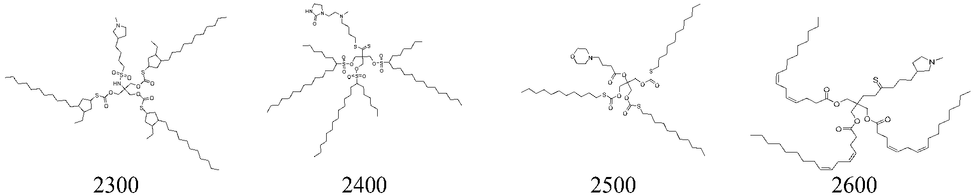

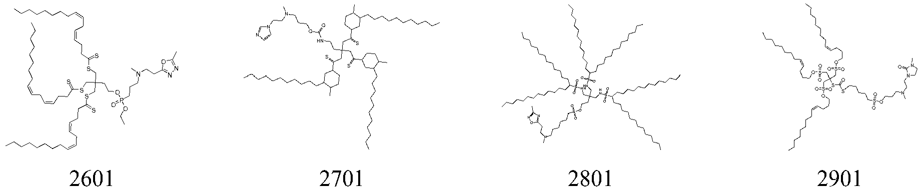

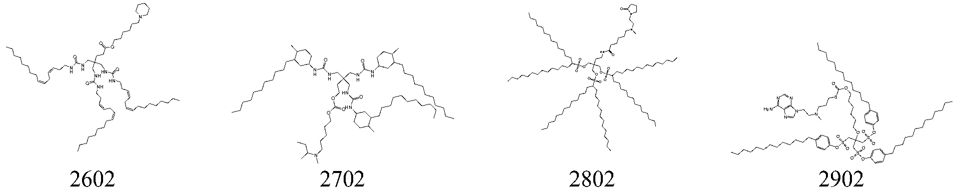

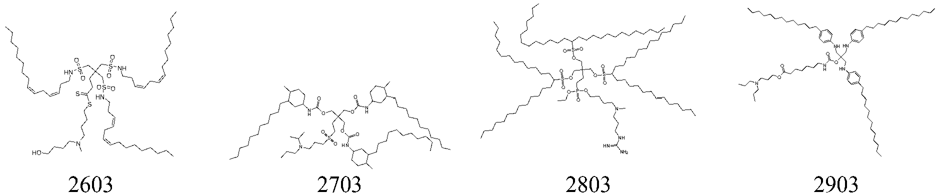

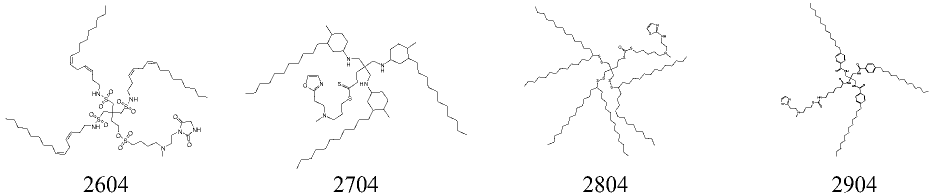

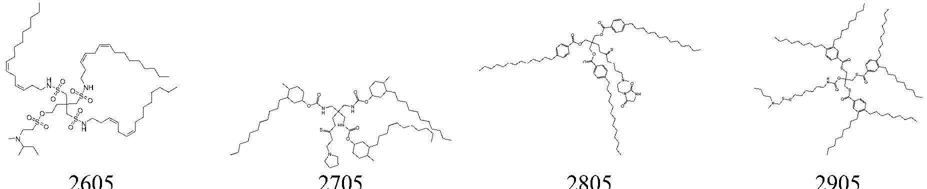

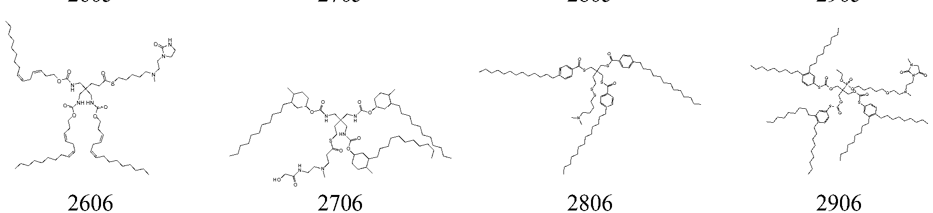

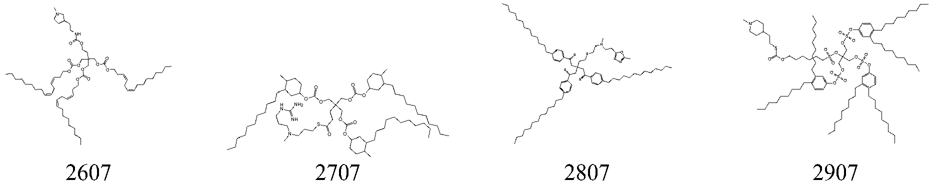

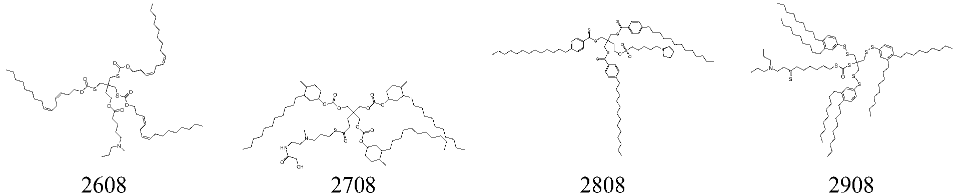

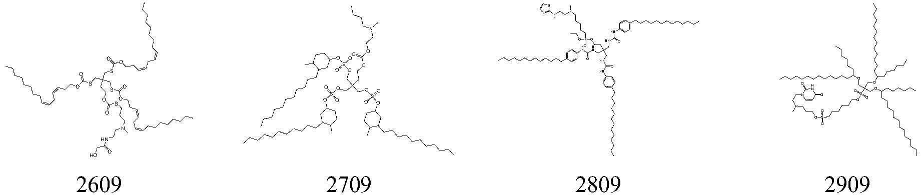

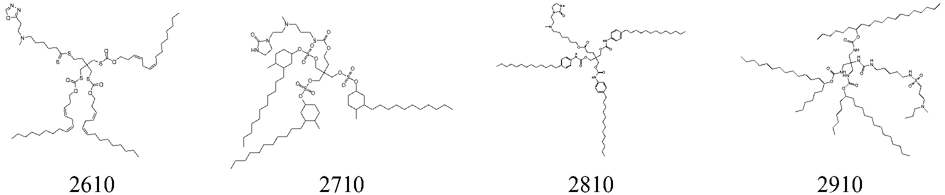

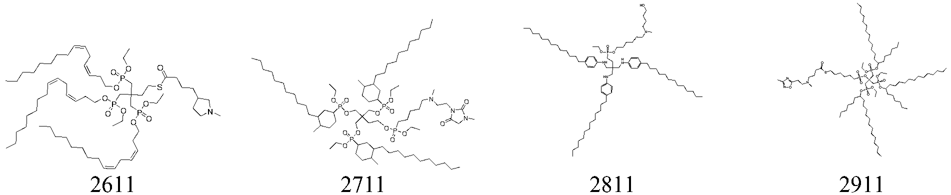

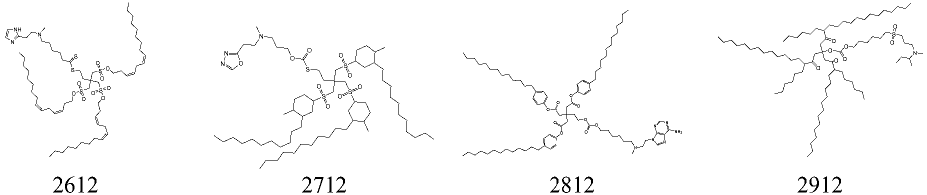

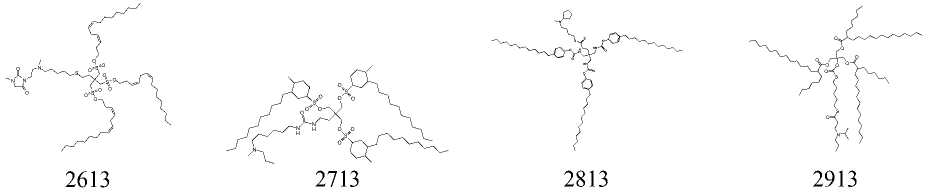

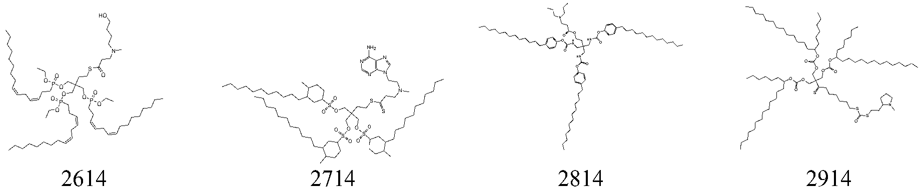

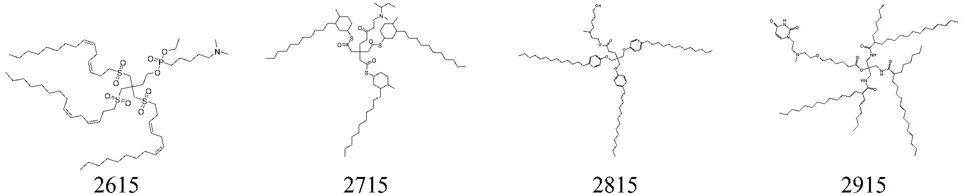

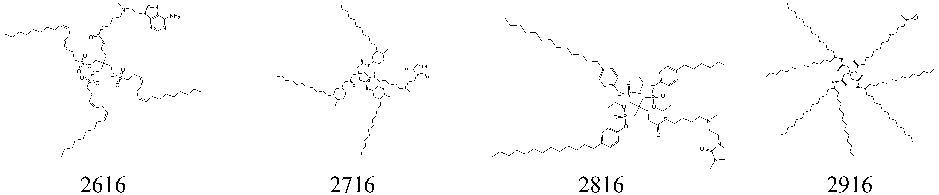

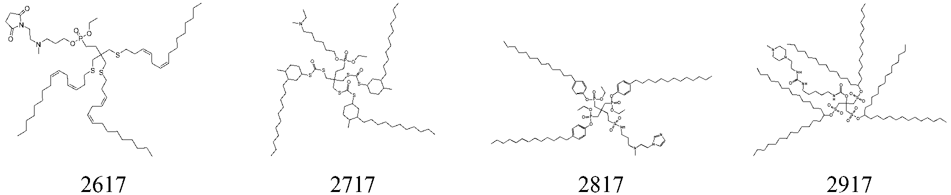

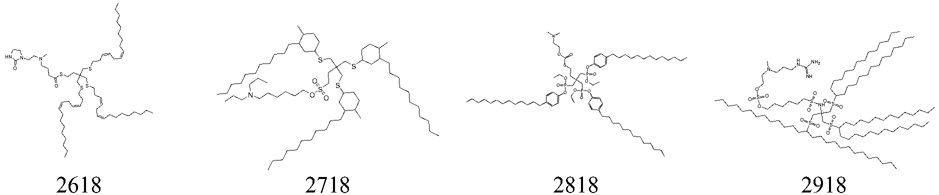

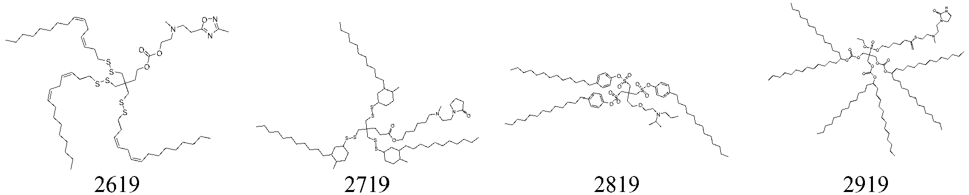

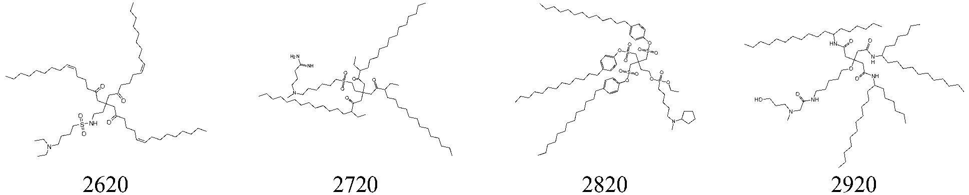

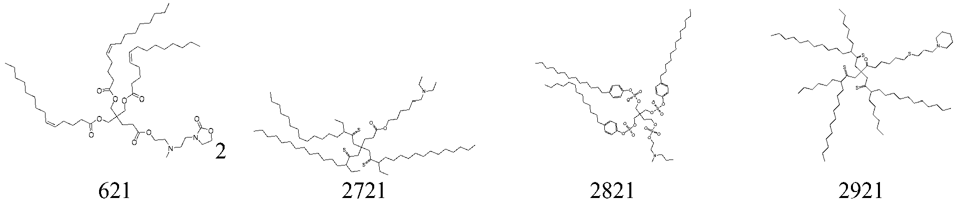

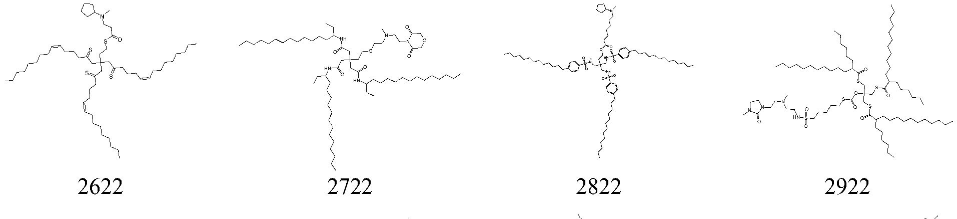

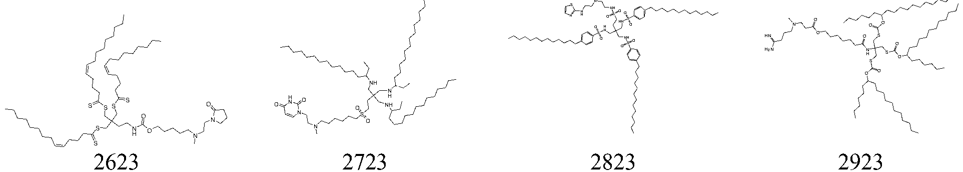

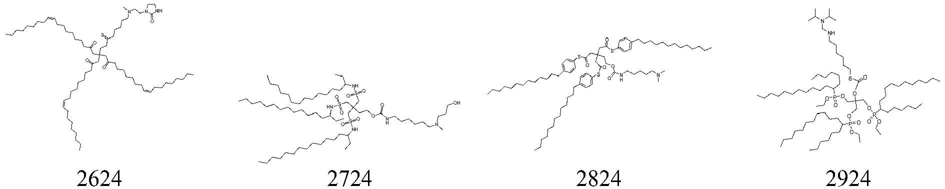

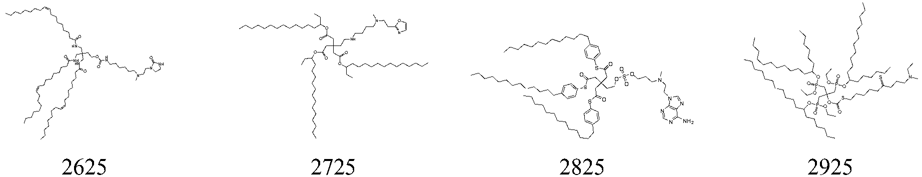

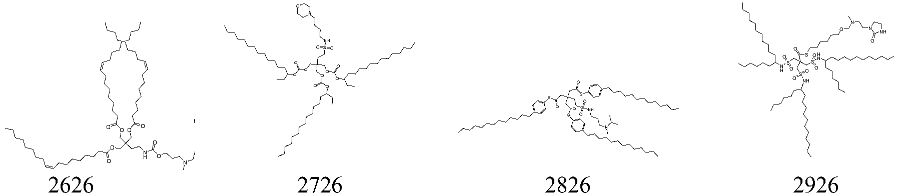

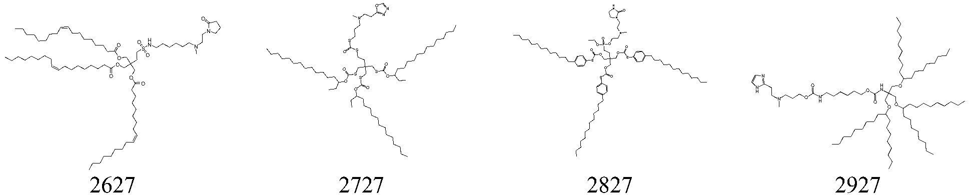

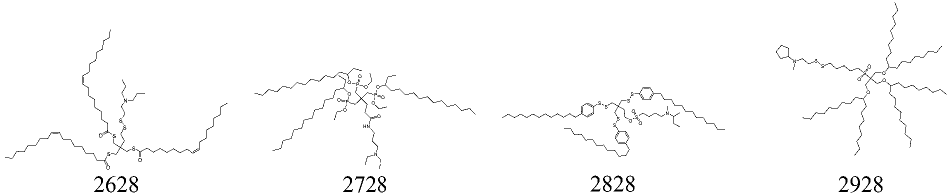

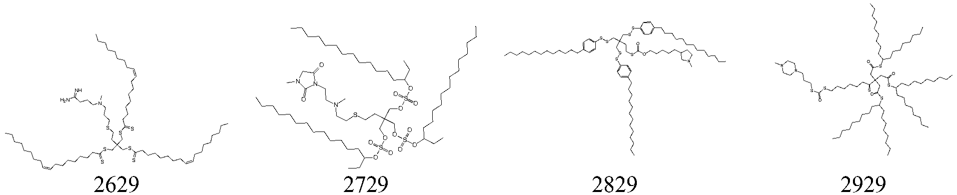

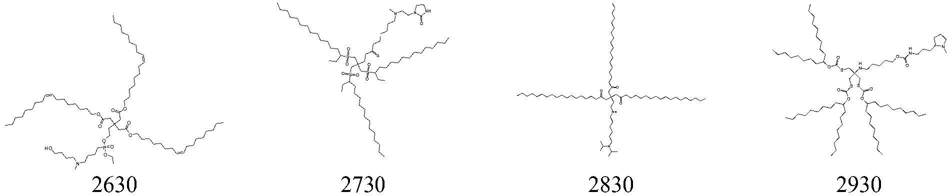

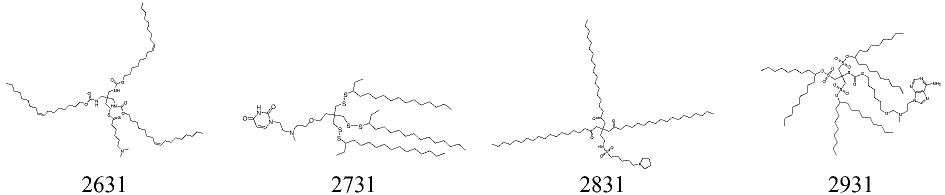

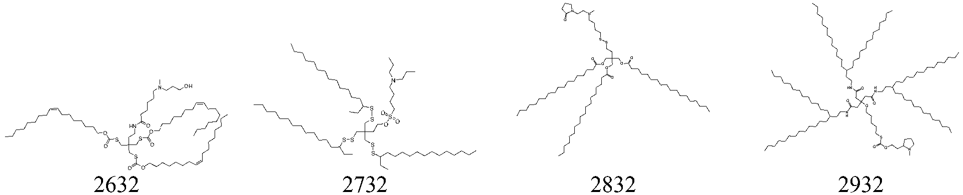

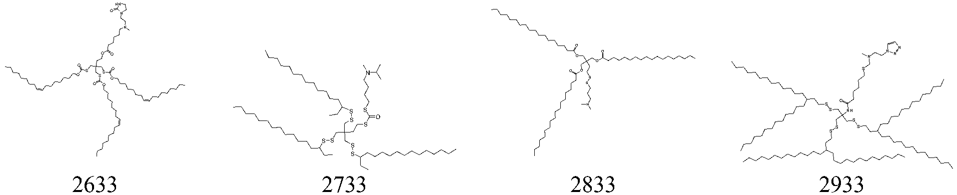

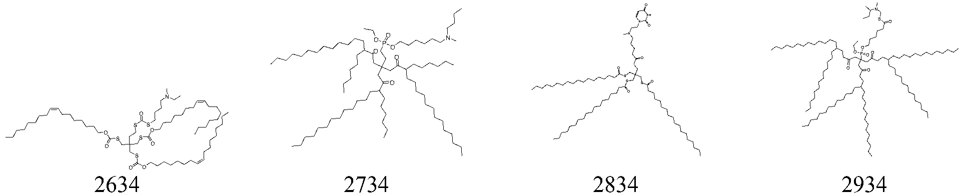

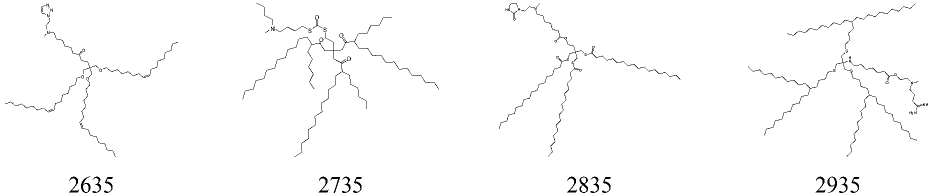

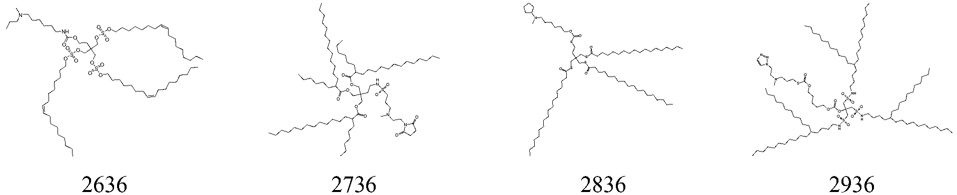

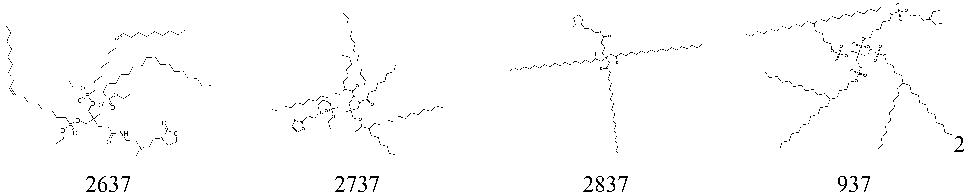

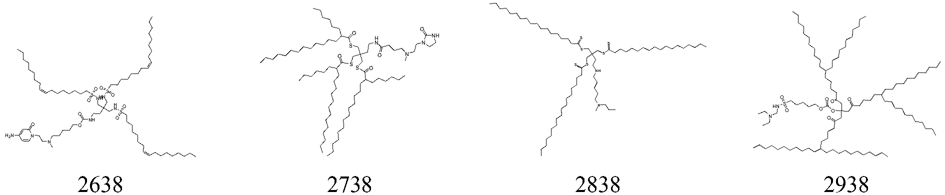

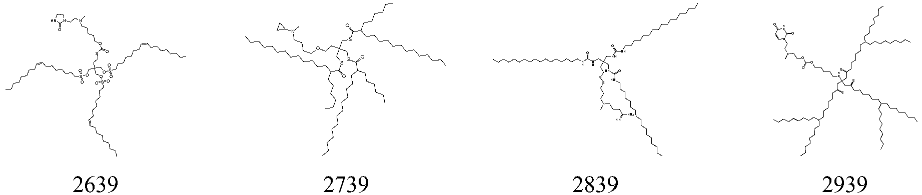

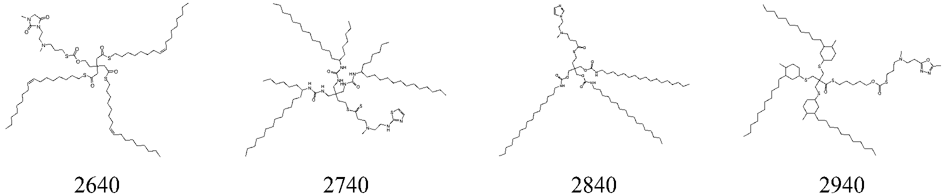

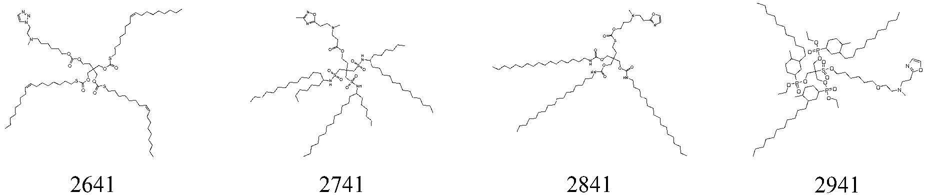

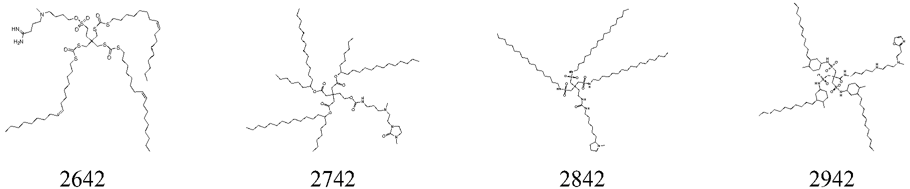

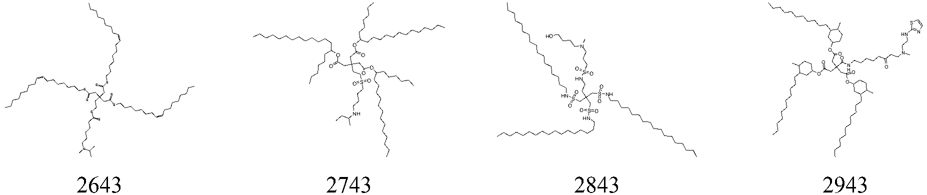

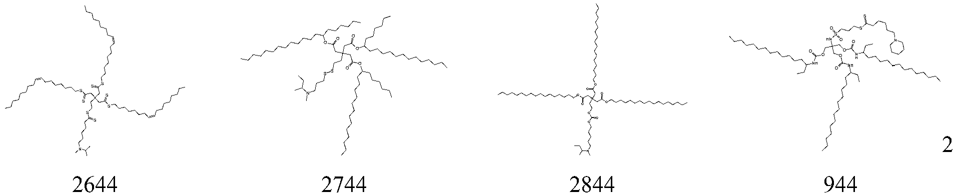

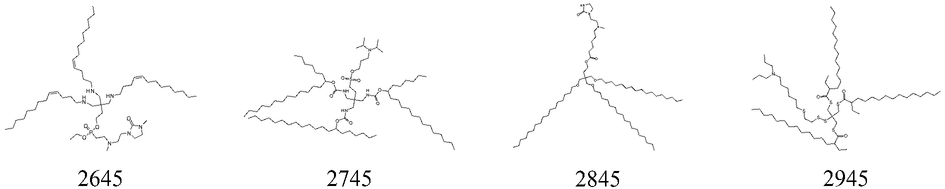

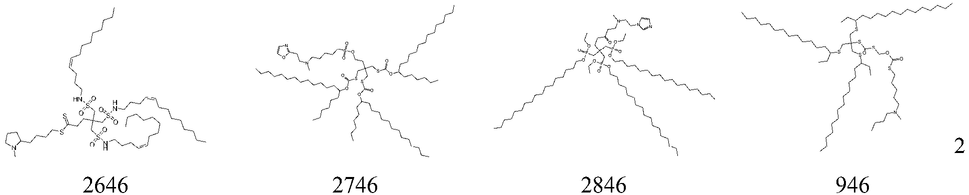

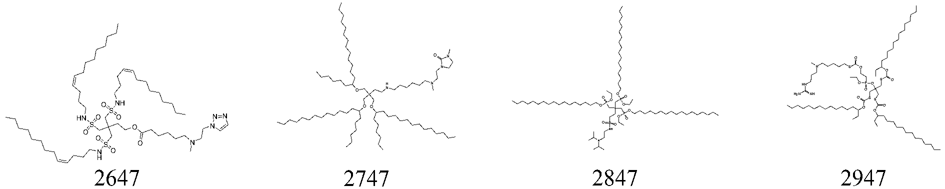

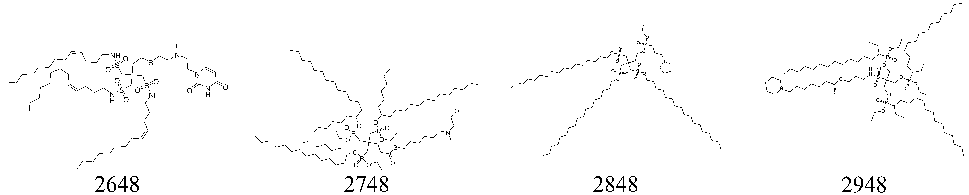

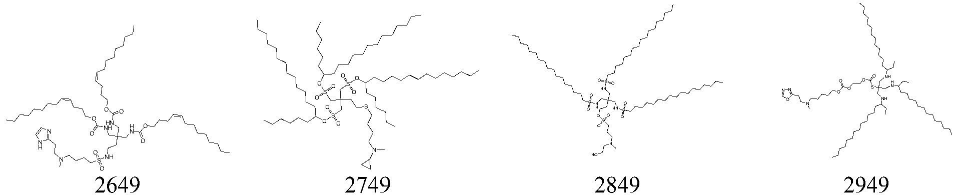

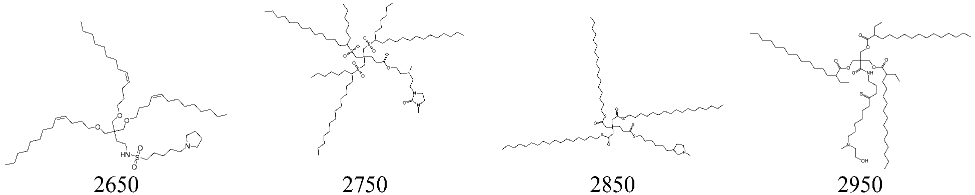

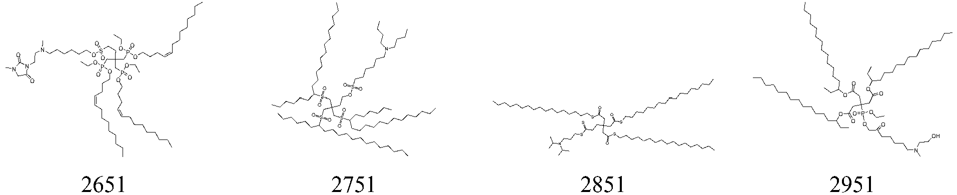

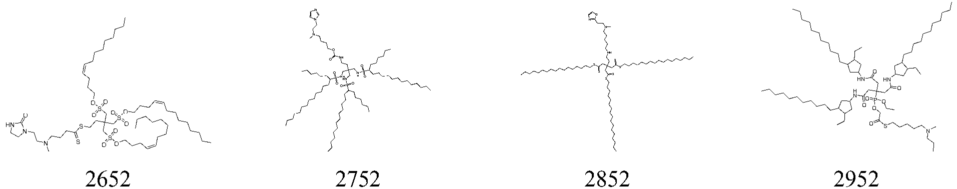

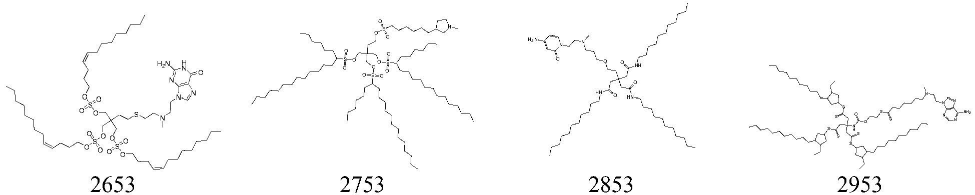

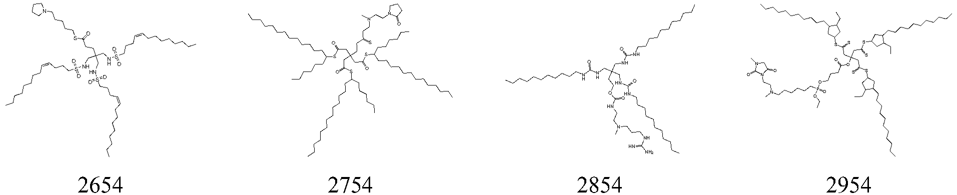

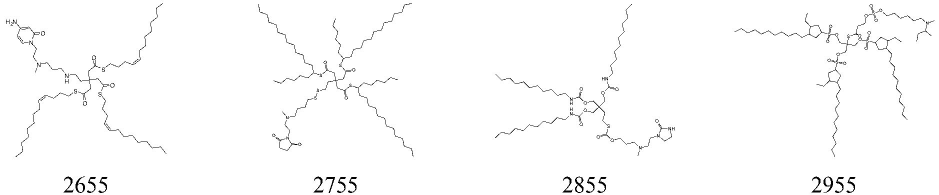

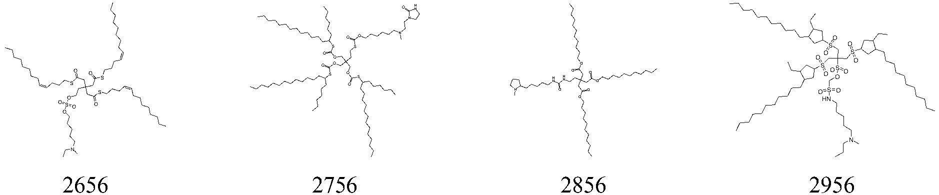

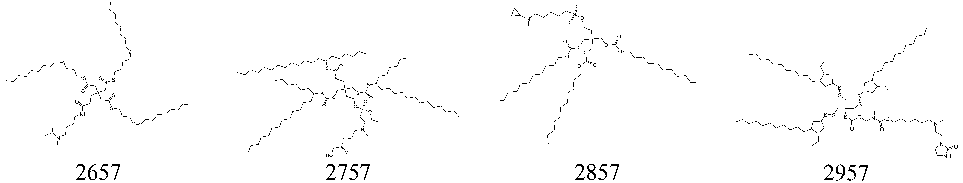

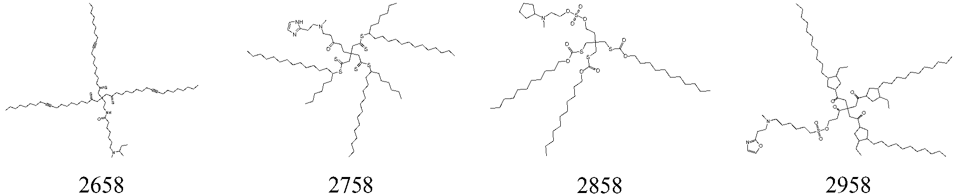

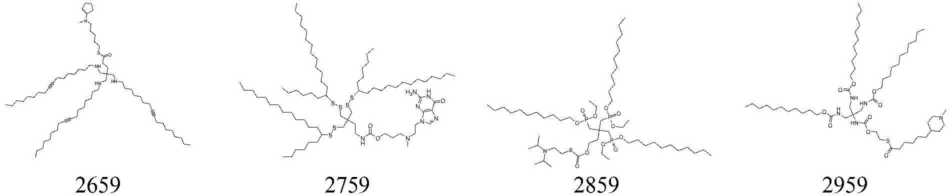

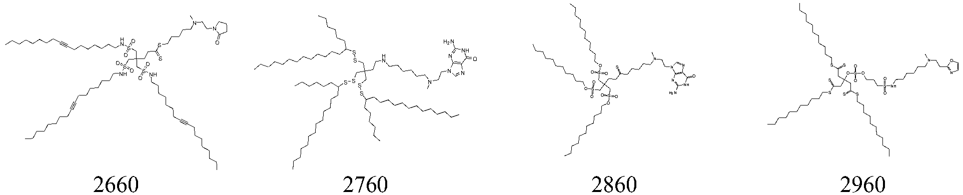

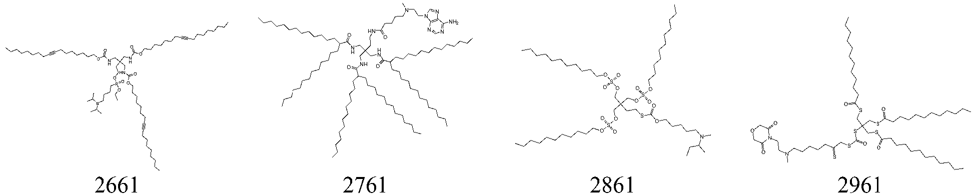

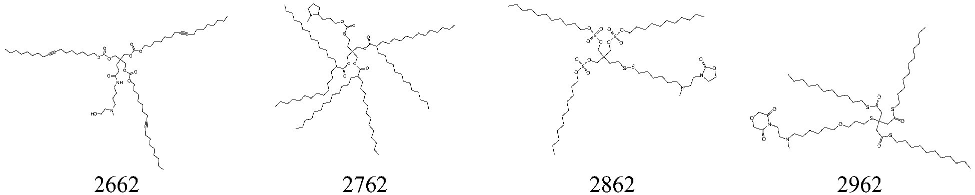

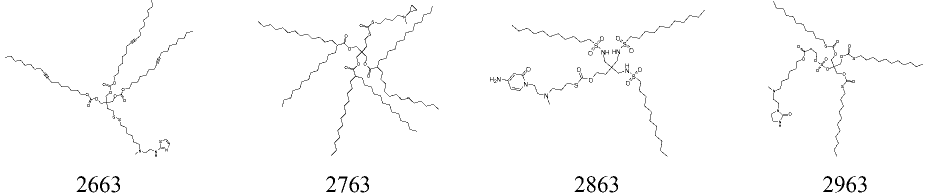

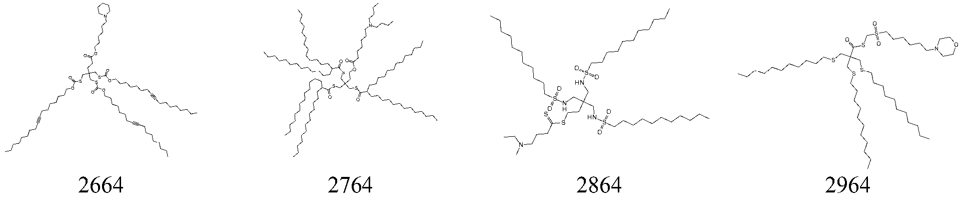

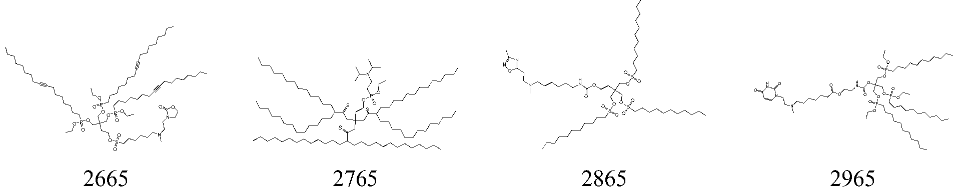

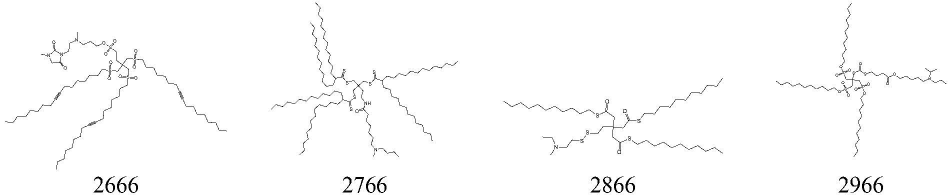

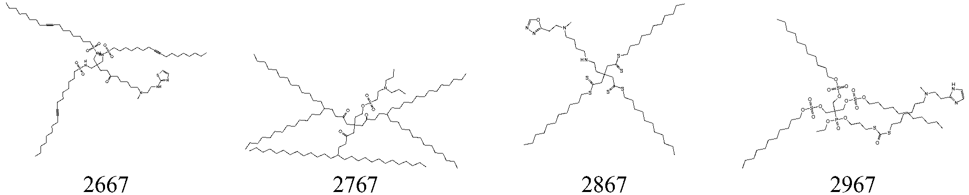

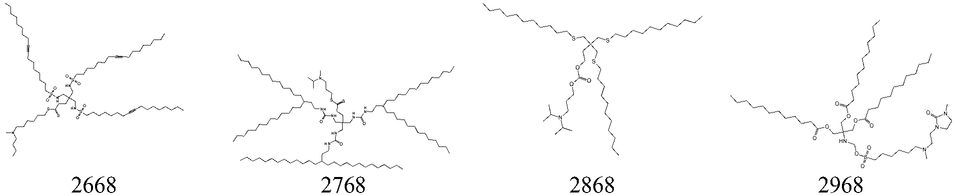

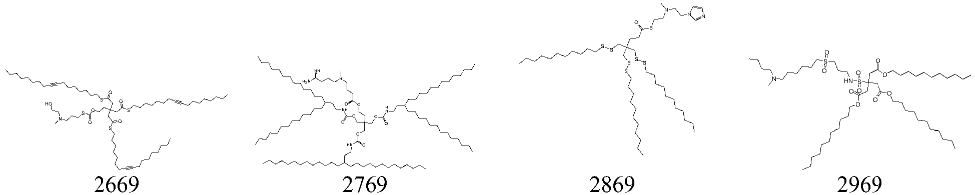

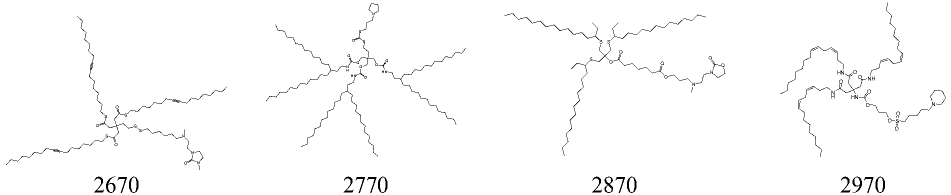

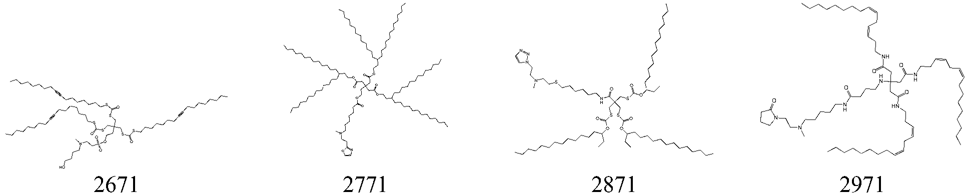

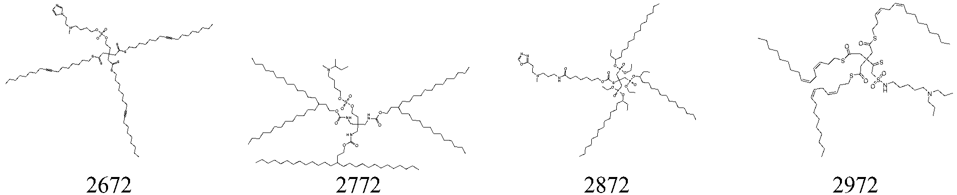

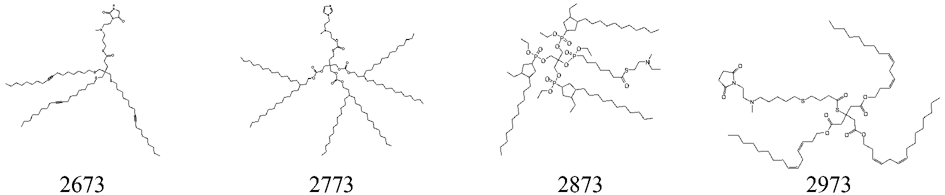

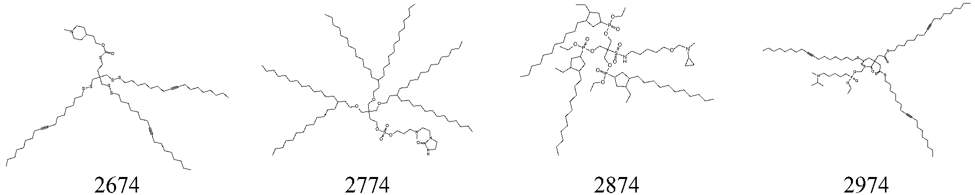

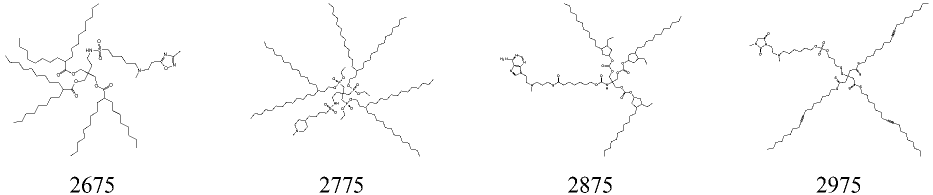

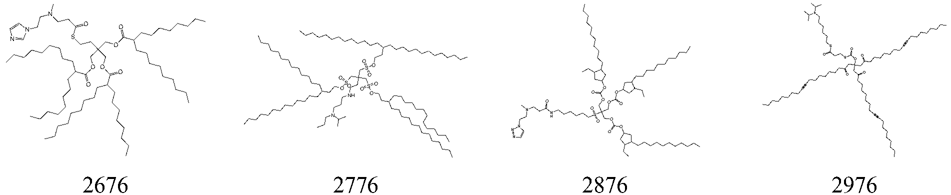

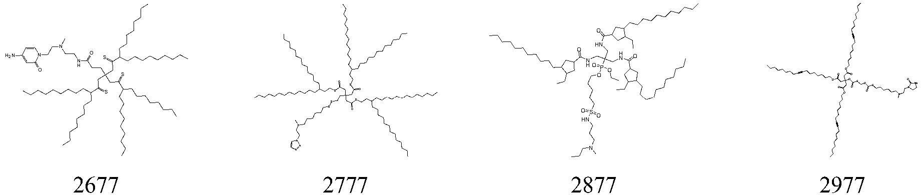

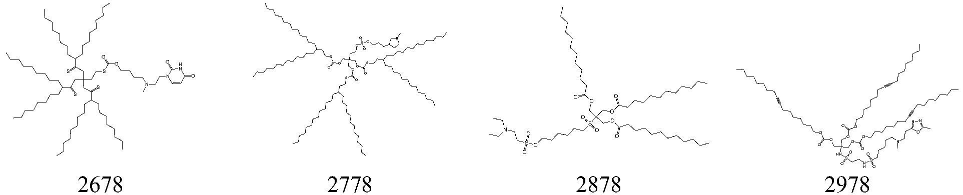

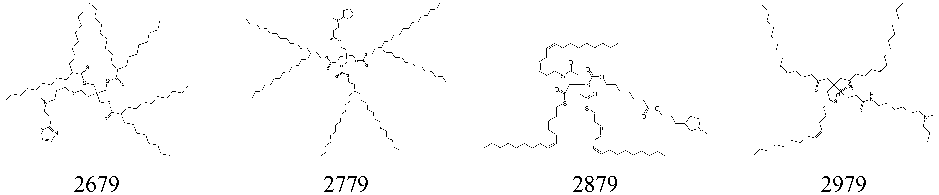

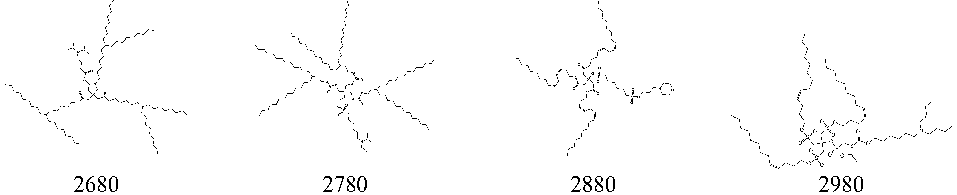

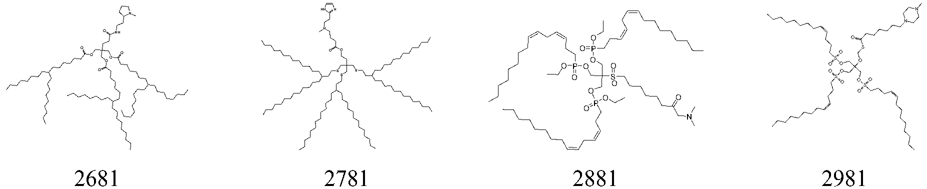

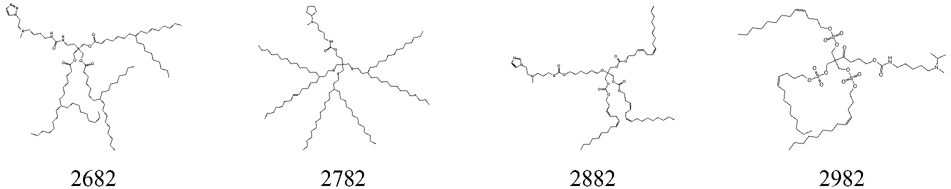

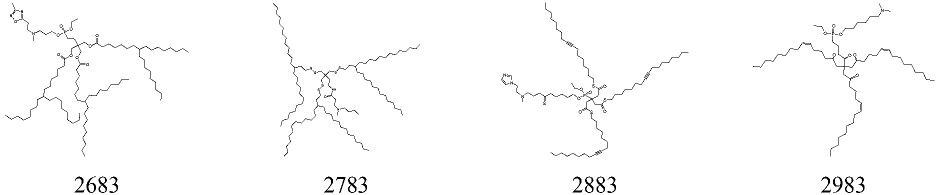

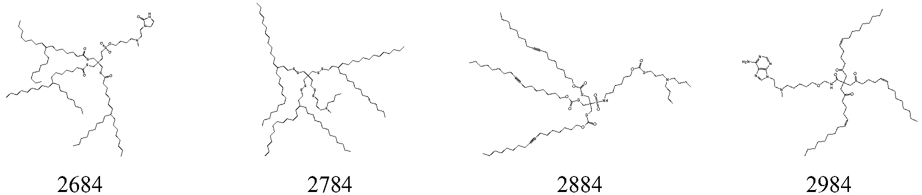

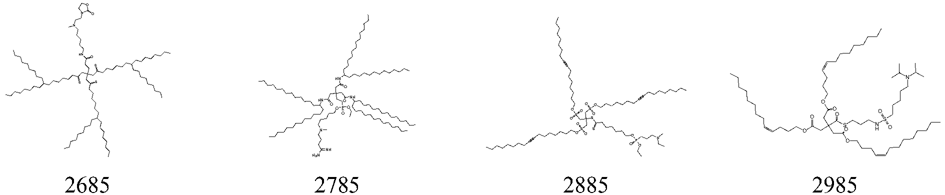

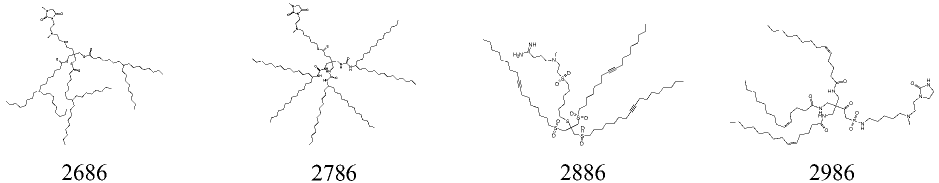

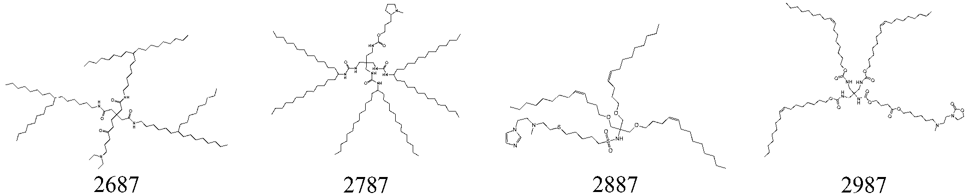

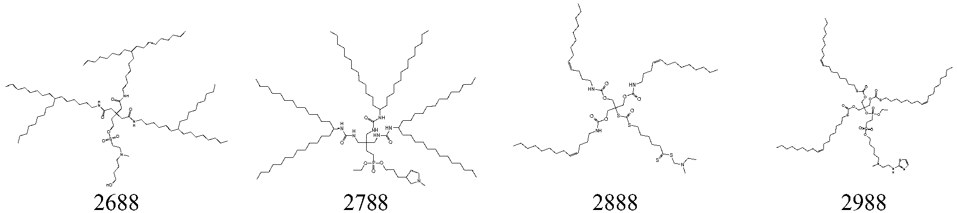

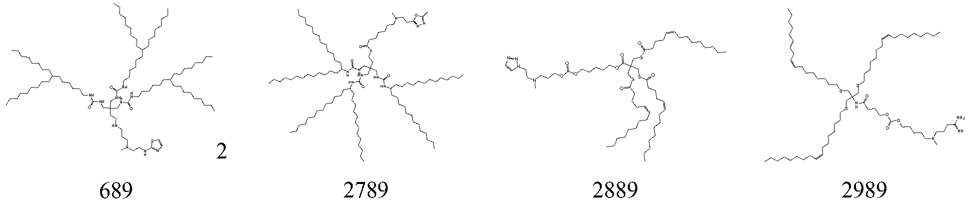

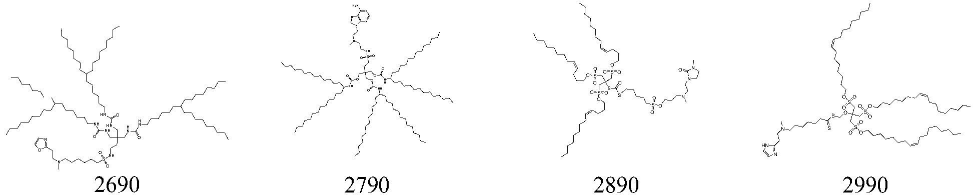

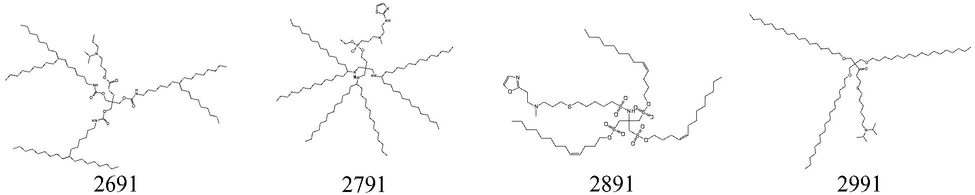

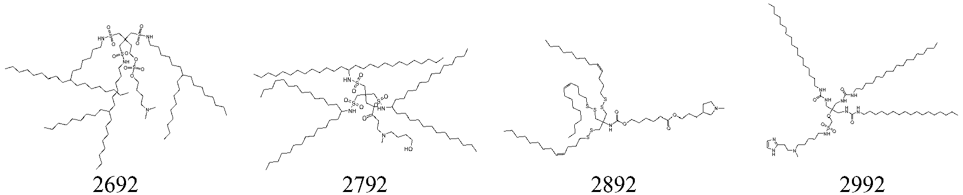

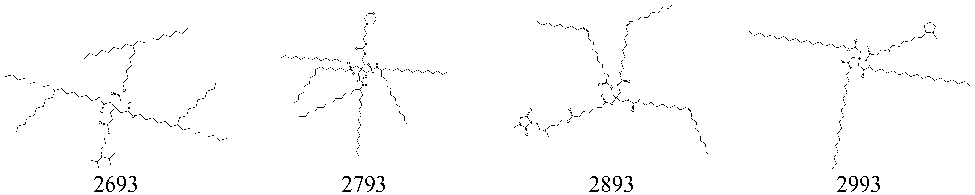

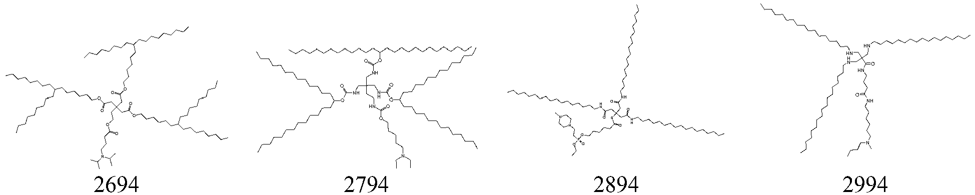

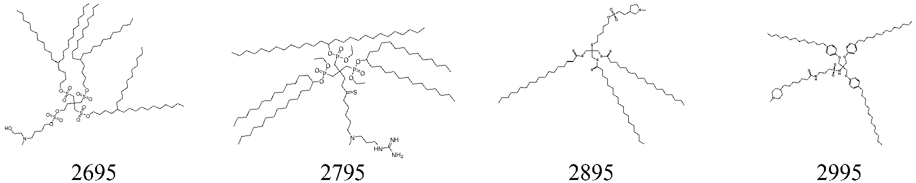

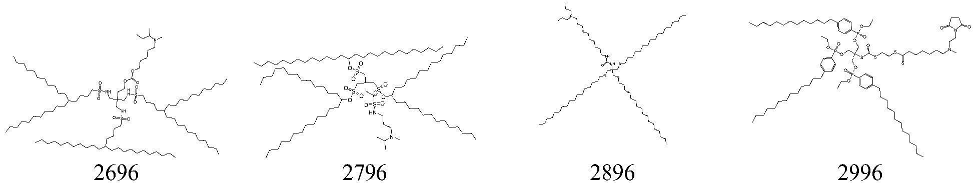

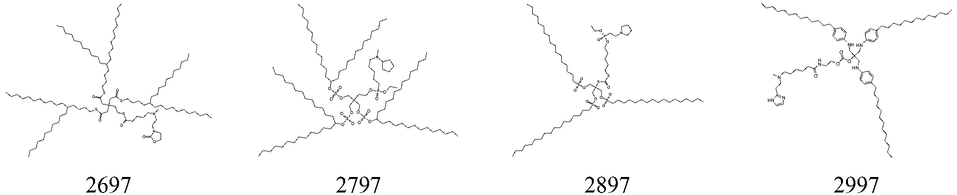

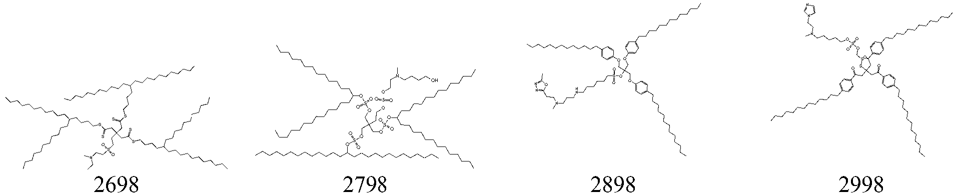

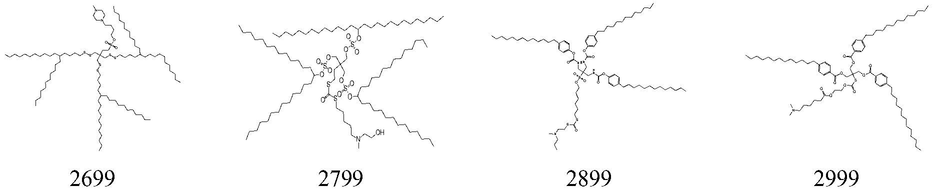

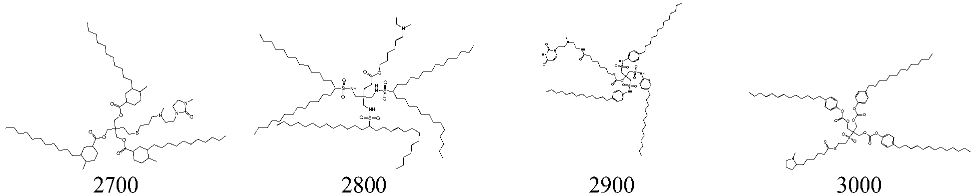

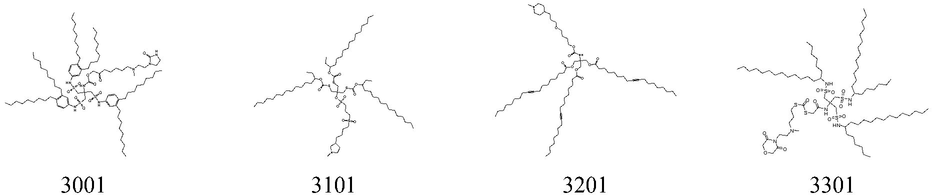

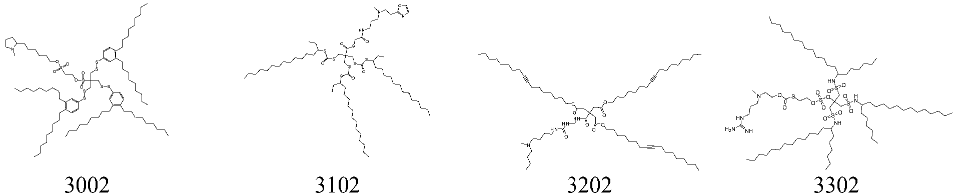

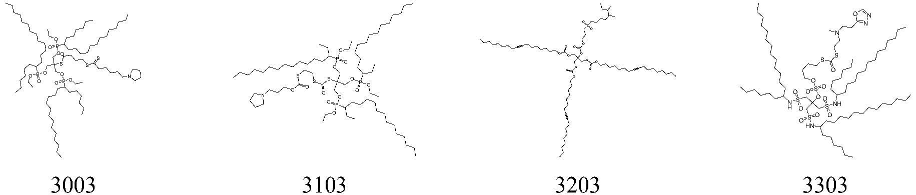

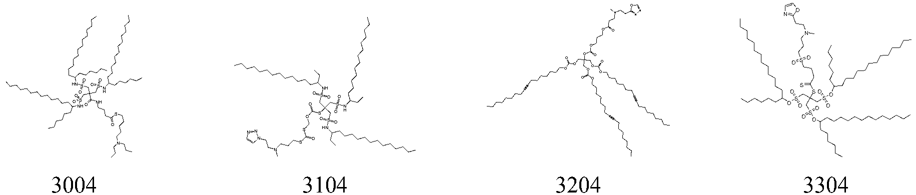

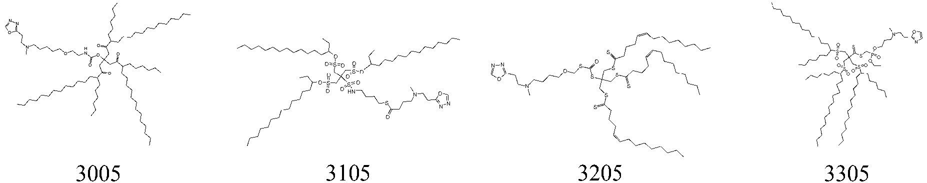

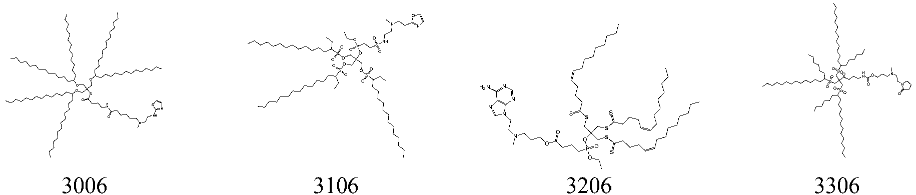

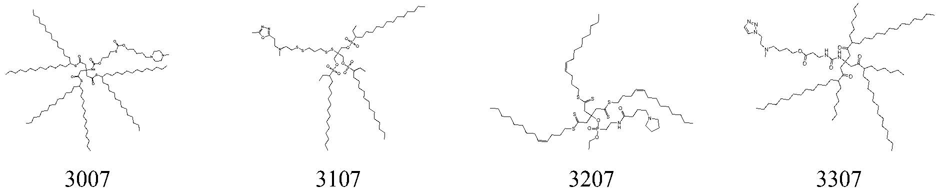

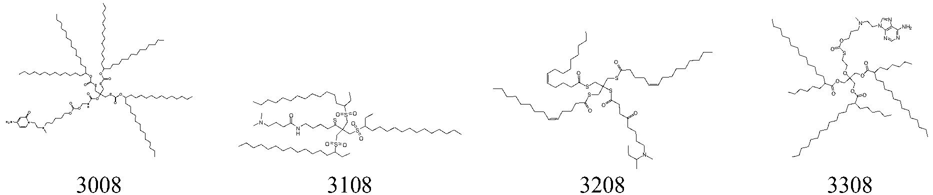

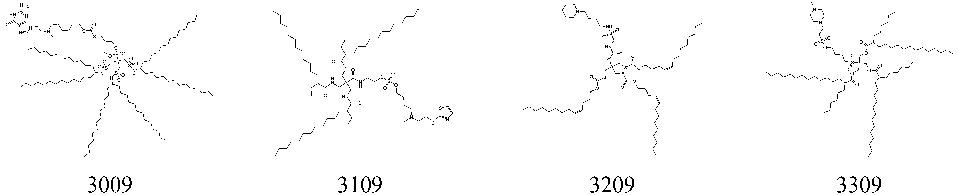

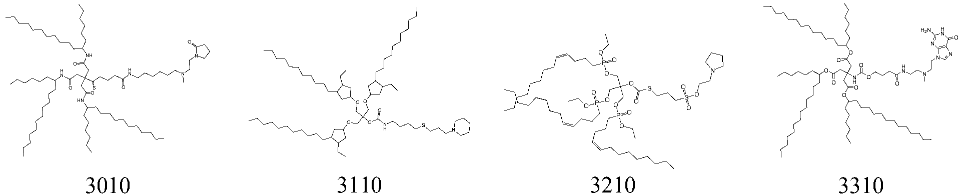

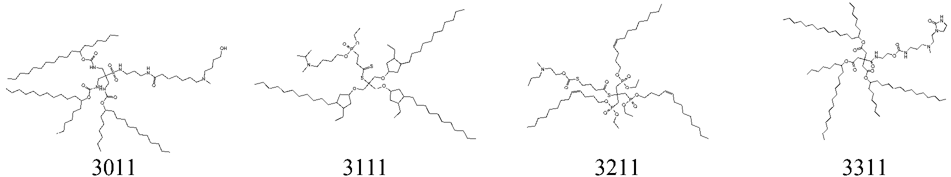

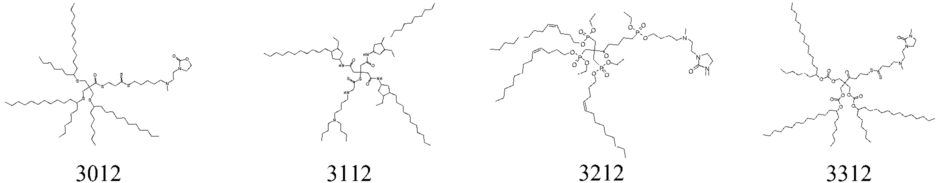

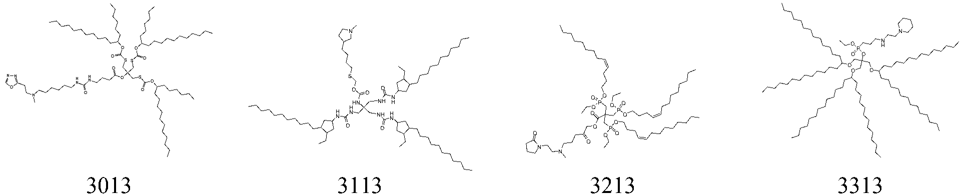

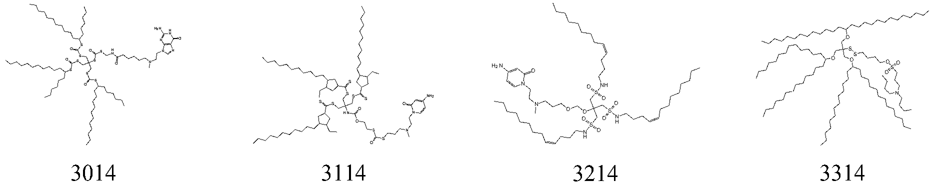

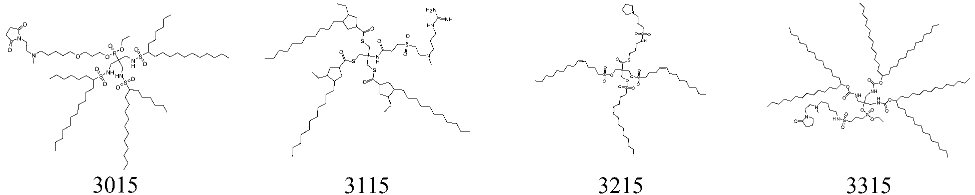

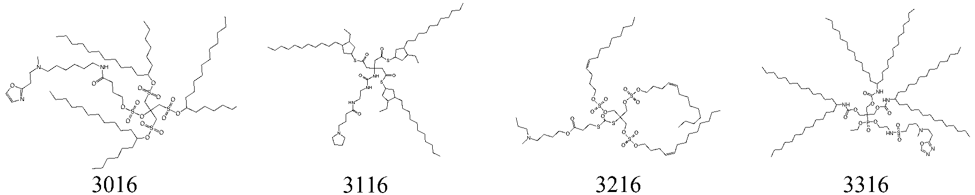

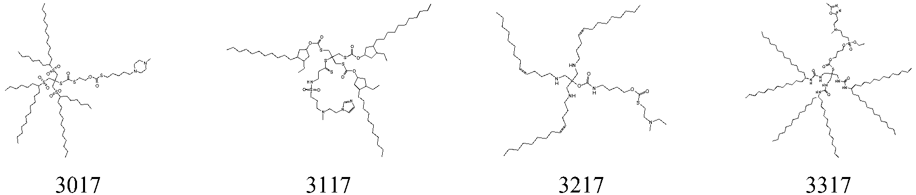

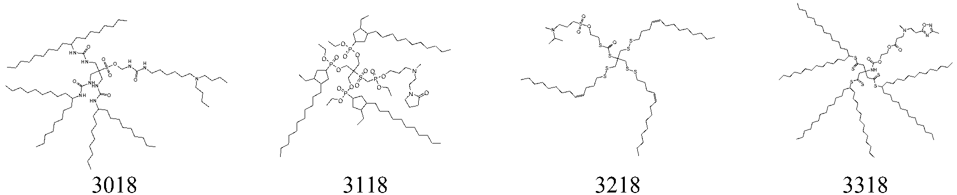

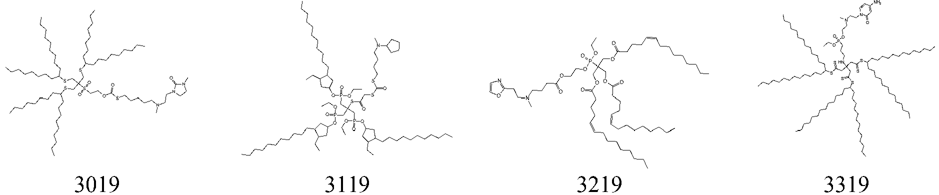

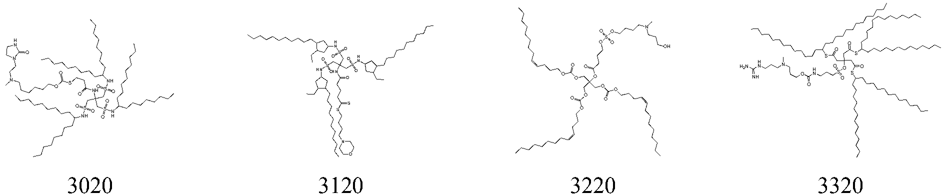

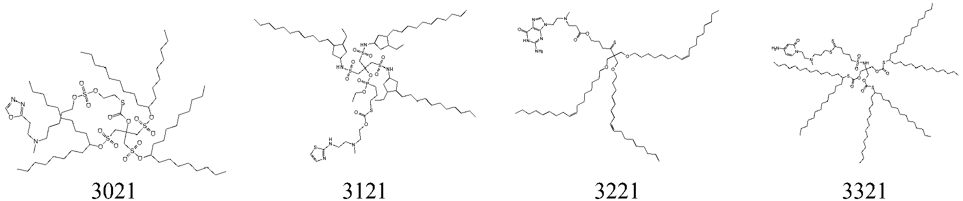

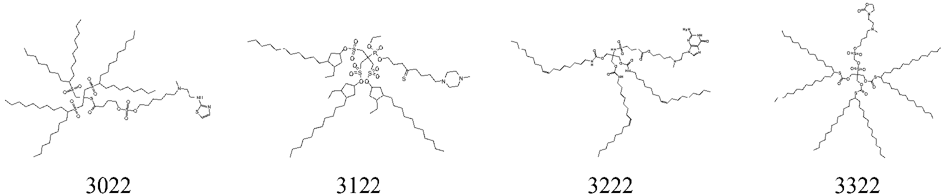

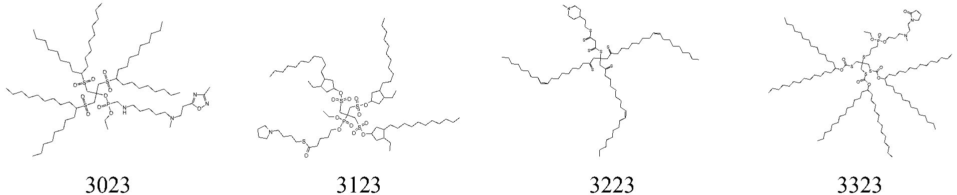

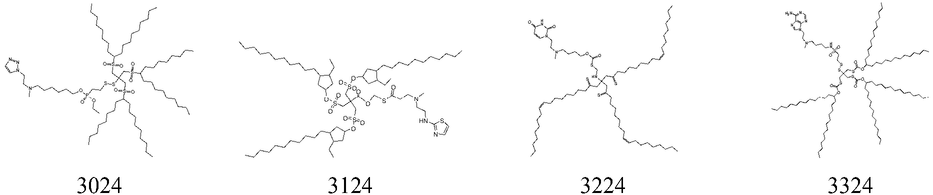

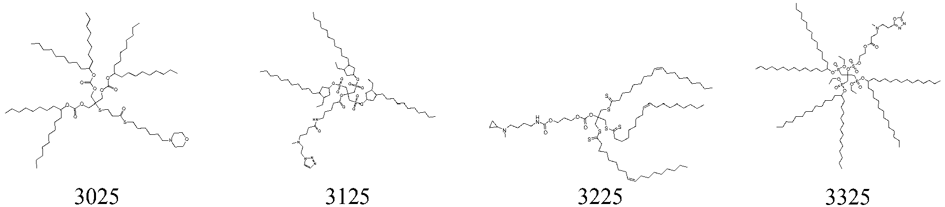

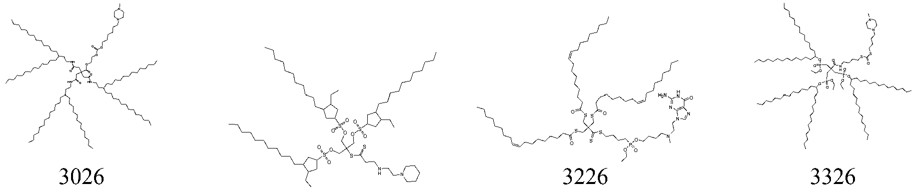

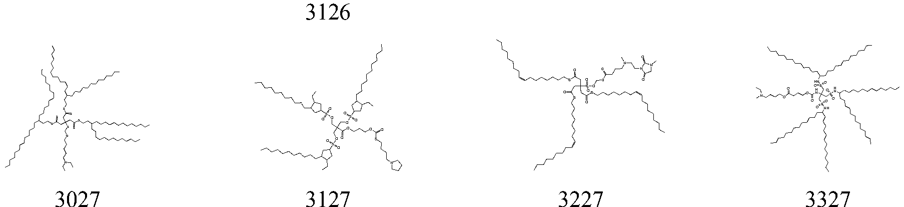

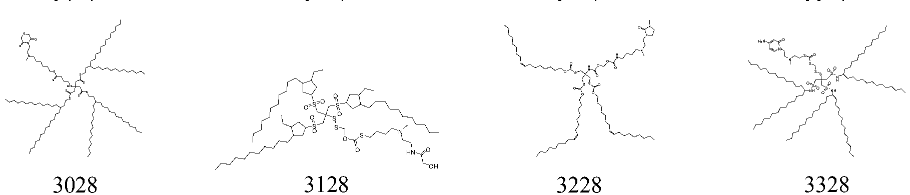

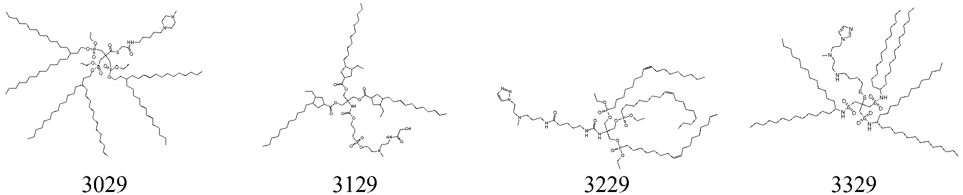

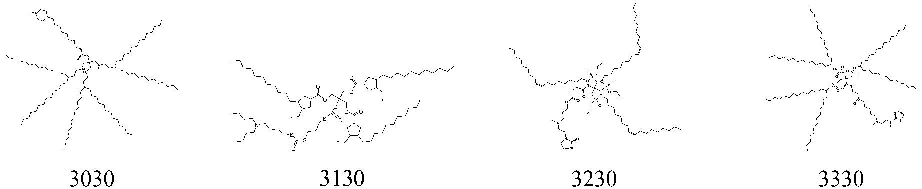

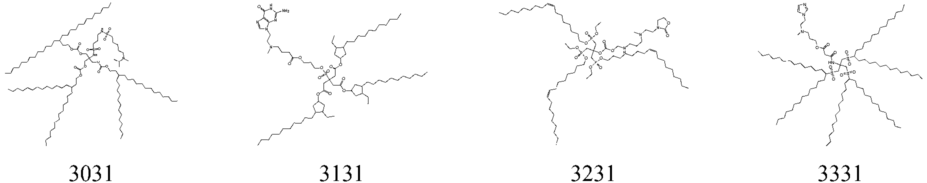

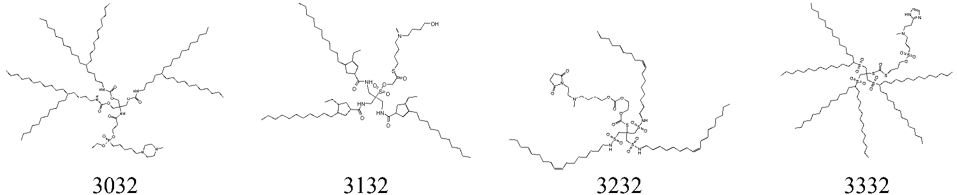

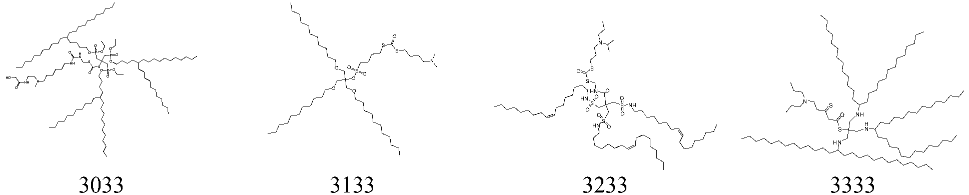

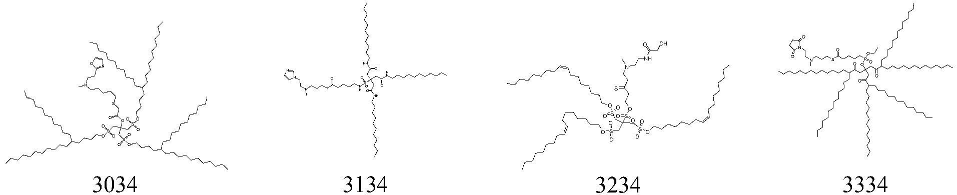

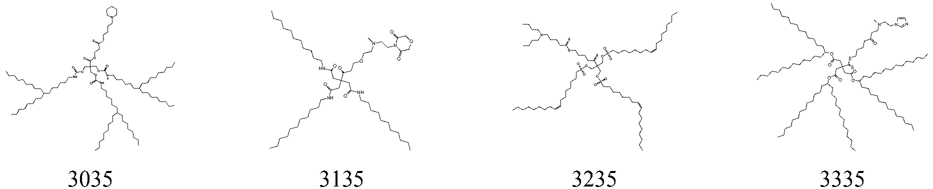

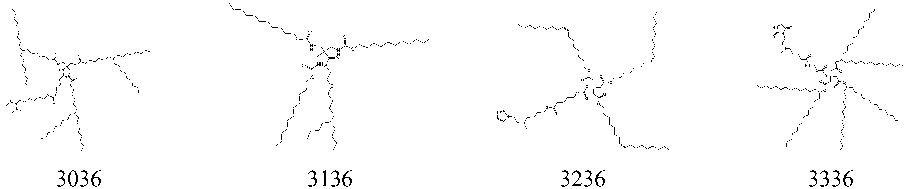

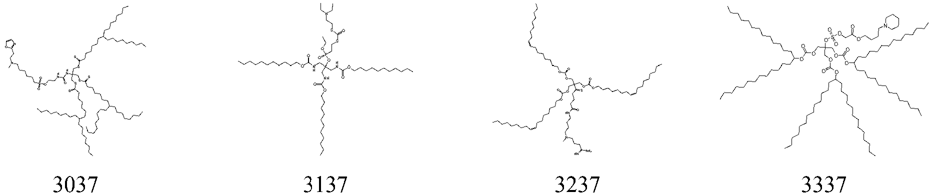

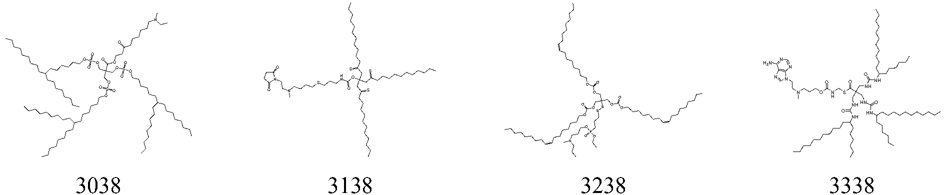

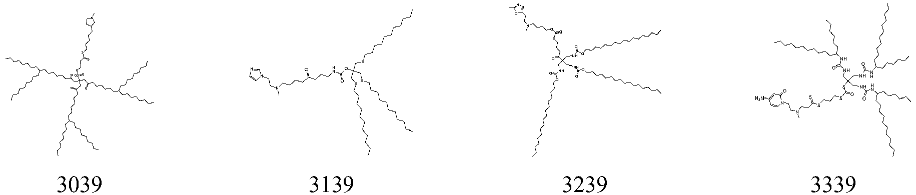

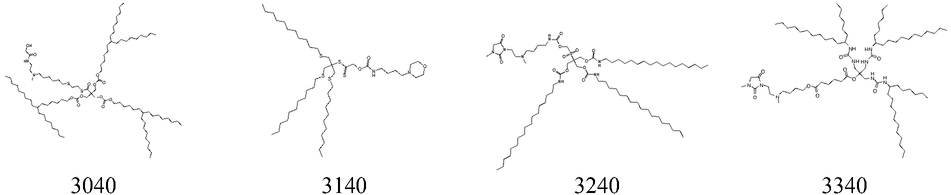

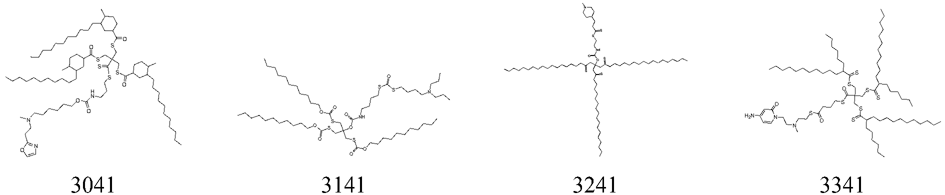

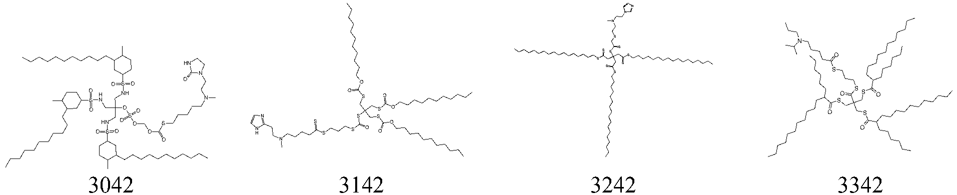

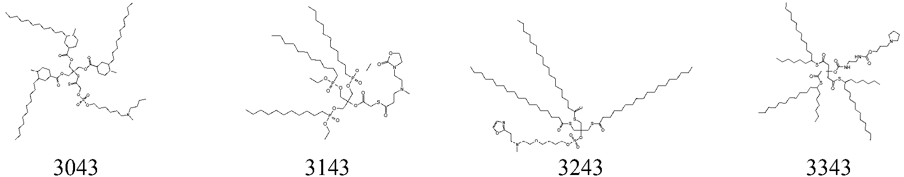

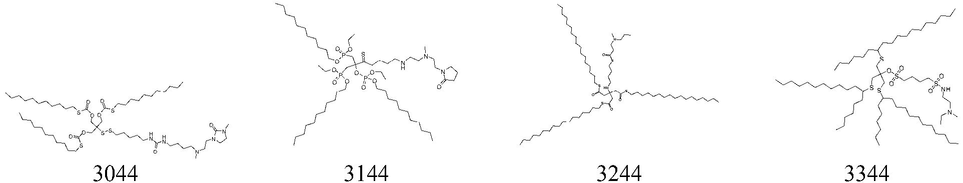

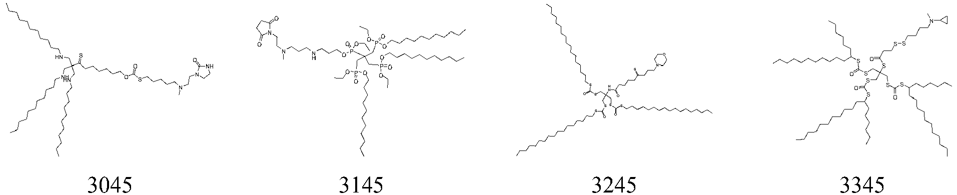

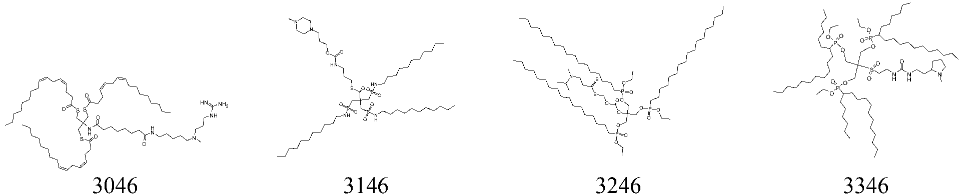

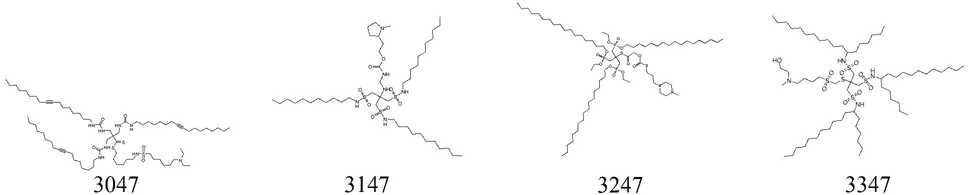

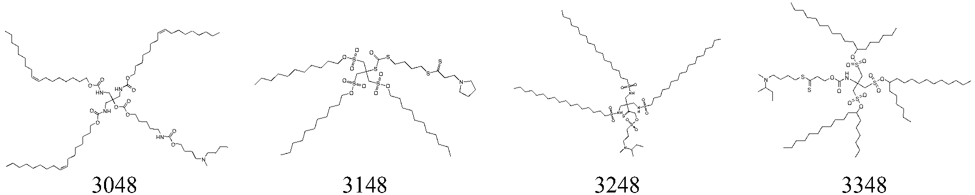

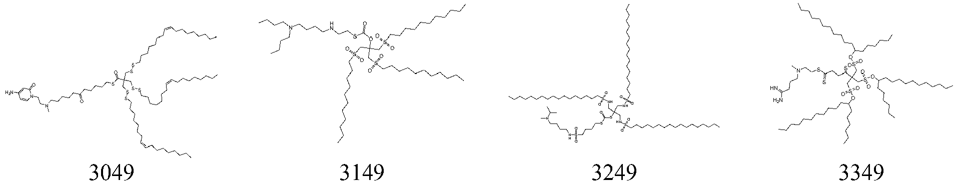

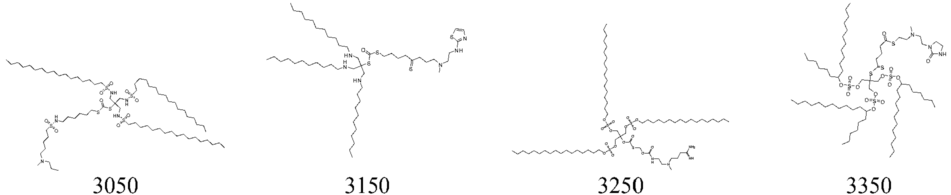

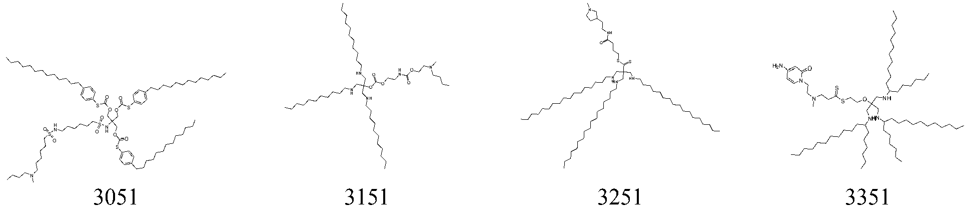

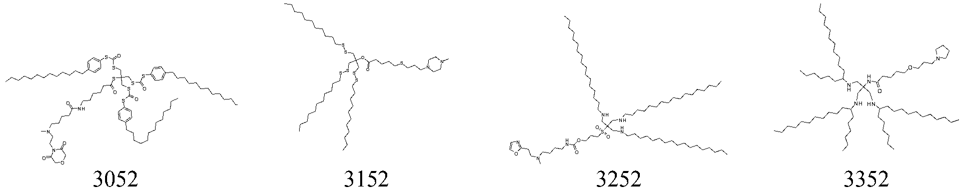

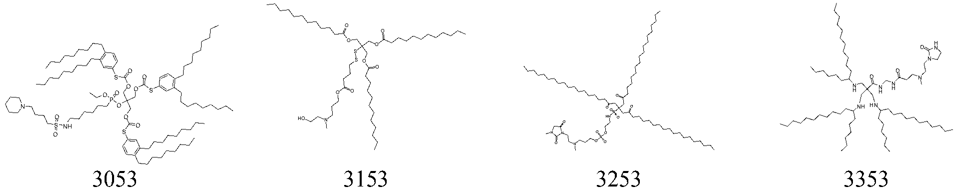

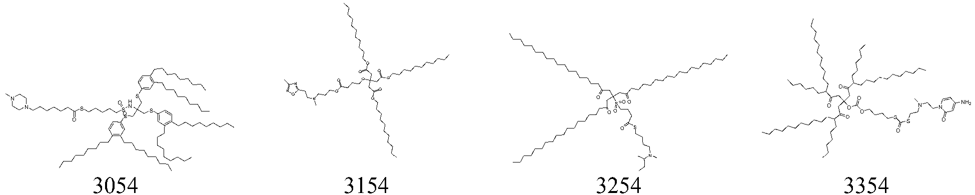

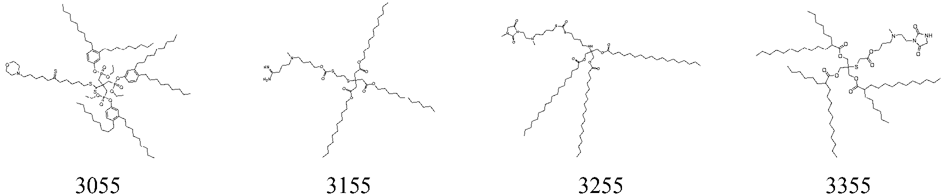

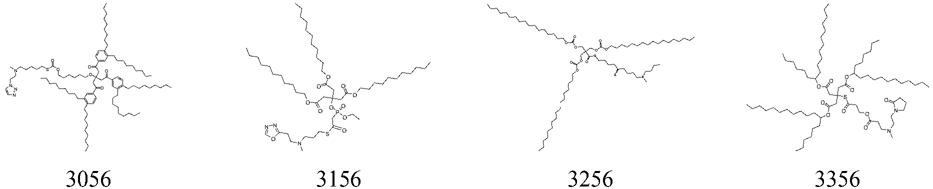

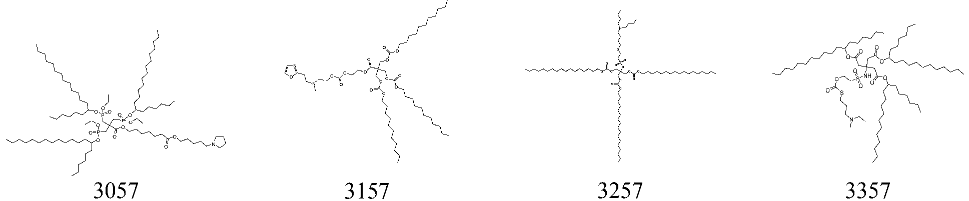

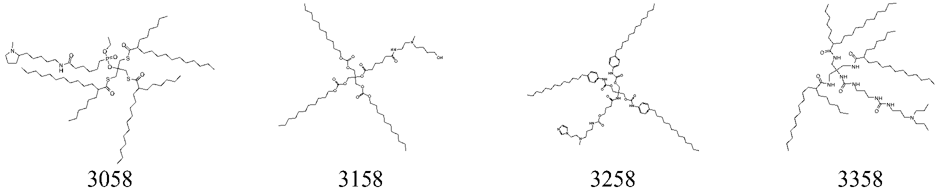

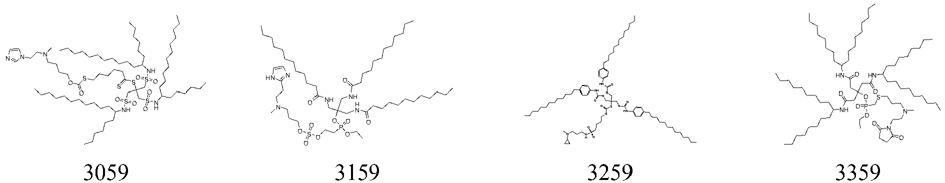

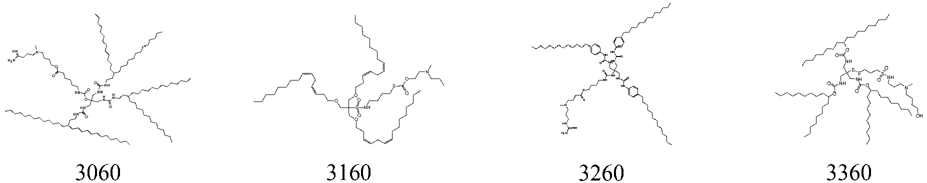

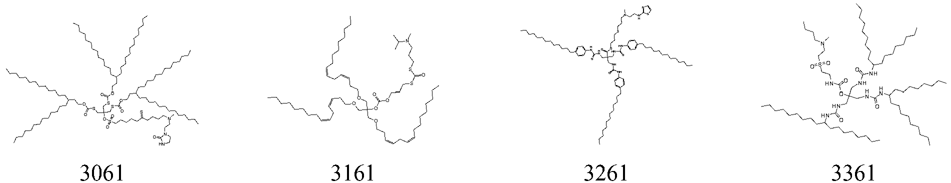

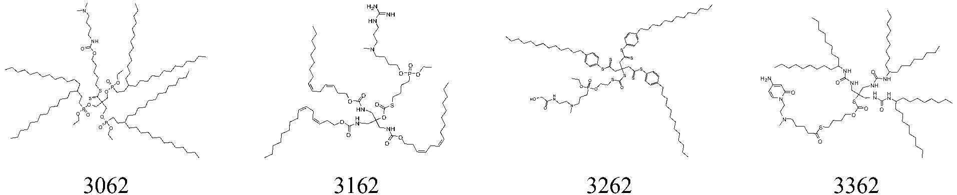

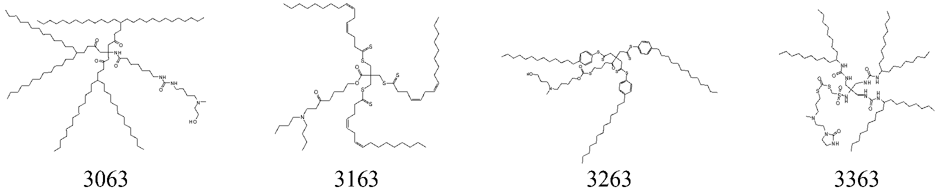

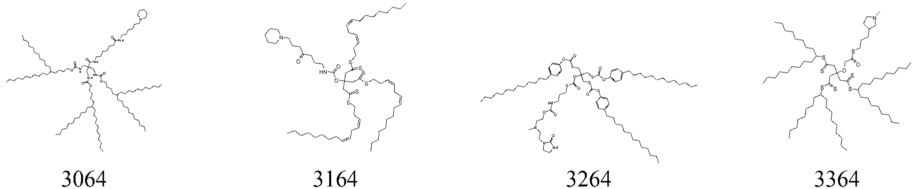

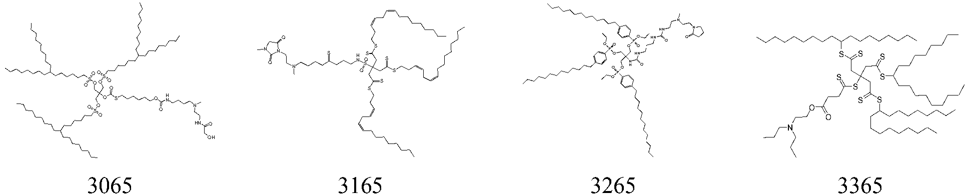

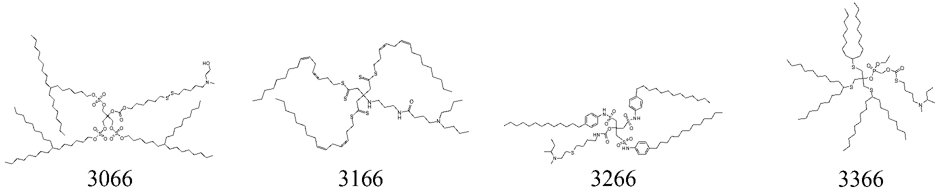

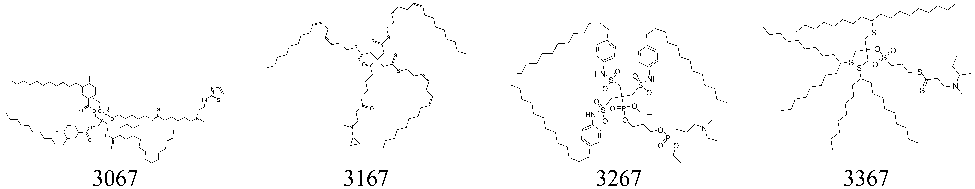

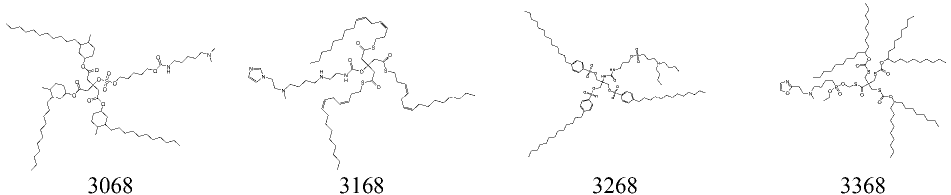

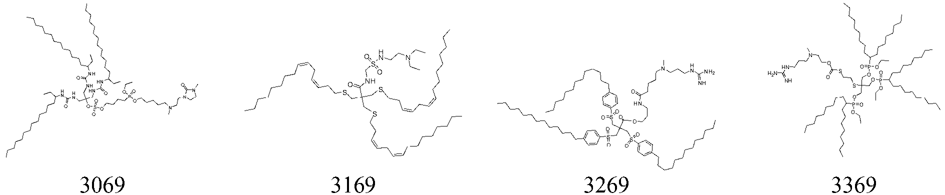

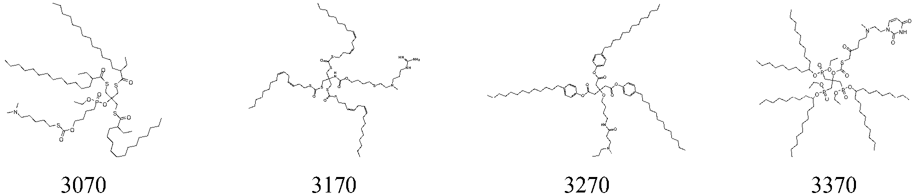

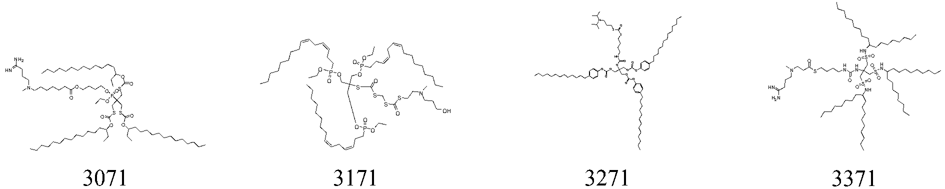

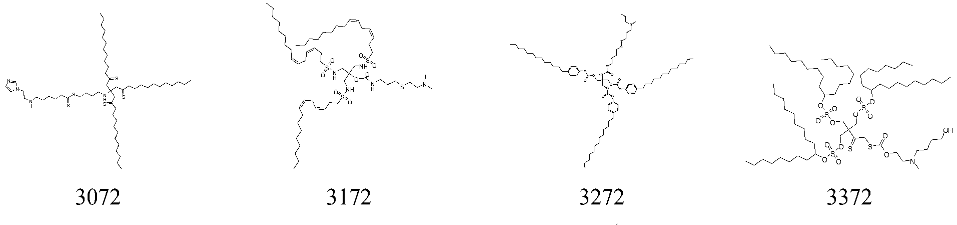

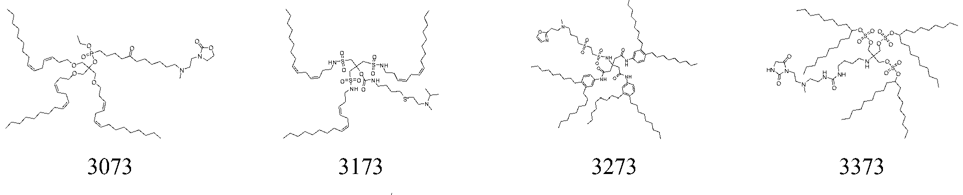

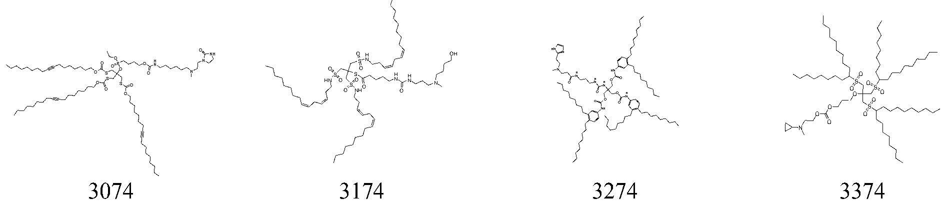

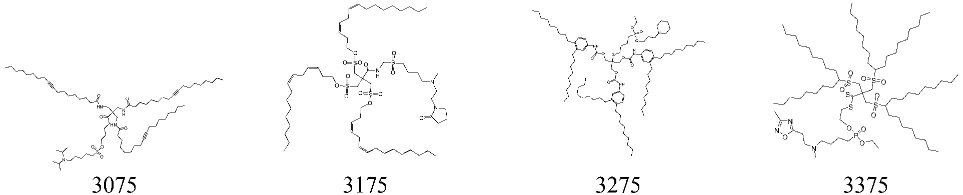

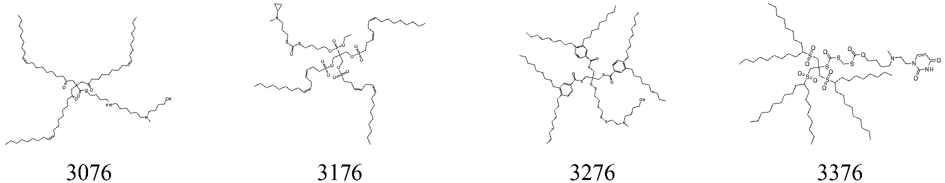

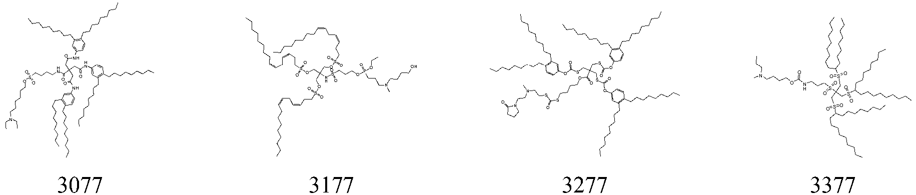

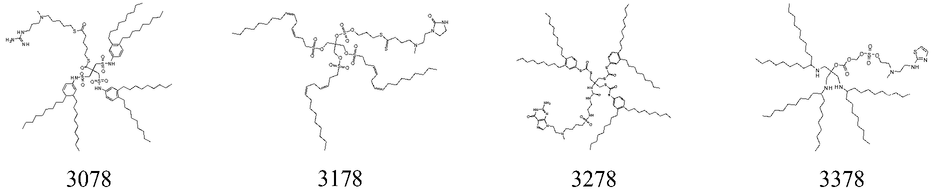

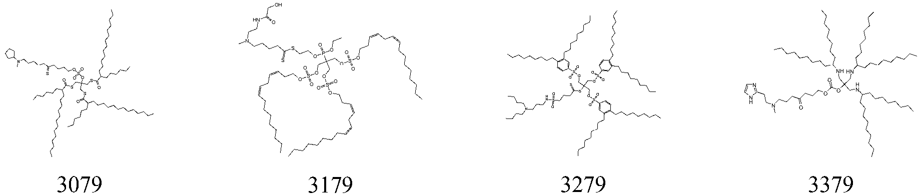

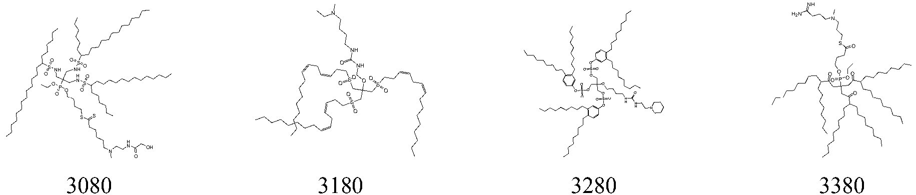

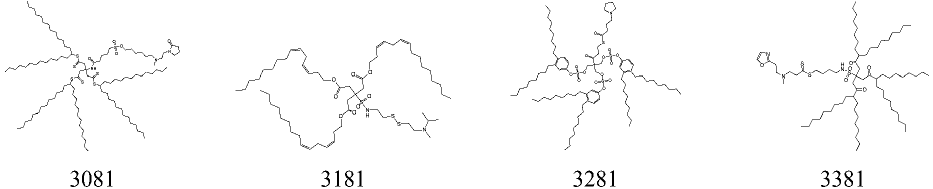

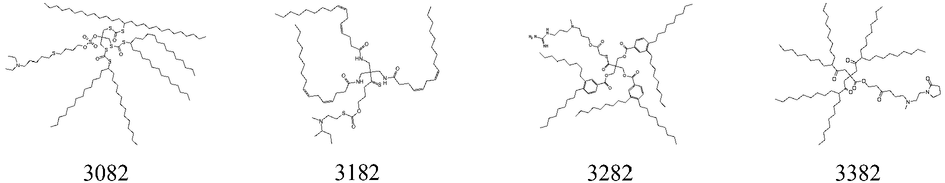

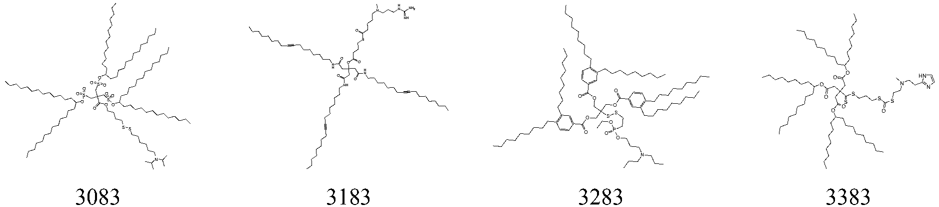

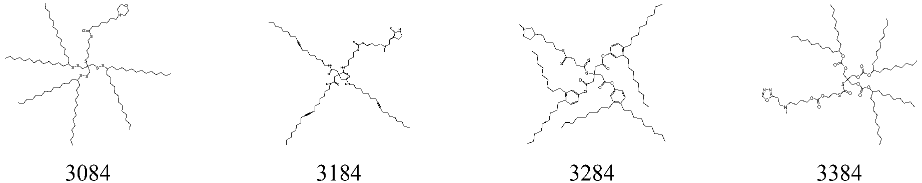

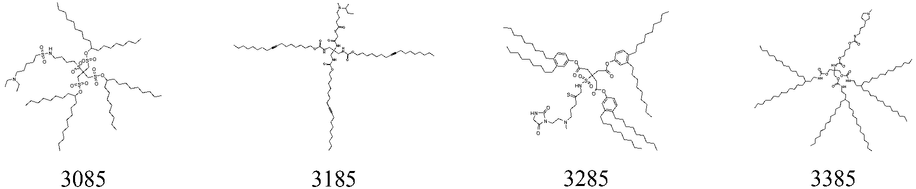

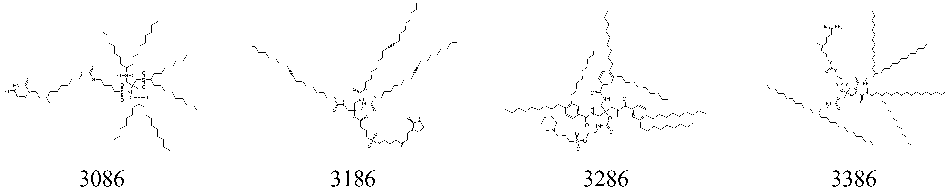

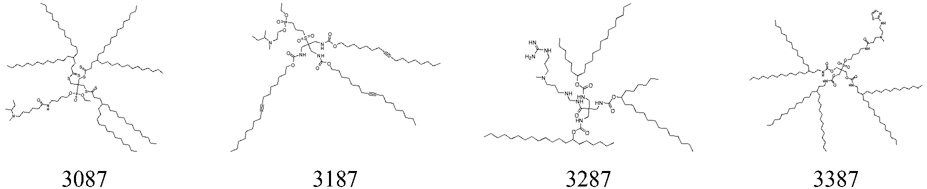

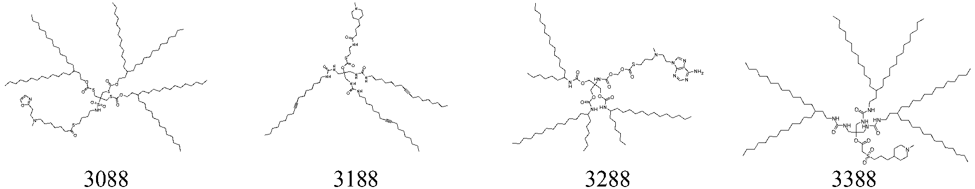

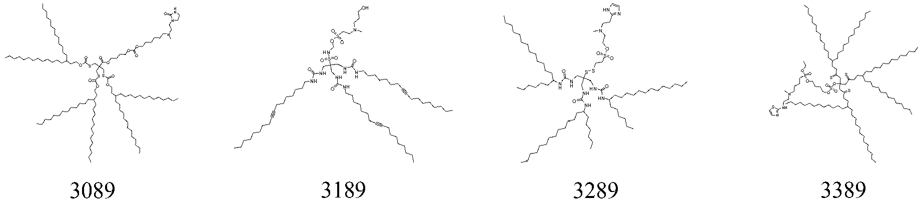

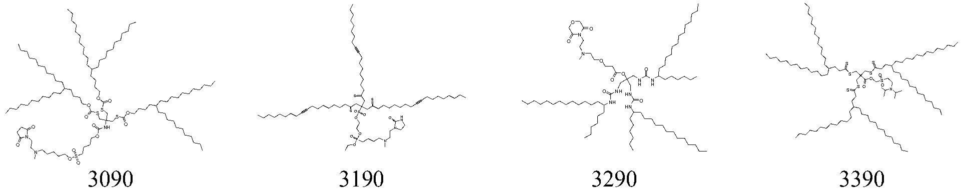

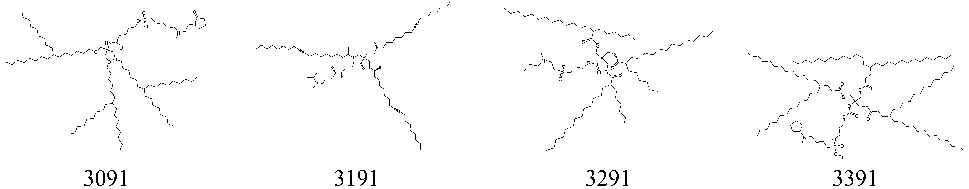

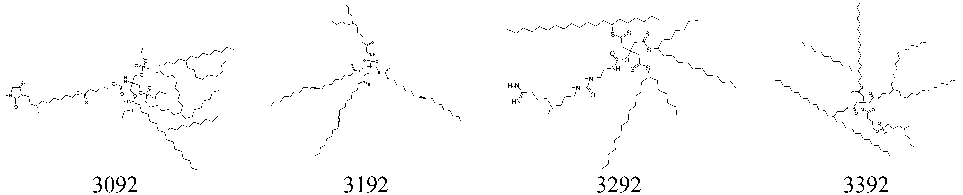

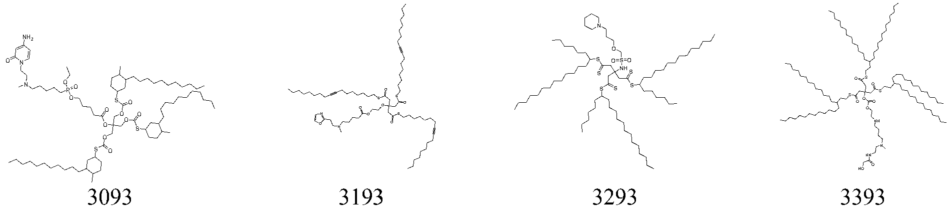

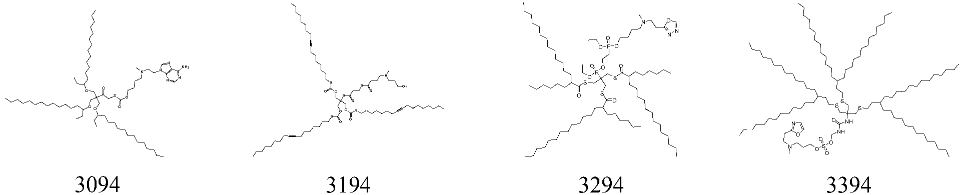

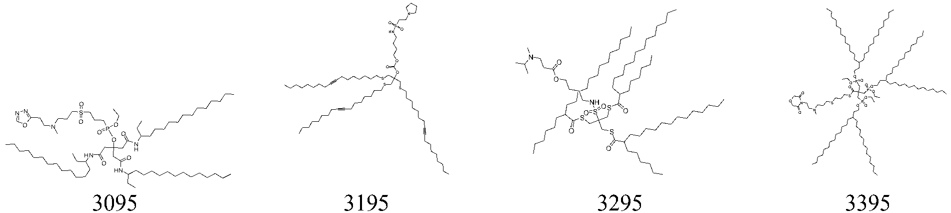

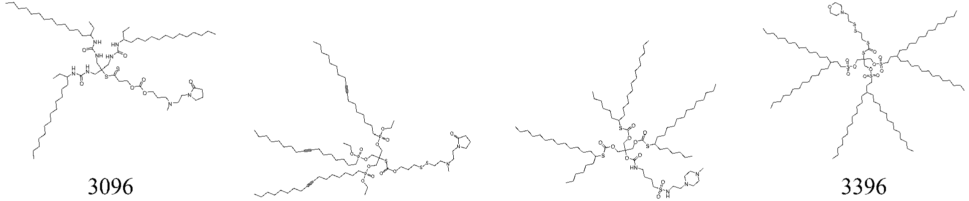

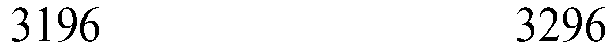

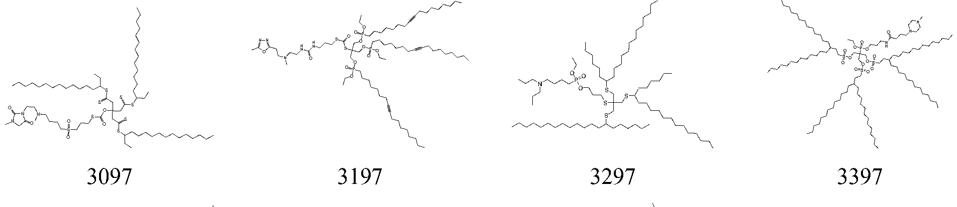

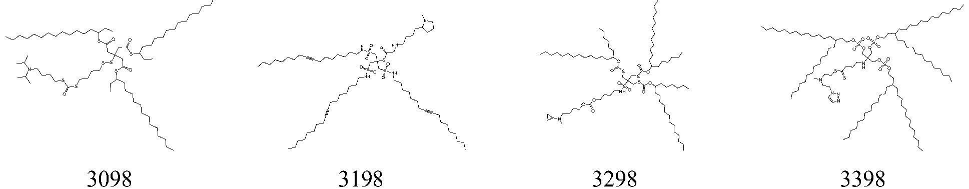

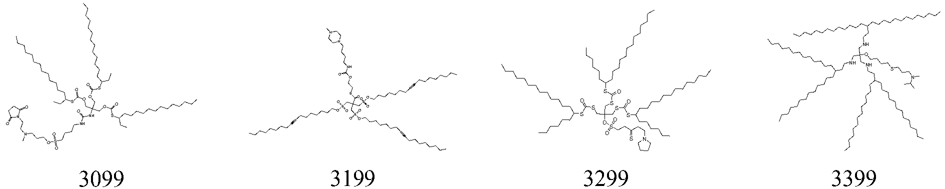

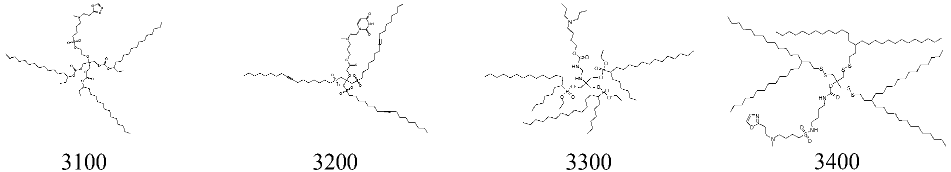

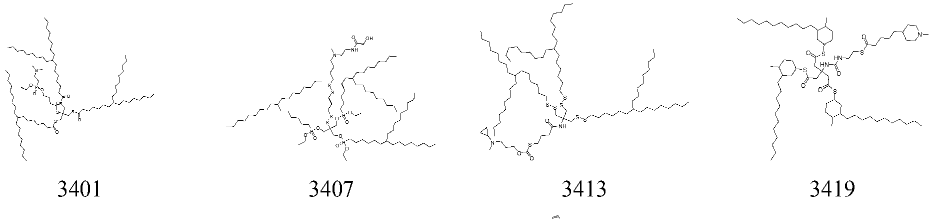

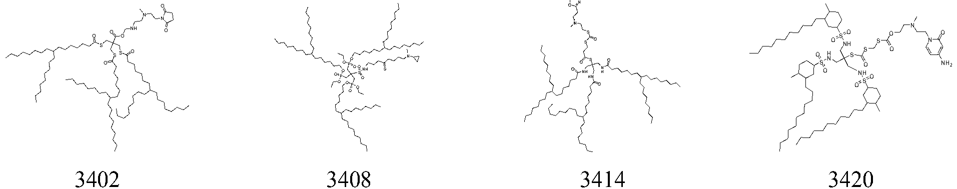

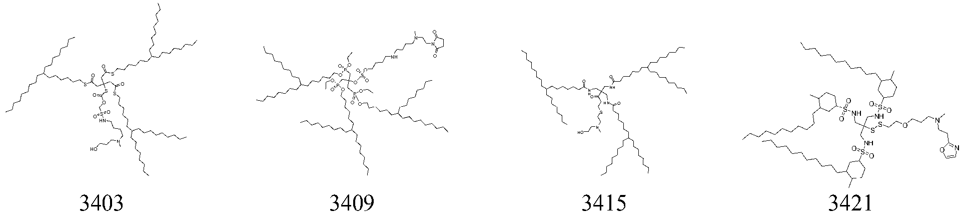

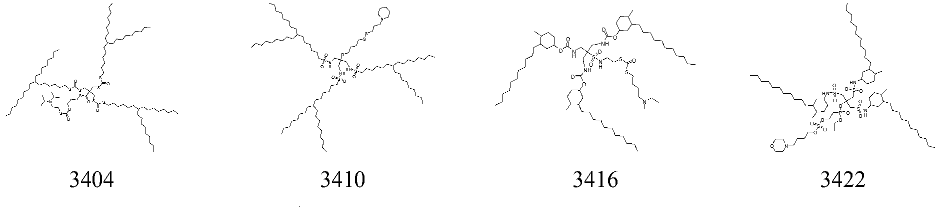

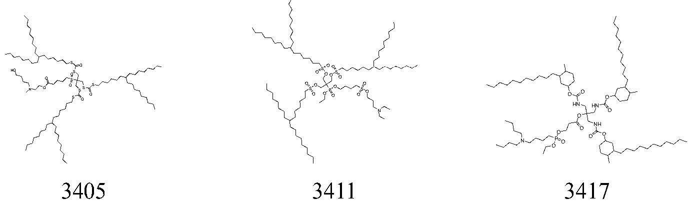

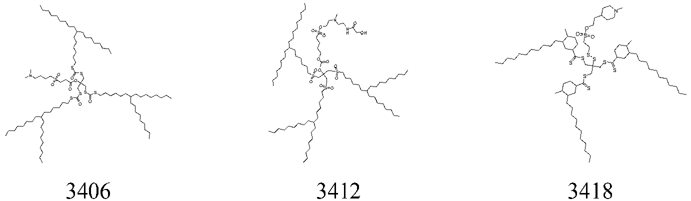

In one embodiment, a method for preparing the first ionizable lipid includes the step of reacting a compound of formula (II) with a compound of formula (III): 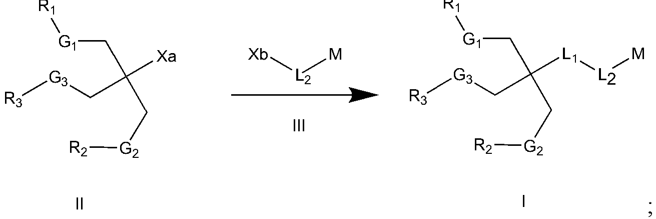 where Xa and Xb are groups containing a leaving group or a nucleophilic group, and Xa and Xb form L₁ via a nucleophilic reaction or a condensation reaction.

In one embodiment, a method for preparing the first ionizable lipid includes the step of reacting a compound of formula (IV) with a compound of formula (V): 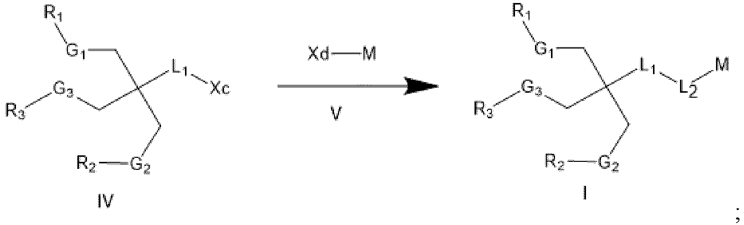 wherein Xc and Xd are groups containing a leaving group or a nucleophilic group, and Xc and Xd form L₂ via a nucleophilic reaction or a condensation reaction.

In one embodiment, a method for preparing the first ionizable lipid includes the step of performing a reaction on a compound of formula (VI): 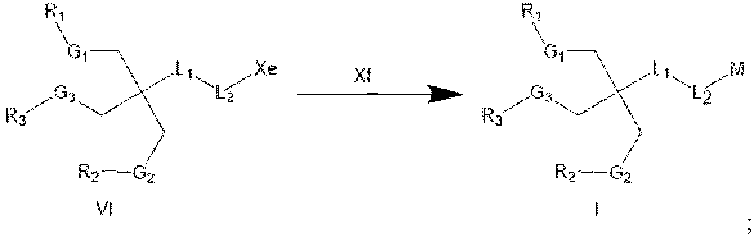 where Xe is a group containing a leaving group or a nucleophilic group, Xf is a compound containing a leaving group or a nucleophilic group, and Xe and Xf form M via a nucleophilic reaction or a condensation reaction.

In one embodiment, a method for preparing the first ionizable lipid includes the steps of reacting a compound of formula (VII) sequentially with a compound of formula (VIII), a compound of formula (IX), and a compound of formula (X): 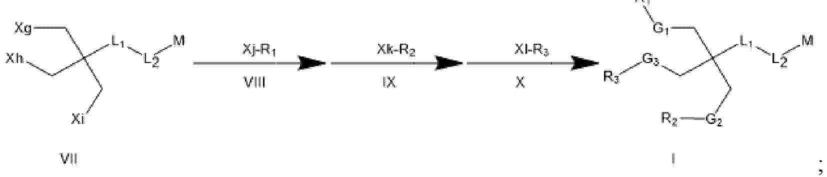 where Xg, Xh, Xi, Xj, Xk, and Xl are groups containing a leaving group or a nucleophilic group, and Xg and Xj form G₁ via a nucleophilic reaction or a condensation reaction, Xh and Kk form G₂ via a nucleophilic reaction or a condensation reaction, and Xi and Xl form G₃ via a nucleophilic reaction or a condensation reaction.

In one embodiment, a method for preparing the first ionizable lipid includes the step of reacting a compound of formula (XI) with a compound of formula (XII): 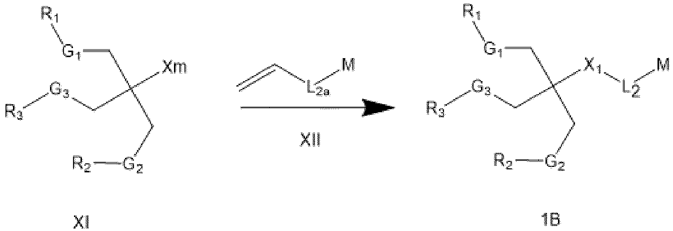 or 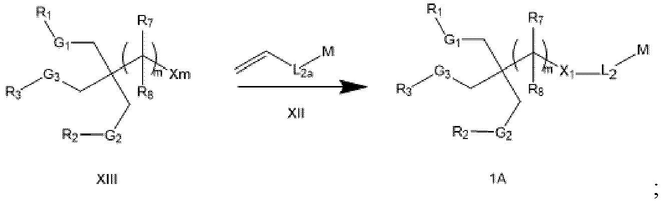 where Xm is a group containing a nucleophilic group, and Xm forms X₁-L₂ with C=C-L₂ₐ of the compound of formula (XII) via an addition reaction.

In one embodiment, R₁, R₂, and R₃ are each independently selected from the following group: 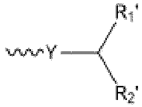
where the meanings of Y, R₁', and R₂' are as defined above; and
the method further includes the steps of forming tail chains R₁, R₂, and R₃:

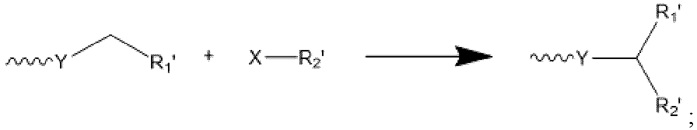

where X is a leaving group.

In one embodiment, the auxiliary lipid is a mixture of a phospholipid and a steroid or a derivative thereof.

In one embodiment, the phospholipid includes at least one of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesteryl-hemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphoethanolamine (DMPE), 1-stearoyl-2-oleoyl-phosphatidylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, and lysophosphatidylethanolamine (LPE).

In one embodiment, the steroid or the derivative thereof includes at least one of cholesterol, cholesterol stearate, coprostanol, sitosterol, ergosterol, campesterol, stigmasterol, brassicasterol, fucosterol, tomatine, ursolic acid, and α-tocopherol.

In one embodiment, in the lipid composition, the amount of the ionizable lipid is 5-90 mol% of the total lipids in the formulation, and the amount of the auxiliary lipid is 10-95 mol% of the total lipids in the formulation.

In one embodiment, the molar ratio of phospholipid to the steroid or the derivative thereof in the auxiliary lipid is (5-80):(5-80).

In one embodiment, the raw materials for the lipid composition further include a PEG-conjugated lipid in an amount of 0.1-15 mol% of the total lipids in the formulation.

In one embodiment, the PEG-conjugated lipid includes at least one of PEG-modified phosphatidylethanolamine, PEG-modified phosphatidic acid, PEG-modified ceramide, PEG-modified dialkylamine, PEG-modified diacylglycerol, and PEG-modified dialkylglycerol.

In one embodiment, the PEG-conjugated lipid includes at least one of PEG-distearyloxypropyl (PEG-DSA), PEG-c-DOMG, PEG-DPPC, polyethylene glycol dimethacrylate (PEG-DMA), 1,2-dimyristoyl-rac-glycero-3-methoxypolyethylene glycol (PEG-DMG), dipalmitoylglycerol-polyethylene glycol (DPG-PEG), 1,2-distearoyl-rac-glycero-3-methoxypolyethylene glycol (DSG-PEG), 2-[(polyethylene glycol)-2000]-N,N-ditetradecylacetamide (ALC-0159), dipalmitoylphosphatidylethanolamine-polyethylene glycol (DPPE-PEG), distearoylphosphatidylethanolamine-polyethylene glycol (PEG-DSPE), dilauroylphosphatidylethanolamine-polyethylene glycol (PEG-DLPE), and dimyristoylphosphatidylethanolamine-polyethylene glycol (PEG-DMPE) lipids..

In one embodiment, in the lipid composition, the amount of the ionizable lipid is 5-80 mol% of the total lipids in the formulation; the amount of the auxiliary lipid is 10-90 mol% of the total lipids in the formulation; and the amount of the PEG-conjugated lipid is 0.1-15 mol% of the total lipids in the formulation.

In one embodiment, the ionizable lipid further includes at least one of a second ionizable lipid and a third ionizable lipid;
the second ionizable lipid is a compound of formula (2), or a salt, a stereoisomer, or a tautomer thereof: 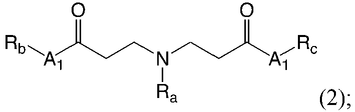
where A₁ is NH or O;
Rₐ is selected from a C₆-C₂₄ alkyl, a C₆-C₂₄ alkenyl, a C₆-C₂₄ cycloalkyl, a C₆-C₂₄ alcohol, and C₆-C₂₄ short-chain polyoxyethylene; the C₆-C₂₄ alkyl, C₆-C₂₄ alkenyl, C₆-C₂₄ cycloalkyl, C₆-C₂₄ alcohol, and C₆-C₂₄ short-chain polyoxyethylene with a linear or branched structure; R_{b} and R_{c} are each independently selected from a C₁-C₁₂ alkyl, a C₂-C₁₂ alkenyl, a C₁-C₁₂ alkynyl, a C₃-C₁₂ cycloalkyl, a C₆-C₁₂ aryl, a C₁-C₁₂ alkyl alcohol, a C₁-C₁₂ heterocyclyl, and an alkylamine;
the alkylamine is 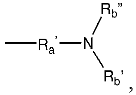 where the Rₐ' is a C₁-C₁₂ alkyl; the R_{b}' and R_{b}" are each independently selected from H, C₁-C₆ alkylamine, 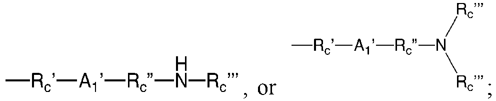 R_{c}" is selected from an unsubstituted or amino-substituted C₁-C₆ alkyl; and Rc‴ is H, 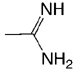 or -R_{c}'-A₁'-R_{c}"-NH₂;
provided that when A₁' is -CO-NH-, -NH-CO-, or -CO-O-, R_{c}' is a C₁-C₆ alkyl; and when A₁' is -CO-, R_{c}' is absent;
the third ionizable lipid is selected from at least one of the following compounds:

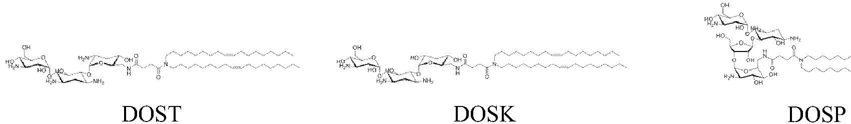

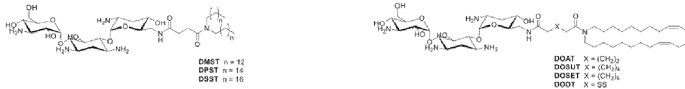

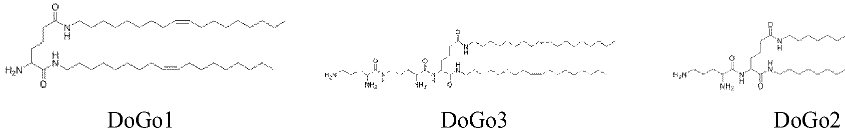

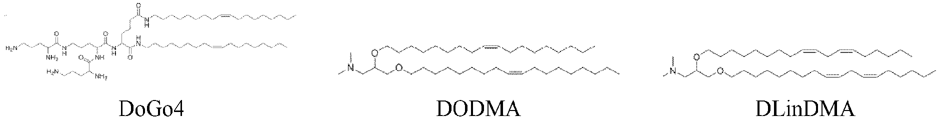

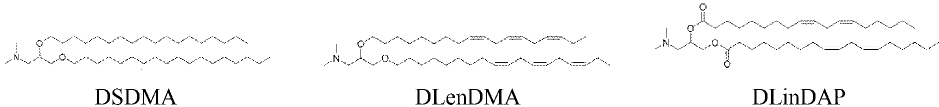

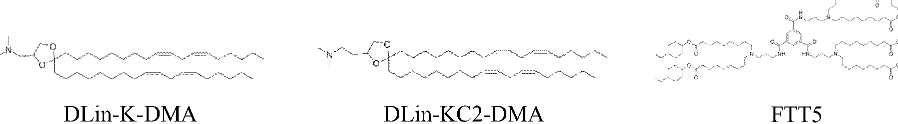

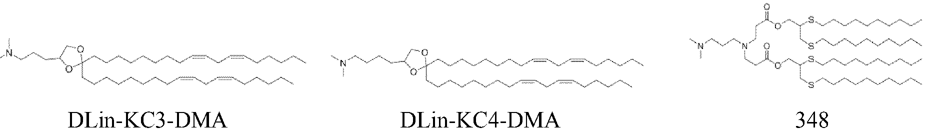

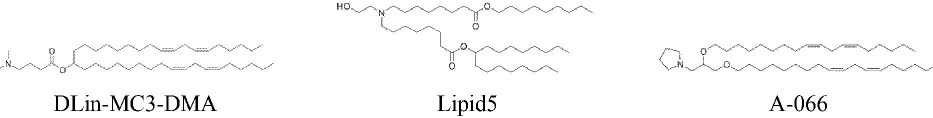

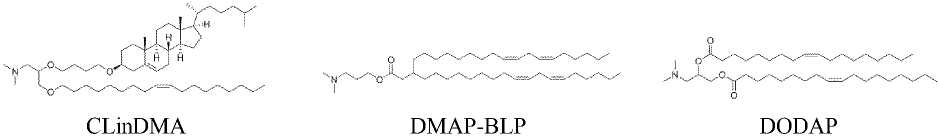

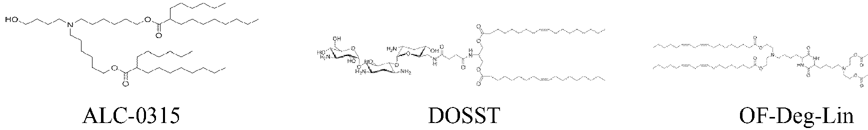

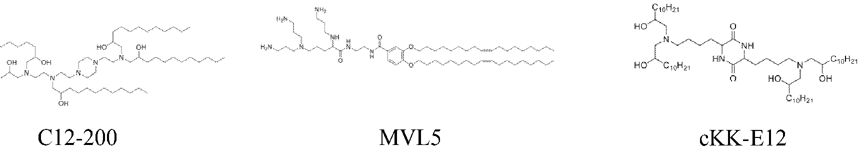

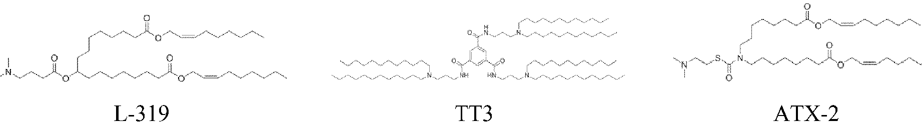

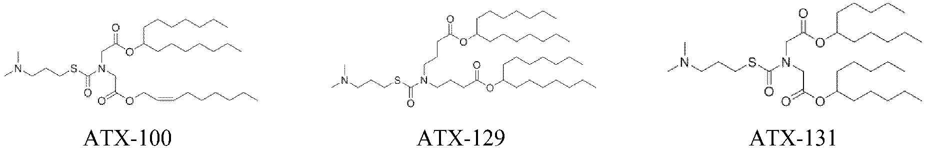

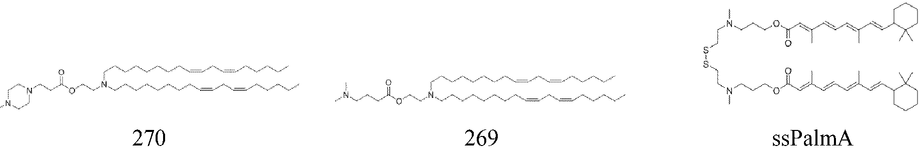

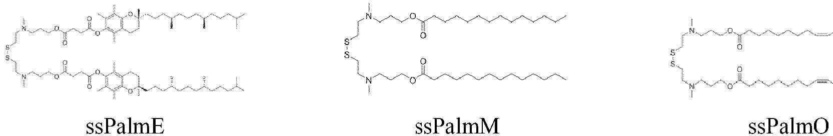

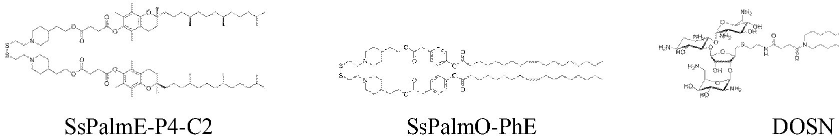

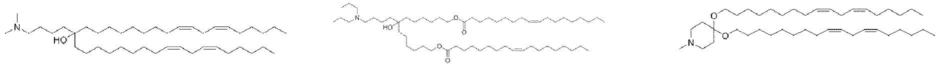

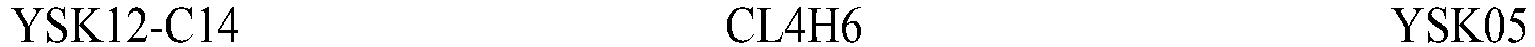

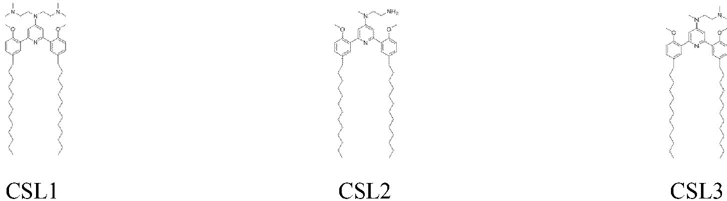

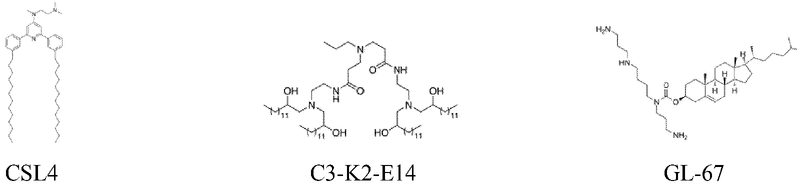

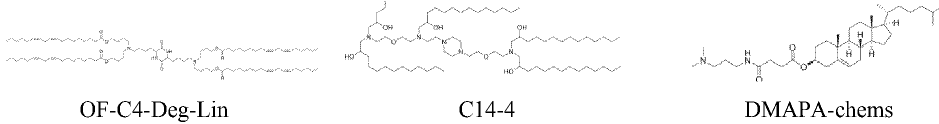

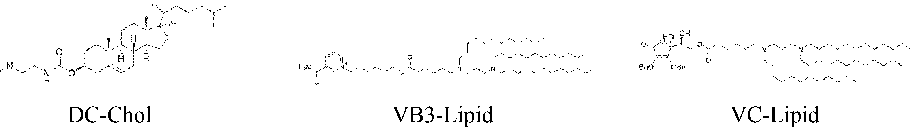

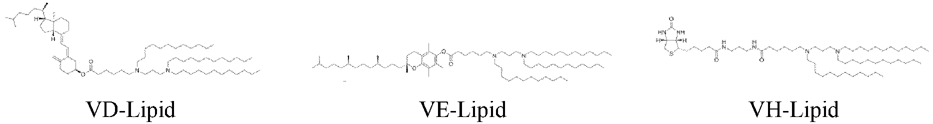

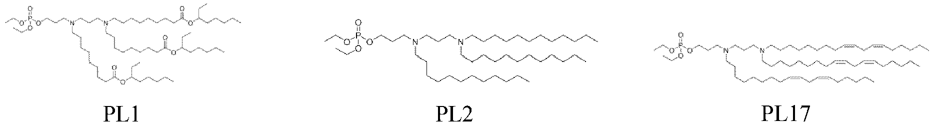

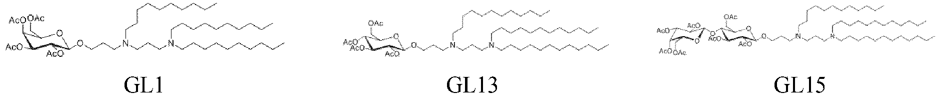

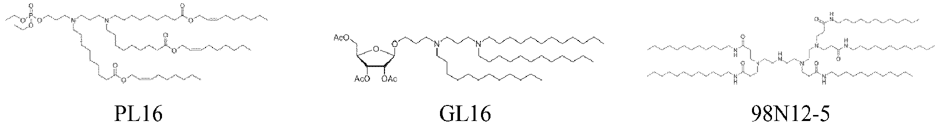

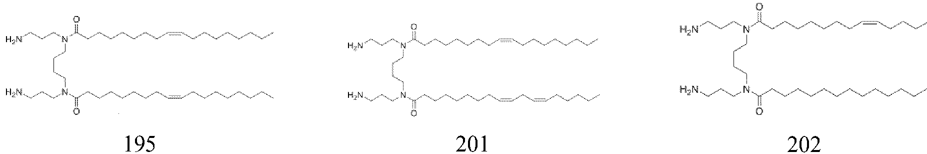

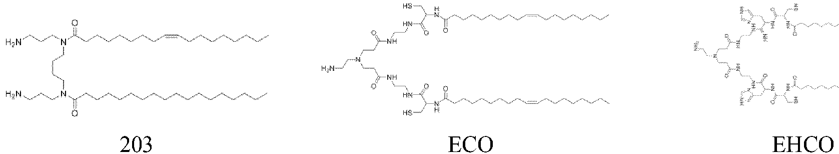

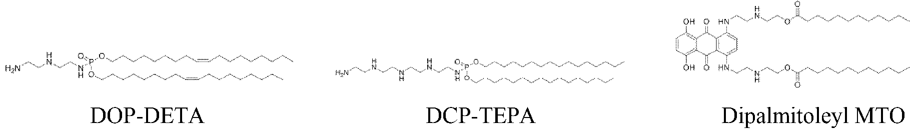

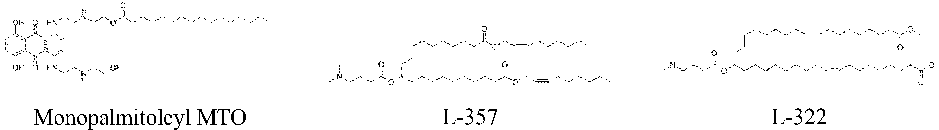

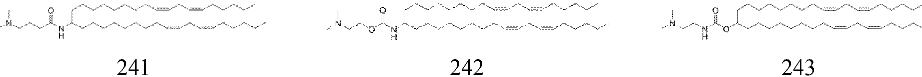

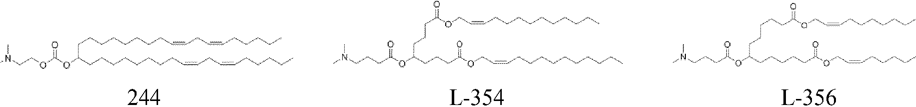

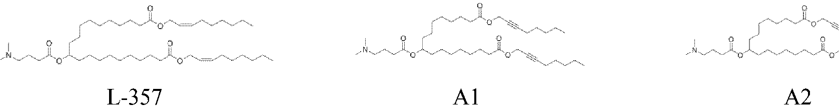

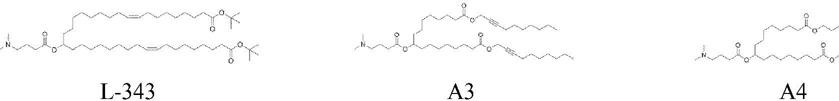

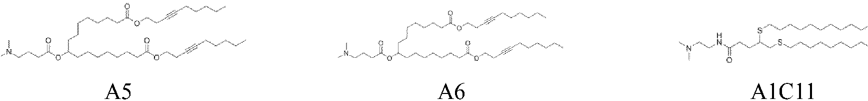

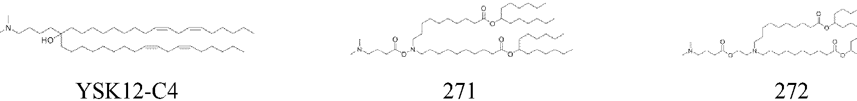

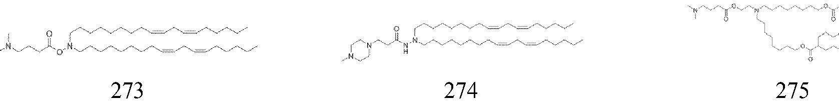

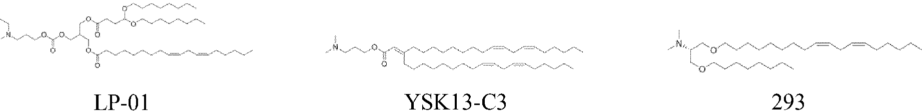

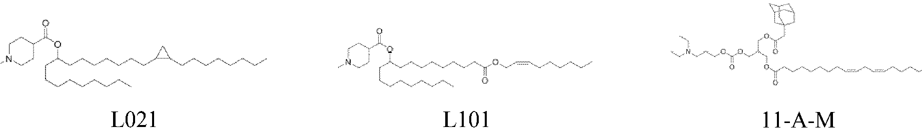

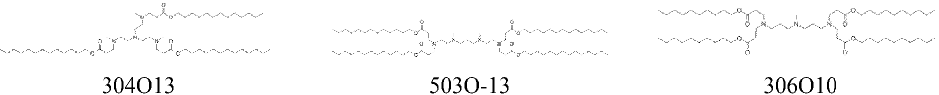

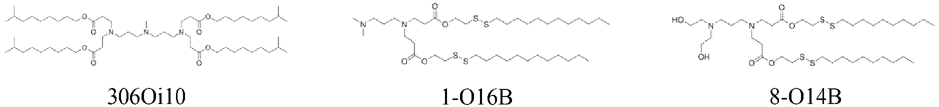

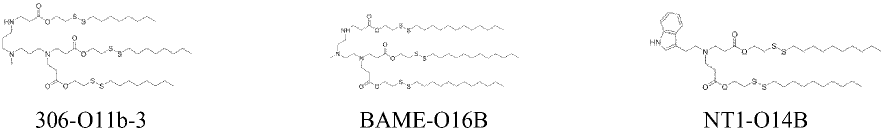

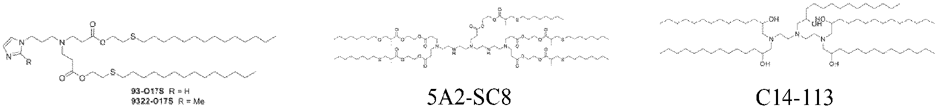

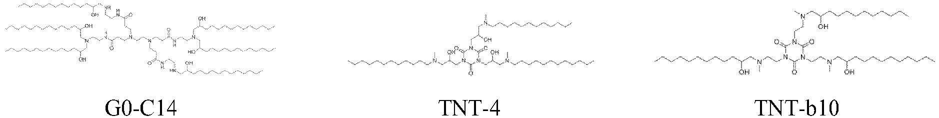

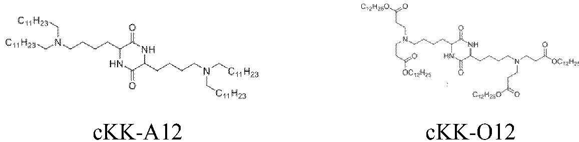

In one embodiment, R_{b} and R_{c} are each independently selected from a C₁-C₁₂ alkyl alcohol and alkylamine.

In one embodiment, the conditions are as follows: when Ra is a C₆-C₂₄ alkenyl, A₁ is NH, and Rb and Rc are each independently selected from a C₁-C₁₂ alkyl alcohol;
when Ra is a branched C₆-C₂₄ alkyl, A₁ is NH, and Rb and Rc are each independently selected from a C₁-C₁₂ alkyl alcohol and an alkylamine;
when Ra is a linear C₆-C₂₄ alkyl, A₁ is NH or O, and Rb and Rc are each independently selected from an alkylamine;
when Ra is a linear C₆-C₂₄ alkyl, A₁ is O, and Rb and Rc are each independently selected from a C₁-C₁₂ alkyl alcohol;
when Ra is a C₆-C₂₄ short-chain polyoxyethylene, A₁ is NH or O, and Rb and Rc are each independently selected from an alkylamine;
or when Ra is a C₆-C₂₄ alkyl alcohol, A₁ is NH or O, and Rb and Rc are each independently selected from an alkylamine.

In one embodiment, Ra is selected from the following compound structures:

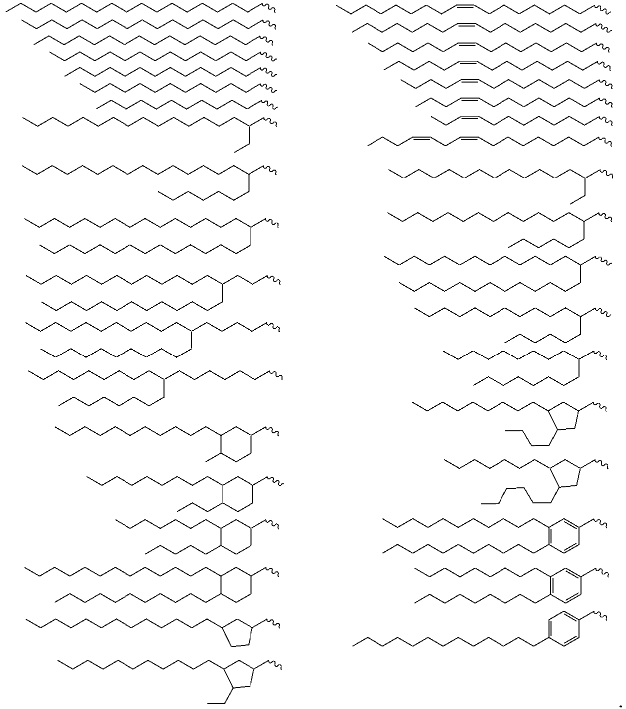

In one embodiment, Rb and Rc are selected from the following compound structures: 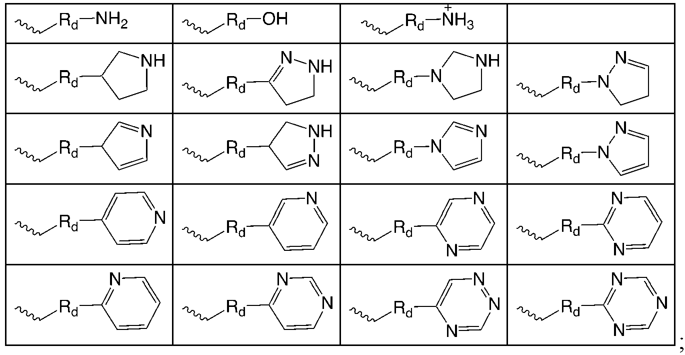 and Rd is selected from a C₁-C₆ alkane or a cycloalkane.

In one embodiment, the compound of formula (2) is selected from at least one of the following compounds:

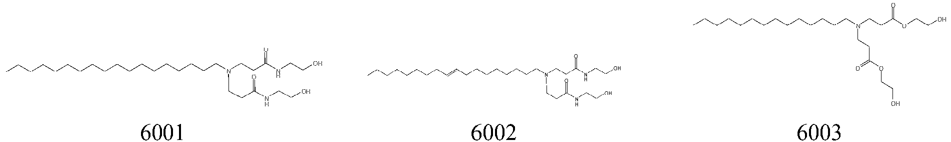

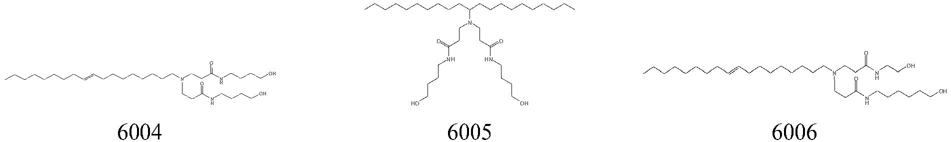

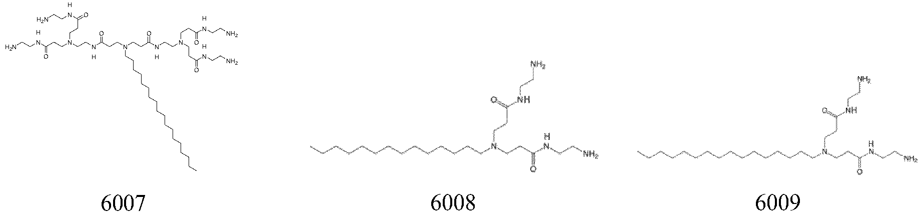

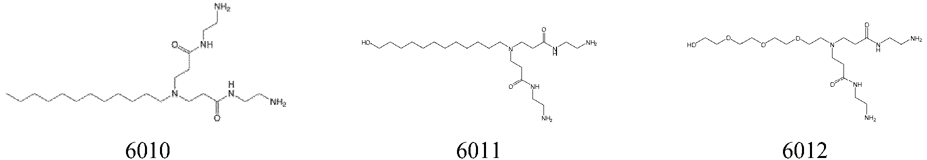

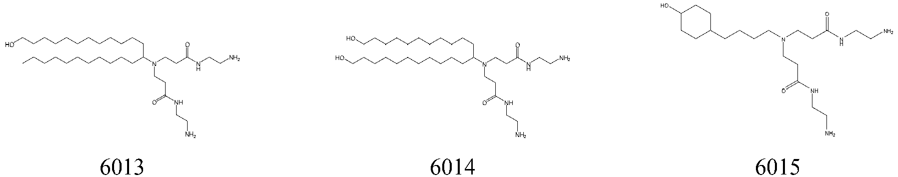

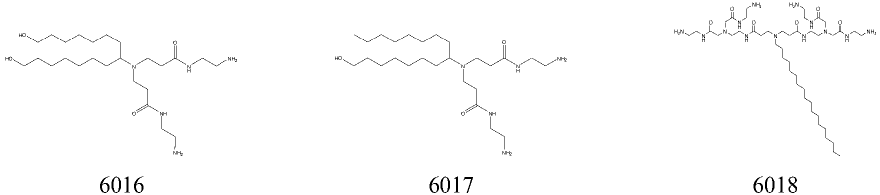

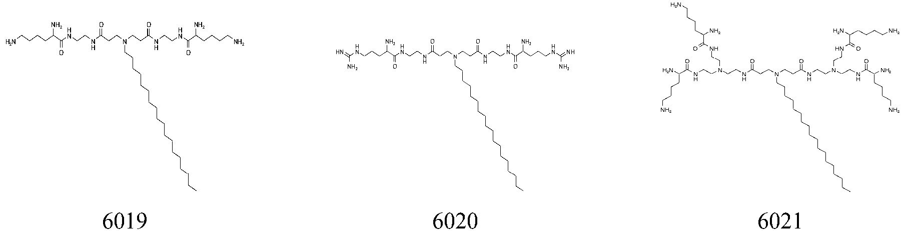

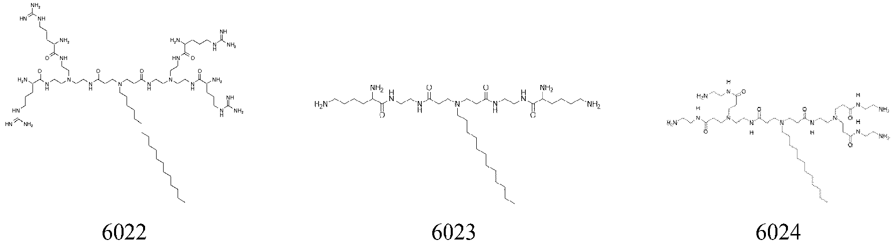

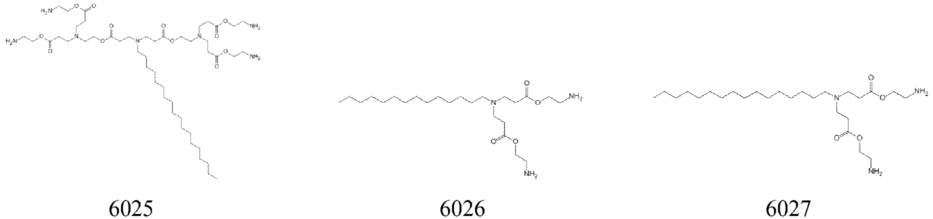

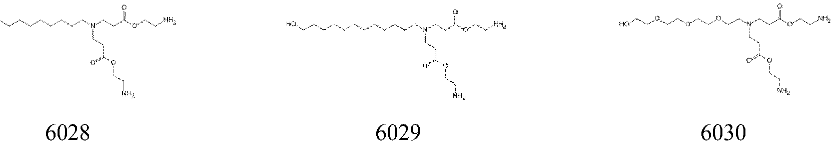

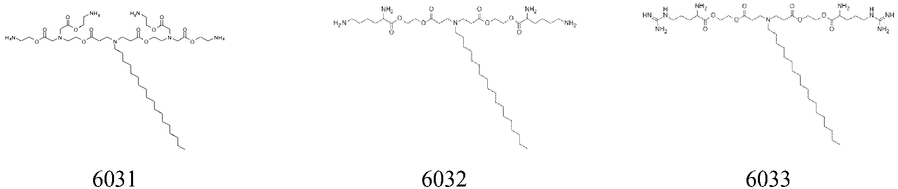

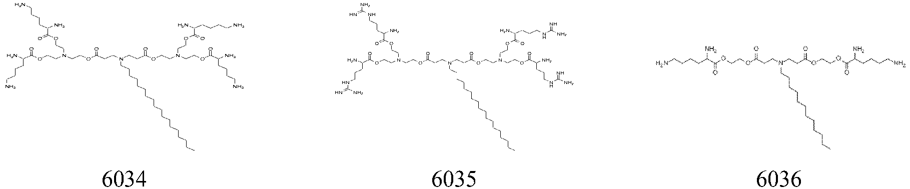

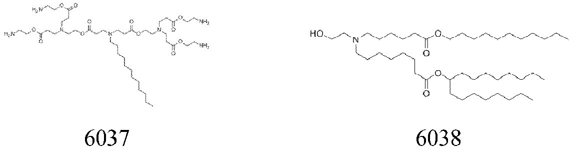

In one embodiment, a method for preparing the second ionizable lipid includes the following reaction step:
performing a reaction on Rₐ"-NH₂ ① with an α,β-unsaturated carbonyl compound ② to generate an ionizable lipid compound ③: 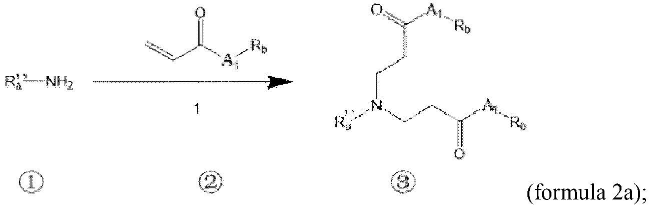
wherein Rₐ"-NH₂ is Rₐ-NH₂ or 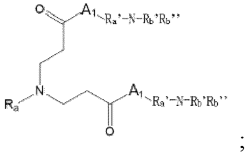
Rb' and Rb" are each H, or Rb' and Rb" are each C₁-C₆ amines, or R_{b}' and R_{b}" are each -R_{c}'-A₁'-R_{c}"-NH₂.

In one embodiment, a method for preparing the second ionizable lipid includes:
1) performing a reaction on Ra"-NH₂ ① with an α,β-unsaturated carbonyl compound ④ to generate a compound ⑤; and
2) performing a reaction on the compound ⑤ with a nucleophile ⑥ to generate an ionizable lipid compound ③; 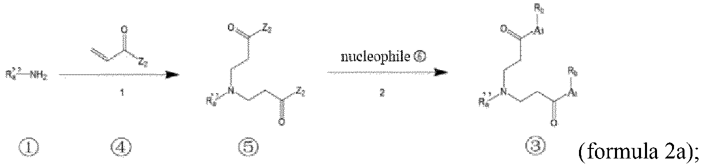 where the nucleophile ⑥ is R_{b}-NH₂ or R_{b}-OH; Ra"-NH₂ is selected from Ra-NH₂ or 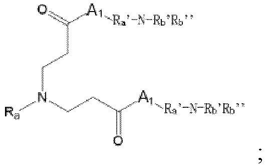 Rb' and Rb" are each H, or Rb' and Rb" are each a C₁-C₆ amine, or Rb' and Rb" are each -R_{c}'-A₁'-R_{c}"-NH₂; and Z₂ is a leaving group, Z₂ is reacted with NH₂ to obtain A₁.

In one embodiment, a method for preparing the second ionizable lipid includes:
1) performing a reaction on Ra"-NH₂ ① with an α,β-unsaturated carbonyl compound ② to generate a compound ⑦;
2) performing a reaction on the compound ⑦ with an α,β-unsaturated carbonyl compound ⑧ to generate a compound ⑨; and
3) performing a reaction on the compound ⑨ with a nucleophile ⑩ to generate an ionizable lipid compound ⑪;

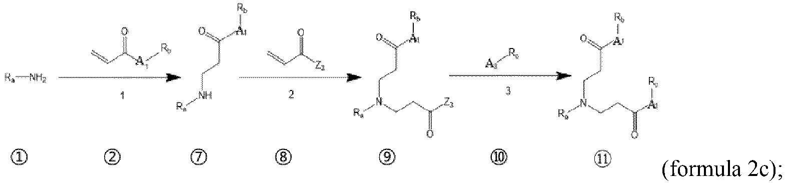

where Ra"-NH₂ is Ra-NH₂, Z₃ is a leaving group, and A₃ reacts with Z₃ to obtain A₁.

In one embodiment, in formulas 2a-2c, at least one nucleophile undergoes a Michael addition reaction with the β-carbon atom of at least one α,β-unsaturated carbonyl compound to generate the double-branched ionizable lipid compound containing a carbon-carbon bond, carbon-oxygen bond, carbon-nitrogen bond, carbon-sulfur bond, or carbon-selenium bond.

In one embodiment, formulas 2a-2b further include a step of performing iterative reactions according to step 1 of formula 2a or steps 1-2 of formula 2b using the compound of formula (2) with a terminal amino group (obtained via the reactions of formulas 2a-2b) as a raw material; and
the compound of formula (2) with a terminal amino group is 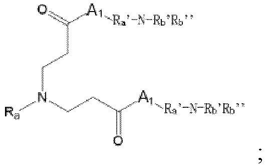
where Rb' and Rb" are each H, or Rb' and Rb" are each a C₁-C₆ amine, or R_{b}' and R_{b}" are each -R_{c}'-A₁'-R_{c}"-NH₂.

In one embodiment, the reaction of formulas 2a-2c further includes a step of reacting a compound of formula (2) with a terminal amino group (obtained via the reaction of formulas 2a-2c) as a starting material with 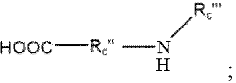
the compound of formula (2) with a terminal amino group is 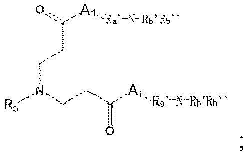
where Rb' and Rb" are each H, or Rb' and Rb" are each a C₁-C₆ amine, or Rb' and Rb" are each -R_{c}'-A₁'-R_{c}"-NH₂.

The leaving group mentioned above refers to the moiety that departs during a nucleophilic reaction or condensation reaction, including but not limited to: H, OH, H₂O, halogens (e.g., F, Cl, Br, and I), cyanate anions, inorganic acids (e.g., nitric acid, sulfuric acid, phosphoric acid), carboxylic acids (e.g., acetic acid, trifluoroacetic acid, benzoic acid, and the like), sulfonic acids (e.g., methanesulfonic acid, trifluoromethanesulfonic acid, benzenesulfonic acid, p-toluenesulfonic acid, p-nitrobenzenesulfonic acid, etc.), carbon dioxide (CO₂), nitrogen gas (N₂), imidazole, alkoxy (R-O-), amino (-NHR, where R is an H-removed alkyl or aryl), phenoxy, tertiary carbocations (e.g., tert-butyl cation), carbocations stabilized by unsaturated systems or heteroatoms, or the various protecting groups mentioned above.

The nucleophilic group mentioned above refers to a molecule or ion capable of donating an electron pair to form a new chemical bond during a chemical reaction; common nucleophilic groups include: hydroxide (HO-), ammonia (NH₃), hydroxylamine (NH₂OH), hydrazine (NH₂-NH₂), substituted hydrazines, nucleophilic halogens (e.g., Cl-, Br-, or I-), hydride anion (H-), azide anion (N₃⁻), cyanate anion (CN-), alcohol or alkoxy anions (e.g., alcohols with the hydroxyl hydrogen removed), amino (including primary amines, secondary amines, and tertiary amines) or amine anions, carbanions (e.g., carbanions in organometallic reagents such as Grignard reagents, organolithium reagents, Gilman reagents, and the like), thiol or thiolate anions, thioethers, enols or enolate anions, enol ethers, enamines, carboxylic acids or carboxylate anions, alkyl or aryl phosphines (e.g., triphenylphosphine), aromatic heterocycles with a lone pair of electrons (e.g., pyridine), and the like.

In one embodiment, the raw materials used in the reaction process also contain protecting groups, and the reaction steps include protection and/or deprotection steps.

Those of ordinary skill in the art will recognize that it may be necessary to use protecting groups to protect certain groups from being affected by reaction conditions. Protecting groups can also be used to distinguish similar functional groups within a molecule. A list of protecting groups, as well as methods for introducing and removing them, can be found in Wuts,P. G. M.,Greene,T. W.,Greene's Protective Groups in Organic Synthesis,4th Edition,John Wiley&Sons: New Jersey,2007.

Protecting groups include, but are not limited to: hydroxyl protecting groups, amino or amine protecting groups, carboxyl protecting groups, aldehyde or ketone protecting groups, and sulfhydryl protecting groups, as well as protecting groups for any combination of such functional groups (e.g., hydroxyl, amino/amine, carboxyl, aldehyde/ketone, sulfhydryl).

In one embodiment, the synthetic route for the first ionizable lipid is as follows: 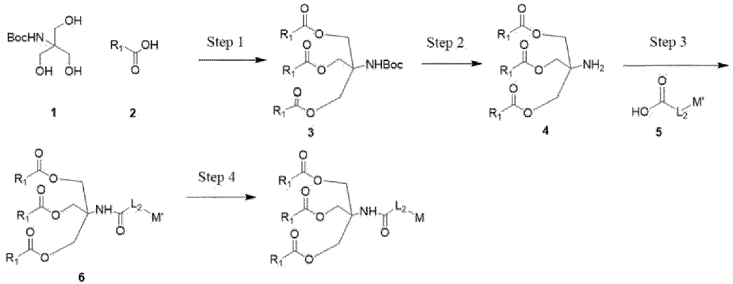 where M' is M or M containing protecting groups.

In one embodiment, the synthetic route for the first ionizable lipid is as follows: 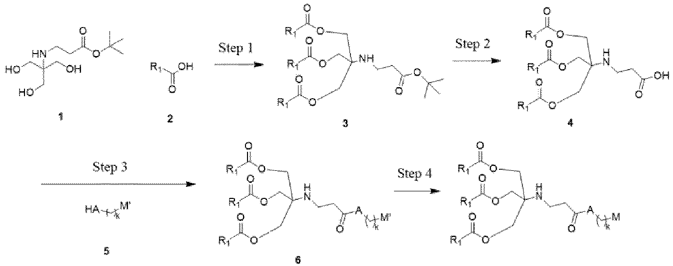 where M' is M or M containing protecting groups; and A is O, NH or S.

In one embodiment, the synthetic route for the first ionizable lipid is as follows: 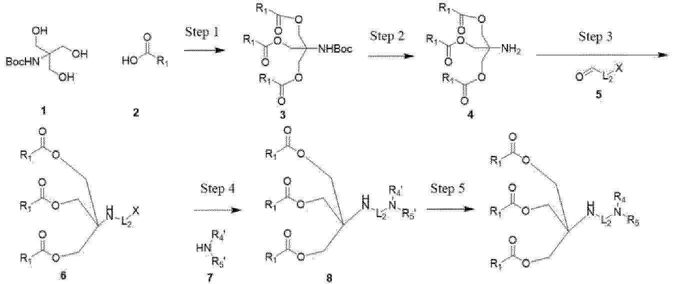 where X is halogen; R₄' is R₄ or R₄ containing protecting groups; and R₅' is R₅ or R₅ containing protecting groups.

In one embodiment, the synthetic route for the first ionizable lipid is as follows: 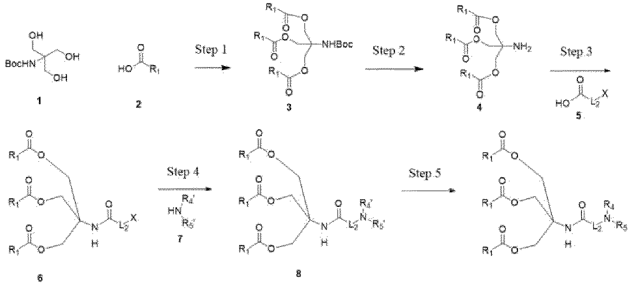 where X is halogen; R₄' is R₄ or R₄ containing protecting groups; and R₅' is R₅ or R₅ containing protecting groups.

In one embodiment, the synthetic route for the first ionizable lipid is as follows: 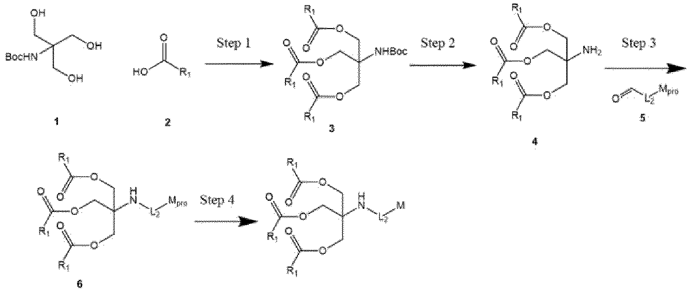 where Mpro is M or M containing protecting groups.

In a second aspect, the present application provides a preparation method of the lipid composition, including the following steps:
1) dissolving the lipid compound(s) in an organic solvent to form an organic phase;
2) using an aqueous solvent as an aqueous phase;
3) mixing the organic phase and the aqueous phase, and performing ultrafiltration to obtain the lipid composition.

In one embodiment, after dissolving the lipid compound in organic phase in step 1), the total lipid concentration is 0.1 mg/mL-30 mg/mL; and the volume ratio of the organic phase to the aqueous phase in step 3) is 1:(1-6), more preferably 1:(2-5). There are many types of organic solvents, which can be classified into 10 major categories based on their chemical structures: (1) aromatic hydrocarbons: benzene, toluene, xylene, and the like; (2) aliphatic hydrocarbons: pentane, hexane, octane, and the like; (3) alicyclic hydrocarbons: cyclohexane, cyclohexanone, tolylcyclohexanone, and the like; (4) halogenated hydrocarbons: chlorobenzene, dichlorobenzene, dichloromethane, and the like; (5) alcohols: methanol, ethanol, isopropanol, and the like; (6) ethers: ethyl ether, propylene oxide, and the like; (7) esters: methyl acetate, ethyl acetate, propyl acetate, and the like; (8) ketones: acetone, methyl butyl ketone, methyl isobutyl ketone, and the like; (9) glycol derivatives: ethylene glycol monomethyl ether, ethylene glycol monoethyl ether, ethylene glycol monobutyl ether, and the like; (10) others: acetonitrile, pyridine, phenol, and the like.

The aqueous solvent in the present application is a solution using water as the solvent, including but not limited to water, a sugar solution, a salt solution, and the like.

In one embodiment, the aqueous solvent includes at least one of water, a sucrose solution, and a buffer salt solution.

In one embodiment, the buffer salt solution has a pH value of 1-10, and the buffer salt concentration is 1-200 mM.

In one embodiment, the organic solvent includes an alcohol, preferably ethanol.

In a third aspect, the present application provides a method for delivering a pharmaceutically active ingredient, where the method uses the aforementioned lipid composition as a carrier; and the pharmaceutically active ingredient includes at least one of a macromolecular substance and a small-molecule substance.

In one embodiment, the macromolecular substance includes a nucleic acid, a protein, a polypeptide, a recombinant human insulin, an interferon, a human growth hormone, an insulin analog, and staphylokinase.

In a fourth aspect, the present application provides use of the aforementioned lipid composition in preparation of a carrier for delivering a pharmaceutically active ingredient.

Compared to the prior art, the present application has the following beneficial effects:
1. The present application provides a lipid composition including degradable components such as ionizable lipids, auxiliary lipids, and PEG-conjugated lipids, while not containing components having a preventive or therapeutic effect.
2. The lipid composition provided in the present application has nanoparticle characteristics, with a particle size of 2-400 nm and a PDI<0.5, and the lipid composition has good stability.
3. The preparation process for the lipid composition in the present application is simple and highly reproducible; effective encapsulation of the active ingredient can be achieved through simple mixing, and no organic reagents are required during binding with nucleic acids, small molecules, or macromolecules, resulting in higher safety.
4. The lipid composition provided in the present application has a wide range of applications, and the use method is simple and highly reproducible.
5. The lipid composition provided in the present application can deliver various active ingredients, such as nucleic acids, macromolecular substances, and small molecules, thereby meeting the delivery needs of different active ingredients; as demonstrated in the examples, it can effectively encapsulate nucleic acid substances such as mRNA, pDNA, and siRNA.

### DETAILED DESCRIPTION OF THE EMBODIMENTS

The synthetic process of the present application is compatible with multiple functional groups; therefore, a wide range of substituted starting materials can be used. These processes generally provide the desired final compound at or near the end of the process, although in some instances, further conversion of the compound into a pharmaceutically acceptable salt may be required. The compounds of the present application can be prepared in various ways using commercially available starting materials, compounds known in the literature, or readily prepared intermediates, employing standard synthetic methods and procedures known to those skilled in the art or evident from the teachings herein. Standard synthetic methods and procedures for preparing organic molecules, as well as functional group transformations and manipulations, can be found in relevant scientific literature or standard textbooks in the field. The following descriptions of synthetic methods are intended to illustrate, rather than limit, the general procedures used to prepare the compounds of the present application.

The compounds in the present application having the formulas described herein can be prepared from commercially available starting materials or starting materials prepared via literature methods, following the processes illustrated in the corresponding general synthetic routes. Variables in each general synthetic route (e.g., R1, R2, R3, and the like) are as defined herein. A person of ordinary skill in the art will appreciate that the order of certain steps, such as the introduction and removal of protecting groups, in the reaction procedures and synthetic schemes described herein may be varied.

The reaction schemes described in the present application may yield various stereoisomers. When a specific stereoisomer is not indicated, the description is understood to encompass all possible stereoisomers produced by the reaction. One of ordinary skill in the art will recognize that the reactions can be optimized to preferentially yield one isomer, or that new schemes can be designed to produce a single isomer. If a mixture is obtained, isomers can be separated using techniques such as preparative thin-layer chromatography, preparative HPLC, preparative chiral HPLC, or preparative SFC.

### (1) Preparation Example 1: Synthesis of a first ionizable lipid

The general synthetic routes for the first ionizable lipid are as shown in general synthetic routes 1 to 5.

### General Synthetic Route 1

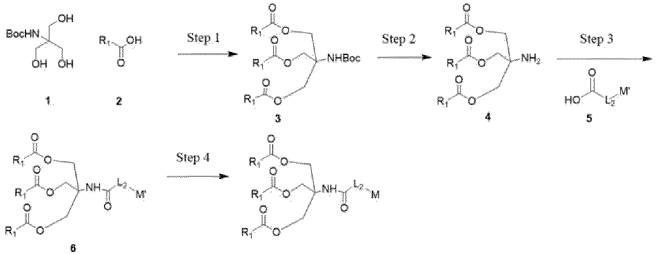

where M' is M or M containing a protecting group.

As illustrated in the general synthetic route 1 above, Boc-aminotris(hydroxymethyl)methane underwent a condensation reaction with an acid (the compound 2) to generate the compound 3. Step 1 might be performed in an organic solvent (e.g., dichloromethane (DCM)) in the presence of reagents such as 1-ethyl-(3-dimethylaminopropyl)carbodiimide hydrochloride (EDCl) and 4-dimethylaminopyridine (DMAP). Step 1 might be carried out at room temperature for 24 hours.

Subsequently, the Boc protecting group was removed from the compound 3 to generate the compound 4. Step 2 might be performed in an organic solvent (e.g., DCM) under catalysis by an acid (such as trifluoroacetic acid).

Next, the compound 4 underwent a condensation reaction with the compound 5 to obtain the compound 6. Step 3 might be performed in an organic solvent (e.g., DCM or DMF) under catalysis by EDCl and DMAP, or dicyclohexylcarbodiimide (DCC).

If the M' group of the compound 6 contained the aforementioned protecting group, the protecting group was removed to obtain the target lipid compound. Step 4 was carried out under reaction conditions suitable for the removal of the selected protecting group.

### General synthetic route 2

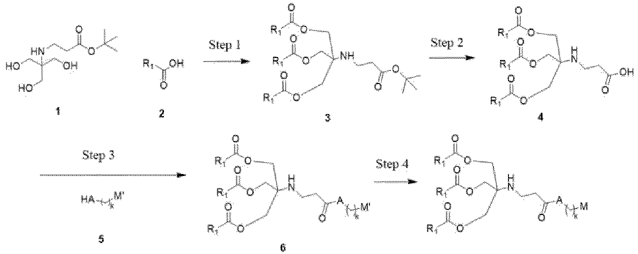

where M' is M or M containing a protecting group; and A is O, NH, or S.

As illustrated in the general synthetic route 2 above, the compound 1 underwent a condensation reaction with the compound 2 to generate the compound 3. Step 1 might be performed in an organic solvent (e.g., DCM) in the presence of EDCl and DMAP.

Subsequently, the tert-butoxy protecting group was removed from the compound 3 to generate the compound 4. Step 2 might be performed in an organic solvent (e.g., DCM) in the presence of an acid (e.g., trifluoroacetic acid) and a cation scavenger (e.g., triisopropylsilane (TiPS)).

Next, the compound 4 underwent a condensation reaction with the compound 5 to obtain the compound 6. Step 3 might be performed in an organic solvent (e.g., DCM or DMF) in the presence of EDCl and DMAP, or DCC.

If the M' group of the compound 6 contained the aforementioned protecting group, the protecting group was removed to obtain the target lipid compound. Step 4 was carried out under deprotection reaction conditions appropriate for the selected protecting group.

### General synthetic route 3

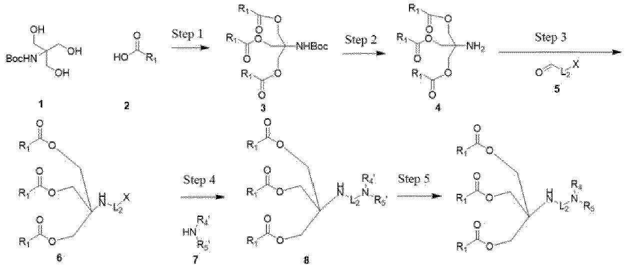

where X is a halogen, such as Cl, Br, or I; R₄' is R₄ or R₄ containing a protecting group; and R₅' is R₅ or R₅ containing a protecting group.

As illustrated in the general synthetic route 3 above, Boc-aminotris(hydroxymethyl)methane underwent a condensation reaction with the compound 2 to generate the compound 3. Step 1 might be performed in an organic solvent (e.g., DCM) in the presence of, for example, EDCl and DMAP. Step 1 can be carried out at room temperature for 24 hours.

Subsequently, the Boc protecting group was removed from the compound 3 to generate the compound 4. Step 2 might be performed in an organic solvent (e.g., DCM) under catalysis by an acid (such as trifluoroacetic acid).

Next, the compound 4 underwent condensation and reduction reactions with a halogen-substituted aldehyde (the compound 5) to obtain the compound 6. Step 3 can be performed in an organic solvent (such as dichloroethane (DCE)) in the presence of a reducing agent (e.g., sodium triacetoxyborohydride (NaBH(OAc)₃)).

Next, the compound6 underwent a nucleophilic reaction with an amine (the compound7) to generate the compound8. Step 4 might be performed in an organic solvent (such as DMF) in the presence of a base (such as a non-nucleophilic organic base (e.g., triethylamine, iPr2EtN) or an inorganic base (e.g., K₂CO₃)) and a catalyst (KI or NaI).

If the R₄' and/or R₅' groups of the compound8 contained the aforementioned protecting groups, the protecting groups were removed to obtain the target lipid compound. Step 5 is carried out under deprotection reaction conditions appropriate for the selected protecting groups.

### General synthetic route 4

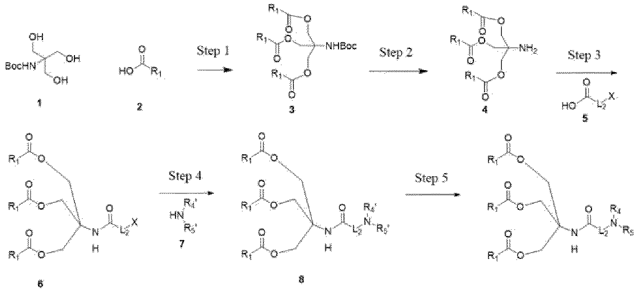

where X is a halogen, such as Cl, Br, or I; R₄' is R₄ or R₄ containing a protecting group; and R₅' is R₅ or R₅ containing a protecting group.

As illustrated in the general synthetic route 4 above, Boc-aminotris(hydroxymethyl)methane underwent a condensation reaction with the compound2 to generate the compound3. Step 1 might be performed in an organic solvent (e.g., DCM) in the presence of, for example, EDCl and DMAP. Step 1 can be carried out at room temperature for 24 hours.

Subsequently, the Boc protecting group was removed from the compound3 to generate the compound4. Step 2 might be performed in an organic solvent (e.g., DCM) under catalysis by an acid (such as trifluoroacetic acid).

Next, the compound4 underwent condensation reactions with a halogen-substituted the compound5 to obtain the compound6. Step 3 might be performed in an organic solvent, such as DCE or DMF, and under catalysis by EDCl and DMAP, or DCC.

Next, the compound6 underwent a nucleophilic reaction with an amine (the compound7) to generate the compound8. Step 4 might be performed in an organic solvent (such as DMF) in the presence of a base (such as a non-nucleophilic organic base (e.g., triethylamine, iPr2EtN) or an inorganic base (e.g., K₂CO₃)) and a catalyst (KI or NaI).

If the R₄' and/or R₅' groups of the compound8 contained the aforementioned protecting groups, the protecting groups were removed to obtain the target lipid compound. Step 5 was carried out under deprotection reaction conditions appropriate for the selected protecting groups.

### General synthetic route 5

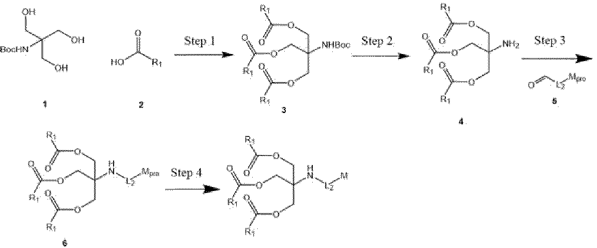

where Mₚᵣₒ is M or M containing a protecting group.

As illustrated in the general synthetic route 5 above, Boc-aminotris(hydroxymethyl)methane underwent a condensation reaction with the compound2 to generate the compound3. Step 1 might be performed in an organic solvent (e.g., DCM) in the presence of, for example, EDCl and DMAP. Step 1 can be carried out at room temperature for 24 hours.

Subsequently, the Boc protecting group was removed from the compound3 to generate the compound4. Step 2 might be performed in an organic solvent (e.g., DCM) under catalysis by an acid (such as trifluoroacetic acid).

Next, the compound4 underwent condensation and reduction reactions with the compound5 to obtain the compound6. Step 3 might be performed in an organic solvent (such as dichloroethane (DCE)) in the presence of a reducing agent (e.g., sodium triacetoxyborohydride (NaBH(OAc)₃)).

If the Mₚᵣₒ group of the compound6 contained the aforementioned protecting group, the protecting group was removed to obtain the target lipid compound. Step 4 was carried out under deprotection reaction conditions appropriate for the selected protecting group.

Furthermore, it should be understood that any specific example of the present application falling within the scope of the prior art may be expressly excluded from any one or more claims. Since such examples are considered known to those of ordinary skill in the art, they may be excluded even if the exclusion is not explicitly stated herein.

All cited sources, such as references, publications, databases, database entries, and techniques cited herein, are incorporated by reference into the present application, even if not explicitly stated as such in the citation. In the event of a conflict between a cited source and the statements in the present application, the statements in the present application shall prevail.

### (1) Synthesis of compounds according to formula (1), (1A), (1B), (1C), (1D), (1E), (1F), (1G), (1H), (1I), (1J), (1K), (1L), or (1M)

### A. General considerations

It should be noted that the starting materials used in the present application are standard commercially available products, and no specific limitations are placed on their sources.

The process routes described below can be used to synthesize compounds 1001-3422 of the present application.

The following abbreviations are used herein:
THF: Tetrahydrofuran
MeCN: Acetonitrile
MeOH: Methanol
PE: Petroleum ether
EA: Ethyl acetate
DMF: N,N-dimethylformamide
EDCl: 1-ethyl-(3-dimethylaminopropyl)carbodiimide hydrochloride
LAH: Lithium aluminum hydride
DCM: Dichloromethane
DMAP: 4-dimethylaminopyridine
LDA: Lithium diisopropylamide
rt: Room temperature
DCE: 1,2-dichloroethane
n-BuLi: n-butyllithium
i-Pr2EtN: N,N-diisopropylethylamine

### B. Synthesis of intermediates

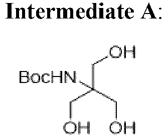

**The intermediate A** was obtained by using the following synthetic route:

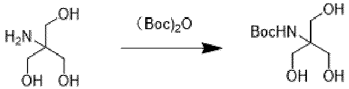

Tris(hydroxymethyl)aminomethane (50.0 g) and di-tert-butyl dicarbonate (Boc₂ O) (99.1 g) were dissolved in a mixed solvent of methanol (300 mL) and H₂ O (30 mL). The mixture was reacted at room temperature for 72 h and purified by silica gel column chromatography (DCM:MeOH = 20:1-10:1) to obtain a white solid compound intermediate A (N-Boc-aminotris(hydroxymethyl)methane) (75.0 g). LCMS (ESI) calcd for C₉ H_{1 9} NOs , [M+H]⁺ m/z 222.13; found 222.25.

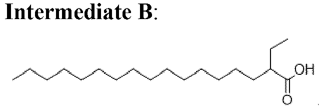

**The intermediate B** was obtained by using the following synthetic route:

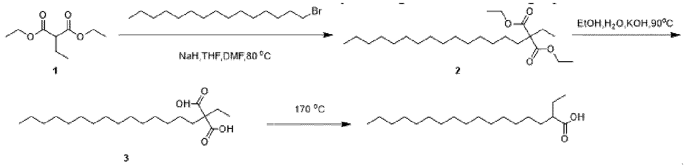

Diethyl 2-ethylmalonate (29.0 g), THF (90 mL), and DMF (30 mL) were added to a single-neck flask. NaH (3.7 g) was added under an ice bath, and the mixture was stirred at room temperature for 30 minutes under a nitrogen atmosphere. 1-Bromopentadecane (15.0 g) was added, and the reaction was carried out at 80°C for 2 hours under nitrogen protection. THF was removed by concentration under reduced pressure, and the reaction mixture was added dropwise to ice water. Ethyl acetate was added, and the mixture was stirred and filtered through diatomite; the organic phase was collected and separated, washed with saturated brine, and purified by silica gel column chromatography (PE:EA = 100:1-50:1) to obtain a compound diethyl 2-ethyl-2-pentadecylmalonate (16.5 g). EtOH (50 mL), H₂0 (50 mL), and KOH (11.3 g) were added to diethyl 2-ethyl-2-pentadecylmalonate (8.0 g), and the reaction was conducted at 90°C for 12 hours. EtOH was removed by concentration under reduced pressure, and dilute hydrochloric acid was added to adjust the reaction system to pH 4-5. Water and ethyl acetate were added; the mixture was stirred and the phases were separated. The organic phase was washed with saturated brine, separated, and purified by column chromatography (PE:EA = 5:1-1:1) to obtain 2-ethyl-2-pentadecylmalonic acid (5.2 g). 2-ethyl-2-pentadecylmalonic acid (10.0 g) was reacted at 170°C for 6 hours in an open vessel, then cooled to room temperature. Water and ethyl acetate were added; and the mixture was stirred and the phases were separated. The organic phase was washed with saturated brine, separated, concentrated, and purified by column chromatography (DCM:MeOH = 20:1-10:1) to obtain the intermediate B (2-ethylheptadecanoic acid) (8.2 g). 1H NMR (400 MHz, CDCl₃) δ 2.31 (tt,J=8.6,5.3 Hz,1H), 1.71-1.46 (m,4H), 1.28 (s,26H), 0.96 (t,J=7.4 Hz,3H), 0.90 (t,J=6.7 Hz,3H).

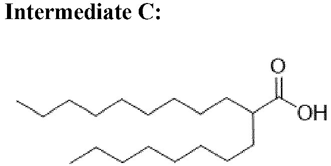

**The intermediate C** was obtained by using the following synthetic route:

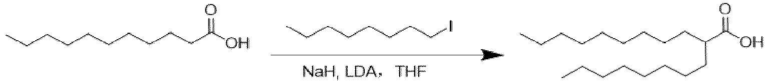

Decanoic acid (50 g) and a solvent THF (500 mL) were added to a three-necked flask. The system was cooled to 0°C, and reactant NaH (23.22 g) was slowly added. After stirring for 1 hour at 0°C under nitrogen protection, LDA (62.19 g) was added dropwise, and stirring was continued for another hour at 0°C under nitrogen protection. Finally, a reactant 1-iodononane (88.52 g) was added dropwise; the mixture was then warmed to room temperature and stirred overnight. The reaction mixture was diluted with 1 L of DCM and washed successively with saturated NH₄Cl solution and water. The organic layer was dried over anhydrous Na₂SO₄, filtered, concentrated, and purified via silica gel column chromatography (PE:EA = 50:1) to finally obtain the intermediate C (2-octylundecanoic acid) (11 g). 1H NMR (400 MHz, Chloroform-d) δ 2.37 (tt, J=8.7, 5.1 Hz, 1H), 1.71-1.58 (m, 2H), 1.48 (dt, J=13.3, 6.7 Hz, 2H), 1.29 (d, J = 9.4 Hz, 25H), 0.90 (t, J=6.8 Hz, 6H).

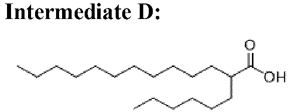

**The intermediate D** was obtained by using the following synthetic route:

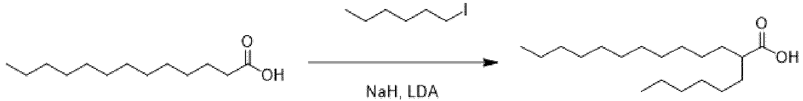

Tridecanoic acid (79.5 g) and THF (800 mL) were added to a three-necked flask. The mixture was cooled to 0°C, and NaH (22.25 g) was added slowly. After the reaction mixture was stirred at 0°C under a nitrogen atmosphere for 1 h, LDA (317.87 g) was slowly added dropwise; stirring was continued at 0°C under nitrogen protection for another hour. 1-iodohexane (94.39 g) was added dropwise, and the mixture was warmed to room temperature and stirred overnight. The reaction mixture was diluted with 1 L of DCM and washed successively with saturated NH₄Cl solution and water. The organic layer was dried over anhydrous Na₂SO₄, filtered, concentrated, and purified via silica gel column chromatography (PE:EA = 50:1) to obtain the intermediate D (2-hexyltridecanoic acid) (26 g). 1H NMR (400 MHz, Chloroform-d) δ 2.36 (tt, J=8.7, 5.4 Hz, 1H), 1.63 (ddd, J=14.3, 8.7, 5.5 Hz, 2H), 1.49 (dq, J=13.5, 6.6 Hz, 2H), 1.29 (d, =10.1 Hz, 26H), 0.90 (t, J=6.6 Hz, 6H).

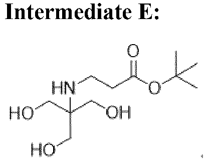

**The intermediate E** was obtained by using the following synthetic route:

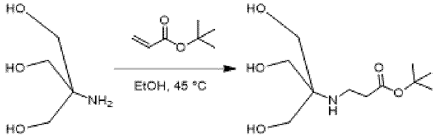

Tris(hydroxymethyl)aminomethane (10.0 g) and tert-butyl acrylate (21.1 g) were dissolved in EtOH (150 mL). The mixture was reacted at 45°C for 30 hours under nitrogen protection. After removing EtOH by concentration under reduced pressure, solvent (PE:EA = 20:1; 100 mL×3) was added for pulping, and the mixture was filtered to obtain a white solid compound Intermediate E (11.5 g). 1H NMR (400 MHz, CDCl₃) δ 3.60 (s, 6H), 2.84 (t, J = 5.9 Hz, 2H), 2.47 (t, J = 5.8 Hz, 2H), 1.48 (s, 9H).

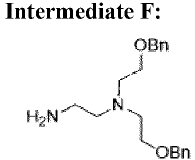

**The intermediate F** was obtained by using the following synthetic route:

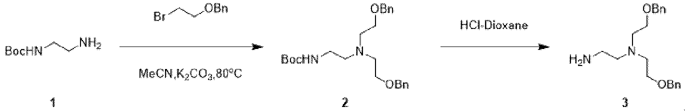

Tert-butyl (2-aminoethyl)carbamate (2.7 g), MeCN (90 mL), benzyl 2-bromoethyl ether (7.99 g), and K₂CO₃ (11.65 g) were added to a single-neck flask. After the mixture was reacted at 80°C overnight, water and ethyl acetate were added for phase separation. The organic phase was washed with saturated brine, separated, concentrated, and purified by silica gel column chromatography (PE:EA = 10:1-5:1) to obtain the compound 2 (5.5 g). Dioxane (30 mL) and a solution of HCl in dioxane (30 mL) were added to the compound 2. After stirring at room temperature for 3 h, the mixture was concentrated under reduced pressure to obtain Compound 3, i.e., Intermediate F (6 g). LCMS (ESI) calcd for C₂₀H₂₈N₂O₂, [M+H]⁺ m/z 329.22; found 329.24.

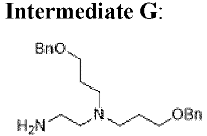

**The intermediate G** was obtained by using the following synthetic route:

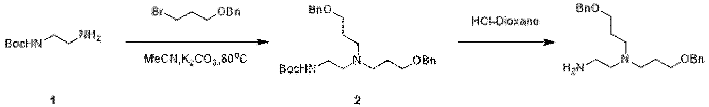

The compound 1 (3.0 g), MeCN (90 mL), 3-(benzyloxy)propyl bromide (9.45 g), and K₂CO₃ (12.94 g) were added to a single-neck flask. After the mixture was reacted at 80°C overnight, water and ethyl acetate were added for phase separation. The organic phase was washed with saturated brine, separated, concentrated, and purified by column chromatography (PE:EA = 10:1-5:1) to obtain the compound 2 (6.9 g). Dioxane (30 mL) and a solution of HCl in dioxane (30 mL) were added to the compound2 (6.9 g). After stirring at room temperature for 3 h, the mixture was concentrated under reduced pressure to obtain the intermediate G (7.5 g). LCMS (ESI) calcd for C₂₂H₃₂N₂O₂, [M+H]⁺ m/z 357.25; found 357.51.

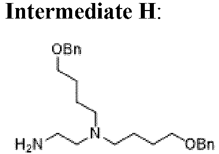

**The intermediate H** was obtained by using the following synthetic route:

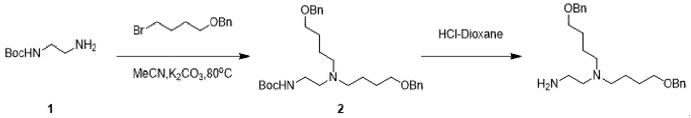

The compound 1 (1.5 g), MeCN (90 mL), benzyl 4-bromobutyl ether (5.02 g), and K₂CO₃ (6.47 g) were added to a single-necked flask. After the mixture was reacted at 80°C overnight, water and ethyl acetate were added for phase separation. The organic phase was washed with saturated brine, separated, concentrated, and purified via column chromatography (PE:EA = 20:1-10:1) to obtain the compound 2 (4.1 g). Dioxane (30 mL) and a solution of HCl in dioxane (30 mL) were added to the compound 2 (4.1 g). After stirring at room temperature for 3 h, the mixture was concentrated under reduced pressure to obtain the intermediate H (4.9 g). LCMS (ESI) calcd for C₂₄H₃₆N₂O₂, [M+H]⁺ m/z 385.28; found 385.56.

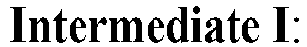

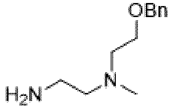

**The intermediate I** was obtained by using the following synthetic route:

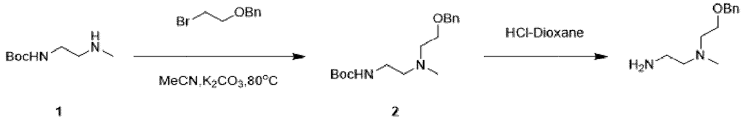

The compound 1 (3.9 g), MeCN (90 mL), benzyl 2-bromoethyl ether (5.78 g), and K₂CO₃ (15.46 g) were added to a single-necked flask. After the mixture was reacted at 80°C overnight, water and ethyl acetate were added, and the mixture was stirred for phase separation. The organic phase was washed with saturated brine, separated, concentrated, and purified via column chromatography (PE:EA = 20:1-10:1) to obtain the compound 2 (6.8 g). Dioxane (30 mL) and a solution of HCl in dioxane (30 mL) were added to the compound 2 (6.8 g). After stirring at room temperature for 3 hours, the mixture was concentrated under reduced pressure to obtain Intermediate I (6.5 g). LCMS (ESI) calcd for C₁₂H₂₀N₂O, [M+H]⁺ m/z 209.16; found 209.31.

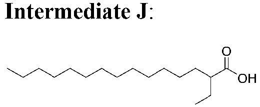

**The intermediate J** was obtained by using the following synthetic route:

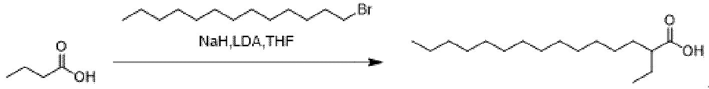

Butyric acid (5.0 g) and THF (100 mL) were added to a single-necked flask. NaH (2.73 g) was added at 0°C, followed by the slow dropwise addition of LDA (56.8 mL). The mixture was reacted at room temperature for 30 min, then 1-bromotridecane was added. After reacting overnight at room temperature, ice water and ethyl acetate were added for phase separation. The organic phase was washed with saturated brine, separated, concentrated, and purified via column chromatography (PE:EA = 10:1-5:1) to obtain the intermediate J (5.0 g). LCMS (ESI) calcd for C₁₇H₃₄O₂: [M+H]⁺ m/z 271.26; found 271.46.

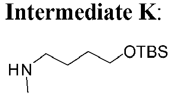

**The intermediate K** was obtained by using the following synthetic route:

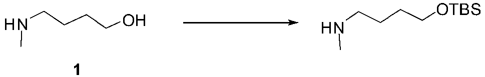

A reactant 1 (24 g), imidazole (19.00 g), and a solvent DCM (200 mL) were added to a three-necked flask. After the mixture was cooled to 0°C, the reactant TBDMSCl (38.57 g) was slowly added. The mixture was stirred at 25°C for 4 hours, then diluted with 300 mL of DCM. The organic phase was washed twice with 1 L of water and dried over anhydrous Na₂SO₄. After filtration and concentration, silica gel was added, and the mixture was purified by column chromatography (eluent: DCM:MeOH = 1:20); the product fractions were collected and concentrated to obtain the intermediate K (28.00 g). 1H NMR (400 MHz, CDCl₃) δ 4.89 (s, 1H), 3.58 (t, J = 6.1 Hz, 2H), 2.66 (t, J = 7.3 Hz, 2H), 2.45 (s, 3H), 1.67-1.40 (m, 4H), 0.84 (s, 9H).

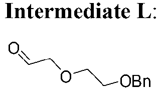

**The intermediate L** was obtained by using the following synthetic route:

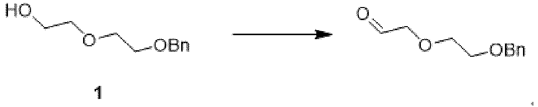

DMSO (3.58 g) and anhydrous solvent DCM (30 mL) were added to a three-necked flask. The mixture was cooled to -78°C, and oxalyl chloride (2.91 g) was added slowly. After the reaction liquid was stirred at -78°C under nitrogen protection for 10 minutes, the compound 1 (3 g) was added dropwise. Stirring was continued at -78°C under nitrogen protection for 1 h. Finally, TEA (9.28 g) was added dropwise, and stirring was continued at -78°C under nitrogen protection for another 0.5 hour. The mixture was diluted with 100 mL of DCM and washed successively with saturated aqueous NH₄Cl solution and water. The organic layer was dried over anhydrous Na₂SO₄, filtered, and concentrated to obtain the intermediate L (3.0 g). LCMS (ESI) calcd for C₁₁H₁₄O₃: [M+H]⁺ m/z 195.09; found 195.23.

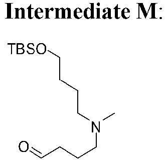

**The intermediate M** was obtained by using the following synthetic route:

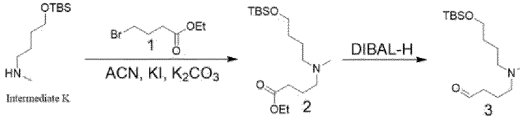

The intermediate K (2.0 g), the compound 1 (1.79 g), and ACN (20 mL) were added to a sealed tube. The reactant K₂CO₃ (3.81 g) and KI (1.52 g) were added, and the mixture was stirred overnight at 70°C under nitrogen protection. The mixture was then diluted with 100 mL of EA and washed successively with saturated aqueous NH₄Cl solution and water. The organic layer was dried over anhydrous Na₂SO₄, filtered, and concentrated. Silica gel was added, and the mixture was purified by column chromatography (eluent: DCM:MeOH = 15:1). The product fractions were collected and concentrated to obtain the compound 2 (2.0 g). The reactant 2 (1.80 g) and the solvent DCM (20 mL) were added to a three-necked flask. The system was purged with nitrogen and cooled to -78°C. DIBAL-H (1.00 g) was added dropwise, and the mixture was stirred at -78°C for 4 hours. The reaction was quenched by the dropwise addition of methanol and sodium carbonate solution, followed by extraction with DCM (100 mL). The organic layer was dried over anhydrous sodium sulfate, filtered, and concentrated to obtain the intermediate M (1.36 g). LCMS (ESI) calcd for C₁₅H₃₃NO₂Si, [M+H]⁺ m/z 288.52; found 288.23.

### C. Compound 1003, synthesized according to the general synthetic route 1

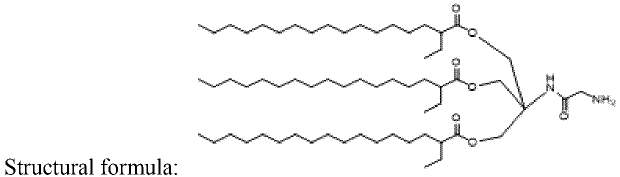

Chemical formula: C₆₃H₁₂₂N₂O₇
Molecular weight: 1019.68

### Step 1: Synthesis of the compound 3 in the general synthetic route 1

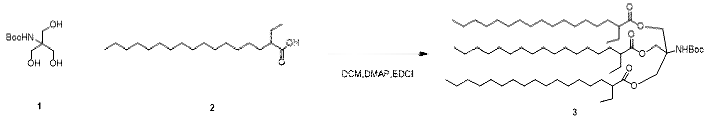

The compound 1 (the intermediate A, 1.0 g), DCM (20 mL), DMAP (2.2 g), and EDCI (3.4 g) were added to the compound 2 (the intermediate B, 4.3 g). The mixture was stirred at room temperature under nitrogen protection for 12 hours. Water and dichloromethane were added, and the mixture was stirred and phase-separated. The organic phase was washed with saturated brine, separated, dried over anhydrous sodium sulfate, concentrated and purified via silica gel column chromatography (PE:EA = 40:1-30:1) to obtain the compound 3 (2.6 g).

### Step 2: Synthesis of the compound 4 in the general synthetic route 1

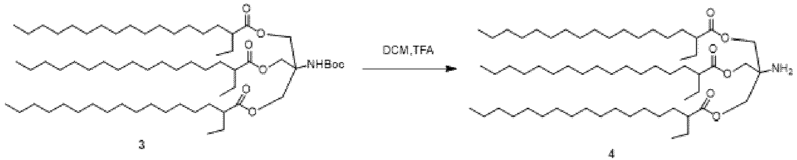

DCM (15 mL) and TFA (5 mL) were added to the compound 3 (2.6 g). The mixture was stirred at room temperature for 3 hours, then concentrated. Purification via silica gel column chromatography (PE:EA = 10:1-5:1) was carried out to obtain the compound 4 (3.2 g).

### Step 3: Synthesis of the compound 6 in the general synthetic route 1

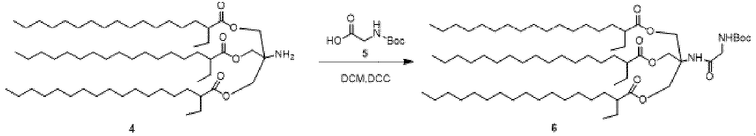

DCM (20 mL), Boc-glycine (Compound 5, 578 mg), and DCC (6.8 g) were added to the compound 4 (3.2 g). The mixture was stirred at room temperature under nitrogen protection for 12 hours. Water and ethyl acetate were added, and the mixture was stirred and phase-separated. The organic phase was washed with saturated brine, separated, dried over anhydrous sodium sulfate, and concentrated. Purification via column chromatography (PE:EA = 5:1-2:1) was carried out to obtain the compound 6 (2.2 g).

### Step 4: Compound 1003

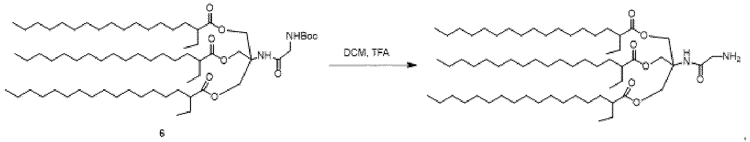

DCM (15 mL) and TFA (5 mL) were added to the compound 6 (2.2 g). The mixture was stirred at room temperature for 12 hours, then concentrated. Purification via column chromatography (DCM:MeOH = 30:1-20:1) was carried out to obtain the compound 1003 (1.3 g). 1H NMR (400 MHz, CDCl₃) δ 4.49 (d, J = 2.6 Hz, 6H), 3.28 (s, 2H), 2.32 (tt, J = 8.6, 5.5 Hz, 3H), 1.62 (q, J = 7.1 Hz, 12H), 1.27 (d, J = 3.4 Hz, 78H), 0.94-0.86 (m, 18H).

### D. Compound 1002, synthesized according to the general synthetic route 1

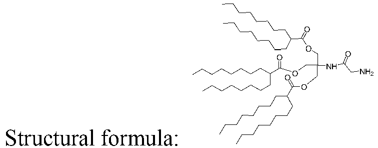

Chemical formula: C₆₀H₁₁₆N₂O₇
Molecular weight: 977.60

The compound was synthesized according to the general synthetic route 1; the process was similar to that for the compound 1003, except that the intermediate C was used as the compound 2 in the general synthetic route 1. ¹H NMR (400 MHz, CDCl₃) δ 4.48 (s, 6H), 3.27 (s, 2H), 2.37 (tt, J = 8.5, 5.5 Hz, 3H), 1.64 (d, J = 6.9 Hz, 6H), 1.50-1.42 (m, 6H), 1.27 (s, 72H), 0.90 (t, J = 6.7 Hz, 18H).

### E. Compound 1004, synthesized according to the general synthetic route 1

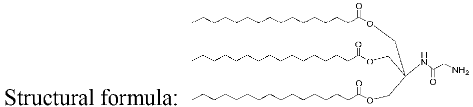

Chemical formula: C₅₄H₁₀₄N₂O₇
Molecular weight: 893.43

The compound was synthesized according to the general synthetic route 1; the process was similar to that for the compound 1003, except that palmitic acid was used as the compound 2 in the general synthetic route 1. ¹H NMR (400 MHz, CDCl₃) δ 7.55 (s, 1H), 4.45 (s, 6H), 3.74 (s, 2H), 2.35 (t, J = 7.6 Hz, 6H), 1.60 (p, J = 6.9 Hz, 6H), 1.27 (s, 72H), 0.90 (t, J = 6.7 Hz, 9H).

### F. Compound 1001, synthesized according to the general synthetic route 1

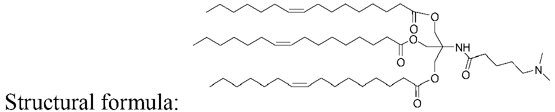

Chemical formula: C₅₉H₁₀₈N₂O₇
Molecular weight: 957.52

The compound was synthesized according to the general synthetic route 1; the process was similar to that for the compound 1003, except that (9Z)-9-hexadecenoic acid was used as the compound 2 in the general synthetic route 1, and 5-(N,N-dimethylamino)pentanoic acid was used as the compound 5 in the general synthetic route 1. ¹H NMR (400 MHz, CDCl₃) δ 5.42-5.30 (m, 6H), 4.51-4.38 (m, 6H), 3.19 (dt, J = 11.3, 5.6 Hz, 2H), 2.97 (t, J = 5.9 Hz, 6H), 2.46 (t, J = 6.6 Hz, 1H), 2.35 (td, J = 7.6, 3.1 Hz, 5H), 2.20 (t, J = 7.6 Hz, 2H), 2.03 (d, J = 6.2 Hz, 12H), 1.89-1.81 (m, 3H), 1.74 (q, J = 7.8 Hz, 2H), 1.67-1.51 (m, 6H), 1.31 (d, J = 7.8 Hz, 48H), 0.90 (t, J = 6.7 Hz, 9H).

### G. Compound 1014, synthesized according to the general synthetic route 2

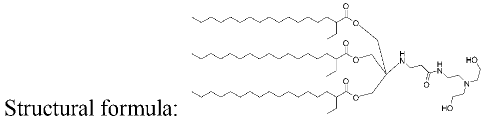

Chemical formula: C₇₀H₁₃₇N₃O₉
Molecular weight: 1164.88

### Step 1: Synthesis of the intermediate 3 in the general synthetic route 2

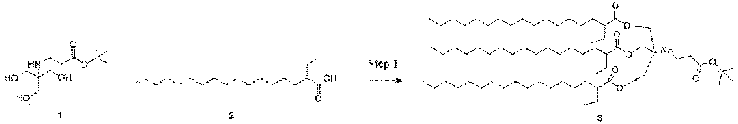

The intermediate 3 was synthesized according to the method described in Step 1 of the general synthetic route 1, except that the intermediate E was used as the compound 1 in the general synthetic route 1.

### Step 2: Synthesis of the compound 4 in the general synthetic route 2

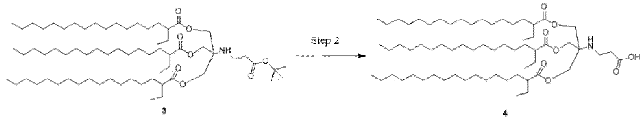

DCM (15 mL), TFA (12 mL), and TiPS (3 mL) were added to the compound 3 (4.8 g). After reacting at room temperature for 12 hours, water and dichloromethane were added, and the mixture was stirred and phase-separated. The organic phase was washed with saturated brine, separated, concentrated, and purified by silica gel column chromatography (DCM:MeOH = 20:1-10:1) to obtain the compound 4 (3.3 g).

### Step 3: Synthesis of the compound 6 in the general synthetic route 2

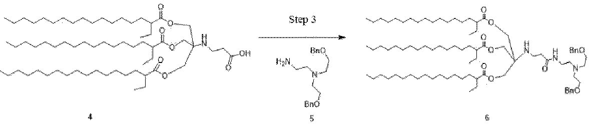

The compound 4 (4.0 g), DCM (80 mL), EDCI (1.1 g), 1-hydroxybenzotriazole (HOBt) (0.78 g), and DIEA (2.5 g) were added to the compound 5 (1.9 g). After the mixture was stirred at room temperature for 12 h, water and dichloromethane were added, and the mixture was stirred and phase-separated. The organic phase was washed with saturated brine, separated, concentrated, and purified by silica gel column chromatography (DCM:MeOH = 30:1-20:1) to obtain the compound 6 (4.0 g).

### Step 4: Compound 1014

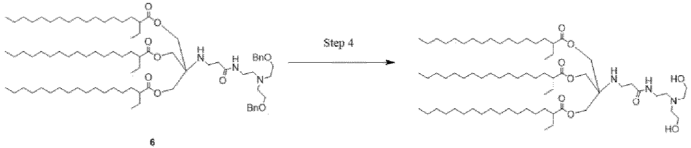

MeOH (30 mL), DCM (10 mL), and Pd/C (4.73 g, 10%) were added to the compound 6 (4.0 g). The mixture was stirred overnight at room temperature under a hydrogen atmosphere, then filtered through diatomite and concentrated, and subjected to silica gel column chromatography (DCM:MeOH 30:1-15:1) to obtain the compound 1014 (2 g). ¹H (400 MHz, CDCl₃) δ 7.38 (t, J = 5.8 Hz, 1H), 4.14-4.05 (m, 6H), 3.58 (t, J = 4.9 Hz, 4H), 3.31 (q, J = 5.7 Hz, 2H), 2.89 (t, J = 6.3 Hz, 2H), 2.64 (q, J = 5.2 Hz, 6H), 2.29 (dq, J = 10.8, 4.4 Hz, 6H), 1.66-1.37 (m, 12H), 1.24 (d, J = 4.3 Hz, 78H), 0.87 (t, J = 7.1 Hz, 18H).

### H. Compound 1015, synthesized according to the general synthetic route 2

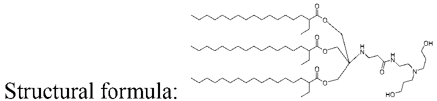

Chemical formula: C₇₂H₁₄₁N₃O₉
Molecular weight: 1192.93

The compound was synthesized according to the general synthetic route 2; the synthesis process was similar to that of the compound 1014, except that the intermediate G was used as the compound 5 in the general synthetic route 2. ¹H (400 MHz, CDCl₃) δ 7.58 (t, J = 5.7 Hz, 1H), 4.15-4.01 (m, 6H), 3.70 (t, J = 5.4 Hz, 4H), 3.39 (q, J = 5.7 Hz, 2H), 2.86 (t, J = 6.2 Hz, 2H), 2.57 (dt, J = 20.5, 5.8 Hz, 6H), 2.36-2.22 (m, 5H), 1.76-1.67 (m, 4H), 1.62-1.35 (m, 13H), 1.23 (d, J = 4.6 Hz, 78H), 0.92-0.80 (m, 18H).

### I. Compound 1020, synthesized according to the general synthetic route 2

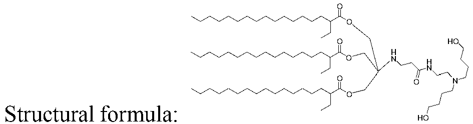

Chemical formula: C₇₄H₁₄₅N₃O₉
Molecular weight: 1220.99
The compound was synthesized according to the general synthetic route 2; the synthesis process was similar to that of the compound 1014, except that the intermediate H was used as the compound 5 in the general synthetic route 2.

### J. Compound 1025, synthesized according to the general synthetic route 2

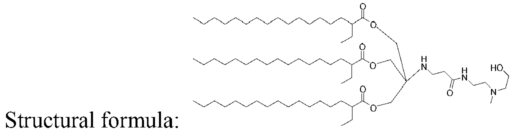

Chemical formula: C₆₉H₁₃₅N₃O₃
Molecular weight: 1134.85

The compound was synthesized according to the general synthetic route 2; the process was similar to that for the compound 1014, except that the intermediate I was used as the compound 5 in the general synthetic route 2. ¹H (400 MHz, CDCl₃) δ 6.98 (t, J = 5.7 Hz, 1H), 4.10 (m, J = 3.8 Hz, 6H), 3.63 (t, J = 5.2 Hz, 2H), 3.35 (q, J = 5.8 Hz, 2H), 2.89 (t, J = 6.1 Hz, 2H), 2.57 (q, J = 5.3 Hz, 4H), 2.32 (s, 3H), 2.28 (q, J = 4.1 Hz, 5H), 1.65-1.41 (m, 13H), 1.24 (d, J = 4.4 Hz, 78H), 0.87 (t, J = 7.1 Hz, 18H).

### K. Compound 1005, synthesized according to the general synthetic route 2

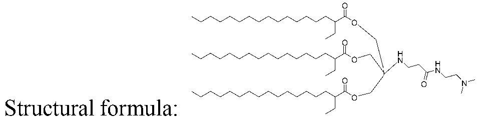

Chemical formula: C₆₈H₁₃₃N₃O₇
Molecular weight: 1104.83

The compound was synthesized according to the general synthetic route 2; the process was similar to that for the compound 1014, except that N,N-dimethylethylenediamine was used as the compound 5 in the general synthetic route 2. ¹H NMR (400 MHz, CDCl₃) δ 4.19-4.07 (m, 6H), 3.40 (d, J = 5.7 Hz, 2H), 2.95-2.87 (m, 2H), 2.82 (s, 4H), 2.64-2.55 (m, 2H), 2.38 (s, 6H), 2.33-2.29 (m, 3H), 1.63-1.55 (m, 6H), 1.49-1.44 (m, 2H), 1.26 (d, J = 5.4 Hz, 78H), 0.89 (td, J = 7.0, 2.8 Hz, 18H).

### L. Compound 1006, synthesized according to the general synthetic route 2

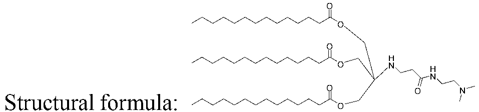

Chemical formula: C₅₃H₁₀₃N₃O₇
Molecular weight: 894.42

The compound was synthesized according to the general synthetic route 2; the process was similar to that for the compound 1014, except that tetradecanoic acid was used as the compound 2 in the general synthetic route 2 and N,N-dimethylethylenediamine was used as Compound 5 in General Synthetic Route 2. ¹H NMR (400 MHz, CDCl₃) δ 7.99 (t, J = 5.8 Hz, 1H), 4.13 (s, 6H), 3.60 (q, J = 5.3 Hz, 2H), 3.38-3.34 (m, 2H), 3.00 (s, 6H), 2.94 (t, J = 5.8 Hz, 2H), 2.45 (t, J = 5.8 Hz, 2H), 2.35 (t, J = 7.6 Hz, 6H), 1.66-1.56 (m, 6H), 1.33-1.23 (m, 60H), 0.90 (t, J = 6.8 Hz, 9H).

### M. Compound 1007, synthesized according to the general synthetic route 2

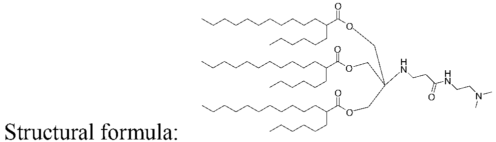

Chemical formula: C₆₈H₁₃₃N₃O₇
Molecular weight: 1104.83

The compound was synthesized according to the general synthetic route 2; the process was similar to that for the compound 1014, except that the intermediate D was used as the compound 2 and N,N-dimethylethylenediamine was used as the compound 5 in the general synthetic route 2. ¹H NMR (400 MHz, Chloroform-d) δ 7.13 (t, J = 5.0 Hz, 1H), 4.13 (s, 6H), 3.33 (q, J = 5.6 Hz, 2H), 2.92 (t, J = 6.0 Hz, 2H), 2.47-2.28 (m, 7H), 2.24 (s, 6H), 1.60 (dq, J = 14.7, 7.2 Hz, 6H), 1.47 (dd, J = 14.2, 6.7 Hz, 6H), 1.27 (d, J = 3.4 Hz, 78H), 0.89 (td, J = 6.7, 2.5 Hz, 18H).

### N. Compound 1008, synthesized according to the general synthetic route 2

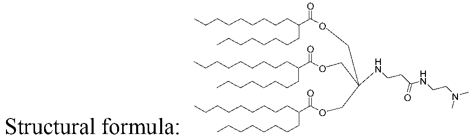

Chemical formula: C₆₈H₁₃₃N₃O₇
Molecular weight: 1104.01

The compound was synthesized according to the general synthetic route 2; the process was similar to that for the compound 1014, except that the intermediate C was used as the compound 2 in the general synthetic route 2 and N,N-dimethylethylenediamine was used as the compound 5 in the general synthetic route 2. ¹H NMR (400 MHz, Chloroform-d) δ 4.13 (s, 6H), 3.33 (q, J = 5.6 Hz, 2H), 2.92 (t, J = 5.9 Hz, 2H), 2.51-2.26 (m, 7H), 2.24 (s, 6H), 1.59 (ddt, J = 14.8, 10.9, 6.4 Hz, 6H), 1.47 (tq, J = 11.0, 5.4 Hz, 6H), 1.26 (s, 77H), 0.89 (t, J = 6.7 Hz, 18H).

### O. Compound 1009, synthesized according to the general synthetic route 2

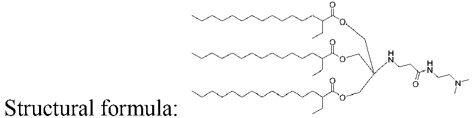

Chemical formula: C₆₂H₁₂₁N₃O₇
Molecular weight: 1020.66

The compound was synthesized according to the general synthetic route 2; the process was similar to that for the compound 1014, except that the intermediate J was used as the compound 2 and N,N-dimethylethylenediamine was used as the compound 5 in the general synthetic route 2. ¹H (400 MHz, CDCl₃) δ 7.04 (t, J = 5.0 Hz, 1H), 4.12 (m, J = 3.3 Hz, 6H), 3.30 (q, J = 5.6 Hz, 2H), 2.91 (t, J = 6.0 Hz, 2H), 2.39 (t, J = 6.0 Hz, 2H), 2.33-2.25 (m, 5H), 2.22 (s, 6H), 1.76-1.54 (m, 12H), 1.24 (m, J = 3.7 Hz, 66H), 0.90-0.84 (m, 18H).

### P. Compound 1011, synthesized according to the general synthetic route 2

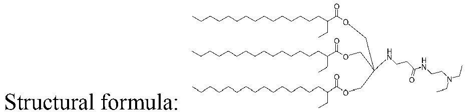

Chemical formula: C₇₀H₁₃₇N₃O₇
Molecular weight: 1132.88

The compound was synthesized according to the general synthetic route 2; the process was similar to that for the compound 1014, except that N,N-diethylethylenediamine was used as the compound 5 in the general synthetic route 2. ¹H NMR (400 MHz, CDCl₃) δ 4.19-4.08 (m, 6H), 3.31 (q, J = 5.8 Hz, 2H), 2.92 (t, 2H), 2.57 (t, J = 6.9 Hz, 6H), 2.37-2.26 (m, 5H), 1.62 (ddd, J = 18.3, 9.0, 4.3 Hz, 12H), 1.27 (d, J = 4.7 Hz, 78H), 1.04 (t, J = 7.1 Hz, 6H), 0.94-0.86 (m, 18H).

### Q. Compound 1012, synthesized according to the general synthetic route 2

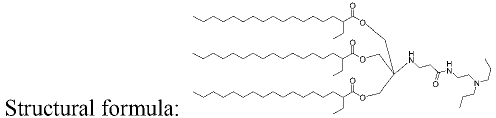

Chemical formula: C₇₂H₁₄₁N₃O₇
Molecular weight: 1160.93

The compound was synthesized according to the general synthetic route 2; the synthesis process was similar to that of the compound 1014, except that N,N-diethylethylenediamine was used as the compound 5 in the general synthetic route 2. ¹H NMR (400 MHz, CDCl₃) δ 6.59 (s, 1H), 4.18-4.07 (m, 6H), 3.29 (q, J = 5.7 Hz, 2H), 2.91 (t, J = 6.2 Hz, 2H), 2.54 (t, J = 6.1 Hz, 2H), 2.40 (t, J = 7.5 Hz, 4H), 2.35-2.27 (m, 5H), 1.63-1.42 (m, 16H), 1.27 (m, J = 4.5 Hz, 78H), 0.93-0.85 (m, 24H).

### R. Compound 1013, synthesized according to the general synthetic route 2

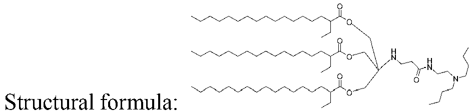

Chemical formula: C₇₄H₁₄₅N₃O₇
Molecular weight: 1188.99

The compound was synthesized according to the general synthetic route 2; the synthesis process was similar to that of the compound 1014, except that N,N-diethylethylenediamine was used as the compound 5 in the general synthetic route 2. ¹H NMR (400 MHz, CDCl₃) δ 4.10 (d, J = 4.1 Hz, 6H), 3.26 (q, J = 5.8 Hz, 2H), 2.89 (t, J = 6.2 Hz, 2H), 2.52 (d, J = 6.6 Hz, 2H), 2.41 (t, J = 7.4 Hz, 4H), 2.33-2.25 (m, 5H), 1.60 (d, J = 11.8 Hz, 16H), 1.24 (d, J = 4.5 Hz, 82H), 0.96-0.79 (m, 24H).

### S. Compound 1029, synthesized according to the general synthetic route 2

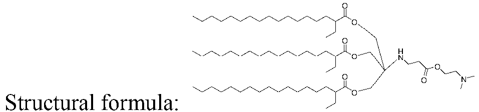

Chemical formula: C₆₈H₁₃₂N₂O₈
Molecular weight: 1105.81

The compound was synthesized according to the general synthetic route 2; the process was similar to that for the compound 1014, except that N,N-diethyl-2-hydroxyethylamine was used as the compound 5 in the general synthetic route 2. ¹H NMR (400 MHz, CDCl₃) δ 4.16 (t, J = 5.8 Hz, 2H), 4.13-4.05 (m, 6H), 2.86 (t, J = 6.3 Hz, 2H), 2.55 (t, J = 5.8 Hz, 2H), 2.44 (t, J = 6.3 Hz, 2H), 2.35-2.20 (m, 9H), 1.60-1.39 (m, 11H), 1.24 (d, J = 4.5 Hz, 80H), 1.02-0.79 (m, 18H).

### T. Compound 1111, synthesized according to the general synthetic route 2

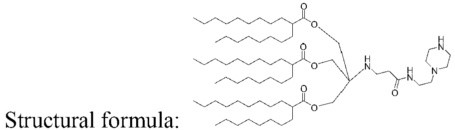

Chemical formula: C₇₀H₁₃₆N₄O₇
Molecular weight: 1145.88

The compound was synthesized according to the general synthetic route 2; the process was similar to that for the compound 1014, except that the intermediate C was used as the compound 2 in the general synthetic route 2 and tert-butyl 4-(2-aminoethyl)piperazine-1-carboxylate was used as the compound 5 in the general synthetic route 2. ¹H NMR (400 MHz, Chloroform-d) δ 4.28 (s, 6H), 3.41 (s, 2H), 3.31 (s, 4H), 3.22 (s, 3H), 2.97-2.80 (m, 5H), 2.70 (s, 5H), 2.46-2.32 (m, 4H), 1.60 (t, J = 7.1 Hz, 6H), 1.54-1.40 (m, 6H), 1.27 (s, 78H), 0.89 (t, J = 6.7 Hz, 18H).

### U. Compound 1118, synthesized according to the general synthetic route 2

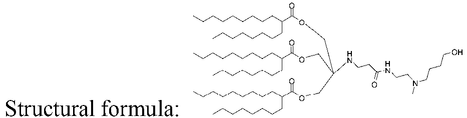

Chemical formula: C₇₁H₁₃₉N₃O₈
Molecular weight: 1162.91

The compound was synthesized according to the general synthetic route 2; the process was similar to that for the compound 1014, except that the intermediate C was used as the compound 2 and the intermediate K was used as the compound 5 in the general synthetic route 2. ¹H NMR (400 MHz, Chloroform-d) δ 4.11 (s, 6H), 3.61 (s, 2H), 3.42 (d, J = 33.5 Hz, 2H), 2.91 (t, J = 6.2 Hz, 3H), 2.36 (dd, J = 9.2, 3.6 Hz, 6H), 1.47 (dd, J = 13.9, 6.2 Hz, 14H), 1.27 (s, 71H), 0.90 (t, J = 6.7 Hz, 18H).

### V. Compound 1010, synthesized according to the general synthetic route 3

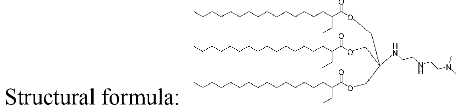

Chemical formula: C₆₇H₁₃₃N₃O₆
Molecular weight: 1076.82

### Steps 1 and 2: Synthesis of the compound 3 and compound 4 in the general synthetic route 3

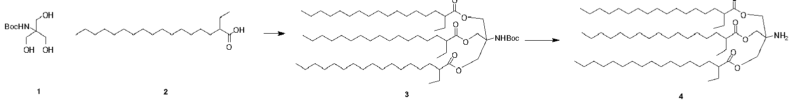

The synthesis was performed according to Steps 1 and 2 of the general synthetic route 1; and the process was identical to that for the compound 1003.

### Step 3: Synthesis of the compound 6 in the general synthetic route 3

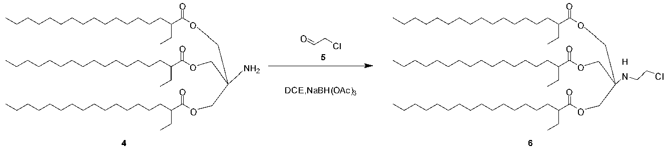

The compound 4 (600 mg), chloroacetaldehyde (Compound 5, 242 mg (40%)), and NaBH(OAc)₃ (394 mg) were added to DCE (60 mL). After reacting overnight at room temperature, water and dichloromethane were added, and the mixture was stirred and phase-separated. The organic phase was washed with saturated brine, separated, and concentrated, and subjected to silica gel column chromatography (DCM:MeOH = 30:1-15:1) to obtain the compound 6 (270 mg).

### Step 4: Synthesis of the compound 1010

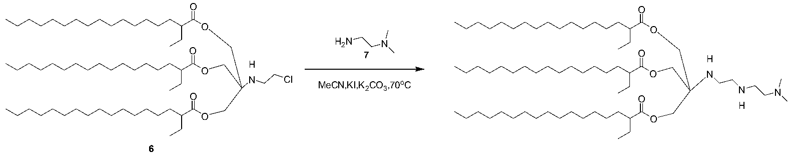

MeCN (20 mL), N,N-dimethylethylenediamine (the compound 7, 229 mg), KI (43 mg), and K₂CO₃ (179 mg) were added to the compound6 (270 mg). The mixture was reacted overnight at 70°C under nitrogen protection, and the compound 1010 (16 mg) was obtained by silica gel column chromatography. ¹H (400 MHz, CDCl₃) δ 4.10 (m, J = 2.9 Hz, 6H), 2.76-2.64 (m, 6H), 2.40 (t, J = 6.2 Hz, 2H), 2.29 (ddd, J = 8.5, 5.6, 2.9 Hz, 3H), 2.22 (s, 6H), 1.97 (s, 6H), 1.50-1.41 (m, 6H), 1.24 (d, J = 4.2 Hz, 78H), 0.87 (t, J = 7.1 Hz, 18H).

### W. Compound 1059, synthesized according to the general synthetic route 3

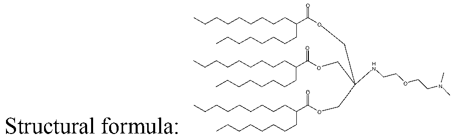

Chemical formula: C₆₇H₁₃₂N₂O₇
Molecular weight: 1077.80

The compound was synthesized according to the general synthetic route 3; the process was similar to that of the compound 1010, except that the intermediate C and the intermediate L were used as the compound 2 and compound 5, respectively, in the general synthetic route 3. Additionally, the compound 6 in the general synthetic route 3 were subjected to functional group transformation via the following process steps:

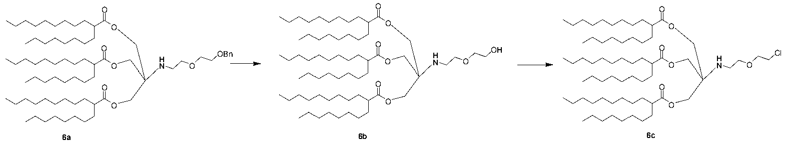

Solvents MeOH (5 mL) and THF (5 mL) were added to the intermediate 6a (1.5 g), followed by the addition of the reactant Pd(OH)₂/C (10%, 0.46 g) and Pd/C (10%, 0.35 g). The mixture was reacted overnight at 25°C under an H₂ atmosphere, then filtered through diatomite. The filtrate was concentrated and purified by silica gel column chromatography (PE:EA 30:1-10:1) to obtain the intermediate 6b (520 mg). DCM (5 mL) was added to the intermediate 6b (470 mg), and SOCl₂ (1064.26 mg, 8.946 mmol) was added dropwise at 0°C. The mixture was transferred to room temperature and reacted for 3 hours. The reaction mixture was extracted with water and DCM; the organic phase was concentrated and purified by silica gel column chromatography (PE:EA 20:1) to obtain the intermediate 6c (181 mg). Dimethylamine (143.39 mg), DMF (2 mL), potassium carbonate (43.96 mg), and potassium iodide (26.39 mg) were added to the intermediate 6c (170 mg). The reaction mixture was stirred at 70°C, filtered, concentrated, and purified by silica gel column chromatography (DCM:MeOH 20:1) to obtain the compound 1059 (91 mg, yield 53.22%). ¹H NMR (400 MHz, Chloroform-d) δ 4.11 (s, 6H), 3.51 (dt, J = 18.3, 5.6 Hz, 4H), 2.81 (dd, J = 10.3, 5.1 Hz, 2H), 2.50 (t, J = 6.0 Hz, 2H), 2.42-2.32 (m, 3H), 2.28 (s, 6H), 2.08-1.98 (m, 1H), 1.53-1.38 (m, 7H), 1.27 (s, 77H), 0.90 (t, J = 6.7 Hz, 18H).

### X. Compound 1112, synthesized according to the general synthetic route 3

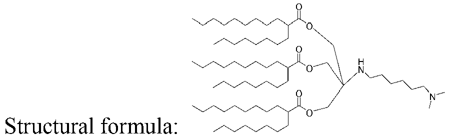

Chemical formula: C₆₉H₁₃₆N₂O₆
Molecular weight: 1089.85

The compound was synthesized according to the general synthetic route 3; the process was similar to that for the compound 1010, except that the intermediate C and 6-bromohexanal were used as the compound 2 and compound 5, respectively, in the general synthetic route 3. ¹H NMR (400 MHz, Chloroform-d) δ 4.11 (s, 6H), 3.11 (s, 1H), 2.57 (t, J = 6.8 Hz, 2H), 2.44-2.19 (m, 12H), 2.04 (q, J = 6.5 Hz, 2H), 1.53-1.38 (m, 13H), 1.27 (s, 92H), 0.90 (t, J = 6.7 Hz, 20H).

### Y. Compound 1113, synthesized according to the general synthetic route 4

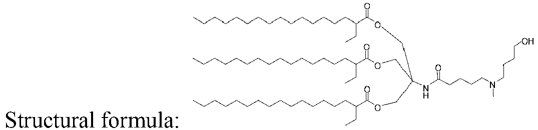

Chemical formula: C₇₁H₁₃₈N₂O₈
Molecular weight: 1147.89

### Steps 1 and 2: Synthesis of the compound 3 and compound 4 in the general synthetic route 4

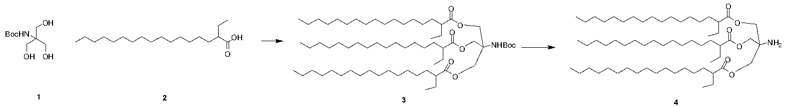

Followed Steps 1 and 2 in the general synthetic route 1; and the synthesis process was identical to that of the compound 1003.

### Step 3: Synthesis of the compound 6 in the general synthetic route 4

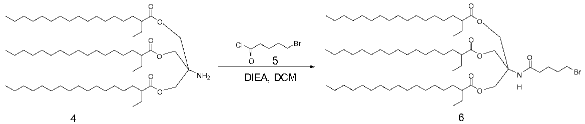

DIEA (0.30 g) and solvent DCM (20 mL) were added to the compound 4. Under nitrogen protection, the mixture was cooled to 0°C, then 5-bromovaleryl chloride (the compound 5, 0.27 g) was added. The mixture was stirred at 25°C for 2 hours, and diluted with 100 mL of DCM. The organic phase was washed twice with 100 mL of water, dried over anhydrous Na₂SO₄, filtered, and concentrated, and subjected to silica gel column chromatography (PE:EA 20:1) to obtain the compound 6 (800 mg).

### Step 4: Synthesis of the compound 8 in the general synthetic route 4

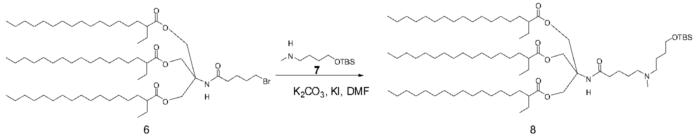

To a three-necked flask, Compound 6 (750.00 mg), the intermediate K (the compound 7, 217.31 mg), and the solvent DMF (20 mL) were added. Reactants K₂CO₃ (184.16 mg) and KI (110.60 mg) were added, and the mixture was stirred at 25°C for 16 hours. The temperature of mixture was raised to 100°C and stirring was carried out for 3 h. The reaction mixture was diluted with 200 mL of EA, then the organic phase was washed twice with 300 mL of saturated brine. The organic phase was dried over anhydrous Na₂SO₄, filtered, concentrated, added with silica gel, and purified by column chromatography (DCM:MeOH 20:1). The product fractions were collected and concentrated to obtain the compound 8 (700.00 mg).

### Step 5: Synthesis of the compound 1113

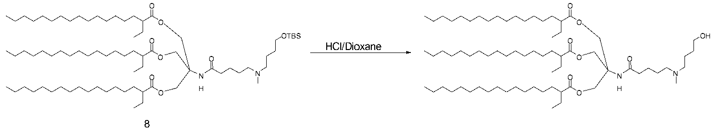

To a three-necked flask, the compound 8 (700.00 mg) and a solvent THF (5 mL) were added, then a solution of HCl in dioxane (1 M, 5.55 mL) was added. After the reaction mixture was stirred at 25°C for 2 hours, the mixture was diluted with 50 mL of EA. The organic phase was washed with a saturated NaHCO₃ solution and water, dried over anhydrous Na₂SO₄, filtered, and concentrated, purified by silica gel column chromatography (MeOH:DCM 1:20) to obtain the compound 1113 (201.1 mg, yield 31.59%). ¹H NMR (400 MHz, CDCl₃) δ 4.50-4.39 (m, 6H), 3.59 (t, J = 4.6 Hz, 2H), 2.49 (s, 3H), 2.35-2.26 (m, 6H), 2.14 (t, J = 7.5 Hz, 2H), 1.75-1.69 (m, 4H),1.66-1.43 (m,17H),1.27 (d,J = 3.7 Hz,78H),0.94-0.87 (m,18H).

### Z. Compound 1115, synthesized according to the general synthetic route 4

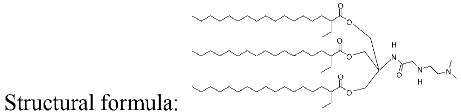

Chemical formula: C₆₇H₁₃₁N₃O₇
Molecular weight: 1090.80

The compound was synthesized according to the general synthetic route 4; the process was similar to that of the compound 1010, except that 2-bromoacetyl chloride and N,N-dimethylethylenediamine were used as the compound 5 and compound 7, respectively, as specified in the general synthetic route 4. ¹H NMR (400 MHz, CDCl₃) δ 7.42 (s, 1H), 4.48 (tt, J = 11.9, 5.7 Hz, 6H), 3.27 (s, 2H), 3.13 (s, 2H), 3.01 (s, 2H), 2.83 (s, 6H), 2.38-2.25 (m, 3H), 1.55 (dtt, J = 36.3, 14.1, 7.3 Hz, 12H), 1.27 (d, J = 3.3 Hz, 78H), 0.90 (td, J = 7.2, 3.4 Hz, 18H).

### AA. Compound 1114, synthesized according to the general synthetic route 5

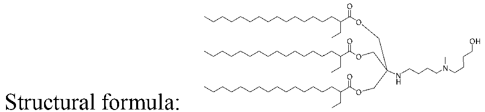

Chemical formula: C₇₀H₁₃₈N₂O₇
Molecular weight: 1119.88

### Steps 1 and 2: Synthesis of the compound 3 and compound 4 in the general synthetic route 5

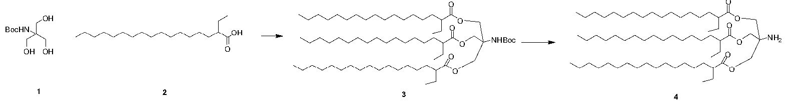

The synthesis was performed according to Steps 1 and 2 of the general synthetic route1; and the process was identical to that used for the synthesis of the compound 1003.

### Step 3: Synthesis of the compound 6 in the general synthetic route 5

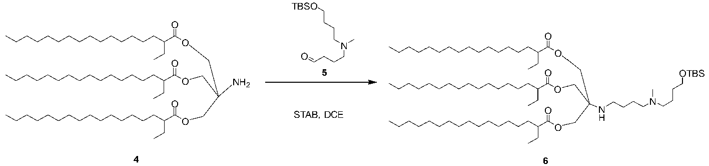

The compound 4 (500 mg), the intermediate M (the compound 5, 298.68 mg), a reactant STAB (328.68 mg), and DCE (10 mL) were added to a three-necked flask. The reaction mixture was stirred under N₂ at 25°C for 16 hours. Then, 100 mL of water was added, and the mixture was extracted twice with EA (100 mL). The organic phases were combined and concentrated. Silica gel was added, and the mixture was purified by column chromatography (DCM:MeOH = 20:1). The product fractions were collected and concentrated to obtain the compound 6 (500 mg).

### Step 4: Synthesis of the compound 1114

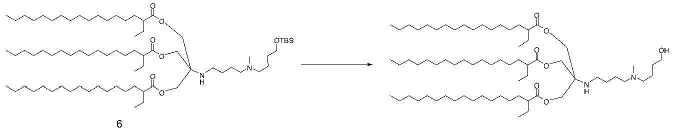

The compound 6 (450 mg) and THF (5 mL) were added to a three-necked flask, followed by the addition of a solution of HCl in dioxane (1 M, 3.65 mL). After stirring at 25°C for 2 hours, the mixture was diluted with 50 mL of EA. The organic phase was washed with 50 mL of a saturated NaHCO₃ solution and water, then dried over anhydrous Na₂ SO₄ . After filtration and concentration, the residue was purified by silica gel column chromatography (MeOH:DCM = 1:20) to obtain the compound 1114 (107 mg, yield 24.84%). ¹H NMR (400 MHz, CDCl₃ ) δ 4.16-4.07 (m, 6H), 3.60 (t, J = 4.8 Hz, 2H), 2.62 (t, J = 7.0 Hz, 2H), 2.49 (s, 3H), 2.31 (tt, J = 8.4, 5.5 Hz, 6H), 1.70 (d, J = 4.6 Hz, 3H), 1.64-1.39 (m, 18H), 1.27 (d, J = 4.0 Hz, 78H), 0.90 (t, J = 7.1 Hz, 18H).

### Z. Compound 1120, synthesized according to the general synthetic route 5

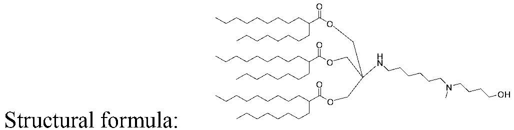

Chemical formula: C₇₂H₁₄₂N₂O₇
Molecular weight: 1147.90

The compound was synthesized according to the general synthetic route 5; the process was similar to that for the compound 1114, except that the intermediate C and 6-bromohexanal were used as the compound 2 and compound 5, respectively, as specified in the general synthetic route 5. ¹H NMR (400 MHz, Chloroform-d) δ 4.11 (s, 6H), 3.63 (s, 2H), 2.57 (t, J = 6.8 Hz, 4H), 2.37 (ddd, J = 8.5, 7.1, 4.3 Hz, 5H), 1.67-1.41 (m, 24H), 1.27 (s, 79H), 0.90 (t, J = 6.7 Hz, 18H).

### (2) Preparation Example 2: Synthesis of the second ionizable lipid

### 1. Preparation of the compound 6001

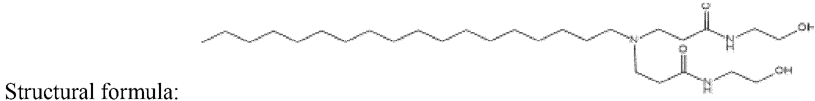

Molecular weight: 499.43
Oleylamine (0.8 g) and hydroxyethyl acrylamide (0.8 g) were added to 6.4 mL of ethanol, and the mixture was stirred and heated to 70°C. After 3.5 h, an additional 0.8 g of hydroxyethyl acrylamide was added. The reaction was continued for 16 h, followed by purification by a reverse-phase column to obtain the compound 6002.

The compound 6002 (0.7 g), Pd/C (0.4 g), and tetrahydrofuran (28 mL) were added to a reaction flask and stirred. The system was purged with hydrogen and maintained under a hydrogen atmosphere using a hydrogen balloon. After 16 hours, the mixture was filtered and purified by normal-phase column chromatography (methanol-DCM system) to obtain 200 mg of the compound 6001. ¹H NMR (400 MHz, CDCl₃) δ 7.54 (t, J = 5.5 Hz, 2H), 3.79-3.68 (m, 4H), 3.42 (dd, J = 10.0, 5.4 Hz, 4H), 2.89 (t, J = 6.0 Hz, 4H), 2.55 (dt, J = 11.8, 6.8 Hz, 6H), 1.54 (s, 2H), 1.27 (d, J = 11.2 Hz, 32H), 0.91 (t, J = 6.8 Hz, 3H).

### 2. Preparation of the compound 6002

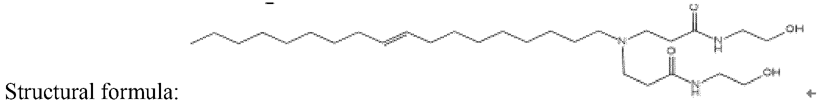

Molecular weight: 497.42
Oleylamine (0.8 g) and hydroxyethyl acrylamide (0.8 g) were added to ethanol (6.4 mL) and heated under stirring to 70°C. After 3.5 hours, an additional 0.8 g of hydroxyethyl acrylamide was added. The reaction was continued for 16 hours, after which the organic solvent was concentrated. The product was purified by reverse-phase column chromatography and lyophilized to obtain 400 mg of the compound 6002. ¹H NMR (400 MHz, CDCl₃) δ 7.31 (s, 2H), 5.38 (dd, J = 13.0, 7.4 Hz, 2H), 3.79-3.66 (m, 4H), 3.41 (dd, J = 10.1, 5.4 Hz, 4H), 2.82-2.72 (m, 4H), 2.44 (dd, J = 15.1, 9.2 Hz, 6H), 2.10-1.99 (m, 4H), 1.46 (s, 2H), 1.29 (s, 24H), 0.90 (t, J = 6.8 Hz, 3H).

### 3. Preparation of the compound 6003

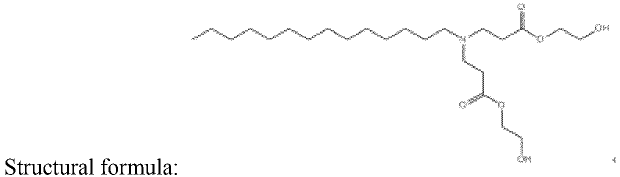

Molecular weight: 445.34
Tetradecylamine (4.00 g), 2-hydroxyethyl acrylate (6.53 g), and tert-butanol (80 mL) were added to a reaction flask; the mixture was stirred and heated to 70°C. After 29 hours, the organic solvent was concentrated, and the product was purified by reverse-phase column chromatography and lyophilized to obtain 1.5 g of the compound 6003. ¹H NMR (400 MHz, CDCl₃) δ 4.29 (t, J = 7.5 Hz, 4H), 3.89-3.75 (m, 4H), 2.81 (t, J = 6.2 Hz, 4H), 2.50 (ddd, J = 23.2, 14.1, 7.1 Hz, 6H), 1.46 (s, 2H), 1.28 (s, 24H), 0.91 (t, J = 6.3 Hz, 3H).

### 4. Preparation of the compound 6004

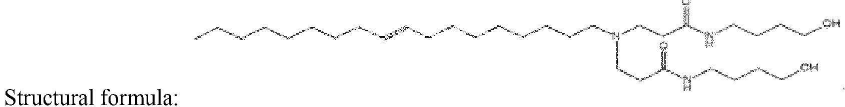

Molecular weight: 553.48
Oleylamine (0.8 g), n-butyl acrylate (1.5 g), and n-butanol (5.6 mL) were added to a reaction flask; the mixture was stirred and heated to 100°C. After 4 hours, an additional 1 mL of n-butyl acrylate was added, and the reaction was continued for 1 hour. The mixture was purified by normal-phase column chromatography (a petroleum ether-ethyl acetate system). Then, sodium hydroxide (0.4 g), water (1 mL), and methanol (10 mL) were added, and the mixture was stirred for 30 minutes to undergo hydrolysis. Concentrated hydrochloric acid (1 mL) and methanol (10 mL) were added to adjust the pH to neutral. The mixture was evaporated under reduced pressure, dissolved in DCM, dried over anhydrous magnesium sulfate, and filtered, and the solvent was removed under reduced pressure. DCM (10 mL), 4-amino-1-butanol (1.06 g), HOBT (0.81 g), and EDCI (2.3 g) were added, and the reaction proceeded at room temperature for 18 hours. After the organic solvent was concentrated, the product was purified by reverse-phase column chromatography and lyophilized to obtain 240 mg of the compound 6004. ¹H NMR (400 MHz, CDCl₃) δ 7.58 (d, J = 16.6 Hz, 2H), 5.44-5.31 (m, 2H), 3.72 (d, J = 16.5 Hz, 4H), 3.31 (d, J = 5.6 Hz, 4H), 3.17 (s, 4H), 2.84 (s, 2H), 2.69 (s, 4H), 2.09-2.01 (m, 4H), 1.67 (d, J = 2.7 Hz, 10H), 1.37-1.26 (m, 24H), 0.91 (t, J = 6.8 Hz, 3H).

### 5. Preparation of the compound 6005

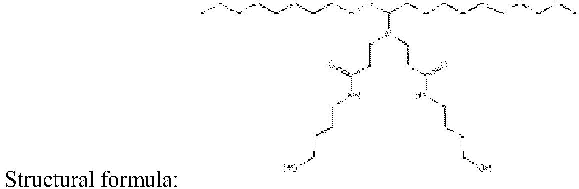

Molecular weight: 597.54

25.00 g of 11-heneicosanone, 62.05 g of ammonium acetate, and 500 mL of methanol were added to a reaction flask and stirred. Then, 6.55 g of sodium cyanoborohydride was added. After reacting for 16 hours, 250 mL of water and 250 mL of DCM were added. The phases were separated, and the aqueous phase was extracted with 50 mL of DCM. The organic phases were combined and purified by normal-phase column chromatography (a methanol-DCM system) to obtain 21.00 g of the compound 6005-A.

21.00 g of the compound 6005-A, 58 mL of n-butyl acrylate, and 100 mL of n-butanol latex were added to a reaction flask, stirred, and heated to 100°C. An additional 10 mL of n-butyl acrylate was added. After 16 hours, the mixture was purified by column chromatography (petroleum ether-ethyl acetate system) to obtain 31.00 g of the compound 6005-B.

A solution prepared from 31.00 g of the compound 6005-B, 6.55 g of sodium hydroxide, 310 mL of methanol, and 31 mL of water was added to a reaction flask and stirred. After 20 minutes, 150 mL of THF was added, and the mixture was stirred and heated to 50°C. After 30 minutes, a solution prepared from 16.13 g of concentrated hydrochloric acid and 160 mL of methanol was added, and the mixture was stirred. After 30 minutes, the solvent was removed under reduced pressure. The residue was dissolved in 300 mL of DCM, dried over anhydrous magnesium sulfate, filtered, and concentrated under reduced pressure to obtain 27.00 g of the compound 6005-C as an oily substance.

27.00 g of the compound 6005-C, 25.00 g of 4-amino-1-butanol, 41.85 g of EDCI, 14.74 g of HOBT, and 310 mL of DCM were added to a reaction flask and stirred. After 18 hours, the organic solvent was concentrated and treated by column chromatography (a methanol-DCM system) to remove excess 4-amino-1-butanol compound to obtain 6.80 g of the compound 6005. ¹H NMR (400 MHz, CDCl₃) δ 3.70 (t, J = 5.6 Hz, 4H), 3.28 (t, J = 5.6 Hz, 4H), 2.74 (t, J = 6.0 Hz, 4H), 2.40-2.45 (m, 1H), 2.34 (t, J = 6.4 Hz, 4H), 1.63-1.65 (m, 8H), 1.25-1.35 (m, 36H), 0.91 (t, J = 6.4 Hz, 6H).

### 6. Preparation of the compound 6006

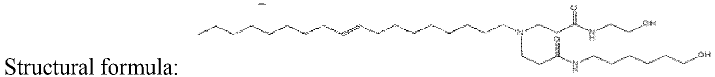

Molecular weight: 553.48

1.00 g of oleylamine, 0.47 g of hydroxyethyl acrylamide, and 8 mL of ethanol were added to a reaction flask; the mixture was stirred and heated to 70°C. After 13 hours, the solvent was removed under reduced pressure, and the residue was purified by column chromatography (methanol-DCM system) to obtain 800 mg of the intermediate 1. Then, 1.5 mL of n-butyl acrylate and 5 mL of n-butanol were added, and the mixture was stirred and heated to 100°C. After 3 hours, the solvent was removed under reduced pressure, and the residue was purified by column chromatography (methanol-DCM system). A solution prepared from 0.4 g of sodium hydroxide, 10 mL of methanol, and 1 mL of water was added, and the mixture was stirred for 1 hour; subsequently, a solution prepared from 1 mL of concentrated hydrochloric acid and 8 mL of methanol was added, and stirring was continued for 30 minutes. The solvent was removed under reduced pressure, and the residue was dissolved in 100 mL of DCM, dried over anhydrous magnesium sulfate, filtered, and concentrated. Finally, 0.45 g of 6-amino-1-hexanol, 0.27 g of HOBT, 0.78 g of EDCI, and 10 mL of DCM were added; the mixture was stirred for 14 hours, concentrated, and purified by column chromatography (methanol-DCM system) to obtain 300 mg of the compound 6006. ¹H NMR (400 MHz, CDCl₃) δ 7.83 (s, 1H), 6.95 (s, 1H), 5.43-5.28 (m, 2H), 3.74-3.67 (m, 2H), 3.64 (t, J = 6.3 Hz, 2H), 3.39 (dd, J = 10.0, 5.3 Hz, 2H), 3.24 (dd, J = 12.9, 6.7 Hz, 2H), 2.74 (t, J = 5.8 Hz, 4H), 2.48-2.42 (m, 2H), 2.41-2.33 (m, 4H), 2.01 (dd, J = 13.6, 6.8 Hz, 4H), 1.66-1.17 (m, 34H), 0.89 (t, J = 6.7 Hz, 3H).

### 7. Compound 6007

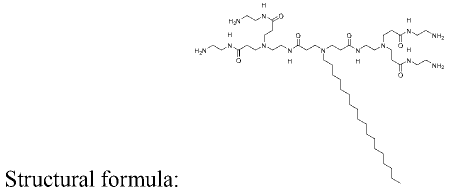

Molecular weight: 953.78

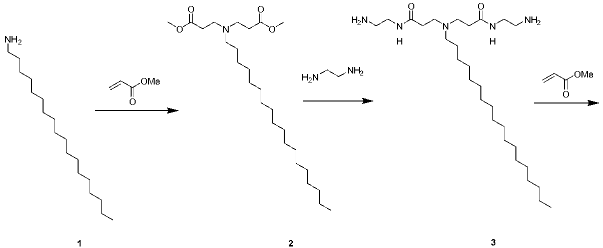

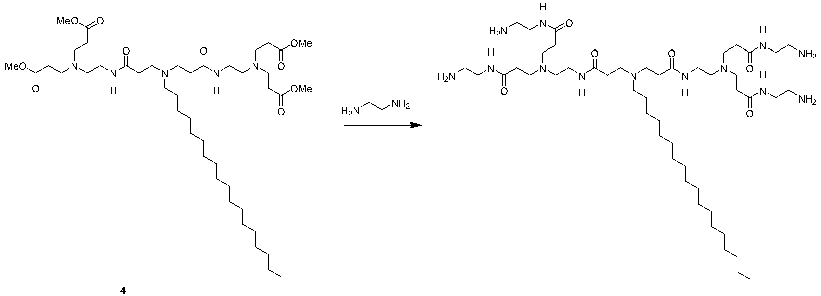

At room temperature, compound octadecylamine (50 g) was added to a three-necked flask, then MeOH (200 mL) was added and stirred for mixing. The mixture was cooled to 5°C in an ice-water bath, and methyl acrylate (31.5 g) was dropwise added slowly. After stirring and allowing the mixture to warm to room temperature, the reaction proceeded at room temperature for 4 hours. The mixture was concentrated under reduced pressure and purified by column chromatography (PE:EA = 20:1-10:1) to obtain the product 2 (68 g). At room temperature, Product 2 (15.0 g), MeOH (100 mL), and ethylenediamine (1.0 g) were added to a single-necked flask. The mixture was heated to 60°C and stirred at this temperature overnight. It was then concentrated under reduced pressure; 30 mL of toluene was added, and the mixture was heated to 60°C to dissolve the solids, slowly cooled to room temperature, and evaporated to dryness under reduced pressure to obtain the compound 3 (15 g). At room temperature, the compound 3 (2 g) and MeOH (20 mL) were added to a single-necked flask and stirred until dissolved. Methyl acrylate (2.5 g) was then slowly added, and the mixture was stirred and heated to 60°C overnight. The reaction was stopped, and the reaction liquid was concentrated under reduced pressure and purified by column chromatography (DCM:MeOH = 50:1-20:1) to obtain the compound 4 (2.5 g). At room temperature, the compound 4 (2.3 g) and ethylenediamine (20 mL) were added to a single-necked flask, and the reaction proceeded overnight at room temperature. The reaction was stopped, and the mixture was concentrated directly under reduced pressure to obtain the compound 6007 (24 mg). ¹H NMR (400 MHz, Methanol-d4) δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz,4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 8. Compounds 6008-6018

### Synthesis of the compound 6009

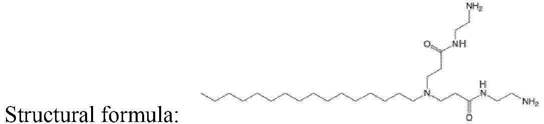

At room temperature and under a nitrogen atmosphere, a solution of freshly recrystallized hexadecylamine (0.03 mol) in methanol (20 mL) was added dropwise to a stirred solution of methyl methacrylate (6 mL) in methanol (20 mL), and the reaction was allowed to proceed overnight. The reaction mixture was concentrated to dryness under reduced pressure at room temperature; the residue was dissolved in chloroform and washed twice with 0.1 M NaOH solution. The chloroform solution was collected and dried over anhydrous calcium chloride, and separated by column chromatography to obtain a colorless oily substance. ¹H-NMR (300 MHz, CDCl₃): 0.78 (t, 3H), 1.16 (s, 30H), 2.38 (m, 6H), 2.71 (t, 4H), 3.57 (m, 6H).

Next, a solution of the aforementioned colorless oily substance (11.05 g) in methanol (20 mL) was added to a solution of 1,2-diaminoethane (75 g) in methanol (100 mL) that was being vigorously stirred at room temperature. Upon completion of the addition, the mixture was stirred for an additional 24 hours at room temperature. The solvent was removed under reduced pressure, and the temperature was maintained at below 40°C. Excess 1,2-diaminoethane was removed using an azeotropic mixture of toluene and methanol (9:1). Residual toluene was removed via azeotropic distillation with methanol. A white powder (10.5 g) was finally obtained; the powder was subjected to repeated recrystallization with chloroform and cyclohexane to obtain a final white solid. ¹H-NMR (300 MHz, CDCl₃): 0.88 (t, 3H), 1.25 (s, 30H), 1.84 (s, 4H), 2.38 (m, 6H), 2.73 (m, 4H), 2.82 (m, 4H), 3.29 (m, 4H), 7.47 (s, 2H).

The preparation method for 6008 and 6009 differed only in the use of an equimolar amount of tetradecylamine instead of hexadecylamine. The compounds 6010-6017 were prepared following the method for 6009, substituting hexadecylamine with an equimolar amount of the corresponding Rₐ-NH₂.

The compound 6018 was prepared following the method for 6007, substituting octadecylamine with an equimolar amount of the corresponding Rₐ-NH₂.

### 9. Compound 6019

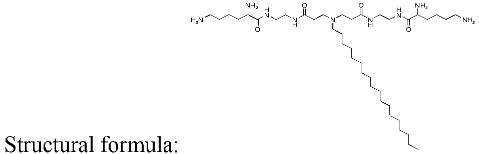

Molecular weight: 753.66

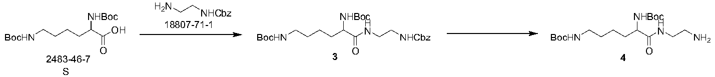

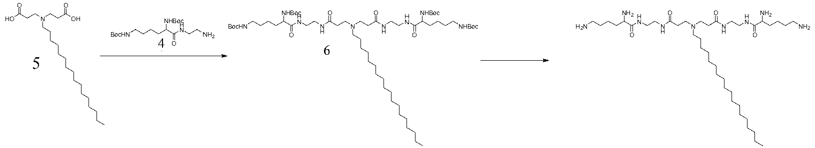

At room temperature, the compound 2483-46-7 (3 g) and DCM (50 mL) were added to a single-necked flask and stirred to mix; then, 18807-71-1 (2.5 g), DCC (2.7 g), and DMAP (1.6 g) were added. The mixture was stirred and allowed to react overnight at room temperature. After concentration under reduced pressure and purification by column chromatography (DCM:MeOH = 5:1), the product 3 (4 g) was obtained. At room temperature, the product 3 (5.0 g), MeOH (50 mL), and Pd/C (1.0 g) were added to a three-necked flask; the system was purged with hydrogen, heated to 50°C, and stirred for 1 hour. The reaction was stopped, and the mixture was cooled to room temperature and filtered. The filtrate was evaporated to dryness to obtain the product 4 (3.5 g). At room temperature, a starting material 5 (1.2 g) and DMF (50 mL) were added to a single-necked flask and stirred until dissolved. Subsequently, the product 4 (3.3 g), HATU (3.2 g), and TEA (0.9 g) were added; the mixture was stirred and allowed to react overnight at room temperature. Water (500 mL) and EA (500 mL) were added, and the mixture was subjected to phase separation and extraction. The organic phase was dried over Na₂SO₄ and concentrated under reduced pressure. The crude product was purified via column chromatography (DCM:MeOH = 20:1-10:1) to obtain the solid 6 (380 mg). At room temperature, the solid 6 (380 mg) and HCl/dioxane (4 M, 1.6 mL) were added to a single-necked flask, and the mixture was reacted for 2 hours at room temperature. After concentration under reduced pressure and purification, the compound 6019 (220 mg) was obtained. 1H NMR (400 MHz, Methanol-d4) δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz, 4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 10. Compound 6020

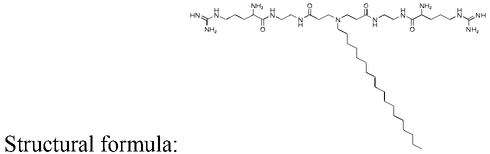

Molecular weight: 810.19

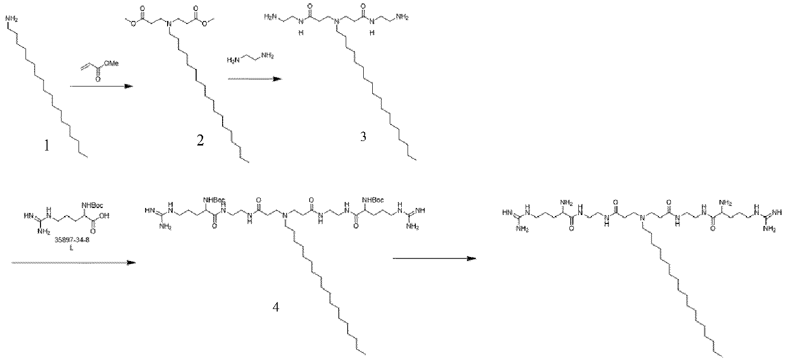

At room temperature, compound octadecylamine (50 g) was added to a three-necked flask, then MeOH (200 mL) was added and stirred for mixing. The mixture was cooled to 5°C in an ice-water bath, and methyl acrylate (31.5 g) was added dropwise. After stirring, the mixture was allowed to warm to room temperature and reacted for 4 hours. The reaction was stopped, concentrated directly under reduced pressure, and purified by column chromatography (PE:EA = 20:1-10:1) to obtain the product 2 (68 g). At room temperature, the product 2 (15.0 g), MeOH (100 mL), and ethylenediamine (1.0 g) were added to a single-necked flask. The mixture was heated to 60°C and reacted at this temperature overnight. The reaction was stopped, and the mixture was concentrated under reduced pressure. Toluene (30 mL) was added, and the mixture was heated to 60°C to dissolve the solids, slowly cooled to room temperature, filtered, and evaporated to dryness under reduced pressure to obtain the compound 3 (15 g). At room temperature, the compound 3 (1.3 g) and DMF (20 mL) were added to a single-necked flask and stirred. Then, CAS No. 35897-34-8 (3.6 g), EDCI (3.0 g), HOBt (2.1 g), and DIEA (2.0 g) were added. The mixture was stirred and allowed to react at room temperature overnight. Water (200 mL) was added, and the mixture was directly lyophilized under reduced pressure to obtain the compound 4 (4 g). At room temperature, the compound 4 (100 mg) and HCl/dioxane (4 M, 2 mL) were added to a single-necked flask, and the mixture was reacted at room temperature for 1 h. The reaction was stopped, and the mixture was concentrated under reduced pressure and purified to obtain the compound 6020. 1H NMR (400 MHz, Methanol-d4) δ 3.90 (t, J = 6.4 Hz, 2H), 3.57-3.34 (m, 10H), 3.24 (dt, J = 16.1, 8.0 Hz, 8H), 2.77 (t, J = 6.5 Hz, 4H), 2.03-1.58 (m, 10H), 1.31 (s, 30H), 1.01-0.82 (m, 3H).

### 11. Compound 6021

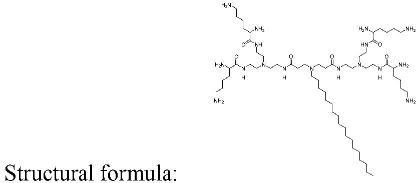

Molecular weight: 1182.79

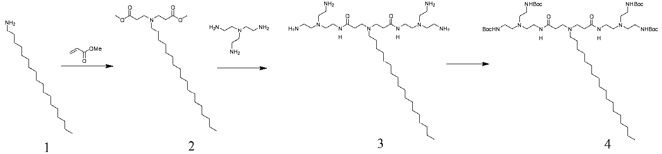

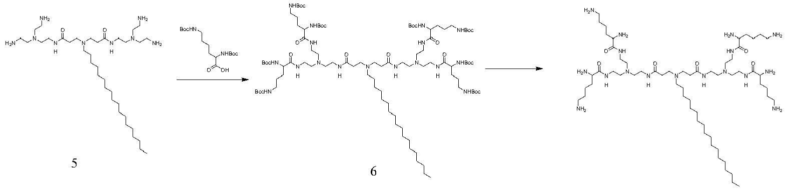

At room temperature, the compound 2 (3.5 g; synthesized using the same process as the product 2 for the compound 6020) was added to a single-necked flask, then MeOH (20 mL) was added and stirred for mixing. TREN (23.0 g) was added, and the mixture was stirred and heated to 60°C, and further stirred overnight. After concentration under reduced pressure, the crude product was lyophilized to obtain the product 3 (25 g). At room temperature, the product 3 (22.0 g) and DCM (200 mL) were added to a single-necked flask, and the mixture was cooled to 0°C. (Boc)₂ O (71.7 g) was added dropwise. Upon completion of the addition, the mixture was allowed to warm to room temperature and reacted for 3 hours. The reaction was quenched with water (200 mL), followed by extraction with DCM (100 mL). The organic phase was dried over sodium sulfate, concentrated under reduced pressure, and purified by column chromatography to obtain a yellow oily substance 4 (5.8 g). At room temperature, the yellow oily substance 4 (1.4 g) was added to a single-necked flask. HCl/dioxane (4 M, 15 mL) was slowly added under an ice-water bath while stirring. After the addition, the mixture was allowed to warm to room temperature and reacted for 0.5 hour. Concentration under reduced pressure to obtain the compound 5 (800 mg). At room temperature, the compound 5 (1.0 g), (S)-2,6-di-tert-butoxycarbonylaminohexanoic acid (2.6 g), EDCI (1.4 g), HOBt (1.0 g), DIEA (1.0 g), and DMF (10 mL) were added to a single-necked flask. The mixture was reacted overnight at room temperature. Water (100 mL) and EA (100 mL) were added, and the layers were separated and extracted. The EA phase was washed once with saturated brine (100 mL), concentrated under reduced pressure, and purified by column chromatography (DCM:MeOH = 10:1) to obtain the compound 6 (1.1 g). At room temperature, the compound 6 (1.2 g), HCl/dioxane (4 M, 10 mL), and a solvent DCM (10 mL) were added to a single-necked flask. The mixture was reacted overnight at room temperature, concentrated under reduced pressure, and purified to obtain the compound 6021 (220 mg). 1H NMR (400 MHz, Methanol-d4) δ 3.96 (t, J = 6.6 Hz, 4H), 3.75 (dt, J = 14.0, 6.6 Hz, 4H), 3.62-3.45 (m, 12H), 3.36-3.33 (m, 14H), 3.25-3.17 (m, 2H), 3.04-2.90 (m, 8H), 2.83 (t, J = 6.9 Hz, 4H), 2.05-1.82 (m, 8H), 1.73 (p, J = 7.7 Hz, 10H), 1.53 (qd, J = 8.3, 7.7, 4.3 Hz, 8H), 1.30 (s, 28H), 0.97-0.87 (m, 3H).

### 12. Compound 6023

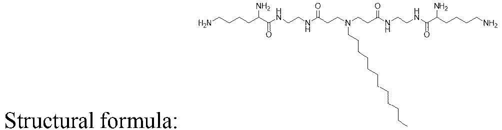

Molecular weight: 670

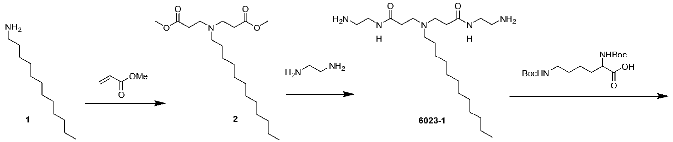

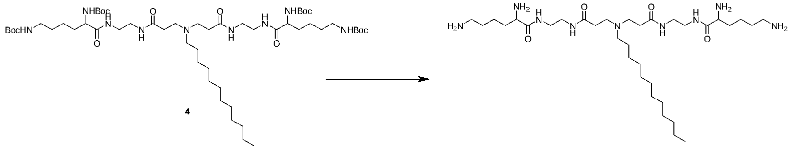

At room temperature, compound dodecylamine (10 g) and MeOH (100 mL) were added to a three-necked flask and stirred for mixing. The mixture was cooled to 5°C in an ice-water bath, and methyl acrylate (10.2 g) was slowly added dropwise. After stirring and allowing the mixture to warm to room temperature, the reaction liquid was reacted at room temperature for 4 hours before being stopped. The mixture was concentrated under reduced pressure and purified by column chromatography (PE:EA = 20:1-10:1) to obtain the product 2 (19 g). At room temperature, the product 2 (19.0 g), MeOH (200 mL), and ethylenediamine (127.7 g) were added to a single-necked flask. The mixture was heated to 60°C and reacted at this temperature overnight. Concentration under reduced pressure was carried out to obtain the compound 3 (20 g). A 500 mg of the compound 3 was purified and lyophilized to obtain the compound 6023-1 (218 mg). At room temperature, the starting material 6023-1 (1.4 g) and DCM (20 mL) were added to a single-necked flask and stirred until dissolved. Then, (S)-2,6-di-tert-butoxycarbonylaminohexanoic acid (3.4 g), EDCI (1.9 g), and DMAP (1.2 g) were added. The mixture was stirred and reacted at room temperature overnight, followed by phase separation, extraction, drying, and concentration under reduced pressure to obtain the compound 4 (3.0 g). At room temperature, the compound 4 (2.8 g) and HCl/1,4-dioxane (4 M, 20 mL) were added to a single-necked flask. The reaction proceeded at room temperature for 2 hours, followed by concentration under reduced pressure, purification, and lyophilization to obtain the compound 6023 (123 mg). 1H NMR (400 MHz, Methanol-d4) δ 3.88 (t, J = 6.6 Hz, 2H), 3.55-3.36 (m, 8H), 3.36-3.33 (m, 2H), 3.31-3.14 (m, 4H), 3.04-2.93 (m, 4H), 2.77 (t, J = 6.5 Hz, 4H), 2.00-1.82 (m, 4H), 1.82-1.66 (m, 6H), 1.50 (qd, J = 8.2, 7.8, 4.2 Hz, 4H), 1.45-1.25 (m, 18H), 0.96-0.87 (m, 3H).

### 13. Compound 6024

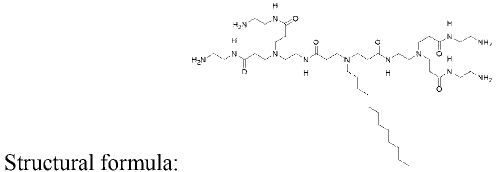

Molecular weight: 870.24

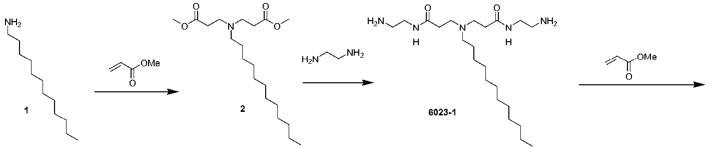

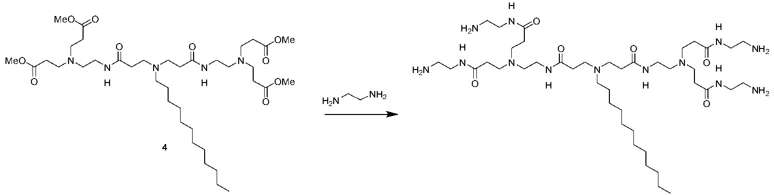

At room temperature, the product 6023-1 (1.4 g; synthesized using the same method as for 6023-1 in the compound 6023) was added to a single-necked flask, followed by addition of MeOH (20 mL); the mixture was stirred until dissolved. Methyl acrylate (2.8 g) was then slowly added, and the mixture was stirred and heated to 60°C. The reaction proceeded overnight at this temperature. Concentration under reduced pressure was carried out to obtain the compound 4 (2.0 g). At room temperature, the compound4 (2.4 g) and ethylenediamine (20 mL) were added to a single-necked flask, and the reaction was carried out overnight at room temperature. Concentration under reduced pressure and purification were carried out to obtain the compound6024 (191 mg). 1H NMR (400 MHz, Methanol-d4) δ 3.69 (t, J = 6.0 Hz, 4H), 3.60-3.48 (m, 20H), 3.42 (t, J = 6.1 Hz, 4H), 3.25-3.19 (m, 2H),3.12 (t, J = 5.8 Hz, 8H), 2.83 (t, J = 6.5 Hz, 12H), 1.78 (tt, J = 11.0, 6.4 Hz, 2H), 1.51-1.18 (m, 20H), 0.91 (t, J = 6.7 Hz, 3H).

### 14. Compound 6026

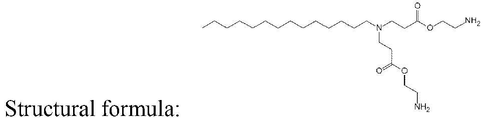

Molecular weight: 443.67

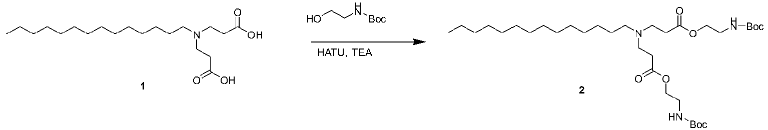

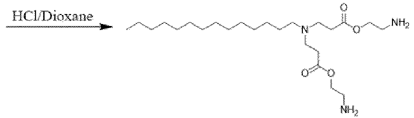

The compound6026 was synthesized following the synthetic route for the compound6019, with the following differences: an equimolar amount of the compound1 was used to replace the starting material 5 of the compound6019, and an equimolar amount of N-(tert-butoxycarbonyl)ethanolamine was used to replace the intermediate 4 of the compound6019. 1H NMR (300 MHz, DMSO) δ: 4.51 (t, J = 7.3 Hz, 4H), 3.76 (t, J = 6.1 Hz, 4H), 3.18 (t, J = 6.7 Hz, 4H), 3.01 (t, J = 5.8 Hz, 2H), 2.49 (t, J = 7.1 Hz, 4H), 1.36-1.26 (m, 24H), 0.89 (t, J = 6.2 Hz, 3H).

The compounds6025-6034 were obtained following the synthetic routes described previously for the aforementioned compounds; the differences were the use of hydroxyethylamine instead of the corresponding ethylenediamine, and the amino group of the hydroxyethylamine was protected first with a protecting group such as Fmoc or Boc. After the reaction was completed, the protecting groups were removed by using conventional methods.

### (3) Exemplary compounds of the first ionizable lipid and properties thereof

The exemplary compounds of the present application and properties thereof are listed in Table 1; they were synthesized according to the process route in Preparation Example 1.

The calculated c-pKa (molnetwork) and LogP (cLogP driver) values for the exemplary compounds of the present application were generated using the ChemDraw module of ChemOffice.

**Table 1 Properties of exemplary compounds of the first ionizable lipid**

| **Number** | **Molecular formula** | **Molecular weight** | **c-pKa** | **cLogP** | **Number** | **Molecular formula** | **Molecular weight** | **c-pKa** | **cLogP** |
|---|---|---|---|---|---|---|---|---|---|
| 1001 | C₅₉H₁₀₈N₂O₇ | 957.52 | 9.83 | 21.43 | 1201 | C₇₁H₁₄₃N₆O | 1102.01 | 9.01 | 28.48 |
| 1002 | C₆₀H₁₁₆N₂O₇ | 977.60 | 6.97 | 24.49 | 1202 | C₉₂H₁₉₀N₆O | 1396.57 | 9.01 | 39.59 |
| 1003 | C₆₃H₁₂₂N₂O₇ | 1019.6 8 | 6.97 | 26.07 | 1203 | C₆₅H₁₃₆N₆O | 1017.84 | 9.01 | 25.31 |
| 1004 | C₅₄H₁₀₄N₂O₇ | 893.43 | 6.97 | 21.97 | 1204 | C₆₅H₁₃₆N₆O | 1017.84 | 9.01 | 25.31 |
| 1005 | C₆₈H₁₃₃N₃O₇ | 1104.8 3 | 9.01 | 26.86 | 1205 | C₉₈H₂₀₂N₆O | 1480.73 | 9.01 | 43.03 |
| 1006 | C₅₃H₁₀₃N₃O₇ | 894.42 | 9.01 | 19.58 | 1206 | C₉₂H₁₉₀N₆O | 1396.57 | 9.01 | 39.86 |
| 1007 | C₆₈H₁₃₃N₃O₇ | 1104.83 | 9.01 | 26.86 | 1207 | C₈₃H₁₇₂N₆O | 1270.33 | 9.01 | 35.10 |
| 1008 | C₆₈H₁₃₃N₃O₇ | 1104.01 | 9.01 | 26.86 | 1208 | C₆₅H₁₃₀N₆O | 1011.80 | 9.01 | 24.06 |
| 1009 | C₆₂H₁₂₁N₃O₇ | 1020.66 | 9.01 | 23.69 | 1209 | C₆₅H₁₃₀N₆O | 1011.80 | 9.01 | 23.97 |
| 1010 | C₆₇H₁₃₃N₃O₆ | 1076.8 2 | 9.78 | 27.13 | 1210 | C₄₄H₉₄N₆O | 723.28 | 9.01 | 14.86 |
| 1011 | C₇₀H₁₃₇N₃O₇ | 1132.8 8 | 9.07 | 27.92 | 1211 | C₅₆H₁₀₆N₆O | 879.50 | 9.01 | 18.30 |
| 1012 | C₇₂H₁₄₁N₃O₇ | 1160.9 3 | 9.10 | 28.98 | 1212 | C₆₂H₁₁₈N₆O | 963.67 | 9.01 | 20.30 |
| 1013 | C₇₄H₁₄₅N₃O₇ | 1188.9 9 | 9.12 | 30.03 | 1213 | C₅₀H₁₀₀N₆O | 801.39 | 9.01 | 16.58 |
| 1014 | C₇₀H₁₃₇N₃O₉ | 1164.8 8 | 7.40 | 26.03 | 1214 | C₆₂H₁₂₄N₆O | 969.71 | 9.01 | 22.93 |
| 1015 | C₇₂H₁₄₁N₃O₉ | 1192.9 3 | 8.33 | 26.61 | 1215 | C₆₂H₁₃₀N₆O | 975.76 | 9.01 | 24.38 |
| 1016 | C₆₅H₁₂₉N₃O₈ | 1080.8 0 | 9.11 | 23.70 | 1216 | C₆₈H₁₁₈N₆O | 1035.73 | 9.01 | 25.13 |
| 1017 | C₇₁H₁₄₁N₃O₈ | 1164.9 0 | 9.10 | 26.87 | 1217 | C₈₃H₁₄₈N₆O | 1246.14 | 9.01 | 32.83 |
| 1018 | C₇₂H₁₄₀N₂O₁₀ | 1193.9 2 | 8.15 | 27.21 | 1218 | C₅₉H₁₂₁N₃OS₆ | 1081.00 | 9.01 | 27.15 |
| 1019 | C₇₂H₁₄₀N₂O₁₀ | 1193.9 2 | 8.15 | 27.21 | 1219 | C₅₉H₁₂₁N₃OS₆ | 1081.00 | 9.01 | 27.15 |
| 1020 | C₇₄H₁₄₅N₃O₉ | 1220.9 9 | 8.74 | 26.06 | 1220 | C₇₁H₁₄₅N₃OS₆ | 1249.32 | 9.01 | 33.50 |
| 1021 | C₇₄H₁₄₅N₃O₉ | 1221.0 0 | 8.74 | 26.06 | 1221 | C₉₂H₁₈₇N₃OS₆ | 1543.89 | 9.01 | 44.61 |
| 1022 | C₇₃H₁₄₅N₃O₈ | 1193.0 0 | 9.51 | 26.33 | 1222 | C₆₅H₁₃₃N₃OS₆ | 1165.16 | 9.01 | 30.32 |
| 1023 | C₇₄H₁₄₄N₂O₁₀ | 1221.9 7 | 8.55 | 26.66 | 1223 | C₁₀₀H₁₉₈N₄O₁₀S ₂ | 1680.82 | 8.90 | 43.24 |
| 1024 | C₇₄H₁₄₄N₂O₁₀ | 1221.9 7 | 8.55 | 26.66 | 1224 | C₉₈H₁₉₉N₃OS₆ | 1628.05 | 9.01 | 48.05 |
| 1025 | C₆₉H₁₃₅N₃O₈ | 1134.8 5 | 8.20 | 26.44 | 1225 | C₉₂H₁₈₇N₃OS₆ | 1543.89 | 9.01 | 44.88 |
| 1026 | C₆₉H₁₃₄N₂O₉ | 1135.8 4 | 8.02 | 27.05 | 1226 | C₈₃H₁₆₉N₃OS₆ | 1417.64 | 9.01 | 40.11 |
| 1027 | C₆₉H₁₃₄N₂O₉ | 1135.8 4 | 8.02 | 27.05 | 1227 | C₆₅H₁₂₇N₃OS₆ | 1159.11 | 9.01 | 29.08 |
| 1028 | C₆₄H₁₂₅N₃O₇ | 1048.7 0 | 9.07 | 24.74 | 1228 | C₆₅H₁₂₇N₃OS₆ | 1159.11 | 9.01 | 28.99 |
| 1029 | C₆₈H₁₃₂N₂O₈ | 1105.8 1 | 8.83 | 27.46 | 1229 | C₄₄H₉₁N₃OS₆ | 870.59 | 9.01 | 19.87 |
| 1030 | C₆₆H₁₂₉N₃O₇ | 1076.8 0 | 9.10 | 25.80 | 1230 | C₅₆H₁₀₃N₃OS₆ | 1026.82 | 9.01 | 23.32 |
| 1031 | C₆₅H₁₂₉N₃O₆ | 1048.8 0 | 9.86 | 26.07 | 1231 | C₆₂H₁₁₅N₃OS₆ | 1110.98 | 9.01 | 25.32 |
| 1032 | C₆₉H₁₃₅N₃O₇ | 1118.9 0 | 9.10 | 27.39 | 1232 | C₅₀H₉₇N₃OS₆ | 948.71 | 9.01 | 21.60 |
| 1033 | C₆₆H₁₂₈N₂O₈ | 1077.8 0 | 8.91 | 26.41 | 1233 | C₆₂H₁₂₁N₃OS₆ | 1117.03 | 9.01 | 27.94 |
| 1034 | C₆₈H₁₃₃N₃O₇ | 1104.8 3 | 9.12 | 26.86 | 1234 | C₆₂H₁₂₇N₃OS₆ | 1123.08 | 9.01 | 29.40 |
| 1035 | C₇₁H₁₃₉N₃O₇ | 1146.9 1 | 9.12 | 28.45 | 1235 | C₆₈H₁₁₅N₃OS₆ | 1183.05 | 9.01 | 27.86 |
| 1036 | C₆₈H₁₃₂N₂O₈ | 1105.8 0 | 8.93 | 27.46 | 1236 | C₈₃H₁₄₅N₃OS₆ | 1393.45 | 9.01 | 35.56 |
| 1037 | C₆₄H₁₂₅N₃O₉ | 1080.7 0 | 7.40 | 22.85 | 1237 | C₆₂H₁₂₁N₃O₄ | 972.67 | 9.01 | 23.48 |
| 1038 | C₆₇H₁₃₁N₃O₉ | 1122.8 0 | 7.40 | 24.44 | 1238 | C₇₄H₁₄₅N₃O₄ | 1140.99 | 9.01 | 29.83 |
| 1039 | C₆₄H₁₂₄N₂O₁₀ | 1081.7 0 | 7.21 | 23.46 | 1239 | C₉₅H₁₈₇N₃O₄ | 1435.56 | 9.01 | 40.94 |
| 1040 | C₆₉H₁₃₅N₃O₉ | 1150.9 0 | 8.33 | 25.02 | 1240 | C₆₈H₁₃₃N₃O₄ | 1056.83 | 9.01 | 26.65 |
| 1041 | C₆₆H₁₂₈N₂O₁₀ | 1109.8 0 | 8.15 | 24.04 | 1241 | C₆₈H₁₃₃N₃O₄ | 1056.83 | 9.01 | 26.65 |
| 1042 | C₆₈H₁₃₂N₂O₁₀ | 1137.8 0 | 8.55 | 23.49 | 1242 | C₁₀₁H₁₉₉N₃O₄ | 1519.72 | 9.01 | 43.72 |
| 1043 | C₇₀H₁₃₈N₄O₈ | 1163.8 9 | 9.40 | 25.69 | 1243 | C₉₅H₁₈₇N₃O₄ | 1435.56 | 9.01 | 40.55 |
| 1044 | C₆₄H₁₂₆N₄O₈ | 1079.7 3 | 9.40 | 22.52 | 1244 | C₈₆H₁₆₉N₃O₄ | 1309.32 | 9.01 | 35.79 |
| 1045 | C₇₀H₁₃₈N₄O₈ | 1163.8 9 | 9.40 | 25.69 | 1245 | C₆₈H₁₂₇N₃O₄ | 1050.78 | 9.01 | 24.75 |
| 1046 | C₇₀H₁₃₈N₄O₈ | 1163.89 | 9.40 | 25.69 | 1246 | C₆₈H₁₂₇N₃O₄ | 1050.78 | 9.01 | 24.66 |
| 1047 | C₆₇H₁₃₁N₃O₈ | 1106.8 0 | 8.95 | 25.14 | 1247 | C₄₇H₉₁N₃O₄ | 762.26 | 9.01 | 15.54 |
| 1048 | C₆₁H₁₁₉N₃O₈ | 1022.6 4 | 8.95 | 21.96 | 1248 | C₅₉H₁₀₃N₃O₄ | 918.49 | 9.01 | 18.99 |
| 1049 | C₆₇H₁₃₁N₃O₈ | 1106.8 0 | 8.95 | 25.14 | 1249 | C₆₅H₁₁₅N₃O₄ | 1002.65 | 9.01 | 20.99 |
| 1050 | C₇₂H₁₃₉NO₉ | 1162.9 0 | 9.10 | 28.52 | 1250 | C₅₃H₉₇N₃O₄ | 840.38 | 9.01 | 17.27 |
| 1051 | C₇₄H₁₃₉N₃O₁₃ | 1278.9 0 | 9.01 | 23.74 | 1251 | C₆₅H₁₂₁N₃O₄ | 1008.70 | 9.01 | 23.61 |
| 1052 | C₇₇H₁₅₀N₂O₇ | 1216.1 0 | 9.83 | 32.01 | 1252 | C₆₅H₁₂₇N₃O₄ | 1014.75 | 9.01 | 25.07 |
| 1053 | C₇₃H₁₃₇N₂O₆P | 1169.8 8 | 9.68 | 25.77 | 1253 | C₇₁H₁₁₅N₃O₄ | 1074.72 | 9.01 | 25.00 |
| 1054 | C₇₄H₁₃₈N₃O₇P | 1212.9 1 | 9.95 | 26.18 | 1254 | C₈₆H₁₄₅N₃O₄ | 1285.12 | 9.01 | 32.69 |
| 1055 | C₇₁H₁₃₆N₃O₃PS ₉ | 1399.4 0 | 8.85 | 32.41 | 1255 | C₉₉H₁₉₅N₃O₃S₄ | 1603.91 | 8.68 | 43.96 |
| 1056 | C₆₈H₁₃₈N₂O₅ | 1063.9 0 | 9.70 | 26.03 | 1256 | C₇₄H₁₄₅N₃O₄ | 1188.99 | 9.01 | 30.03 |
| 1057 | C₇₀H₁₄₀N₄O₆S₂ | 1198.0 3 | 9.67 | 24.35 | 1257 | C₉₅H₁₈₇N₃O₇ | 1483.55 | 9.01 | 41.14 |
| 1058 | C₆₇H₁₃₂N₂O₇ | 1077.8 0 | 9.45 | 27.29 | 1258 | C₁₀₄H₂₀₀N₃O₇P S₄ | 1763.97 | 7.94 | 41.36 |
| 1059 | C₆₇H₁₃₂N₂O₇ | 1077.8 0 | 9.45 | 27.29 | 1259 | C₁₀₃H₂₀₀N₂OS₇ | 1707.17 | 10.09 | 46.14 |
| 1060 | C₆₇H₁₃₃N₃O₈S | 1140.8 7 | 8.74 | 26.96 | 1260 | C₁₀₁H₁₉₀N₃O₇ | 1567.72 | 9.01 | 44.59 |
| 1061 | C₇₃H₁₂₅N₃O₅ | 1124.8 2 | 9.77 | 23.41 | 1261 | C₉₅H₁₈₇N₃O₇ | 1483.55 | 9.01 | 41.41 |
| 1062 | C₆₇H₁₁₃N₃O₁₀S₃ | 1216.8 3 | 9.73 | 22.69 | 1262 | C₈₆H₁₆₉N₃O₇ | 1357.31 | 9.01 | 36.65 |
| 1063 | C₆₅H₁₀₉N₃O₁₀S₃ | 1188.7 8 | 9.01 | 21.48 | 1263 | C₆₈H₁₂₇N₃O₇ | 1098.78 | 9.01 | 25.62 |
| 1064 | C₆₈H₁₃₉N₃OS₆ | 1207.2 4 | 9.01 | 32.18 | 1264 | C₆₈H₁₂₇N₃O₇ | 1098.78 | 9.01 | 25.53 |
| 1065 | C₆₈H₁₃₉N₃OS₆ | 1207.2 4 | 9.01 | 32.18 | 1265 | C₄₇H₉₁N₃O₇ | 810.26 | 9.01 | 16.41 |
| 1066 | C₇₄H₁₅₄N₃O₁₃P₃ | 1386.9 8 | 9.01 | 26.51 | 1266 | C₅₉H₁₀₃N₃O₇ | 966.49 | 9.01 | 19.85 |
| 1067 | C₇₄H₁₅₄N₃O₁₃P₃ | 1386.9 8 | 9.01 | 26.51 | 1267 | C₆₅H₁₁₅N₃O₇ | 1050.65 | 9.01 | 21.86 |
| 1068 | C₇₁H₁₃₁N₃O₇ | 1138.8 4 | 5.07 | 26.81 | 1268 | C₅₃H₉₇N₃O₇ | 888.37 | 9.01 | 18.13 |
| 1069 | C₇₁H₁₃₁N₃O₇ | 1138.8 4 | 5.04 | 26.81 | 1269 | C₆₅H₁₂₁N₃O₇ | 1056.70 | 9.01 | 24.48 |
| 1070 | C₆₉H₁₃₀N₄O₇ | 1127.8 2 | 5.03 | 26.38 | 1270 | C₆₅H₁₂₇N₃O₇ | 1062.74 | 9.01 | 25.93 |
| 1071 | C₆₉H₁₃₀N₄O₇ | 1127.8 2 | 5.00 | 26.38 | 1271 | C₇₁H₁₁₅N₃O₇ | 1122.71 | 9.01 | 26.17 |
| 1072 | C₇₀H₁₃₅N₃O₈ | 1146.8 6 | 6.83 | 26.82 | 1272 | C₈₆H₁₄₅N₃O₇ | 1333.12 | 9.01 | 33.87 |
| 1073 | C₇₀H₁₃₅N₃O₈ | 1146.8 6 | 6.83 | 26.82 | 1273 | C₆₂H₁₂₁N₃O₇ | 1020.66 | 9.01 | 23.69 |
| 1074 | C₇₁H₁₃₈N₄O₈ | 1175.9 1 | 7.89 | 26.41 | 1274 | C₇₄H₁₄₅N₃O₄ | 1188.99 | 9.01 | 30.03 |
| 1075 | C₇₁H₁₃₈N₄O₈ | 1175.9 1 | 7.89 | 26.41 | 1275 | C₉₅H₁₈₇N₃O₇ | 1483.55 | 9.01 | 41.14 |
| 1076 | C₇₁H₁₃₈N₄O₉ | 1191.9 0 | 7.80 | 26.25 | 1276 | C₆₈H₁₃₃N₃O₇ | 1104.83 | 9.01 | 26.86 |
| 1077 | C₇₁H₁₃₈N₄O₉ | 1191.9 0 | 7.80 | 26.25 | 1277 | C₆₈H₁₃₃N₃O₇ | 1104.83 | 9.01 | 26.86 |
| 1078 | C₇₁H₁₃₈N₄O₉ | 1191.9 0 | 7.75 | 27.09 | 1278 | C₁₀₁H₁₉₉N₃O₇ | 1567.72 | 9.01 | 44.59 |
| 1079 | C₇₁H₁₃₈N₄O₉ | 1191.9 0 | 7.75 | 27.09 | 1279 | C₉₅H₁₈₇N₃O₇ | 1483.55 | 9.01 | 41.41 |
| 1080 | C₇₀H₁₃₈N₄O₉S | 1211.9 5 | 7.61 | 26.56 | 1280 | C₈₆H₁₆₉N₃O₇ | 1357.31 | 9.01 | 36.65 |
| 1081 | C₇₀H₁₃₈N₄O₉S | 1211.9 5 | 7.61 | 26.56 | 1281 | C₆₈H₁₂₇N₃O₇ | 1098.78 | 9.01 | 25.62 |
| 1082 | C₇₂H₁₃₉N₅O₃ | 1202.9 3 | 7.82 | 26.46 | 1282 | C₆₈H₁₂₇N₃O₇ | 1098.78 | 9.01 | 25.53 |
| 1083 | C₇₂H₁₃₉N₅O₈ | 1202.93 | 7.82 | 26.46 | 1283 | C₄₇H₉₁N₃O₇ | 810.26 | 9.01 | 16.41 |
| 1084 | C₇₂H₁₃₇N₅O₇S | 1216.9 8 | 7.95 | 28.08 | 1284 | C₅₉H₁₀₃N₃O₇ | 966.49 | 9.01 | 19.85 |
| 1085 | C₇₂H₁₃₇N₅O₇S | 1216.9 8 | 7.95 | 28.08 | 1285 | C₆₅H₁₁₅N₃O₇ | 1050.65 | 9.01 | 21.86 |
| 1086 | C₇₄H₁₃₈N₈O₇ | 1251.9 7 | 5.88 | 25.00 | 1286 | C₅₃H₉₇N₃O₇ | 888.37 | 9.01 | 18.13 |
| 1087 | C₇₄H₁₃₈N₈O₇ | 1251.9 7 | 5.88 | 25.00 | 1287 | C₆₅H₁₂₁N₃O₇ | 1056.70 | 9.01 | 24.48 |
| 1088 | C₇₄H₁₃₈N₈O₈ | 1267.9 7 | 7.59 | 25.58 | 1288 | C₆₅H₁₂₇N₃O₇ | 1062.74 | 9.01 | 25.93 |
| 1089 | C₇₄H₁₃₈N₈O₈ | 1267.9 7 | 7.59 | 25.58 | 1289 | C₇₁H₁₁₅N₃O₇ | 1122.71 | 9.01 | 24.80 |
| 1090 | C₇₃H₁₃₇N₅O₉ | 1228.9 3 | 7.07 | 26.15 | 1290 | C₈₆H₁₄₅N₃O₇ | 1333.12 | 9.01 | 32.49 |
| 1091 | C₇₃H₁₃₇N₅O₉ | 1228.9 3 | 7.07 | 26.15 | 1291 | C₆₂H₁₂₁N₃O₁₀ | 1068.66 | 9.01 | 23.51 |
| 1092 | C₇₄H₁₃₉N₅O₈ | 1226.9 5 | 7.47 | 26.45 | 1292 | C₇₄H₁₄₅N₃O₁₀ | 1236.98 | 9.01 | 29.86 |
| 1093 | C₇₄H₁₃₉N₅O₈ | 1226.9 5 | 7.47 | 26.45 | 1293 | C₉₅H₁₈₇N₃O₁₀ | 1531.55 | 9.01 | 40.96 |
| 1094 | C₇₀H₁₃₈N₆O₇ | 1175.9 1 | 8.12 | 25.90 | 1294 | C₆₈H₁₃₃N₃O₁₀ | 1152.82 | 9.01 | 26.68 |
| 1095 | C₇₀H₁₃₈N₆O₇ | 1175.9 1 | 8.12 | 25.90 | 1295 | C₆₈H₁₃₃N₃O₁₀ | 1152.82 | 9.01 | 26.68 |
| 1096 | C₇₂H₁₄₂N₆O₉S | 1268.0 2 | 7.63 | 26.40 | 1296 | C₁₀₁H₁₉₉N₃O₁₀ | 1615.71 | 9.01 | 44.41 |
| 1097 | C₇₂H₁₄₂N₆O₉S | 1268.0 2 | 7.63 | 26.40 | 1297 | C₉₅H₁₈₇N₃O₁₀ | 1531.55 | 9.01 | 41.23 |
| 1098 | C₇₃H₁₄₃N₅O₉S | 1267.0 3 | 7.51 | 27.09 | 1298 | C₈₆H₁₆₉N₃O₁₀ | 1405.31 | 9.01 | 36.47 |
| 1099 | C₇₃H₁₄₃N₅O₉S | 1267.0 3 | 7.51 | 27.09 | 1299 | C₆₈H₁₂₇N₃O₁₀ | 1146.77 | 9.01 | 25.44 |
| 1100 | C₇₄H₁₄₅N₅O₉S | 1281.0 6 | 8.31 | 27.25 | 1300 | C₆₈H₁₂₇N₃O₁₀ | 1146.77 | 9.01 | 25.35 |
| 1101 | C₇₃H₁₄₃N₅O₉S | 1267.0 3 | 7.51 | 27.09 | 1301 | C₄₇H₉₁N₃O₁₀ | 858.26 | 9.01 | 16.23 |
| 1102 | C₇₂H₁₃₉N₃O₇ | 1158.9 2 | 10.26 | 27.41 | 1302 | C₅₉H₁₀₃N₃O₁₀ | 1014.48 | 9.01 | 19.68 |
| 1103 | C₇₁H₁₃₈N₄O₇ | 1159.9 1 | 9.78 | 26.04 | 1303 | C₆₅H₁₁₅N₃O₁₀ | 1098.65 | 9.01 | 21.68 |
| 1104 | C₆₉H₁₃₃N₃O₇ | 1116.8 4 | 9.32 | 25.52 | 1304 | C₅₃H₉₇N₃O₁₀ | 936.37 | 9.01 | 17.95 |
| 1105 | C₆₉H₁₃₃N₃O₇ | 1116.8 4 | 9.32 | 25.52 | 1305 | C₆₅H₁₂₁N₃O₁₀ | 1104.69 | 9.01 | 24.30 |
| 1106 | C₆₉H₁₃₆N₆O₇ | 1161.8 8 | 7.46 | 24.33 | 1306 | C₆₅H₁₂₇N₃O₁₀ | 1110.74 | 9.01 | 25.75 |
| 1107 | C₆₃H₁₂₄N₆O₇ | 1077.7 2 | 7.46 | 21.15 | 1307 | C₇₁H₁₁₅N₃O₁₀ | 1170.71 | 9.01 | 24.31 |
| 1108 | C₆₉H₁₃₆N₆O₇ | 1161.8 8 | 7.46 | 24.33 | 1308 | C₈₆H₁₄₅N₃O₁₀ | 1381.12 | 9.01 | 32.00 |
| 1109 | C₆₉H₁₃₆N₆O₇ | 1161.8 8 | 7.46 | 24.33 | 1309 | C₆₂H₁₂₄N₆O₄ | 1017.71 | 9.01 | 20.89 |
| 1110 | C₇₀H₁₃₆N₄O₇ | 1145.8 8 | 8.82 | 26.41 | 1310 | C₇₄H₁₄₈N₆O₄ | 1186.04 | 9.01 | 27.24 |
| 1111 | C₇₀H₁₃₆N₄O₇ | 1145.8 8 | 8.82 | 26.41 | 1311 | C₉₅H₁₉₀N₆O₄ | 1480.60 | 9.01 | 38.35 |
| 1112 | C₆₉H₁₃₆N₂O₆ | 1089.8 5 | 10.19 | 28.16 | 1312 | C₆₈H₁₃₆N₆O₄ | 1101.87 | 9.01 | 24.06 |
| 1113 | C₇₁H₁₃₈N₂O₈ | 1147.8 9 | 9.69 | 26.58 | 1313 | C₆₈H₁₃₆N₆O₄ | 1101.87 | 9.01 | 24.06 |
| 1114 | C₇₀H₁₃₈N₂O₇ | 1119.8 8 | 9.84 | 26.71 | 1314 | C₁₀₁H₂₀₂N₆O₄ | 1564.76 | 9.01 | 41.79 |
| 1115 | C₆₇H₁₃₁N₃O₇ | 1090.8 0 | 9.50 | 26.91 | 1315 | C₉₅H₁₉₀N₆O₄ | 1480.60 | 9.01 | 38.62 |
| 1116 | C₆₉H₁₃₅N₃O₈ | 1134.8 5 | 9.52 | 27.58 | 1316 | C₈₆H₁₇₂N₆O₄ | 1354.36 | 9.01 | 33.85 |
| 1117 | C₇₁H₁₄₁N₃O₆ | 1132.9 2 | 9.88 | 29.03 | 1317 | C₆₈H₁₃₀N₆O₄ | 1095.83 | 9.01 | 22.82 |
| 1118 | C₇₁H₁₃₉N₃O₈ | 1162.9 1 | 8.87 | 26.46 | 1318 | C₆₈H₁₃₀N₆O₄ | 1095.83 | 9.01 | 22.73 |
| 1119 | C₇₁H₁₃₃NO₉S₅ | 1305.1 4 | 9.96 | 25.12 | 1319 | C₄₇H₉₄N₆O₄ | 807.31 | 9.01 | 13.61 |
| 1120 | C₇₂H₁₄₂N₂O₇ | 1147.90 | 10.05 | 27.76 | 1320 | C₅₉H₁₀₆N₆O₄ | 963.53 | 9.01 | 17.06 |
| 1121 | C₇₃H₁₃₉N₅O₁₅S₄ | 1455.1 7 | 7.80 | 23.62 | 1321 | C₅₉H₁₀₆N₆O₄ | 963.53 | 9.01 | 17.06 |
| 1122 | C₆₈H₁₂₉NO₁₃S₃ | 1264.9 5 | 8.49 | 25.91 | 1322 | C₅₃H₁₀₀N₆O₄ | 885.42 | 9.01 | 15.34 |
| 1123 | C₇₇H₁₅₂N₂O₇ | 1218.0 7 | 9.99 | 30.68 | 1323 | C₆₅H₁₂₄N₆O₄ | 1053.74 | 9.01 | 21.68 |
| 1124 | C₆₇H₁₃₃N₃O₈S | 1140.8 7 | 8.74 | 26.96 | 1324 | C₆₅H₁₃₀N₆O₄ | 1059.79 | 9.01 | 23.14 |
| 1125 | C₆₉H₁₃₆N₄O₇ | 1133.8 7 | 9.61 | 27.37 | 1325 | C₇₁H₁₁₈N₆O₄ | 1119.76 | 9.01 | 23.40 |
| 1126 | C₆₅H₁₂₁N₃O₇ | 1056.7 0 | 9.01 | 23.82 | 1326 | C₈₆H₁₄₈N₆O₄ | 1330.17 | 9.01 | 31.09 |
| 1127 | C₆₉H₁₃₆N₂O₉ | 1137.8 5 | 8.31 | 26.05 | 1327 | C₆₂H₁₂4N₆O₄ | 1017.71 | 9.01 | 20.89 |
| 1128 | C₇₀H₁₃₉N₂O₁₁P | 1215.8 6 | 7.77 | 26.53 | 1328 | C₇₄H₁₄₈N₆O₄ | 1186.04 | 9.01 | 27.24 |
| 1129 | C₆₈H₁₃₄N₂O₇S₂ | 1155.9 5 | 8.84 | 27.80 | 1329 | C₉₅H₁₉₀N₆O₄ | 1480.60 | 9.01 | 38.35 |
| 1130 | C₇₄H₁₅₄N₃O₁₃P₃ | 1386.9 8 | 9.01 | 26.51 | 1330 | C₆₈H₁₃₆N₆O₄ | 1101.87 | 9.01 | 24.06 |
| 1131 | C₈₄H₁₅₉N₃O₈ | 1339.2 1 | 9.46 | 31.70 | 1331 | C₆₈H₁₃₆N₆O₄ | 1101.87 | 9.01 | 24.06 |
| 1132 | C₈₇H₁₄₇N₃O₈ | 1363.1 5 | 9.46 | 32.11 | 1332 | C₁₀₁H₂₀₁N₅O₅ | 1565.75 | 9.01 | 42.72 |
| 1133 | C₇₁H₁₃₁N₃O₇ | 1138.8 4 | 5.07 | 26.81 | 1333 | C₉₅H₁₈₉N₅O₅ | 1481.59 | 9.01 | 39.55 |
| 1134 | C₆₉H₁₃₀N₄O₇ | 1127.8 2 | 5.00 | 26.38 | 1334 | C₈₆H₁₇₁N₅O₅ | 1355.34 | 9.01 | 34.79 |
| 1135 | C₇₁H₁₃₈N₄O₈ | 1175.9 1 | 7.89 | 26.41 | 1335 | C₆₈H₁₃₀N₆O₄ | 1095.83 | 9.01 | 22.82 |
| 1136 | C₁₀₈H₂₀₄N₂O₉S₂ | 1738.9 4 | 8.56 | 46.05 | 1336 | C₆₈H₁₂₉N₅O₅ | 1096.81 | 9.01 | 23.66 |
| 1137 | C₉₉H₁₉₅NO₆S₃ | 1591.8 3 | 7.68 | 42.68 | 1337 | C₄₇H₉₃N₅O₅ | 808.29 | 9.01 | 14.55 |
| 1138 | C₇₂H₁₃₇N₅O₇S | 1216.9 8 | 7.95 | 28.08 | 1338 | C₅₉H₁₀₅N₅O₅ | 964.52 | 9.01 | 17.99 |
| 1139 | C₇₄H₁₃₈N₈O₇ | 1251.9 7 | 5.88 | 25.00 | 1339 | C₆₅H₁₁₇N₅O₅ | 1048.68 | 9.01 | 19.99 |
| 1140 | C₇₀H₁₃₈N₆O₇ | 1175.9 1 | 8.12 | 25.90 | 1340 | C₅₃H₉₉N₅O₅ | 886.40 | 9.01 | 16.27 |
| 1141 | C₇₃H₁₄₃N₅O₉S | 1267.0 3 | 7.51 | 27.09 | 1341 | C₆₅H₁₂₃N₅O₅ | 1054.73 | 9.01 | 22.62 |
| 1142 | C₇₀H₁₃₅N₃O₇ | 1130.8 6 | 9.98 | 26.32 | 1342 | C₆₅H₁₂₉N₅O₅ | 1060.78 | 9.01 | 24.07 |
| 1143 | C₆₅H₁₁₅N₃O₇ | 1050.6 0 | 9.01 | 21.20 | 1343 | C₇₁H₁₁₇N₅O₅ | 1120.75 | 9.01 | 24.31 |
| 1144 | C₇₅H₁₄₁₄N₃O₈ | 1212.9 7 | 9.18 | 28.86 | 1344 | C₈₆H₁₄₈N₆O₄ | 1330.17 | 9.01 | 31.76 |
| 1145 | C₂₀H₁₁₈N₂O₁₁S₃ | 1259.9 0 | 9.49 | 22.27 | 1345 | C₆₂H₁₂₄N₆O₇ | 1065.71 | 9.01 | 22.93 |
| 1146 | C₆₈H₁₃₄N₂O₇S₂ | 1155.9 5 | 9.14 | 27.39 | 1346 | C₇₄H₁₄₈N₆O₇ | 1234.03 | 9.01 | 29.28 |
| 1147 | C₈₆H₁₆₇N₃O₅S₂ | 1387.4 0 | 6.75 | 35.10 | 1347 | C₉₅H₁₉₀N₆O₇ | 1528.60 | 9.01 | 40.39 |
| 1148 | C₆₉H₁₂₀N₆O₂S₄ | 1194.0 0 | 8.63 | 24.08 | 1348 | C₆₈H₁₃₆N₆O₇ | 1149.87 | 9.01 | 26.10 |
| 1149 | C₇₆H₁₅₄N₆O₃S | 1232.2 0 | 7.65 | 30.39 | 1349 | C₆₈H₁₃₆N₆O₇ | 1149.87 | 9.01 | 26.10 |
| 1150 | C₉₁H₁₈₆N₄S₆ | 1528.9 0 | 9.77 | 43.45 | 1350 | C₁₀₁H₂₀₂N₆O₇ | 1612.76 | 9.01 | 43.83 |
| 1151 | C₇₈H₁₄₂N₆O₅ | 1244.0 0 | NA | 27.09 | 1351 | C₉₅H₁₉₀N₆O₇ | 1528.60 | 9.01 | 40.66 |
| 1152 | C₁₀₅H₁₈₁N₃O₁₂ | 1677.6 0 | 9.16 | 40.21 | 1352 | C₈₆H₁₇₂N₆O₇ | 1402.36 | 9.01 | 35.90 |
| 1153 | C₆₉H₁₃₁N₅O₅ | 1110.8 0 | 7.41 | 22.95 | 1353 | C₆₈H₁₃₀N₆O₇ | 1143.82 | 9.01 | 24.86 |
| 1154 | C₇₆H₁₃₉N₃S₃ | 1191.1 0 | 8.91 | 30.35 | 1354 | C₆₈H₁₃₀N₆O₇ | 1143.82 | 9.01 | 24.77 |
| 1155 | C₆₆H₁₃₂N₅O₁₀P | 1186.8 0 | 8.65 | 24.15 | 1355 | C₄₇H₉₄N₆O₇ | 855.30 | 9.01 | 15.66 |
| 1156 | C₇₀H₁₂₀N₇O₈P | 1218.7 0 | 9.55 | 21.96 | 1356 | C₅₉H₁₀₆N₆O₇ | 1011.53 | 9.01 | 19.10 |
| 1157 | C₇₂H₁₃₅N₅O₁₂S₃ | 1359.08 | 7.99 | 23.39 | 1357 | C₆₅H₁₁₈N₆O₇ | 1095.69 | 9.01 | 21.10 |
| 1158 | C₇₂H₁₃₀N₂O₉ | 1167.8 4 | 8.05 | 25.65 | 1358 | C₅₃H₁₀₀N₆O₇ | 933.42 | 9.01 | 17.38 |
| 1159 | C₇₁H₁₃₉N₃O₃S₆ | 1275.3 0 | 9.04 | 31.51 | 1359 | C₆₅H₁₂₄N₆O₇ | 1101.74 | 9.01 | 23.73 |
| 1160 | C₆₉H₁₃₈N₄O₉S₃ | 1264.1 0 | 7.48 | 28.35 | 1360 | C₆₅H₁₃₀N₆O₇ | 1107.79 | 9.01 | 25.18 |
| 1161 | C₇₃H₁₄₂N₂O₈ | 1175.9 0 | 9.39 | 27.24 | 1361 | C₇₁H₁₁₈N₆O₇ | 1167.76 | 9.01 | 24.18 |
| 1162 | C₈₅H₁₆₇N₅O₁₀ | 1419.3 0 | 9.49 | 27.91 | 1362 | C₈₆H₁₄₈N₆O₇ | 1378.16 | 9.01 | 31.87 |
| 1163 | C₅₉H₁₂₁N₃O₄ | 936.63 | 9.01 | 22.86 | 1363 | C₆₂H₁₂₄N₆O₇ | 1065.71 | 9.01 | 22.93 |
| 1164 | C₇₁H₁₄₅N₃O₄ | 1104.9 6 | 9.01 | 29.21 | 1364 | C₇₄H₁₄₈N₆O₇ | 1234.03 | 9.01 | 29.28 |
| 1165 | C₉₂H₁₈₇N₃O₄ | 1399.5 3 | 9.01 | 40.32 | 1365 | C₉₅H₁₉₀N₆O₇ | 1528.60 | 9.01 | 40.39 |
| 1166 | C₆₅H₁₃₃N₃O₄ | 1020.8 0 | 9.01 | 26.04 | 1366 | C₆₈H₁₃₆N₆O₇ | 1149.87 | 9.01 | 26.10 |
| 1167 | C₆₅H₁₃₃N₃O₄ | 1020.8 0 | 9.01 | 26.04 | 1367 | C₆₈H₁₃₆N₆O₇ | 1149.87 | 9.01 | 26.10 |
| 1168 | C₉₈H₁₉₉N₃O₄ | 1483.6 9 | 9.01 | 43.77 | 1368 | C₁₀₁H₂₀₂N₆O₇ | 1612.76 | 9.01 | 43.83 |
| 1169 | C₉₂H₁₈₇N₃O₄ | 1399.5 3 | 9.01 | 40.59 | 1369 | C₉₅H₁₉₀N₆O₇ | 1528.60 | 9.01 | 40.66 |
| 1170 | C₈₃H₁₆₉N₃O₄ | 1273.2 8 | 9.01 | 35.83 | 1370 | C₈₆H₁₇₂N₆O₇ | 1402.36 | 9.01 | 35.90 |
| 1171 | C₆₅H₁₂₇N₃O₄ | 1014.7 5 | 9.01 | 24.80 | 1371 | C₆₈H₁₃₀N₆O₇ | 1143.82 | 9.01 | 24.86 |
| 1172 | C₆₅H₁₂₇N₃O₄ | 1014.7 5 | 9.01 | 24.71 | 1372 | C₆₈H₁₃₀N₆O₇ | 1143.82 | 9.01 | 24.77 |
| 1173 | C₄₄H₉₁N₃O₄ | 726.23 | 9.01 | 15.59 | 1373 | C₄₇H₉₄N₆O₇ | 855.30 | 9.01 | 15.66 |
| 1174 | C₅₆H₁₀₃N₃O₄ | 882.46 | 9.01 | 19.03 | 1374 | C₅₉H₁₀₆N₆O₇ | 1011.53 | 9.01 | 19.10 |
| 1175 | C₆₂H₁₁₅N₃O₄ | 966.62 | 9.01 | 21.04 | 1375 | C₆₅H₁₁₈N₆O₇ | 1095.69 | 9.01 | 21.10 |
| 1176 | C₅₀H₉₇N₃O₄ | 804.34 | 9.01 | 17.31 | 1376 | C₅₃H₁₀₀N₆O₇ | 933.42 | 9.01 | 17.38 |
| 1177 | C₆₂H₁₂₁N₃O₄ | 972.67 | 9.01 | 23.66 | 1377 | C₆₅H₁₂₄N₆O₇ | 1101.74 | 9.01 | 23.73 |
| 1178 | C₆₂H₁₂₇N₃O₄ | 978.71 | 9.01 | 25.11 | 1378 | C₆₅H₁₃₀N₆O₇ | 1107.79 | 9.01 | 25.18 |
| 1179 | C₆₈H₁₁₅N₃O₄ | 1038.68 | 9.01 | 26.06 | 1379 | C₇₁H₁₁₈N₆O₇ | 1167.76 | 9.01 | 25.06 |
| 1180 | C₈₃H₁₄₅N₃O₄ | 1249.0 9 | 9.01 | 33.76 | 1380 | C₈₆H₁₄₈N₆O₇ | 1378.16 | 9.01 | 32.76 |
| 1181 | C₅₉H₁₂₁N₃OS₃ | 984.82 | 9.01 | 25.15 | 1381 | C₆₂H₁₂₇N₉O₄ | 1062.76 | 9.01 | 22.06 |
| 1182 | C₇₁H₁₄₅N₃OS₃ | 1153.1 4 | 9.01 | 31.50 | 1382 | C₇₄H₁₅₁N₉O₄ | 1231.08 | 9.01 | 28.41 |
| 1183 | C₉₂H₁₈₇N₃OS₃ | 1447.7 1 | 9.01 | 42.61 | 1383 | C₉₅H₁₉₃N₉O₄ | 1525.65 | 9.01 | 39.52 |
| 1184 | C₆₅H₁₃₃N₃OS₃ | 1068.9 8 | 9.01 | 28.32 | 1384 | C₆₈H₁₃₉N₉O₄ | 1146.92 | 9.01 | 25.24 |
| 1185 | C₆₅H₁₃₃N₃OS₃ | 1068.9 8 | 9.01 | 28.32 | 1385 | C₆₈H₁₃₉N₉O₄ | 1146.92 | 9.01 | 25.24 |
| 1186 | C₉₈H₁₉₉N₃OS₃ | 1531.8 7 | 9.01 | 46.05 | 1386 | C₁₀₁H₂₀₅N₉O₄ | 1609.81 | 9.01 | 42.97 |
| 1187 | C₉₂H₁₈₇N₃OS₃ | 1447.7 1 | 9.01 | 42.88 | 1387 | C₉₅H₁₉₃N₉O₄ | 1525.65 | 9.01 | 39.79 |
| 1188 | C₈₃H₁₆₉N₃OS₃ | 1321.4 6 | 9.01 | 38.12 | 1388 | C₈₆H₁₇₅N₉O₄ | 1399.41 | 9.01 | 35.03 |
| 1189 | C₆₅H₁₂₇N₃OS₃ | 1062.9 3 | 9.01 | 27.08 | 1389 | C₆₈H₁₃₃N₉O₄ | 1140.87 | 9.01 | 24.00 |
| 1190 | C₆₅H₁₂₇N₃OS₃ | 1062.9 3 | 9.01 | 26.99 | 1390 | C₆₈H₁₃₃N₉O₄ | 1140.87 | 9.01 | 23.91 |
| 1191 | C₄₄H₉₁N₃OS₃ | 774.41 | 9.01 | 17.88 | 1391 | C₄₇H₉₇N₉O₄ | 852.35 | 9.01 | 14.79 |
| 1192 | C₅₆H₁₀₃N₃OS₃ | 930.64 | 9.01 | 21.32 | 1392 | C₅₉H₁₀₉N₉O₄ | 1008.58 | 9.01 | 18.23 |
| 1193 | C₆₂H₁₁₅N₃OS₃ | 1014.8 0 | 9.01 | 23.32 | 1393 | C₆₅H₁₂₁N₉O₄ | 1092.74 | 9.01 | 20.24 |
| 1194 | C₅₀H₉₇N₃OS₃ | 852.53 | 9.01 | 19.60 | 1394 | C₅₃H₁₀₃N₉O₄ | 930.47 | 9.01 | 16.51 |
| 1195 | C₆₂H₁₂₁N₃OS₃ | 1020.8 5 | 9.01 | 25.95 | 1395 | C₆₅H₁₂₇N₉O₄ | 1098.79 | 9.01 | 22.86 |
| 1196 | C₆₂H₁₂₇N₃OS₃ | 1026.9 0 | 9.01 | 27.40 | 1396 | C₆₅H₁₃₃N₉O₄ | 1104.84 | 9.01 | 24.31 |
| 1197 | C₆₈H₁₁₅N₃OS₃ | 1086.8 7 | 9.01 | 27.48 | 1397 | C₇₁H₁₂₁N₉O₄ | 1164.81 | 9.01 | 23.93 |
| 1198 | C₈₃H₁₄₅N₃OS₃ | 1297.2 7 | 9.01 | 35.18 | 1398 | C₈₆H₁₅₁N₉O₄ | 1375.21 | 9.01 | 31.63 |
| 1199 | C₅₉H₁₂₄N₆O | 933.68 | 9.01 | 22.13 | 1399 | C₆₂H₁₂₁N₃OS₃ | 1020.85 | 9.01 | 24.00 |
| 1200 | C₅₉H₁₂₄N₆O | 933.68 | 9.01 | 22.13 | 1400 | C₇₄H₁₄₅N₃OS₃ | 1189.17 | 9.01 | 30.34 |
| 1401 | C₉₅H₁₈₇N₃OS₃ | 1483.7 4 | 9.01 | 41.45 | 1601 | C₆₅H₁₃₆N₆O₇S₃ | 1210.04 | 9.01 | 24.53 |
| 1402 | C₆₈H₁₃₃N₃OS₃ | 1105.0 1 | 9.01 | 27.17 | 1602 | C₆₅H₁₃₆N₆O₇S₃ | 1210.04 | 9.01 | 24.53 |
| 1403 | C₆₈H₁₃₃N₃OS₃ | 1105.0 1 | 9.01 | 27.17 | 1603 | C₉₈H₂₀₂N₆O₇S₃ | 1672.93 | 9.01 | 42.26 |
| 1404 | C₁₀₁H₁₉₉N₃OS₃ | 1567.9 0 | 9.01 | 44.90 | 1604 | C₉₂H₁₉₀N₆O₇S₃ | 1588.77 | 9.01 | 39.08 |
| 1405 | C₉₅H₁₈₇N₃OS₃ | 1483.7 4 | 9.01 | 41.72 | 1605 | C₈₃H₁₇₂N₆O₇S₃ | 1462.52 | 9.01 | 34.32 |
| 1406 | C₈₆H₁₆₉N₃OS₃ | 1357.5 0 | 9.01 | 36.96 | 1606 | C₆₅H₁₃₀N₆O₇S₃ | 1203.99 | 9.01 | 23.29 |
| 1407 | C₆₈H₁₂₇N₃OS₃ | 1098.9 6 | 9.01 | 25.93 | 1607 | C₆₅H₁₃₀N₆O₇S₃ | 1203.99 | 9.01 | 23.20 |
| 1408 | C₆₈H₁₂₇N₃OS₃ | 1098.9 6 | 9.01 | 25.84 | 1608 | C₄₄H₉₄N₆O₇S₃ | 915.47 | 9.01 | 14.08 |
| 1409 | C₄₇H₉₁N₃OS₃ | 810.44 | 9.01 | 16.72 | 1609 | C₅₆H₁₀₆N₆O₇S₃ | 1071.70 | 9.01 | 17.52 |
| 1410 | C₅₉H₁₀₃N₃OS₃ | 966.67 | 9.01 | 20.17 | 1610 | C₆₂H₁₁₈N₆O₇S₃ | 1155.86 | 9.01 | 19.53 |
| 1411 | C₆₅H₁₁₅N₃OS₃ | 1050.8 3 | 9.01 | 22.17 | 1611 | C₅₀H₁₀₀N₆O₇S₃ | 993.59 | 9.01 | 15.80 |
| 1412 | C₅₃H₉₇N₃OS₃ | 888.56 | 9.01 | 18.44 | 1612 | C₆₂H₁₂₄N₆O₇S₃ | 1161.91 | 9.01 | 22.15 |
| 1413 | C₆₅H₁₂₁N₃OS₃ | 1056.8 8 | 9.01 | 24.79 | 1613 | C₆₂H₁₃₀N₆O₇S₃ | 1167.96 | 9.01 | 23.60 |
| 1414 | C₆₅H₁₂₇N₃OS₃ | 1062.9 3 | 9.01 | 26.24 | 1614 | C₆₈H₁₁₈N₆O₇S₃ | 1227.93 | 9.01 | 23.60 |
| 1415 | C₇₁H₁₁₅N₃OS₃ | 1122.9 0 | 9.01 | 25.38 | 1615 | C₈₃H₁₄₈N₆O₇S₃ | 1438.33 | 9.01 | 31.29 |
| 1416 | C₈₆H₁₄₅N₃OS₃ | 1333.3 1 | 9.01 | 33.07 | 1616 | C₅₉H₁₂₁N₃O₁₀S₃ | 1128.83 | 9.01 | 22.22 |
| 1417 | C₆₂H₁₂₁N₃OS₆ | 1117.0 3 | 9.01 | 26.24 | 1617 | C₇₁H₁₄₅N₃O₁₀S₃ | 1297.15 | 9.01 | 28.57 |
| 1418 | C₇₄H₁₄₅N₃OS₆ | 1285.3 5 | 9.01 | 32.59 | 1618 | C₉₂H₁₈₇N₃O₁₀S₃ | 1591.72 | 9.01 | 39.68 |
| 1419 | C₉₅H₁₈₇N₃OS₆ | 1579.9 2 | 9.01 | 43.70 | 1619 | C₆₅H₁₃₃N₃O₁₀S₃ | 1212.99 | 9.01 | 25.39 |
| 1420 | C₆₈H₁₃₃N₃OS₆ | 1201.1 9 | 9.01 | 29.41 | 1620 | C₆₅H₁₃₃N₃O₁₀S₃ | 1212.99 | 9.01 | 25.39 |
| 1421 | C₆₈H₁₃₃N₃OS₆ | 1201.1 9 | 9.01 | 29.41 | 1621 | C₉₈H₁₉₉N₃O₁₀S₃ | 1675.88 | 9.01 | 43.12 |
| 1422 | C₁₀₁H₁₉₉N₃OS₆ | 1664.0 8 | 9.01 | 47.14 | 1622 | C₉₂H₁₈₇N₃O₁₀S₃ | 1591.72 | 9.01 | 39.95 |
| 1423 | C₉₅H₁₈₇N₃OS₆ | 1579.9 2 | 9.01 | 43.97 | 1623 | C₈₃H₁₆₉N₃O₁₀S₃ | 1465.48 | 9.01 | 35.19 |
| 1424 | C₈₆H₁₆₉N₃OS₆ | 1453.6 8 | 9.01 | 39.20 | 1624 | C₆₅H₁₂₇N₃O₁₀S₃ | 1206.94 | 9.01 | 24.15 |
| 1425 | C₆₈H₁₂₇N₃OS₆ | 1195.1 4 | 9.01 | 28.17 | 1625 | C₆₅H₁₂₇N₃O₁₀S₃ | 1206.94 | 9.01 | 24.06 |
| 1426 | C₆₈H₁₂₇N₃OS₆ | 1195.1 4 | 9.01 | 28.08 | 1626 | C₄₄H₉₁N₃O₁₀S₃ | 918.42 | 9.01 | 14.95 |
| 1427 | C₄₇H₉₁N₃OS₆ | 906.62 | 9.01 | 18.96 | 1627 | C₅₆H₁₀₃N₃O₁₀S₃ | 1074.65 | 9.01 | 18.39 |
| 1428 | C₅₉H₁₀₃N₃OS₆ | 1062.8 5 | 9.01 | 22.41 | 1628 | C₆₂H₁₁₅N₃O₁₀S₃ | 1158.81 | 9.01 | 20.39 |
| 1429 | C₆₅H₁₁₅N₃OS₆ | 1147.0 1 | 9.01 | 24.41 | 1629 | C₅₀H₉₇N₃O₁₀S₃ | 996.54 | 9.01 | 16.67 |
| 1430 | C₅₃H₉₇N₃OS₆ | 984.74 | 9.01 | 20.69 | 1630 | C₆₂H₁₂₁N₃O₁₀S₃ | 1164.86 | 9.01 | 23.02 |
| 1431 | C₆₅H₁₂₁N₃OS₆ | 1153.0 6 | 9.01 | 27.03 | 1631 | C₆₂H₁₂₇N₃O₁₀S₃ | 1170.91 | 9.01 | 24.47 |
| 1432 | C₆₅H₁₂₇N₃OS₆ | 1159.1 1 | 9.01 | 28.49 | 1632 | C₆₈H_{11S}N₃O₁₀S₃ | 1230.88 | 9.01 | 24.27 |
| 1433 | C₇₁H₁₁₅N₃O₄₈S₅ | 1203.0 2 | 9.01 | 26.60 | 1633 | C₈₃H₁₄₅N₃O₁₀S₃ | 1441.28 | 9.01 | 31.96 |
| 1434 | C₈₆H₁₄₅N₃OS₆ | 1429.4 9 | 9.01 | 34.45 | 1634 | C₅₉H₁₂₁N₃O₁₀S₃ | 1128.83 | 9.01 | 22.22 |
| 1435 | C₆₂H₁₂₁N₃OS₆ | 1117.0 3 | 9.01 | 26.24 | 1635 | C₇₁H₁₄₅N₃O₁₀S₃ | 1297.15 | 9.01 | 28.57 |
| 1436 | C₇₄H₁₄₅N₃OS₆ | 1285.3 5 | 9.01 | 32.59 | 1636 | C₉₂H₁₈₇N₃O₁₀S₃ | 1591.72 | 9.01 | 39.68 |
| 1437 | C₉₅H₁₈₇N₃OS₆ | 1579.9 2 | 9.01 | 43.70 | 1637 | C₆₅H₁₃₃N₃O₁₀S₃ | 1212.99 | 9.01 | 25.39 |
| 1438 | C₆₈H₁₃₃N₃OS₆ | 1201.1 9 | 9.01 | 29.41 | 1638 | C₆₅H₁₃₃N₃O₁₀S₃ | 1212.99 | 9.01 | 25.39 |
| 1439 | C₆₈H₁₃₃N₃OS₆ | 1201.1 9 | 9.01 | 29.41 | 1639 | C₉₈H₁₉₉N₃O₁₀S₃ | 1675.88 | 9.01 | 43.12 |
| 1440 | C₁₀₁H₁₉₉N₃OS₆ | 1664.08 | 9.01 | 47.14 | 1640 | C₉₂H₁₈₇N₃O₁₀S₃ | 1591.72 | 9.01 | 39.95 |
| 1441 | C₉₅H₁₈₇N₃OS₆ | 1579.9 2 | 9.01 | 43.97 | 1641 | C₈₃H₁₆₉N₃O₁₀S₃ | 1465.48 | 9.01 | 35.19 |
| 1442 | C₈₆H₁₆₉N₃OS₆ | 1453.6 8 | 9.01 | 39.20 | 1642 | C₆₅H₁₂₇N₃O₁₀S₃ | 1206.94 | 9.01 | 24.15 |
| 1443 | C₆₈H₁₂₇N₃OS₆ | 1195.1 4 | 9.01 | 28.17 | 1643 | C₆₅H₁₂₇N₃O₁₀S₃ | 1206.94 | 9.01 | 24.06 |
| 1444 | C₆₈H₁₂₇N₃OS₆ | 1195.1 4 | 9.01 | 28.08 | 1644 | C₄₄H₉₁N₃O₁₀S₃ | 918.42 | 9.01 | 14.95 |
| 1445 | C₄₇H₉₁N₃OS₆ | 906.62 | 9.01 | 18.96 | 1645 | C₅₆H₁₀₃N₃O₁₀S₃ | 1074.65 | 9.01 | 18.39 |
| 1446 | C₅₉H₁₀₃N₃OS₆ | 1062.8 5 | 9.01 | 22.41 | 1646 | C₆₂H₁₁₅N₃O₁₀S₃ | 1158.81 | 9.01 | 20.39 |
| 1447 | C₆₅H₁₁₅N₃OS₆ | 1147.0 1 | 9.01 | 24.41 | 1647 | C₅₀H₉₇N₃O₁₀S₃ | 996.54 | 9.01 | 16.67 |
| 1448 | C₅₃H₉₇N₃OS₆ | 984.74 | 9.01 | 20.69 | 1648 | C₆₂H₁₂₁N₃O₁₀S₃ | 1164.86 | 9.01 | 23.02 |
| 1449 | C₆₅H₁₂₁N₃OS₆ | 1153.0 6 | 9.01 | 27.03 | 1649 | C₆₂H₁₂₇N₃O₁₀S₃ | 1170.91 | 9.01 | 24.47 |
| 1450 | C₆₅H₁₂₇N₃OS₆ | 1159.1 1 | 9.01 | 28.49 | 1650 | C₆₈H₁₁₅N₃O₁₀S₃ | 1230.88 | 9.01 | 23.31 |
| 1451 | C₇₁H₁₁₅N₃OS₆ | 1219.0 8 | 9.01 | 27.44 | 1651 | C₈₃H₁₄₅N₃O₁₀S₃ | 1441.28 | 9.01 | 31.01 |
| 1452 | C₈₆H₁₄₅N₃OS₆ | 1429.4 9 | 9.01 | 35.13 | 1652 | C₆₀H₁₂₃N₃O₁₃S₃ | 1190.85 | 9.01 | 23.97 |
| 1453 | C₆₂H₁₂₁N₃O₄S₃ | 1068.8 5 | 9.01 | 25.13 | 1653 | C₇₂H₁₄₇N₃O₁₃S₃ | 1359.18 | 9.01 | 30.32 |
| 1454 | C₇₄H₁₄₅N₃O₄S₃ | 1237.1 7 | 9.01 | 31.48 | 1654 | C₉₃H₁₈₉N₃O₁₃S₃ | 1653.74 | 9.01 | 41.43 |
| 1455 | C₉₅H₁₈₇N₃O₄S₃ | 1531.7 4 | 9.01 | 42.59 | 1655 | C₆₆H₁₃₅N₃O₁₃S₃ | 1275.01 | 9.01 | 27.15 |
| 1456 | C₆₈H₁₃₃N₃O₄S₃ | 1153.0 1 | 9.01 | 28.30 | 1656 | C₆₆H₁₃₅N₃O₁₃S₃ | 1275.01 | 9.01 | 27.15 |
| 1457 | C₆₈H₁₃₃N₃O₄S₃ | 1153.0 1 | 9.01 | 28.30 | 1657 | C₉₉H₂₀₁N₃O₁₃S₃ | 1737.91 | 9.01 | 44.78 |
| 1458 | C₁₀₁H₁₉₉N₃O₄S₃ | 1615.9 0 | 9.01 | 46.03 | 1658 | C₉₃H₁₈₉N₃O₁₃S₃ | 1653.74 | 9.01 | 41.61 |
| 1459 | C₉₅H₁₈₇N₃O₄S₃ | 1531.7 4 | 9.01 | 42.86 | 1659 | C₈₄H₁₇₁N₃O₁₃S₃ | 1527.50 | 9.01 | 36.85 |
| 1460 | C₈₆H₁₆₉N₃O₄S₃ | 1405.5 0 | 9.01 | 38.09 | 1660 | C₆₅H₁₂₇N₃O₁₃S₃ | 1254.94 | 9.01 | 25.28 |
| 1461 | C₆₈H₁₂₇N₃O₄S₃ | 1146.9 6 | 9.01 | 27.06 | 1661 | C₆₅H₁₂₇N₃O₁₃S₃ | 1254.94 | 9.01 | 25.19 |
| 1462 | C₆₈H₁₂₇N₃O₄S₃ | 1146.9 6 | 9.01 | 26.97 | 1662 | C₄₅H₉₃N₃O₁₃S₃ | 980.45 | 9.01 | 16.61 |
| 1463 | C₄₇H₉₁N₃O₄S₃ | 858.44 | 9.01 | 17.85 | 1663 | C₅₆H₁₀₃N₃O₁₃S₃ | 1122.65 | 9.01 | 19.52 |
| 1464 | C₅₉H₁₀₃N₃O₄S₃ | 1014.6 7 | 9.01 | 21.30 | 1664 | C₆₂H₁₁₅N₃O₁₃S₃ | 1206.81 | 9.01 | 21.53 |
| 1465 | C₆₅H₁₁₅N₃O₄S₃ | 1098.8 3 | 9.01 | 23.30 | 1665 | C₅₀H₉₇N₃O₁₃S₃ | 1044.53 | 9.01 | 17.80 |
| 1466 | C₅₃H₉₇N₃O₄S₃ | 936.56 | 9.01 | 19.58 | 1666 | C₆₂H₁₂₁N₃O₁₃S₃ | 1212.86 | 9.01 | 24.15 |
| 1467 | C₆₅H₁₂₁N₃O₄S₃ | 1104.8 8 | 9.01 | 25.92 | 1667 | C₆₂H₁₂₇N₃O₁₃S₃ | 1218.91 | 9.01 | 25.60 |
| 1468 | C₆₅H₁₂₇N₃O₄S₃ | 1110.9 3 | 9.01 | 27.38 | 1668 | C₆₈H₁₁₅N₃O₁₃S₃ | 1278.88 | 9.01 | 25.80 |
| 1469 | C₇₁H₁₁₅N₃O₄S₃ | 1170.9 0 | 9.01 | 26.31 | 1669 | C₈₃H₁₄₅N₃O₁₃S₃ | 1489.28 | 9.01 | 33.49 |
| 1470 | C₈₆H_{14S}N₃O₄S₃ | 1381.3 0 | 9.01 | 34.00 | 1670 | C₅₉H₁₂₁N₃O₇S₃ | 1080.83 | 9.01 | 19.96 |
| 1471 | C₆₂H₁₂₁N₃O₄S₃ | 1068.8 5 | 9.01 | 25.13 | 1671 | C₅₉H₁₂₁N₃O₇S₃ | 1080.83 | 9.01 | 19.96 |
| 1472 | C₆₂H₁₂₁N₃O₄S₃ | 1068.8 5 | 9.01 | 25.13 | 1672 | C₇₁H₁₄₅N₃O₇S₃ | 1249.16 | 9.01 | 26.30 |
| 1473 | C₇₄H₁₄₅N₃O₄S₃ | 1237.1 7 | 9.01 | 31.48 | 1673 | C₉₂H₁₈₇N₃O₇S₃ | 1543.72 | 9.01 | 37.41 |
| 1474 | C₉₅H₁₈₇N₃O₄S₃ | 1531.7 4 | 9.01 | 42.59 | 1674 | C₆₅H₁₃₃N₃O₇S₃ | 1164.99 | 9.01 | 23.13 |
| 1475 | C₆₈H₁₃₃N₃O₄S₃ | 1153.0 1 | 9.01 | 28.30 | 1675 | C₆₅H₁₃₃N₃O₇S₃ | 1164.99 | 9.01 | 23.13 |
| 1476 | C₆₈H₁₃₃N₃O₄S₃ | 1153.0 1 | 9.01 | 28.30 | 1676 | C₉₈H₁₉₉N₃O₇S₃ | 1627.88 | 9.01 | 40.86 |
| 1477 | C₁₀₁H₁₉₉N₃O₄S₃ | 1615.9 0 | 9.01 | 46.03 | 1677 | C₉₂H₁₈₇N₃O₇S₃ | 1543.72 | 9.01 | 37.68 |
| 1478 | C₉₅H₁₈₇N₃O₄S₃ | 1531.7 4 | 9.01 | 42.86 | 1678 | C₈₃H₁₆₉N₃O₇S₃ | 1417.48 | 9.01 | 32.92 |
| 1479 | C₈₆H₁₆₉N₃O₄S₃ | 1405.50 | 9.01 | 38.09 | 1679 | C₆₅H₁₂₇N₃O₇S₃ | 1158.95 | 9.01 | 21.89 |
| 1480 | C₆₈H₁₂₇N₃O₄S₃ | 1146.9 6 | 9.01 | 27.06 | 1680 | C₆₅H₁₂₇N₃O₇S₃ | 1158.95 | 9.01 | 21.80 |
| 1481 | C₆₈H₁₂₇N₃O₆S | 1114.8 4 | 9.01 | 26.01 | 1681 | C₄₄H₉₁N₃O₇S₃ | 870.43 | 9.01 | 12.68 |
| 1482 | C₄₇H₉₁N₃O₄S₃ | 858.46 | 9.01 | 17.85 | 1682 | C₅₆H₁₀₃N₃O₇S₃ | 1026.65 | 9.01 | 16.12 |
| 1483 | C₅₉H₁₀₃N₃O₄S₃ | 1014.6 9 | 9.01 | 21.30 | 1683 | C₆₂H₁₁₅N₃O₇S₃ | 1110.82 | 9.01 | 18.13 |
| 1484 | C₆₅H₁₁₅N₃O₄S₃ | 1098.8 5 | 9.01 | 23.30 | 1684 | C₅₀H₉₇N₃O₇S₃ | 948.54 | 9.01 | 14.40 |
| 1485 | C₅₃H₉₇N₃O₄S₃ | 936.58 | 9.01 | 19.58 | 1685 | C₆₂H₁₂₁N₃O₇S₃ | 1116.86 | 9.01 | 20.75 |
| 1486 | C₆₅H₁₂₁N₃O₄S₃ | 1104.9 0 | 9.01 | 25.92 | 1686 | C₆₂H₁₂₇N₃O₇S₃ | 1122.91 | 9.01 | 22.20 |
| 1487 | C₆₅H₁₂₇N₃O₄S₃ | 1110.9 5 | 9.01 | 27.38 | 1687 | C₆₈H₁₁₅N₃O₇S₃ | 1182.88 | 9.01 | 22.60 |
| 1488 | C₇₁H₁₁₅N₃O₄S₃ | 1170.9 2 | 9.01 | 26.44 | 1688 | C₈₃H₁₄₅N₃O₇S₃ | 1393.29 | 9.01 | 30.30 |
| 1489 | C₈₆H₁₄₅N₃O₄S₃ | 1381.3 2 | 9.01 | 34.14 | 1689 | C₆₄H₁₃₂N₂O₄ | 993.77 | 9.45 | 26.47 |
| 1490 | C₆₂H₁₂₁N₃O₇S₃ | 1116.8 6 | 9.01 | 26.05 | 1690 | C₆₄H₁₃₂N₂O₄ | 993.77 | 9.45 | 26.47 |
| 1491 | C₇₄H₁₄₅N₃O₇S₃ | 1285.1 9 | 9.01 | 32.39 | 1691 | C₆₇H₁₃₂N₂O₇ | 1077.80 | 9.45 | 27.29 |
| 1492 | C₉₅H₁₈₇N₃O₇S₃ | 1579.7 6 | 9.01 | 43.50 | 1692 | C₆₇H₁₃₂N₂O₇ | 1077.80 | 9.45 | 27.29 |
| 1493 | C₆₈H₁₃₃N₃O₇S₃ | 1201.0 3 | 9.01 | 29.22 | 1693 | C₆₇H₁₃₂N₂O₇ | 1077.80 | 9.46 | 27.29 |
| 1494 | C₆₈H₁₃₃N₃O₇S₃ | 1201.0 3 | 9.01 | 29.22 | 1694 | C₆₇H₁₃₂N₂O₇ | 1077.80 | 9.46 | 27.29 |
| 1495 | C₁₀₁H₁₉₉N₃O₇S₃ | 1663.9 2 | 9.01 | 46.95 | 1695 | C₆₄H₁₃₂N₂O₃S | 1009.83 | 9.80 | 26.96 |
| 1496 | C₉₅H₁₈₇N₃O₇S₃ | 1579.7 6 | 9.01 | 43.77 | 1696 | C₆₄H₁₃₂N₂O₃S | 1009.83 | 9.80 | 26.96 |
| 1497 | C₈₆H₁₆₉N₃O₇S₃ | 1453.5 1 | 9.01 | 39.01 | 1697 | C₆₇H₁₃₂N₂O₆S | 1093.86 | 9.79 | 27.78 |
| 1498 | C₆₈H₁₂₇N₃O₇S₃ | 1194.9 8 | 9.01 | 27.98 | 1698 | C₆₇H₁₃₂N₂O₆S | 1093.86 | 9.79 | 27.78 |
| 1499 | C₆₈H₁₂₇N₃O₇S₃ | 1194.9 8 | 9.01 | 27.89 | 1699 | C₆₇H₁₃₂N₂O₆S | 1093.86 | 9.81 | 27.78 |
| 1500 | C₄₇H₉₁N₃O₇S₃ | 906.46 | 9.01 | 18.77 | 1700 | C₆₇H₁₃₂N₂O₆S | 1093.86 | 9.81 | 27.78 |
| 1501 | C₅₉H₁₀₃N₃O₇S₃ | 1062.6 9 | 9.01 | 22.21 | 1701 | C₆₄H₁₃₃N₃O₃ | 992.79 | 9.78 | 26.31 |
| 1502 | C₆₅H₁₁₅N₃O₇S₃ | 1146.8 5 | 9.01 | 24.22 | 1702 | C₆₄H₁₃₃N₃O₃ | 992.79 | 9.78 | 26.31 |
| 1503 | C₅₃H₉₇N₃O₇S₃ | 984.57 | 9.01 | 20.49 | 1703 | C₆₇H₁₃₃N₃O₆ | 1076.82 | 9.78 | 27.13 |
| 1504 | C₆₅H₁₂₁N₃O₇S₃ | 1152.9 0 | 9.01 | 26.84 | 1704 | C₆₇H₁₃₃N₃O₆ | 1076.82 | 9.78 | 27.13 |
| 1505 | C₆₅H₁₂₇N₃O₇S₃ | 1158.9 5 | 9.01 | 28.29 | 1705 | C₆₇H₁₃₃N₃O₆ | 1076.82 | 9.79 | 27.13 |
| 1506 | C₇₁H₁₁₅N₃O₇S₃ | 1218.9 2 | 9.01 | 27.44 | 1706 | C₆₇H₁₃₃N₃O₆ | 1076.82 | 9.79 | 27.13 |
| 1507 | C₈₆H₁₄₅N₃O₇S₃ | 1429.3 2 | 9.01 | 35.13 | 1707 | C₆₄H₁₃₂N₂O₃S₂ | 1041.89 | 9.65 | 27.39 |
| 1508 | C₆₂H₁₂₁N₃O₇S₃ | 1116.8 6 | 9.01 | 26.05 | 1708 | C₆₄H₁₃₂N₂O₃S₂ | 1041.89 | 9.65 | 27.39 |
| 1509 | C₇₄H₁₄₅N₃O₇S₃ | 1285.1 9 | 9.01 | 32.39 | 1709 | C₆₇H₁₃₂N₂O₆S₂ | 1125.92 | 9.65 | 28.21 |
| 1510 | C₉₅H₁₈₇N₃O₇S₃ | 1579.7 6 | 9.01 | 43.50 | 1710 | C₆₇H₁₃₂N₂O₆S₂ | 1125.92 | 9.65 | 28.21 |
| 1511 | C₆₈H₁₃₃N₃O₇S₃ | 1201.0 3 | 9.01 | 29.22 | 1711 | C₆₇H₁₃₂N₂O₆S₂ | 1125.92 | 9.65 | 28.21 |
| 1512 | C₆₈H₁₃₃N₃O₇S₃ | 1201.0 3 | 9.01 | 29.22 | 1712 | C₆₇H₁₃₂N₂O₆S₂ | 1125.92 | 9.65 | 28.21 |
| 1513 | C₁₀₁H₁₉₉N₃O₇S₃ | 1663.9 2 | 9.01 | 46.95 | 1713 | C₆₅H₁₃₂N₂O₄ | 1005.78 | 8.95 | 26.46 |
| 1514 | C₉₅H₁₈₇N₃O₇S₃ | 1579.7 6 | 9.01 | 43.77 | 1714 | C₆₅H₁₃₂N₂O₄ | 1005.78 | 8.95 | 26.46 |
| 1515 | C₈₆H₁₆₉N₃O₇S₃ | 1453.5 1 | 9.01 | 39.01 | 1715 | C₆₈H₁₃₂N₂O₇ | 1089.81 | 8.95 | 27.28 |
| 1516 | C₆₈H₁₂₇N₃O₇S₃ | 1194.9 8 | 9.01 | 27.98 | 1716 | C₆₈H₁₃₂N₂O₇ | 1089.81 | 8.95 | 27.28 |
| 1517 | C₆₈H₁₂₇N₃O₇S₃ | 1194.9 8 | 9.01 | 27.89 | 1717 | C₆₈H₁₃₂N₂O₇ | 1089.81 | 8.96 | 27.28 |
| 1518 | C₄₇H₉₁N₃O₇S₃ | 906.46 | 9.01 | 18.77 | 1718 | C₆₈H₁₃₂N₂O₇ | 1089.81 | 8.96 | 27.28 |
| 1519 | C₅₉H₁₀₃N₃O₇S₃ | 1062.6 9 | 9.01 | 22.21 | 1719 | C₆₅H₁₃₂N₂O₅ | 1021.78 | 8.83 | 26.64 |
| 1520 | C₆₅H₁₁₅N₃O₇S₃ | 1146.8 5 | 9.01 | 24.22 | 1720 | C₆₅H₁₃₂N₂O₅ | 1021.78 | 8.83 | 26.64 |
| 1521 | C₅₃H₉₇N₃O₇S₃ | 984.57 | 9.01 | 20.49 | 1721 | C₆₈H₁₃₂N₂O₈ | 1105.81 | 8.83 | 27.46 |
| 1522 | C₆₅H₁₂₁N₃O₇S₃ | 1152.9 0 | 9.01 | 26.84 | 1722 | C₆₈H₁₃₂N₂O₈ | 1105.81 | 8.83 | 27.46 |
| 1523 | C₆₅H₁₂₇N₃O₇S₃ | 1158.9 5 | 9.01 | 28.29 | 1723 | C₆₈H₁₃₂N₂O₈ | 1105.81 | 8.83 | 27.46 |
| 1524 | C₇₁H₁₁₅N₃O₇S₃ | 1218.9 2 | 9.01 | 25.64 | 1724 | C₆₈H₁₃₂N₂O₈ | 1105.81 | 8.83 | 27.46 |
| 1525 | C₈₆H₁₄₅N₃O₇S₃ | 1429.3 2 | 9.01 | 33.34 | 1725 | C₆₅H₁₃₂N₂O₅ | 1021.78 | 8.67 | 26.64 |
| 1526 | C₆₂H₁₂₁N₃O₄S₆ | 1165.0 7 | 9.01 | 27.16 | 1726 | C₆₅H₁₃₂N₂O₅ | 1021.78 | 8.67 | 26.64 |
| 1527 | C₇₄H₁₄₅N₃O₄S₆ | 1333.3 9 | 9.01 | 33.50 | 1727 | C₆₈H₁₃₂N₂O₈ | 1105.81 | 8.67 | 27.46 |
| 1528 | C₉₅H₁₈₇N₃O₄S₆ | 1627.9 6 | 9.01 | 44.61 | 1728 | C₆₈H₁₃₂N₂O₈ | 1105.81 | 8.67 | 27.46 |
| 1529 | C₆₈H₁₃₃N₃O₄S₆ | 1249.2 3 | 9.01 | 30.33 | 1729 | C₆₈H₁₃₂N₂O₈ | 1105.81 | 8.67 | 27.46 |
| 1530 | C₆₈H₁₃₃N₃O₄S₆ | 1249.2 3 | 9.01 | 30.33 | 1730 | C₆₈H₁₃₂N₂O₈ | 1105.81 | 8.67 | 27.46 |
| 1531 | C₁₀₁H₁₉₉N₃O₄S₆ | 1712.1 2 | 9.01 | 48.06 | 1731 | C₆₅H₁₃₂N₂O₆ | 1037.78 | 8.66 | 26.60 |
| 1532 | C₉₅H₁₈₇N₃O₄S₆ | 1627.9 6 | 9.01 | 44.88 | 1732 | C₆₅H₁₃₂N₂O₆ | 1037.78 | 8.66 | 26.60 |
| 1533 | C₈₆H₁₆₉N₃O₄S₆ | 1501.7 2 | 9.01 | 40.12 | 1733 | C₆₈H₁₃₂N₂O₉ | 1121.81 | 8.66 | 27.43 |
| 1534 | C₆₈H₁₂₇N₃O₄S₆ | 1243.1 8 | 9.01 | 29.09 | 1734 | C₆₈H₁₃₂N₂O₉ | 1121.81 | 8.66 | 27.43 |
| 1535 | C₆₈H₁₂₇N₃O₄S₆ | 1243.1 8 | 9.01 | 29.00 | 1735 | C₆₈H₁₃₂N₂O₉ | 1121.81 | 8.66 | 27.43 |
| 1536 | C₄₇H₉₁N₃O₄S₆ | 954.66 | 9.01 | 19.88 | 1736 | C₆₈H₁₃₂N₂O₉ | 1121.81 | 8.66 | 27.43 |
| 1537 | C₅₉H₁₀₃N₃O₄S₆ | 1110.8 9 | 9.01 | 23.32 | 1737 | C₆₅H₁₃₃N₃O₄ | 1020.80 | 8.84 | 26.04 |
| 1538 | C₆₅H₁₁₅N₃O₄S₆ | 1195.05 | 9.01 | 25.33 | 1738 | C₆₅H₁₃₃N₃O₄ | 1020.80 | 8.84 | 26.04 |
| 1539 | C₅₃H₉₇N₃O₄S₆ | 1032.7 8 | 9.01 | 21.60 | 1739 | C₆₈H₁₃₃N₃O₇ | 1104.83 | 8.83 | 26.86 |
| 1540 | C₆₅H₁₂₁N₃O₄S₆ | 1201.1 0 | 9.01 | 27.95 | 1740 | C₆₈H₁₃₃N₃O₇ | 1104.83 | 8.84 | 26.86 |
| 1541 | C₆₅H₁₂₇N₃O₄S₆ | 1207.1 5 | 9.01 | 29.40 | 1741 | C₆₈H₁₃₃N₃O₇ | 1104.83 | 8.84 | 26.86 |
| 1542 | C₇₁H₁₁₅N₃O₄S₆ | 1267.1 2 | 9.01 | 28.55 | 1742 | C₆₈H₁₃₃N₃O₇ | 1104.83 | 8.84 | 26.86 |
| 1543 | C₈₆H₁₄₅N₃O₄S₆ | 1477.5 2 | 9.01 | 36.24 | 1743 | C₆₅H₁₃₃N₃O₅ | 1036.79 | 8.85 | 26.48 |
| 1544 | C₆₅H₁₃₆N₃O₁₀P₃ | 1212.7 4 | 9.01 | 21.98 | 1744 | C₆₅H₁₃₃N₃O₅ | 1036.79 | 8.85 | 26.48 |
| 1545 | C₇₇H₁₆₀N₃O₁₀P₃ | 1381.0 6 | 9.01 | 28.33 | 1745 | C₆₈H₁₃₃N₃O₈ | 1120.82 | 8.85 | 27.30 |
| 1546 | C₉₈H₂₀₂N₃O₁₀P₃ | 1675.6 3 | 9.01 | 39.44 | 1746 | C₆₈H₁₃₃N₃O₈ | 1120.82 | 8.85 | 27.30 |
| 1547 | C₇₁H₁₄₈N₃O₁₀P₃ | 1296.9 0 | 9.01 | 25.16 | 1747 | C₆₈H₁₃₃N₃O₈ | 1120.82 | 8.85 | 27.30 |
| 1548 | C₇₁H₁₄₈N₃O₁₀P₃ | 1296.9 0 | 9.01 | 25.16 | 1748 | C₆₈H₁₃₃N₃O₈ | 1120.82 | 8.85 | 27.30 |
| 1549 | C₁₀₄H₂₁₄N₃O₁₀P₃ | 1759.7 9 | 9.01 | 42.88 | 1749 | C₆₅H₁₃₃N₃O₅ | 1036.79 | 8.75 | 26.48 |
| 1550 | C₉₈H₂₀₂N₃O₁₀P₃ | 1675.6 3 | 9.01 | 39.71 | 1750 | C₆₅H₁₃₃N₃O₅ | 1036.79 | 8.75 | 26.48 |
| 1551 | C₈₉H₁₈₄N₃O₁₀P₃ | 1549.3 8 | 9.01 | 34.95 | 1751 | C₆₈H₁₃₃N₃O₈ | 1120.82 | 8.75 | 27.30 |
| 1552 | C₇₁H₁₄₂N₃O₁₀P₃ | 1290.8 5 | 9.01 | 23.91 | 1752 | C₆₈H₁₃₃N₃O₈ | 1120.82 | 8.75 | 27.30 |
| 1553 | C₇₁H₁₄₂N₃O₁₀P₃ | 1290.8 5 | 9.01 | 23.82 | 1753 | C₆₈H₁₃₃N₃O₈ | 1120.82 | 8.75 | 27.30 |
| 1554 | C₅₀H₁₀₆N₃O₁₀P₃ | 1002.3 3 | 9.01 | 14.71 | 1754 | C₆₈H₁₃₃N₃O₈ | 1120.82 | 8.75 | 27.30 |
| 1555 | C₆₂H₁₁₈N₃O₁₀P₃ | 1158.5 6 | 9.01 | 18.15 | 1755 | C₆₅H₁₃₄N₄O₄ | 1035.81 | 8.95 | 26.29 |
| 1556 | C₆₈H₁₃₀N₃O₁₀P₃ | 1242.7 2 | 9.01 | 20.16 | 1756 | C₆₅H₁₃₄N₄O₄ | 1035.81 | 8.95 | 26.29 |
| 1557 | C₅₆H₁₁₂N₃O₁₀P₃ | 1080.4 4 | 9.01 | 16.43 | 1757 | C₆₈H₁₃₄N₄O₇ | 1119.84 | 8.95 | 27.11 |
| 1558 | C₆₈H₁₃₆N₃O₁₀P₃ | 1248.7 7 | 9.01 | 22.78 | 1758 | C₆₈H₁₃₄N₄O₇ | 1119.84 | 8.95 | 27.11 |
| 1559 | C₆₈H₁₄₂N₃O₁₀P₃ | 1254.8 2 | 9.01 | 24.23 | 1759 | C₆₈H₁₃₄N₄O₇ | 1119.84 | 8.95 | 27.11 |
| 1560 | C₇₄H₁₃₀N₃O₁₀P₃ | 1314.7 9 | 9.01 | 23.74 | 1760 | C₆₈H₁₃₄N₄O₇ | 1119.84 | 8.95 | 27.11 |
| 1561 | C₈₉H₁₆₀N₃O₁₀P₃ | 1525.1 9 | 9.01 | 31.43 | 1761 | C₆₅H₁₃₂N₂O₃S | 1021.84 | 9.29 | 26.71 |
| 1562 | C₆₅H₁₃₆N₃O₁₀P₃ | 1212.7 4 | 9.01 | 21.98 | 1762 | C₆₅H₁₃₂N₂O₃S | 1021.84 | 9.29 | 26.71 |
| 1563 | C₇₇H₁₆₀N₃O₁₀P₃ | 1381.0 6 | 9.01 | 28.33 | 1763 | C₆₈H₁₃₂N₂O₆S | 1105.87 | 9.29 | 27.53 |
| 1564 | C₉₈H₂₀₂N₃O₁₀P₃ | 1675.6 3 | 9.01 | 39.44 | 1764 | C₆₈H₁₃₂N₂O₆S | 1105.87 | 9.29 | 27.53 |
| 1565 | C₇₁H₁₄₈N₃O₁₀P₃ | 1296.9 0 | 9.01 | 25.16 | 1765 | C₆₈H₁₃₂N₂O₆S | 1105.87 | 9.31 | 27.53 |
| 1566 | C₇₁H₁₄₈N₃O₁₀P₃ | 1296.9 0 | 9.01 | 25.16 | 1766 | C₆₈H₁₃₂N₂O₆S | 1105.87 | 9.31 | 27.53 |
| 1567 | C₁₀₄H₂₁₄N₃O₁₀P₃ | 1759.7 9 | 9.01 | 42.88 | 1767 | C₆₅H₁₃₂N₂O₃S₂ | 1053.90 | 9.35 | 27.19 |
| 1568 | C₉₈H₂₀₂N₃O₁₀P₃ | 1675.6 3 | 9.01 | 39.71 | 1768 | C₆₅H₁₃₂N₂O₃S₂ | 1053.90 | 9.35 | 27.19 |
| 1569 | C₈₉H₁₈₄N₃O₁₀P₃ | 1549.3 8 | 9.01 | 34.95 | 1769 | C₆₈H₁₃₂N₂O₆S₂ | 1137.93 | 9.35 | 28.02 |
| 1570 | C₇₁H₁₄₂N₃O₁₀P₃ | 1290.8 5 | 9.01 | 23.91 | 1770 | C₆₈H₁₃₂N₂O₆S₂ | 1137.93 | 9.35 | 28.02 |
| 1571 | C₇₁H₁₄₂N₃O₁₀P₃ | 1290.8 5 | 9.01 | 23.82 | 1771 | C₆₈H₁₃₂N₂O₆S₂ | 1137.93 | 9.35 | 28.02 |
| 1572 | C₅₀H₁₀₆N₃O₁₀P₃ | 1002.3 3 | 9.01 | 14.71 | 1772 | C₆₈H₁₃₂N₂O₆S₂ | 1137.93 | 9.35 | 28.02 |
| 1573 | C₆₂H₁₁₈N₃O₁₀P₃ | 1158.5 6 | 9.01 | 18.15 | 1773 | C₆₅H₁₃₂N₂O₃S₂ | 1053.90 | 9.13 | 27.19 |
| 1574 | C₆₈H₁₃₀N₃O₁₀P₃ | 1242.7 2 | 9.01 | 20.16 | 1774 | C₆₅H₁₃₂N₂O₃S₂ | 1053.90 | 9.13 | 27.19 |
| 1575 | C₅₆H₁₁₂N₃O₁₀P₃ | 1080.44 | 9.01 | 16.43 | 1775 | C₆₈H₁₃₂N₂O₆S₂ | 1137.93 | 9.13 | 28.02 |
| 1576 | C₆₈H₁₃₆N₃O₁₀P₃ | 1248.7 7 | 9.01 | 22.78 | 1776 | C₆₈H₁₃₂N₂O₆S₂ | 1137.93 | 9.13 | 28.02 |
| 1577 | C₆₈H₁₄₂N₃O₁₀P₃ | 1254.8 2 | 9.01 | 24.23 | 1777 | C₆₈H₁₃₂N₂O₆S₂ | 1137.93 | 9.14 | 28.02 |
| 1578 | C₇₄H₁₃₀N₃O₁₀P₃ | 1314.7 9 | 9.01 | 24.34 | 1778 | C₆₈H₁₃₂N₂O₆S₂ | 1137.93 | 9.14 | 28.02 |
| 1579 | C₈₉H₁₆₀N₃O₁₀P₃ | 1525.1 9 | 9.01 | 32.03 | 1779 | C₆₅H₁₃₂N₂O₄S | 1037.84 | 9.20 | 26.95 |
| 1580 | C₅₉H₁₂₄N₆O₇S₃ | 1125.8 8 | 9.01 | 21.35 | 1780 | C₆₅H₁₃₂N₂O₄S | 1037.84 | 9.20 | 26.95 |
| 1581 | C₇₁H₁₄₈N₆O₇S₃ | 1294.2 0 | 9.01 | 27.70 | 1781 | C₆₈H₁₃₂N₂O₇S | 1121.87 | 9.20 | 27.78 |
| 1582 | C₉₂H₁₉₀N₆O₇S₃ | 1588.7 7 | 9.01 | 38.81 | 1782 | C₆₈H₁₃₂N₂O₇S | 1121.87 | 9.20 | 27.78 |
| 1583 | C₆₅H₁₃₆N₆O₇S₃ | 1210.0 4 | 9.01 | 24.53 | 1783 | C₆₈H₁₃₂N₂O₇S | 1121.87 | 9.20 | 27.78 |
| 1584 | C₆₅H₁₃₆N₆O₇S₃ | 1210.0 4 | 9.01 | 24.53 | 1784 | C₆₈H₁₃₂N₂O₇S | 1121.87 | 9.20 | 27.78 |
| 1585 | C₉₈H₂₀₂N₆O₇S₃ | 1672.9 3 | 9.01 | 42.26 | 1785 | C₆₅H₁₃₂N₂O₄S | 1037.84 | 8.80 | 26.95 |
| 1586 | C₉₂H₁₉₀N₆O₇S₃ | 1588.7 7 | 9.01 | 39.08 | 1786 | C₆₅H₁₃₂N₂O₄S | 1037.84 | 8.80 | 26.95 |
| 1587 | C₈₃H₁₇₂N₆O₇S₃ | 1462.5 2 | 9.01 | 34.32 | 1787 | C₆₈H₁₃₂N₂O₇S | 1121.87 | 8.80 | 27.78 |
| 1588 | C₆₅H₁₃₀N₆O₇S₃ | 1203.9 9 | 9.01 | 23.29 | 1788 | C₆₈H₁₃₂N₂O₇S | 1121.87 | 8.80 | 27.78 |
| 1589 | C₆₅H₁₃₀N₆O₇S₃ | 1203.9 9 | 9.01 | 23.20 | 1789 | C₆₈H₁₃₂N₂O₇S | 1121.87 | 8.80 | 27.78 |
| 1590 | C₄₄H₉₄N₆O₇S₃ | 915.47 | 9.01 | 14.08 | 1790 | C₆₈H₁₃₂N₂O₇S | 1121.87 | 8.80 | 27.78 |
| 1591 | C₅₆H₁₀₆N₆O₇S₃ | 1071.7 0 | 9.01 | 17.52 | 1791 | C₆₅H₁₃₂N₂O₅S | 1053.84 | 8.70 | 27.15 |
| 1592 | C₆₂O₁₁₈N₆O₇S₃ | 1155.8 6 | 9.01 | 19.53 | 1792 | C₆₅H₁₃₂N₂O₅S | 1053.84 | 8.70 | 27.15 |
| 1593 | C₅₀H₁₀₀N₆O₇S₃ | 993.59 | 9.01 | 15.80 | 1793 | C₆₈H₁₃₂N₂O₈S | 1137.87 | 8.70 | 27.97 |
| 1594 | C₆₂H₁₂₄N₆O₇S₃ | 1161.9 1 | 9.01 | 22.15 | 1794 | C₆₈H₁₃₂N₂O₈S | 1137.87 | 8.70 | 27.97 |
| 1595 | C₆₂H₁₃₀N₆O₇S₃ | 1167.9 6 | 9.01 | 23.60 | 1795 | C₆₈H₁₃₂N₂O₈S | 1137.87 | 8.70 | 27.97 |
| 1596 | C₆₈H₁₁₈N₆O₇S₃ | 1227.9 3 | 9.01 | 23.53 | 1796 | C₆₈H₁₃₂N₂O₈S | 1137.87 | 8.70 | 27.97 |
| 1597 | C₈₃H₁₄₈N₆O₇S₃ | 1438.3 3 | 9.01 | 31.23 | 1797 | C₆₅H₁₃₂N₂O₅S | 1053.84 | 9.00 | 27.15 |
| 1598 | C₅₉H₁₂₄N₆O₇S₃ | 1125.8 8 | 9.01 | 21.35 | 1798 | C₆₅H₁₃₂N₂O₅S | 1053.84 | 9.00 | 27.15 |
| 1599 | C₇₁H₁₄₈N₆O₇S₃ | 1294.2 0 | 9.01 | 27.70 | 1799 | C₆₈H₁₃₂N₂O₈S | 1137.87 | 9.00 | 27.97 |
| 1600 | C₉₂H₁₉₀N₆O₇S₃ | 1588.7 7 | 9.01 | 38.81 | 1800 | C₆₈H₁₃₂N₂O₈S | 1137.87 | 9.00 | 27.97 |
| 1801 | C₆₈H₁₃₂N₂O₈S | 1137.8 7 | 9.00 | 27.97 | 2001 | C₆₉H₁₃₇N₅O₅ | 1116.88 | 7.65 | 24.63 |
| 1802 | C₆₈H₁₃₂N₂O₈S | 1137.8 7 | 9.00 | 27.97 | 2002 | C₆₉H₁₃₇N₅O₅ | 1116.88 | 7.65 | 24.63 |
| 1803 | C₆₅H₁₃₂N₂O₄S₂ | 1069.9 0 | 9.06 | 27.39 | 2003 | C₇₂H₁₃₇N₅O₈ | 1200.91 | 7.65 | 25.45 |
| 1804 | C₆₅H₁₃₂N₂O₄S₂ | 1069.9 0 | 9.06 | 27.39 | 2004 | C₇₂H₁₃₇N₅O₈ | 1200.91 | 7.65 | 25.45 |
| 1805 | C₆₈H₁₃₂N₂O₇S₂ | 1153.9 3 | 9.06 | 28.21 | 2005 | C₇₂H₁₃₇N₅O₈ | 1200.91 | 7.65 | 25.45 |
| 1806 | C₆₈H₁₃₂N₂O₇S₂ | 1153.9 3 | 9.06 | 28.21 | 2006 | C₇₂H₁₃₇N₅O₈ | 1200.91 | 7.65 | 25.45 |
| 1807 | C₆₈H₁₃₂N₂O₇S₂ | 1153.9 3 | 9.06 | 28.21 | 2007 | C₆₉H₁₃₆N₄O₅ | 1101.87 | 7.90 | 25.58 |
| 1808 | C₆₈H₁₃₂N₂O₇S₂ | 1153.9 3 | 9.06 | 28.21 | 2008 | C₆₉H₁₃₆N₄O₅ | 1101.87 | 7.90 | 25.58 |
| 1809 | C₆₆H₁₃₇N₂O₆P | 1085.8 0 | 8.79 | 26.39 | 2009 | C₇₂H₁₃₆N₄O₈ | 1185.90 | 7.90 | 26.41 |
| 1810 | C₆₆H₁₃₇N₂O₆P | 1085.8 0 | 8.79 | 26.39 | 2010 | C₇₂H₁₃₆N₄O₈ | 1185.90 | 7.90 | 26.41 |
| 1811 | C₆₉H₁₃₇N₂O₉P | 1169.8 3 | 8.79 | 27.21 | 2011 | C₇₂H₁₃₆N₄O₈ | 1185.90 | 7.90 | 26.41 |
| 1812 | C₆₉H₁₃₇N₂O₉P | 1169.8 3 | 8.79 | 27.21 | 2012 | C₇₂H₁₃₆N₄O₈ | 1185.90 | 7.90 | 26.41 |
| 1813 | C₆₉H₁₃₇N₂O₉P | 1169.83 | 8.79 | 27.21 | 2013 | C₆₉H₁₃₇N₅O₄ | 1100.89 | 8.23 | 25.73 |
| 1814 | C₆₉H₁₃₇N₂O₉P | 1169.8 3 | 8.79 | 27.21 | 2014 | C₆₉H₁₃₇N₅O₄ | 1100.89 | 8.23 | 25.73 |
| 1815 | C₆₆H₁₃₇N₂O₆P | 1085.8 0 | 8.41 | 26.39 | 2015 | C₇₂H₁₃₇N₅O₇ | 1184.92 | 8.23 | 26.56 |
| 1816 | C₆₆H₁₃₇N₂O₆P | 1085.8 0 | 8.41 | 26.39 | 2016 | C₇₂H₁₃₇N₅O₇ | 1184.92 | 8.23 | 26.56 |
| 1817 | C₆₉H₁₃₇N₂O₉P | 1169.8 3 | 8.41 | 27.21 | 2017 | C₇₂H₁₃₇N₅O₇ | 1184.92 | 8.23 | 26.56 |
| 1818 | C₆₉H₁₃₇N₂O₉P | 1169.8 3 | 8.41 | 27.21 | 2018 | C₇₂H₁₃₇N₅O₇ | 1184.92 | 8.23 | 26.56 |
| 1819 | C₆₉H₁₃₇N₂O₉P | 1169.8 3 | 8.41 | 27.21 | 2019 | C₆₉H₁₃₇N₅O₄ | 1100.89 | 7.75 | 26.13 |
| 1820 | C₆₉H₁₃₇N₂O₉P | 1169.8 3 | 8.41 | 27.21 | 2020 | C₆₉H₁₃₇N₅O₄ | 1100.89 | 7.75 | 26.13 |
| 1821 | C₆₄H₁₃₃N₃O₅S | 1056.8 4 | 8.74 | 26.14 | 2021 | C₇₂H₁₃₇N₅O₇ | 1184.92 | 7.75 | 26.95 |
| 1822 | C₆₄H₁₃₃N₃O₅S | 1056.8 4 | 8.74 | 26.14 | 2022 | C₇₂H₁₃₇N₅O₇ | 1184.92 | 7.75 | 26.95 |
| 1823 | C₆₇H₁₃₃N₃O₈S | 1140.8 7 | 8.74 | 26.96 | 2023 | C₇₂H₁₃₇N₅O₇ | 1184.92 | 7.75 | 26.95 |
| 1824 | C₆₇H₁₃₃N₃O₈S | 1140.8 7 | 8.74 | 26.96 | 2024 | C₇₂H₁₃₇N₅O₇ | 1184.92 | 7.75 | 26.95 |
| 1825 | C₆₇H₁₃₃N₃O₈S | 1140.8 7 | 8.74 | 26.96 | 2025 | C₆₈H₁₃₆N₆O₄ | 1101.87 | 7.19 | 25.85 |
| 1826 | C₆₇H₁₃₃N₃O₈S | 1140.8 7 | 8.74 | 26.96 | 2026 | C₆₈H₁₃₆N₆O₄ | 1101.87 | 7.19 | 25.85 |
| 1827 | C₆₄H₁₃₃N₃O₅S | 1056.8 4 | 7.78 | 26.14 | 2027 | C₇₁H₁₃₆N₆O₇ | 1185.90 | 7.19 | 26.67 |
| 1828 | C₆₄H₁₃₃N₃O₅S | 1056.8 4 | 7.78 | 26.14 | 2028 | C₇₁H₁₃₆N₆O₇ | 1185.90 | 7.19 | 26.67 |
| 1829 | C₆₇H₁₃₃N₃O₈S | 1140.8 7 | 7.78 | 26.96 | 2029 | C₇₁H₁₃₆N₆O₇ | 1185.90 | 7.19 | 26.67 |
| 1830 | C₆₇H₁₃₃N₃O₈S | 1140.8 7 | 7.78 | 26.96 | 2030 | C₇₁H₁₃₆N₆O₇ | 1185.90 | 7.19 | 26.67 |
| 1831 | C₆₇H₁₃₃N₃O₈S | 1140.8 7 | 7.78 | 26.96 | 2031 | C₆₉H₁₃₇N₅O₄S | 1132.95 | 7.95 | 27.26 |
| 1832 | C₆₇H₁₃₃N₃O₈S | 1140.8 7 | 7.78 | 26.96 | 2032 | C₆₉H₁₃₇N₅O₄S | 1132.95 | 7.95 | 27.26 |
| 1833 | C₆₄H₁₃₂N₂O₆S | 1057.8 3 | 7.60 | 26.33 | 2033 | C₈₉H₁₇₆N₆O₉S | 1506.48 | 7.74 | 33.24 |
| 1834 | C₆₄H₁₃₂N₂O₆S | 1057.8 3 | 7.60 | 26.33 | 2034 | C₈₈H₁₇₅N₅O₉ | 1447.39 | 8.17 | 35.73 |
| 1835 | C₆₇H₁₃₂N₂O₉S | 1141.8 6 | 7.60 | 27.15 | 2035 | C₇₂H₁₃₇N₅O₇S | 1216.98 | 7.96 | 28.08 |
| 1836 | C₆₇H₁₃₂N₂O₉S | 1141.8 6 | 7.60 | 27.15 | 2036 | C₇₂H₁₃₇N₅O₇S | 1216.98 | 7.96 | 28.08 |
| 1837 | C₆₇H₁₃₂N₂O₉S | 1141.8 6 | 7.60 | 27.15 | 2037 | C₇₁H₁₃₈N₈O₅ | 1183.94 | 7.59 | 24.76 |
| 1838 | C₆₇H₁₃₂N₂O₉S | 1141.8 6 | 7.60 | 27.15 | 2038 | C₇₁H₁₃₈N₈O₅ | 1183.94 | 7.59 | 24.76 |
| 1839 | C₆₄H₁₃₂N₂O₆S | 1057.8 3 | 8.32 | 26.33 | 2039 | C₉₃H₁₇₉N₅O₁₁S₃ | 1639.66 | 7.56 | 35.27 |
| 1840 | C₆₄H₁₃₂N₂O₆S | 1057.8 3 | 8.32 | 26.33 | 2040 | C₉₃H₁₈₀N₅O₁₂P S | 1623.52 | 8.52 | 37.28 |
| 1841 | C₆₇H₁₃₂N₂O₉S | 1141.8 6 | 8.32 | 27.15 | 2041 | C₇₄H₁₃₈N₈O₈ | 1267.97 | 7.59 | 25.58 |
| 1842 | C₆₇H₁₃₂N₂O₉S | 1141.8 6 | 8.32 | 27.15 | 2042 | C₇₄H₁₃₈N₈O₈ | 1267.97 | 7.59 | 25.58 |
| 1843 | C₆₇H₁₃₂N₂O₉S | 1141.8 6 | 8.32 | 27.15 | 2043 | C₆₈H₁₄₀N₆O₄ | 1105.91 | 8.62 | 25.32 |
| 1844 | C₆₇H₁₃₂N₂O₉S | 1141.8 6 | 8.32 | 27.15 | 2044 | C₆₈H₁₄₀N₆O₄ | 1105.91 | 8.62 | 25.32 |
| 1845 | C₆₄H₁₃₂N₂O₇S | 1073.8 3 | 8.15 | 26.57 | 2045 | C₇₁H₁₄₀N₆O₇ | 1189.94 | 8.62 | 26.14 |
| 1846 | C₆₄H₁₃₂N₂O₇S | 1073.8 3 | 8.15 | 26.57 | 2046 | C₇₁H₁₄₀N₆O₇ | 1189.94 | 8.62 | 26.14 |
| 1847 | C₆₇H₁₃₂N₂O₁₀S | 1157.8 6 | 8.15 | 27.39 | 2047 | C₇₁H₁₄₀N₆O₇ | 1189.94 | 8.62 | 26.14 |
| 1848 | C₆₇H₁₃₂N₂O₁₀S | 1157.8 6 | 8.15 | 27.39 | 2048 | C₇₁H₁₄₀N₆O₇ | 1189.94 | 8.62 | 26.14 |
| 1849 | C₆₇H₁₃₂N₂O₁₀S | 1157.8 6 | 8.15 | 27.39 | 2049 | C₆₈H₁₃₉N₅O₄ | 1090.89 | 8.61 | 25.54 |
| 1850 | C₆₇H₁₃₂N₂O₁₀S | 1157.86 | 8.15 | 27.39 | 2050 | C₆₈H₁₃₉N₅O₄ | 1090.89 | 8.61 | 25.54 |
| 1851 | C₆₄H₁₃₂N₂O₅S | 1041.8 3 | 7.83 | 25.84 | 2051 | C₇₁H₁₃₉N₅O₇ | 1174.92 | 8.61 | 26.36 |
| 1852 | C₆₄H₁₃₂N2O₅S | 1041.8 3 | 7.83 | 25.84 | 2052 | C₇₁H₁₃₉N₅O₇ | 1174.92 | 8.61 | 26.36 |
| 1853 | C₆₇H₁₃₂N₂O₈S | 1125.8 6 | 7.83 | 26.66 | 2053 | C₇₁H₁₃₉N₅O₇ | 1174.92 | 8.61 | 26.36 |
| 1854 | C₆₇H₁₃₂N₂O₈S | 1125.8 6 | 7.83 | 26.66 | 2054 | C₇₁H₁₃₉N₅O₇ | 1174.92 | 8.61 | 26.36 |
| 1855 | C₆₇H₁₃₂N₂O₈S | 1125.8 6 | 7.84 | 26.66 | 2055 | C₆₉H₁₃₉N₅O₅ | 1118.90 | 7.82 | 25.64 |
| 1856 | C₆₇H₁₃₂N₂O₈S | 1125.8 6 | 7.84 | 26.66 | 2056 | C₆₉H₁₃₉N₅O₅ | 1118.90 | 7.82 | 25.64 |
| 1857 | C₆₇H₁₃₇N₃O₄ | 1048.8 5 | 9.07 | 27.10 | 2057 | C₉₀H₁₇₆N₂OS₈ | 1558.89 | 9.96 | 42.56 |
| 1858 | C₆₇H₁₃₇N₃O₄ | 1048.8 5 | 9.07 | 7.10 | 2058 | C₈₉H₁₇₄N₂OS₈ | 1544.86 | 8.77 | 40.62 |
| 1859 | C₇₀H₁₃₇N₃O₇ | 1132.8 8 | 9.07 | 27.92 | 2059 | C₇₂H₁₃₉N₅O₈ | 1202.93 | 7.82 | 26.46 |
| 1860 | C₇₀H₁₃₇N₃O₇ | 1132.8 8 | 9.07 | 27.92 | 2060 | C₇₂H₁₃₉N₅O₈ | 1202.93 | 7.82 | 26.46 |
| 1861 | C₇₀H₁₃₇N₃O₇ | 1132.8 8 | 9.07 | 27.92 | 2061 | C₇₀H₁₄₁N₅O₅ | 1132.93 | 7.84 | 26.35 |
| 1862 | C₇₀H₁₃₇N₃O₇ | 1132.8 8 | 9.07 | 27.92 | 2062 | C₇₀H₁₄₁N₅O₅ | 1132.93 | 7.84 | 26.35 |
| 1863 | C₆₉H₁₄₁N₃O₄ | 1076.9 0 | 9.10 | 28.15 | 2063 | C₇₃H₁₄₁N₅O₈ | 1216.96 | 7.84 | 27.17 |
| 1864 | C₆₉H₁₄₁N₃O₄ | 1076.9 0 | 9.09 | 28.15 | 2064 | C₇₃H₁₄₁N₅O₈ | 1216.96 | 7.84 | 27.17 |
| 1865 | C₇₂H₁₄₁N₃O₇ | 1160.9 3 | 9.10 | 28.98 | 2065 | C₇₃H₁₄₁N₅O₈ | 1216.96 | 7.84 | 27.17 |
| 1866 | C₉₅H₁₉₄N₄O₉S₅ | 1696.9 2 | 9.86 | 40.86 | 2066 | C₇₃H₁₄₁N₅O₈ | 1216.96 | 7.84 | 27.17 |
| 1867 | C₇₂H₁₄₁N₃O₇ | 1160.9 3 | 9.10 | 28.98 | 2067 | C₆₉H₁₃₇N₅O₆ | 1132.88 | 7.19 | 25.38 |
| 1868 | C₇₂H₁₄₁N₃O₇ | 1160.9 3 | 9.10 | 28.98 | 2068 | C₆₉H₁₃₇N₅O₆ | 1132.88 | 7.19 | 25.38 |
| 1869 | C₇₁H₁₄₅N₃O₄ | 1104.9 6 | 9.12 | 29.21 | 2069 | C₇₂H₁₃₇N₅O₉ | 1216.91 | 7.06 | 26.20 |
| 1870 | C₇₁H₁₄₅N₃O₄ | 1104.9 6 | 9.12 | 29.21 | 2070 | C₇₂H₁₃₇N₅O₉ | 1216.91 | 7.06 | 26.20 |
| 1871 | C₇₄H₁₄₅N₃O₇ | 1188.9 9 | 9.12 | 30.03 | 2071 | C₇₂H₁₃₇N₅O₉ | 1216.91 | 7.06 | 26.20 |
| 1872 | C₉₆H₁₉₁NO₁₄S₅ | 1743.8 8 | 7.67 | 41.25 | 2072 | C₇₂H₁₃₇N₅O₉ | 1216.91 | 7.06 | 26.20 |
| 1873 | C₇₄H₁₄₅N₃O₇ | 1188.9 9 | 9.12 | 30.03 | 2073 | C₆₉H₁₃₈N₄O₆ | 1119.88 | 7.66 | 26.29 |
| 1874 | C₇₄H₁₄₅N₃O₇ | 1188.9 9 | 9.12 | 30.03 | 2074 | C₆₉H₁₃₈N₄O₆ | 1119.88 | 7.66 | 26.29 |
| 1875 | C₆₆H₁₃₅N₃O₄ | 1034.8 2 | 9.04 | 26.57 | 2075 | C₇₂H₁₃₈N₄O₉ | 1203.91 | 7.66 | 27.11 |
| 1876 | C₆₆H₁₃₅N₃O₄ | 1034.8 2 | 9.04 | 26.57 | 2076 | C₇₂H₁₃₈N₄O₉ | 1203.91 | 7.66 | 27.11 |
| 1877 | C₆₉H₁₃₅N₃O₇ | 1118.8 5 | 9.04 | 27.39 | 2077 | C₇₂H₁₃₈N₄O₉ | 1203.91 | 7.66 | 27.11 |
| 1878 | C₆₉H₁₃₅N₃O₇ | 1118.8 5 | 9.04 | 27.39 | 2078 | C₇₂H₁₃₈N₄O₉ | 1203.91 | 7.66 | 27.11 |
| 1879 | C₆₉H₁₃₅N₃O₇ | 1118.8 5 | 9.04 | 27.39 | 2079 | C₇₀H₁₄₀N₄O₅ | 1117.91 | 7.94 | 26.15 |
| 1880 | C₆₉H₁₃₅N₃O₇ | 1118.8 5 | 9.04 | 27.39 | 2080 | C₇₀H₁₄₀N₄O₅ | 1117.91 | 7.93 | 26.15 |
| 1881 | C₆₇H₁₃₇N₃O₄ | 1048.8 5 | 9.05 | 27.10 | 2081 | C₇₃H₁₄₀N₄O₈ | 1201.94 | 7.94 | 26.97 |
| 1882 | C₆₇H₁₃₇N₃O₄ | 1048.8 5 | 9.05 | 27.10 | 2082 | C₇₃H₁₄₀N₄O₈ | 1201.94 | 7.93 | 26.97 |
| 1883 | C₇₀H₁₃₇N₃O₇ | 1132.8 8 | 9.05 | 27.92 | 2083 | C₇₃H₁₄₀N₄O₈ | 1201.94 | 7.94 | 26.97 |
| 1884 | C₇₀H₁₃₇N₃O₇ | 1132.8 8 | 9.05 | 27.92 | 2084 | C₇₃H₁₄₀N₄O₈ | 1201.94 | 7.94 | 26.97 |
| 1885 | C₇₀H₁₃₇N₃O₇ | 1132.8 8 | 9.05 | 27.92 | 2085 | C₇₀H₁₃₈N₄O₆ | 1131.90 | 7.19 | 25.85 |
| 1886 | C₇₀H₁₃₇N₃O₇ | 1132.8 8 | 9.05 | 27.92 | 2086 | C₇₀H₁₃₈N₄O₆ | 1131.90 | 7.19 | 25.85 |
| 1887 | C₆₈H₁₃₉N₃O₄ | 1062.88 | 9.06 | 27.63 | 2087 | C₇₃H₁₃₈N₄O₉ | 1215.93 | 7.17 | 26.67 |
| 1888 | C₆₈H₁₃₉N₃O₄ | 1062.8 8 | 9.06 | 27.63 | 2088 | C₇₃H₁₃₈N₄O₉ | 1215.93 | 7.17 | 26.67 |
| 1889 | C₇₁H₁₃₉N₃O₇ | 1146.9 1 | 9.06 | 28.45 | 2089 | C₇₃H₁₃₈N₄O₉ | 1215.93 | 7.17 | 26.67 |
| 1890 | C₇₁H₁₃₉N₃O₇ | 1146.9 1 | 9.06 | 28.45 | 2090 | C₇₃H₁₃₈N₄O₉ | 1215.93 | 7.17 | 26.67 |
| 1891 | C₇₁H₁₃₉N₃O₇ | 1146.9 1 | 9.06 | 28.45 | 2091 | C₇₀H₁₃₉N₅O₆ | 1146.91 | 7.19 | 25.65 |
| 1892 | C₇₁H₁₃₉N₃O₇ | 1146.9 1 | 9.06 | 28.45 | 2092 | C₇₀H₁₃₉N₅O₆ | 1146.91 | 7.19 | 25.65 |
| 1893 | C₆₇H₁₃₇N₃O₄ | 1048.8 5 | 9.11 | 26.88 | 2093 | C₇₃H₁₃₉N₅O₉ | 1230.94 | 7.08 | 26.47 |
| 1894 | C₆₇H₁₃₇N₃O₄ | 1048.8 5 | 9.11 | 26.88 | 2094 | C₇₃H₁₃₉N₅O₉ | 1230.94 | 7.08 | 26.47 |
| 1895 | C₇₀H₁₃₇N₃O₇ | 1132.8 8 | 9.11 | 27.70 | 2095 | C₇₃H₁₃₉N₅O₉ | 1230.94 | 7.08 | 26.47 |
| 1896 | C₇₀H₁₃₇N₃O₇ | 1132.8 8 | 9.11 | 27.70 | 2096 | C₇₃H₁₃₉N₅O₉ | 1230.94 | 7.08 | 26.47 |
| 1897 | C₇₀H₁₃₇N₃O₇ | 1132.8 8 | 9.11 | 27.70 | 2097 | C₇₀H₁₄₁N₅O₅ | 1132.93 | 7.84 | 26.35 |
| 1898 | C₇₀H₁₃₇N₃O₇ | 1132.8 8 | 9.11 | 27.70 | 2098 | C₇₀H₁₄₁N₅O₅ | 1132.93 | 7.84 | 26.35 |
| 1899 | C₆₈H₁₃₉N₃O₄ | 1062.8 8 | 9.12 | 27.41 | 2099 | C₇₃H₁₄₁N₅O₈ | 1216.96 | 7.84 | 27.17 |
| 1900 | C₆₈H₁₃₉N₃O₄ | 1062.8 8 | 9.12 | 27.41 | 2100 | C₇₃H₁₄₁N₅O₈ | 1216.96 | 7.84 | 27.17 |
| 1901 | C₇₁H₁₃₉N₃O₇ | 1146.9 1 | 9.12 | 28.23 | 2101 | C₇₃H₁₄₁N₅O₅ | 1216.96 | 7.84 | 27.17 |
| 1902 | C₇₁H₁₃₉N₃O₇ | 1146.9 1 | 9.12 | 28.23 | 2102 | C₇₃H₁₄₁N₅O₈ | 1216.96 | 7.84 | 27.17 |
| 1903 | C₇₁H₁₃₉N₃O₇ | 1146.9 1 | 9.12 | 28.23 | 2103 | C₇₀H₁₃₈N₄O₇ | 1147.89 | 7.19 | 26.06 |
| 1904 | C₇₁H₁₃₉N₃O₇ | 1146.9 1 | 9.12 | 28.23 | 2104 | C₇₀H₁₃₈N₄O₇ | 1147.89 | 7.19 | 26.06 |
| 1905 | C₆₉H₁₄₁N₃O₄ | 1076.9 0 | 9.20 | 27.71 | 2105 | C₆₈H₁₃₅N₅O₅ | 1102.86 | 7.65 | 24.36 |
| 1906 | C₆₉H₁₄₁N₃O₄ | 1076.9 0 | 9.20 | 27.71 | 2106 | C₆₈H₁₃₅N₅O₅ | 1102.86 | 7.65 | 24.36 |
| 1907 | C₇₂H₁₄₁N₃O₇ | 1160.9 3 | 9.20 | 28.54 | 2107 | C₇₁H₁₃₅N₅O₈ | 1186.89 | 7.65 | 25.18 |
| 1908 | C₇₂H₁₄₁N₃O₇ | 1160.9 3 | 9.20 | 28.54 | 2108 | C₇₁H₁₃₅N₅O₈ | 1186.89 | 7.65 | 25.18 |
| 1909 | C₇₂H₁₄₁N₃O₇ | 1160.9 3 | 9.20 | 28.54 | 2109 | C₇₁H₁₃₅N₅O₈ | 1186.89 | 7.65 | 25.18 |
| 1910 | C₇₂H₁₄₁N₃O₇ | 1160.9 3 | 9.20 | 28.54 | 2110 | C₇₁H₁₃₅N₅O₈ | 1186.89 | 7.65 | 25.18 |
| 1911 | C₆₉H₁₄₁N₃O₄ | 1076.9 0 | 9.15 | 27.93 | 2111 | C₆₉H₁₃₆N₄O₅ | 1101.87 | 7.90 | 25.58 |
| 1912 | C₆₉H₁₄₁N₃O₄ | 1076.9 0 | 9.15 | 27.93 | 2112 | C₆₉H₁₃₆N₄O₅ | 1101.87 | 7.90 | 25.58 |
| 1913 | C₇₂H₁₄₁N₃O₇ | 1160.9 3 | 9.15 | 28.76 | 2113 | C₇₂H₁₃₆N₄O₈ | 1185.90 | 7.90 | 26.41 |
| 1914 | C₇₂H₁₄₁N₃O₇ | 1160.9 3 | 9.15 | 28.76 | 2114 | C₇₂H₁₃₆N₄O₈ | 1185.90 | 7.90 | 26.41 |
| 1915 | C₇₂H₁₄₁N₃O₇ | 1160.9 3 | 9.15 | 28.76 | 2115 | C₇₂H₁₃₆N₄O₈ | 1185.90 | 7.90 | 26.41 |
| 1916 | C₇₂H₁₄₁N₃O₇ | 1160.9 3 | 9.15 | 28.76 | 2116 | C₇₂H₁₃₆N₄O₅ | 1185.90 | 7.90 | 26.41 |
| 1917 | C₆₇H₁₃₅N₃O₄ | 1046.8 3 | 8.42 | 26.62 | 2117 | C₇₀H₁₃₇N₅O₆ | 1144.89 | 7.19 | 25.33 |
| 1918 | C₆₇H₁₃₅N₃O₄ | 1046.8 3 | 8.42 | 26.62 | 2118 | C₇₀H₁₃₇N₅O₆ | 1144.89 | 7.19 | 25.33 |
| 1919 | C₇₀H₁₃₅N₃O₇ | 1130.8 6 | 8.42 | 27.44 | 2119 | C₁₀₀H₁₉₅NO₉S₂ | 1619.78 | 9.12 | 42.45 |
| 1920 | C₇₀H₁₃₅N₃O₇ | 1130.8 6 | 8.42 | 27.44 | 2120 | C₉₉H₁₈₉N₃O₉ | 1565.61 | 8.77 | 41.14 |
| 1921 | C₇₀H₁₃₅N₃O₇ | 1130.8 6 | 8.42 | 27.44 | 2121 | C₇₃H₁₃₇N₅O₉ | 1228.92 | 7.07 | 26.15 |
| 1922 | C₇₀H₁₃₅N₃O₇ | 1130.8 6 | 8.42 | 27.44 | 2122 | C₇₃H₁₃₇N₅O₉ | 1228.92 | 7.07 | 26.15 |
| 1923 | C₆₉H₁₃₉N₃O₄ | 1074.8 9 | 8.99 | 27.51 | 2123 | C₇₁H₁₃₉N₅O₅ | 1142.92 | 7.47 | 25.63 |
| 1924 | C₆₉H₁₃₉N₃O₄ | 1074.89 | 8.99 | 27.51 | 2124 | C₇₁H₁₃₉N₅O₅ | 1142.92 | 7.47 | 25.63 |
| 1925 | C₇₂H₁₃₉N₃O₇ | 1158.9 2 | 8.99 | 28.33 | 2125 | C₁₀₃H₁₉₄N₂O₁₅S ₂ | 1764.80 | 8.09 | 42.63 |
| 1926 | C₇₂H₁₃₉N₃O₇ | 1158.9 2 | 8.99 | 28.33 | 2126 | C₁₀₁H₁₉₆N₂O₁₄S | 1694.74 | 9.65 | 42.49 |
| 1927 | C₇₂H₁₃₉N₃O₇ | 1158.9 2 | 9.00 | 28.33 | 2127 | C₇₄H₁₃₉N₅O₈ | 1226.95 | 7.47 | 26.45 |
| 1928 | C₇₂H₁₃₉N₃O₇ | 1158.9 2 | 9.00 | 28.33 | 2128 | C₇₄H₁₃₀N₅O₅ | 1226.95 | 7.47 | 26.45 |
| 1929 | C₆₇H₁₃₅N₃O₄ | 1046.8 3 | 8.77 | 26.65 | 2129 | C₇₁H₁₃₈N₈O₄ | 1167.94 | 7.58 | 25.74 |
| 1930 | C₆₇H₁₃₅N₃O₄ | 1046.8 3 | 8.77 | 26.65 | 2130 | C₇₁H₁₃₈N₈O₄ | 1167.94 | 7.58 | 25.74 |
| 1931 | C₇₀H₁₃₅N₃O₇ | 1130.8 6 | 8.77 | 27.47 | 2131 | C₈₈H₁₇₂N₄O₇ | 1398.36 | 7.81 | 35.59 |
| 1932 | C₇₀H₁₃₅N₃O₇ | 1130.8 6 | 8.77 | 27.47 | 2132 | C₈₄H₁₇₁NO₇S | 1339.35 | 10.02 | 36.46 |
| 1933 | C₇₀H₁₃₅N₃O₇ | 1130.8 6 | 8.77 | 27.47 | 2133 | C₇₄H₁₃₈N₈O₇ | 1251.97 | 7.58 | 26.56 |
| 1934 | C₇₀H₁₃₅N₃O₇ | 1130.8 6 | 8.77 | 27.47 | 2134 | C₇₄H₁₃₈N₈O₇ | 1251.97 | 7.58 | 26.56 |
| 1935 | C₆₈H₁₃₇N₃O₄ | 1060.8 6 | 9.40 | 26.63 | 2135 | C₆₀H₁₀₀N₂O₈S | 1009.53 | 7.01 | 18.88 |
| 1936 | C₆₈H₁₃₇N₃O₄ | 1060.8 6 | 9.40 | 26.63 | 2136 | C₆₁H₁₀₅N₃O₆S₃ | 1072.71 | 8.34 | 21.30 |
| 1937 | C₇₁H₁₃₇N₃O₇ | 1144.8 9 | 9.40 | 27.45 | 2137 | C₆₀H₁₀₅NO₂S₄ | 1000.75 | 8.96 | 22.13 |
| 1938 | C₇₁H₁₃₇N₃O₇ | 1144.8 9 | 9.40 | 27.45 | 2138 | C₆₃H₁₁₄N₆O₇ | 1067.64 | 10.00 | 21.45 |
| 1939 | C₇₁H₁₃₇N₃O₇ | 1144.8 9 | 9.40 | 27.45 | 2139 | C₅₉H₁₀₁NO₁₀ | 984.45 | 8.35 | 21.09 |
| 1940 | C₇₁H₁₃₇N₃O₇ | 1144.8 9 | 9.40 | 27.45 | 2140 | C₆₀H₁₀₈N₄O₆S | 1013.61 | 8.40 | 19.22 |
| 1941 | C₆₈H₁₃₇N₃O₄ | 1060.8 6 | 9.62 | 25.87 | 2141 | C₆₄H₁₁₈N₃O₇P | 1072.64 | 8.79 | 20.87 |
| 1942 | C₆₈H₁₃₇N₃O₄ | 1060.8 6 | 9.62 | 25.87 | 2142 | C₅₆H₁₀₃NO₉S₄ | 1062.68 | 10.13 | 19.46 |
| 1943 | C₇₁H₁₃₇N₃O₇ | 1144.8 9 | 9.62 | 26.69 | 2143 | C₅₉H₁₀₇NO₉S₄ | 1102.74 | 9.62 | 17.16 |
| 1944 | C₇₁H₁₃₇N₃O₇ | 1144.8 9 | 9.62 | 26.69 | 2144 | C₆₀H₁₁₀N₂O₁₂S₃ | 1147.72 | 8.95 | 19.15 |
| 1945 | C₇₁H₁₃₇N₃O₇ | 1144.8 9 | 9.62 | 26.69 | 2145 | C₆₄H₁₁₃N₃O₄S₃ | 1084.80 | 10.17 | 23.74 |
| 1946 | C₇₁H₁₃₇N₃O₇ | 1144.8 9 | 9.62 | 26.69 | 2146 | C₆₀H₁₀₄N₂O₅S₃ | 1029.68 | 9.96 | 22.15 |
| 1947 | C₆₈H₁₃₇N₃O₄ | 1060.8 6 | 8.87 | 27.21 | 2147 | C₆₃H₁₀₉N₃O₆S₄ | 1132.82 | 9.65 | 22.68 |
| 1948 | C₆₈H₁₃₇N₃O₄ | 1060.8 6 | 8.87 | 27.21 | 2148 | C₆₂H₁₁₀N₂O₂S₇ | 1139.99 | 10.03 | 24.47 |
| 1949 | C₇₁H₁₃₇N₃O₇ | 1144.8 9 | 8.87 | 28.03 | 2149 | C₅₇H₁₀₂N₆O₃S | 951.54 | 8.64 | 15.85 |
| 1950 | C₇₁H₁₃₇N₃O₇ | 1144.8 9 | 8.87 | 28.03 | 2150 | C₅₃H₉₇NO₇ | 860.36 | 9.79 | 19.82 |
| 1951 | C₇₁H₁₃₇N₃O₇ | 1144.8 9 | 8.87 | 28.03 | 2151 | C₅₇H₁₀₃N₃O₂S₃ | 958.65 | 8.96 | 18.52 |
| 1952 | C₇₁H₁₃₇N₃O₇ | 1144.8 9 | 8.87 | 28.03 | 2152 | C₅₆H₁₀₃NS₄ | 918.69 | 10.21 | 21.10 |
| 1953 | C₆₈H₁₃₈N₄O₄ | 1075.8 8 | 8.77 | 25.07 | 2153 | C₅₆H₁₀₃NO₃S₇ | 1062.86 | 9.96 | 23.06 |
| 1954 | C₆₈H₁₃₈N₄O₄ | 1075.8 8 | 8.77 | 25.07 | 2154 | C₆₄H₁₁₉NO₈S₄ | 1158.89 | 8.40 | 26.14 |
| 1955 | C₇₁H₁₃₈N₄O₇ | 1159.9 1 | 8.77 | 25.89 | 2155 | C₆₇H₁₂₂N₄O₅S₄ | 1191.98 | 8.15 | 26.63 |
| 1956 | C₇₁H₁₃₈N₄O₇ | 1159.9 1 | 8.77 | 25.89 | 2156 | C₇₀H₁₂₈N₂O₅S₅ | 1238.10 | 8.80 | 28.64 |
| 1957 | C₇₁H₁₃₈N₄O₇ | 1159.9 1 | 8.77 | 25.89 | 2157 | C₆₇H₁₂₂N₂O₅S₄ | 1163.96 | 7.06 | 25.24 |
| 1958 | C₇₁H₁₃₈N₄O₇ | 1159.9 1 | 8.77 | 25.89 | 2158 | C₆₄H₁₁₇NOS₇ | 1141.07 | 9.43 | 28.02 |
| 1959 | C₆₇H₁₃₅N₃O₅ | 1062.8 3 | 7.19 | 26.00 | 2159 | C₇₂H₁₄₂N₇O₆P | 1232.94 | 10.10 | 26.28 |
| 1960 | C₆₇H₁₃₅N₃O₅ | 1062.8 3 | 7.19 | 26.00 | 2160 | C₇₂H₁₃₀N₆O₇S₂ | 1255.99 | 8.52 | 24.50 |
| 1961 | C₁₀₄H₁₉₇N₃O₇S₅ | 1762.03 | 7.97 | 43.84 | 2161 | C₆₇H₁₃₀N₆O₂ | 1051.82 | 8.75 | 23.16 |
| 1962 | C₉₉H₂₀₃N₂O₁₄P₃ S₂ | 1802.7 5 | 7.87 | 40.39 | 2162 | C₆₉H₁₃₀N₄O₇ | 1127.82 | 9.95 | 26.16 |
| 1963 | C₇₀H₁₃₅N₃O₈ | 1146.8 6 | 6.84 | 26.82 | 2163 | C₇₂H₁₂₉N₉O₆S | 1248.94 | 8.63 | 24.39 |
| 1964 | C₇₀H₁₃₅N₃O₈ | 1146.8 6 | 6.83 | 26.82 | 2164 | C₇₀H₁₃₁NO₁₀ | 1146.82 | 10.01 | 27.80 |
| 1965 | C₆₉H₁₃₉N₃O₄ | 1074.8 9 | 9.80 | 26.43 | 2165 | C₆₇Hₗ₂₁N₃O₈S₃ | 1192.90 | 7.17 | 27.01 |
| 1966 | C₆₉H₁₃₉N₃O₄ | 1074.8 9 | 9.80 | 26.43 | 2166 | C₆₆H₁₂₃N₃O₇S | 1102.78 | 7.67 | 23.92 |
| 1967 | C₇₂H₁₃₉N₃O₇ | 1158.9 2 | 9.80 | 27.25 | 2167 | C₇₂H₁₄₀N₂O₁₃P₄ | 1365.81 | 8.13 | 23.27 |
| 1968 | C₇₂H₁₃₉N₃O₇ | 1158.9 2 | 9.80 | 27.25 | 2168 | C₇₃H₁₃₉N₄O₁₃P₃ | 1373.85 | 7.94 | 22.39 |
| 1969 | C₇₂H₁₃₉N₃O₇₊₊ | 1158.9 2 | 9.80 | 27.25 | 2169 | C₆₄H₁₂₅N₃O₇S₃ | 1144.90 | 9.63 | 22.96 |
| 1970 | C₇₂H₁₃₉N₃O₇ | 1158.9 2 | 9.80 | 27.25 | 2170 | C₆₈H₁₂₇N₃O₇S₃ | 1194.96 | 10.18 | 28.67 |
| 1971 | C₆₆H₁₃₅N₃O₅ | 1050.8 2 | 8.20 | 25.62 | 2171 | C₆₉H₁₂₉NO₃S₇ | 1245.21 | 10.02 | 29.71 |
| 1972 | C₆₆H₁₃₅N₃O₅ | 1050.8 2 | 8.20 | 25.62 | 2172 | C₆₄H₁₁₈N₂O₂S₆ | 1140.02 | 7.72 | 28.06 |
| 1973 | C₆₉H₁₃₅N₃O₈ | 1134.8 5 | 8.20 | 26.44 | 2173 | C₆₅H₁₂₅NO₃S₆ | 1161.08 | 9.59 | 30.57 |
| 1974 | C₆₉H₁₃₅N₃O₈ | 1134.8 5 | 8.20 | 26.44 | 2174 | C₆₇H₁₂₉NO₂S₇ | 1205.19 | 10.25 | 31.99 |
| 1975 | C₆₉H₁₃₅N₃O₈ | 1134.8 5 | 8.20 | 26.44 | 2175 | C₆₃H₁₂₁N₃OS₈ | 1193.16 | 8.62 | 29.09 |
| 1976 | C₆₉H₁₃₅N₃O₈ | 1134.8 5 | 8.20 | 26.44 | 2176 | C₅₆H₁₀₄N₄O₃S₂ | 945.59 | 10.08 | 17.35 |
| 1977 | C₆₇H₁₃₇N₃O₅ | 1064.8 5 | 8.67 | 25.91 | 2177 | C₅₅H₁₀₃N₁₁O₄ | 982.50 | 7.63 | 16.96 |
| 1978 | C₆₇H₁₃₇N₃O₅ | 1064.8 5 | 8.67 | 25.91 | 2178 | C₅₅H₁₀₀N₄O₈S | 977.48 | 9.61 | 18.82 |
| 1979 | C₇₀H₁₃₇N₃O₈ | 1148.8 8 | 8.67 | 26.73 | 2179 | C₅₇H₁₀₈N₁₀O₈S₄ | 1189.79 | 8.50 | 16.73 |
| 1980 | C₇₀H₁₃₇N₃O₈ | 1148.8 8 | 8.67 | 26.73 | 2180 | C₅₃H₁₀₂N₆O₁₀S₄ | 1111.67 | 8.18 | 14.64 |
| 1981 | C₇₀H₁₃₇N₃O₈ | 1148.8 8 | 8.67 | 26.73 | 2181 | C₅₉H₁₀₀N₆O₈ | 1021.48 | 8.42 | 18.23 |
| 1982 | C₇₀H₁₃₇N₃O₈ | 1148.8 8 | 8.67 | 26.73 | 2182 | C₅₄H₉₈N₂O₈ | 903.38 | 8.85 | 20.07 |
| 1983 | C₆₈H₁₃₉N₃O₅ | 1078.8 8 | 8.87 | 25.64 | 2183 | C₅₆H₁₀₄N₄O₁₀S | 1025.53 | 9.53 | 19.39 |
| 1984 | C₆₈H₁₃₉N₃O₅ | 1078.8 8 | 8.87 | 25.64 | 2184 | C₅₃H₉₈N₄O₇S₂ | 967.51 | 8.78 | 17.62 |
| 1985 | C₇₁H₁₃₉N₃O₈ | 1162.9 1 | 8.87 | 26.46 | 2185 | C₅₄H₁₀₃NO₆S | 894.48 | 9.81 | 18.88 |
| 1986 | C₉₃H₁₈₇NO₈S₆ | 1639.8 8 | 8.87 | 39.61 | 2186 | C₆₀H₁₁₅N₄O₁₂P₃ S | 1209.58 | 7.38 | 16.63 |
| 1987 | C₇₁H₁₃₉N₃O₈ | 1162.9 1 | 8.87 | 26.46 | 2187 | C₄₈H₉₁NO₁₄S₄ | 1034.49 | 8.57 | 18.09 |
| 1988 | C₇₁H₁₃₉N₃O₈ | 1162.9 1 | 8.87 | 26.46 | 2188 | C₅₃H₉₇N₃O₇S₃ | 984.55 | 7.65 | 15.34 |
| 1989 | C₆₈H₁₃₈N₄O₆ | 1107.8 7 | 7.80 | 25.43 | 2189 | C₅₃H₁₀₀N₄O₁₀S₄ | 1081.64 | 8.02 | 15.11 |
| 1990 | C₆₈H₁₃₈N₄O₆ | 1107.8 7 | 7.80 | 25.43 | 2190 | C₅₉H₁₀₄N₂O₈S₄ | 1097.73 | 8.07 | 20.70 |
| 1991 | C₉₈H₁₉₅N₅O₅S₃ | 1619.8 5 | 7.45 | 41.60 | 2191 | C₅₇H₁₀₃NO₆S₄ | 1026.69 | 10.16 | 22.82 |
| 1992 | C₉₈H₁₉₃NO₁₀S₃ | 1641.8 0 | 9.82 | 42.89 | 2192 | C₅₇H₁₀₃NO₆S₆ | 1090.81 | 8.72 | 25.21 |
| 1993 | C₇₁H₁₃₈N₄O₉ | 1191.9 0 | 7.80 | 26.25 | 2193 | C₅₇H₁₀₃NO₆S₇ | 1122.87 | 9.87 | 23.86 |
| 1994 | C₇₁H₁₃₈N₄O₉ | 1191.9 0 | 7.80 | 26.25 | 2194 | C₅₇H₉₉N₃O₈S₇ | 1178.85 | 7.78 | 21.15 |
| 1995 | C₆₉H₁₃₇N₅O₅ | 1116.8 8 | 7.49 | 25.53 | 2195 | C₅₆H₁₀₉N₂O₃PS ₆ | 1081.83 | 8.96 | 22.34 |
| 1996 | C₆₉H₁₃₇N₅O₅ | 1116.8 8 | 7.49 | 25.53 | 2196 | C₆₉H₁₂₁NO₅S | 1076.79 | 9.98 | 23.52 |
| 1997 | C₇₂H₁₃₇N₅O₈ | 1200.9 1 | 7.49 | 26.35 | 2197 | C₇₀H₁₂₂N₄O₂S | 1147.95 | 8.85 | 22.42 |
| 1998 | C₇₂H₁₃₇N₅O₈ | 1200.91 | 7.49 | 26.35 | 2198 | C₇₂H₁₂₃N₉O₇ | 1226.83 | 8.52 | 18.69 |
| 1999 | C₇₂H₁₃₇N₅O₈ | 1200.9 1 | 7.49 | 26.35 | 2199 | C₆₇H₁₁₇N₅O₅ | 1072.70 | 8.36 | 19.23 |
| 2000 | C₇₂H₁₃₇N₅O₈ | 1200.9 1 | 7.49 | 26.35 | 2200 | C₆₈H₁₂₇N₁₁O₄ | 1162.84 | 9.49 | 19.64 |
| 2201 | C₆₈H₁₂₅N₇O₅ | 1120.7 9 | 9.91 | 22.00 | 2401 | C₇₇H₁₅₇N₃O₁₀S₃ | 1381.31 | 10.29 | 31.76 |
| 2202 | C₇₀H₁₂₆N₁₀O₇S | 1251.9 0 | 7.99 | 20.23 | 2402 | C₇₇H₁₄₈N₄O₇S₇ | 1466.52 | 7.62 | 33.01 |
| 2203 | C₆₇H₁₁₈N₈O₈ | 1163.7 3 | 7.71 | 21.54 | 2403 | C₇₆H₁₄₇NO₂S₆ | 1299.42 | 10.02 | 34.04 |
| 2204 | C₆₇H₁₂₃N₇O₂ | 1058.7 7 | 8.87 | 20.62 | 2404 | C₇₆H₁₅₃NOS₈ | 1353.60 | 10.09 | 38.26 |
| 2205 | C₆₇H₁₂₂N₁₀O | 1083.7 8 | 8.58 | 19.27 | 2405 | C₇₅H₁₄₉N₃O₂S₈ | 1381.57 | 8.73 | 35.21 |
| 2206 | C₆₃H₁₁₈N₄O₆S₅ | 1187.9 6 | 9.68 | 21.42 | 2406 | C₉₉H₁₉₃N₇O₆ | 1577.68 | 8.36 | 38.16 |
| 2207 | C₆₅H₁₁₁NO₇S | 1050.6 6 | 8.10 | 22.75 | 2407 | C₉₉H₁₈₉N₃O₅S₃ | 1597.82 | 8.77 | 42.25 |
| 2208 | C₇₀H₁₁₉N₃O₉S₅ | 1307.0 3 | 7.99 | 25.56 | 2408 | C₉₆H₁₈₉NO₃S₅ | 1565.91 | 9.91 | 45.50 |
| 2209 | C₆₇H₁₁₈N₂O₁₂S₅ | 1303.9 8 | 8.08 | 20.95 | 2409 | C₁₀₆H₂₀₅NO₅ | 1573.81 | 9.90 | 46.33 |
| 2210 | C₆₄H₁₁₇NO₁₃S₃ | 1204.8 1 | 9.78 | 23.12 | 2410 | C₁₀₇H₂₀₉N₅O₅ | 1645.88 | 8.96 | 44.62 |
| 2211 | C₆₁H₁₁₂N₂O₁₄S₃ | 1193.7 5 | 9.49 | 22.15 | 2411 | C₁₀₅H₂₀₄N₆O₃S₂ | 1662.96 | 8.70 | 42.53 |
| 2212 | C₇₂H₁₃₃N₄O₁₂P₃ | 1339.7 9 | 8.12 | 20.87 | 2412 | C₁₀₆H₂₀₅N₃O₉ | 1665.82 | 8.80 | 45.11 |
| 2213 | C₆₈H₁₂₈NO₁₀P₃ S₂ | 1276.8 1 | 9.04 | 21.27 | 2413 | C₁₀₄H₂₀₄N₂O₃S₃ | 1626.99 | 9.75 | 44.82 |
| 2214 | C₆₅H₁₁₇NO₇S₄ | 1152.8 9 | 9.86 | 19.82 | 2414 | C₁₀₄H₂₀₁NOS₇ | 1706.23 | 9.86 | 49.18 |
| 2215 | C₆₆H₁₂₀N₆O₈S₃ | 1221.9 0 | 9.07 | 19.62 | 2415 | C₁₀H₂₀₄N₃O₅P S₆ | 1812.18 | 7.51 | 47.43 |
| 2216 | C₆₈H₁₂₄N₂O₁₀S₅ | 1290.0 4 | 9.08 | 22.31 | 2416 | C₁₀₅H₂₀₆N₆O₆S | 1680.91 | 8.82 | 42.03 |
| 2217 | C₆₃H₁₁₂N₄O₂S₆ | 1149.9 7 | 7.37 | 24.03 | 2417 | C₁₀₃H₂₀₄N₃O₄S₂ | 1682.95 | 8.74 | 43.18 |
| 2218 | C₇₀H₁₃₁N₃O₄S₂ | 1142.9 5 | 8.36 | 25.63 | 2418 | C₁₀₆H₂₁₁N₇O₂ | 1615.90 | 8.51 | 43.80 |
| 2219 | C₆₇H₁₃₀N₂O₅ | 1043.7 9 | 9.54 | 27.28 | 2419 | C₁₀₃H₂₁₁N₄O₃P | 1584.82 | 9.91 | 45.36 |
| 2220 | C₆₈H₁₃₁NO₅S₄ | 1171.0 4 | 8.83 | 28.53 | 2420 | C₁₀₄H₂₀₈N₆O₆ | 1638.84 | 8.96 | 43.83 |
| 2221 | C₈₇H₁₆₉NO₅S | 1341.3 7 | 9.31 | 36.31 | 2421 | C₁₀₇H₂₀₈N₆O₁₀S | 1770.94 | 8.19 | 44.86 |
| 2222 | C₈₈H₁₇₂N₂O₅ | 1338.3 5 | 9.01 | 37.81 | 2422 | C₁₀₂H₂₀₂N₄O₈S | 1644.82 | 9.95 | 44.99 |
| 2223 | C₈₇H₁₆₉NO₆S₃ | 1421.4 9 | 9.94 | 39.01 | 2423 | C₁₀₆H₂₀₈N₄O₇S₃ | 1747.05 | 9.64 | 47.63 |
| 2224 | C₉₂H₁₇₅N₆O₇PS 3 | 1604.6 0 | 7.83 | 37.76 | 2424 | C₁₀₆H₂₀₇N₄O₉P S₃ | 1809.02 | 8.18 | 47.75 |
| 2225 | C₈₇H₁₇₀N₄O₂S₆ | 1496.7 0 | 8.30 | 39.06 | 2425 | C₁₀₂H₂₀₁NO₉S₄ | 1714.00 | 9.33 | 48.93 |
| 2226 | C₈₂H₁₆₈N₄OS | 1258.3 3 | 9.43 | 35.75 | 2426 | C₁₀₄H₂₀₁NO₇S₄ | 1706.02 | 9.76 | 48.92 |
| 2227 | C₈₉H₁₇₃NO₉S₄ | 1529.6 0 | 9.78 | 39.84 | 2427 | C₁₀₂H₂₀₃NO₅ | 1523.75 | 9.86 | 45.85 |
| 2228 | C₈₇H₁₇₅NO₁₆S₄ | 1619.5 9 | 9.94 | 38.90 | 2428 | C₁₀₈H₂₁₄N₇O₁₂P₃ | 1895.86 | 7.23 | 41.61 |
| 2229 | C₈₉H₁₇₇NO₁₃S₄ | **1597.6** 3 | 10.04 | 39.39 | 2429 | C₁₀₀H₁₉₉N₃O₁₅S₅ | 1844.03 | 7.71 | 46.10 |
| 2230 | C₈₅H₁₇₂N₆O₁₀S₄ | 1566.5 8 | 8.43 | 34.65 | 2430 | C₉₉H₁₉₉NO₁₄S₅ | 1788.01 | 8.79 | 44.17 |
| 2231 | C₈₅H₁₇₅N₅O₉S₃ | 1507.5 4 | 9.58 | 34.71 | 2431 | C₁₀₃H₂₀₆N₆O₇S₄ | 1769.08 | 8.96 | 44.54 |
| 2232 | C₈₅H₁₇₄N₂O₁₁S₄ | 1528.6 0 | 8.94 | 37.92 | 2432 | C₁₀₃H₂₀₉N₆O₁₀P S₃ | 1819.01 | 8.49 | 41.31 |
| 2233 | C₈₅H₁₇₁N₃O₁₀S₄ | 1523.5 8 | 8.67 | 35.43 | 2433 | C₉₉H₂₀₁N₃O₁₀S₃ | 1689.91 | 9.61 | 44.80 |
| 2234 | C₉₀H₁₇₃N₃O₆S₄ | 1521.6 6 | 8.82 | 38.34 | 2434 | C₁₀₁H₂₀₅NO₁₀S₅ | 1754.08 | 9.99 | 43.99 |
| 2235 | C₉₀H₁₇₃NO₃S₈ | 1573.91 | 9.95 | 43.51 | 2435 | C₁₀₄H₁₉₉N₃O₅S₅ | 1732.09 | 7.64 | 46.37 |
| 2236 | C₇₂H₁₄₀N₆O₅ | 1169.9 5 | 8.47 | 24.78 | 2436 | C₁₀₃H₁₉₉N₃O₉S₄ | 1752.00 | 7.51 | 47.66 |
| 2237 | C₆₆H₁₃₇N₇O₂ | 1060.8 7 | 8.33 | 24.92 | 2437 | C₉₇H₁₉₃N₇O₅S | 1569.72 | 9.22 | 38.28 |
| 2238 | C₇₃H₁₄₁N₃O₇S₂ | 1237.0 8 | 9.74 | 28.32 | 2438 | C₉₃H₁₈₈N₆O₇S₄ | 1630.83 | 7.70 | 37.71 |
| 2239 | C₆₈H₁₃₆N₄O₈ | 1137.8 6 | 9.16 | 26.01 | 2439 | C₁₀₀H₁₉₆N₄O₈ | 1582.69 | 9.91 | 43.00 |
| 2240 | C₇₀H₁₄₀N₄O₁₀S | 1229.9 7 | 9.11 | 27.00 | 2440 | C₉₉H₁₉₂N₂O₉S₄ | 1682.90 | 8.81 | 44.24 |
| 2241 | C₆₈H₁₃₃NO₁₁S | 1172.8 7 | 9.51 | 27.24 | 2441 | C₉₄H₁₉₁NO₃S | 1415.63 | 10.10 | 42.86 |
| 2242 | C₆₉H₁₃₁NO₁₀S | 1166.8 7 | 9.12 | 27.95 | 2442 | C₁₀H₂₀₅N₂O₁₁P₃ | 1704.66 | 10.14 | 43.84 |
| 2243 | C₆₇H₁₃₅NO₄S | 1050.8 9 | 9.96 | 27.64 | 2443 | C₉₂H₁₈₆N₂O₁₁S₃ | 1592.70 | 8.75 | 40.86 |
| 2244 | C₇₀H₁₄₅N₂O₁₂P₃ | 1299.8 5 | 7.89 | 25.09 | 2444 | C₉₄H₁₈₇N₃O₁₅S₃ | 1695.74 | 7.43 | 40.74 |
| 2245 | C₇₄H₁₅₂NO₁₂P₃ S | 1373.0 1 | 9.94 | 27.12 | 2445 | C₉₃H₁₉₀N₂O₆S₃ | 1528.75 | 10.06 | 38.72 |
| 2246 | C₆₉H₁₃₈N₂O₁₀S₃ | 1252.0 7 | 9.92 | 27.38 | 2446 | C₉₄H₁₈₇N₃O₁₂S₄ | 1679.81 | 7.37 | 39.57 |
| 2247 | C₆₉H₁₃₉NO₁₅S₄ | 1351.1 3 | 9.90 | 28.99 | 2447 | C₁₀₁H₁₉₆N₃O₇P S₆ | 1788.07 | 8.79 | 44.46 |
| 2248 | C₇₂H₁₃₈N₆O₁₃S₅ | 1456.2 6 | 8.64 | 26.43 | 2448 | C₉₄H₁₈₇NOS₆ | 1539.94 | 9.73 | 45.76 |
| 2249 | C₇₀H₁₃₇N₃O₉S₃ | 1261.0 8 | 8.66 | 22.65 | 2449 | C₇₇H₁₂₀N₆O₃S₂ | 1241.98 | 8.70 | 26.41 |
| 2250 | C₆₇H₁₃₈N₆O₇S₃ | 1236.0 8 | 8.87 | 26.36 | 2450 | C₇₅H₁₂₄N₄O₆ | 1177.84 | 10.20 | 25.90 |
| 2251 | C₆₉H₁₃₇N₃O₁₃S₃ | 1313.0 6 | 8.54 | 25.97 | 2451 | C₇₂H₁₁₅NO₆S₂ | 1154.85 | 10.06 | 27.89 |
| 2252 | C₇₂H₁₃₇N₃O₂S₆ | 1269.3 1 | 8.65 | 29.55 | 2452 | C₇₆H₁₂₅N₅O₄S₃ | 1269.07 | 9.66 | 26.77 |
| 2253 | C₇₄H₁₄₆NO₃PS₆ | 1321.3 6 | 10.13 | 32.30 | 2453 | C₇₅H₁₁₇N₃O₂S₈ | 1349.32 | 7.66 | 27.75 |
| 2254 | C₉₀H₁₄₇N₃O₈ | 1399.1 8 | 7.90 | 32.42 | 2454 | C₇₇H₁₂₃N₅O₆S | 1246.93 | 8.19 | 24.95 |
| 2255 | C₈₉H₁₄₉NO₆S | 1361.2 4 | 9.32 | 34.59 | 2455 | C₇₆H₁₂₇N₇O₄ | 1202.90 | 9.00 | 24.97 |
| 2256 | C₈₈H₁₄₆N₂O₇S | 1376.2 1 | 6.19 | 33.17 | 2456 | C₇₅H₁₂₆N₆O₈ | 1239.87 | 9.84 | 25.43 |
| 2257 | C₈₈H₁₅₀N₄O₄S | 1360.2 6 | 10.02 | 32.47 | 2457 | C₆₈H₁₁₈NaO₃S | 1071.78 | 9.27 | 26.81 |
| 2258 | C₉₂H₁₅₄N₆O₃S | 1488.4 8 | 9.03 | 34.03 | 2458 | C₇₄H₁₂₈N₄O₈S₄ | 1330.13 | 10.33 | 26.97 |
| 2259 | C₈₉H₁₅₀N₂O₇ | 1360.1 9 | 10.09 | 35.36 | 2459 | C₇₄H₁₁₉NO₉ | 1166.76 | 10.00 | 26.77 |
| 2260 | C₈₈H₁₄₄N₂O₇S | 1374.1 9 | 8.78 | 34.21 | 2460 | C₇₂H₁₁₈N₂O₁₀ | 1171.74 | 9.32 | 24.67 |
| 2261 | C₉₀H₁₅₅N₇O₅ | 1415.2 7 | 9.54 | 32.12 | 2461 | C₇₈H₁₂₁N₃O₁₂S₂ | 1356.97 | 8.21 | 25.60 |
| 2262 | C₉₀H₁₅₈N₅O₅P | 1421.2 6 | 8.10 | 33.64 | 2462 | C₈₀H₁₃₇N₂O₁₁P₃ | 1395.90 | 8.95 | 25.51 |
| 2263 | C₉₀H₁₅₃N₇O₉S | 1509.3 2 | 8.41 | 32.06 | 2463 | C₇₇H₁₃₅N₄O₁₀P₃ S | 1401.93 | 7.50 | 25.37 |
| 2264 | C₉₃H₁₆₄N₃O₁₁P₃ S | 1625.3 3 | 8.74 | 32.17 | 2464 | C₆₉H₁₁₈N₄O₉S₄ | 1275.99 | 7.50 | 25.37 |
| 2265 | C₉₂H₁₆₄NO₁₄P₃ S | 1633.3 1 | 7.95 | 33.25 | 2465 | C₇₁H₁₁₉NO₁₃S₄ | 1322.99 | 9.94 | 25.39 |
| 2266 | C₈₈H₁₅₀N₄O₁₂S₄ | 1584.4 5 | 8.03 | 31.72 | 2466 | C₇₂H₁₁₅N₃O₃S₄ | 1199.00 | 8.42 | 26.27 |
| 2267 | C₈₈H₁₅₃N₃O₁₃S₃ | 1557.4 0 | 9.96 | 35.94 | 2467 | C₇₃H₁₁₈N₂O₂S₇ | 1280.23 | 9.68 | 28.94 |
| 2268 | C₉₃H₁₆₇N₂O₁₁P₃ | 1582.2 8 | 10.00 | 33.87 | 2468 | C₇₀H₁₃₃NO₄S₂ | 1116.97 | 9.97 | 28.02 |
| 2269 | C₉₇H₁₆₈N₃O₁₂P₃ S | 1693.4 1 | 8.60 | 32.71 | 2469 | C₆₇H₁₂₉NO₇ | 1060.77 | 8.51 | 28.62 |
| 2270 | C₈₇H₁₅₁N₃O₉S₃ | 1479.3 8 | 8.83 | 30.34 | 2470 | C₆₈H₁₂₇N₃O₂S₃ | 1114.98 | 8.46 | 26.40 |
| 2271 | C₈₇H₁₅₄N₆O₇S₅ | 1556.5 6 | 8.94 | 32.10 | 2471 | C₆₆H₁₂₄N₄S₄ | 1102.01 | 7.24 | 26.38 |
| 2272 | C₈₇H₁₄₃NO₅S₇ | 1507.57 | 9.36 | 38.83 | 2472 | C₆₇H₁₃₅N₇O₃S | 1118.93 | 8.80 | 26.76 |
| 2273 | C₈₈H₁₄₄N₂O₅S₆ | 1502.5 3 | 8.66 | 36.59 | 2473 | C₆₅H₁₃₃N₅O₂ | 1016.81 | 9.94 | 25.89 |
| 2274 | C₈₉H₁₅₃N₅OS₃ | 1405.4 4 | 9.38 | 36.17 | 2474 | C₆₂H₁₂₇N₅O₈S₃ | 1166.93 | 8.18 | 24.53 |
| 2275 | C₈₈H₁₅₂N₄O₂S₄ | 1426.4 8 | 8.67 | 36.49 | 2475 | C₆₇H₁₃₈N₄O₆S₅ | 1256.20 | 9.66 | 27.43 |
| 2276 | C₅₃H₁₀₁N₇O₅ | 916.43 | 9.37 | 14.69 | 2476 | C₆₅H₁₃₄N₆O₇S₅ | 1272.16 | 8.65 | 24.58 |
| 2277 | C₄₈H₉₁NO₉S | 858.32 | 7.45 | 18.24 | 2477 | C₆₉H₁₂₉N₅O₈ | 1156.82 | 7.35 | 25.88 |
| 2278 | C₄₉H₉₃NO₇S | 840.35 | 9.87 | 18.01 | 2478 | C₆₄H₁₂₃NO₁₀S | 1098.75 | 8.66 | 26.59 |
| 2279 | C₅₀H₉₂N₄O₆S₃ | 941.51 | 8.18 | 17.07 | 2479 | C₆₈H₁₃₀N₄O₈S₅ | 1292.14 | 8.76 | 29.68 |
| 2280 | C₇₃H₁₃₅NO₅ | 1106.8 8 | 9.49 | 28.47 | 2480 | C₇₀H₁₂₉N₃O₉S₄ | 1285.08 | 7.88 | 28.40 |
| 2281 | C₇₀H₁₃₂N₄O₃S₂ | 1141.9 8 | 9.64 | 24.97 | 2481 | C₇₁H₁₄₆NO₁₃P₃ S | 1346.93 | 9.92 | 26.28 |
| 2282 | C₇₀H₁₂₆N₄O₅S₃ | 1200.0 0 | 8.18 | 25.90 | 2482 | C₆₅H₁₃₁NO₇S₅ | 1199.10 | 9.82 | 22.46 |
| 2283 | C₇₆H₁₃₈N₃O₈PS ₃ | 1349.1 3 | 8.02 | 27.79 | 2483 | C₆₂H₁₂₈N₄O₇S₅ | 1202.06 | 9.66 | 24.80 |
| 2284 | C₇₀H₁₂₅N₃O₂S₄ | 1169.0 6 | 8.09 | 24.80 | 2484 | C₆₇H₁₃₆N₂O₂S₃ | 1098.04 | 9.60 | 31.20 |
| 2285 | C₆₉H₁₃₀N₄O₅S | 1127.8 9 | 8.62 | 22.64 | 2485 | C₆₇H₁₃₄N₄O₂S₆ | 1220.24 | 8.81 | 29.65 |
| 2286 | C₆₆H₁₂₉N₇O₄S | 1116.8 7 | 8.42 | 23.80 | 2486 | C₅₀H₉₆N₄O₅ | 833.34 | 7.85 | 14.83 |
| 2287 | C₆₉H₁₃₁N₅O₈S | 1190.9 0 | 8.72 | 26.48 | 2487 | C₄₉H₁₀₁N₈O₈P | 961.37 | 7.10 | 14.90 |
| 2288 | C₆₉H₁₃₂N₄O₉S | 1193.9 0 | 9.26 | 26.76 | 2488 | C₄₈H₁₀₀N₄S | 765.42 | 10.17 | 17.18 |
| 2289 | C₆₇H₁₃₄N₄O₂S | 1059.9 0 | 9.61 | 25.59 | 2489 | C₅₀H₉₉N₅O₁₂S₃ | 1058.57 | 7.89 | 15.01 |
| 2290 | C₇₁H₁₃₈N₆O₈S₃ | 1300.1 2 | 8.97 | 23.96 | 2490 | C₅₃H₁₀₀N₆O₉ | 965.42 | 8.13 | 16.59 |
| 2291 | C₆₈H₁₃₆N₄O₁₀S₄ | 1298.1 2 | 9.75 | 23.58 | 2491 | C₄₉H₉₆N₆O₇ | 881.34 | 8.08 | 16.13 |
| 2292 | C₇₂H₁₃₀N₄O₉S₂ | 1259.9 8 | 8.25 | 26.34 | 2492 | C₅₂H₉₆N₄O₇S₄ | 1017.63 | 8.99 | 20.64 |
| 2293 | C₇₀H₁₂₇N₃O₁₁ | 1186.8 0 | 8.54 | 23.99 | 2493 | C₅₀H₉₁N₃O₇S₅ | 1006.63 | 8.54 | 18.41 |
| 2294 | C₇₂H₁₃₁NO₉S₃ | 1251.0 4 | 7.85 | 29.49 | 2494 | C₄₆H₉₃NO₅S | 772.32 | 9.48 | 16.00 |
| 2295 | C₇₀H₁₃₆NO₆P | 1118.8 3 | 9.96 | 26.88 | 2495 | C₄₉H₁₀₂NO₁₀P₃ S₂ | 1022.41 | 9.45 | 15.88 |
| 2296 | C₇₅H₁₅₀NO₁₁P₃ S | 1367.0 1 | 10.01 | 26.33 | 2496 | C₄₄H₉₀N₂O₁₁S₅ | 983.54 | 7.61 | 14.61 |
| 2297 | C₆₈H₁₂₉N₅O₁₀S₃ | 1273.0 1 | 8.02 | 23.99 | 2497 | C₅₀H₉₇N₃O₁₃S₅ | 1108.67 | 9.10 | 18.90 |
| 2298 | C₆₂H₁₂₁NO₇S₃ | 1088.8 5 | 8.69 | 22.15 | 2498 | C₄₆H₉₆N₄O₇S₅ | 977.63 | 9.97 | 15.88 |
| 2299 | C₇₂H₁₃₄N₂O₄S₃ | 1188.0 7 | 8.69 | 30.53 | 2499 | C₅₁H₉₇NO₈S₃ | 948.54 | 9.74 | 20.24 |
| 2300 | C₇₀H₁₃₀N₂O₈S₄ | 1256.0 8 | 9.65 | 29.21 | 2500 | C₄₈H₈₉NO₉S₃ | 920.44 | 7.12 | 19.34 |
| 2301 | C₇₀H₁₂₀NO₆S₄ | 1209.0 7 | 9.97 | 29.90 | 2501 | C₅₀H₉₈N₂O₅S₇ | 1031.81 | 10.01 | 21.72 |
| 2302 | C₇₃H₁₃₃N₃O₆S₄ | 1277.1 5 | 8.91 | 29.86 | 2502 | C₄₉H₈₉N₃O₅S₇ | 1024.74 | 7.38 | 20.15 |
| 2303 | C₇₀H₁₂₇N₃O₅S₇ | 1315.2 7 | 8.15 | 29.29 | 2503 | C₈₈H₁₇₄N₄O₁₀S | 1480.44 | 8.64 | 37.76 |
| 2304 | C₆₉H₁₃₃NO₂S₄ | 1137.1 0 | 10.08 | 29.80 | 2504 | C₉₁H₁₈₀NO₁₂P | 1511.41 | 9.64 | 38.49 |
| 2305 | C₇₂H₁₄₂N₂O₉S | 1212.0 0 | 10.03 | 27.69 | 2505 | C₈₇H₁₇₂N₂O₁₃S₃ | 1550.51 | 7.44 | 36.68 |
| 2306 | C₇₀H₁₃₅NO₂S₃ | 1119.0 6 | 9.94 | 28.41 | 2506 | C₈₉H₁₇₇NO₁₃S₄ | 1597.63 | 9.89 | 39.95 |
| 2307 | C₇₄H₁₄₃NS₄ | 1175.2 3 | 10.31 | 30.33 | 2507 | C₈₇H₁₇₉N₅O₈S₄ | 1551.66 | 9.72 | 35.89 |
| 2308 | C₇₃H₁₄₂N₆O₉S | 1280.0 4 | 8.87 | 27.51 | 2508 | C₉₁H₁₇₃N₃O₅S₇ | 1613.82 | 8.49 | 39.46 |
| 2309 | C₇₁H₁₄₃N₇O₂ | 1126.9 7 | 8.80 | 25.07 | 2509 | C₈₆H₁₇₃NO₂S₃ | 1349.52 | 8.69 | 40.39 |
| 2310 | C₆₇H₁₃₆N₆O₄ | 1089.8 6 | 7.38 | 25.24 | 2510 | C₈₇H₁₇₇NO₃S₄ | 1413.62 | 9.80 | 39.79 |
| 2311 | C₆₅H₁₃₆N₄O₂S | 1037.90 | 9.89 | 25.86 | 2511 | C₆₈H₁₃₈N₆OS | 1087.95 | 8.34 | 25.64 |
| 2312 | C₇₀H₁₃₃NO₆S | 1116.9 0 | 9.78 | 27.91 | 2512 | C₇₃H₁₄₁N₅O₇S | 1233.02 | 8.19 | 27.42 |
| 2313 | C₇₂H₁₃₉NO₈ | 1146.9 0 | 8.29 | 30.37 | 2513 | C₇₆H₁₄₈N₆O₁₀ | 1306.05 | 8.77 | 28.04 |
| 2314 | C₇₂H₁₃₇N₃O₈S | 1204.9 7 | 8.10 | 27.01 | 2514 | C₇₇H₁₄₅N₅O₁₂ | 1333.03 | 8.22 | 27.36 |
| 2315 | C₇₀H₁₃₅NO₈S₃ | 1215.0 5 | 8.87 | 31.23 | 2515 | C₇₂H₁₃₉NO₇S₄ | 1259.14 | 9.28 | 30.05 |
| 2316 | C₆₉H₁₃₄N₂O₉S₃ | 1232.0 4 | 8.56 | 29.17 | 2516 | C₇₄H₁₄₈N₃O₆P | 1206.99 | 9.19 | 27.09 |
| 2317 | C₆₇H₁₃₁N₃O₆ | 1074.8 0 | 8.05 | 24.99 | 2517 | C₇₃H₁₄₃N₃O₇S₅ | 1335.26 | 9.00 | 27.79 |
| 2318 | C₇₁H₁₄₁N₃O₆ | 1132.9 2 | 8.87 | 27.00 | 2518 | C₇₄H₁₄₄N₂O₅S₅ | 1302.27 | 8.94 | 31.06 |
| 2319 | C₆₉H₁₃₈N₄O₁₅S₃ | 1360.0 8 | 8.57 | 27.02 | 2519 | C₇₄H₁₄₁NO₈S₄ | 1301.18 | 9.84 | 32.75 |
| 2320 | C₇₆H₁₅₂N₃O₁₁P₃ S₂ | 1441.1 2 | 8.65 | 25.02 | 2520 | C₇₅H₁₄₃N₅O₆S₆ | 1403.36 | 8.02 | 29.84 |
| 2321 | C₇₀H₁₄₅N₅O₆S₃ | 1249.1 6 | 10.17 | 27.30 | 2521 | C₇₅H₁₄₃NOS₆ | 1267.34 | 10.03 | 31.57 |
| 2322 | C₇₀H₁₃₃NO₅S₇ | 1293.3 0 | 9.72 | 32.57 | 2522 | C₆₉H₁₃₅N₃OS₈ | 1231.28 | 9.26 | 31.21 |
| 2323 | C₉₈H₁₉₃N₃O₅ | 1493.6 4 | 9.64 | 41.19 | 2523 | C₆₉H₁₃₅N₃OS₈ | 1279.34 | 8.11 | 32.33 |
| 2324 | C₉₇H₁₉₂N₂O₃ | 1434.6 1 | 9.81 | 43.28 | 2524 | C₉₁H₁₅₁NO₆ | 1355.21 | 9.47 | 35.21 |
| 2325 | C₁₀₀H₁₉₃N₃O₃S₅ | 1646.0 0 | 9.08 | 44.26 | 2525 | C₈₉H₁₅₁NO₈S | 1395.25 | 9.10 | 33.28 |
| 2326 | C₁₀₀H₁₉₄N₂O₂S₄ | 1584.9 3 | 8.96 | 42.63 | 2526 | C₉₃H₁₅₆N₆O₅ | 1438.31 | 8.90 | 31.09 |
| 2327 | C₉₇H₁₉₀N₂OS₆ | 1593.0 0 | 7.95 | 44.89 | 2527 | C₉₀H₁₄₉NO₈S | 1405.24 | 9.77 | 36.24 |
| 2328 | C₁₀₁H₁₉₈N₈O₆S | 1652.8 1 | 9.03 | 41.03 | 2528 | C₉₁H₁₅₇N₇O₄ | 1413.30 | 9.81 | 33.65 |
| 2329 | C₉₈H₁₉₃N₅O₈ | 1569.6 5 | 9.94 | 42.17 | 2529 | C₉₃H₁₅₈N₆O₉S | 1536.38 | 9.06 | 33.18 |
| 2330 | C₉₈H₁₉₆N₆O₂S | 1522.7 5 | 8.97 | 42.54 | 2530 | C₈₈H₁₅₄N₆O₂ | 1328.24 | 9.69 | 33.16 |
| 2331 | C₉₅H₁₈₇NO₈S | 1503.6 1 | 8.62 | 43.07 | 2531 | C₈₉H₁₅₈N₄O₇S₅ | 1556.56 | 9.19 | 36.70 |
| 2332 | C₁₀₀H₁₉₆N₄O₁₀ | 1614.6 9 | 8.95 | 42.32 | 2532 | C₉₁H₁₅₅N₅O₈ | 1447.27 | 9.75 | 33.92 |
| 2333 | C₉₆H₁₈₉NO₉S₂ | 1565.7 0 | 9.90 | 43.36 | 2533 | C₉₁H₁₅₃N₃O₁₀S | 1481.30 | 9.45 | 33.16 |
| 2334 | C₉₈H₁₈₉NO₈S₃ | 1605.7 9 | 7.84 | 44.98 | 2534 | C₈₈H₁₅₄N₄O₅ | 1348.22 | 9.44 | 33.71 |
| 2335 | C₁₀₄H₂₁₀N₃O₂P₃ | 1787.7 5 | 8.95 | 40.77 | 2535 | C₈₅H₁₄₀NO₁₁S₄ | 1489.36 | 9.07 | 32.92 |
| 2336 | C₉₅H₁₉₂NO₉PS₃ | 1619.7 5 | 9.61 | 38.95 | 2536 | C₈₃H₁₄₅NO₂S₃ | 1285.26 | 8.63 | 34.83 |
| 2337 | C₉₆H₁₉₆N₆O₉S₃ | 1674.8 6 | 9.53 | 39.50 | 2537 | C₈₉H₁₅₂N₄O₂S₆ | 1502.58 | 8.69 | 33.48 |
| 2338 | C₉₆H₁₉₄N₆O₉S₄ | 1704.9 1 | 8.66 | 39.31 | 2538 | C₈₇H₁₅₃NO₄S₇ | 1501.60 | 9.11 | 35.87 |
| 2339 | C₉₇H₁₉₇NO₉S₅ | 1681.9 8 | 10.29 | 43.64 | 2539 | C₉₀H₁₅₃N₃O₂S₇ | 1533.65 | 8.67 | 35.66 |
| 2340 | C₁₀₀H₁₉₃N₅O₉S₃ | 1705.8 7 | 8.02 | 43.88 | 2540 | C₉₀H₁₅₅N₃O₃S₇ | 1551.67 | 8.78 | 37.08 |
| 2341 | C₉₃H₁₈₅NO₂S₄ | 1477.7 8 | 8.83 | 44.69 | 2541 | C₅₀H₉₂N₄O₈ | 877.31 | 7.61 | 14.76 |
| 2342 | C₉₆H₁₉₅NS₅ | 1523.9 6 | 10.32 | 46.50 | 2542 | C₅₄H₁₀₁N₃O₁₀S | 984.47 | 8.61 | 17.32 |
| 2343 | C₉₆H₁₉₂N₄O₂S₇ | 1659.0 9 | 9.15 | 45.27 | 2543 | C₅₅H₁₀₇N₃OS₃ | 922.66 | 10.35 | 19.69 |
| 2344 | C₇₂H₁₃₇N₇O₆ | 1196.9 3 | 9.55 | 23.01 | 2544 | C₇₃H₁₃₃N₃O₇ | 1164.88 | 8.78 | 26.27 |
| 2345 | C₇₅H₁₃₆N₆O₆S | 1250.0 2 | 8.12 | 23.71 | 2545 | C₇₂H₁₃₉N₄O₆P | 1187.90 | 8.50 | 25.02 |
| 2346 | C₇₃H₁₃₃N₃O₃S₄ | 1229.1 5 | 8.60 | 25.60 | 2546 | C₇₆H₁₄₅N₉O₆ | 1281.05 | 8.95 | 25.90 |
| 2347 | C₇₂H₁₃₄N₂O₂S₆ | 1252.2 8 | 8.91 | 31.69 | 2547 | C₇₂H₁₃₃N₅O₁₀ | 1228.88 | 7.28 | 26.13 |
| 2348 | C₇₃H₁₃₅NO₂S₇ | 1283.36 | 10.25 | 31.89 | 2548 | C₇₅H₁₄₈N₄O₂ | 1138.04 | 10.25 | 29.53 |
| 2349 | C₇₄H₁₃₈N₆O₈S | 1272.0 2 | 8.96 | 26.06 | 2549 | C₇₂H₁₃₈N₆O₃ | 1135.93 | 8.47 | 25.31 |
| 2350 | C₇₁H₁₃₂N₆O₈ | 1197.8 7 | 8.73 | 24.03 | 2550 | C₇₂H₁₃₁N₃O₁₁S | 1246.91 | 7.71 | 26.16 |
| 2351 | C₇₁H₁₃₇N₅O₈S | 1220.9 7 | 10.21 | 27.15 | 2551 | C₇₆H₁₃₅N₃O₁₃ | 1298.92 | 8.06 | 26.23 |
| 2352 | C₇₀H₁₂₇NO₇S | 1126.8 5 | 8.28 | 27.40 | 2552 | C₇₃H₁₃₃N₃O₁₃S | 1292.93 | 8.53 | 24.60 |
| 2353 | C₆₆H₁₂₄N₄O₃S₂ | 1085.8 8 | 7.45 | 24.89 | 2553 | C₇₂H₁₃₅NO₁₃S | 1254.93 | 9.37 | 28.42 |
| 2354 | C₆₉H₁₃₃NO₃S₂ | 1088.9 6 | 10.01 | 27.96 | 2554 | C₇₅H₁₄₉N₂O₁₁P₃ | 1347.94 | 8.85 | 27.16 |
| 2355 | C₇₃H₁₄₁N₂O₁₃P₃ S | 1379.9 2 | 7.73 | 24.08 | 2555 | C₇₅H₁₄₈N₃O₁₀P₃ S | 377.00 | 9.09 | 25.62 |
| 2356 | C₇₂H₁₃₆N₄O₁₅5₄ | 1426.1 6 | 8.39 | 26.10 | 2556 | C₇₂H₁₃₅N₃O₉S₄ | 1315.12 | 8.02 | 23.79 |
| 2357 | C₆₆H₁₂₇NO₈S₄ | 1191.0 1 | 9.56 | 23.38 | 2557 | C₆₇H₁₃₃N₅O₇S₃ | 1217.01 | 9.55 | 24.42 |
| 2358 | C₆₉H₁₃₄N₆O₈S₄ | 1304.1 3 | 8.63 | 23.43 | 2558 | C₆₈H₁₃₇N₇O₈S₄ | 1309.12 | 8.59 | 23.56 |
| 2359 | C₇₀H₁₃₄N₂O₁₁S₃ | 1276.0 5 | 9.01 | 25.11 | 2559 | C₆₆H₁₃₀N₄O₉S₄ | 1252.03 | 7.70 | 24.69 |
| 2360 | C₇₁H₁₂₉N₃O₆S₆ | 1313.2 3 | 7.83 | 29.32 | 2560 | C₆₉H₁₃₁NO₁₀S₄ | 1263.04 | 9.72 | 26.82 |
| 2361 | C₇₅H₁₃₈N₃O₇PS₆ | 1417.3 2 | 8.79 | 29.20 | 2561 | C₆₉H₁₃₄N₂O₁₁S₄ | 1296.07 | 9.65 | 25.47 |
| 2362 | C₇₃H₁₂₈N₆O₆S₇ | 1410.3 3 | 8.05 | 29.45 | 2562 | C₇₂H₁₃₇N₃O₁₄S₄ | 1397.13 | 7.96 | 24.84 |
| 2363 | C₆₉H₁₃₃N₃O₄S₃ | 1165.0 4 | 8.75 | 27.33 | 2563 | C₇₆H₁₃₄N₆O₅S₆ | 1404.30 | 8.44 | 29.07 |
| 2364 | C₇₀H₁₃₃N₂O₄PS₃ | 1194.0 2 | 8.64 | 27.09 | 2564 | C₇₅H₁₃₈N₄O₄S₇ | 1384.37 | 7.99 | 27.97 |
| 2365 | C₆₈H₁₃₁N₅O₅ | 1098.8 3 | 8.96 | 24.38 | 2565 | C₇₂H₁₃₃NO₂S₇ | 1269.28 | 9.22 | 29.39 |
| 2366 | C₆₆H₁₃₀N₄O₄ | 1043.7 9 | 9.95 | 23.32 | 2566 | C₇₆H₁₃₉NS₈ | 1323.43 | 9.99 | 32.54 |
| 2367 | C₆₂H₁₂₄N₄O₅S | 1037.7 6 | 8.99 | 22.55 | 2567 | C₆₈H₁₃₂N₂OS₆ | 1186.18 | 9.61 | 29.43 |
| 2368 | C₆₇H₁₃₀N₆O₃S₂ | 1131.9 5 | 9.38 | 22.39 | 2568 | C₇₆H₁₄₆N₆O₈ | 1272.04 | 7.91 | 25.83 |
| 2369 | C₆₇H₁₃₁N₅O₅S₂ | 1150.9 5 | 8.80 | 22.94 | 2569 | C₇₄H₁₄₂N₂O₆S₃ | 1252.14 | 7.97 | 29.92 |
| 2370 | C₆₅H₁₃₀N₄O₃S₂ | 1079.9 1 | 10.12 | 23.75 | 2570 | C₇₆H₁₄₇N₃O₇S₄ | 1343.27 | 8.74 | 29.77 |
| 2371 | C₆₄H₁₂₁NO₅S₄ | 1112.9 4 | 9.72 | 26.66 | 2571 | C₆₈H₁₃₃NO₆S₄ | 1189.05 | 9.29 | 28.76 |
| 2372 | C₆₃H₁₂₇N₇O₆S | 1110.8 2 | 8.83 | 20.79 | 2572 | C₇₃H₁₄₅N₅O₅ | 1172.99 | 9.61 | 27.70 |
| 2373 | C₆₃H₁₂₆N₆OS₂ | 1047.8 8 | 8.76 | 23.90 | 2573 | C₇₁H₁₃₀N₅O₁₀S | 1254.98 | 7.51 | 27.38 |
| 2374 | C₆₁H₁₁₉NO₁₄S | 1122.6 8 | 7.69 | 23.72 | 2574 | C₆₉H₁₄₂N₄O₂ | 1059.92 | 10.05 | 27.68 |
| 2375 | C₆₆H₁₂₈N₃O₁₃P | 1202.7 3 | 7.46 | 24.54 | 2575 | C₇₁H₁₄₄N₆O₃S | 1162.03 | 8.83 | 25.65 |
| 2376 | C₇₃H₁₄₅N₄O₁₁P₃ | 1347.9 0 | 8.61 | 22.75 | 2576 | C₇₁H₁₄₈N₄O₁₀S₄ | 1346.22 | 9.78 | 25.99 |
| 2377 | C₇₂H₁₅₀NO₁₃P₃ S | 1362.9 7 | 10.23 | 26.58 | 2577 | C₇₅H₁₄₁N₃O₉S₂ | 1293.09 | 9.29 | 29.12 |
| 2378 | C₅₈H₁₂₁N₅O₈S₃ | 1112.8 3 | 7.95 | 21.32 | 2578 | C₇₅H₁₄₆N₃O₁₀P S₃ | 1377.16 | 8.69 | 28.51 |
| 2379 | C₆₁H₁₂₈N₄O₉S₄ | 1189.9 8 | 9.93 | 23.20 | 2579 | C₆₉H₁₃₈N₄O₅S | 1135.95 | 7.81 | 25.35 |
| 2380 | C₇₉H₁₅₀N₆O₅S₂ | 1328.2 4 | 8.02 | 27.18 | 2580 | C₈₀H₁₆₁N₃O₁₃P₄ | 1497.07 | 8.34 | 25.40 |
| 2381 | C₈₀H₁₅₆N₆O₇ | 1314.1 6 | 8.79 | 28.87 | 2581 | C₆₈H₁₃₈N₂O₁₃S₄ | 1320.09 | 7.81 | 24.98 |
| 2382 | C₇₇H₁₅₃N₅O₉S | 1325.1 6 | 8.63 | 27.52 | 2582 | C₇₃H₁₅₆N₃O₁₁P₃ | 1405.04 | 8.90 | 25.51 |
| 2383 | C₇₉H₁₅₆N₄O₅S | 1274.2 1 | 9.99 | 30.04 | 2583 | C₇₃H₁₄₃N₉O₇S₅ | 1419.30 | 8.50 | 27.26 |
| 2384 | C₇₈H₁₄₈N₂O₃S₇ | 1386.5 2 | 8.53 | 34.02 | 2584 | C₇₅H₁₄₆N₄O₄S₃ | 1264.20 | 9.75 | 28.36 |
| 2385 | C₇₇H₁₄₈N₂O₅S₇ | 1406.51 | 8.37 | 33.09 | 2585 | C₇₆H₁₄₁N₇O₅S₃ | 1329.19 | 8.44 | 6.51 |
| 2386 | C₇₈H₁₄₉N₃S₇ | 1353.5 4 | 9.35 | 33.71 | 2586 | C₇₃H₁₄₃N₃S₈ | 1319.45 | 9.73 | 31.55 |
| 2387 | C₈₀H₁₅₅N₇O₅ | 1295.1 6 | 9.05 | 28.08 | 2587 | C₆₈H₁₃₇NO₂S₇ | 1225.27 | 9.56 | 32.84 |
| 2388 | C₇₇H₁₅₅N₇O₃S₂ | 1291.2 7 | 9.72 | 30.97 | 2588 | C₁₀₀H₁₉₂N₂O₄S | 1518.71 | 9.01 | 41.94 |
| 2389 | C₇₇H₁₅₃N₉O₂S | 1269.2 0 | 8.46 | 28.74 | 2589 | C₉₈H₁₈₉NOS₅ | 1557.90 | 7.61 | 43.84 |
| 2390 | C₇₆H₁₅₆N₄O₇S₃ | 1334.3 1 | 8.94 | 31.72 | 2590 | C₉₈H₁₉₄NO₃PS₃ | 1561.79 | 8.48 | 42.90 |
| 2391 | C₇₆H₁₅₄N₆O₈S₃ | 1376.3 0 | 8.64 | 28.57 | 2591 | C₉₉H₁₉₄N₄O₇S | 1584.72 | 9.65 | 43.10 |
| 2392 | C₇₈H₁₄₆N₂O₈ | 1240.0 3 | 8.71 | 30.11 | 2592 | C₉₉H₁₉₄N₄O₈S₂ | 1632.78 | 7.76 | 43.54 |
| 2393 | C₇₆H₁₄₈NO₉P | 1250.9 9 | 8.86 | 31.56 | 2593 | C₉₆H₁₉₆N₄O₃S | 1486.71 | 9.59 | 42.11 |
| 2394 | C₇₈H₁₅₀N₆O₉S | 1348.1 6 | 7.69 | 29.53 | 2594 | C₉₇H₁₉₅N₅O₄S | 1527.72 | 8.65 | 40.06 |
| 2395 | C₇₇H₁₅₀N₄O₇S | 1276.1 3 | 9.43 | 30.93 | 2595 | C₁₀₂H₁₉₉N₅O₉ | 1639.74 | 8.75 | 41.94 |
| 2396 | C₇₇H₁₄₇NO₇S₄ | 1327.2 9 | 9.86 | 34.36 | 2596 | C₉₈H₁₉₇N₄O₁₀P | 1622.65 | 8.51 | 41.00 |
| 2397 | C₇₅H₁₄₉NO₁₁S₄ | 1369.2 8 | 9.91 | 31.06 | 2597 | C₉₉H₁₉₅NO₆S₄ | 1623.89 | 10.09 | 47.23 |
| 2398 | C₇₉H₁₆₂NO₁₁P₃ S | 1427.1 5 | 9.02 | 30.52 | 2598 | C₁₀₅H₂₁₄NO₁₀P₃ | 1743.79 | 10.19 | 43.28 |
| 2399 | C₇₆H₁₅₇N₆O₁₀P S₃ | 1442.3 0 | 8.06 | 28.96 | 2599 | C₉₈H₁₉₃N₃O₁₂S₄ | 1733.87 | 8.00 | 41.24 |
| 2400 | C₇₅H₁₄₉N₃O₁₀S₅ | 1413.3 6 | 8.76 | 28.82 | 2600 | C₆₃H₁₀₇NO₆S | 1006.62 | 9.80 | 21.64 |
| 2601 | C₆₅H₁₁₂N₃O₄PS ₆ | 1223.0 0 | 7.95 | 21.13 | 2801 | C₉₉H₂₀₀N₆O₁₀S₄ | 1762.96 | 8.40 | 40.16 |
| 2602 | C₆₆H₁₁₉N₇O₅ | 1090.7 2 | 10.00 | 21.99 | 2802 | C₁₀₀H₁₉₉N₃O₁₁S ₃ | 1715.88 | 8.98 | 40.36 |
| 2603 | C₆₁H₁₁₄N₄O₇S₅ | 1175.9 4 | 9.86 | 19.74 | 2803 | C₉₈H₂₀₁N₄O₁₂P S₃ | 1754.86 | 9.49 | 39.38 |
| 2604 | C₆₁H₁₁₂N₆₀O₁₁S₄ | 1233.8 7 | 7.61 | 17.67 | 2804 | C₉₉H₁₉₅N₃OS₉ | 1732.21 | 8.95 | 48.29 |
| 2605 | C₅₈H₁₁₀N₄O₉S₄ | 1135.8 0 | 7.67 | 19.80 | 2805 | C₇₇H₁₂₁N₃O₈S | 1248.89 | 8.03 | 26.31 |
| 2606 | C₆₆H₁₁₆N₆O₈S | 1153.7 5 | 8.85 | 21.05 | 2806 | C₇₂H₁₁₇NO₃S₅ | 1205.03 | 10.07 | 28.49 |
| 2607 | C₆₂H₁₀₆N₂O₁₁ | 1055.5 3 | 9.60 | 20.49 | 2807 | C₇₄H₁₁₇N₃OS₄ | 1193.01 | 8.45 | 25.19 |
| 2608 | C₆₃H₁₀₉NO₈S₃ | 1104.7 6 | 9.87 | 23.85 | 2808 | C₇₅H₁₂₁NO₃S₇ | 1309.22 | 9.65 | 27.97 |
| 2609 | C₆₃H₁₀₈N₂O₁₀S₄ | 1181.8 3 | 8.42 | 23.27 | 2809 | C₇₉H₁₃₄N₉O₆PS | 1369.03 | 8.95 | 25.87 |
| 2610 | C₆₅H₁₀₉N₃O₇S₅ | 1204.9 4 | 8.71 | 22.79 | 2810 | C₇₉H₁₃₀N₆O₉ | 1307.94 | 8.95 | 26.58 |
| 2611 | C₆₆H₁₂₂NO₁₀P₃ S | 1214.6 9 | 9.74 | 20.96 | 2811 | C₇₆H₁₃₅N₄O₄P | 1199.91 | 10.01 | 25.75 |
| 2612 | C₆₃H₁₁₁N₃O₉S₅ | 1214.9 3 | 9.29 | 21.07 | 2812 | C₈₁H₁₂₆N₆O₉ | 1327.93 | 8.71 | 26.39 |
| 2613 | C₆₃H₁₁₃N₃O₁₁S₄ | 1216.8 7 | 8.22 | 20.49 | 2813 | C₇₇H₁₂₆N₄O₆S₂ | 1268.00 | 9.98 | 27.83 |
| 2614 | C₆₅H₁₂₂NO₁₁P₃ S | 1218.6 8 | 8.62 | 19.71 | 2814 | C₇₅H₁₂₄N₄O₈ | 1209.84 | 8.73 | 27.26 |
| 2615 | C₆₀H₁₁₁₂NO₉PS₃ | 1118.7 3 | 10.00 | 17.50 | 2815 | C₇₁H₁₁₉NO₆ | 1082.73 | 8.65 | 26.27 |
| 2616 | C₆₃H₁₀₈N₆O₁₁S₄ | 1253.8 6 | 8.07 | 20.48 | 2816 | C₈₁H₁₃₈N₃O₁₂P₃ S | 1470.99 | 8.02 | 25.79 |
| 2617 | C₆₃H₁₁₃N₂O₅PS 3 | 1105.7 8 | 7.69 | 21.48 | 2817 | C₇₈H₁₃₅N₄O₁₁P₃ S | 1429.94 | 8.22 | 25.01 |
| 2618 | C₆₁H₁₀₉N₃O₂S₄ | 1044.8 3 | 8.31 | 22.42 | 2818 | C₇₄H₁₂₈NO₁₁P₃ S | 1332.82 | 8.72 | 26.25 |
| 2619 | C₆₀H₁₀₅N₃O₄S₆ | 1124.9 2 | 7.15 | 23.05 | 2819 | C₇₁₁H₁₂₁NO₁₀S₃ | 1244.93 | 9.53 | 26.84 |
| 2620 | C₅₆H₁₀₄N₂O₅S | 917.52 | 9.66 | 18.46 | 2820 | C₇₆H₁₃₀NO₁₂PS 3 | 1377.02 | 9.99 | 26.39 |
| 2621 | C₅₇H₁₀₀N₂O₁₀ | 973.43 | 7.44 | 19.35 | 2821 | C₆₉H₁₁₇NO₁₆S₄ | 1344.93 | 8.21 | 27.40 |
| 2622 | C₅₇H₁₀₁NOS₄ | 944.71 | 8.76 | 21.30 | 2822 | C₇₆H₁₃₀N₄O₈S₃ | 1324.08 | 10.01 | 27.21 |
| 2623 | C₆₁H₁₀₉N₃O₃S₆ | 1124.96 | 8.89 | 21.53 | 2823 | C₇₁H₁₂₁N₇O₈S₅ | 1361.09 | 7.65 | 25.06 |
| 2624 | C₇₂H₁₃₁N₃O₄S | 1134.9 2 | 8.90 | 25.07 | 2824 | C₇₄H₁₂₀N₂O₅S₃ | 1213.96 | 9.95 | 27.23 |
| 2625 | C₇₃H₁₃₇N₇O₆ | 1208.9 4 | 8.96 | 23.56 | 2825 | C₇₇H₁₂₀N₆O₇S₄ | 1370.08 | 7.80 | 26.86 |
| 2626 | C₇₂H₁₂₇N₇O₉ | 1234.8 5 | 8.10 | 24.19 | 2826 | C₇₃H₁₂₀N₂O₅S₄ | 1234.01 | 9.58 | 27.28 |
| 2627 | C₇₃H₁₃₅N₃O₉S | 1230.9 6 | 9.09 | 25.63 | 2827 | C₇₆H₁₂₄N₃O₁₀P S₃ | 1366.99 | 7.57 | 26.82 |
| 2628 | C₆₈H₁₂₇NO₃S_{S} | 1167.1 0 | 9.70 | 29.88 | 2828 | C₇₂H₁₂₃NO₃S₇ | 1275.20 | 9.67 | 28.90 |
| 2629 | C₆₈H₁₂₇N₃S₇ | 1211.2 5 | 9.60 | 27.84 | 2829 | C₇₄H₁₂₃NO₂S₇ | 1283.22 | 9.86 | 30.94 |
| 2630 | C₇₁H₁₃₄NO₁₀P | 1192.8 2 | 9.64 | 24.22 | 2830 | C₇₂H₁₄₂N₂O₃ | 1083.94 | 9.36 | 28.29 |
| 2631 | C₆₈H₁₂₈N₄O₆S₂ | 1161.9 3 | 10.07 | 26.57 | 2831 | C₆₉H₁₃₄N₂O₅S | 1103.90 | 9.68 | 26.54 |
| 2632 | C₇₀H₁₃₀N₂O₅S₃ | 1224.0 2 | 9.71 | 26.56 | 2832 | C₇₁H₁₃₆N₂O₇S₂ | 1194.00 | 8.99 | 28.63 |
| 2633 | C₇₂H₁₃₁N₃O₉S₃ | 1279.0 5 | 8.83 | 27.55 | 2833 | C₆₅H₁₂₇NO₆S₂ | 1082.85 | 9.93 | 28.44 |
| 2634 | C₆₈H₁₂₅NO₇S₅ | 1229.0 8 | 9.96 | 29.51 | 2834 | C₇₂H₁₃₃N₃O₆S₃ | 1233.05 | 7.96 | 27.03 |
| 2635 | C₆₉H₁₃₀N₄O₄ | 1079.8 2 | 8.27 | 25.32 | 2835 | C₇₃H₁₃₉N₃O₆S₃ | 1251.11 | 8.84 | 28.79 |
| 2636 | C₆₈H₁₃₂N₂O₁₄S₃ | 1298.0 0 | 10.19 | 27.01 | 2836 | C₇₃H₁₃₉NO₅S₄ | 1239.16 | 10.13 | 31.72 |
| 2637 | C₇₂H₁₄₀N₃O₁₂P₃ | 1332.8 4 | 7.62 | 23.57 | 2837 | C₆₉H₁₃₁NOS₅ | 1151.11 | 9.73 | 29.40 |
| 2638 | C₇₁H₁₃₅N₇O₉S₃ | 1327.1 0 | 8.42 | 23.39 | 2838 | C₆₉H₁₃₆N₂S₆ | 1186.22 | 10.20 | 29.92 |
| 2639 | C₆₉H₁₃₁N₃O₁₂S₄ | 1323.0 8 | 8.78 | 25.51 | 2839 | C₆₈H₁₃₉N₉O₃S₂ | 1195.04 | 9.53 | 26.63 |
| 2640 | C₇₁H₁₂₇N₃O₇S₄ | 1263.0 8 | 7.70 | 27.37 | 2840 | C₇₀H₁₃₄N₆O₇S | 1203.94 | 8.24 | 27.19 |
| 2641 | C₇₂H₁₃₀N₄O₉S₃ | 1292.0 5 | 8.32 | 28.21 | 2841 | C₇₀H₁₃₃N₅O₉S | 1220.92 | 8.39 | 26.98 |
| 2642 | C₆₉H₁₂₉N₃O₆S₇ | 1321.2 7 | 9.35 | 27.16 | 2842 | C₆₉H₁₄₂N₆O₇S₃ | 1264.11 | 9.97 | 26.55 |
| 2643 | C₇₀H₁₂₉NS₈ | 1241.2 9 | 10.16 | 30.09 | 2843 | C₆₅H₁₃₇N₅O₉S₄ | 1261.08 | 8.86 | 24.06 |
| 2644 | C₅₈H₁₀₇N₄O₈S | 1021.5 9 | 10.16 | 30.09 | 2844 | C₇₁H₁₃₇NO₉ | 1148.88 | 10.05 | 28.77 |
| 2645 | C₅₆H₁₁₁N₆O₄P | 963.52 | 7.19 | 17.58 | 2845 | C₆₉H₁₃₇N₃O₆ | 1104.87 | 8.83 | 26.42 |
| 2646 | C₅₅H₁₀₆N₄O₆S₅ | 1079.8 1 | 9.87 | 19.57 | 2846 | C₇₂H₁₄₄N₃O₁₀P₃ | 1304.88 | 7.67 | 25.50 |
| 2647 | C₅₆H₁₀₇N₇O₈S₃ | 1102.7 1 | 8.21 | 17.61 | 2847 | C₇₁H₁₄₉N₂O₁₁P₃ S | 1331.96 | 8.90 | 26.60 |
| 2648 | C₅₄H₁₀₂N₆O₈S₄ | 1091.7 1 | 7.81 | 16.98 | 2848 | C₆₆H₁₃₄NO₁₂PS₃ | 1260.95 | 9.07 | 25.89 |
| 2649 | C₅₈H₁₀₇N₇O₈S | 1062.6 0 | 8.83 | 17.19 | 2849 | C₆₃H₁₃₂N₄O₁₀S₄ | 1234.01 | 8.10 | 24.36 |
| 2650 | C₅₄H₁₀₄N₂O₅S | 893.50 | 9.68 | 18.78 | 2850 | C₇₂H₁₃₇NO₃S₅ | 1225.19 | 9.90 | 31.49 |
| 2651 | C₆₄H₁₂₄N₃O₁₄P₃ S | 1284.6 9 | 8.20 | 17.97 | 2851 | C₇₀H₁₃₅NO₃S₅ | 1199.15 | 9.97 | 30.96 |
| 2652 | C₅₅H₁₀₃N₃O₁₀S₅ | 1126.7 7 | 8.47 | 18.72 | 2852 | C₇₁H₁₃₅N₃OS₆ | 1239.24 | 9.56 | 28.40 |
| 2653 | C₅₅H₁₀₀N₆O₁₃S₄ | 1181.7 0 | 8.28 | 18.03 | 2853 | C₅₃H₁₀₀N₆O₅ | 901.42 | 8.32 | 14.69 |
| 2654 | C₅₅H₁₀₆N₄O₇S₄ | 1063.7 4 | 9.80 | 19.25 | 2854 | C₅₀H₁₀₃N₁₁O₅ | 938.45 | 8.48 | 15.09 |
| 2655 | C₅₉H₁₀₄N₄O₄S₃ | 1029.7 1 | 9.13 | 19.59 | 2855 | C₅₂H₁₀₀N₆O₉S | 985.46 | 8.31 | 17.51 |
| 2656 | C₅₆H₁₀₃NO₇S₄ | 1030.7 1 | 9.87 | 21.24 | 2856 | C₅₃H₁₀₁N₃O₇ | 892.40 | 9.95 | 18.39 |
| 2657 | C₅₇H₁₀₄N₂OS₆ | 1025.8 7 | 10.01 | 20.81 | 2857 | C₅₁H₉₇NO₁₂S | 948.39 | 9.30 | 17.75 |
| 2658 | C₇₂H₁₂₈N₂OS₃ | 1134.0 3 | 10.16 | 24.30 | 2858 | C₅₀H₉₅NO₁₀S₄ | 998.55 | 8.15 | 20.65 |
| 2659 | C₆₉H₁₂₈N₄OS | 1061.8 8 | 10.02 | 24.28 | 2859 | C₅₄H₁₁₂NO₁₁P₃ S | 1076.47 | 9.21 | 18.64 |
| 2660 | C₇₀H₁₂₉N₅O₇S₅ | 1313.1 6 | 8.99 | 22.78 | 2860 | C₅₃H₁₀₀N₆O₁₀S₄ | 1109.65 | 8.67 | 15.63 |
| 2661 | C₇₂H₁₃₃N₄O₉P | 1229.85 | 9.96 | 23.06 | 2861 | C₅₀H₁₀₁NO₁₄S₄ | 1068.59 | 10.07 | 20.13 |
| 2662 | C₆₈H₁₂₀N₂O₁₁ | 1141.7 1 | 9.11 | 21.06 | 2862 | C₅₁H₁₀₂N₂O₁₄S₅ | 1127.68 | 8.80 | 20.04 |
| 2663 | C₇₂H₁₂₅N₃O₉S₃ | 1273.0 0 | 9.10 | 26.58 | 2863 | C₅₁H₁₀N₆O₉S₄ | 1069.63 | 8.09 | 16.22 |
| 2664 | C₇₂H₁₂₅NO₈S₃ | 1228.9 9 | 10.00 | 27.22 | 2864 | C₄₆H₉₆N₄O₆S₅ | 961.60 | 9.69 | 17.23 |
| 2665 | C₇₅H₁₄₁N₂O₁₄P₃ S | 1419.9 4 | 8.57 | 22.34 | 2865 | C₅₂H₁₀₂N₄O₁₂S₃ | 1071.58 | 8.63 | 16.45 |
| 2666 | C₆₇H₁₂₁N₃O₁₁S₄ | 1272.9 5 | 7.36 | 19.18 | 2866 | C₄₇H₉₁NO₃S₅ | 878.55 | 9.65 | 20.22 |
| 2667 | C₆₉H₁₂₆N₆O₇S₄ | 1280.0 4 | 9.00 | 22.36 | 2867 | C₅₁H₉₆N₄OS₆ | 973.72 | 9.50 | 17.13 |
| 2668 | C₆₉H₁₃₀N₄O₆S₅ | 1272.1 2 | 10.21 | 24.79 | 2868 | C₄₉H₉₉NO₃S₃ | 846.51 | 9.67 | 20.47 |
| 2669 | C₆₇H₁₁₇NO₆S₄ | 1160.9 1 | 8.83 | 24.72 | 2869 | C₄₈H₉₃N₃OS₇ | 952.71 | 7.90 | 20.75 |
| 2670 | C₇₃H₁₂₉N₃O₄S₅ | 1273.1 5 | 8.98 | 26.99 | 2870 | C₆₈H₁₃₂N₂O₆S₃ | 1169.99 | 8.20 | 27.11 |
| 2671 | C₆₇H₁₁₉NO₇S₇ | 1275.1 1 | 7.43 | 24.74 | 2871 | C₆₉H₁₃₁N₅O₇S₄ | 1271.07 | 8.05 | 28.00 |
| 2672 | C₇₀H₁₂₁N₃O₄S₇ | 1293.1 7 | 8.47 | 24.89 | 2872 | C₇₄H₁₄₇N₄O₁₃P₃ S | 1425.99 | 8.26 | 21.77 |
| 2673 | C₆₇H₁₁₉N₃O₃S₄ | 1142.9 5 | 7.75 | 24.17 | 2873 | C₇₈H₁₅₅NO₁₃P₄ S | 1471.05 | 9.23 | 26.84 |
| 2674 | C₆₆H₁₁₉NO₂S₇ | 1183.1 0 | 9.76 | 27.41 | 2874 | C₇₄H₁₄₇N₂O₁₂P₃ S | 1381.97 | 7.99 | 25.04 |
| 2675 | C₇₄H₁₄₂N₄O₉S | 1264.0 3 | 8.40 | 26.85 | 2875 | C₈₀H₁₄₁N₇O₁₂S | 1425.10 | 8.28 | 27.74 |
| 2676 | C₇₂H₁₃₅N₃O₇S | 1186.9 5 | 7.50 | 28.39 | 2876 | C₇₅H₁₃₇N₅O₁₂S | 1333.00 | 7.02 | 25.80 |
| 2677 | C₇₄H₁₃₈N₄O₂S₃ | 1212.1 2 | 7.44 | 26.73 | 2877 | C₇₅H₁₄₆N₅O₈PS | 1309.05 | 9.53 | 24.72 |
| 2678 | C₇₅H₁₃₉N₃O₄S₄ | 1275.1 9 | 7.91 | 28.49 | 2878 | C₅₂H₁₀₁NO₁₁S₂ | 980.50 | 7.66 | 18.55 |
| 2679 | C₇₂H₁₃₆N₂O₂S₆ | 1254.2 5 | 8.75 | 29.44 | 2879 | C₆₈H₁₁₅NO₇S₄ | 1186.91 | 9.71 | 24.43 |
| 2680 | C₉₁H₁₇₇NO₄S | 1381.4 8 | 9.69 | 39.41 | 2880 | C₆₅H₁₁₃NO₁₀S₅ | 1228.92 | 7.12 | 22.76 |
| 2681 | C₈₉H₁₇₂N₂O₇ | 1382.3 6 | 9.42 | 37.27 | 2881 | C₆₅H₁₂₂NO₁₂P₃ S | 1234.67 | 7.76 | 19.16 |
| 2682 | C₉₁H₁₇₆N₆O₇ | 1466.4 4 | 8.11 | 36.76 | 2882 | C₆₈H₁₁₅N₃O₁₂S | 1198.74 | 8.37 | 22.77 |
| 2683 | C₉₂H₁₇₈N₃O₁₀P | 1517.4 2 | 8.01 | 36.76 | 2883 | C₇₅H₁₃₀N₃O₆PS 4 | 1329.09 | 8.51 | 25.28 |
| 2684 | C₉₁H₁₇₇N₃O₇S₄ | 1553.6 7 | 8.56 | 37.79 | 2884 | C₇₆H₁₃₆N₂O₁₀S₅ | 1398.23 | 9.74 | 30.25 |
| 2685 | C₉₃H₁₇₉N₃O₃S₃ | 1483.6 5 | 8.65 | 37.45 | 2885 | C₇₀H₁₃₀NO₁₂PS 5 | 1369.08 | 9.35 | 22.77 |
| 2686 | C₉₀H₁₇₄N₄O₂S₆ | 1536.7 7 | 9.13 | 38.77 | 2886 | C₆₈H₁₂₇N₃O₅S₅ | 1275.08 | 7.49 | 18.47 |
| 2687 | C₈₉H₁₇₆N₄O₄ | 1366.4 1 | 9.65 | 36.39 | 2887 | C₅₇₆H₁₀₇N₄O₅S₂ | 979.61 | 8.60 | 19.07 |
| 2688 | C₈₈H₁₇₆N₄O₇S | 1434.4 6 | 7.44 | 34.46 | 2888 | C₅₇H₁₀₄N₄O₇S₄ | 1085.72 | 8.19 | 20.14 |
| 2689 | C₉₀H₁₈₀N₁₀O₃S | 1482.5 6 | 9.39 | 37.26 | 2889 | C₆₁H₁₀₆N₄O₈S₃ | 1119.72 | 7.67 | 21.03 |
| 2690 | C₉₃H₁₈₅N₉O₆S | 1557.6 2 | 8.83 | 35.92 | 2890 | C₅₉H₁₁₁N₃O₁₄S₆ | 1278.90 | 8.13 | 18.51 |
| 2691 | C₉₁H₁₈₀N₄O₈ | 1458.4 6 | 9.68 | 39.20 | 2891 | C₅₇H₁₀₇N₃O₁₂S₅ | 1186.79 | 8.93 | 18.22 |
| 2692 | C₈₄H₁₇₄N₄O₁₀S₄ | 1528.5 7 | 9.73 | 35.64 | 2892 | C₅₈H₁₀₈N₂O₄S₆ | 1089.87 | 9.71 | 23.62 |
| 2693 | C₉₁H₁₇₇NO₈ | 1413.4 2 | 9.74 | 39.59 | 2893 | C₇₅H₁₃₃N₃O₁₂S₃ | 1365.08 | 7.62 | 28.16 |
| 2694 | C₉₁H₁₇₇NO₈ | 1413.4 2 | 9.63 | 39.59 | 2894 | C₇₃H₁₄₄N₅O₇PS | 1267.02 | 8.77 | 23.15 |
| 2695 | C₉₄H₁₉₁NO₁₄S₄ | 1687.8 0 | 8.92 | 40.68 | 2895 | C₇₀H₁₃₅NO₃S_{S} | 1295.33 | 9.28 | 29.89 |
| 2696 | C₉₈H₂₀₀N₄O₉S₃ | 1674.8 8 | 10.05 | 42.36 | 2896 | C₇₁H₁₄₅N₃OS₄ | 1185.20 | 10.13 | 32.12 |
| 2697 | C₁₀₁H₁₉₄N₂O₆S₄ | 1660.9 1 | 8.50 | 44.51 | 2897 | C₆₉H₁₃₈NO₁₃PS 5 | 1381.13 | 8.17 | 26.27 |
| 2698 | C₉₅H₁₈₇NO₂S₇ | 1599.97 | 7.87 | 43.73 | 2898 | C₇₆H₁₂₈N₄O₇S | 1241.94 | 9.76 | 26.18 |
| 2699 | C₉₆H₁₉₄N₂O₃S₇ | 1649.0 4 | 8.91 | 43.81 | 2899 | Cr₇H₁₂₈N₄O₁₀S₃ | 1366.07 | 9.13 | 28.18 |
| 2700 | C₇₃H₁₃₅N₃O₇S | 1198.9 6 | 8.83 | 27.72 | 2900 | C₇₈H₁₂₉N₇O₁₁S₄ | 1469.17 | 7.08 | 24.13 |
| 2701 | C₇₃H₁₃₂N₄O₂S₃ | 1194.0 7 | 8.26 | 26.95 | 2901 | C₇₆H₁₃₀N₆O₁₀S₆ | 1480.27 | 8.32 | 26.25 |
| 2702 | C₇₄H₁₄₃N₇O₆ | 1227.0 0 | 10.05 | 27.15 | 2902 | C₇₉H₁₂₈N₆O₁₂S₄ | 1482.16 | 8.21 | 26.97 |
| 2703 | C₇₂H₁₃₈N₄O₈S | 1219.9 8 | 9.16 | 26.82 | 2903 | C₇₈H₁₃₅N₅O₄ | 1206.97 | 9.64 | 29.41 |
| 2704 | C₇₀H₁₃₃N₅OS₂ | 1124.9 9 | 8.67 | 26.08 | 2904 | C₈₀H₁₂₉N₇O₇ | 1300.95 | 8.42 | 24.11 |
| 2705 | C₇₀H₁₃₀N₄O₆S | 1155.8 9 | 9.03 | 26.72 | 2905 | C₉₂H₁₅₆N₂O₅S₅ | 1530.57 | 8.87 | 38.21 |
| 2706 | C₇₁H₁₃₃N₅O₉S | 1232.9 3 | 7.56 | 25.84 | 2906 | C₉₅H₁₆₀N₃O₁₂P S₃ | 1663.49 | 7.57 | 35.94 |
| 2707 | C₇₂H₁₃₄N₄O₁₀S | 1247.9 4 | 9.32 | 26.54 | 2907 | C₉₁H₁₅₇NO₁₇S₅ | 1697.55 | 9.77 | 37.07 |
| 2708 | C₇₂H₁₃₂N₂O₁₂S | 1249.9 1 | 8.50 | 26.64 | 2908 | C₉₂H₁₅₉NOS₉ | 1583.83 | 9.44 | 41.50 |
| 2709 | C₆₈H₁₃₁NO₁₅S₃ | 1298.9 7 | 8.73 | 27.84 | 2909 | C₈₀H₁₅₇N₃O₁₁S₂ | 1401.27 | 7.36 | 30.07 |
| 2710 | C₇₀H₁₃₃N₃O₁₅S₄ | 1385.0 8 | 8.44 | 27.06 | 2910 | C₈₀H₁₆₁N₇O₉S | 1397.27 | 9.05 | 31.05 |
| 2711 | C₈₀H₁₅₇N₃O₁₄P₄ | 1509.0 4 | 8.07 | 24.70 | 2911 | C₈₆H₁₇₂N₃O₁₄P₃ S₂ | 1629.37 | 7.98 | 30.43 |
| 2712 | C₆₉H₁₂₉N₃O₉S₄ | 1273.0 4 | 8.14 | 23.02 | 2912 | C₈₂H₁₅₉NO₈S | 1319.23 | 9.17 | 32.07 |
| 2713 | C₇₁H₁₃₉N₃O₁₀S₃ | 1291.0 8 | 10.20 | 26.84 | 2913 | C₈₂H₁₅₇NO₉S₂ | 1365.28 | 8.94 | 34.05 |
| 2714 | C₇₁H₁₃₀N₆O₉S₅ | 1372.1 5 | 7.66 | 25.94 | 2914 | C₈₂H₁₅₅NO₁₀S₃ | 1411.32 | 9.46 | 33.89 |
| 2715 | C₇₁H₁₃₁NO₄S₃ | 1159.0 1 | 9.05 | 28.83 | 2915 | C₈₂H₁₅₆N₆O₈ | 1354.18 | 7.58 | 27.67 |
| 2716 | C₇₄H₁₃₆N₄O₅S₃ | 1258.1 1 | 9.28 | 26.98 | 2916 | C₁₀₂H₂₀₀N₄O₆S | 1610.80 | 9.30 | 41.11 |
| 2717 | C₇₄H₁₄₀NO₆PS₆ | 1363.2 7 | 10.18 | 30.16 | 2917 | C₉₉H₁₉₉N₅O₁₂S₃ | 1747.88 | 8.74 | 39.52 |
| 2718 | C₇₂H₁₄₁NO₃S₄ | 1197.1 6 | 10.22 | 30.35 | 2918 | C₉₈H₂₀₁N₅O₁₂S₅ | 1802.01 | 7.78 | 38.29 |
| 2719 | C₇₄H₁₄₀N₂O₃S₆ | 1298.3 0 | 9.07 | 30.62 | 2919 | C₁₀₄H₂₀₂N₃O₁₃P S₂ | 1797.86 | 8.16 | 42.89 |
| 2720 | C₆₈H₁₃₃N₃O₅S | 1104.8 9 | 9.56 | 23.55 | 2920 | C₇₂H₁₄₃N₅O₆ | 1174.96 | 7.02 | 24.11 |
| 2721 | C₆₈H₁₃₁NO₂S₃ | 1090.9 8 | 10.20 | 27.08 | 2921 | C₇₅H₁₄₃NOS₄ | 1203.22 | 9.90 | 30.72 |
| 2722 | C₆₆H₁₂₇N₅O₇ | 1102.7 7 | 7.40 | 22.13 | 2922 | C₇₆H₁₄₆N₄O₈S₅ | 1404.32 | 7.57 | 29.76 |
| 2723 | C₆₇H₁₃₄N₆O₄S | 1119.9 1 | 8.03 | 22.12 | 2923 | C₇₆H₁₄₆N₄O₉S₃ | 1356.20 | 8.27 | 30.39 |
| 2724 | C₆₅H₁₃₅N₅O₉S₃ | 1227.0 0 | 9.81 | 23.41 | 2924 | C₇₈H₁₆₁N₂O₁₁P₃ S | 1428.13 | 9.86 | 28.23 |
| 2725 | C₆₇H₁₂₇N₃P₇ | 1086.7 7 | 9.14 | 23.56 | 2925 | C₇₇H₁₅₆NO₁₁P₃ S₂ | 1429.13 | 9.80 | 27.74 |
| 2726 | C₆₅H₁₂₆N₂O₁₂S | 1159.7 8 | 7.67 | 23.67 | 2926 | C₇₂H₁₄₆N₆O₉S₄ | 1368.23 | 7.44 | 25.66 |
| 2727 | C₆₆H₁₂₃N₃O₈S₅ | 1247.0 2 | 8.30 | 26.43 | 2927 | C₇₅H₁₄₇N₅O₇ | 1231.03 | 8.75 | 27.63 |
| 2728 | C₆₈H₁₄₁N₂O₁₀P₃ | 1239.8 0 | 9.68 | 23.81 | 2928 | C₇₁H₁₄₃NO₅S₃ | 1187.11 | 9.63 | 29.70 |
| 2729 | C₆₃H₁₂₅N₃O₁₄S₄ | 1276.9 4 | 7.77 | 24.47 | 2929 | C₇₇H₁₄₆N₂O₄S₆ | 1356.38 | 8.92 | 30.90 |
| 2730 | C₆₇H₁₃₃N₃O₇S₄ | 1221.0 5 | 8.93 | 22.48 | 2930 | C₇₅H₁₄₅N₃O₈S₃ | 1313.18 | 9.69 | 30.82 |
| 2731 | C₆₃H₁₂₃N₃O₃S₆ | 1163.0 5 | 7.51 | 26.47 | 2931 | C₇₄H₁₄₂N₆O₁₁S₅ | 1452.28 | 7.20 | 26.83 |
| 2732 | C₆₄H₁₃₁NO₃S₇ | 1187.1 8 | 9.65 | 28.98 | 2932 | C₁₀₇H₂₁₀N₄O₆S | 1680.94 | 9.30 | 44.75 |
| 2733 | C₆₃H₁₂₇NOS₈ | 1171.1 9 | 10.03 | 29.92 | 2933 | C₁₀₃H₂₀₅N₅OS₇ | 1754.23 | 7.43 | 49.14 |
| 2734 | C₈₂H₁₆₂NO₆P | 1289.1 7 | 10.20 | 32.78 | 2934 | C₁₀₈H₂₁₂NO₇PS | 1699.92 | 7.96 | 46.71 |
| 2735 | C₇₉H₁₅₃NO₄S₂ | 1245.22 | 9.98 | 34.17 | 2935 | C₉₉H₂₀₀N₄O₂S₃ | 1574.89 | 9.31 | 44.17 |
| 2736 | C₇₉H₁₅₁N₃O₁₀S | 1335.1 5 | 7.16 | 30.27 | 2936 | C₁₀₀H₁₉₉N₇O₁₁S ₄ | 1803.97 | 7.82 | 41.43 |
| 2737 | C₇₉H₁₅₁N₂O₁₀P | 1320.0 5 | 7.65 | 29.97 | 2937 | C₉₇H₁₉₇NO₁₉S_{S} | 1841.93 | 9.29 | 43.58 |
| 2738 | C₇₉H₁₅₂N₄O₅S₃ | 1334.2 9 | 8.33 | 31.06 | 2938 | C₉₉H₁₉₄N₂O₈S | 1572.71 | 7.31 | 41.98 |
| 2739 | C₇₇H₁₄₉NO₄S₃ | 1249.2 2 | 9.44 | 32.44 | 2939 | C₉₄H₁₇₈N₄O₅S₃ | 1540.66 | 7.70 | 37.45 |
| 2740 | C₇₈H₁₅₃N₉O₃S₃ | 1361.3 2 | 8.05 | 31.75 | 2940 | C₇₄H₁₃₇N₃O₄S₅ | 1293.23 | 8.28 | 28.37 |
| 2741 | C₇₅H₁₅₀N₆O₉S₃ | 1376.2 4 | 7.11 | 28.59 | 2941 | C₇₃H₁₅₁N₂O₁₄P₃ S | 1466.05 | 8.41 | 24.98 |
| 2742 | C₈₀H₁₅₄N₄O₉ | 1316.1 3 | 8.39 | 31.34 | 2942 | C₇₃H₁₄₁N₇O₈S₃ | 1341.15 | 9.86 | 23.70 |
| 2743 | C₇₈H₁₅₃NO₈S | 1265.1 4 | 9.73 | 30.75 | 2943 | C₇₆H₁₃₆N₄O₈S | 1266.00 | 7.96 | 27.68 |
| 2744 | C₇₇H₁₅₁NO₆S₂ | 1251.1 8 | 10.04 | 33.91 | 2944 | C₆₉H₁₃₅N₅O₈S₃ | 1259.05 | 9.93 | 26.72 |
| 2745 | C₇₈H₁₅₆N₄O₉S | 1326.1 8 | 9.48 | 31.83 | 2945 | C₆₉H₁₃₅NO₃S₆ | 1219.20 | 10.26 | 30.26 |
| 2746 | C₈₀H₁₅₂N₂O₁₀S₄ | 1430.3 4 | 8.79 | 32.39 | 2946 | C₆₅H₁₂₉NO₃S₆ | 1165.11 | 10.03 | 29.30 |
| 2747 | C₇₉H₁₆₀N₄O₄ | 1230.1 7 | 9.90 | 31.06 | 2947 | C₇₀H₁₃₇N₄O₁₁P S₄ | 1370.10 | 9.59 | 27.67 |
| 2748 | C₈₂H₁₆₈NO₁₁P₃ S | 1469.2 2 | 9.35 | 31.16 | 2948 | C₇₃H₁₄₉N₂O₁₃P₃ S | 1387.98 | 9.89 | 25.38 |
| 2749 | C₇₄H₁₄₉NO₉S₄ | 1325.2 4 | 9.52 | 31.12 | 2949 | C₆₆H₁₃₀N₆O₆S | 1135.86 | 8.58 | 21.52 |
| 2750 | C₇₆H₁₅₁N₃O₉S₃ | 1347.2 4 | 7.62 | 28.28 | 2950 | C₆₉H₁₃₂N₂O₈S | 1149.88 | 9.31 | 25.41 |
| 2751 | C₈₀H₁₆₃NO₉S₄ | 1411.4 2 | 10.02 | 31.61 | 2951 | C₆₇H₁₃₀NO₁₁P | 1156.75 | 9.20 | 24.28 |
| 2752 | C₇₇H₁₅₅N₇O₈S₃ | 1403.3 1 | 8.62 | 28.92 | 2952 | C₇₄H₁₄₁N₄O₇PS | 1262.00 | 10.07 | 25.50 |
| 2753 | C₇₇H₁₅₅NO₁₂S₄ | 1415.3 2 | 9.90 | 31.42 | 2953 | C₇₉H₁₃₉N₇O₂S₈ | 1475.51 | 8.66 | 30.35 |
| 2754 | C₇₉H₁₅₀N₂O₄S₄ | 1320.3 2 | 8.20 | 32.38 | 2954 | C₈₀H₁₄₆N₃O₇PS 6 | 1485.40 | 8.03 | 29.10 |
| 2755 | C₈₀H₁₅₂N₂O₅S₅ | 1382.4 0 | 8.23 | 33.60 | 2955 | C₇₂H₁₃₉NO₁₄S₅ | 1403.20 | 10.24 | 26.99 |
| 2756 | C₈₂H₁₅₇N₃O₉S₄ | 1457.4 1 | 8.95 | 34.69 | 2956 | C₆₈H₁₃₄N₂O₁₁S₅ | 1316.12 | 9.97 | 23.02 |
| 2757 | C₇₈H₁₅₃N₂O₈PS 6 | 1470.4 2 | 7.17 | 32.63 | 2957 | C₇₃H₁₃₈N₄O₅S₇ | 1376.35 | 8.95 | 30.58 |
| 2758 | C₇₈H₁₄₇N₃OS₆ | 1335.4 1 | 8.18 | 32.47 | 2958 | C₇₆H₁₃₆N₂O₈S | 1237.99 | 8.81 | 25.65 |
| 2759 | C₇₈H₁₅₁N₇O₃S₆ | 1427.4 7 | 8.10 | 32.55 | 2959 | C₅₅H₁₀₅N₅O₉S | 1012.53 | 9.99 | 19.03 |
| 2760 | C₈₀H₁₅₇N₇OS₆ | 1425.5 4 | 9.93 | 32.94 | 2960 | C₅₅H₁₀₃N₃O₇S₈ | 1174.92 | 9.06 | 19.72 |
| 2761 | C₁₀₃H₁₉₈N₁₀O₄ | 1640.7 8 | 8.43 | 38.34 | 2961 | C₅₅H₉₈N₂O₇S₆ | 1091.76 | 8.02 | 19.51 |
| 2762 | C₉₉H₁₉₁NO₈S | 1555.6 8 | 9.65 | 43.86 | 2962 | C₅₆H₁₀₄N₂O₇S₄ | 1045.70 | 8.13 | 19.65 |
| 2763 | C₉₉H₁₉₁NO₇S₂ | 1571.7 4 | 9.34 | 44.32 | 2963 | C₅₅H₁₀₃N₃O₁₃S₄ | 1142.68 | 8.95 | 20.08 |
| 2764 | C₁₀₅H₂₀₅NO₅S₃ | 1657.9 8 | 10.13 | 47.57 | 2964 | C₄₉H₉₇NO₄S₅ | 924.62 | 7.78 | 18.16 |
| 2765 | C₁₀₀H₁₉₈NO₃PS 3 | 1589.8 4 | 8.58 | 43.46 | 2965 | C₆₀H₁₁₇N₄O₁₅P₃ | 1227.53 | 8.09 | 15.71 |
| 2766 | C₁₀₂H₂₀₀N₂OS₆ | 1663.0 9 | 10.10 | 45.62 | 2966 | C₅₃H₁₀₅NO₁₂S₅ | 1108.72 | 10.13 | 18.40 |
| 2767 | C₁₀₄H₂₀₅NO₆S | 1597.8 5 | 7.66 | 46.47 | 2967 | C₅₅H₁₀₈N₃O₁₆P S₅ | 1258.75 | 9.37 | 19.39 |
| 2768 | C₁₀₄H₂₀₉N₇O₃S₂ | 1669.9 8 | 9.88 | 46.29 | 2968 | C₅₄H₁₀₄N₄O₁₀S | 1001.50 | 8.74 | 17.69 |
| 2769 | C₁₀₅H₂₀₈N₆O₈ | 1682.8 5 | 9.03 | 44.74 | 2969 | C₅₄H₁₀₆N₂O₁₀S₂ | 1007.57 | 10.02 | 18.69 |
| 2770 | C₁₀₄H₂₀₄N₄P₇S₂ | 1686.9 2 | 9.42 | 47.15 | 2970 | C₆₆H₁₁₇N₅O₈S | 1140.75 | 9.75 | 19.41 |
| 2771 | C₁₀₉H₂₀₈N₂O₉ | 1690.8 7 | 8.92 | 46.22 | 2971 | C₆₈H₁₂₁N₇O₅ | 1116.76 | 8.93 | 17.67 |
| 2772 | C₁₀₅H₂₁₀N₄O₁₀S | 1720.91 | 9.84 | 46.22 | 2972 | C₆₅H₁₁₄N₂O₅S₅ | 1163.94 | 10.02 | 23.50 |
| 2773 | C₁₀₅H₂₀₁N₃O₁₁S | 1713.8 3 | 7.46 | 46.28 | 2973 | C₆₈H₁₁₄N₂O₉S₂ | 1167.78 | 8.32 | 21.74 |
| 2774 | C₁₀₂H₂₀₅N₃O₈S | 1633.8 3 | 8.04 | 44.66 | 2974 | C₇₁H₁₂₆NO₄PS₇ | 1313.19 | 10.09 | 25.85 |
| 2775 | C₁₀₉H₂₂₃N₂O₁₁P ₃S | 1862.9 7 | 9.95 | 45.08 | 2975 | C₇₄H₁₂₉N₃O₆S₉ | 1445.40 | 8.19 | 27.04 |
| 2776 | C₁₀₂H₂₀₈N₂O₉S₃ | 1702.9 7 | 10.14 | 46.00 | 2976 | C₇₄H₁₂₉NO₆S₂ | 1192.97 | 10.35 | 24.90 |
| 2777 | C₁₀₇H₂₀₈N₄O₃S₅ | 1759.1 7 | 8.34 | 49.21 | 2977 | C₇₃H₁₂₅N₃O₁₁S₂ | 1284.93 | 8.96 | 23.77 |
| 2778 | C₁₀₄H₂₀₃NO₁₀S₄ | 1756.0 1 | 9.55 | 48.00 | 2978 | C₇₁H₁₂₅N₅O₁₄S₂ | 1336.92 | 8.57 | 20.70 |
| 2779 | C₁₀₅H₂₀₃NO₇S₄ | 1720.0 2 | 8.74 | 49.41 | 2979 | C₅₉H₁₀₈N₂O₃S₄ | 1021.76 | 10.20 | 20.34 |
| 2780 | C₁₀₈H₂₁₃NO₆S₇ | 1846.3 1 | 10.05 | 50.72 | 2980 | C₆₁H₁₁₈NO₁₄PS 4 | 1248.82 | 10.25 | 22.37 |
| 2781 | C₁₀₃H₂₀₃N₃O₂S₃ | 1611.9 6 | 8.66 | 46.73 | 2981 | C₅₆H₁₀₆N₂O₁₁S₄ | 1111.71 | 8.96 | 17.71 |
| 2782 | C₁₀₄H₂₀₈N₂O₂S₃ | 1615.0 0 | 9.88 | 47.93 | 2982 | C₅₇H₁₀₈N₂O₁₅S₃ | 1157.67 | 10.05 | 20.10 |
| 2783 | C₁₀₁H₂₀₄N₂OS₆ | 1655.1 2 | 8.86 | 49.01 | 2983 | C₆₀H₁₁₀NO₇P | 988.51 | 10.15 | 18.89 |
| 2784 | C₁₀₁H₂₀₅NS₈ | 1690.2 4 | 9.98 | 51.49 | 2984 | C₆₁H₁₀₅N₇O₅ | 1016.55 | 8.67 | 17.66 |
| 2785 | C₁₀₂H₂₀₅N₆O₆P | 1642.7 7 | 9.54 | 39.05 | 2985 | C₆₁H₁₁₂N₂O₉S₂ | 1081.69 | 10.11 | 20.51 |
| 2786 | C₁₀₃H₂₀₃N₉O₅S₂ | 1711.9 3 | 8.13 | 42.14 | 2986 | C₅₉H₁₀₉N₇O₇S | 1060.62 | 8.74 | 15.29 |
| 2787 | C₉₉H₁₉₈N₈O₅ | 1580.7 2 | 9.68 | 41.14 | 2987 | C₇₅H₁₃₈N₆O₁₂ | 1315.96 | 8.80 | 26.03 |
| 2788 | C₁₀₀H₂₀₂N₇O₆P | 1629.7 3 | 9.66 | 40.59 | 2988 | C₇₃H₁₃₄N₃O₁₂P S₅ | 1437.16 | 8.84 | 29.13 |
| 2789 | C₁₀₃H₂₀₃N₉O₅S | 1679.8 8 | 8.70 | 41.15 | 2989 | C₇₀H₁₃₄N₄O₄S₃ | 1192.05 | 9.54 | 26.89 |
| 2790 | C₁₀₀H₁₉₄N₁₀O₈S | 1696.7 7 | 7.27 | 40.42 | 2990 | C₆₉H₁₂₉N₃O₁₀S₅ | 1321.10 | 9.31 | 24.87 |
| 2791 | C₉₈H₁₉₉N₆O₃PS | 1572.7 4 | 8.26 | 41.72 | 2991 | C₇₀H₁₄₁NO₄S | 1092.96 | 10.37 | 29.21 |
| 2792 | C₉₆H₁₉₄N₄O₈S₃ | 1616.8 0 | 8.77 | 39.52 | 2992 | C₇₀H₁₄₀N₁₀O₆S | 1250.01 | 9.15 | 24.15 |
| 2793 | C₉₅H₁₉₄N₆O₈S₃ | 1644.8 1 | 7.43 | 39.83 | 2993 | C₇₂Hi₃₇NO₄S₅ | 1241.19 | 9.95 | 30.25 |
| 2794 | C₁₀₀H₁₉₉N₅O₈ | 1599.7 2 | 10.02 | 42.80 | 2994 | C₇₁H₁₄₆N₆O₂ | 1115.99 | 10.21 | 26.99 |
| 2795 | C₁₀₄H₂₁₃N₄O₉P₃ S | 1788.8 5 | 9.70 | 40.93 | 2995 | C₇₇H₁₃₁N₃O₆S | 1226.97 | 9.99 | 28.80 |
| 2796 | C₉₄H₁₉₂N₂O₁₁S₄ | 1654.8 1 | 9.58 | 41.21 | 2996 | C₈₄H₁₄₁N₂O₁₂P₃ S₄ | 1592.22 | 8.25 | 26.39 |
| 2797 | C₁₀₀H₂₀₂NO₁₅P S₃ | 1785.8 6 | 9.99 | 43.01 | 2997 | C₇₆H₁₂₇N₇0₄ | 1202.90 | 9.28 | 25.07 |
| 2798 | C₉₄H₁₉₁NO₁₇S₄ | 1735.7 9 | 8.03 | 41.32 | 2998 | C₇₆H₁₂₁N₃O₇S₃ | 1285.00 | 8.58 | 26.20 |
| 2799 | C₉₆H₁₉₃NO₁₄S₅ | 1745.8 9 | 9.20 | 43.57 | 2999 | C₇₅H₁₁₉NOioS | 1226.83 | 10.03 | 27.76 |
| 2800 | C₉₇H₁₉₈N₄O_{S}S₃ | 1644.8 5 | 10.17 | 41.87 | 3000 | C₇₇H₁₂₃NO₁₁S₃ | 1335.01 | 9.89 | 27.55 |
| 3001 | C₉₀H₁₅₇N₇O₁₀S₃ | 1593.4 7 | 8.81 | 31.48 | 3201 | C₇₂H₁₂₆N₂O₉ | 1163.81 | 9.97 | 23.54 |
| 3002 | C₈₉H₁₅₅NO₆S₈ | 1591.7 0 | 9.94 | 37.95 | 3202 | Cr₇₀H₁₂₄N₄O₈ | 1149.78 | 9.97 | 23.36 |
| 3003 | C₈₄H₁₆₈NO₁₀P₃ S₃ | 1541.3 7 | 9.83 | 31.59 | 3203 | C₇₁H₁₂₅NO₁₄S | 1248.83 | 9.73 | 22.70 |
| 3004 | C₈₀H₁₆₃N₅O₈S₄ | 1451.4 5 | 10.12 | 31.84 | 3204 | C₇₀H₁₁₇N₃O₁₅ | 1240.71 | 7.26 | 20.93 |
| 3005 | C₈₀H₁₅₂N₄O₇ | 1282.1 2 | 8.76 | 29.06 | 3205 | C₅₇H₉₉N₃O₃S₈ | 1130.92 | 8.63 | 19.99 |
| 3006 | C₁₀₁H₁₉₈N₄O₅S₂ | 1612.8 4 | 9.00 | 42.07 | 3206 | C₆₃H₁₀₉N₆O₅PS ₆ | 1253.94 | 8.12 | 21.93 |
| 3007 | C₁₀₃H₁₉₉N₃O₇S₄ | 1719.9 8 | 8.96 | 44.17 | 3207 | C₅₈H₁₀₅N₂O₄PS ₆ | 1117.82 | 9.26 | 21.79 |
| 3008 | C₁₀₄H₁₉₆N₄O₁₂S ₃ | 1790.9 1 | 8.40 | 43.89 | 3208 | C₅₉H₁₀₅NO₅S₄ | 1036.73 | 9.99 | 21.27 |
| 3009 | C₁₀₅H₂₀₈N₉O₁₂PS₄ | 1948.08 | 8.72 | 40.74 | 3209 | C₅₇H₁₀₃N₃O₁₀S₄ | 1118.70 | 9.68 | 21.46 |
| 3010 | C₇₉H₁₅₂N₆O₅S | 1298.1 8 | 9.08 | 25.22 | 3210 | C₅₉H₁₁₄NO₁₄P₃ S₂ | 1218.60 | 8.06 | 17.66 |
| 3011 | C₇₆H₁₅₂N₆O₁₀S | 1342.1 4 | 9.91 | 26.73 | 3211 | C₆₀H₁₁₆NO₁₂P₃ S₂ | 1200.62 | 8.76 | 19.01 |
| 3012 | C₇₃H₁₄₂N₂O₃S₆ | 1288.3 1 | 8.72 | 30.69 | 3212 | C₆₆H₁₂₉N₃O₁₄P₄ | 1312.66 | 8.69 | 16.22 |
| 3013 | C₇₇H₁₄₅N₅O₁₀S₃ | 1397.2 1 | 8.79 | 29.01 | 3213 | C₆₂H₁₁₇N₂O₁₃P₃ | 1191.54 | 8.34 | 16.88 |
| 3014 | C₇₇H₁₄₁N₇O₆S₈ | 1517.5 0 | 8.62 | 30.23 | 3214 | C₅₅H₁₀₄N₆O₉S₃ | 1089.65 | 8.26 | 15.77 |
| 3015 | C₇₅H₁₅₂N₅O₁₂P S₃ | 1443.2 2 | 8.29 | 25.88 | 3215 | C₅₄H₁₀₂N₂O₁₂S₅ | 1131.71 | 8.76 | 17.82 |
| 3016 | C₇₄H₁₄₅N₃O_{1S}S₄ | 1445.2 2 | 9.05 | 25.98 | 3216 | C₅₅H₁₀₃NO₁₅S₅ | 1178.72 | 9.92 | 19.53 |
| 3017 | C₇₂H₁₄₂N₂O₉S₆ | 1372.2 9 | 8.96 | 24.57 | 3217 | C₅₄H₁₀₅N₅O₄S | 920.52 | 9.05 | 18.70 |
| 3018 | C₇₇H₁₅₇N₉O₇S | 1353.2 2 | 10.26 | 29.33 | 3218 | C₅₃H₁₀₁NO₄S₉ | 1104.93 | 8.98 | 24.27 |
| 3019 | C₇₃H₁₄₅N₃O₅S₅ | 1305.2 8 | 8.80 | 30.66 | 3219 | C₆₀H₁₀₅N₂O₁₂P | 1077.48 | 8.29 | 18.94 |
| 3020 | C₇₄H₁₄₉N₇O₁₀S₄ | 1425.2 8 | 8.95 | 26.66 | 3220 | C₅₈H₁₀₅NO₁₆S | 1104.53 | 9.39 | 18.70 |
| 3021 | C₇₁H₁₃₉N₃O₁₆S₅ | 1451.2 0 | 8.48 | 24.73 | 3221 | C₇₀H₁₂₆N₆O₆S | 1179.87 | 7.23 | 23.31 |
| 3022 | C₇₄H₁₄₅N₃O₁₁S₆ | 1445.3 4 | 9.07 | 26.30 | 3222 | C₇₄H₁₃₄N₁₀O₁₁S | 1372.00 | 8.45 | 23.42 |
| 3023 | C₇₂H₁₄₅N₄O₁₀P S₃ | 1354.1 2 | 8.61 | 23.41 | 3223 | C₆₉H₁₂₃NS₇ | 1191.17 | 9.86 | 26.81 |
| 3024 | C₇₃H₁₄₇N₄O₉PS₅ | 1416.2 7 | 8.33 | 24.01 | 3224 | C₇₁H₁₂₆N₄O₄S₄ | 1228.05 | 7.91 | 25.16 |
| 3025 | C₇₄H₁₄₁NO₁₁S₂ | 1285.0 6 | 7.98 | 28.99 | 3225 | C₇₁H₁₂₈N₂O₅S₆ | 1282.17 | 9.30 | 28.34 |
| 3026 | C₁₀₉H₂₁₃N₅O₈ | 1721.9 3 | 8.96 | 42.65 | 3226 | C₇₇H₁₃₇N₆O₇PS ₅ | 1450.25 | 8.46 | 25.30 |
| 3027 | C₁₀₄H₂₀₅NOS₇ | 1710.2 1 | 10.17 | 49.05 | 3227 | C₇₀H₁₂₅N₃O₁₀S₄ | 1297.02 | 7.45 | 26.12 |
| 3028 | C₁₁₁H₂₁₃N₃O₃S₈ | 1894.4 2 | 8.11 | 50.12 | 3228 | C₇₂H₁₃N₅O₁₁S₃ | 1339.04 | 8.71 | 27.24 |
| 3029 | C₁₁₀H₂₂₂N₃O₁₁P ₃S | 1887.9 8 | 8.96 | 42.00 | 3229 | C₇₅H₁₄₆N₇O₁₁P₃ | 1414.95 | 7.81 | 22.60 |
| 3030 | C₁₁₀H₂₂₂N₃O₁₁P ₃S | 1887.9 8 | 8.96 | 42.00 | 3230 | C₇₃H₁₄₀N₃O₁₄P₃ S | 1408.91 | 8.27 | 22.79 |
| 3031 | C₁₀₆H₂₁N₃O₁₃S₂ | 1799.9 8 | 10.02 | 45.91 | 3231 | C₇₂H₁₄₀N₃O₁₄P₃ | 1364.84 | 8.46 | 23.22 |
| 3032 | C₁₀₃H₂₀₅N₆O₁₀P | 1718.7 8 | 8.96 | 40.53 | 3232 | C₇₀H₁₃₁N₅O₁₃S₄ | 1379.08 | 8.01 | 22.50 |
| 3033 | C₁₀₅H₂₁₃N₄O₁₃P ₃S₂ | 1896.9 2 | 8.94 | 39.79 | 3233 | C₆₇H₁₃₁N₅O₈S₅ | 1295.11 | 9.77 | 25.44 |
| 3034 | C₉₈H₁₉₂N₂O₁₂S₄ | 1718.8 6 | 9.02 | 40.76 | 3234 | C₆₅H₁₂₄N₂O₁₄S₅ | 1318.01 | 7.85 | 23.07 |
| 3035 | C₉₃H₁₈₀N₄O₈S | 1514.5 4 | 9.95 | 38.53 | 3235 | C₇₃H₁₃₉NO₉S₆ | 1367.27 | 10.04 | 28.32 |
| 3036 | C₉₆H₁₈₆N₂O₂S₈ | 1657.0 4 | 10.34 | 44.16 | 3236 | C₇₃H₁₃₀N₄O₈S₃ | 1288.04 | 8.18 | 26.62 |
| 3037 | C₉₄H₁₈₀N₄O₅S₇ | 1670.9 2 | 8.81 | 39.99 | 3237 | C₇₂H₁₃₂N₄O₁₀S | 1245.93 | 9.51 | 23.80 |
| 3038 | C₈₉H₁₇₇NO₁₅S₃ | 1597.5 7 | 10.12 | 38.26 | 3238 | C₇₀H₁₃₂NO₁₂PS ₂ | 1274.91 | 9.58 | 26.32 |
| 3039 | C₉₂H₁₇₇NO₆S₃ | 1489.6 1 | 9.88 | 38.99 | 3239 | C₇₃H₁₃₈N₆O₁₀S | 1292.00 | 8.50 | 25.82 |
| 3040 | C₉₃H₁₈₀N₂O₁₂S₂ | 1582.5 9 | 8.97 | 38.02 | 3240 | C₇₁H₁₃₇N₇O₁₂S | 1312.97 | 7.95 | 25.01 |
| 3041 | C₇₈H₁₃₉N₃O₆S₅ | 1375.2 8 | 9.03 | 28.75 | 3241 | C₇₀H₁₃₂N₂O₂S₅ | 1194.14 | 9.86 | 28.40 |
| 3042 | C₇₁H₁₃₈N₆O₁₃S₅ | 1444.2 1 | 8.79 | 24.44 | 3242 | C₆₈H₁₂₇N₃S₉ | 1275.32 | 8.31 | 29.94 |
| 3043 | C₇₄H₁₃₇NO₁₀S₃ | 1297.0 9 | 10.18 | 28.33 | 3243 | C₇₀H₁₃₂N₂O₉S₄ | 1274.07 | 8.35 | 27.50 |
| 3044 | C₅₇H₁₀₉N₅O₈S₅ | 1152.8 3 | 8.75 | 21.16 | 3244 | C₆₉H₁₃₄N₂O₄S₄ | 1184.08 | 8.84 | 29.64 |
| 3045 | C₅₆H₁₁₂N₆O₃S₂ | 981.67 | 8.79 | 18.08 | 3245 | C₇₁H₁₃₄N₂O₅S₇ | 1320.28 | 7.60 | 30.36 |
| 3046 | C₇₀H₁₂₄N₆O₅S₃ | 1225.9 8 | 9.62 | 21.57 | 3246 | C₇₀H₁₄₂NO₁₂P₃ S₂ | 1346.94 | 8.97 | 25.79 |
| 3047 | C₇₅H₁₄₀N₃O₅S₃ | 1330.1 8 | 10.00 | 23.56 | 3247 | C₇₂H₁₄₅N₂O₁₂P₃ S₂ | 1388.00 | 8.91 | 24.47 |
| 3048 | C₇₄H₁₃₉N₅O₁₁ | 1274.9 5 | 9.94 | 26.43 | 3248 | C₆₅H₁₃₄N₄O₉S₅ | 1276.11 | 8.95 | 25.16 |
| 3049 | C₇₅H₁₃₇N₃O₃S₇ | 1353.3 6 | 8.51 | 29.95 | 3249 | C₆₈H₁₄₁N₅O₉S₆ | 1365.26 | 9.94 | 24.68 |
| 3050 | C₇₁H₁₄₇N₅O₉S₆ | 1407.3 4 | 9.97 | 26.49 | 3250 | C₆₅H₁₃₀N₄O₁₆S₄ | 1352.01 | 8.42 | 25.08 |
| 3051 | C₈₁H₁₃₇N₃O₁₀S₅ | 1473.3 0 | 9.99 | 30.81 | 3251 | C₆₇H₁₃₅N₅OS₂ | 1090.97 | 9.62 | 24.49 |
| 3052 | C₈₂H₁₂₉N₃O₃S₇ | 1509.3 7 | 7.82 | 29.90 | 3252 | C₆₉H₁₃₈N₆O₅S | 1163.96 | 8.79 | 24.62 |
| 3053 | C₉₆H₁₆₅N₂O₁₁P S₄ | 1682.5 9 | 9.47 | 37.52 | 3253 | C₇₀H₁₃₄N₄O₁₁S₂ | 1271.98 | 7.29 | 25.51 |
| 3054 | C₉₃H₁₆₃N₃O₃S₅ | 1531.6 4 | 8.96 | 37.35 | 3254 | C₇₁H₁₃₇NO₆S₂ | 1165.00 | 10.07 | 27.92 |
| 3055 | C₉₈H₁₇₂NO₁₀P₃ S₃ | 1713.5 5 | 7.92 | 34.58 | 3255 | C₇₄H₁₄₀N₄O₉S₂ | 1294.07 | 8.00 | 27.90 |
| 3056 | C₉₆H₁₆₀N₄O₆S | 1498.4 2 | 8.16 | 36.02 | 3256 | C₇₃H₁₃₉NO₁₀S₂ | 1255.03 | 10.04 | 28.57 |
| 3057 | C₈₇H₁₇₄NO₁₃P₃ | 1535.2 6 | 9.76 | 31.67 | 3257 | C₇₂H₁₄₁NO₁₃S | 1260.97 | 10.11 | 29.32 |
| 3058 | C₇₉H₁₅₃N₂O₇PS ₃ | 1370.2 5 | 9.95 | 30.27 | 3258 | C₇₈H₁₂₆N₈O₉ | 1319.91 | 8.30 | 26.16 |
| 3059 | C₇₅H₁₄₈N₆O₈S₆ | 1454.4 0 | 8.59 | 27.41 | 3259 | C₇₆H₁₂₅N₅O₁₀S₂ | 1332.98 | 8.90 | 26.29 |
| 3060 | C₁₁₀H₂₂₀N₁₀O₇ | 1795.0 3 | 9.47 | 43.87 | 3260 | C₇₈H₁₃₄N₁₂O₄S₂ | 1368.13 | 9.28 | 25.15 |
| 3061 | C₁₁₀H₂₁₃N₃O₁₀S ₅ | 1898.2 2 | 8.79 | 48.01 | 3261 | C₇₇H₁₂₇N₉O₅S₂ | 1323.04 | 8.94 | 26.78 |
| 3062 | C₁₁₀H₂₂₃N₂O₁₁P ₃S₂ | 1907.0 4 | 9.90 | 44.27 | 3262 | C₇₇H₁₂₅N₂O₆PS ₈ | 1462.31 | 8.08 | 28.61 |
| 3063 | C₁₀₉H₂₁₄N₄O₆ | 1676.9 3 | 9.12 | 44.30 | 3263 | C₇₇H₁₂₃NO₄S₇ | 1351.25 | 9.51 | 29.37 |
| 3064 | C₁₀₇H₂₁₀N₆O₈ | 1708.8 9 | 10.02 | 43.55 | 3264 | C₇₇H₁₂₂N₄O₁₁S₄ | 1408.08 | 7.58 | 26.28 |
| 3065 | C₉₃H₁₈₅N₃O₁₅S₄ | 1713.75 | 8.68 | 36.47 | 3265 | C₈H₁₄₁N₆O₁₂P₃ | 1483.97 | 7.90 | 24.37 |
| 3066 | C₉₁H₁₈₃NO₁₆S₅ | 1707.7 6 | 9.33 | 39.98 | 3266 | C₇₃H₁₂₇N₅O₈S₄ | 1331.09 | 9.94 | 26.26 |
| 3067 | C₈₀H₁₄₆N₃O₉PS ₃ | 1421.2 1 | 9.01 | 30.06 | 3267 | C₇₄H₁₃₂N₄O₁₂P₂ S₃ | 1428.01 | 9.34 | 25.27 |
| 3068 | C₇₄H₁₃₈N₂O₁₂S | 1279.9 8 | 9.95 | 27.25 | 3268 | C₇₆H₁₃₁N₃O₁₃S₄ | 1423.13 | 9.02 | 27.31 |
| 3069 | C₇₃H₁₄₈N₉O₁₁P S | 1391.0 7 | 8.77 | 22.93 | 3269 | C₇₅H₁₂₇N₅O₉S₃ | 1339.05 | 9.48 | 21.87 |
| 3070 | C₆₉H₁₃₄NO₈PS₄ | 1265.0 4 | 9.98 | 27.73 | 3270 | C₇₅H₁₂₂N₂O₈ | 1179.81 | 8.88 | 25.72 |
| 3071 | C₇₃H₁₄₂N₃O₁₁P S₃ | 1365.1 0 | 9.62 | 27.62 | 3271 | C₇₉H₁₂₈N₂O₁₂S₂ | 1362.01 | 9.85 | 28.34 |
| 3072 | C₅₆H₁₀₄N₄S₅ | 993.78 | 8.81 | 19.28 | 3272 | C₇₆H₁₂₄N₂O₁₁S₂ | 1305.95 | 9.98 | 27.72 |
| 3073 | C₆₈H₁₂₃N₂O₉P | 1143.7 1 | 8.74 | 20.97 | 3273 | C₉₁H₁₅₄N₆O₈S₂ | 1524.39 | 8.48 | 30.75 |
| 3074 | C₇₇H₁₃₇N₄O₁₂P S₃ | 1438.1 1 | 8.96 | 25.55 | 3274 | Co₂H₁₅₅N₉O₈ | 1515.31 | 8.03 | 32.69 |
| 3075 | C₇₄H₁₃₄N₄O₇S | 1223.9 7 | 10.08 | 21.69 | 3275 | C₉₃H₁₆₁N₄O₉PS | 1542.36 | 9.40 | 34.53 |
| 3076 | C₇₄H₁₃₈N₂O₆ | 1151.9 3 | 10.06 | 24.52 | 3276 | C₉₀H₁₅₃NO₄S₇ | 1537.64 | 9.51 | 36.88 |
| 3077 | C₉₄H₁₆₃N₅O₇S | 1507.4 3 | 10.19 | 33.88 | 3277 | C₉₄H₁₅₄N₂O₁₀S₅ | 1632.57 | 8.01 | 35.49 |
| 3078 | C₉₂H₁₆₃N₇O₃S₅ | 1655.6 6 | 9.65 | 32.84 | 3278 | C₉₄H₁₅₅N₉O₇S₇ | 1747.75 | 8.18 | 34.85 |
| 3079 | C₈₃H₁₅₉NO₇S₅ | 1443.4 8 | 10.08 | 34.53 | 3279 | C₉₁H₁₆₀N₂O₁₁S₆ | 1650.64 | 9.59 | 34.80 |
| 3080 | C₈₁H₁₆₆N₅O₁₁P S₅ | 1577.5 2 | 8.86 | 29.78 | 3280 | C₈₈H₁₅₃N₃O₁₄S₃ | 1573.38 | 8.83 | 34.94 |
| 3081 | C₁₀₆H₂₀₅N₃O₅S₇ | 1826.2 4 | 9.06 | 44.87 | 3281 | C₈₇H₁₄₇NO₁₄S₄ | 1559.37 | 9.24 | 35.58 |
| 3082 | C₁₀₁H₁₉₉NO₇S₈ | 1796.1 8 | 10.22 | 47.81 | 3282 | C₉₀H₁₅₀N₄O₉S | 1464.27 | 9.12 | 33.75 |
| 3083 | C₁₀₂H₂₀₅NO₁₁S₅ | 1782.0 6 | 10.35 | 44.92 | 3283 | C₉₂H₁₅₈NO₉PS₂ | 1517.37 | 9.60 | 37.32 |
| 3084 | C₁₀₀H₁₉₉NO₂S₈ | 1704.18 | 7.88 | 48.22 | 3284 | C₉₂H₁₅₁NO₆S₄ | 1495.46 | 9.87 | 35.38 |
| 3085 | C₇₁H₁₄₆N₂O₁₃S₅ | 1396.2 5 | 9.98 | 26.51 | 3285 | C₉₀H₁₄₈N₄O₁₀S₂ | 1510.31 | 7.57 | 32.53 |
| 3086 | C₇₆H₁₄₈N₁O₁₂S₅ | 1470.3 4 | 8.20 | 25.26 | 3286 | C₉₀H₁₅₅N₅O₈S | 1467.32 | 8.94 | 33.22 |
| 3087 | C₁₁₁H₂₁₉N₂O₄P S₆ | 1869.3 2 | 10.16 | 48.79 | 3287 | C₇₇H₁₅₅N₉O₇ | 1319.14 | 9.48 | 28.87 |
| 3088 | C₁₁₁H₂₁₃N₃O₁₀8 ₅ | 1910.2 4 | 8.95 | 48.91 | 3288 | C₈₁H₁₅₂N₁₀O₁₀S | 1458.23 | 8.16 | 30.05 |
| 3089 | C₁₁₂H₂₁₅N₃O₁₂S ₃ | 1892.1 4 | 8.95 | 48.47 | 3289 | C₇₇H₁₅₃N₉O₆S₃ | 1397.31 | 7.51 | 28.31 |
| 3090 | C₁₀₅H₂₀₁N₃O₁₃S ₄ | 1842.0 1 | 8.05 | 44.33 | 3290 | C₇₉H₁₅₄N₈O₉ | 1360.15 | 7.29 | 28.84 |
| 3091 | C₉₃H₁₈₅N₃O₈S | 1505.5 8 | 8.82 | 36.48 | 3291 | C₇₇H₁₄₉NO₃S₈ | 1393.52 | 7.90 | 34.02 |
| 3092 | C₁₀₀H₁₉₉N₄O₁₃P ₃S₂ | 1822.7 5 | 8.21 | 37.37 | 3292 | C₇₉H₁₅₄N₆O₃S₆ | 1428.50 | 9.20 | 32.59 |
| 3093 | C₈₁H₁₄₆N₃O₁₂P S₃ | 1481.2 2 | 8.46 | 28.15 | 3293 | C₇₆H₁₄₈N₂O₃S₇ | 1362.45 | 9.46 | 34.16 |
| 3094 | C₆₇H₁₂₆N₆O₅S₂ | 1159.9 0 | 8.50 | 24.22 | 3294 | C₈₂H₁₅₉N₃O₁₀P₂ S₃ | 1505.31 | 8.51 | 30.25 |
| 3095 | C₆₈H₁₃₃N₆O₉PS | 1241.8 8 | 7.67 | 19.74 | 3295 | C₇₈H₁₅₂N₂₇S₄ | 1358.32 | 8.76 | 32.96 |
| 3096 | C₆₉H_{134N}sO_{7S2} | 1252.0 0 | 8.41 | 22.96 | 3296 | C₇₉H₁₅₄N₄O₁₀S₄ | 1448.36 | 8.70 | 31.46 |
| 3097 | C₇₀H₁₃₁N₃O₆S₈ | 1367.3 1 | 7.69 | 26.18 | 3297 | C₇₉H₁₆₂NO₃PS₄ | 1333.38 | 9.86 | 33.98 |
| 3098 | C₇₀H₁₃₅NO₄S₇ | 1279.2 7 | 10.12 | 30.32 | 3298 | C₈₀H₁₅₄N₂O₁₁S₄ | 1448.35 | 9.26 | 33.61 |
| 3099 | C₇₀H₁₃₂N₄O₁₂S₄ | 1350.0 8 | 7.46 | 26.35 | 3299 | C₇₆H₁₄₅NO₆S₈ | 1425.48 | 8.81 | 34.83 |
| 3100 | C₆₆H₁₂₅N₃O₁₁S₄ | 1264.9 8 | 8.20 | 24.53 | 3300 | C₈₂H₁₇₀N₃O₁₁P₃ | 1467.19 | 9.97 | 30.57 |
| 3101 | C₆₇H₁₂₉NO₁₁S₅ | 1285.0 6 | 9.67 | 26.84 | 3301 | C₇₇H₁₅₂N₆O₁₁S₅ | 1498.39 | 7.59 | 29.12 |
| 3102 | C₆₇H₁₂₃N₃O₆S₇ | 1291.1 6 | 8.47 | 27.22 | 3302 | C₇₄H₁₅₃N₇O₁₂S₅ | 1493.38 | 8.28 | 28.08 |
| 3103 | C₆₉H₁₃₈NO₁₂P₃ S₃ | 1362.9 6 | 9.26 | 24.34 | 3303 | C₇₆H₁₅₂N₆O₁₂S₆ | 1534.44 | 7.73 | 28.03 |
| 3104 | C₆₃H₁₂₅N₇O₉S₆ | 1317.0 9 | 7.80 | 22.84 | 3304 | C₇₇H₁₅₀N₂O₁₂S₆ | 1488.41 | 7.93 | 28.36 |
| 3105 | C₆₅H₁₂₈N₄O₁₃S₅ | 1334.0 5 | 8.14 | 22.01 | 3305 | C₇₇H₁₅₁N₂O₁₀P S₅ | 1456.33 | 8.39 | 26.01 |
| 3106 | C₆₄H₁₂₈N₃O₁₅P S₄ | 1338.9 5 | 7.40 | 22.22 | 3306 | C₇₉H₁₅₅N₃O₁₀S₃ | 1403.30 | 8.45 | 27.66 |
| 3107 | C₆₃H₁₂₅N₃O₁₀S₇ | 1309.1 2 | 8.27 | 22.61 | 3307 | C₈₀H₁₅₂N₆O₆ | 1294.13 | 8.06 | 30.31 |
| 3108 | C₆₃H₁₂₆N₂O₇S₄ | 1151.9 5 | 9.53 | 20.16 | 3308 | C₈₁H₁₅₀N₆O₉S | 1384.18 | 8.07 | 31.20 |
| 3109 | C₆₈H₁₃₁N₇O₈S₂ | 1238.9 6 | 8.67 | 22.69 | 3309 | C₇₈H₁₅₂N₂O₁₁S₂ | 1358.20 | 8.66 | 29.10 |
| 3110 | C₇₁H₁₃₆N₂O₅S | 1129.9 4 | 9.90 | 27.52 | 3310 | C₈₂H₁₅₂N₈O₁₀ | 1410.16 | 7.54 | 29.00 |
| 3111 | C₇₁H₁₃₈NO₆PS₂ | 1196.9 8 | 9.97 | 25.57 | 3311 | C₈₀H₁₅₃N₅O₁₀ | 1345.13 | 8.37 | 30.38 |
| 3112 | C₇₅H₁₄₃N₅O₃S₂ | 1227.1 2 | 10.12 | 25.60 | 3312 | C₈₂H₁₅₅N₃O₁₁S₂ | 1423.27 | 8.49 | 32.30 |
| 3113 | C₇₂Hₗ₁₃₈N₈O₅S | 1228.0 1 | 9.88 | 25.83 | 3313 | C₉₇H₁₉₇N₂O₆P | 1518.62 | 9.48 | 42.37 |
| 3114 | C₇₆H₁₃₄N₄O₄S₈ | 1424.4 1 | 8.11 | 29.40 | 3314 | C₁₀₀H₂₀₃NO₆S₃ | 1611.90 | 9.02 | 44.49 |
| 3115 | C₇₁H₁₃₃N₅O₆S₄ | 1281.1 1 | 7.36 | 26.07 | 3315 | C₁₀₄H₂₀₇N₆O₁₂P S | 1796.86 | 8.77 | 40.97 |
| 3116 | C₇₂H₁₃₂N₄O₅S₃ | 1230.0 5 | 9.26 | 27.72 | 3316 | C₁₀₀H₁₉₈N₇O₁₂P S | 1753.75 | 7.59 | 39.40 |
| 3117 | C₇₃H₁₃₂N₄O₈S₆ | 1386.2 4 | 7.72 | 28.90 | 3317 | C₁₀₃H₂₀₄N₉O₉P S | 1775.85 | 7.96 | 38.97 |
| 3118 | C₇₇H₁₅₂N₂O₁₅P₄ S | 1502.0 2 | 8.23 | 24.37 | 3318 | C₁₀₀H₁₉₀N₄O₅S₆ | 1721.00 | 7.09 | 43.77 |
| 3119 | C₇₇H₁₄₈NO₁₁P₃ S₃ | 1453.1 3 | 9.91 | 26.96 | 3319 | C₁₀₃H₁₉₉N₄O₄P S₆ | 1781.08 | 7.23 | 43.88 |
| 3120 | C₇₁H₁₃₇N₅O₅S₅ | 1349.2 0 | 7.82 | 25.24 | 3320 | C₁₀₀H₁₉₇N₅O₈S₄ | 1725.94 | 9.14 | 42.88 |
| 3121 | C₇₂H₁₃₉N₆O₁₁PS₅ | 1456.21 | 7.67 | 26.24 | 3321 | C₁₀₄H₁₉₈N₄O₉S₆ | 1841.11 | 8.25 | 44.96 |
| 3122 | C₇₃H₁₄₁N₂O₁₂P S₄ | 1398.1 5 | 8.95 | 24.70 | 3322 | C₉₉H₁₉₂N₂O₁₄S₅ | 1794.92 | 8.35 | 43.66 |
| 3123 | C₇₃H₁₄₀NO₁₃PS₄ | 1399.1 3 | 9.72 | 26.53 | 3323 | C₁₀₃H₂₀₁N₂O₁₀P S₄ | 1786.96 | 8.46 | 44.74 |
| 3124 | C₆₉H₁₂₇N₃O₁₂S₅ | 1351.0 8 | 7.54 | 25.78 | 3324 | C₁₀₂H₁₉₅N₇O₅S₆ | 1840.08 | 8.41 | 43.99 |
| 3125 | C₇₂H₁₃₅N₅O₁₂S₃ | 1359.0 8 | 7.71 | 24.04 | 3325 | C₁₀₂H₂₀₄N₃O₁₆P ₃S | 1853.74 | 7.25 | 38.61 |
| 3126 | C₆₈H₁₃₀N₂O₉S₅ | 1280.0 9 | 9.58 | 25.45 | 3326 | C₁₀₆H₂₁₄N₃O₁₁P ₃S₂ | 1863.93 | 8.94 | 40.89 |
| 3127 | C₇₁H₁₃₃NO₁₃S₄ | 1337.0 8 | 9.69 | 26.71 | 3327 | C₉₇H₁₉₇N₅O₁₀S₃ | 1689.85 | 9.91 | 40.33 |
| 3128 | C₆₉H₁₃₂N₂O₁₀S₆ | 1342.1 8 | 8.71 | 22.55 | 3328 | C₉₇H₁₉₂N₆O₈S₇ | 1795.06 | 7.50 | 39.79 |
| 3129 | C₇₂H₁₃₃N₃O₁₃S | 1280.9 2 | 7.06 | 25.48 | 3329 | C₉₇H₁₉₇N₇O₆S₅ | 1717.99 | 9.70 | 39.61 |
| 3130 | C₇₈H₁₄₃NO₉S₃ | 1335.1 8 | 10.04 | 30.97 | 3330 | C₉₉H₁₉₃N₃O₁₀S₇ | 1810.06 | 8.90 | 42.09 |
| 3131 | C₇₆H₁₃₄N₆O₁₂S | 1356.0 0 | 8.01 | 25.46 | 3331 | C₉₇H₁₉₀N₄O₉S₃ | 1652.79 | 8.14 | 38.15 |
| 3132 | C₇₂H₁₃₆N₄O₅S₂ | 1250.0 2 | 9.77 | 23.12 | 3332 | C₉₈H₁₉₃N₃O₁₀S₆ | 1765.99 | 8.13 | 37.98 |
| 3133 | C₄₈H₉₇NO₇S₃ | 896.48 | 9.93 | 17.95 | 3333 | C₉₆H₁₉₄N₄OS₂ | 1484.76 | 9.09 | 41.92 |
| 3134 | C₅₅H₁₀₅N₇O₆S | 992.55 | 8.64 | 14.03 | 3334 | C₁₀₅H₂₀₁N₂O₉P S | 1698.80 | 7.87 | 42.52 |
| 3135 | C₅₂H₉₇N₅O₈ | 920.37 | 7.30 | 14.27 | 3335 | C₁₀₂H₁₉₃N₃O₇S₂ | 1637.80 | 7.73 | 42.08 |
| 3136 | C₅₅H₁₀₈N₄O₆S₃ | 1017.6 7 | 10.20 | 20.03 | 3336 | C₁₀₁H₁₉₂N₄O₁₂ | 1654.66 | 7.87 | 39.92 |
| 3137 | C₅₁H₁₀₁N₄O₁₀P S₂ | 1025.4 8 | 9.12 | 18.42 | 3337 | C₉₉H₁₉₁NO₁₅S | 1667.67 | 9.69 | 42.63 |
| 3138 | C₅₅H₁₀₁N₃O₄S₄ | 996.67 | 8.27 | 18.05 | 3338 | C₇₅H₁₄₃N₁₃O₆S | 1355.11 | 8.07 | 25.23 |
| 3139 | C₅₂H₁₀₀N₄O₃₈S₃ | 925.58 | 8.64 | 18.64 | 3339 | C₇₆H₁₄₅N₉O₅S₄ | 1393.29 | 7.57 | 26.41 |
| 3140 | C₄₈H₉₄N₂O₃S₅ | 907.59 | 7.71 | 18.31 | 3340 | C₇₃H₁₅₁N₉O₉ | 1359.12 | 7.94 | 25.79 |
| 3141 | C₅₆H₁₀₆N₂O₉S₅ | 1111.7 7 | 10.02 | 23.32 | 3341 | C₇₆H₁₃₉N₃O₃S₈ | 1399.45 | 7.63 | 30.64 |
| 3142 | C₅₅H₉₉N₃O₇S₇ | 1138.83 | 9.16 | 21.40 | 3342 | C₇₆H₁₄₅NO₅S₅ | 1313.30 | 9.99 | 31.73 |
| 3143 | C₅₄H₁₀₇N₂O₁₄P₃ S | 1133.4 3 | 7.27 | 15.46 | 3343 | C₇₂H₁₃₇N₃O₇S₃ | 1253.08 | 9.28 | 29.58 |
| 3144 | C₅₇H₁₁₆N₃O₁₀P₃ S | 1128.5 5 | 9.57 | 15.21 | 3344 | C₆₇H₁₃₈N₂O₅S₅ | 1212.15 | 8.77 | 28.81 |
| 3145 | C₅₈H₁₁₉N₃O₁₄P₄ | 1206.4 9 | 9.37 | 15.64 | 3345 | C₇₂H₁₃₇NO₄S₉ | 1369.43 | 9.47 | 33.57 |
| 3146 | C₅₀H₁₀₂N₆O₉S₄ | 1059.6 4 | 8.91 | 14.11 | 3346 | C₇₄H₁₅₂N₃O₁₂P₃ S | 1401.02 | 9.40 | 26.10 |
| 3147 | C₄₆H₉₆N₆O₈S₃ | 957.49 | 9.30 | 14.92 | 3347 | C₆₆H₁₃₈N₄O₉S₅ | 1292.15 | 8.80 | 23.19 |
| 3148 | C₄₉H₉₅NO₁₀S₇ | 1082.7 2 | 8.89 | 17.71 | 3348 | C₇₁H₁₄₂N₂O₁₁S₅ | 1360.22 | 10.10 | 28.27 |
| 3149 | C₅₂H₁₀₆N₂O₈S₄ | 1015.6 7 | 10.20 | 15.94 | 3349 | C₆₉H₁₃₇N₃O₉S₆ | 1345.23 | 8.87 | 26.02 |
| 3150 | C₅₂H₁₀₂N₆OS₄ | 955.67 | 8.71 | 17.72 | 3350 | C₇₁H₁₃₉N₃O₁₄S₆ | 1451.26 | 7.99 | 27.43 |
| 3151 | C₄₈H₉₉N₅O₅ | 826.35 | 8.78 | 16.86 | 3351 | C₇₁H₁₄₀N₆O₂S₂ | 1174.06 | 7.56 | 26.04 |
| 3152 | C₅₀H₁₀₀N₂O₂S₇ | 985.78 | 8.94 | 20.77 | 3352 | C₇₀H₁₄₃N₅O₂ | 1086.95 | 9.65 | 26.48 |
| 3153 | C₅₀H₉₅NO₉S₂ | 918.43 | 8.74 | 17.41 | 3353 | C₆₉H₁₄₀N₃O₃ | 1129.93 | 7.47 | 24.72 |
| 3154 | C₅₃H₉₇N₃O₁₀ | 936.37 | 8.02 | 17.12 | 3354 | C₇₇H₁₄₁N₃O₈S₂ | 1301.11 | 7.55 | 28.26 |
| 3155 | C₅₂H₉₉N₃O_{S}S₂ | 958.50 | 9.45 | 17.41 | 3355 | C₇₂H₁₃₅N₃O₁₀S | 1234.94 | 7.57 | 26.66 |
| 3156 | C₅₂H₉₆N₃O₁₁PS | 1002.3 8 | 8.12 | 16.02 | 3356 | C₇₄H₁₃₈N₂O₁₀S | 1247.98 | 7.46 | 27.52 |
| 3157 | C₅₂H₉₂N₂O₁₅ | 985.31 | 7.52 | 16.32 | 3357 | C₇₀H₁₃₆N₂O₁₀S₂ | 1229.98 | 9.66 | 28.88 |
| 3158 | C₅₃H₁₀₀N₂O₁₃ | 973.38 | 8.88 | 15.78 | 3358 | C₇₅H₁₅₀N₈O₅ | 1244.08 | 9.70 | 26.96 |
| 3159 | C₅₃H₁₀₃N₆O₁₀P S | 1047.4 7 | 8.46 | 14.49 | 3359 | C₇₄H₁₄₄N₅O₈PS | 1295.03 | 8.00 | 25.17 |
| 3160 | C₆₀H₁₁₀N₂O₇S₂ | 1035.6 7 | 8.77 | 21.53 | 3360 | C₇₂H₁₄₅N₅O₆S₃ | 1321.16 | 9.35 | 26.34 |
| 3161 | C₆₁H₁₀₉NO₇S₂ | 1032.6 6 | 9.76 | 22.04 | 3361 | C₇₁H₁₄₄N₈O₇S | 1254.04 | 7.70 | 26.61 |
| 3162 | C₆₈H₁₂₄N₇O₁₁P S | 1278.8 1 | 9.49 | 18.68 | 3362 | C₇₉H₁₅₁N₉O₇S₂ | 1403.25 | 8.31 | 26.66 |
| 3163 | C₆₈H₁₁₇NO₃S₆ | 1189.0 5 | 9.07 | 26.17 | 3363 | C₇₂H₁₄₄N₁₀O₇S₃ | 1358.19 | 8.43 | 26.21 |
| 3164 | C₆₅H₁₁₀N₂O₃S₆ | 1159.9 7 | 9.44 | 24.53 | 3364 | C₇₁H₁₃₅NO₂S₇ | 1259.29 | 9.74 | 30.80 |
| 3165 | C₆₇H₁₁₄N₄O₄S₈ | 1296.15 | 8.05 | 23.10 | 3365 | C₇₃H₁₃₉NO₂S₈ | 1319.40 | 8.81 | 32.79 |
| 3166 | C₆₇H₁₁₉N₃OS₆ | 1175.0 7 | 9.63 | 26.04 | 3366 | C₇₁H₁₄₄NO₅PS₄ | 1251.15 | 10.03 | 31.16 |
| 3167 | C₆₄H₁₀₇NO₂S₆ | 1114.9 2 | 8.40 | 23.72 | 3367 | C₆₉H₁₃₉NO₃S₆ | 1223.23 | 9.21 | 31.23 |
| 3168 | C₆₅H₁₁₁N₅O₅S₃ | 1138.8 1 | 8.82 | 21.52 | 3368 | C₇₅H₁₄₁N₂O₁₁P S₄ | 1406.17 | 8.64 | 28.77 |
| 3169 | C₅₇H₁₀₅N₃O₃S₄ | 1008.7 2 | 8.60 | 22.28 | 3369 | C₇₄H₁₅₃N₄O₁₁P₃ S₂ | 1432.10 | 8.31 | 25.59 |
| 3170 | C₆₄H₁₁₃N₅O₅S₇ | 1257.0 6 | 9.42 | 23.90 | 3370 | C₇₇H₁₅₀N₃O₁₄P₃ S | 1467.04 | 7.45 | 24.68 |
| 3171 | C₆₄H₁₁₈NO₁₂P₃ S₄ | 1314.8 0 | 8.70 | 19.42 | 3371 | C₇₁H₁₄₆N₈O₈S₄ | 1368.24 | 8.39 | 25.23 |
| 3172 | C₅₇H₁₀₇N₅O₈S₄ | 1118.7 5 | 9.82 | 18.80 | 3372 | C₆₈H₁₃₅NO₁₅S₅ | 1367.12 | 8.51 | 26.84 |
| 3173 | C₆₀H₁₁₃N₅O₈S₄ | 1160.8 3 | 9.93 | 19.66 | 3373 | C₇₁H₁₄₂N₆O₁₅S₃ | 1416.12 | 7.02 | 26.05 |
| 3174 | C₆₃H₁₁₈N₆O₉S₄ | 1231.9 1 | 9.47 | 17.86 | 3374 | C₆₈H₁₃₅NO₁₀S₃ | 1223.00 | 8.08 | 24.60 |
| 3175 | C₆₂H₁₁₁N₃O₁₃S₄ | 1234.8 2 | 8.51 | 17.77 | 3375 | C₇₃H₁₄₄N₃O₁₀P S₅ | 1415.24 | 8.25 | 22.65 |
| 3176 | C₆₃H₁₁₄NO₁₃PS ₅ | 1284.8 7 | 9.07 | 21.47 | 3376 | C₇₂H₁₃₇N₃O₁₁S₆ | 1413.26 | 7.91 | 23.79 |
| 3177 | C₆₂H₁₁₇N₂O₁₅P S₄ | 1289.8 3 | 9.18 | 18.80 | 3377 | C₇₀H₁₄₂N₂O₁₀S₄ | 1300.15 | 9.93 | 24.88 |
| 3178 | C₆₂H₁₁₁N₃O₁₄S₆ | 1314.9 4 | 8.47 | 19.91 | 3378 | C₆₈H₁₃₆N₆O₇S₂ | 1213.99 | 7.07 | 26.69 |
| 3179 | C₆₃H₁₁₅N₂O₁₇P S₅ | 1363.8 8 | 8.72 | 20.79 | 3379 | C₇₂H₁₄₂N₆O₄ | 1155.97 | 8.79 | 26.00 |
| 3180 | C₅₈H₁₀₇N₃O₈S₃ | 1070.6 9 | 9.95 | 16.48 | 3380 | C₇₅H₁₄₆N₃O₇PS | 1265.04 | 9.30 | 28.09 |
| 3181 | C₆₀H₁₀₆N₂O₈S₃ | 1079.6 9 | 9.71 | 22.06 | 3381 | C₇₄H₁₃₉N₃O₆S₃ | 1263.12 | 8.03 | 27.70 |
| 3182 | C₆₄H₁₁₂N₄O₅S₂ | 1081.7 4 | 9.13 | 19.93 | 3382 | C₇₅H₁₄₀N₂O₇ | 1181.95 | 8.49 | 27.43 |
| 3183 | C₇₂H₁₂₉N₇O₆S | 1220.9 3 | 9.47 | 19.86 | 3383 | C₇₄H₁₃₇N₃O₇S₄ | 1309.16 | 8.31 | 28.35 |
| 3184 | C₇₄H₁₃₁N₇O₆S₂ | 1279.0 3 | 8.74 | 20.89 | 3384 | C₇₄H₁₃₇N₃O₁₄S₂ | 1357.04 | 8.49 | 25.65 |
| 3185 | C₆₉H₁₂₃N₅O₈S | 1182.8 3 | 9.47 | 23.43 | 3385 | C₁₀₇H₂₀₉N₅O₁₀S | 1757.93 | 9.69 | 46.30 |
| 3186 | C₇₁H₁₂₆N₆O₁₀S₃ | 1320.0 0 | 8.10 | 21.56 | 3386 | C₁₀₅H₂₀₈N₆O₁₂S | 1778.91 | 9.08 | 44.20 |
| 3187 | C₇₀H₁₂₉N₄O₁₁P S | 1265.8 5 | 8.87 | 23.05 | 3387 | C₁₀₇H₂₀₉N₇O₁₀S ₂ | 1818.01 | 7.78 | 44.86 |
| 3188 | C₇₁H₁₂₈N₈O₆S | 1221.9 1 | 9.84 | 21.09 | 3388 | C₁₀₅H₂₀₉N₇O₇S | 1713.93 | 9.76 | 43.29 |
| 3189 | C₆₅H₁₂₂N₈O₉S₂ | 1223.8 6 | 7.01 | 20.16 | 3389 | C₁₁₀H₂₁₄N₃O₇P S₅ | 1882.21 | 8.72 | 46.15 |
| 3190 | C₇₂H₁₂₈N₃O₇PS ₄ | 1307.0 4 | 8.58 | 21.87 | 3390 | C₁₀₄H₂₀₃NO₄S₇ | 1756.19 | 7.73 | 48.33 |
| 3191 | C₆₉H₁₂₀N₂O₂S₇ | 1234.1 5 | 9.59 | 25.76 | 3391 | C₁₁₀H₂₁₄NO₈PS ₄ | 1838.13 | 9.76 | 48.15 |
| 3192 | C₇₂H₁₂₈N₂O₃S₇ | 1294.2 5 | 9.98 | 27.71 | 3392 | C₁₀₅H₂₀₅NO₈S₅ | 1770.09 | 8.22 | 48.48 |
| 3193 | C₇₁H₁₂₀N₂O₇S₃ | 1209.9 3 | 8.72 | 23.50 | 3393 | C₁₀₆H₂₀₇N₃O₈S₃ | 1748.01 | 8.86 | 45.55 |
| 3194 | C₆₉H₁₁₉NO₇S₇ | 1299.1 3 | 9.31 | 26.45 | 3394 | C₁₀₂H₂₀₂N₄O₆S₄ | 1709.00 | 8.10 | 46.12 |
| 3195 | C₆₆H₁₂₀N₂O₅S₄ | 1149.9 3 | 7.54 | 24.03 | 3395 | C₁₁₁H₂₂₁N₂O₁₃P ₃S₂ | 1949.03 | 7.97 | 44.55 |
| 3196 | C₇₄H₁₃₇N₂O₁₂P₃ S₃ | 1436.0 1 | 8.52 | 22.06 | 3396 | C₁₀₀H₁₉₉NO₁₁S₇ | 1816.11 | 7.43 | 44.88 |
| 3197 | C₇₄H₁₃₆N₅O₁₂P₃ S₂ | 1444.9 7 | 7.61 | 19.31 | 3397 | C₁₀₅H₂₁₂N₃O₁₃P S₃ | 1852.01 | 8.82 | 42.85 |
| 3198 | C₆₆H₁₂₃N₅O₆S₅ | 1243.0 4 | 9.94 | 20.38 | 3398 | C₁₀₂H₂₀₃N₅O₁₂S ₅ | 1852.07 | 7.51 | 45.60 |
| 3199 | C₆₈H₁₂₃N₃O₁₂S₄ | 1302.9 8 | 8.93 | 19.98 | 3399 | C₁₀₂H₂₁₀N₄OS | 1540.89 | 10.13 | 45.50 |
| 3200 | C₆₇H₁₁₇N₃O₉S₅ | 1268.9 8 | 7.82 | 18.53 | 3400 | C₁₀₆H₂₁₀N₄O₅S₇ | 1845.29 | 8.50 | 47.42 |
| 3401 | C₈₉H₁₇₄NO₇PS₄ | 1529.5 8 | 8.39 | 38.72 | 3413 | C₈₈H₁₇₄N₂O₃S₇ | 1532.79 | 8.91 | 42.54 |
| 3402 | C₉₀H₁₇₁N₃O₇S₃ | 1503.55 | 7.71 | 37.84 | 3414 | C₉₁H₁₇₃N₅O₆S₃ | 1529.59 | 7.96 | 34.92 |
| 3403 | C₈₉H₁₇₄N₂O₈S₅ | 1560.6 8 | 9.46 | 37.71 | 3415 | C₈₇H₁₇₂N₄O₅S | 1386.42 | 8.88 | 34.57 |
| 3404 | C₉₂H₁₇₇NO₆S₈ | 1649.9 1 | 9.21 | 43.98 | 3416 | C₇₁H₁₃₅N₅O₉S₃ | 1299.07 | 9.96 | 27.23 |
| 3405 | C₉₁H₁₇₇NO₈S₇ | 1637.8 4 | 8.69 | 40.23 | 3417 | C₇₈H₁₄₉N₄O₁₁P | 1350.04 | 9.88 | 28.11 |
| 3406 | C₈₈H₁₇₁NO₇S₇ | 1579.7 6 | 9.60 | 39.68 | 3418 | C₇₁H₁₃₁NO₃S₉ | 1335.37 | 9.68 | 29.97 |
| 3407 | C₉₄H₁₉₃N₂O₁₁P₃ S₄ | 1748.7 4 | 8.86 | 36.61 | 3419 | C₇₅H₁₃₇N₃O₅S₄ | 1289.18 | 9.96 | 29.92 |
| 3408 | C₉₄H₁₉₁N₂O₁₁P₃ S₂ | 1682.6 1 | 9.39 | 36.62 | 3420 | C₇₁H₁₃₂N₆O₁₀S₆ | 1422.23 | 7.13 | 23.90 |
| 3409 | C₉₇H₁₉₆N₃O₁₅P₃ S | 1769.6 2 | 9.74 | 34.98 | 3421 | C₆₉H₁₃₃N₅O₈S₅ | 1321.15 | 8.74 | 23.45 |
| 3410 | C₈₈H₁₃₀N₄O₇S₅ | 1566.7 3 | 9.79 | 37.89 | 3422 | C₇₀H₁₃₉N₄O₁₄P S₄ | 1420.11 | 7.55 | 24.35 |
| 3411 | C₈₉H₁₈₂NO₁₅PS ₄ | 1665.6 4 | 9.36 | 36.96 | | | | | |
| 3412 | C₈₈H₁₇₆N₂O₁₄S₄ | 1614.6 2 | 7.11 | 32.69 | | | | | |

The aforementioned properties of the exemplary compounds of the first ionizable lipid in the present application indicate that they can be used as excellent surfactants, and are particularly suitable for preparing lipid nanoparticles for use as drug delivery carriers.

### (4) Preparation of lipid composition

| **Example** | **Formula Composition** | **Molar Percentage %** |
|---|---|---|
| 1 | Compound 6008/Compound 1008/DSPC/Chol/ALC-0159 | 15/25/15/42.5/2.5 |
| 2 | Compound 6007/Compound 1025/DSPC/Chol/ALC-0159 | 1/46.5/10/41.5/1 |
| 3 | Compound 6019/Compound 1012/DOPE/Chol/ALC-0159 | 2/25/15/56/2 |
| 4 | Compound 6008/Compound 1111/DSPC/Chol/ALC-0159 | 20/20/15/42/3 |
| 5 | Compound 6020/Compound 1113/DOPE/Chol/ALC-0159 | 5/30/20/44/1 |
| 6 | Compound 6004/Compound 1114/DOPE/Chol/ALC-0159 | 2.5/35/12.5/49/1 |
| 7 | Compound 6006/Compound 1008/DSPC/Chol/ALC-0159 | 5/45/10/39/1 |
| 8 | Compound 6001/Compound 1012/POPC/Chol/PEG-DMG | 5/20/15/59/1 |
| 9 | Compound 1011/Compound 6038/DOPE/Chol/PEG-DMG | 20/30/15/43.5/1.5 |
| 10 | Compound 6002/Compound 1009/POPE/Chol/ALC-0159 | 5/30/23/41.75/0.25 |
| 11 | Compound 6009/Compound 1059/DSPC/Chol/PEG-DSPE | 15/15/20/50/5 |
| 12 | Compound 6010/Compound 1118/DSPC/Chol/PEG-DSPE | 5/25/40/29.5/0.5 |
| 13 | Compound 6007/Compound 1120/DOPE/Chol/PEG-DSPE | 5/25/10/59/1 |
| 14 | Compound 6009/Compound 1015/DOPE/Chol/PEG-DSPE | 5/25/30/39/1 |
| 15 | Compound 6007/Compound 1011/DOPE/Chol/PEG-DSPE | 5/25/48/20/2 |
| 16 | Compound 1008/ALC-0315/DOPE/Chol/PEG-DSPE | 10/30/10/49/1 |
| 17 | Compound 6008/Compound 1005/DSPC/Chol/ALC-0159 | 5/40/15/39.9/0.1 |
| 18 | Compound 1113/DSPC/Chol/PEG-DSPE | 50/10/39.5/0.5 |
| 19 | Compound 1009/DSPC/Chol/ALC-0159 | 47.3/9.6/41.4/1.7 |
| 20 | Compound 1011/DOPE/Chol/ALC-0159 | 30/20/49/1 |
| 21 | Compound 1008/DOPE/Chol/ALC-0159 | 40/18/41/1 |
| 22 | Compound 1115/DSPC/Chol/ALC-0159 | 45/12/41.5/1.5 |
| 23 | Compound 1120/ALC-0315/DSPC/Chol/ALC-0159 | 2/40/10/46/2 |
| 24 | Compound 6024/Compound 1011/ALC-0315/DSPC/Chol/PEG-DMG | 4/20/25/50/1 |

Lipid compositions were separately prepared according to the aforementioned molar ratios, using the following specific method:

The lipid raw materials were dissolved in ethanol to form a lipid-ethanol solution, and the total concentration of all lipids in ethanol was 8 mg/mL. The lipid-ethanol solution was mixed with a 50 mM citrate buffer (pH 4.0) at a volume ratio of 1:3 in nanoparticle preparation equipment. The mixture was then subjected to ultrafiltration, and the sample was collected to obtain the lipid composition, which had a final lipid concentration of 2 mg/mL.

### (5) Determination of particle size and Zeta potential of nanoparticles

1. Particle size and polydispersity index (PDI) determination: The average particle size and PDI of the nanoparticle sample solutions from the examples were measured using a Malvern ZetaSizer Nano ZS90 via dynamic light scattering. The measurement angle was 90°, the dispersant refractive index was 1.330, and the test temperature was 25°C.
2. Zeta potential: The Zeta potential of the nanoparticle sample solutions from the examples was measured using a Malvern ZetaSizer Nano ZS90 based on electrophoretic light scattering (ELS) technology; the dispersant refractive index was 1.330, and the test temperature was 25°C.

Data regarding the average particle size, PDI, and Zeta potential of the lipid compositions prepared using the exemplary compounds of each first ionizable lipid and second ionizable lipid are presented in Table 2.

| **Example** | **Particle Size nm** | **PDI** | **Potential mV** |
|---|---|---|---|
| 1 | 65.85 | 0.111 | 16.3 |
| 2 | 70.71 | 0.085 | 13.2 |
| 3 | 62.21 | 0.057 | 12.6 |
| 4 | 64.55 | 0.135 | 18.1 |
| 5 | 71.07 | 0.034 | -9.67 |
| 6 | 62.32 | 0.055 | -9.30 |
| 7 | 92.25 | 0.146 | 14.0 |
| 8 | 72.94 | 0.099 | -7.56 |
| 9 | 71.07 | 0.034 | 9.67 |
| 10 | 88.10 | 0.053 | 11.4 |
| 11 | 40.21 | 0.089 | 10.8 |
| 12 | 51.46 | 0.036 | -13.4 |
| 13 | 52.75 | 0.065 | 18.2 |
| 14 | 49.35 | 0.102 | 13.8 |
| 15 | 46.89 | 0.104 | 15.6 |
| 16 | 54.05 | 0.027 | 11.7 |
| 17 | 177.9 | 0.089 | 18.0 |
| 18 | 69.25 | 0.162 | 10.8 |
| 19 | 54.56 | 0.164 | -4.12 |
| 20 | 103.9 | 0.09 | -13.6 |
| 21 | 134.9 | 0.099 | -10.8 |
| 22 | 63.46 | 0.131 | -12.5 |
| 23 | 81.31 | 0.052 | -2.35 |
| 24 | 90.24 | 0.115 | 12.8 |

Table 2 Data summary of particle size, PDI, and Zeta potential for the lipid compositions

As shown in Table 2, the lipid compositions provided in the present application had small and uniform particle sizes and exhibited a neutral or weakly charged property.

### (6) Stability Study

The lipid compositions prepared in Examples 1, 6, and 23 were placed in a constant temperature and constant humidity chamber at 25°C and 60% RH for one month to evaluate their stability (particle size, PDI, and Zeta potential); the results are shown in Table 3.

**Table 3 Summary of stability data for lipid compositions**

| **Example** | **Storage Time** | **Particle Size nm** | **PDI** | **Potential mV** |
|---|---|---|---|---|
| 1 | 0 | 65.85 | 0.111 | 16.3 |
| | One Month | 70.61 | 0.102 | 15.4 |
| 6 | 0 | 62.32 | 0.055 | -9.30 |
| | One Month | 66.11 | 0.057 | -8.51 |
| 23 | 0 | 81.31 | 0.052 | -2.35 |
| | One Month | 79.56 | 0.089 | -5.64 |

As shown in Table 3, the lipid nanoparticles provided in the present application exhibited no significant changes in particle size, PDI, or Zeta potential after storage for one month at 25°C and 60% RH, demonstrating good stability.

### (7) Evaluation of the encapsulation effect of the lipid composition for nucleic acid substances

A nucleic acid was used as an active ingredient, which was prepared to a 40 ng/µL solution (Solution 1) by using nuclease-free water; the lipid composition was diluted 2-fold with nuclease-free water to obtain Solution 2; and Solution 1 and Solution 2 were mixed in equal volumes and vortexed for 2-3 seconds to obtain the lipid composition-nucleic acid complex, followed by test of the encapsulation efficiency.

The encapsulation efficiency testing methods were as follows:

The encapsulation efficiency of mRNA in the lipid nanoparticles was determined using the Quant-it RiboGreen RNA Assay Kit (Thermo Fisher Scientific, UK) in accordance with the manufacturer's operating instructions.

The encapsulation efficiency of pDNA in the lipid nanoparticles was determined using the dsDNA HS Kit (Vazyme/EQ121) in accordance with the manufacturer's operating instructions.

The encapsulation efficiency of siRNA in the lipid nanoparticles was determined using the Qubit^{®} microRNA Reagent Kit in accordance with the manufacturer's operating instructions.

The test results are shown in Table 4.

**Table 4 Encapsulation efficiency of lipid composition-nucleic acid complexes**

| **Lipid Composition** | **Encapsulated Substance** | **Encapsulation efficiency %** |
|---|---|---|
| Example 1 | Luc-mRNA (Molecular weight: 634873.2) | 91.6 |
| Example 4 | Luc-mRNA (Molecular weight: 634873.2) | 85.4 |
| Example 6 | Luc-pDNA (Molecular weight: 4464547.9) | 96.5 |
| Example 8 | CY3-siRNA (Molecular weight: 12958) | 82.6 |
| Example 10 | Luc-pDNA (Molecular weight: 4464547.9) | 98.8 |
| Example 13 | CY3-siRNA (Molecular weight: 12958) | 86.7 |
| Example 15 | CY3-siRNA (Molecular weight: 12958) | 90.5 |
| Example 16 | GAPDH-siRNA (Molecular weight: 13300) | 94.2 |
| Example 17 | eGFP-pDNA (Molecular weight: 3766037.9) | 97.5 |
| Example 20 | eGFP-mRNA (Molecular weight: 315851.5) | 90.4 |
| Example 22 | eGFP-pDNA (Molecular weight: 3766037.9) | 89.6 |
| Example 23 | eGFP-mRNA (Molecular weight: 315851.5) | 99.7 |

### (8) Evaluation of the encapsulation effect of lipid compositions for small molecular substances

Using a small molecular substance as the active ingredient, a solution at a concentration of 300 ng/µL was prepared using nuclease-free water (Solution 1). The lipid compositions prepared in Examples 2, 4-5, 8, 11, and 23 of section (4) were used as the solution 2. The solution 1 and solution 2 were mixed in equal volumes and vortexed for 2-3 seconds to obtain the lipid composition-small molecular substance complex, and the encapsulation efficiency was tested.

### Encapsulation efficiency test method:

Ultrafiltration centrifugation was performed on the LNPs containing the encapsulated components using ultrafiltration centrifuge tubes (Amicon Ultra-4 Centrifugal Filters, molecular weight cut-off: 30 kDa). The amount of free drug in the centrifuge tube and the amount of encapsulated drug retained on the filter membrane were determined by using high-performance liquid chromatography (HPLC). The formula for calculating encapsulation efficiency is: Encapsulation Efficiency (EE%) = amount of retained drug / (amount of retained drug + amount of free drug) × 100%. The test results are shown in Table 5.

**Table 5 Encapsulation efficiency of lipid composition-small molecular substance complexes**

| **Lipid Composition** | **Encapsulated Substance** | **Encapsulation efficiency %** |
|---|---|---|
| Example 2 | Dexamethasone sodium metasulfobenzoate | 85.4 |
| Example 4 | Sodium chondroitin sulfate | 82.6 |
| Example 5 | Sodium hyaluronate (Molecular weight 23800) | 90.5 |
| Example 8 | Sodium cromoglycate | 89.6 |
| Example 11 | Diclofenac sodium | 94.6 |
| Example 23 | Betamethasone sodium phosphate | 97.6 |

### (9) Evaluation of the encapsulation effect of the lipid composition for macromolecular substances

Using a macromolecular substance as the active ingredient, a solution at a concentration of 200 ng/µL was prepared using nuclease-free water (Solution 1). The lipid compositions prepared in Examples 3, 5, 7, 14, and 22 of section (4) were used as the solution 2. The solution 1 and solution 2 were mixed in equal volumes and vortexed for 2-3 seconds to obtain the lipid composition-macromolecular substance complex, and the encapsulation efficiency was tested.

Encapsulation efficiency testing method: The nanoparticles were added to an ultrafiltration tube with a molecular weight cut-off (MWCO) of 100×10³ kDa. Free drug was separated via centrifugation. The amount of free drug in the centrifuge tube and the amount of encapsulated drug retained on the filter membrane were determined by RP-HPLC, and the encapsulation efficiency was calculated. The formula for calculating encapsulation efficiency is: Encapsulation Efficiency (EE%) = Amount of retained drug / (Amount of retained drug + Amount of free drug) × 100%.

The test results are shown in Table 6.

**Table 6 Encapsulation efficiency of lipid composition-macromolecular substance complexes**

| **Lipid Composition** | **Encapsulated Substance** | **Encapsulation efficiency %** |
|---|---|---|
| Example 3 | Recombinant human insulin | 90.5 |
| Example 5 | Interferon | 81.5 |
| Example 7 | Human growth hormone | 86.5 |
| Example 14 | Insulin analogues | 95.6 |
| Example 22 | Staphylokinase | 87.6 |

The above studies demonstrated that the lipid composition provided in the present application exhibited excellent encapsulation effect for nucleic acids, small molecular or macromolecular substances, and the encapsulation efficiency is higher than 80%. The lipid composition can be used as delivery carrier for active ingredients.

The scope of the present application is not limited to the specific technical means disclosed above but also encompasses technical solutions formed by any combination of the aforementioned technical characteristics. While the foregoing describes specific embodiments of the present application, it should be noted that persons of ordinary skill in the art may make various improvements and modifications without departing from the principles of the application; such improvements and modifications are also considered to fall within the scope of protection of the present application.

## Claims

1. A lipid composition, **characterized by** comprising the following components: an ionizable lipid and an auxiliary lipid, wherein the lipid composition does not contain a component having a preventive or therapeutic effect; the ionizable lipid comprises a first ionizable lipid, and the first ionizable lipid is selected from a compound of formula (1), or a salt, a stereoisomer, or a tautomer thereof: 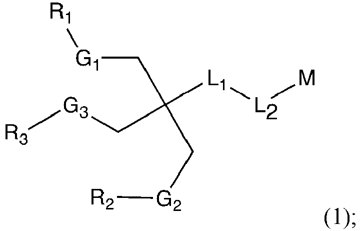
wherein R₁, R₂, and R₃ are each independently H, a C₅₋₄₀ linear or branched alkyl , a C₅₋₄₀ linear or branched alkenyl, a C₅₋₄₀ linear or branched alkynyl, a 3- to 6-membered saturated or partially unsaturated cyclic hydrocarbon group containing 1-3 side chains, or a 6- to 10-membered aromatic group containing 1-3 side chains; the side chains are independently selected from C₁₀₋₃₀ linear or branched alkyls, C₁₀₋₃₀ linear or branched alkenyls, and C₁₀₋₃₀ linear or branched alkynyls; with the proviso that at most one of R₁, R₂, and R₃ is H;
M is selected from -NR₄R₅, a saturated or partially unsaturated 3- to 6-membered heterocyclyl containing at least one nitrogen atom, and a 6- to 10-membered heteroaryl containing at least one nitrogen atom, wherein the heterocyclyl or heteroaryl is unsubstituted or substituted by one or more -OH, carboxyl, amino, oxo, or halogen groups;
R₄ and R₅ are each independently H, a C₁₋₆ linear or branched alkyl, a C₂₋₆ linear or branched alkenyl, or a C₂₋₆ linear or branched alkynyl, wherein the C₁₋₆ linear or branched alkyl, C₂₋₆ linear or branched alkenyl, or C₂₋₆ linear or branched alkynyl is unsubstituted or substituted by one or more -OH, carboxyl, aminoamide group, amidino, guanidino, or halogen groups;
G₁, G₂, and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)- or -OP(=O)(OR₆)O-; wherein R₆ and R₁₃ are each independently selected from H, hydroxyl, a C₁₋₃₀ linear or branched alkyl or cycloalkyl, and a C₂₋₃₀ linear or branched alkenyl;
L₁ is selected from -X₁- or -(CR₇R₈)ₘ-X₁-, wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)- or -OP(=O)(OR₁₄)O-; wherein m is an integer of 2-6; R₇ and R₈ are each independently selected from H, hydroxyl, halogen, a C₁₋₆ linear or branched alkyl or cycloalkyl, and a C₂₋₆ linear or branched alkenyl; and R₁₄ and R₁₅ are each independently selected from H, a C₁₋₃₀ linear or branched alkyl or cycloalkyl, and a C₂₋₃₀ linear or branched alkenyl;
L₂ is -(CR₉R₁₀)ₙ- or -(CR₉R₁₀)ₙ-X₂-(CR₁₁R₁₂)ₖ-, wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, -NR₁₆-S(=O)₂-, -P(=O)(OR₁₆)O-, -OP(=O)(OR₁₆)-, or -OP(=O)(OR₁₆)O-; n is an integer from 1 to 6; k is an integer from 1 to 6; R₉, R₁₀, R₁₁, and R₁₂ are each independently H, hydroxyl, halogen, a C₁₋₆ linear or branched alkyl or cycloalkyl, or a C₂₋₆ linear or branched alkenyl; and R₁₆ and R₁₇ are each independently selected from H, a C₁₋₃₀ linear or branched alkyl or cycloalkyl, and a C₂₋₃₀ linear or branched alkenyl;
wherein the alkyl, cycloalkyl, and alkenyl mentioned in R₄ to R₁₇ are unsubstituted or substituted by one or more groups selected from hydroxyl, mercapto, amino, substituted amino, and halogen; and
the salt is not a quaternary ammonium salt.

2. The lipid composition according to claim 1, wherein the R₁, R₂, and R₃ are each independently selected from the following groups: 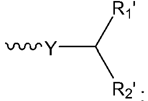 wherein Y is absent, or is a C₁₋₃₀ linear or branched alkyl or cycloalkyl, a C₂₋₂₀ linear or branched alkenyl, or a C₂₋₂₀ linear or branched alkynyl; R₁' and R₂' are each independently H, a C₁₋₃₀ linear or branched alkyl, a C₂₋₃₀ linear or branched alkenyl, or a C₂₋₃₀ linear or branched alkynyl; and the total carbon chain length of Y, R₁', and R₂' is 8-40.

3. The lipid composition according to claim 1, wherein R₁, R₂, and R₃ are each independently selected from the following groups: 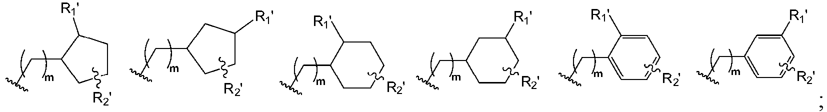 wherein R₁' and R₂' are each independently selected from H, a C₁₋₃₀ linear or branched alkyl, a C₂₋₃₀ linear or branched alkenyl, or a C₂₋₃₀ linear or branched alkynyl, and the total carbon chain length of R₁' and R₂' is 8-30.

4. The lipid composition according to claim 1, wherein R₁, R₂, and R₃ are each independently selected from any one of the following groups:

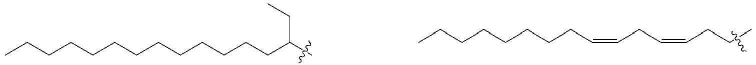

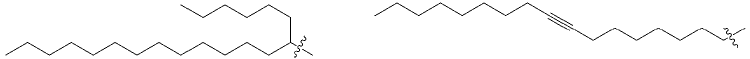

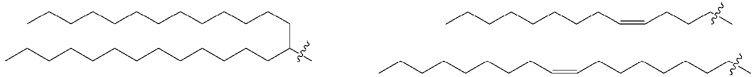

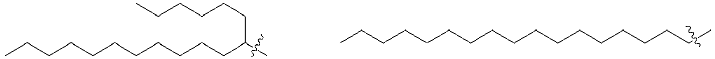

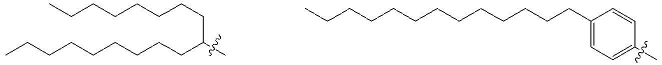

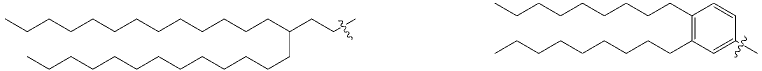

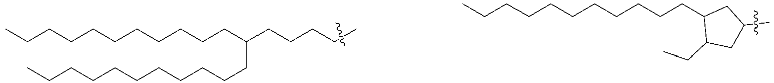

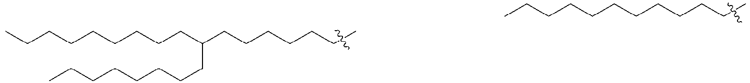

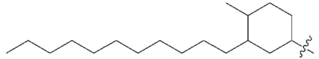

5. The lipid composition according to claim 1, wherein G₁, G₂, and G₃ are each independently selected from -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)-, or -OP(=O)(OR₆)O-.

6. The lipid composition according to claim 1, wherein L₁ is selected from -(CR₇R₈)ₘ-X₁-, and X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)-, or -OP(=O)(OR₁₄)O-.

7. The lipid composition according to claim 1, wherein L₂ is -(CR₉R₁₀)ₙ-X₂-(CR₁₁R₁₂)ₖ-, and X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)-, or -OP(=O)(OR₁₆)O-.

8. The lipid composition according to claim 1, wherein M is selected from the following structures: 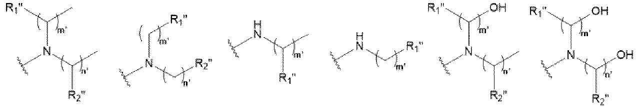 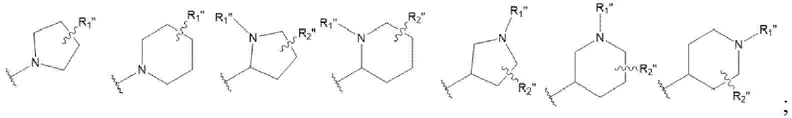 wherein m' and n' are each independently an integer from 0 to 6; R₁" and R₂" are each independently selected from H, a C₁₋₆ alkyl, a C₂₋₆ alkenyl, guanidino, amidino, amido, aliphatic amino, or a 3- to 10-membered nitrogen-containing heterocycle; the nitrogen-containing heterocycle is selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine, and pyrimidine, and tautomeric forms thereof, which are unsubstituted or optionally substituted with one or more organic groups selected from hydroxy, mercapto, amino, substituted amino, halogen, a C₁₋₆ alkyl, a C₂₋₆ alkenyl, a C₂₋₆ alkynyl, and a C₆₋₁₄ aryl.

9. The lipid composition according to claim 1, wherein the compound of formula (1) is selected from a compound represented by formula (1A):

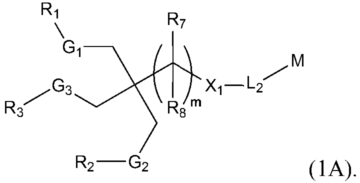

10. The lipid composition according to claim 1, wherein the compound of formula (1) is selected from a compound represented by formula (1B):

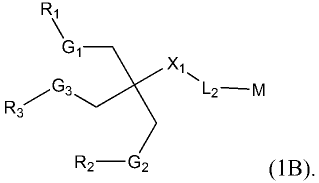

11. The lipid composition according to claim 1, wherein the compound of formula (1) is selected from a compound represented by formula (1C):

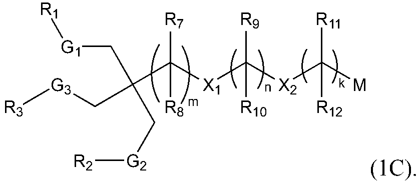

12. The lipid composition according to claim 10, wherein the compound of formula (1) is selected from a compound represented by formula (1D):

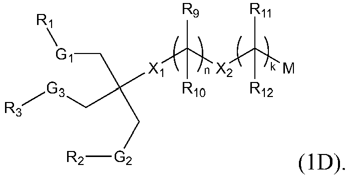

13. The lipid composition according to claim 12, wherein the compound of formula (1) is selected from a compound represented by formula (1E):

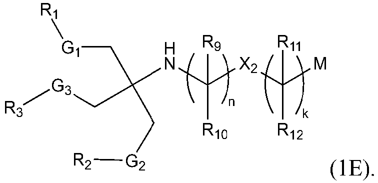

14. The lipid composition according to claim 13, wherein the compound of formula (1) is selected from a compound represented by formula (1F):

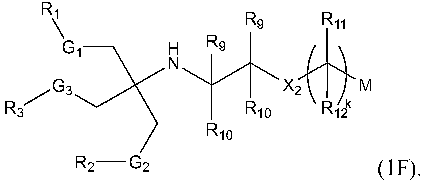

15. The lipid composition according to claim 14, wherein the compound of formula (1) is selected from a compound represented by formula (1G):

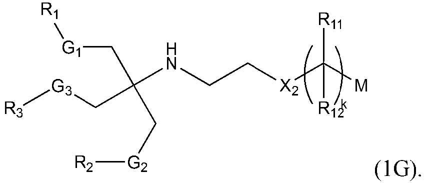

16. The lipid composition according to claim 14, wherein the compound of formula (1) is selected from a compound represented by formula (1H):

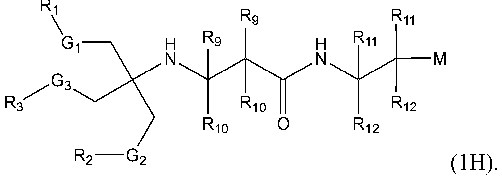

17. The lipid composition according to claim 1, wherein the compound of formula (1) is selected from a compound represented by formula (1I):

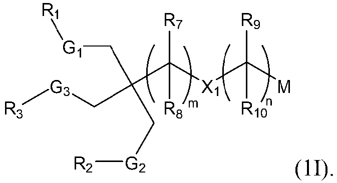

18. The lipid composition according to claim 10, wherein the compound of formula (1) is selected from a compound represented by formula (1J):

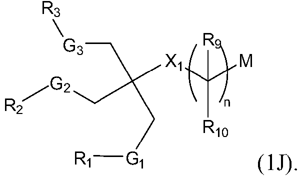

19. The lipid composition according to claim 17, wherein the compound of formula (1) is selected from a compound represented by formula (1K):

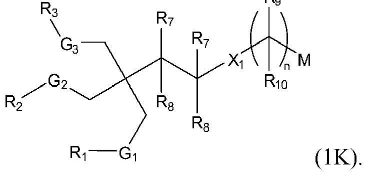

20. The lipid composition according to claim 2, wherein, Y is absent, and the compound of formula (1) is selected from a compound represented by formula (1L): 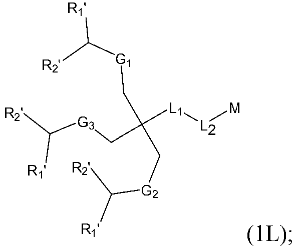 wherein and R2' are each independently selected from H, a C1-30 linear or branched alkyl, a C₂₋₃₀ linear or branched alkenyl, and a C₂₋₃₀ linear or branched alkynyl, and the total carbon chain length of R1' and R2' is 8-40.

21. The lipid composition according to claim 2, wherein the compound of formula (1) is selected from a compound represented by formula (1M):

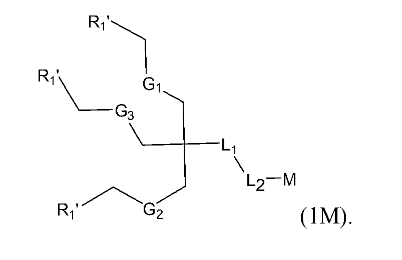

22. The lipid composition according to claim 1, wherein the compound of formula (1) is selected from:

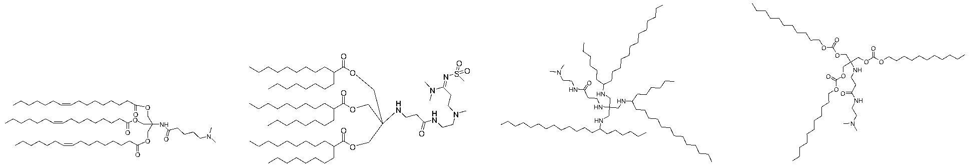

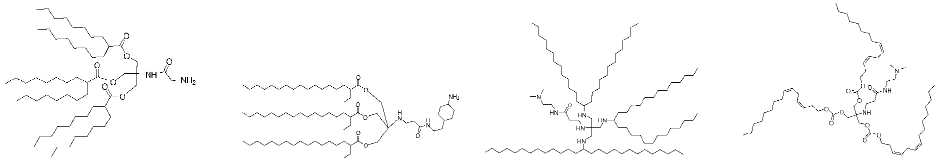

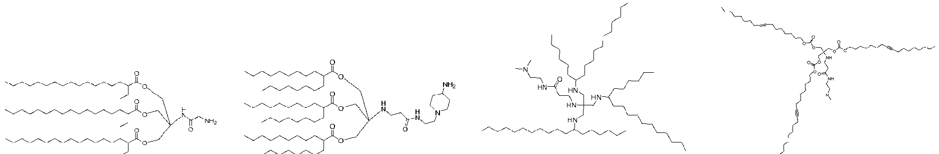

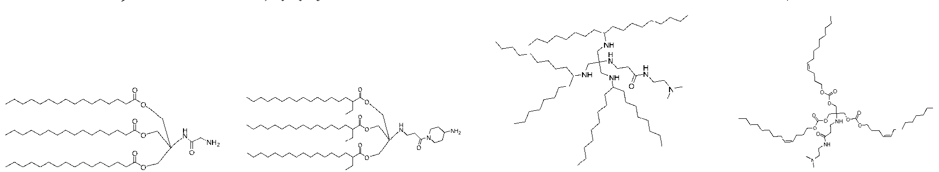

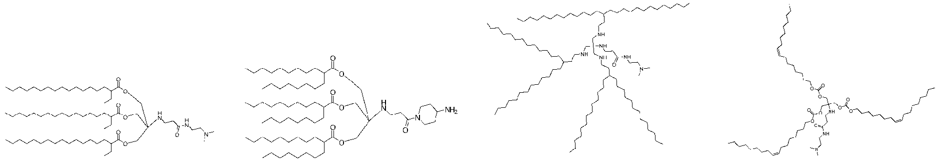

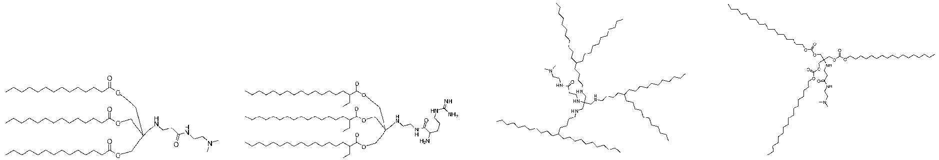

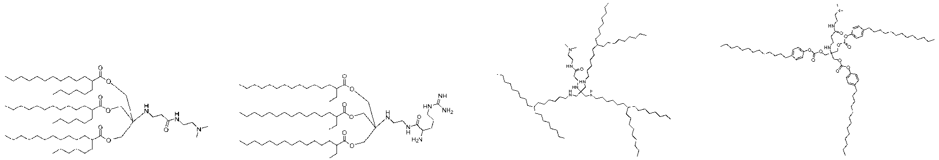

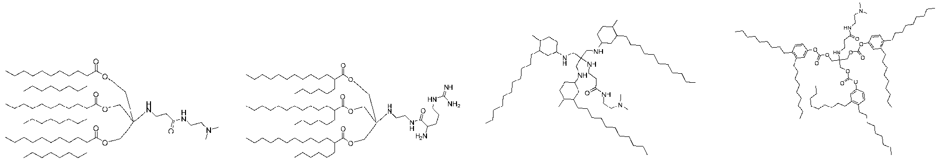

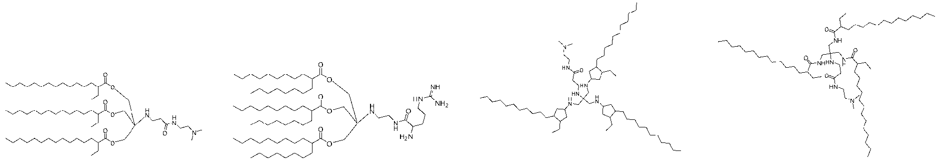

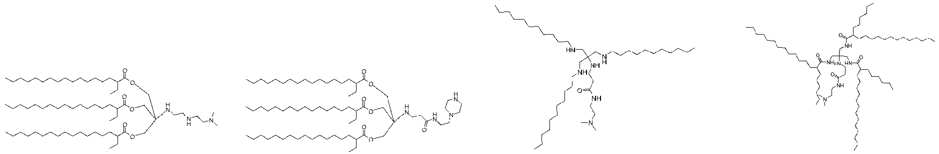

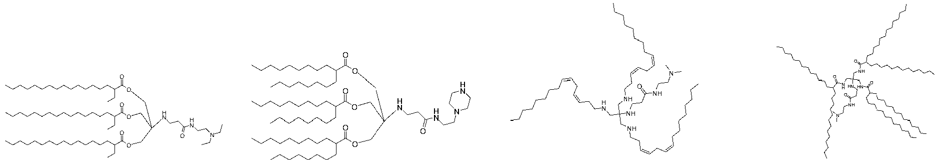

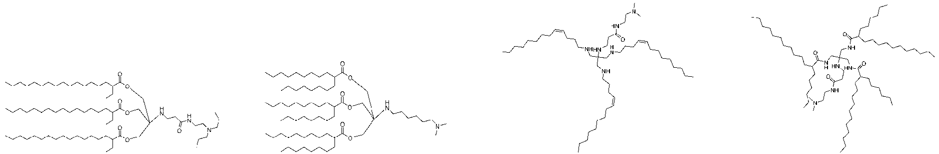

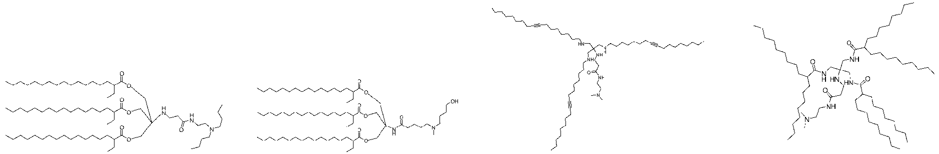

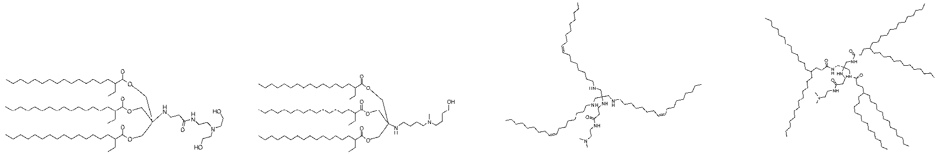

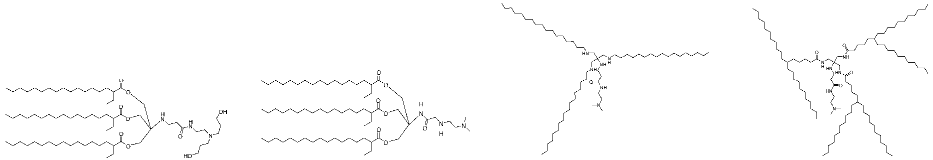

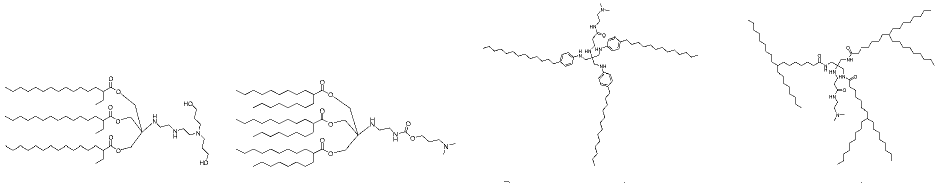

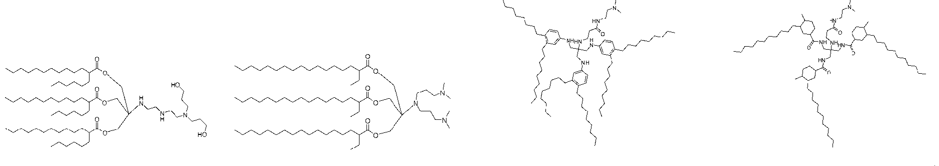

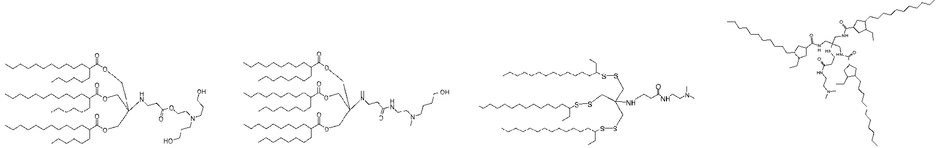

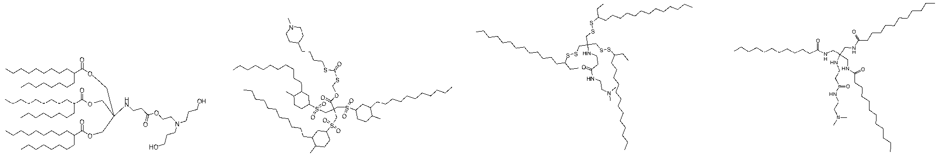

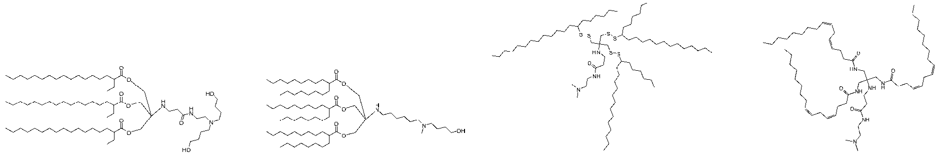

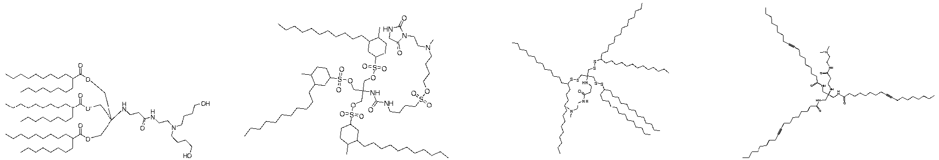

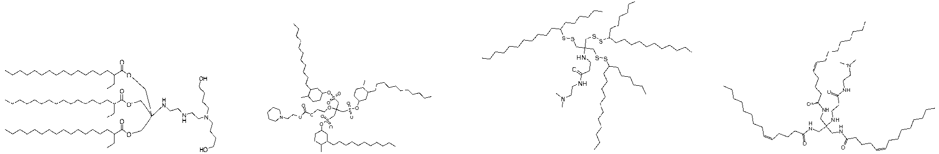

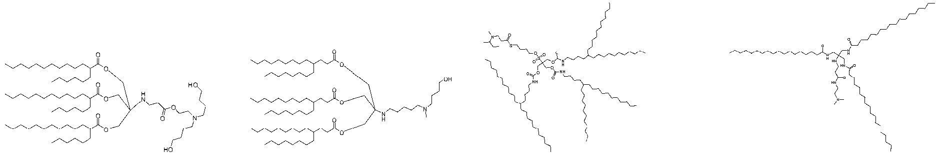

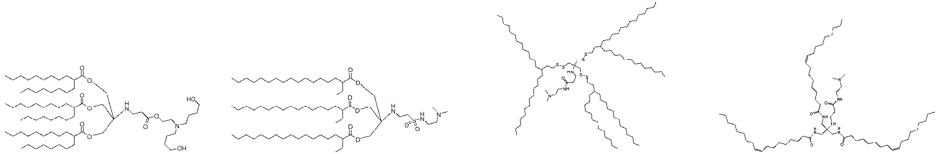

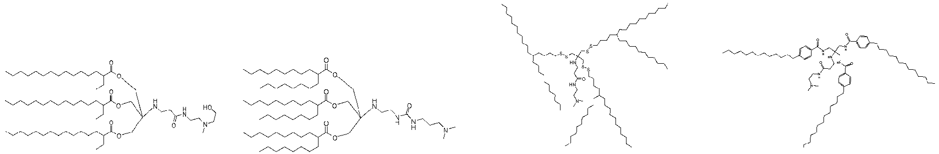

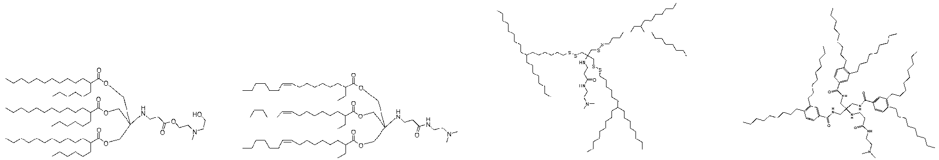

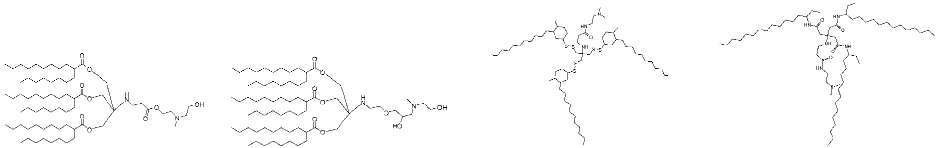

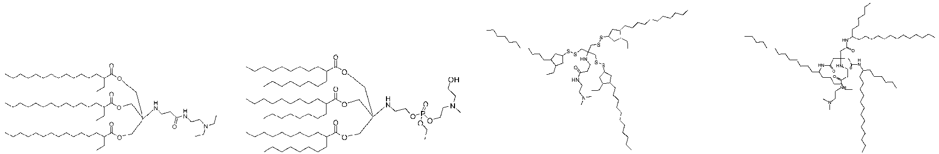

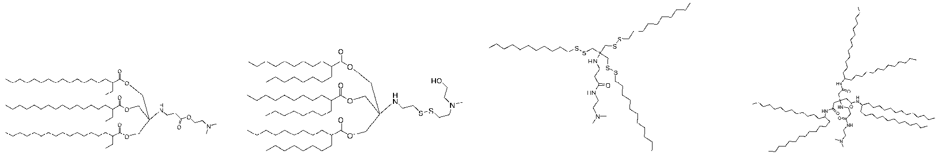

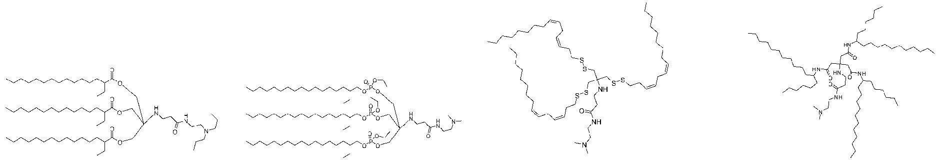

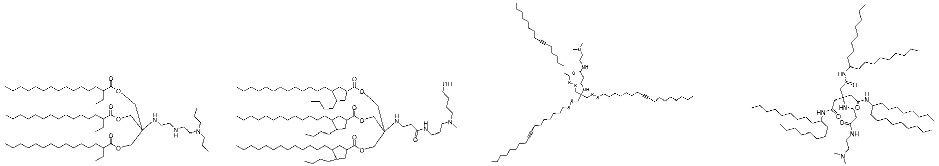

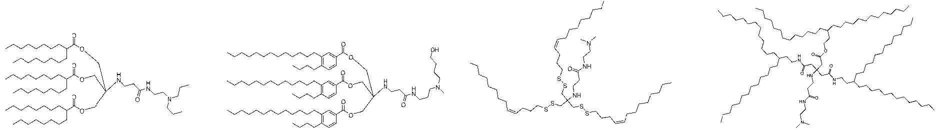

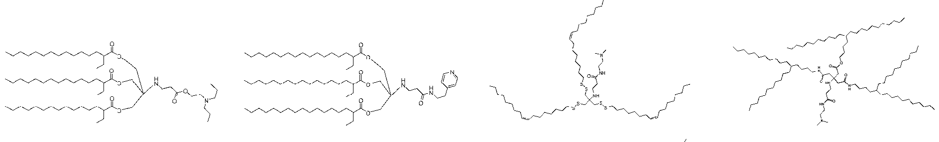

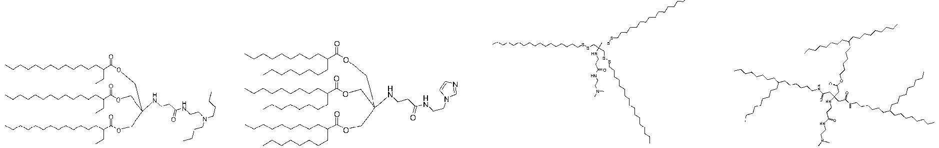

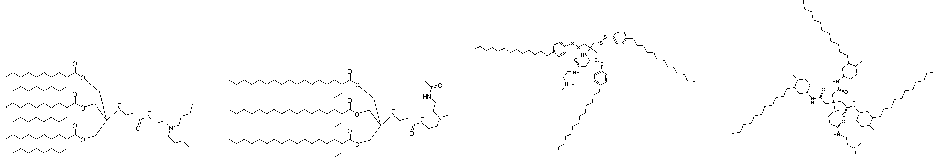

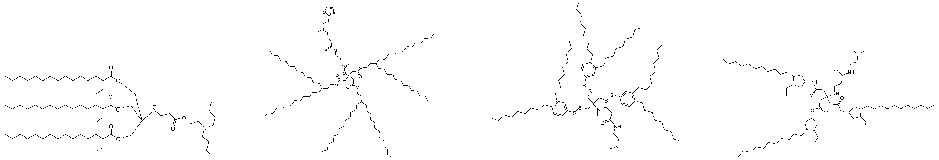

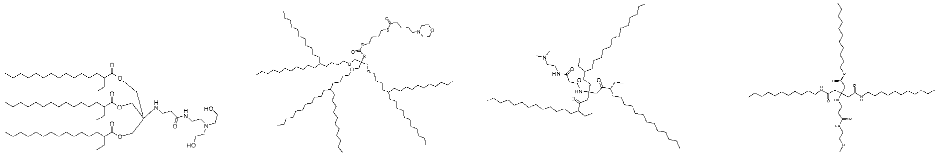

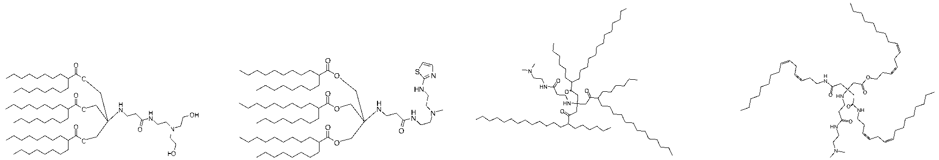

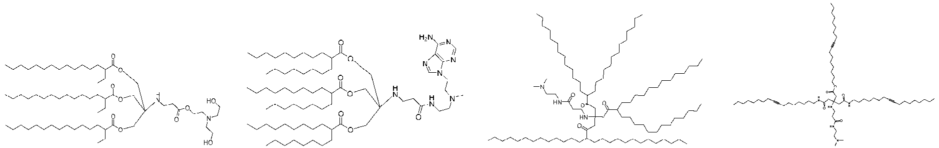

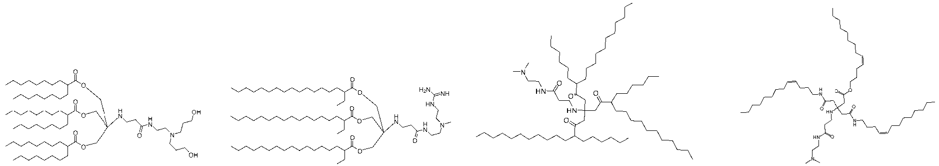

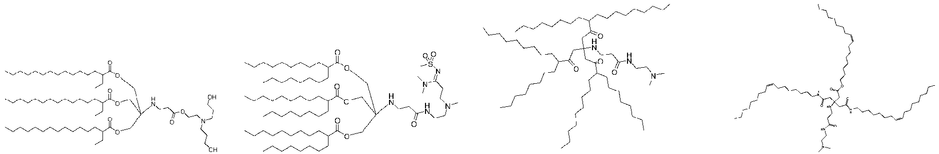

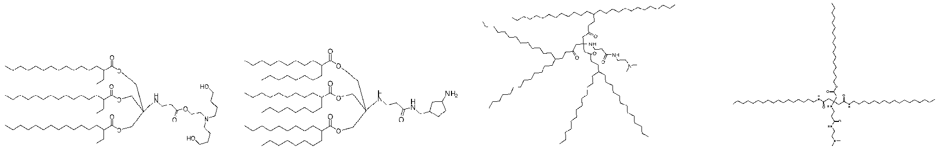

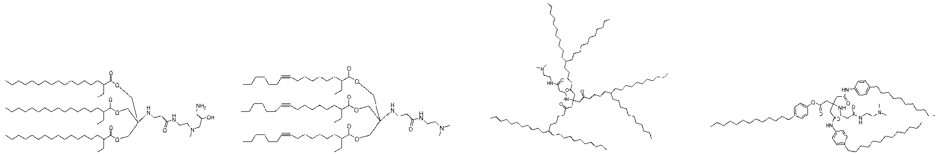

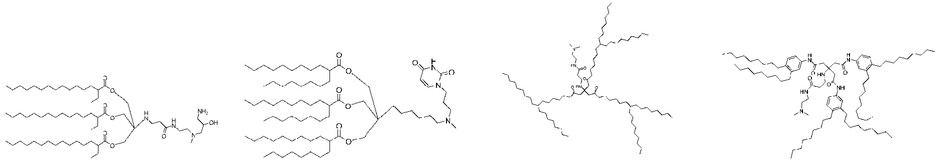

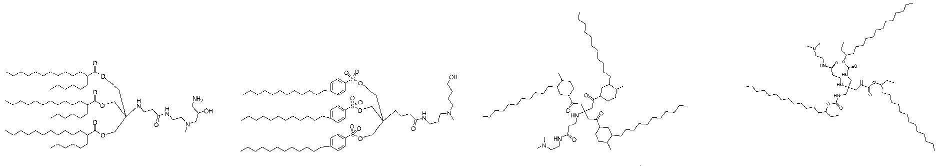

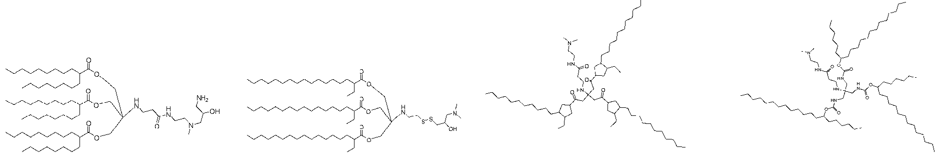

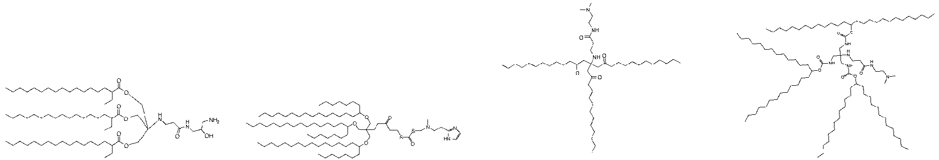

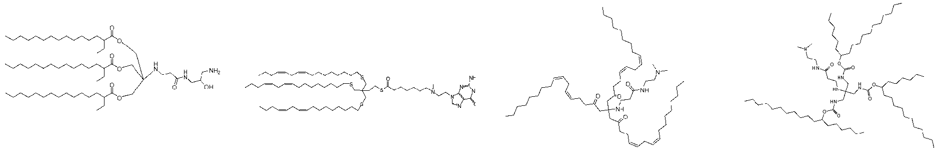

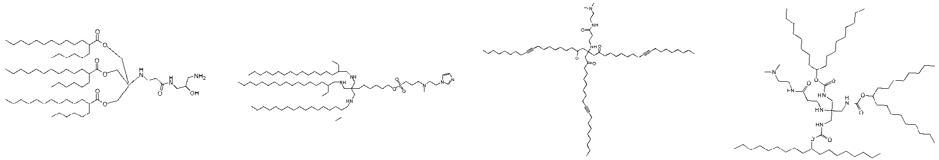

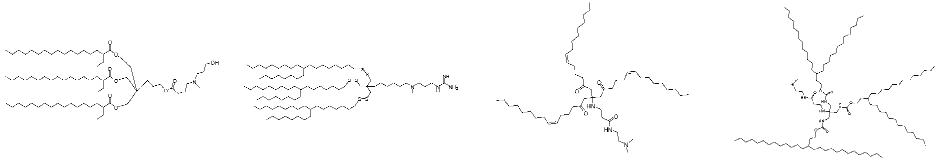

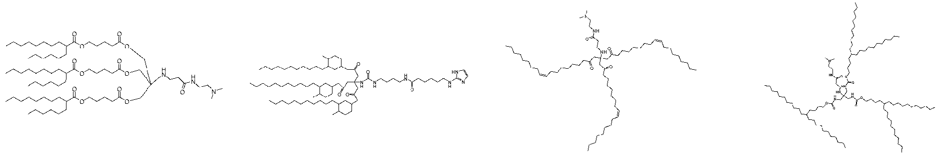

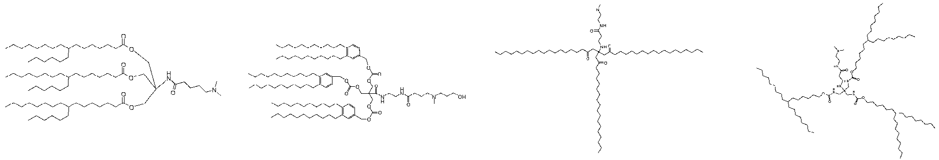

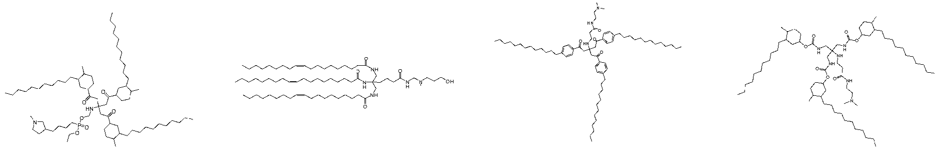

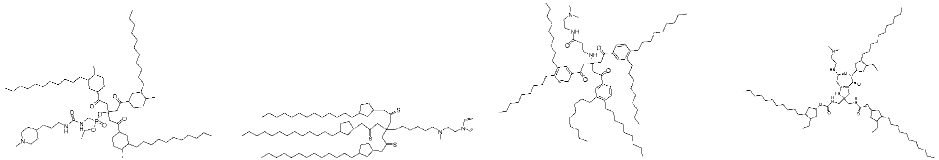

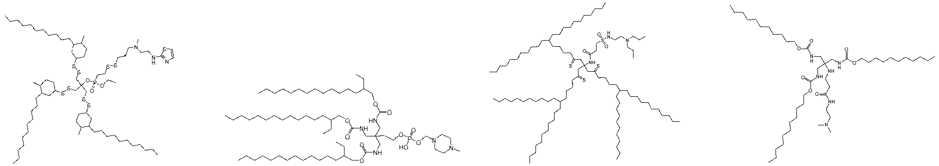

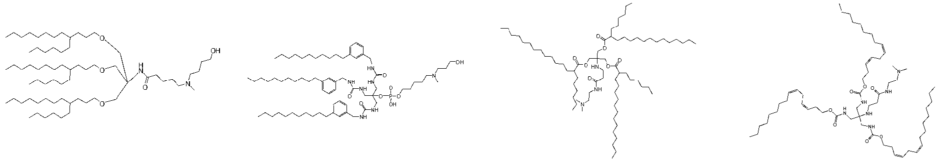

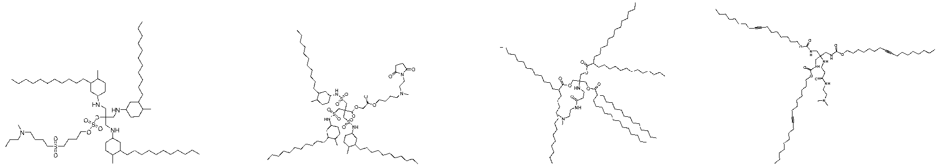

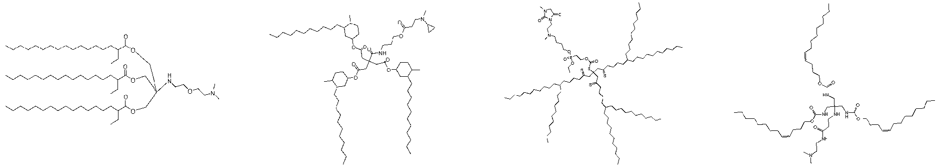

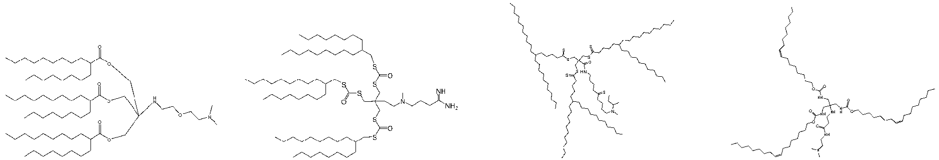

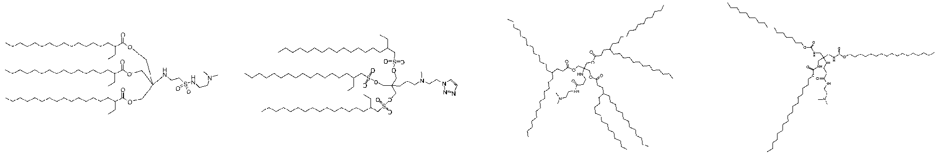

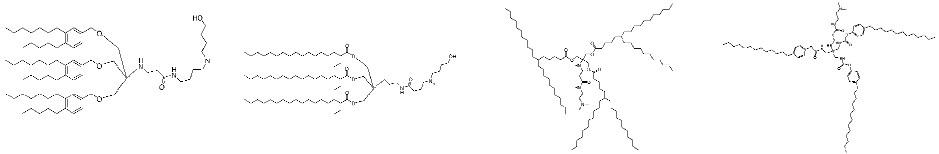

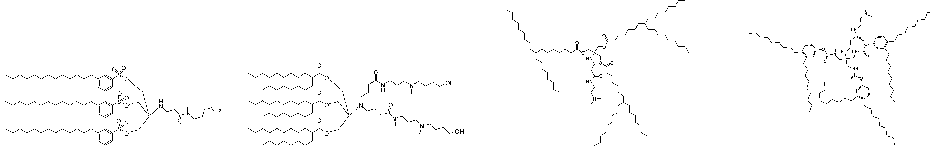

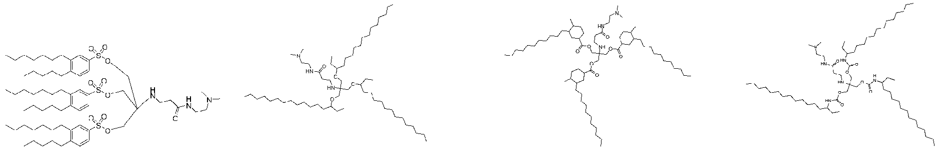

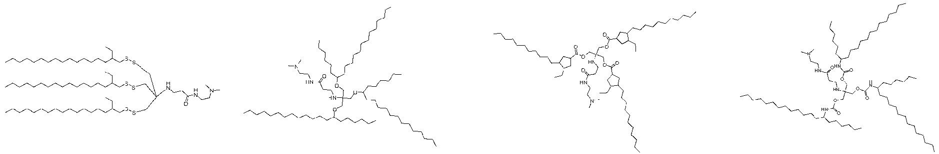

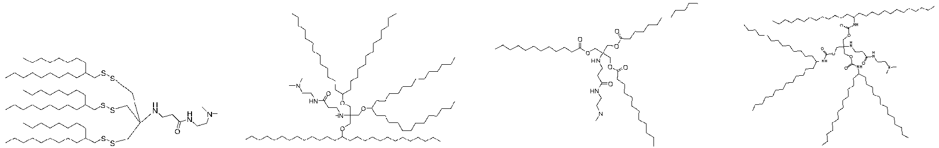

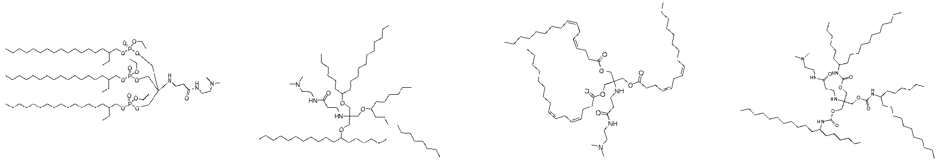

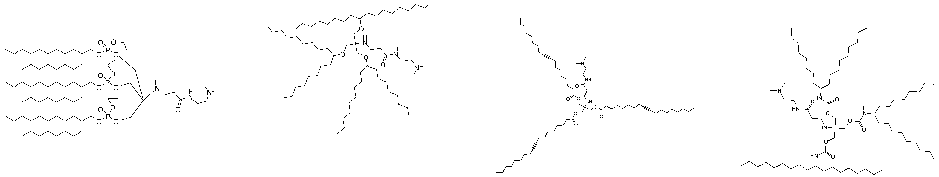

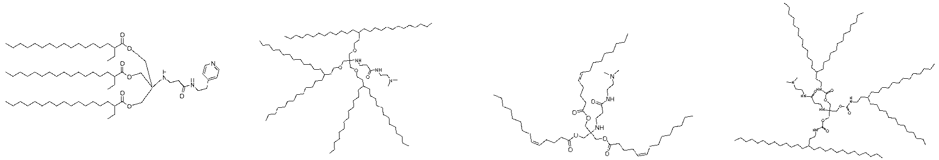

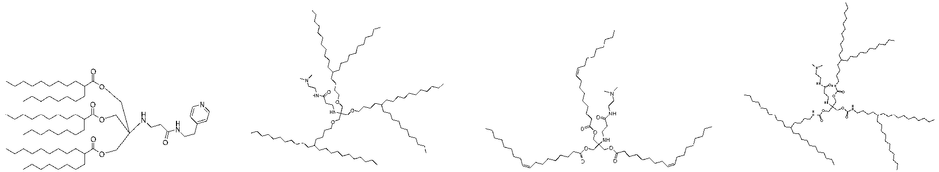

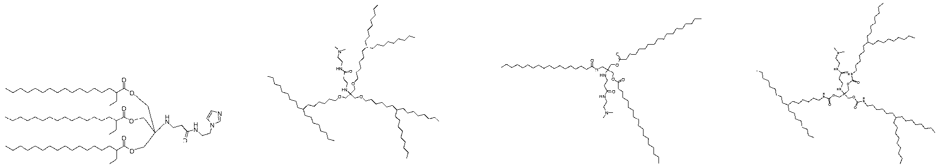

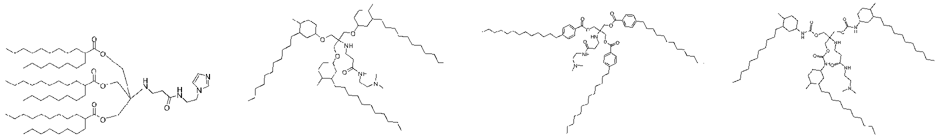

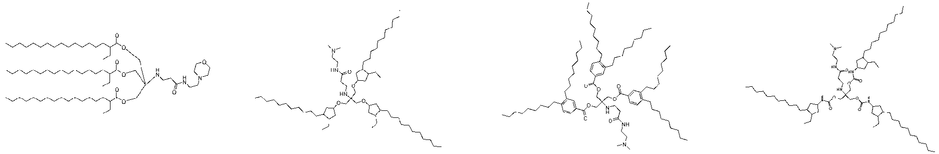

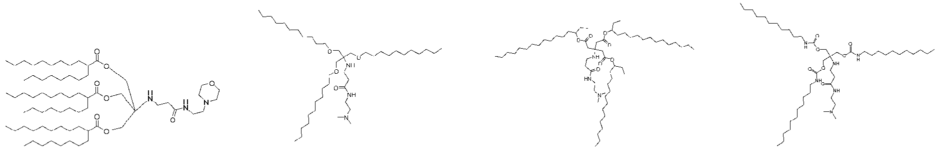

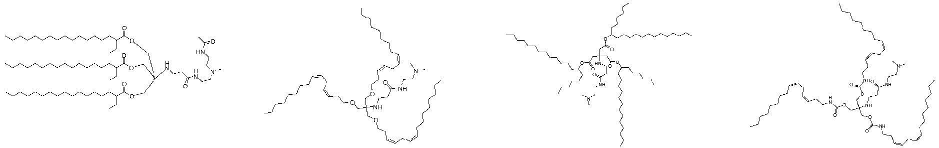

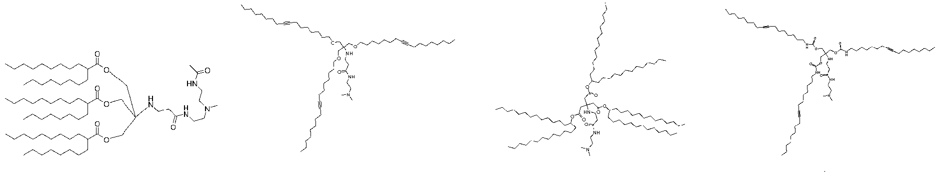

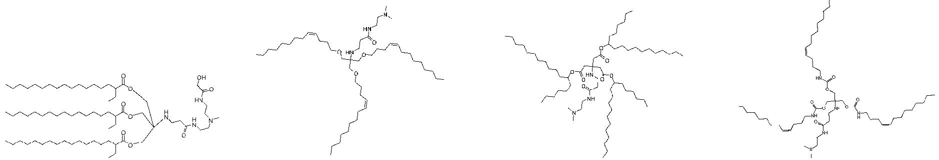

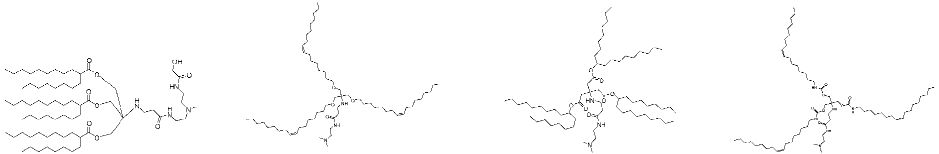

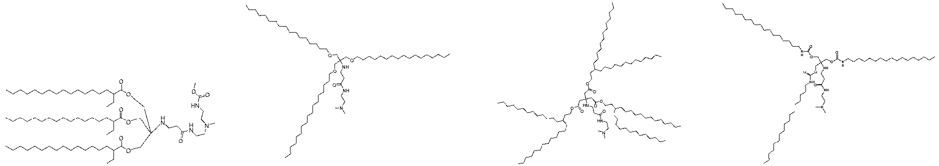

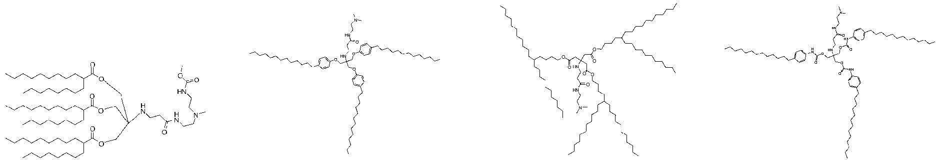

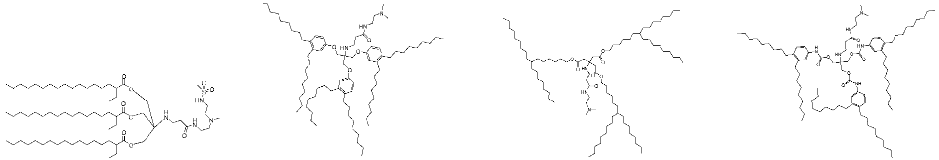

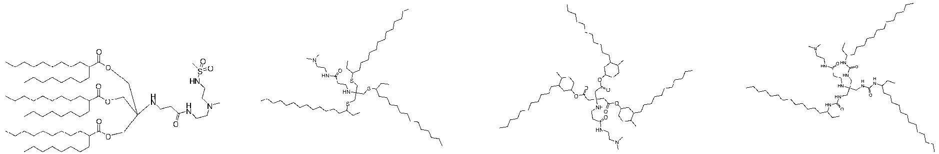

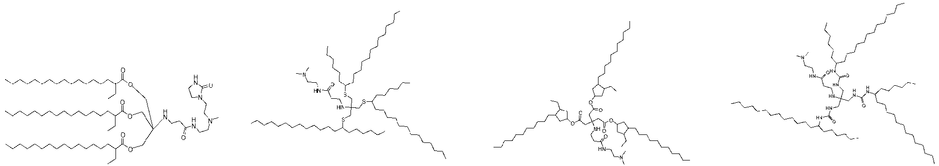

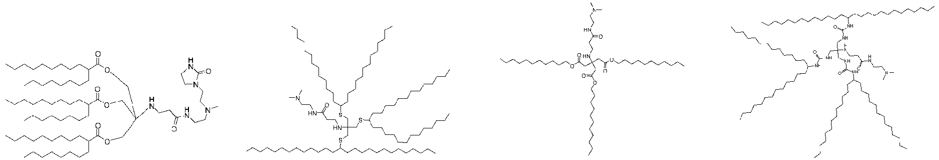

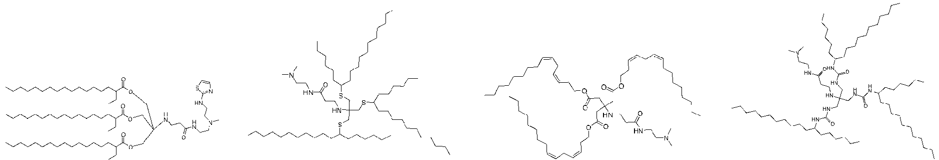

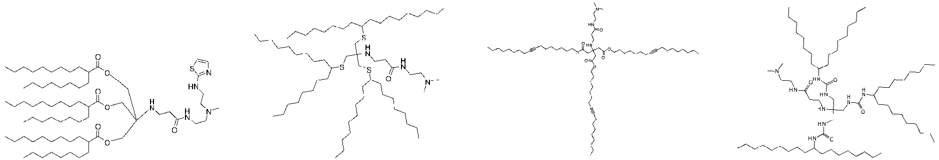

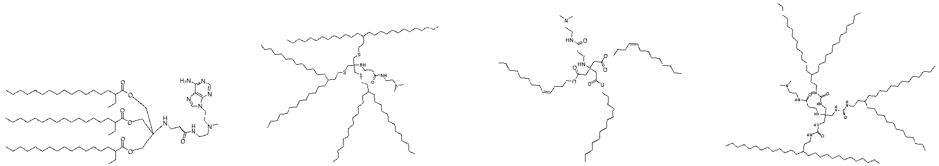

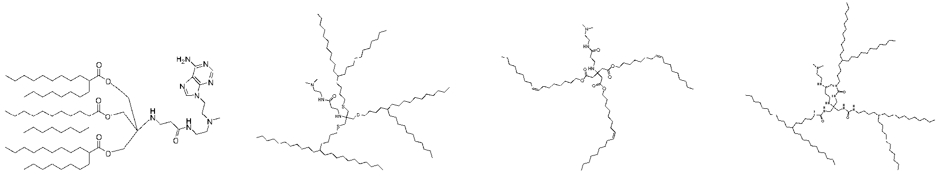

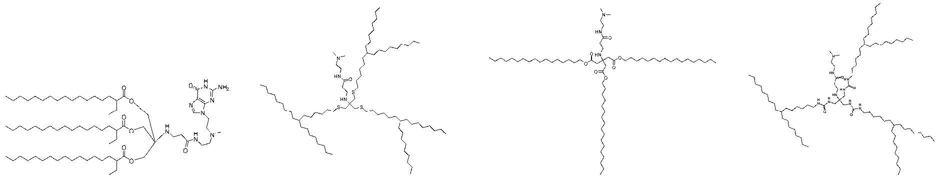

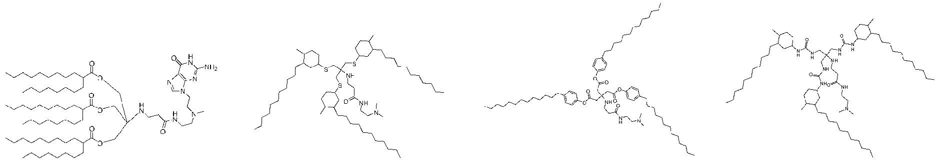

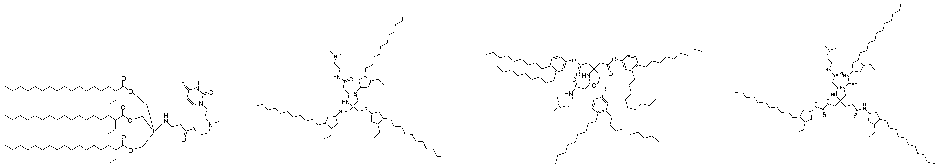

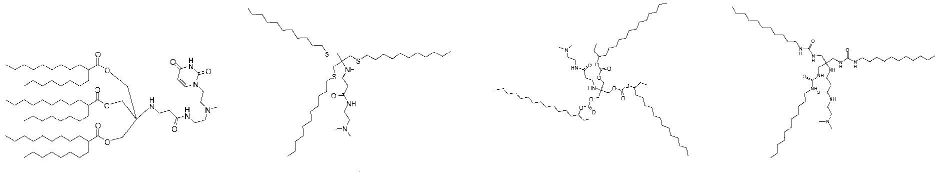

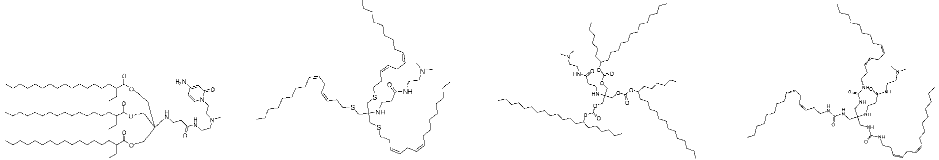

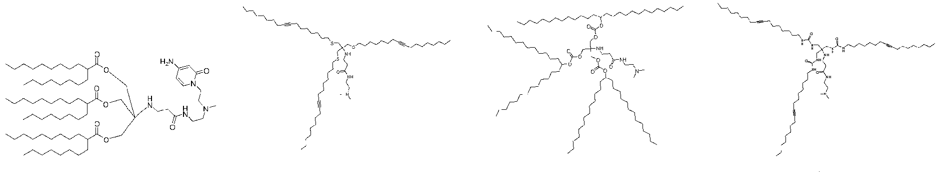

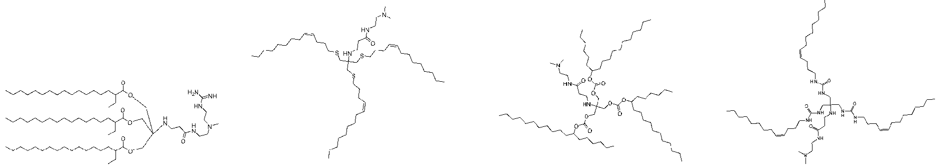

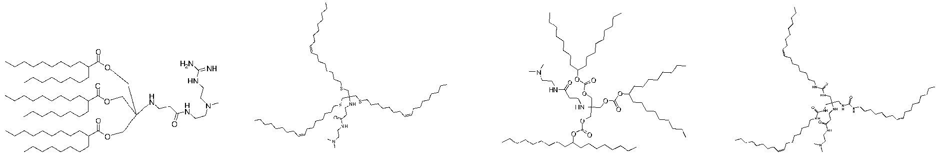

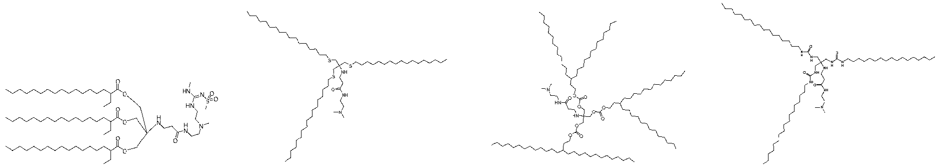

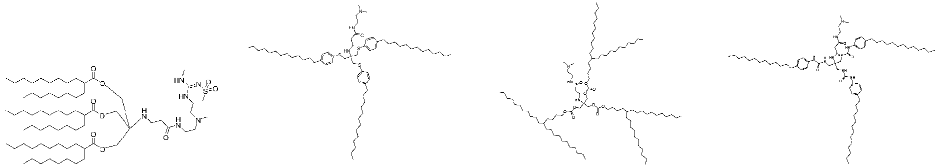

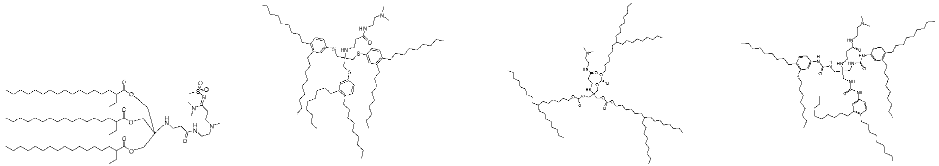

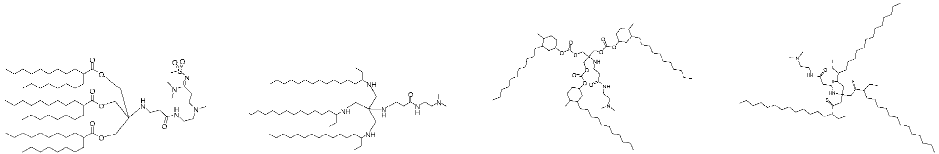

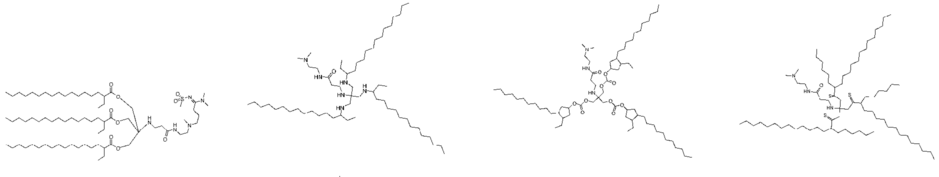

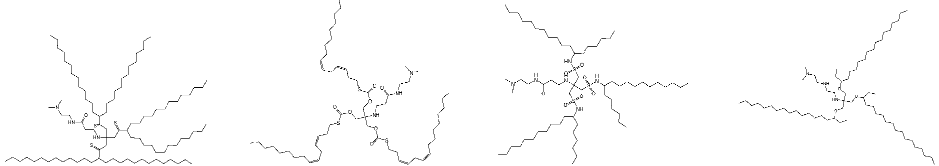

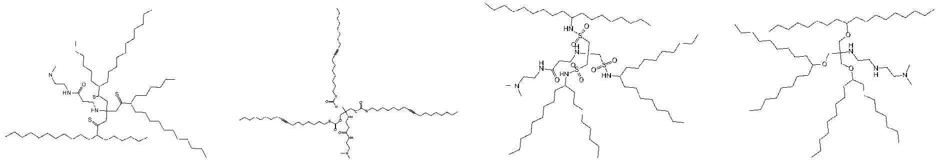

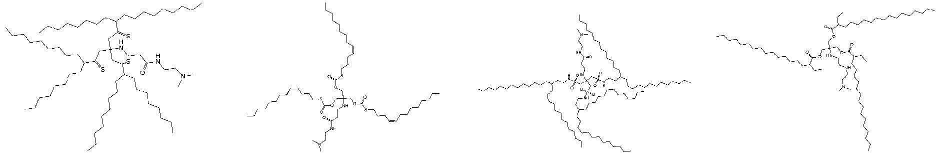

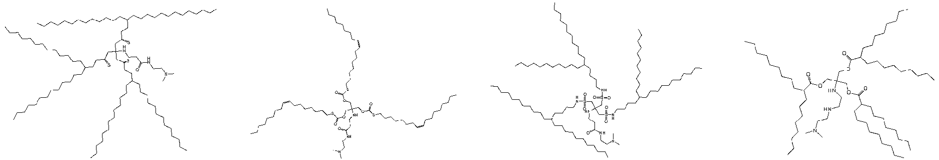

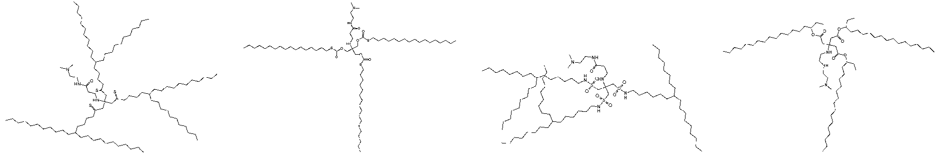

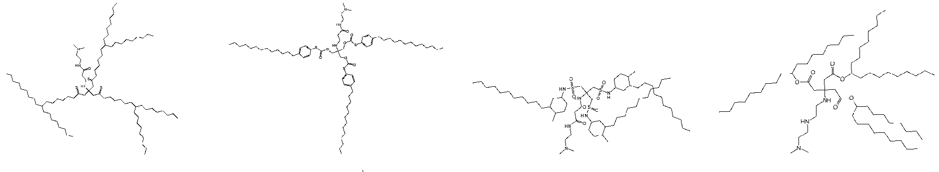

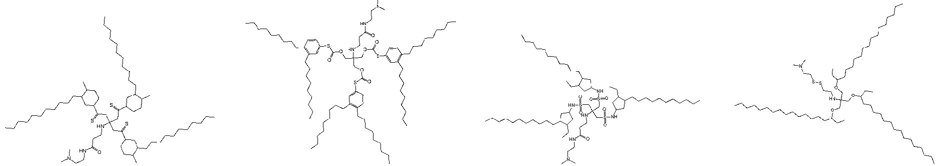

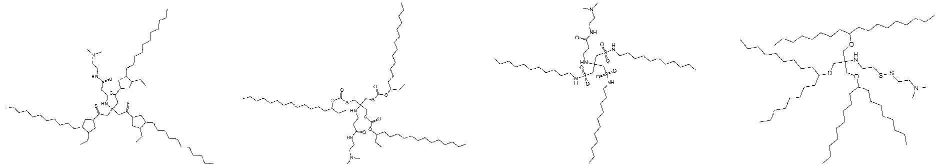

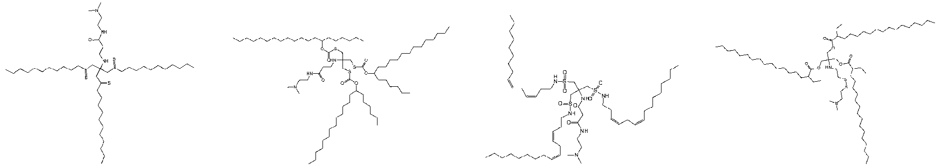

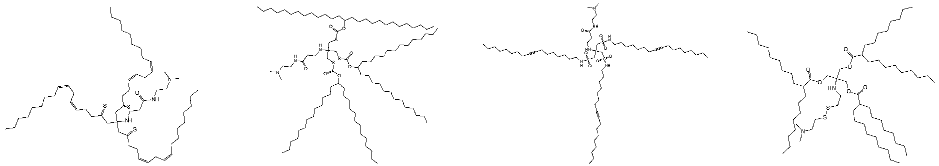

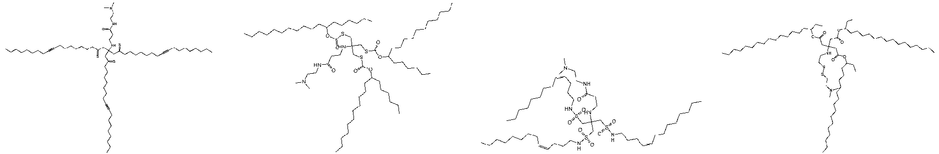

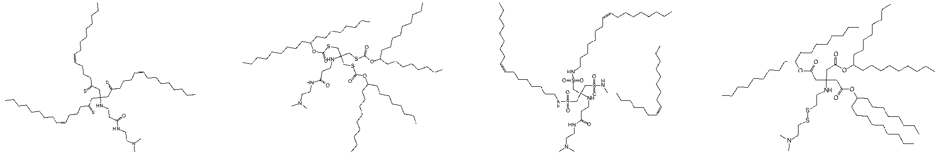

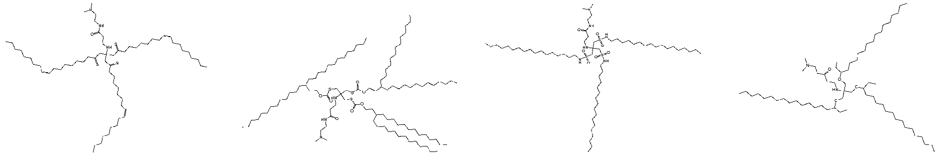

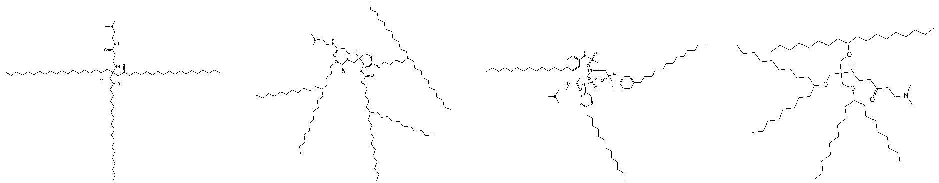

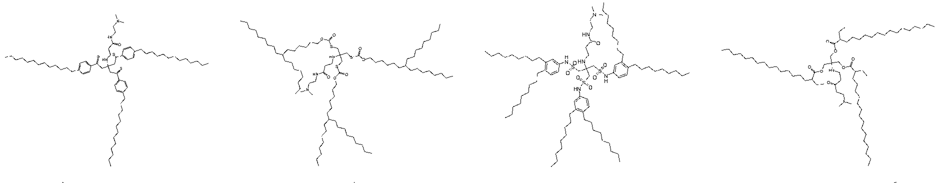

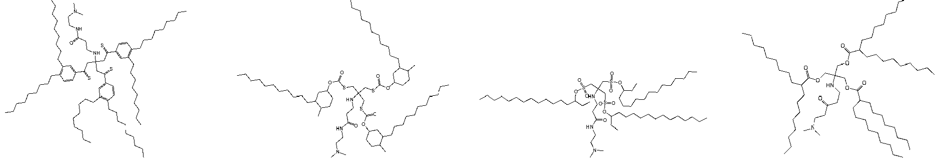

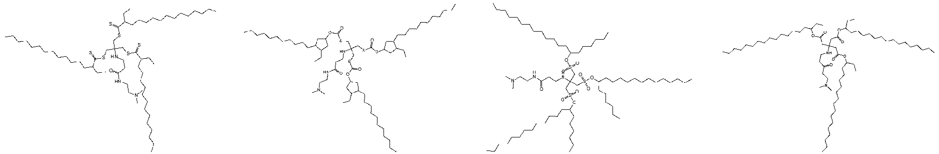

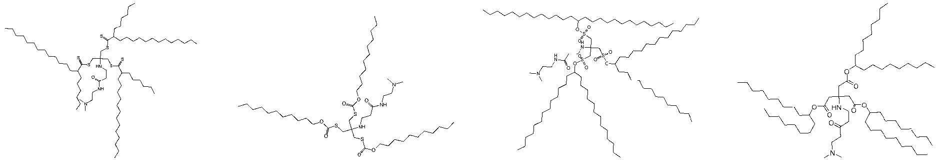

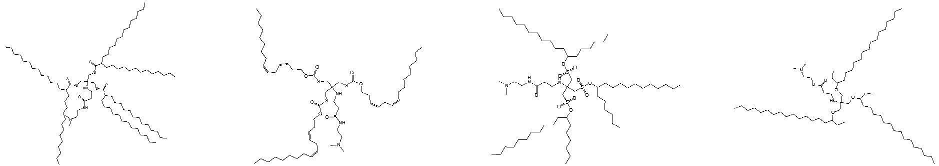

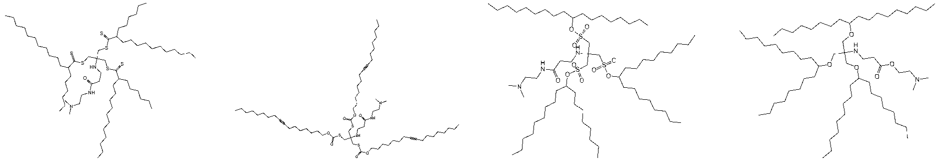

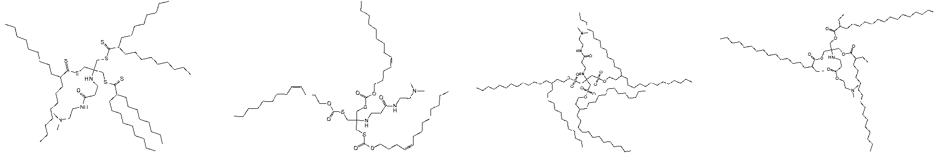

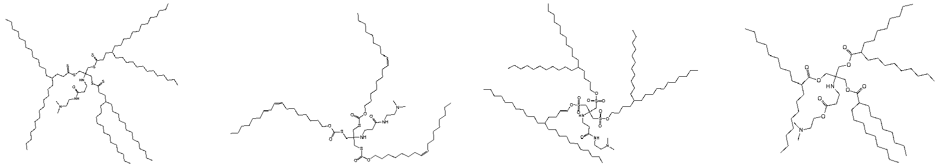

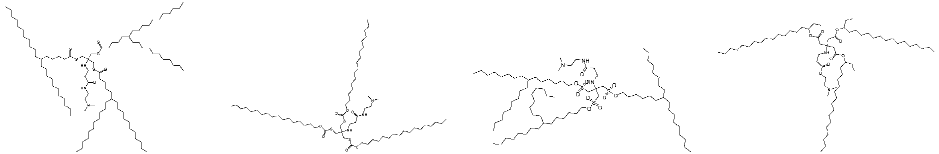

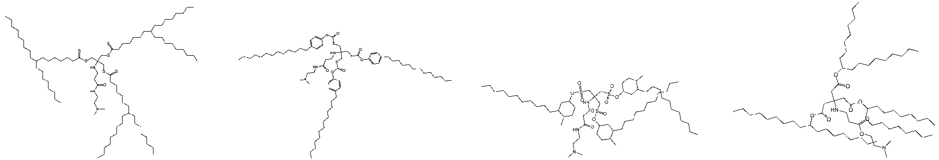

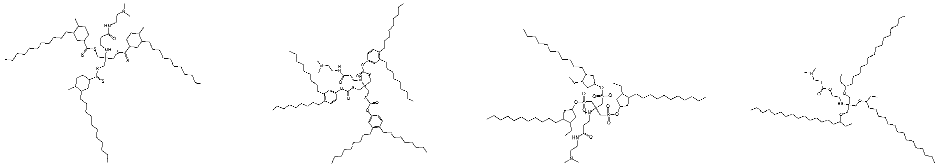

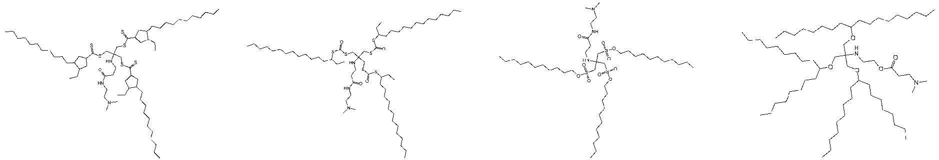

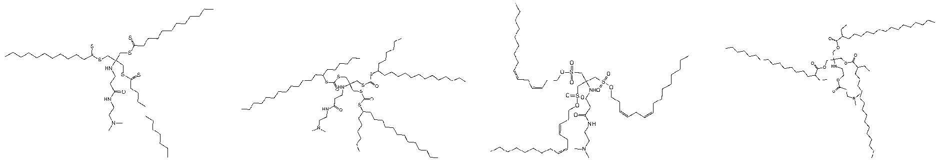

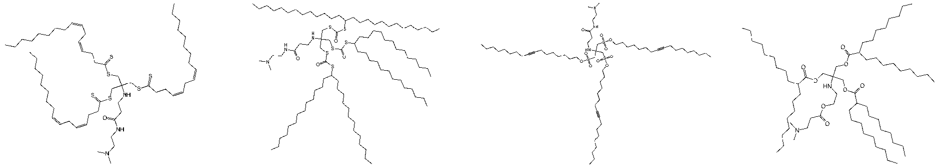

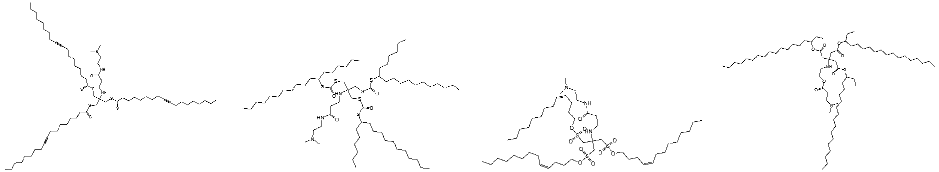

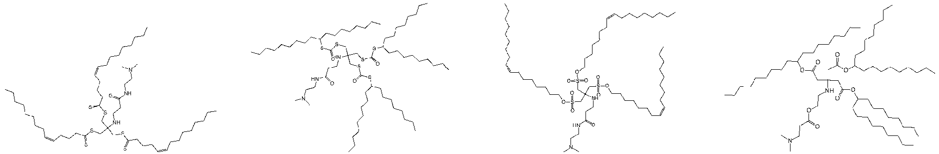

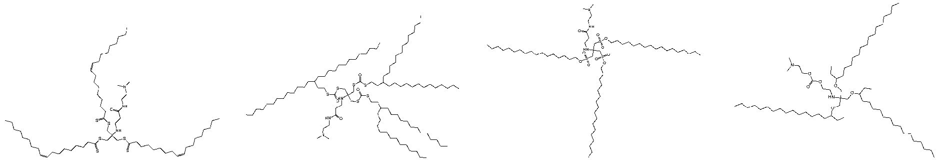

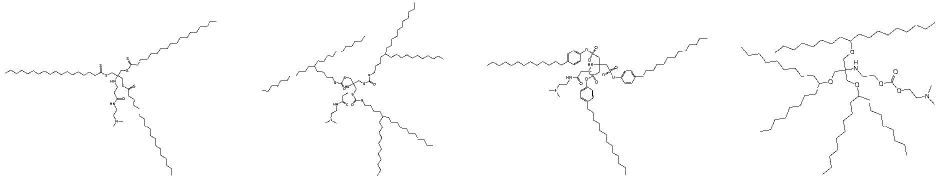

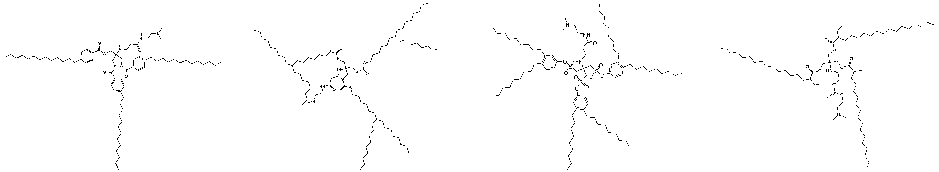

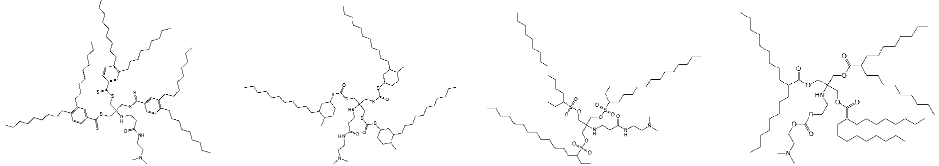

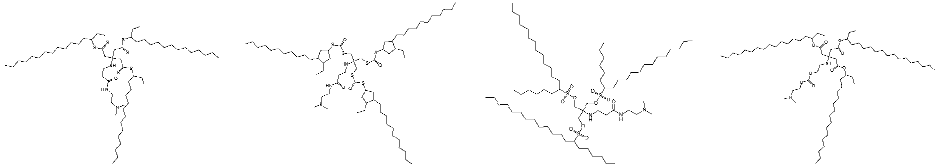

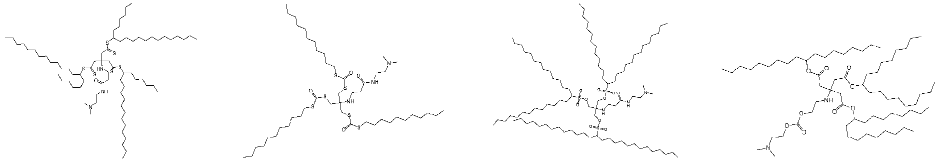

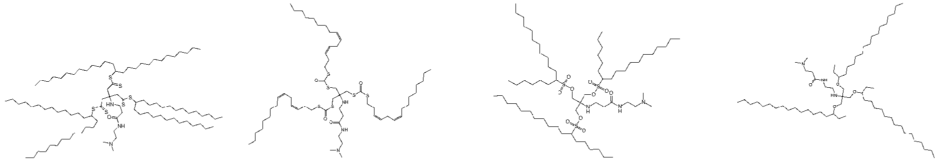

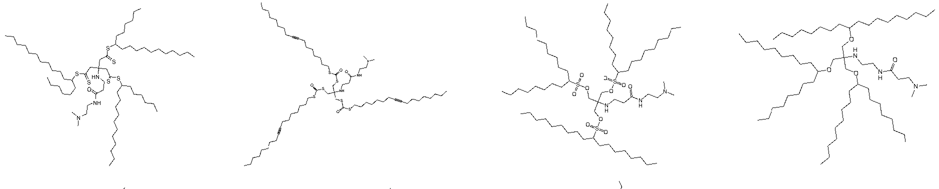

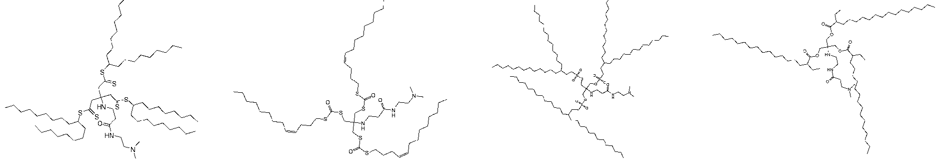

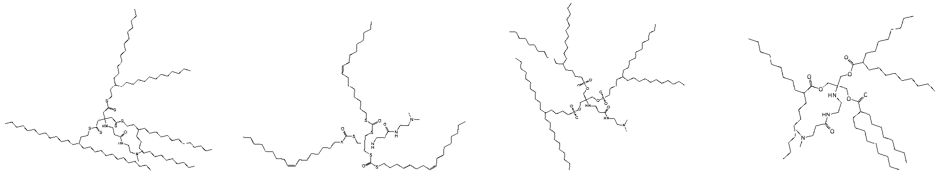

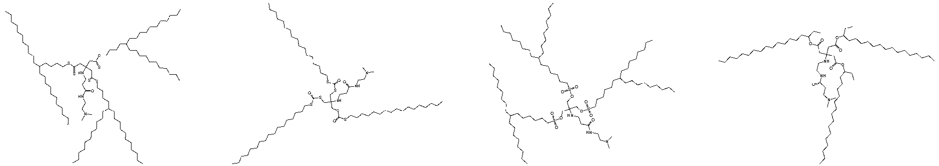

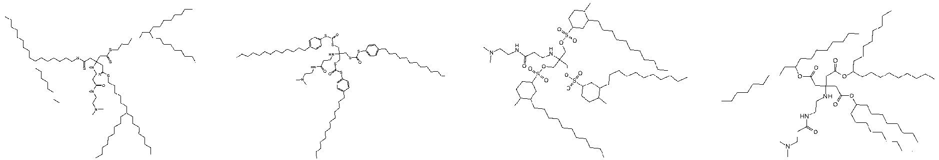

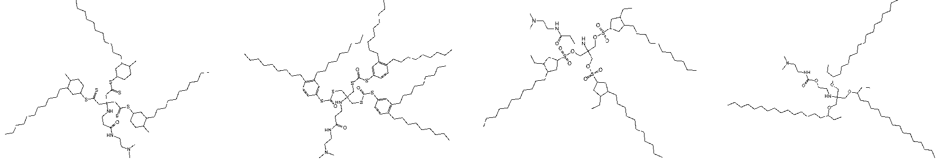

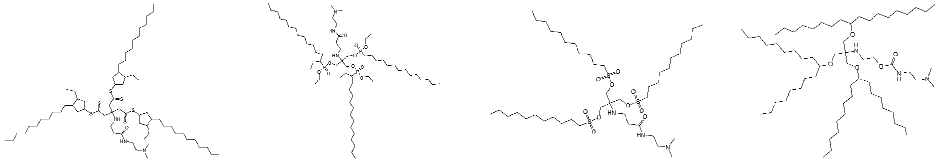

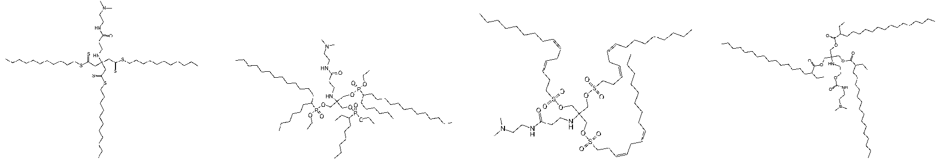

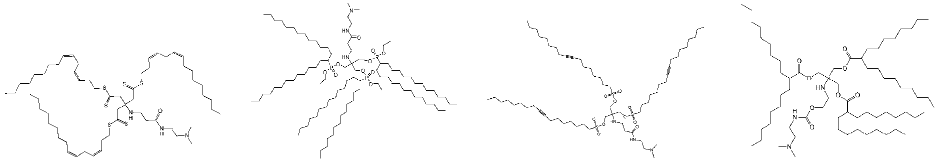

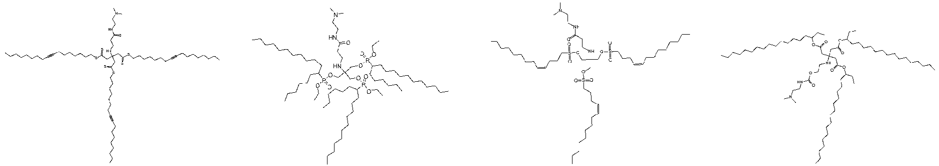

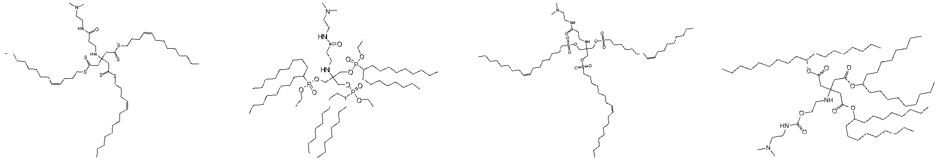

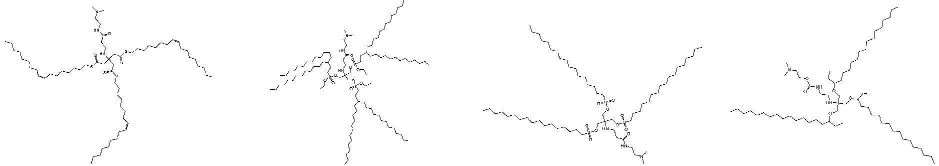

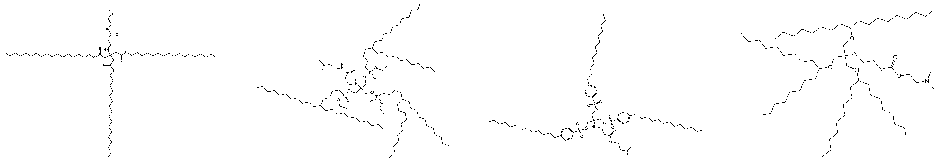

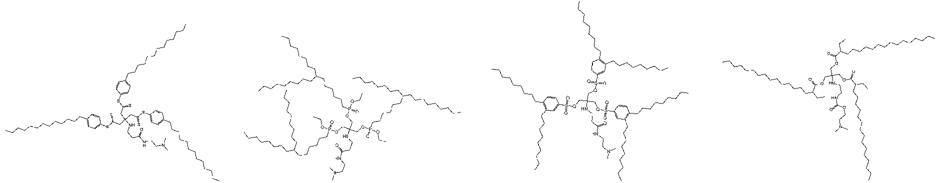

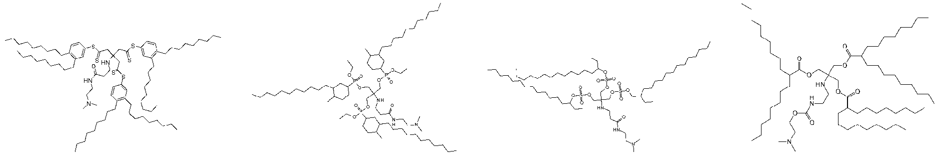

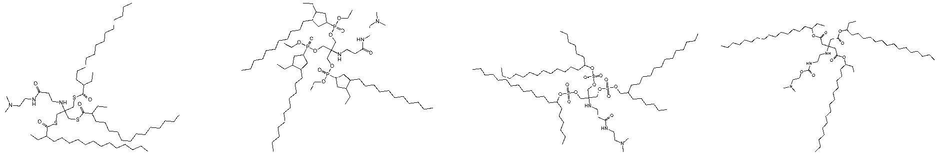

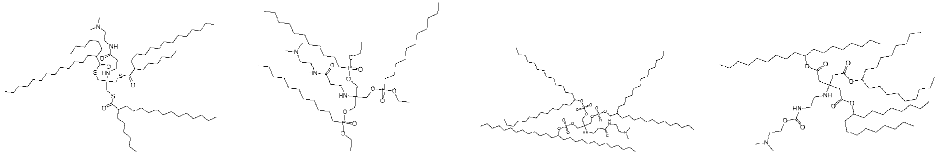

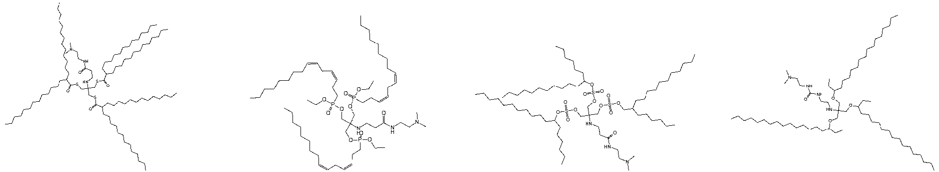

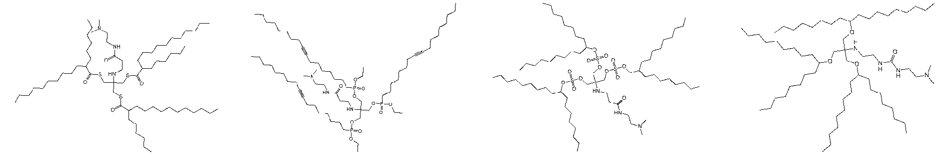

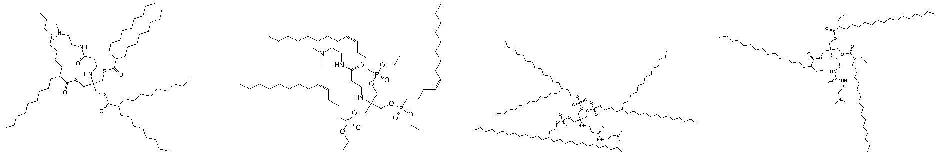

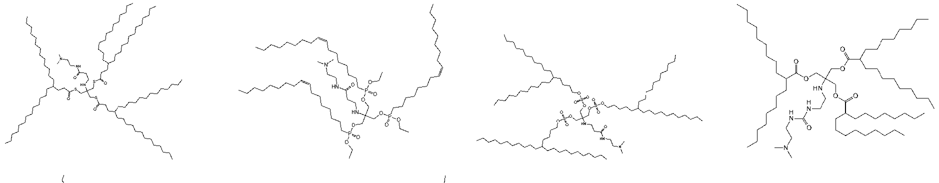

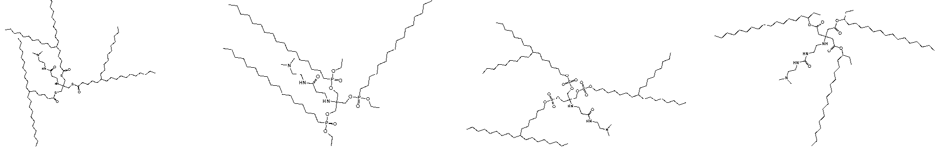

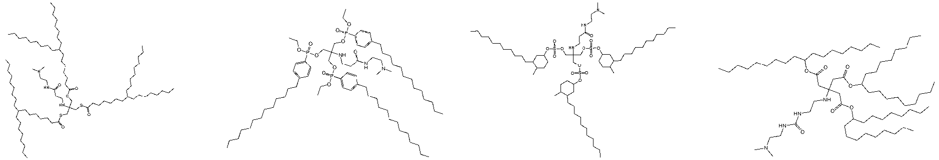

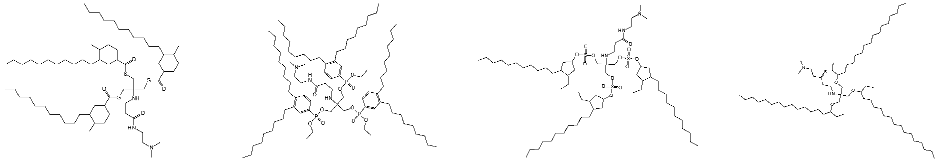

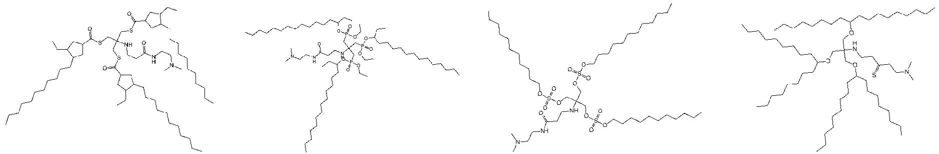

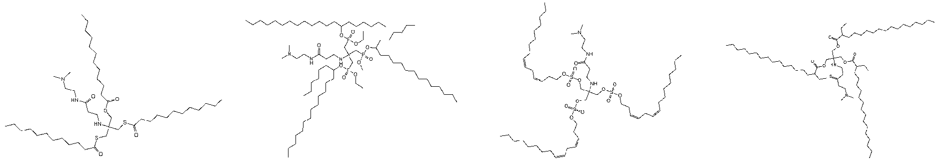

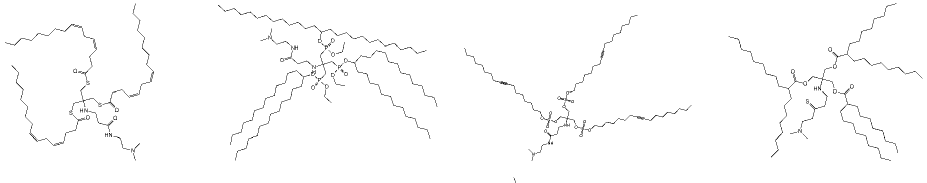

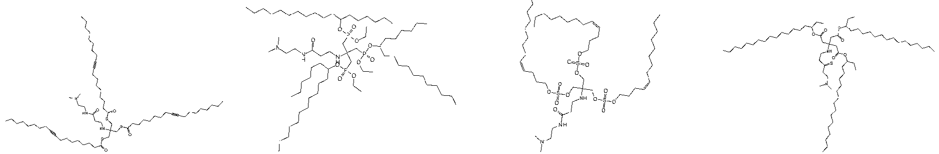

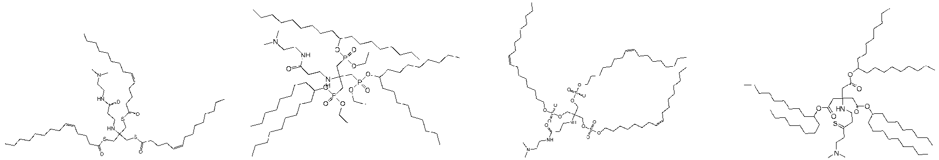

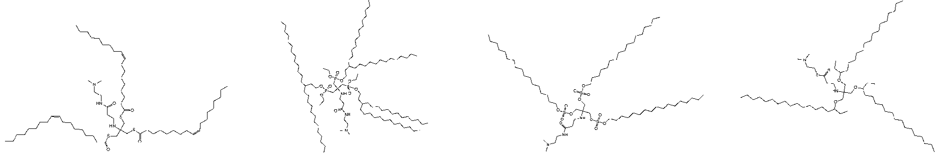

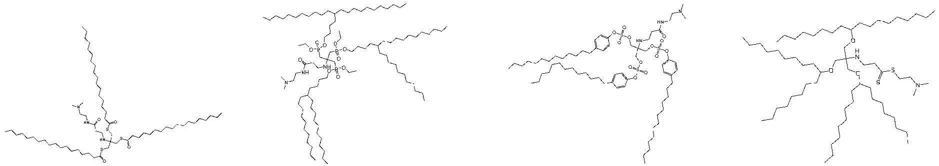

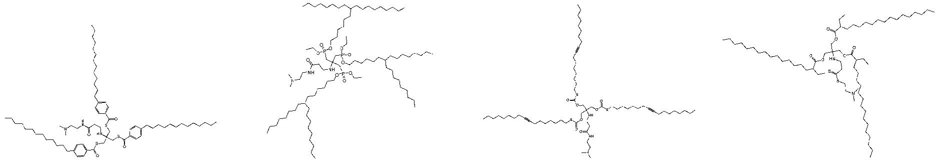

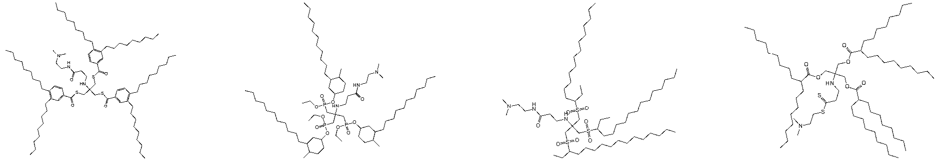

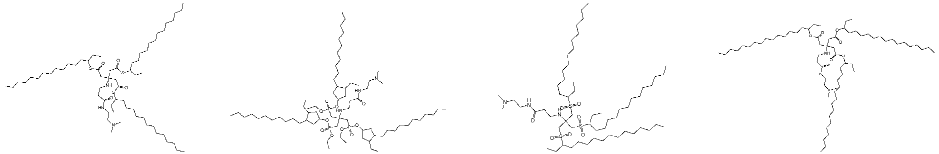

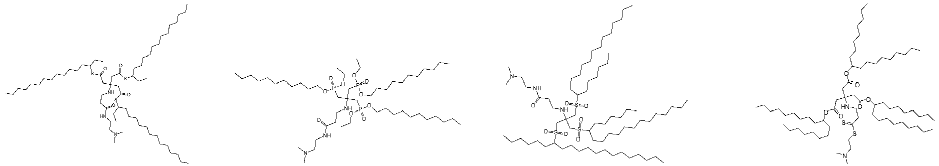

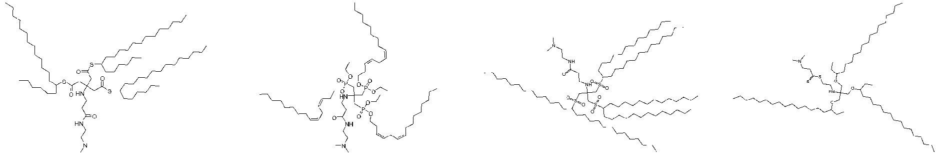

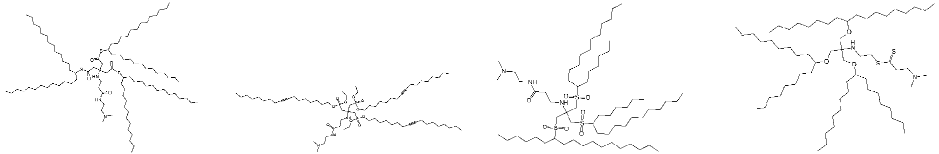

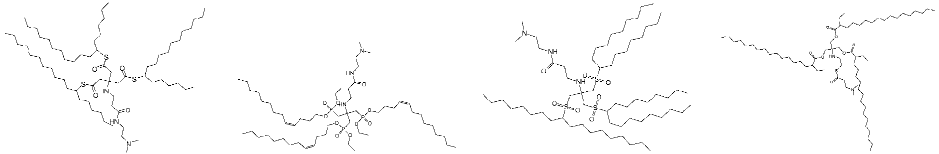

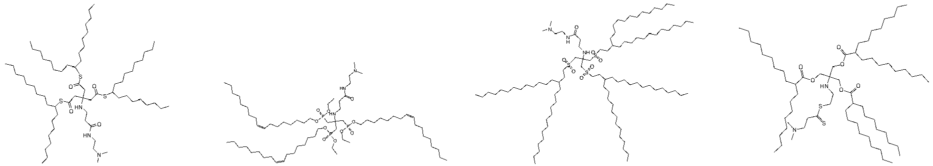

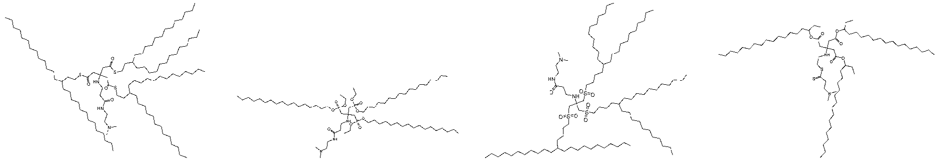

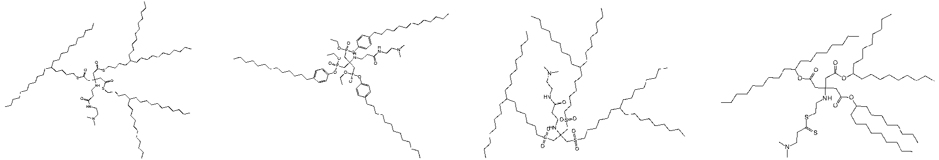

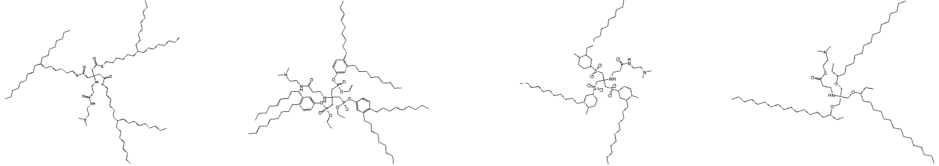

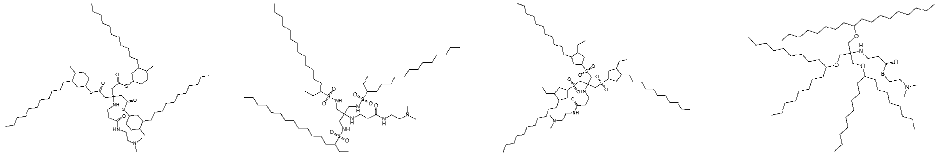

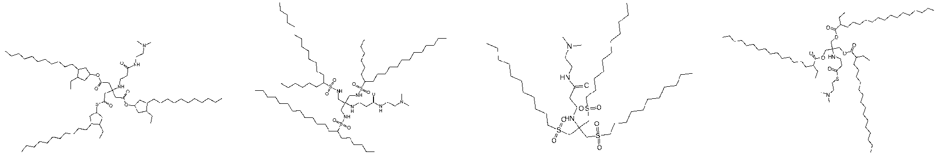

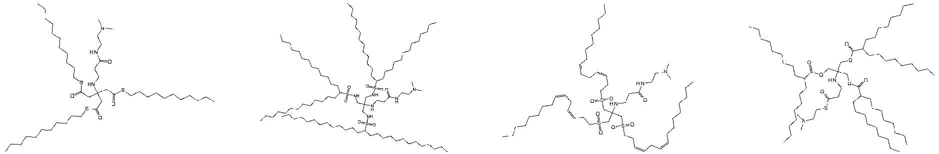

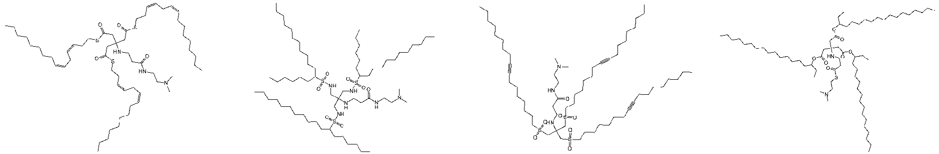

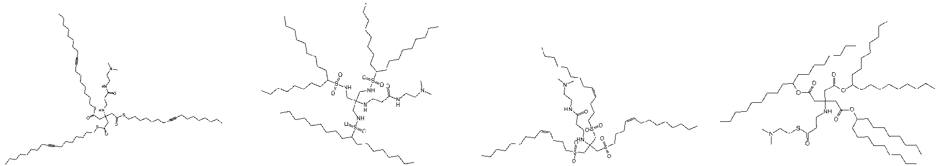

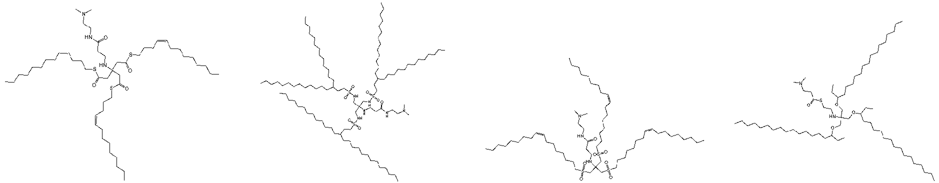

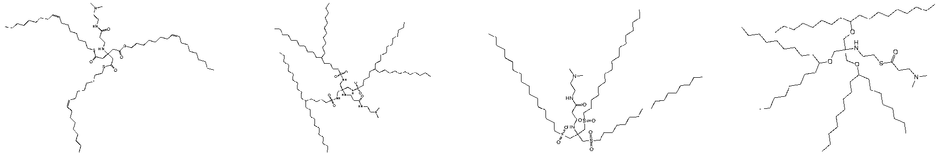

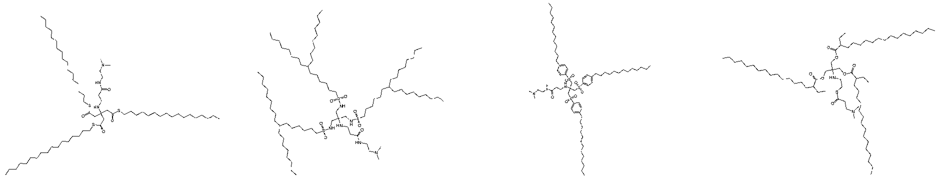

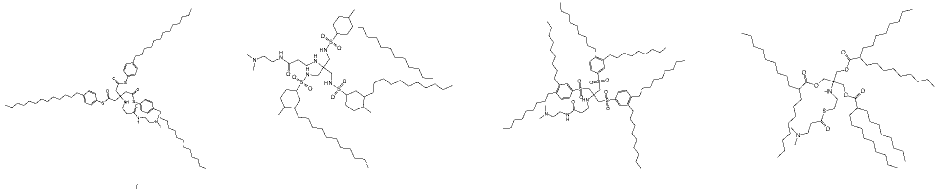

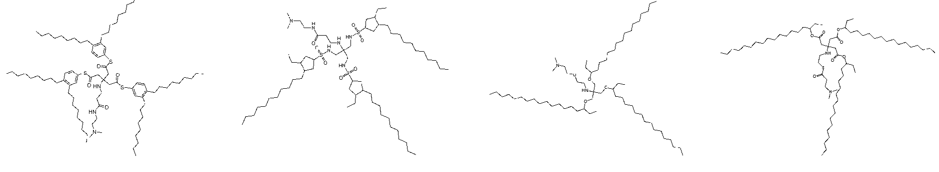

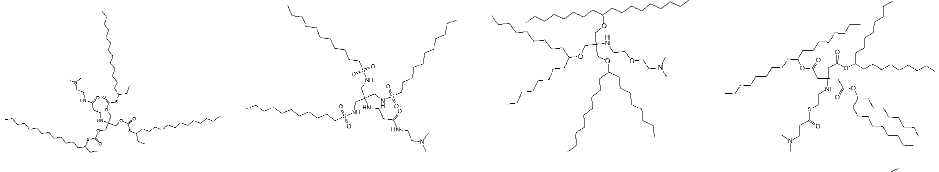

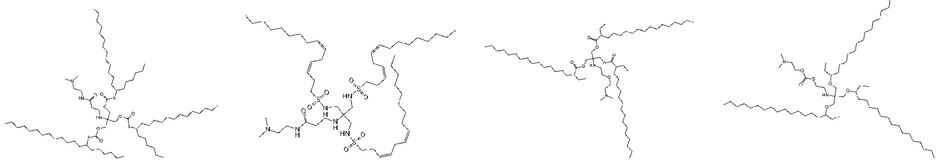

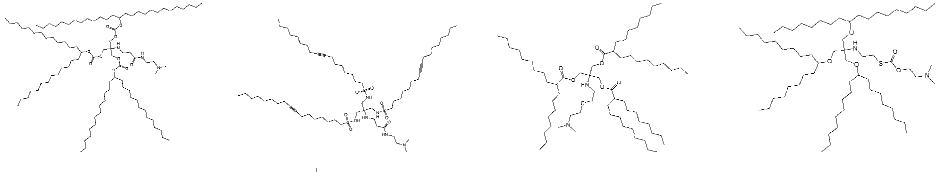

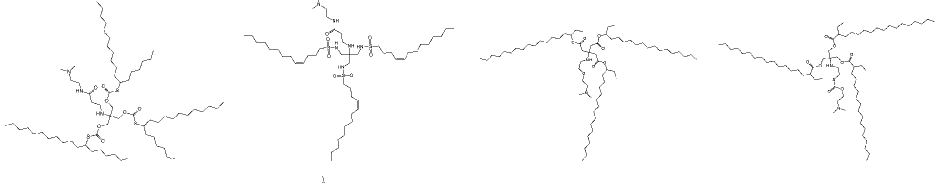

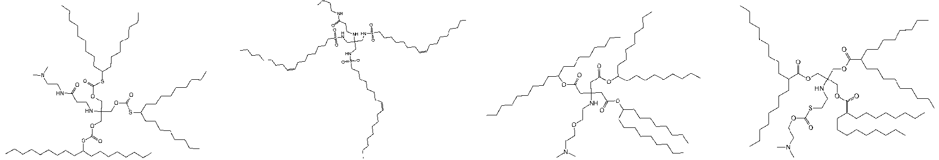

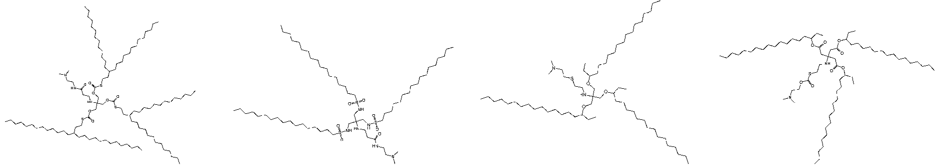

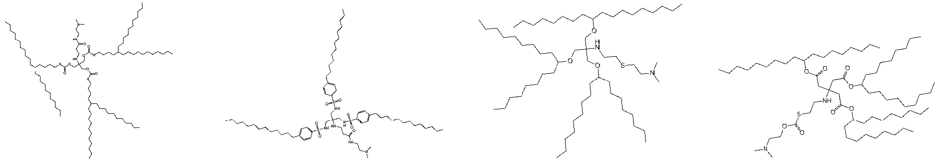

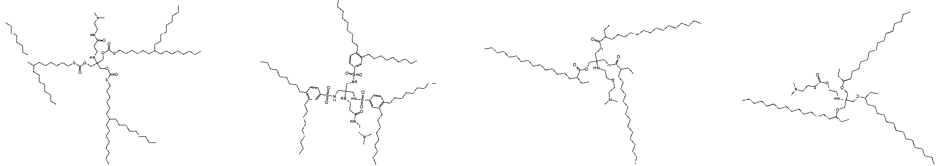

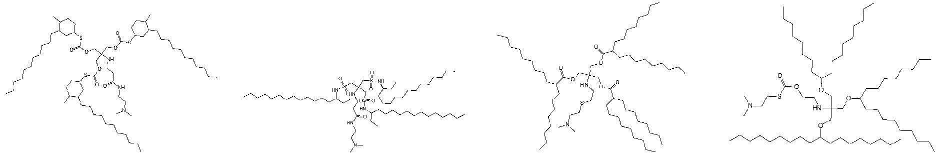

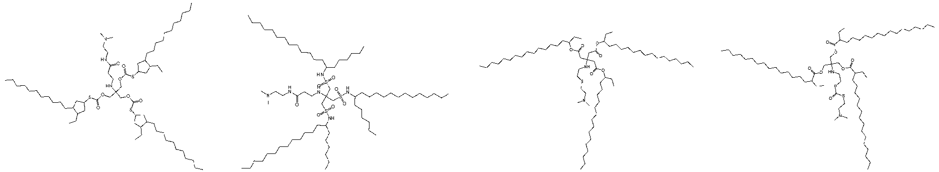

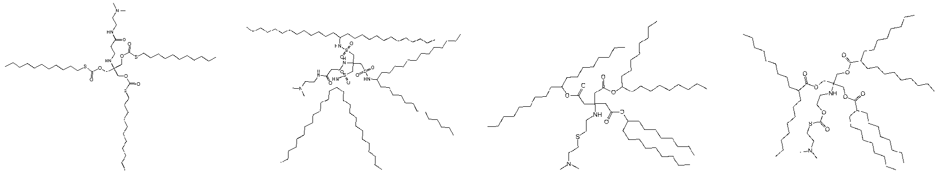

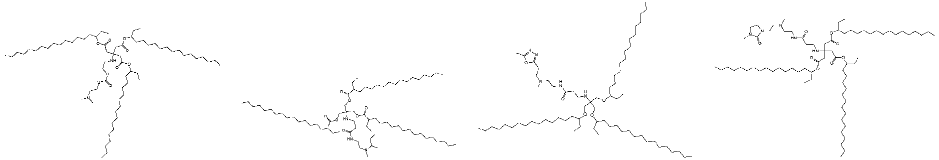

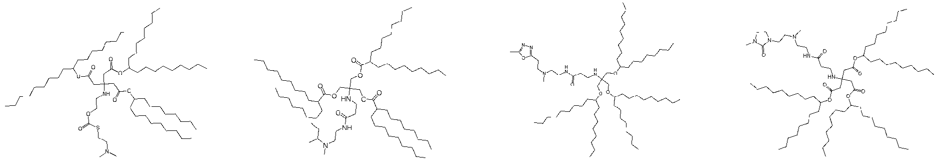

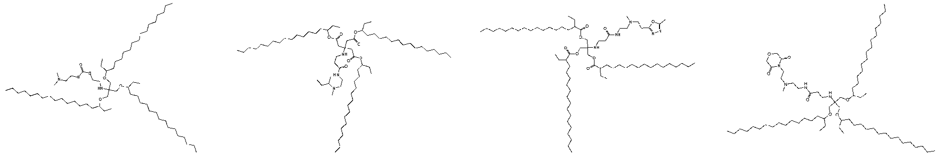

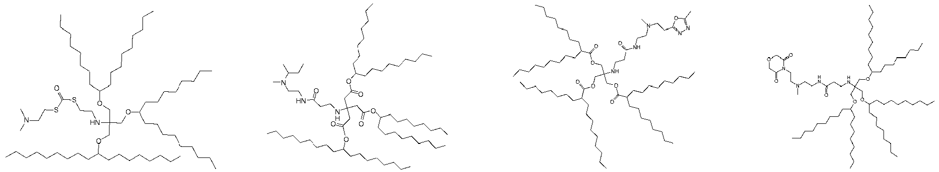

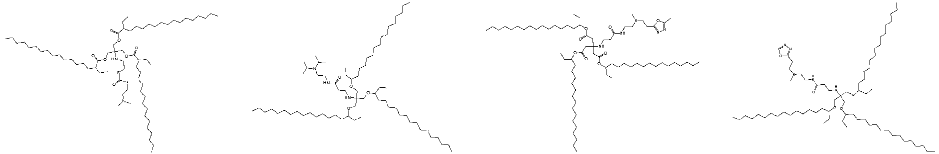

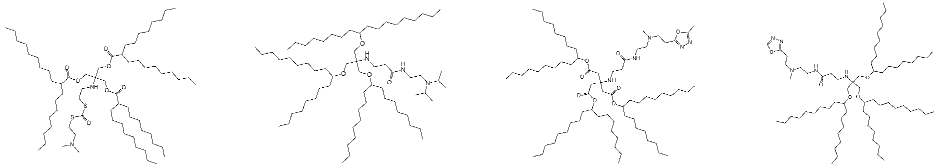

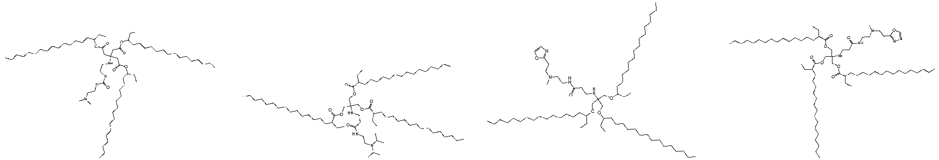

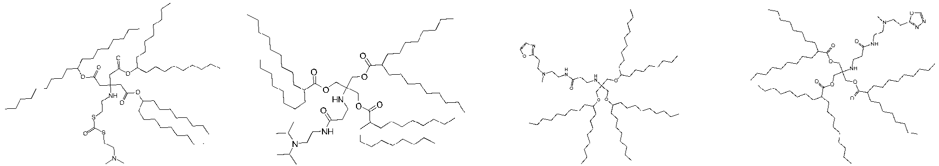

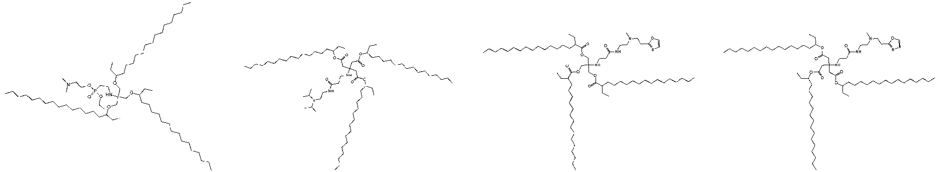

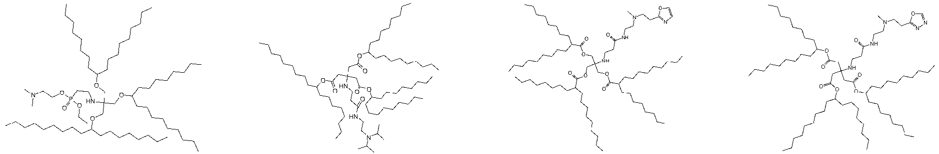

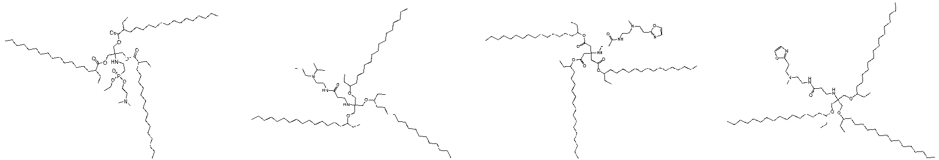

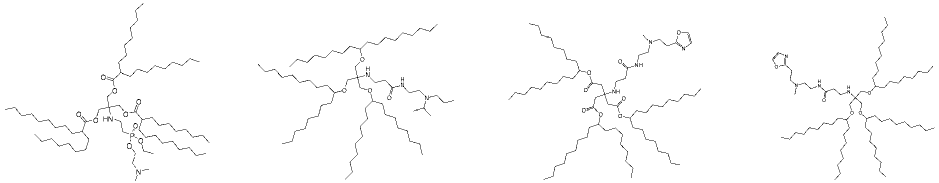

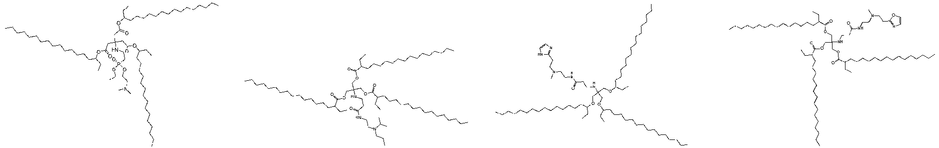

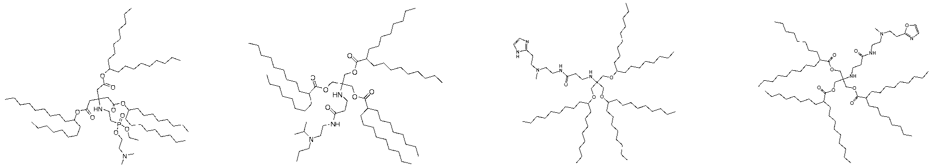

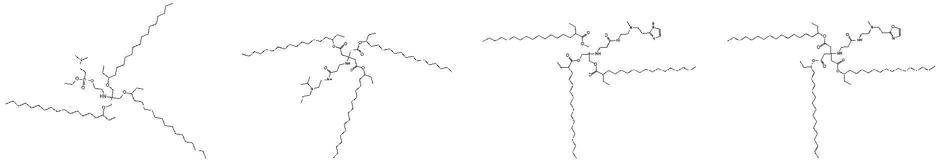

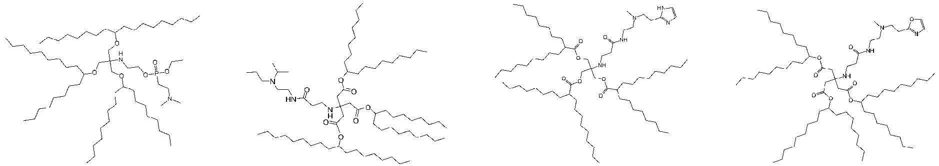

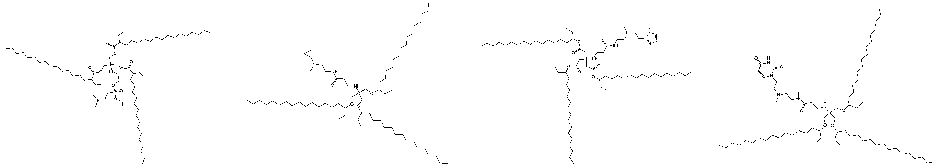

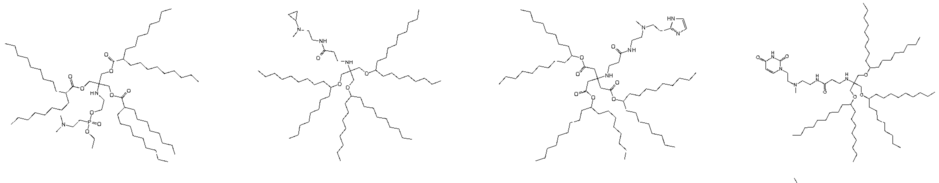

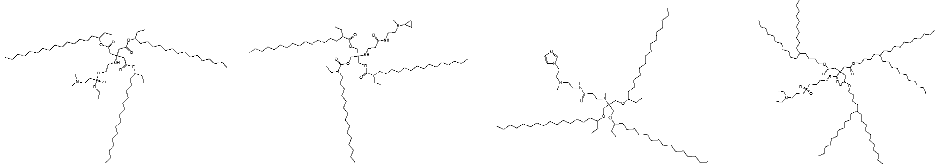

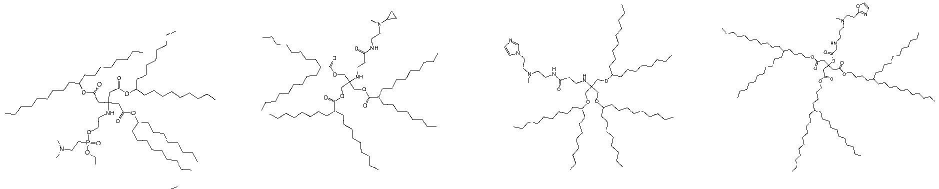

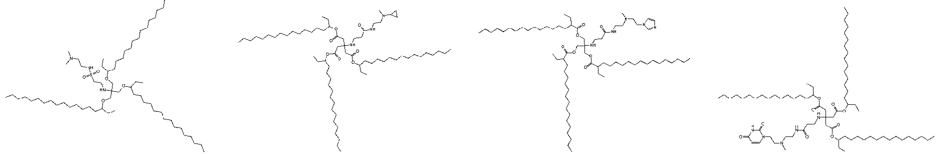

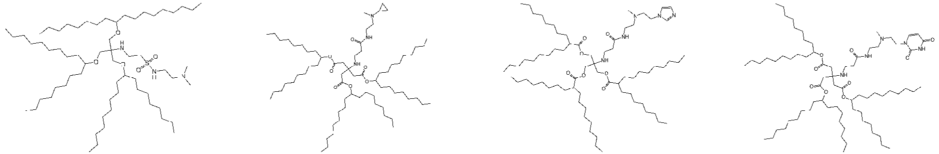

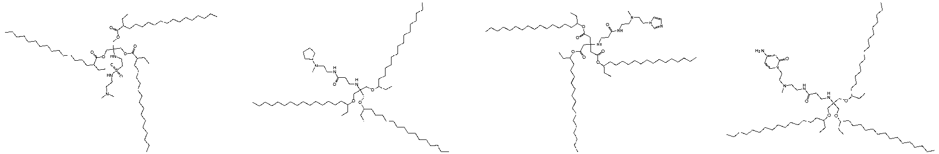

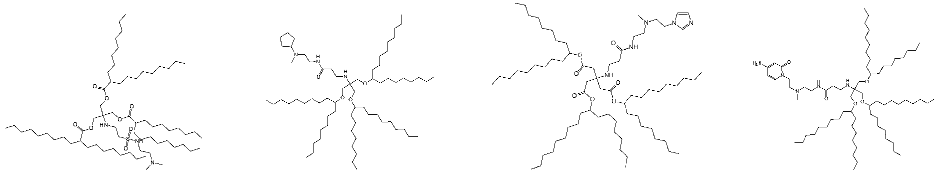

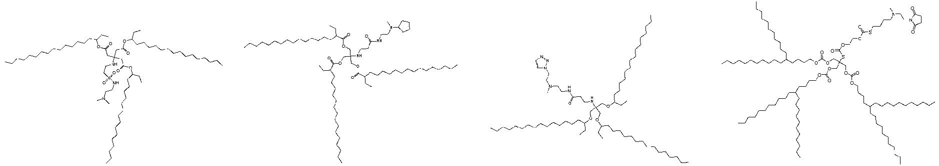

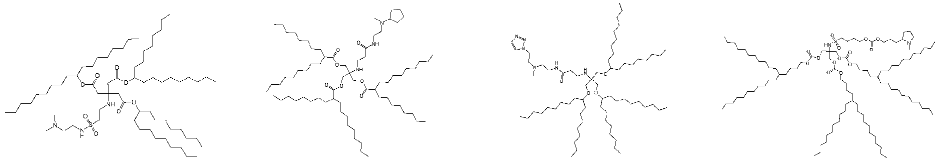

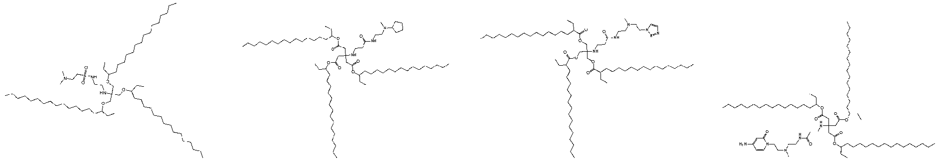

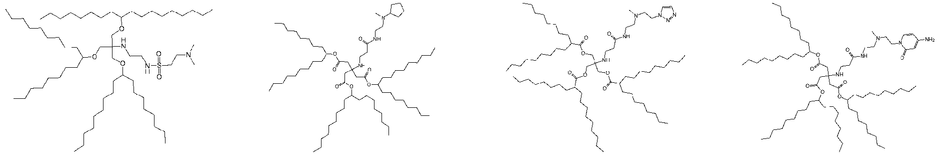

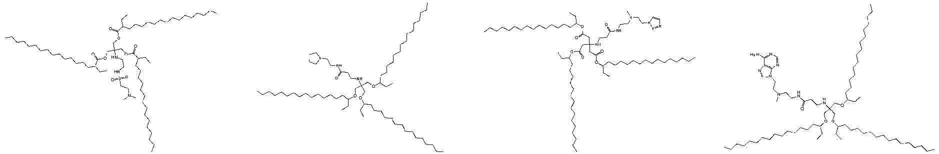

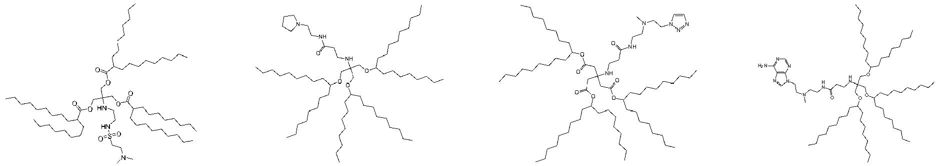

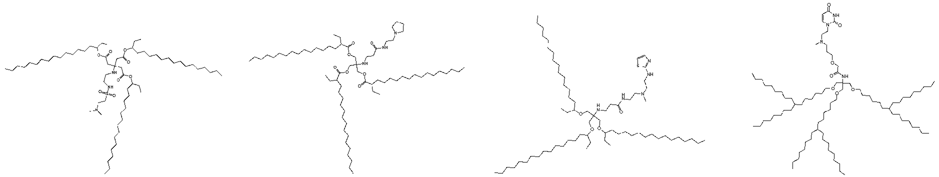

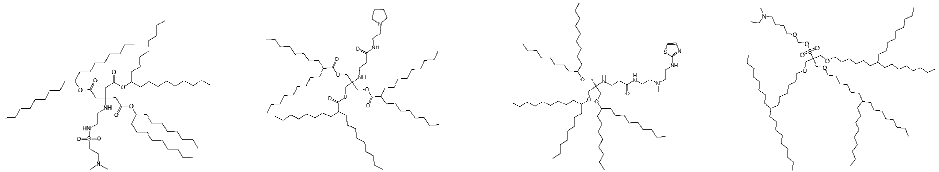

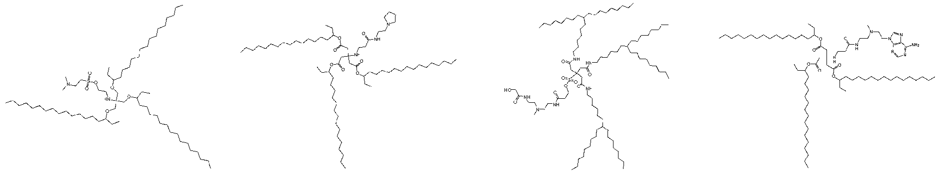

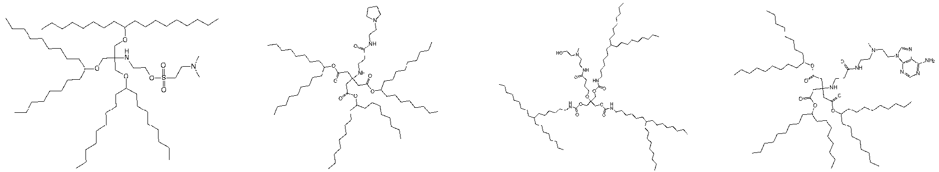

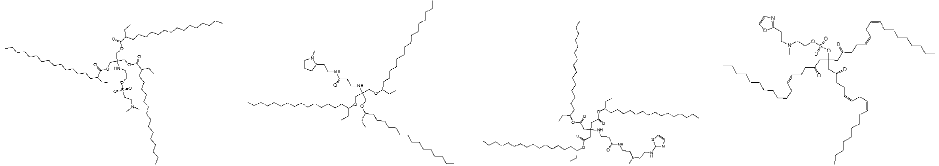

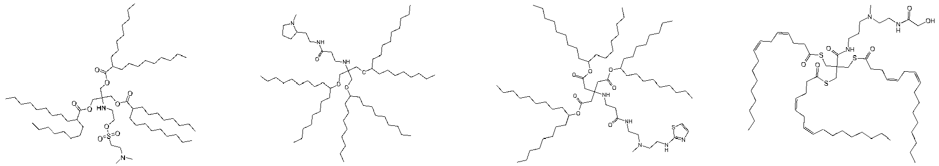

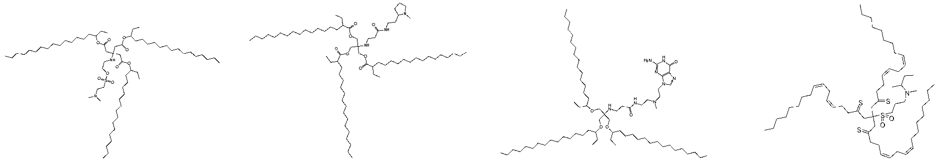

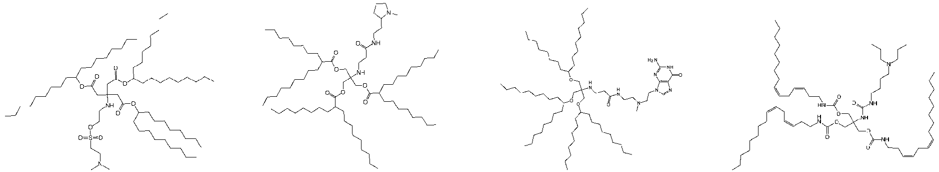

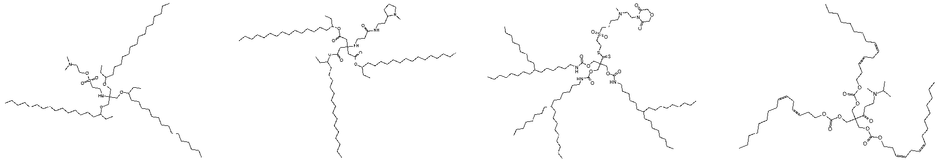

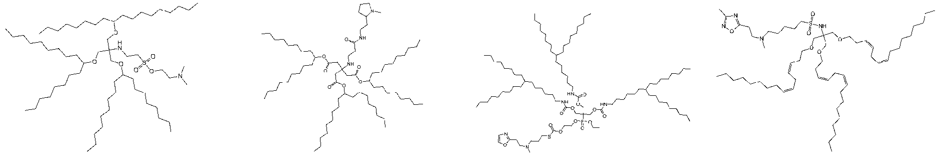

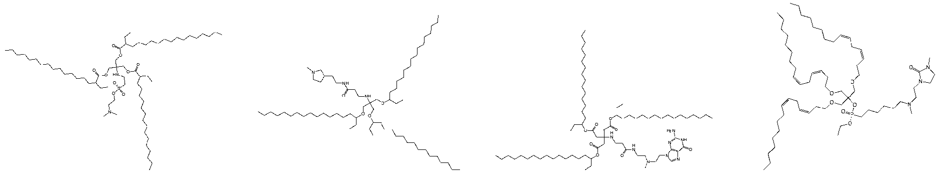

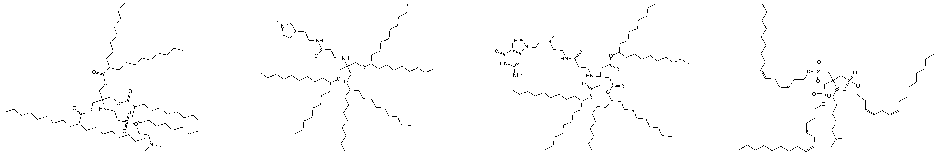

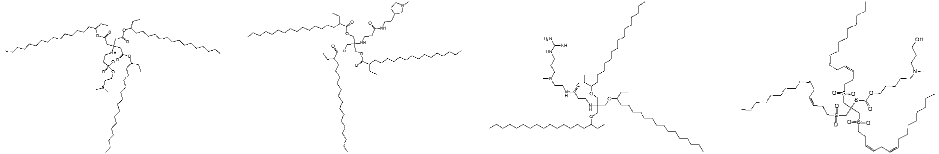

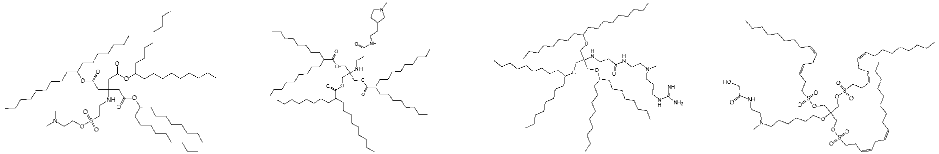

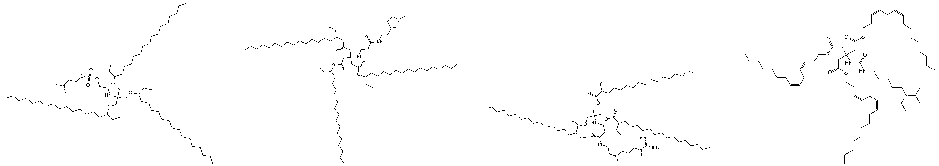

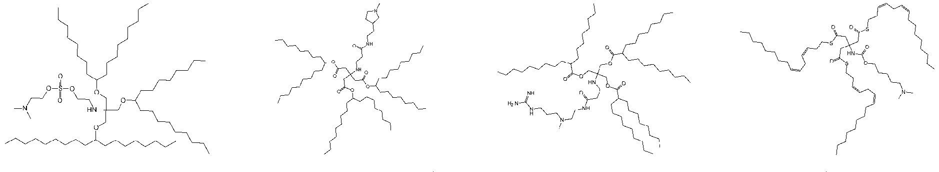

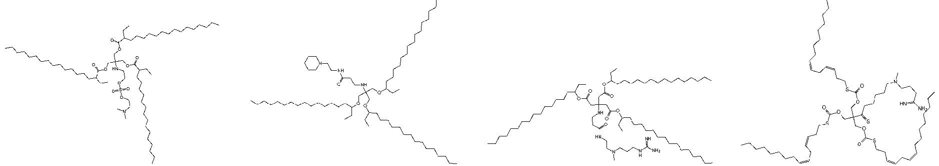

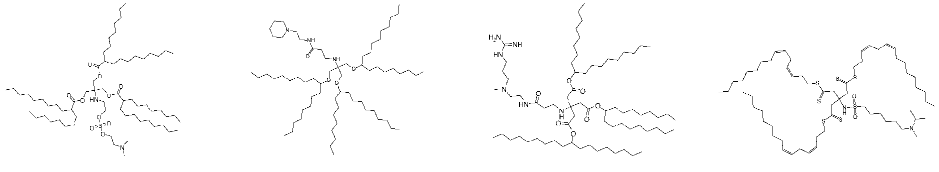

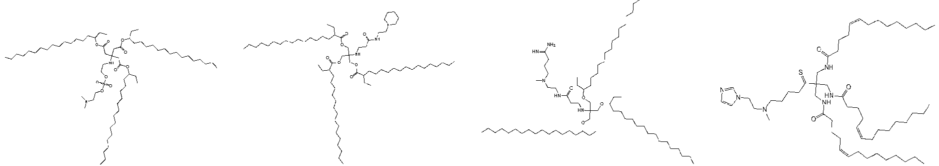

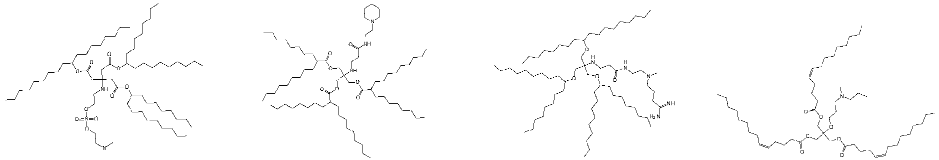

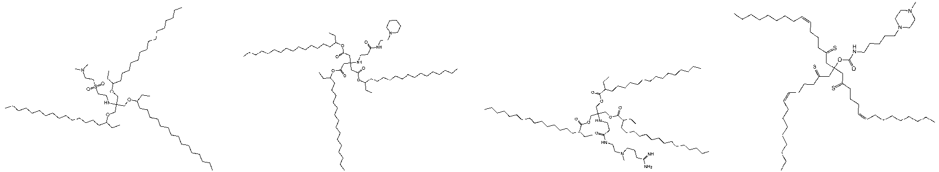

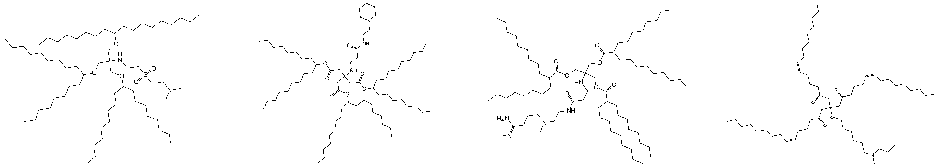

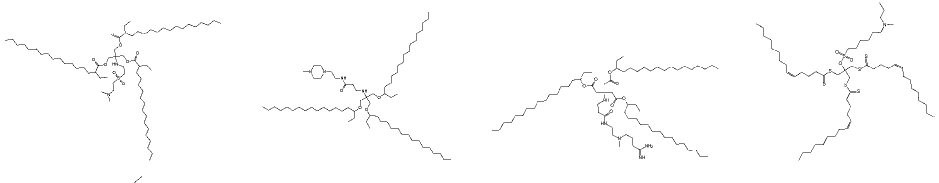

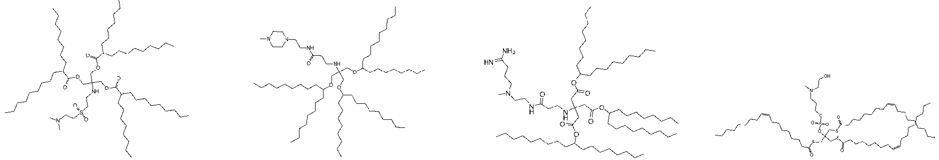

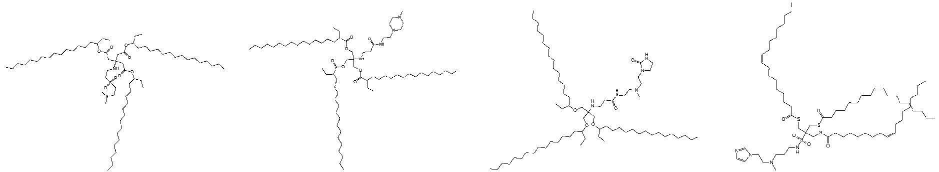

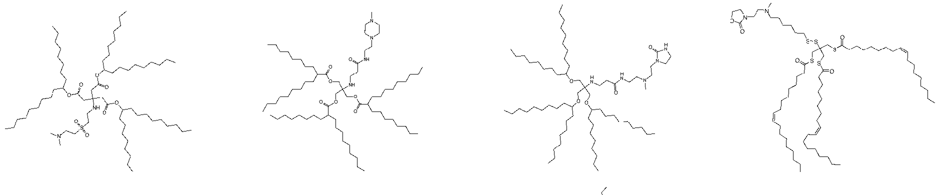

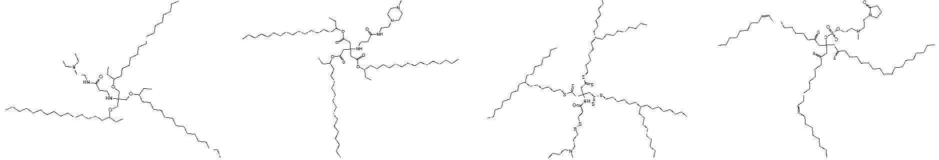

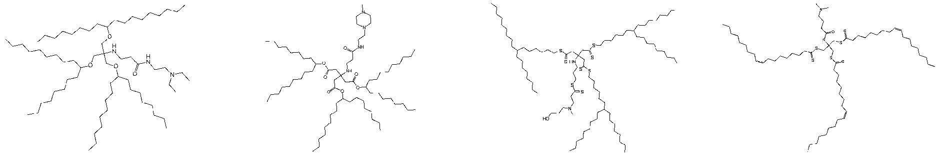

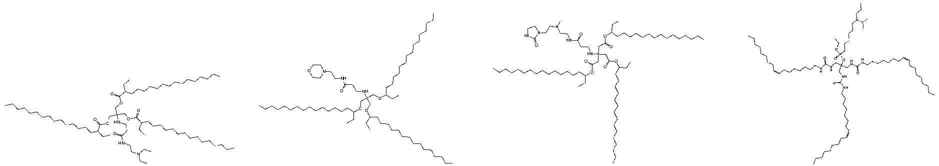

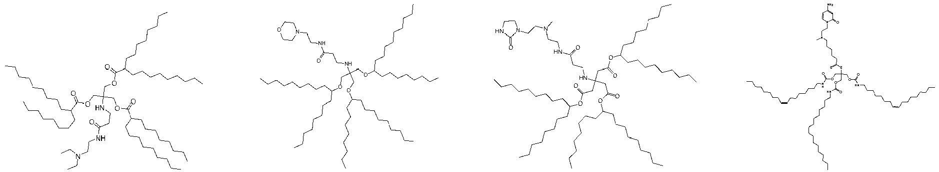

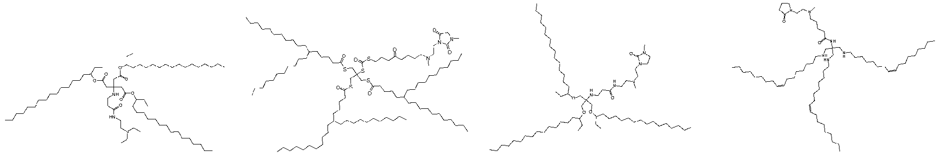

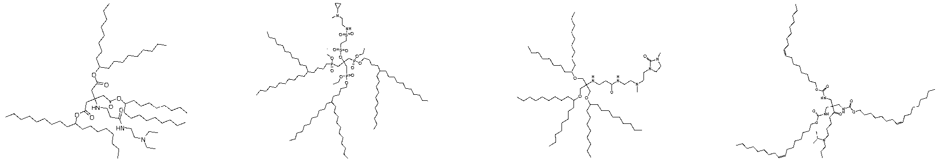

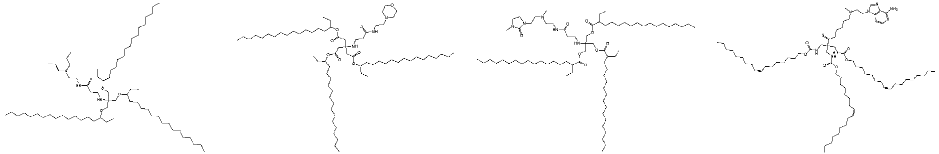

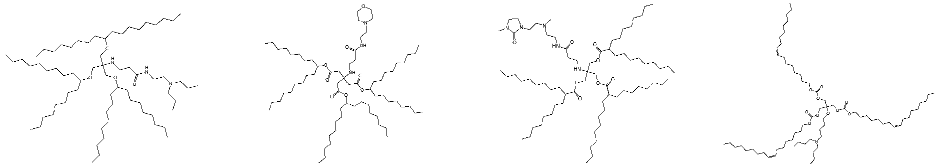

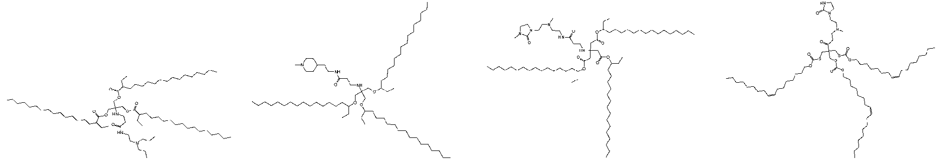

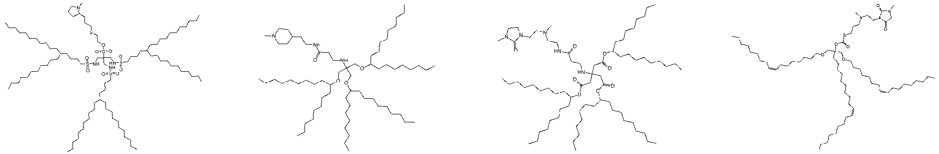

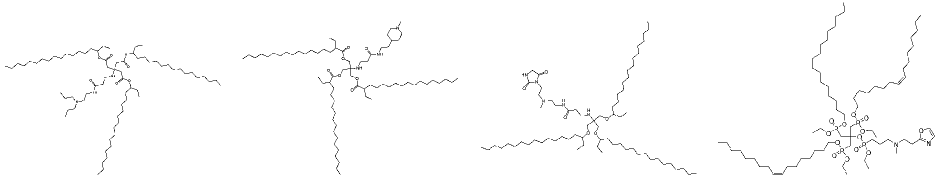

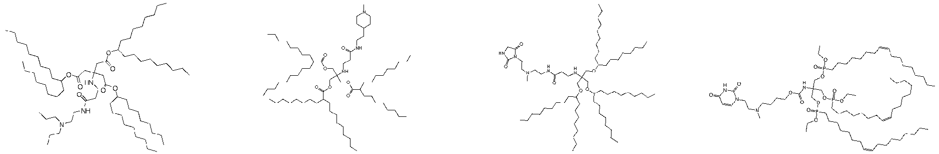

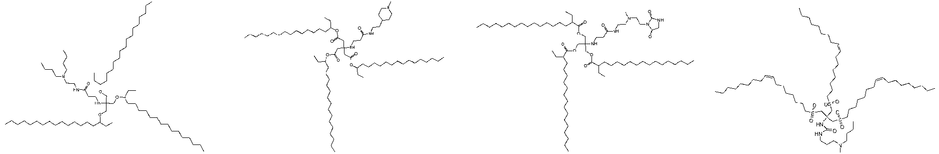

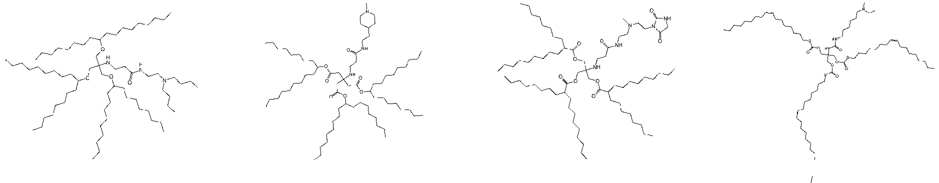

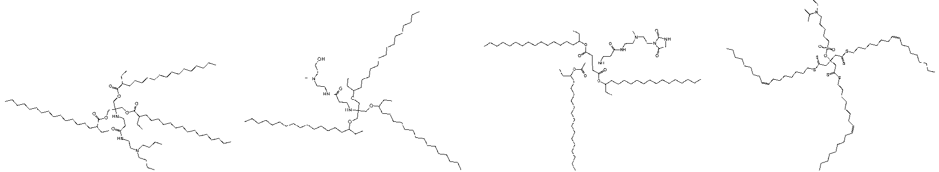

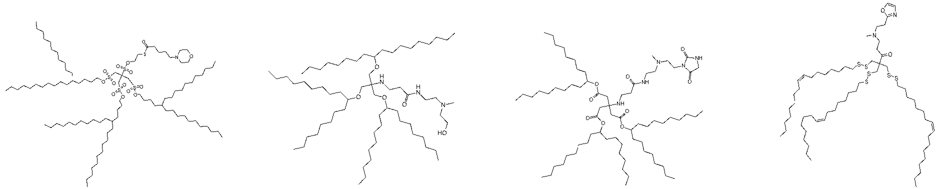

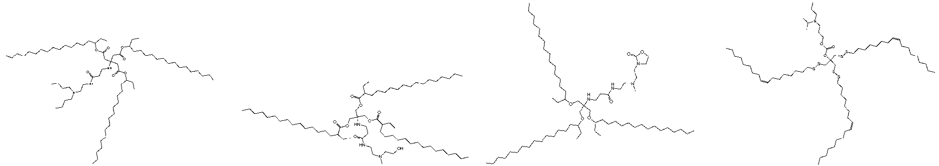

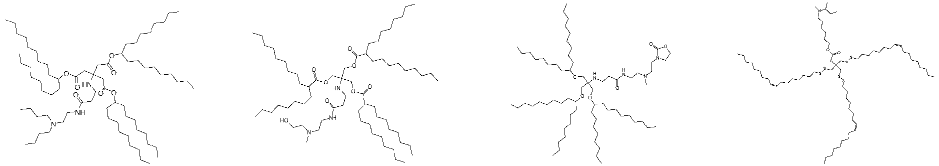

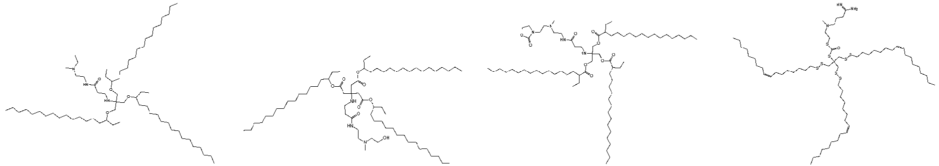

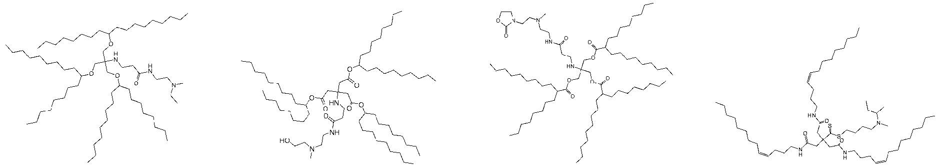

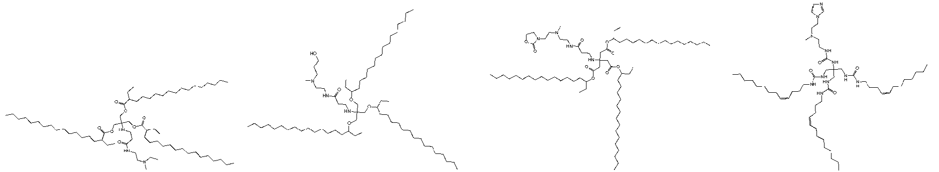

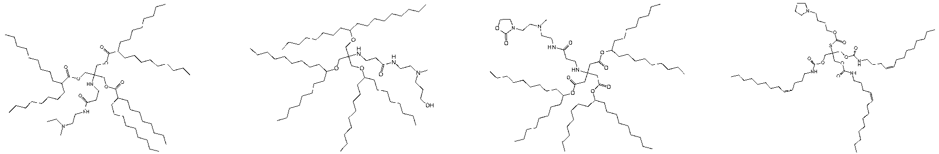

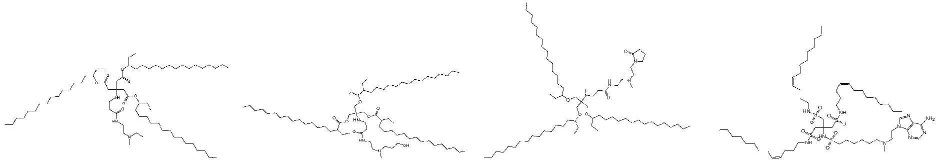

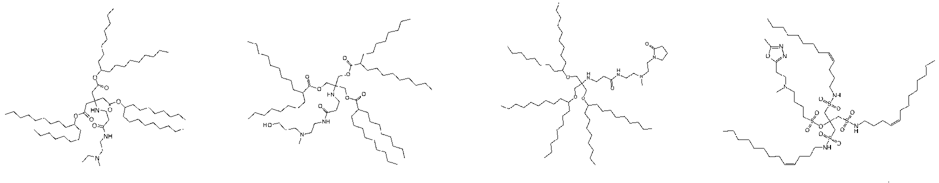

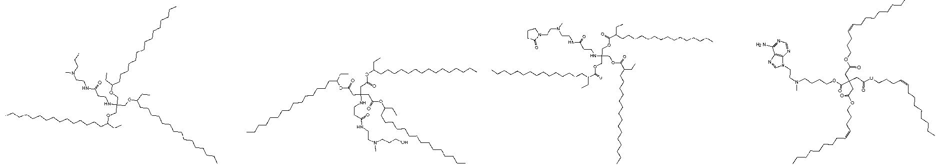

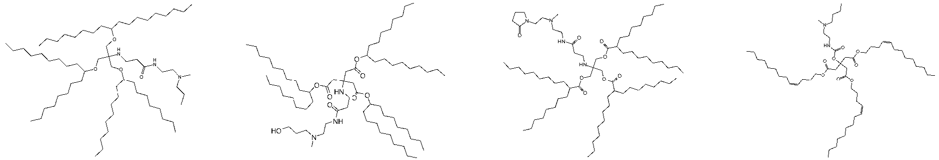

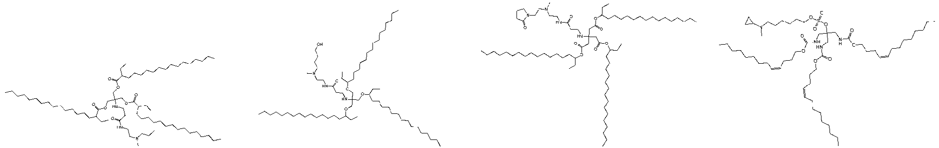

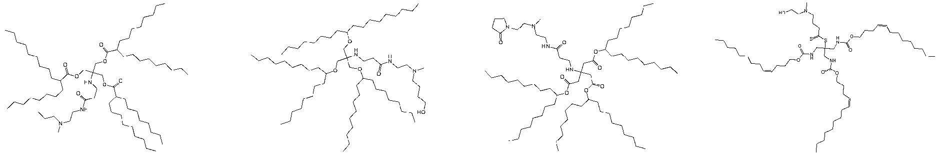

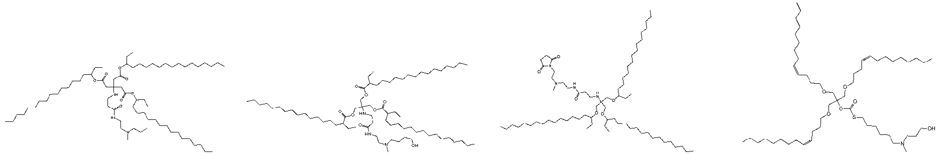

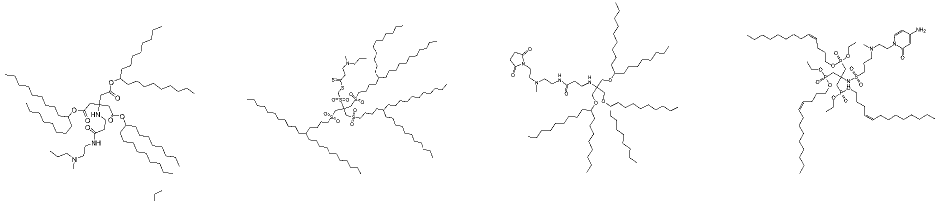

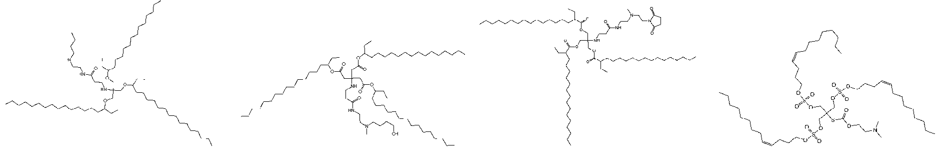

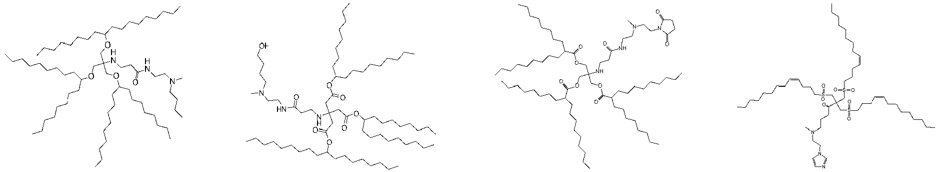

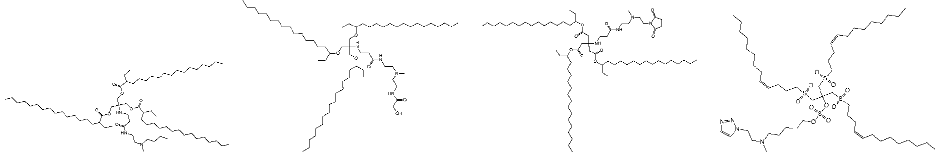

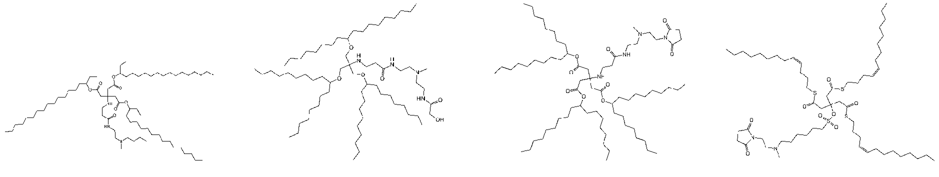

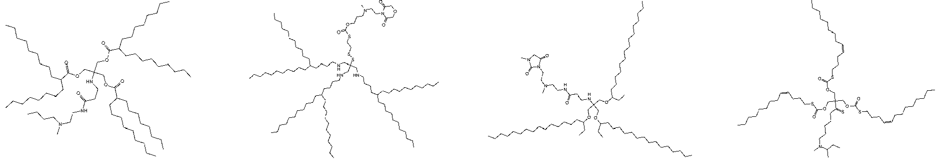

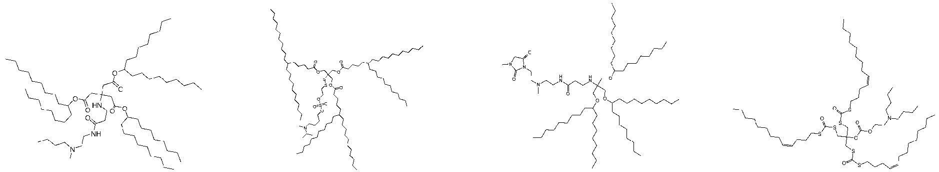

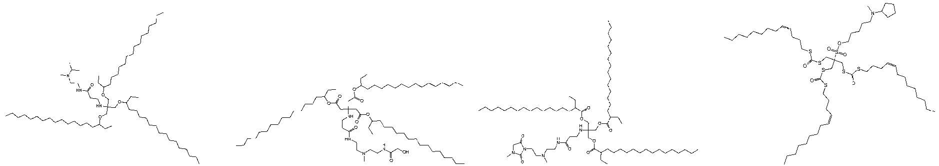

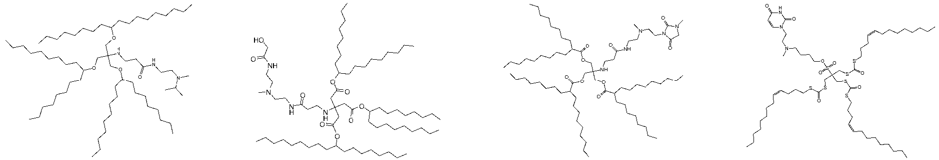

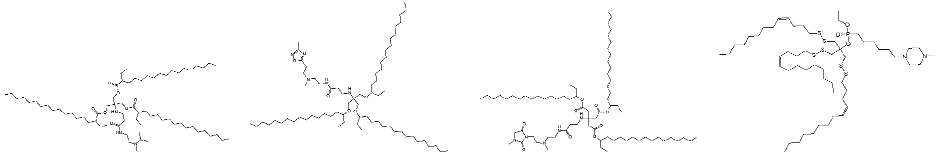

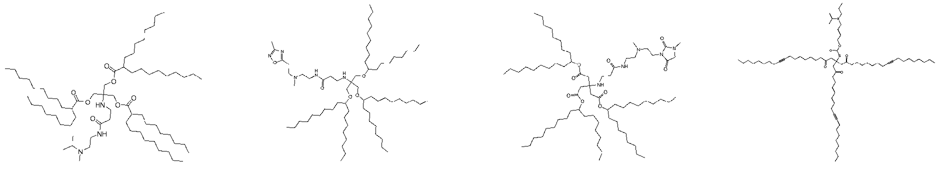

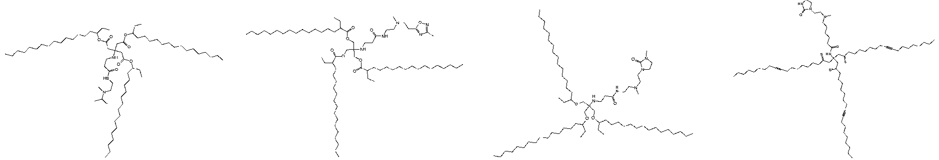

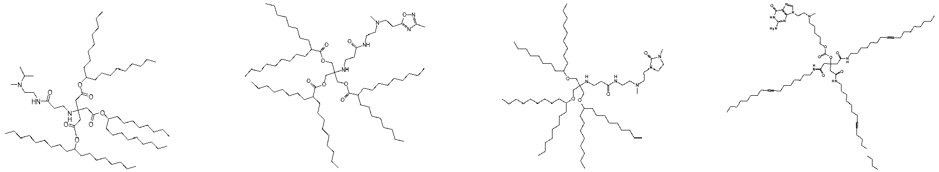

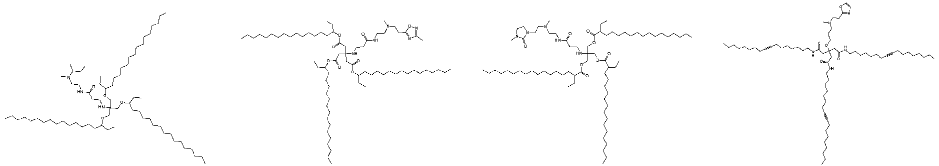

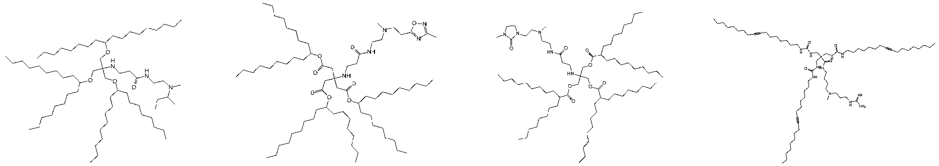

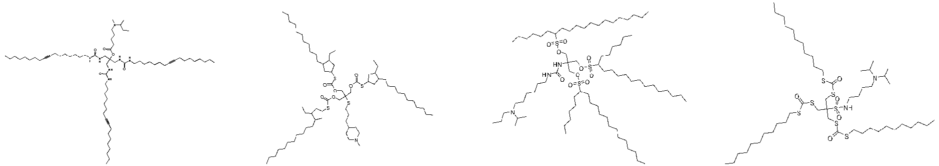

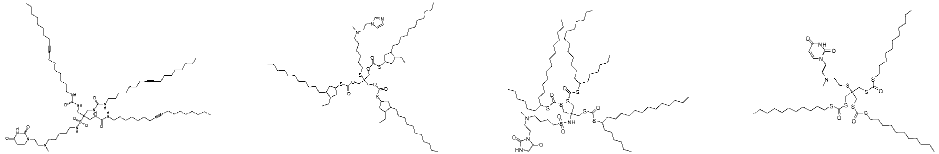

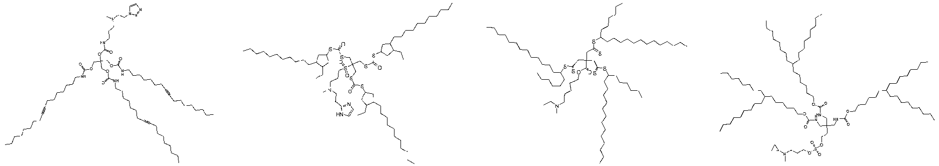

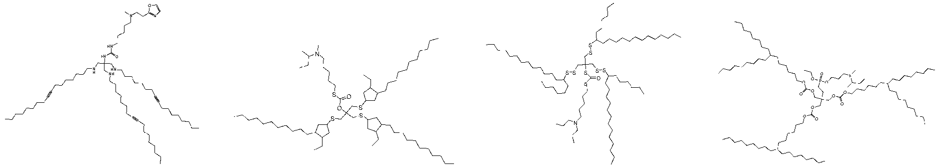

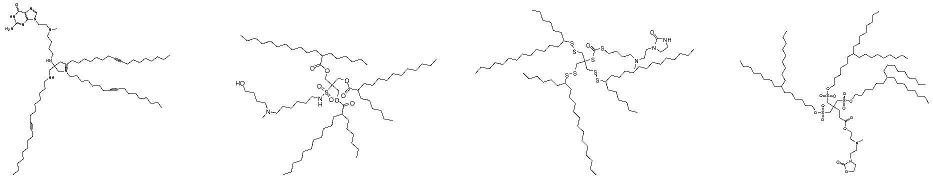

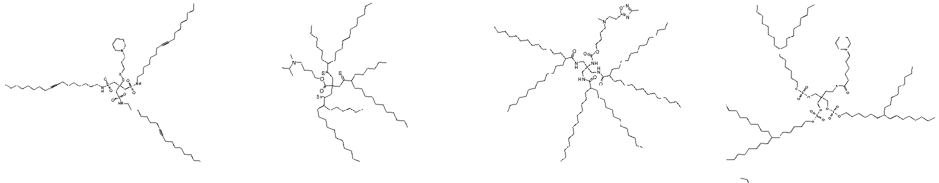

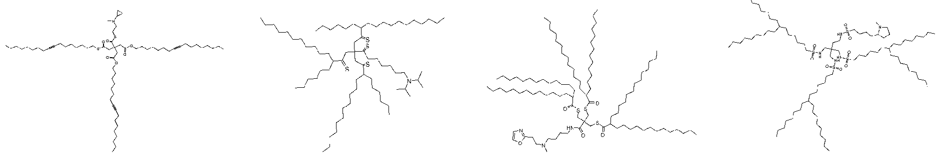

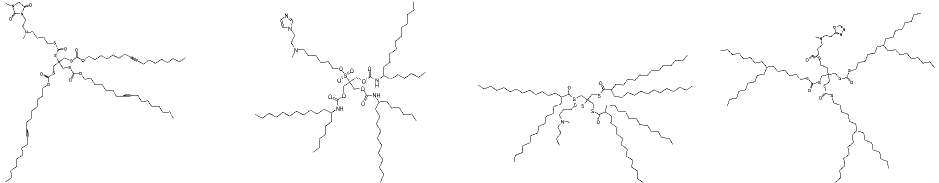

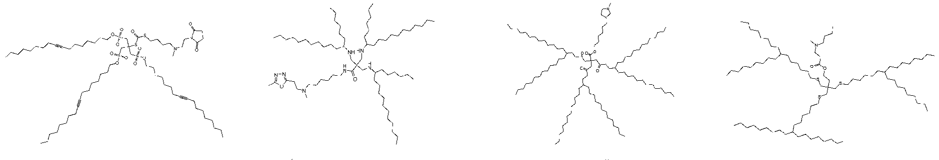

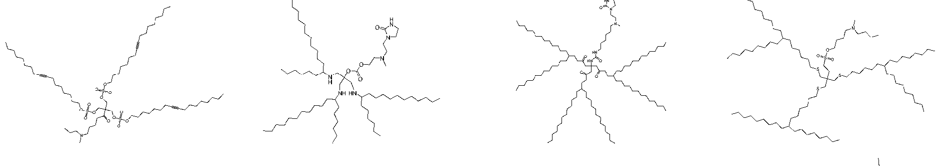

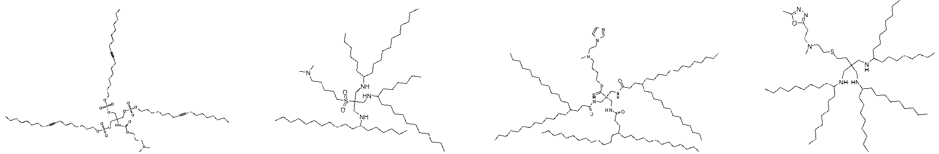

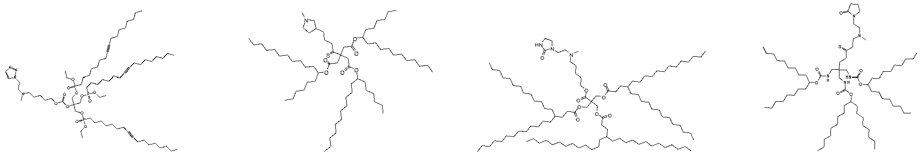

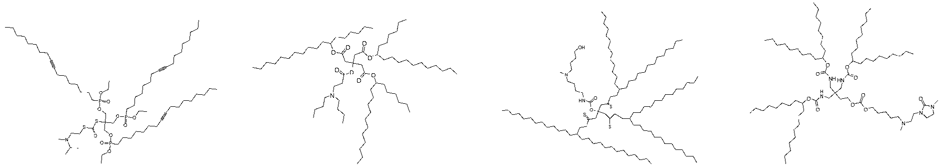

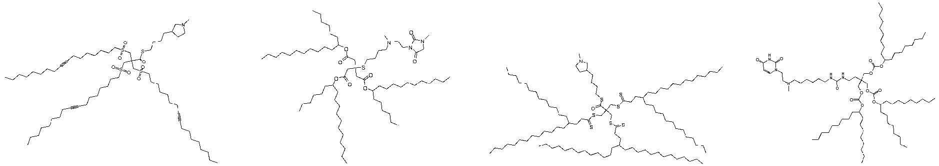

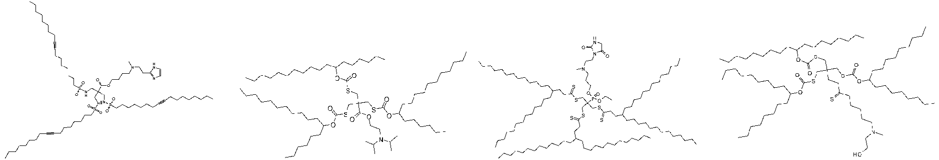

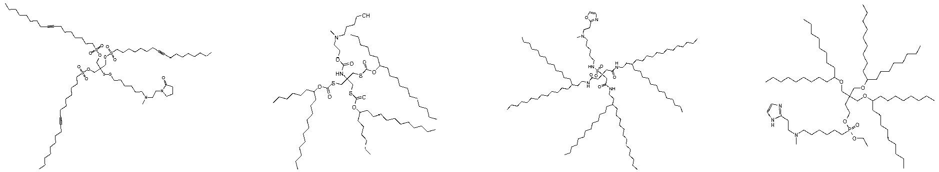

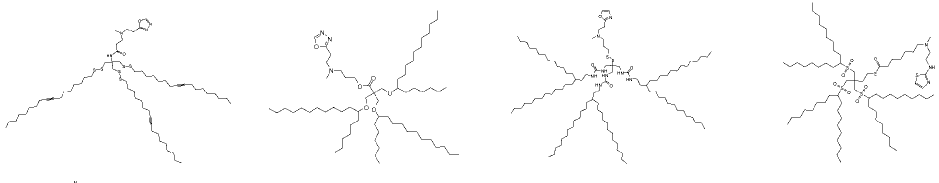

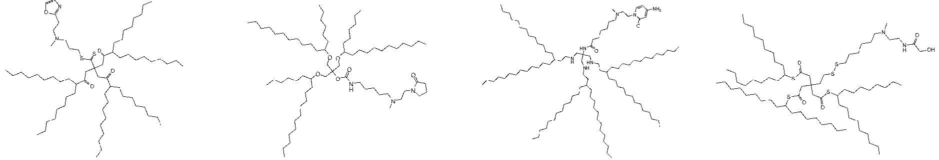

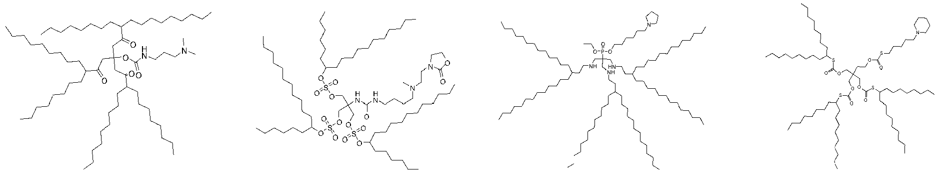

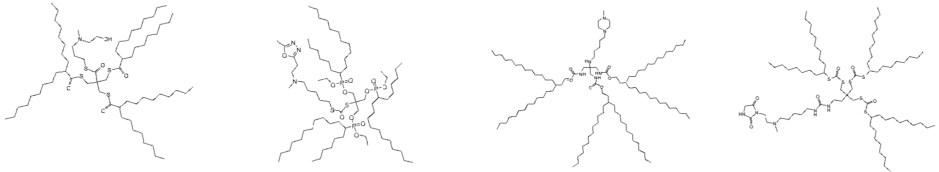

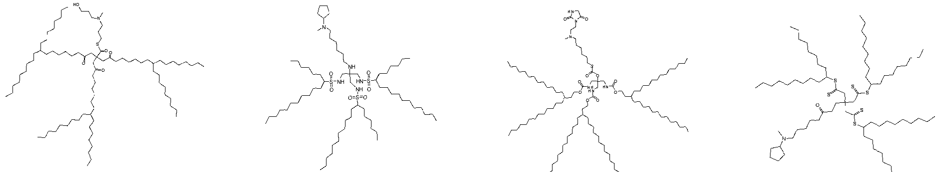

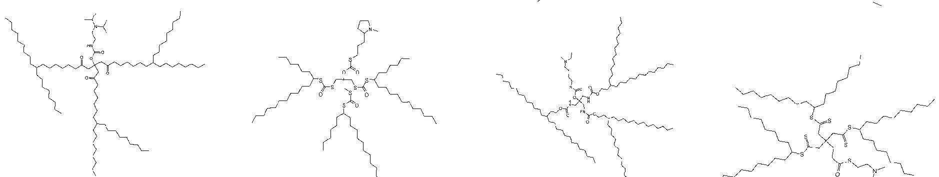

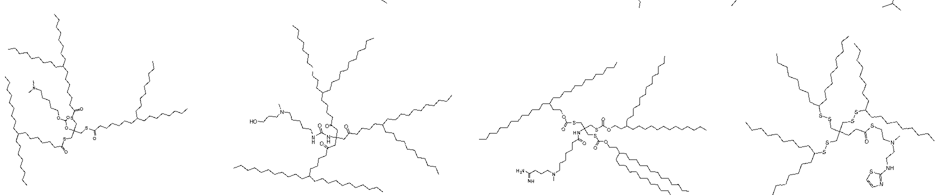

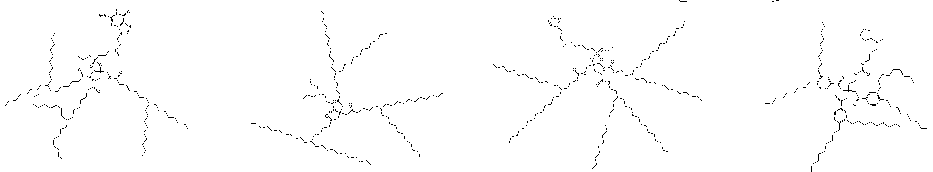

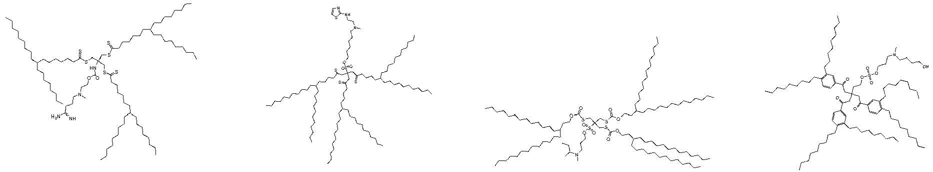

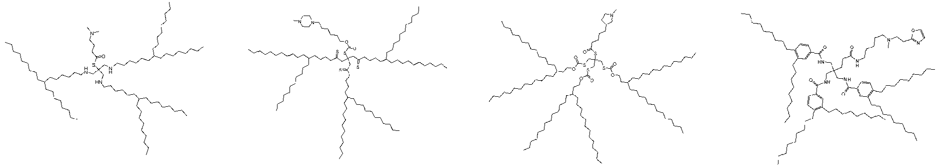

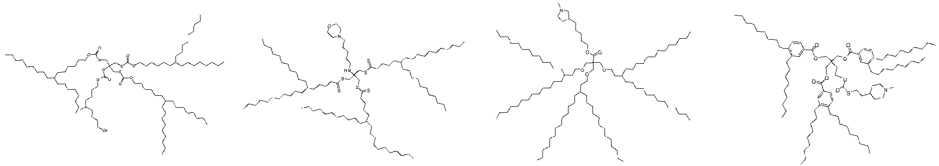

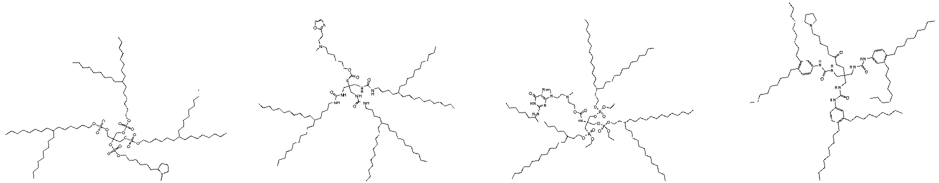

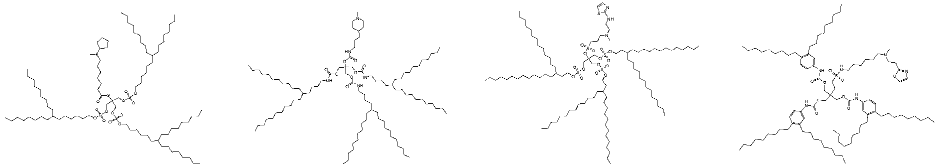

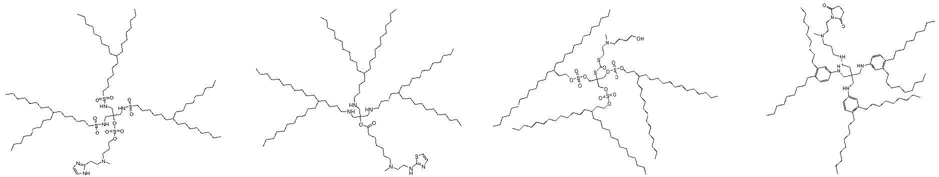

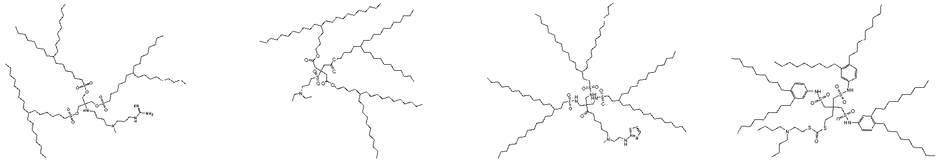

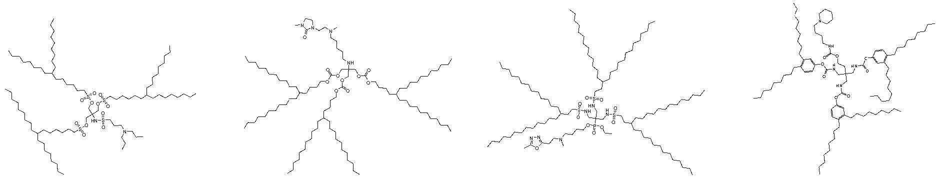

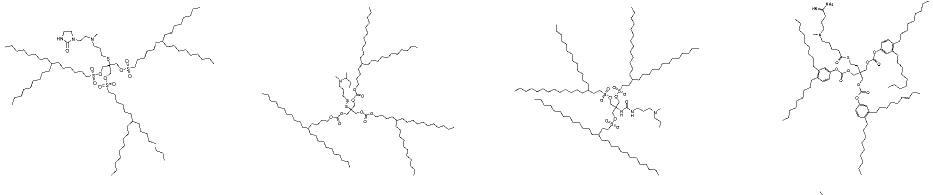

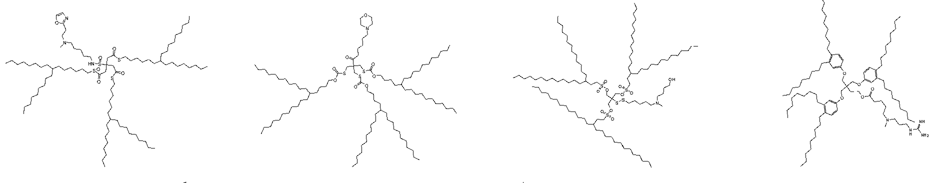

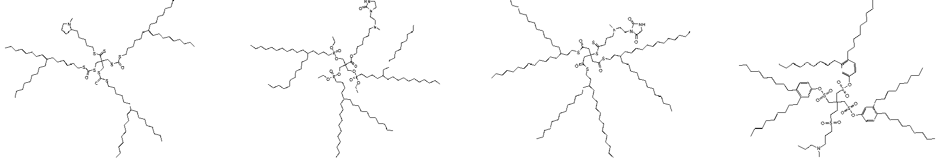

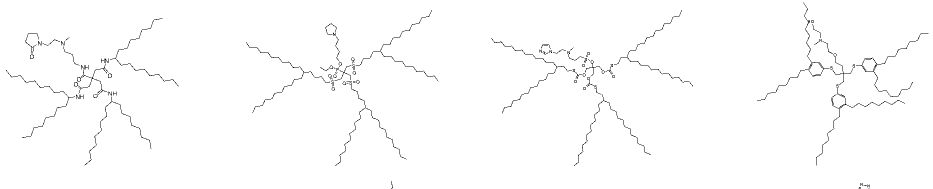

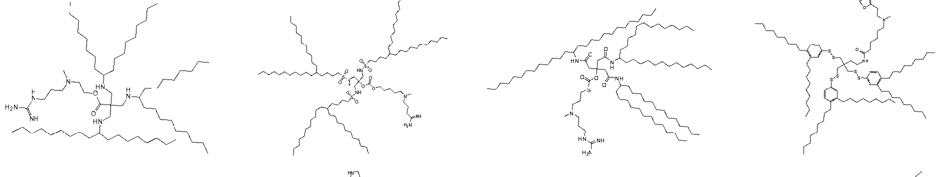

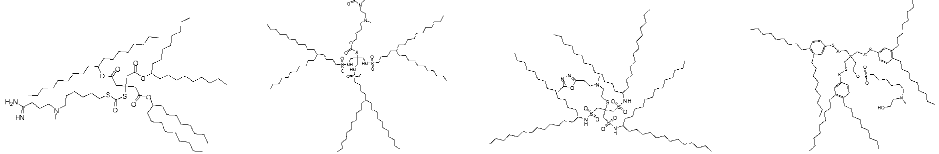

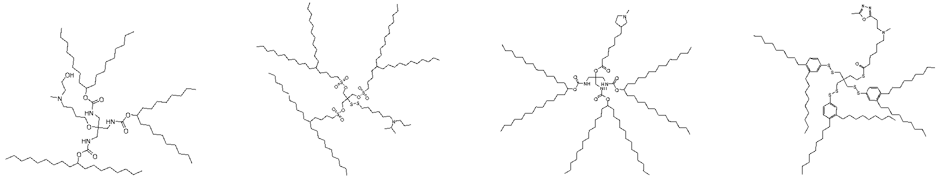

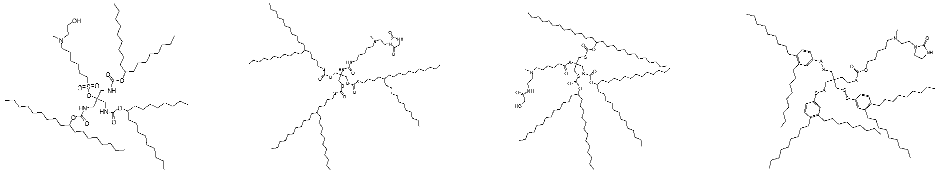

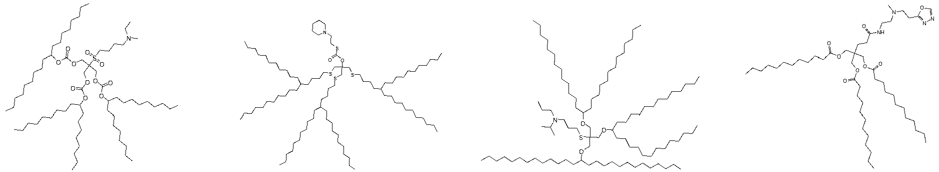

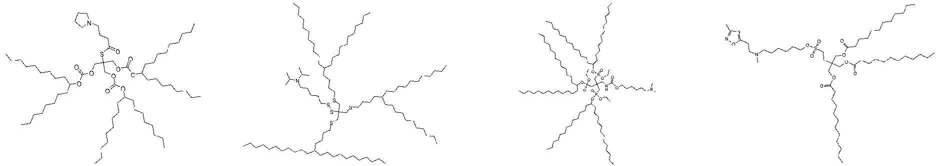

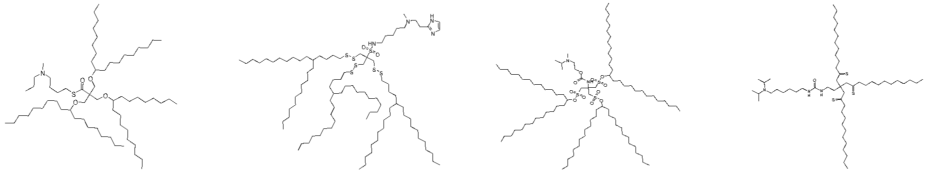

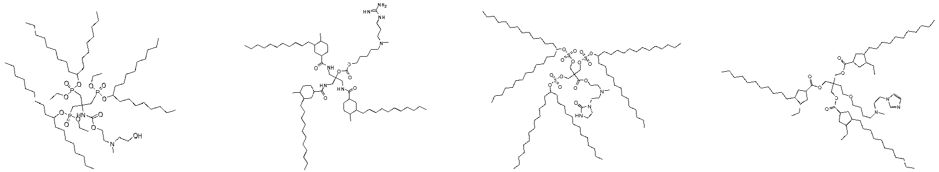

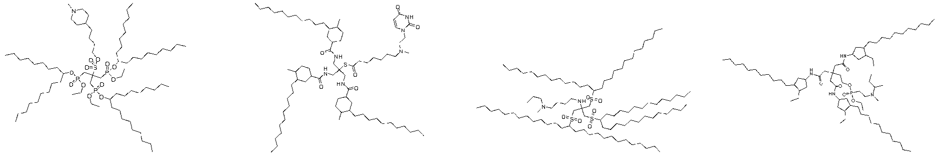

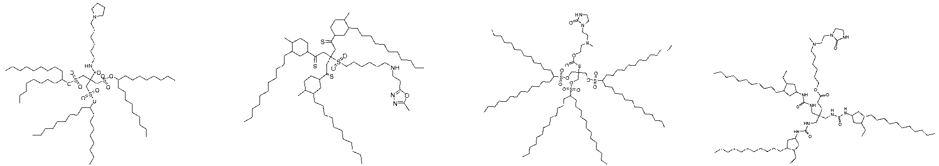

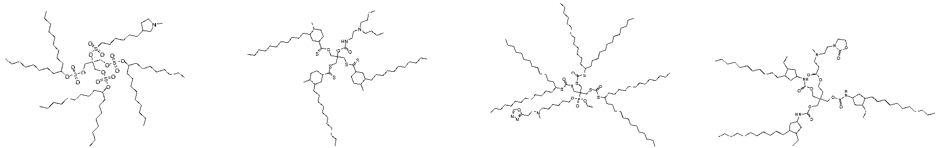

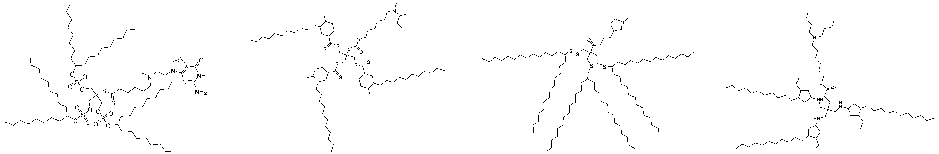

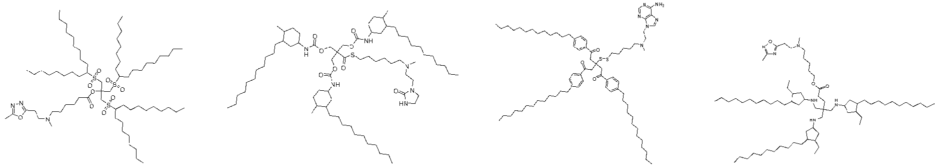

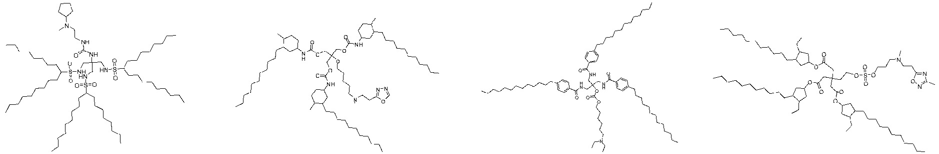

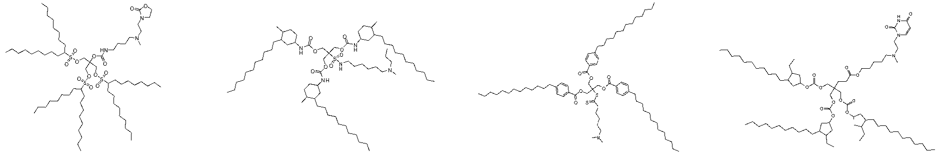

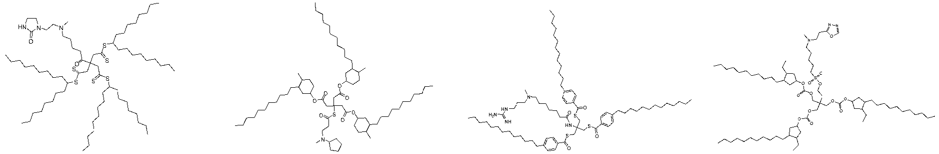

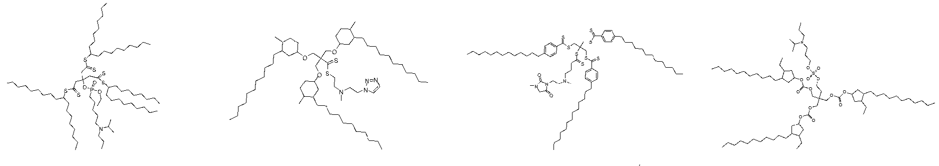

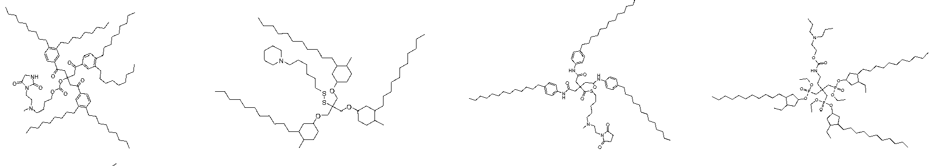

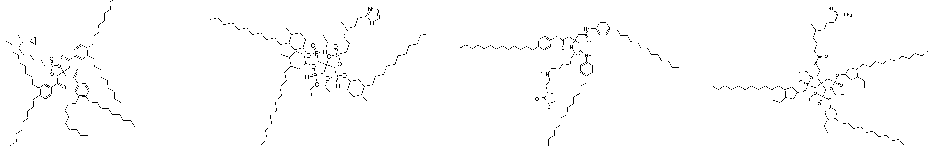

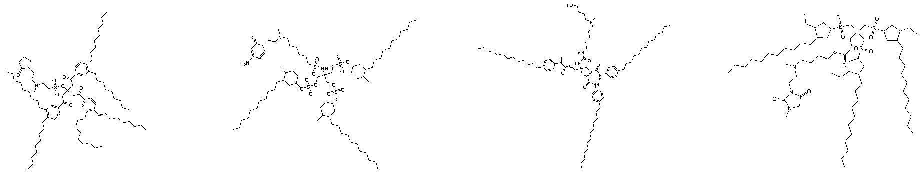

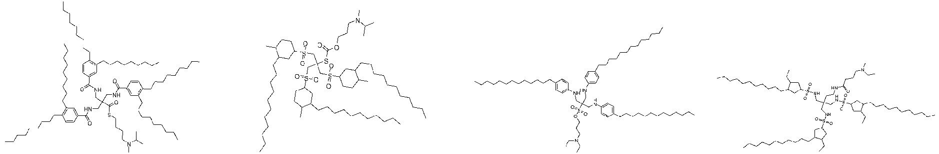

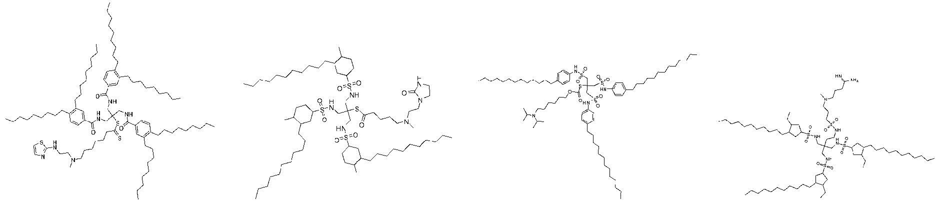

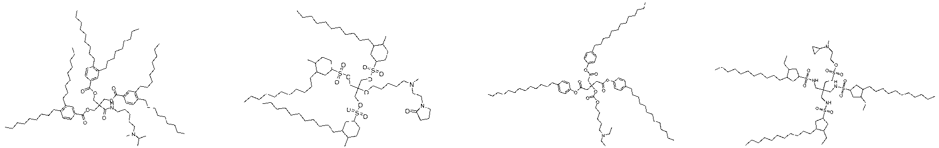

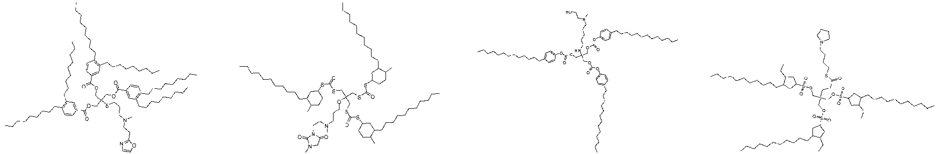

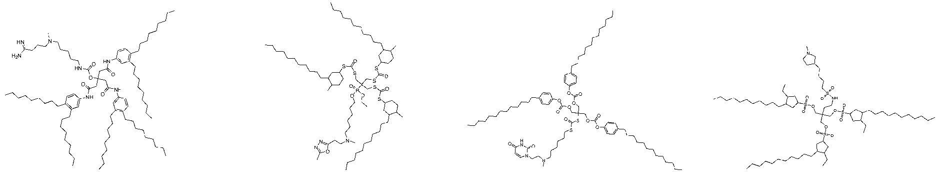

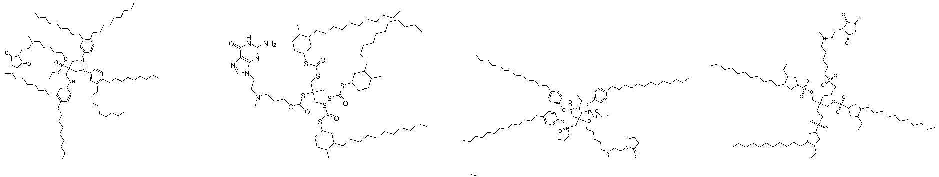

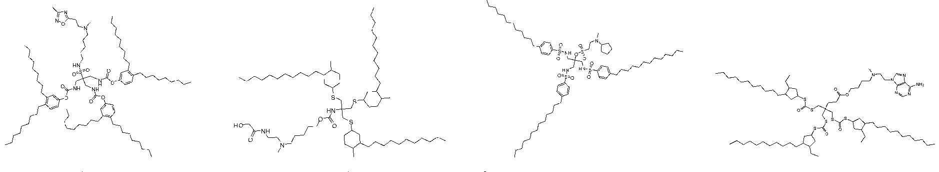

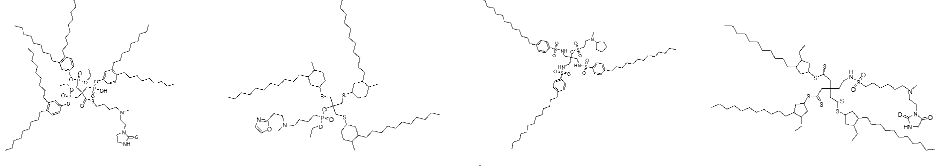

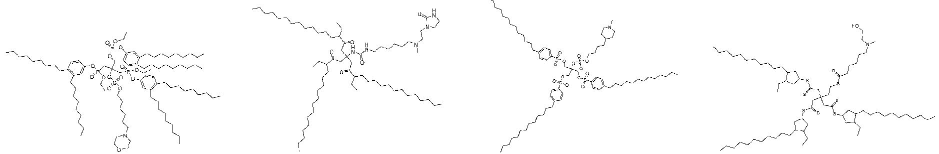

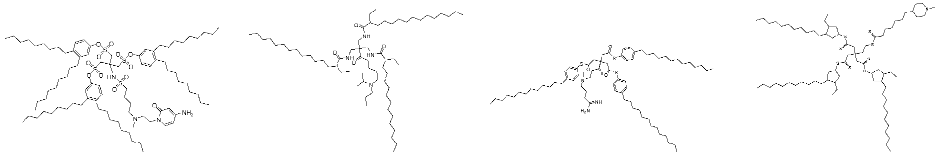

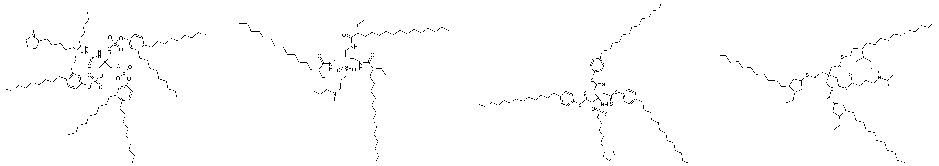

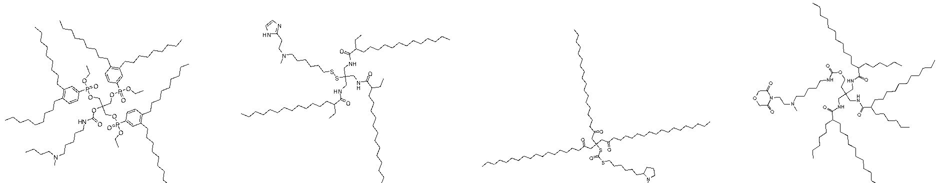

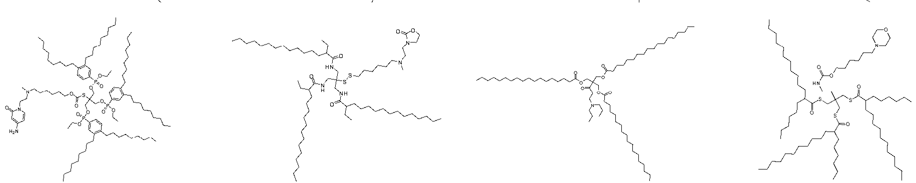

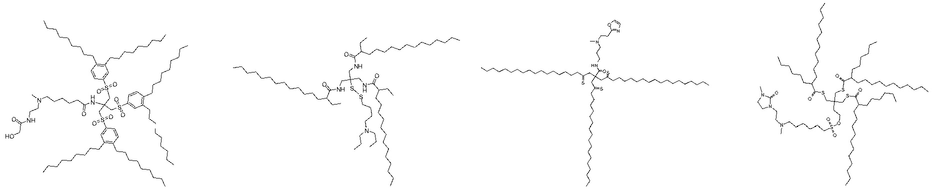

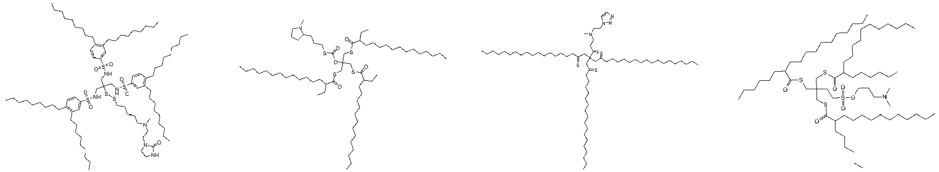

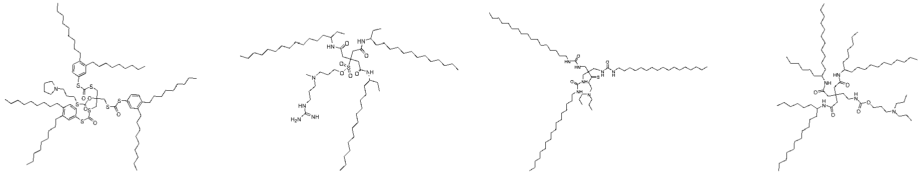

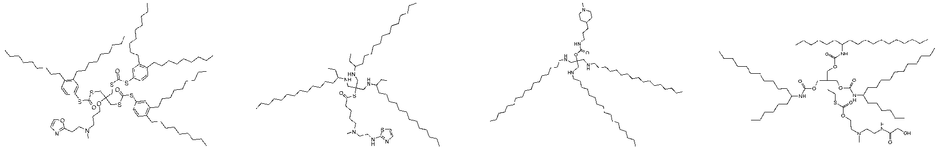

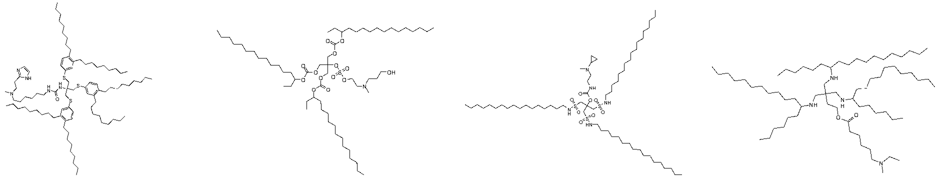

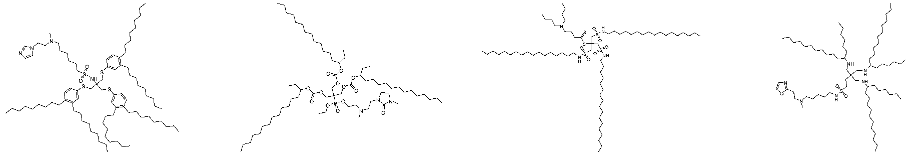

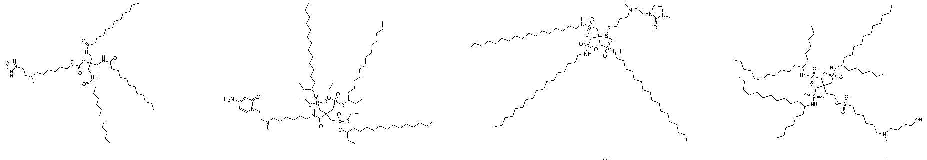

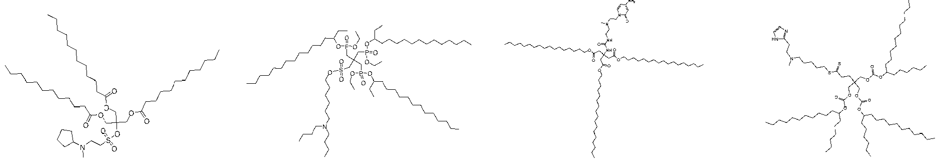

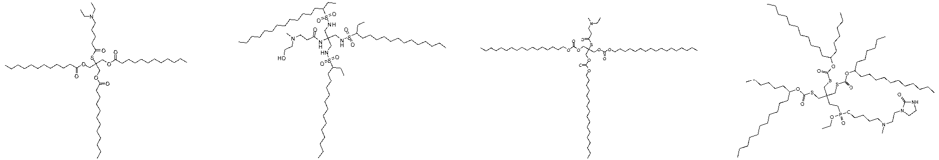

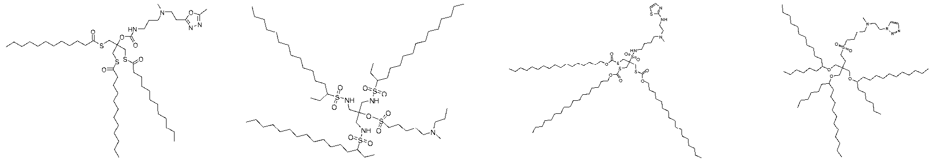

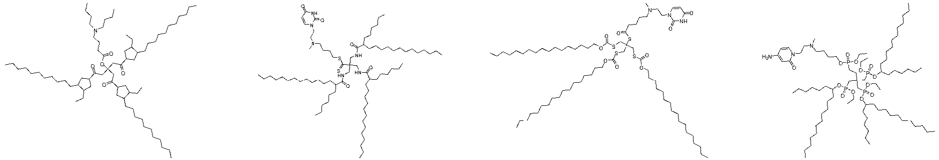

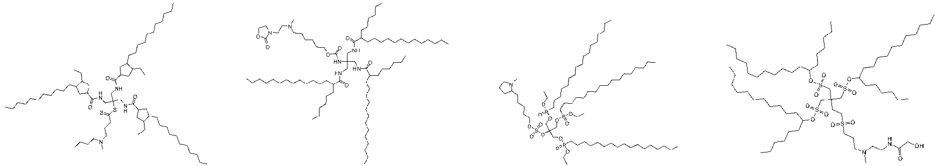

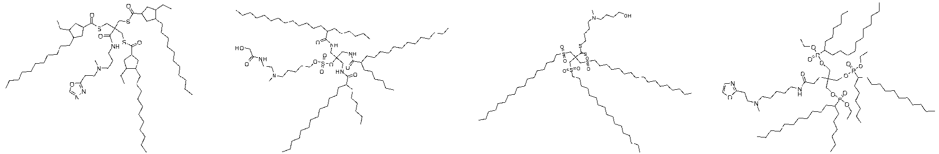

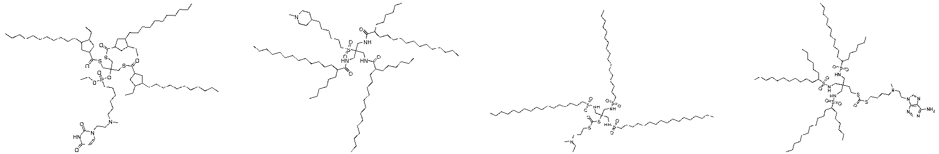

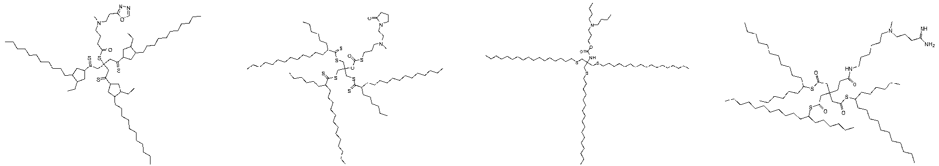

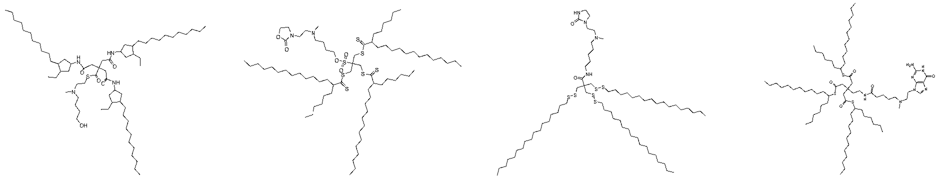

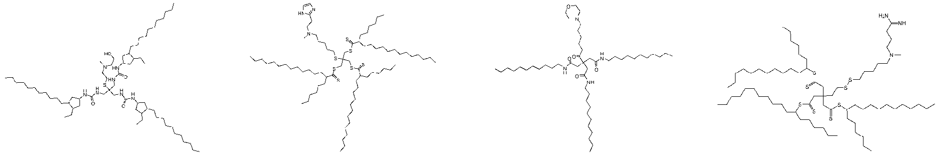

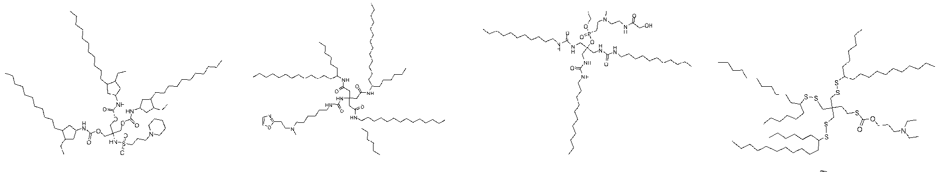

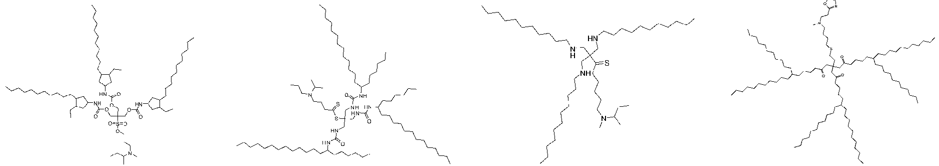

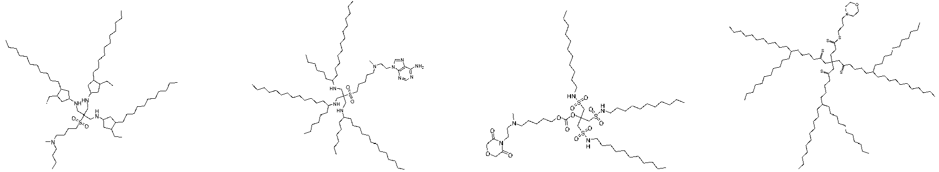

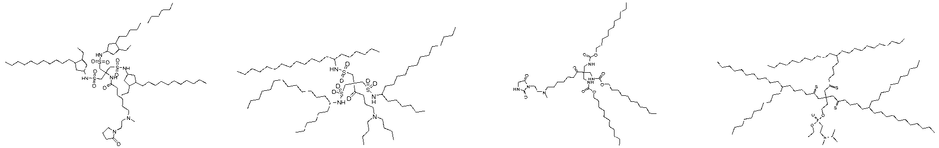

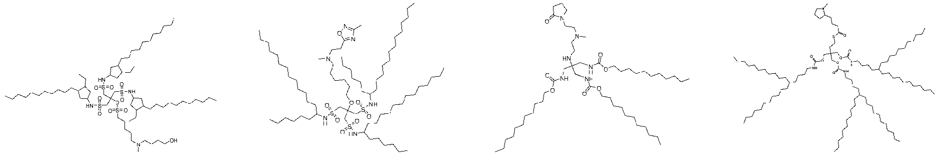

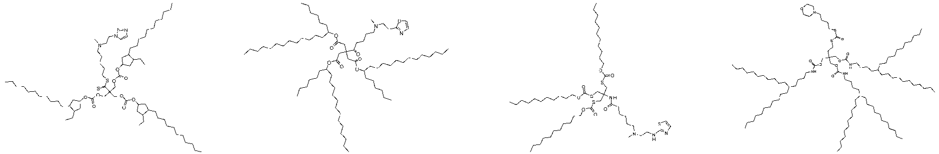

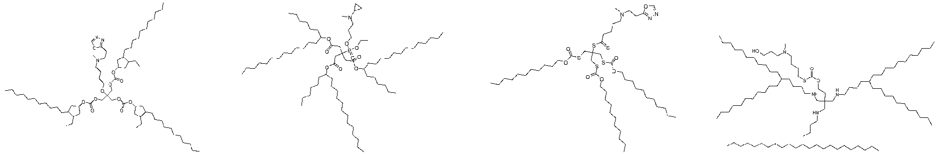

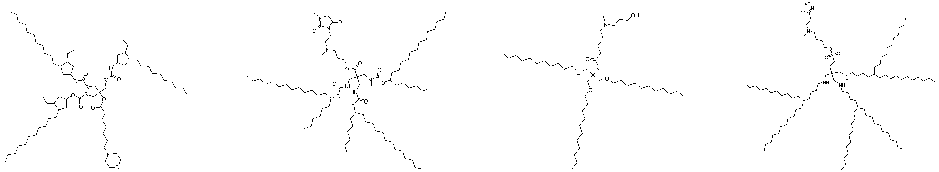

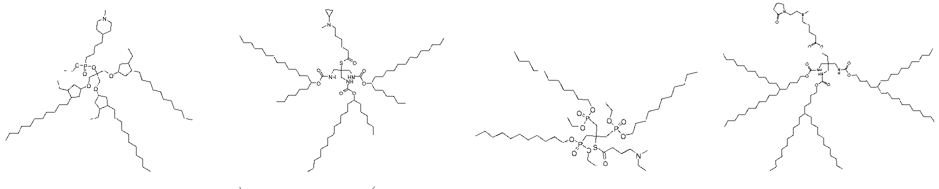

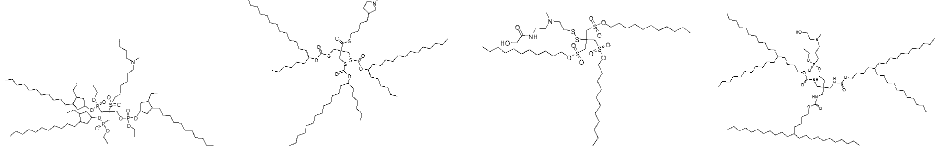

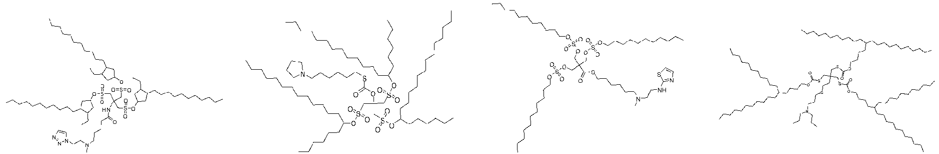

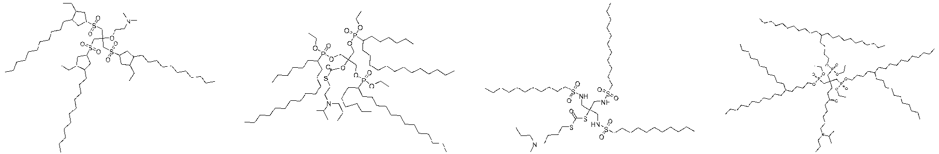

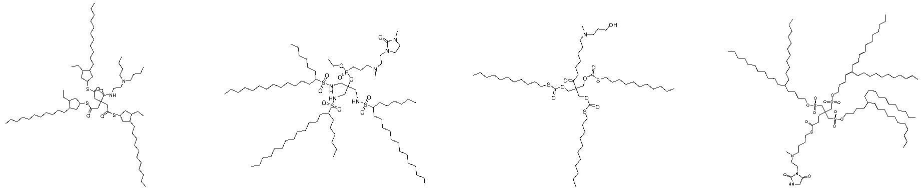

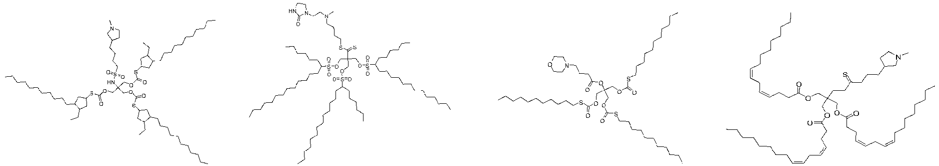

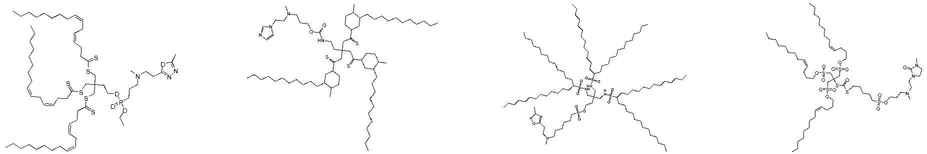

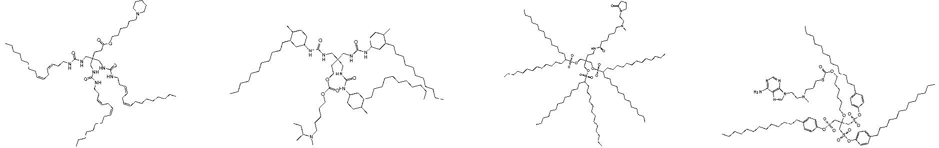

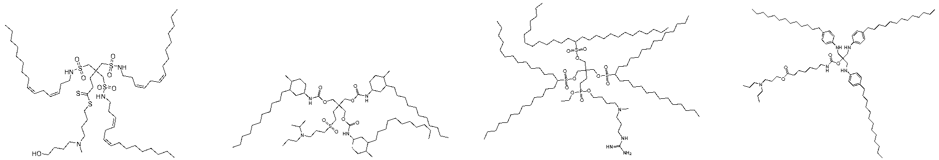

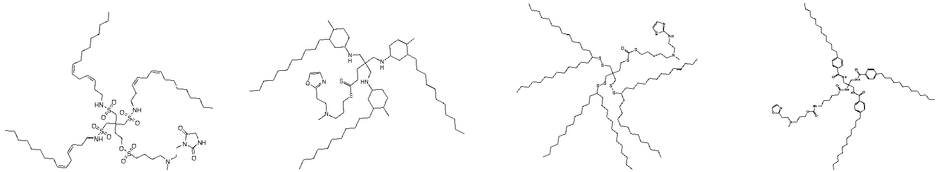

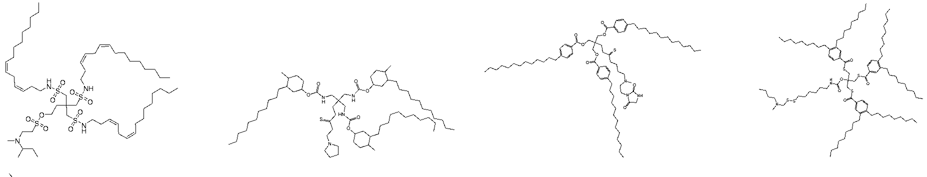

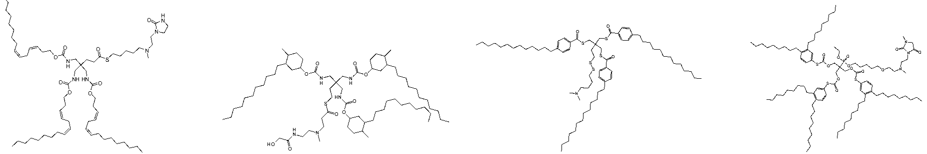

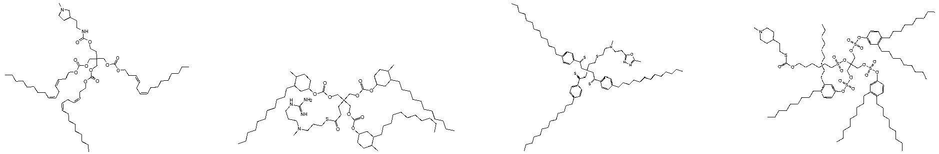

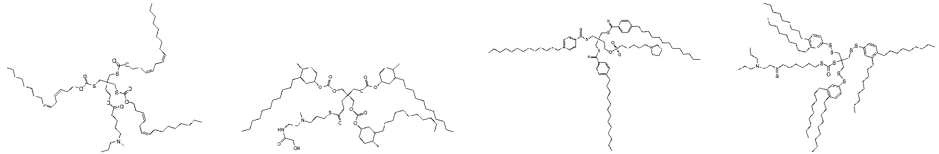

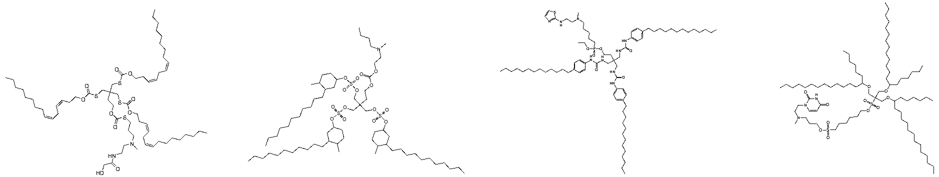

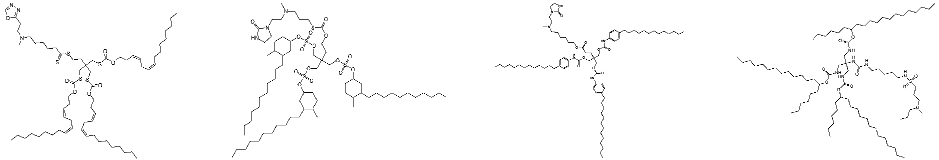

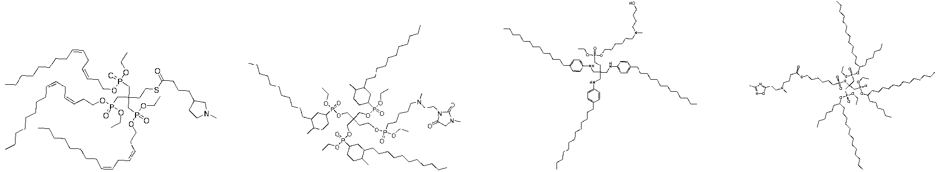

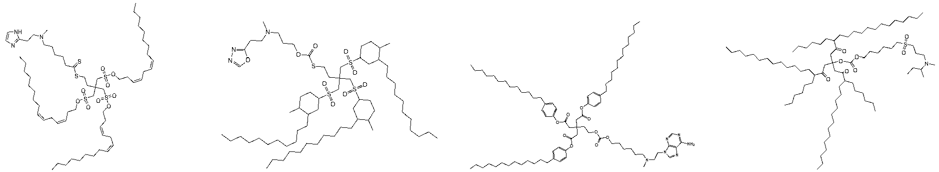

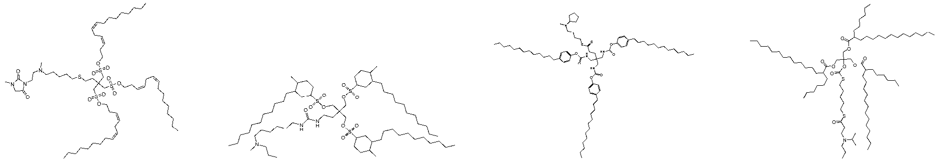

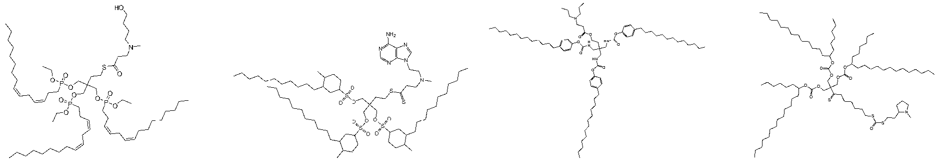

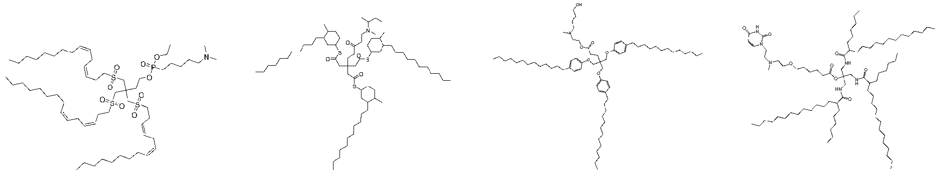

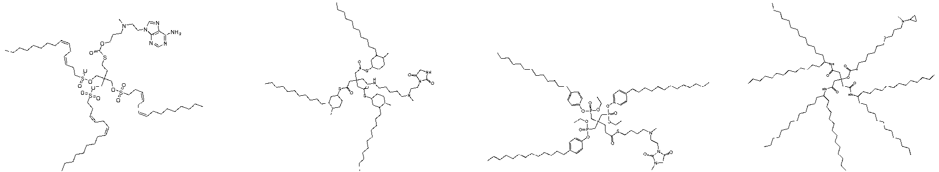

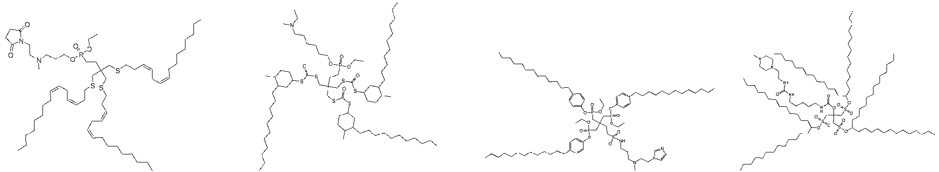

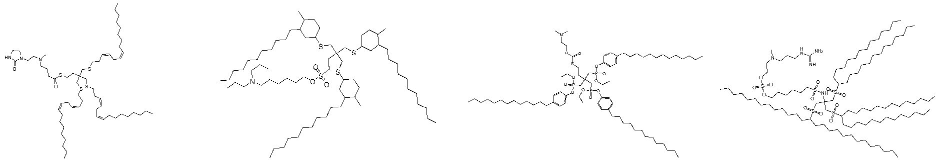

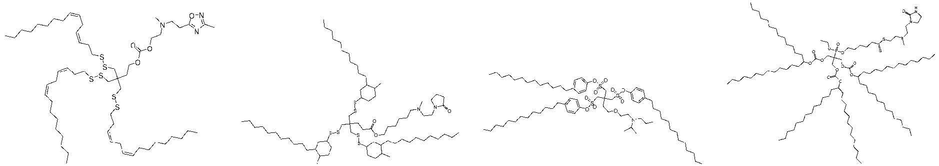

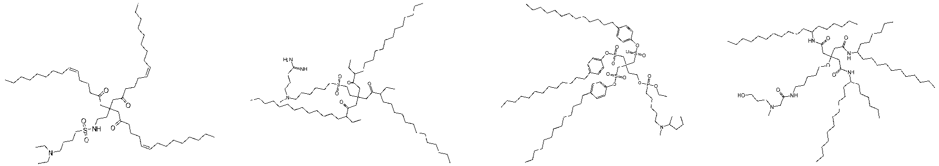

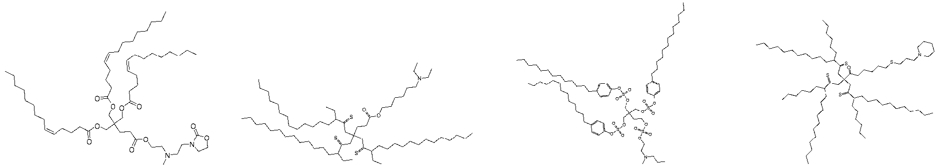

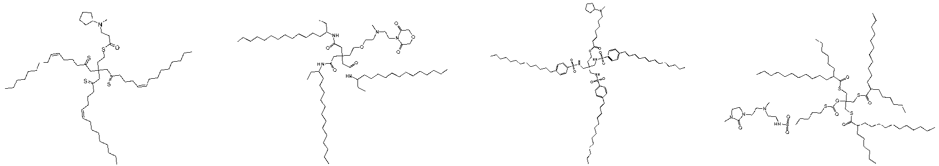

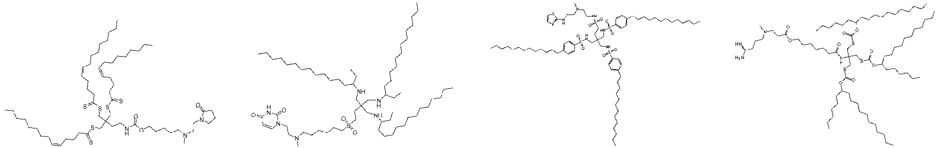

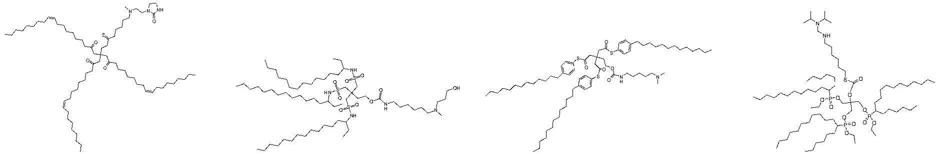

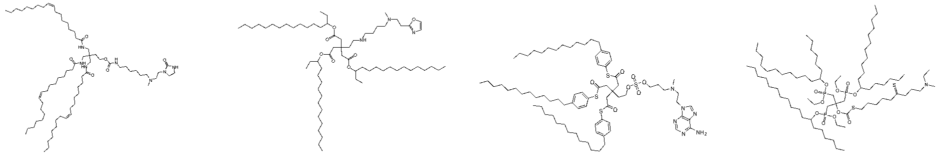

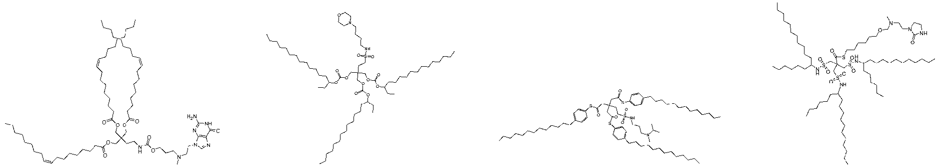

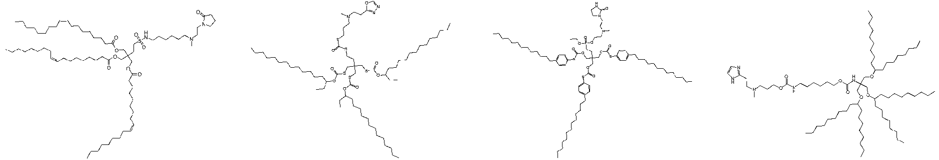

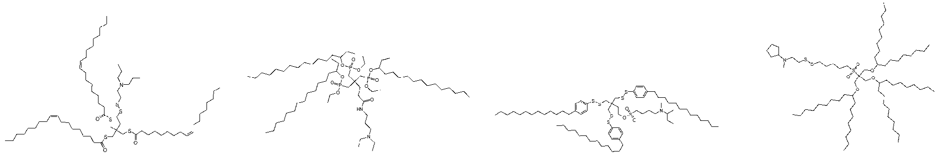

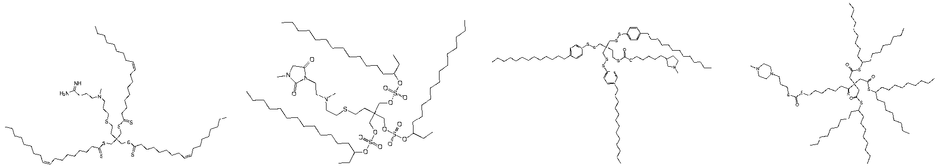

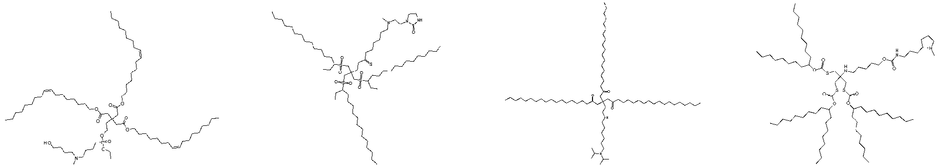

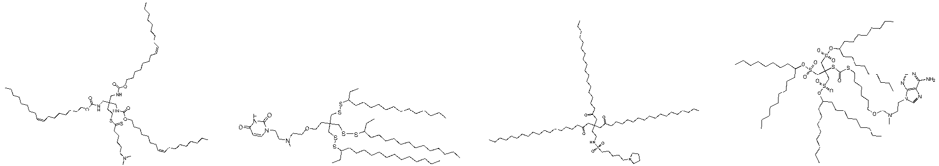

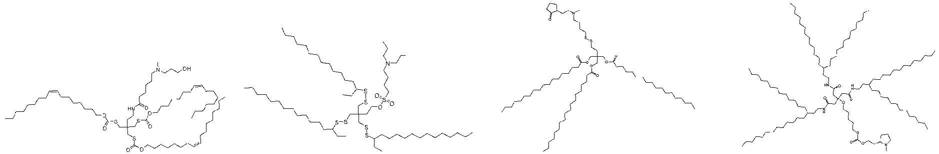

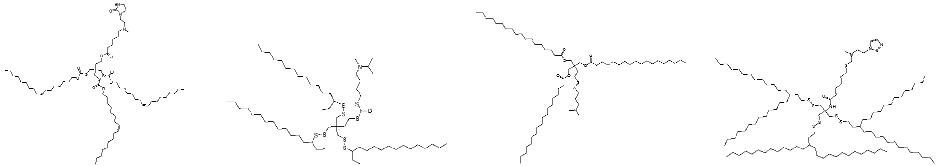

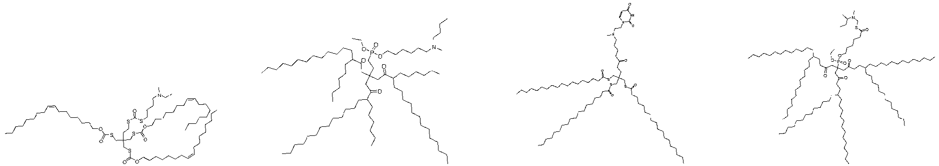

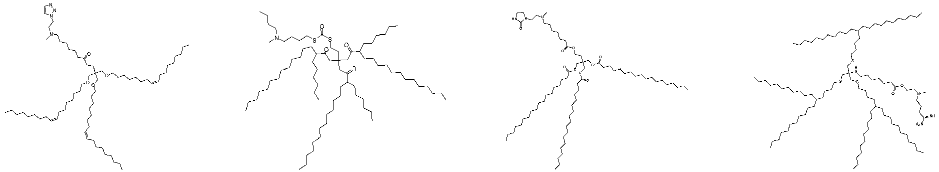

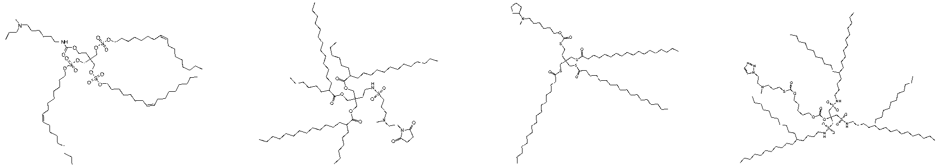

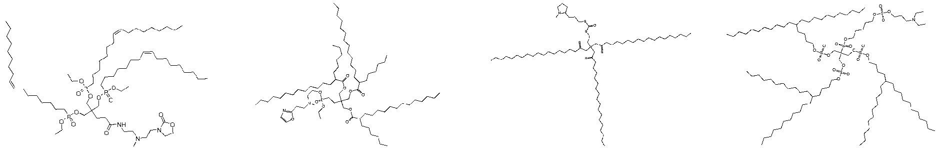

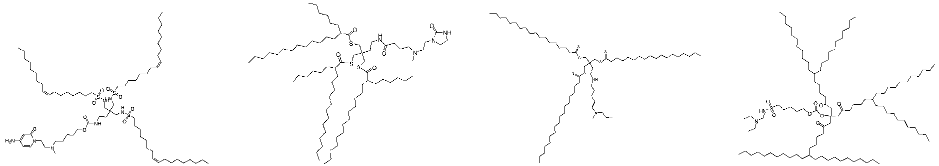

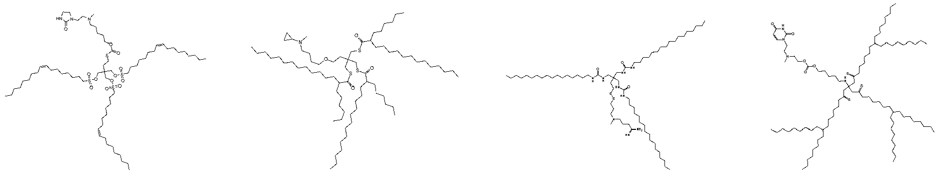

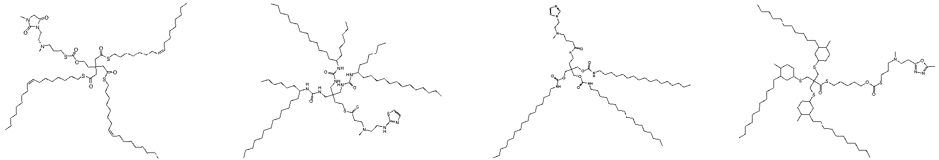

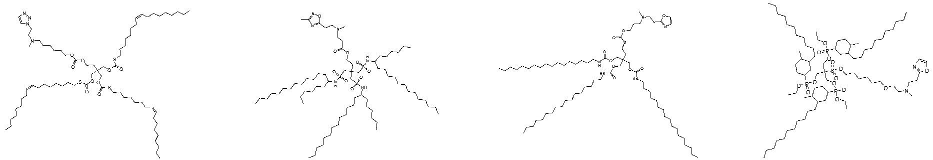

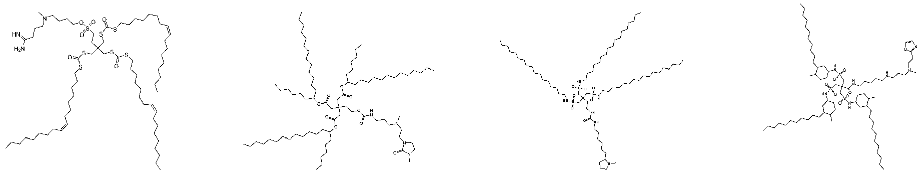

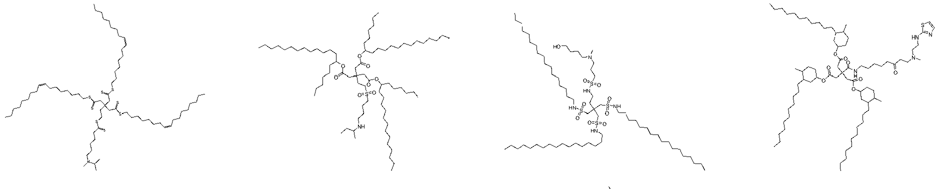

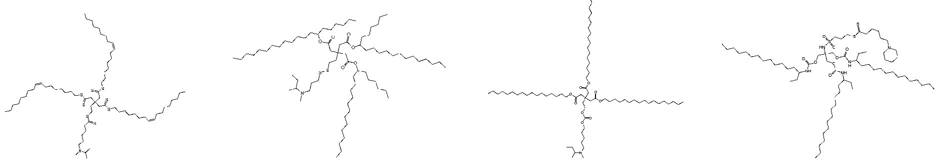

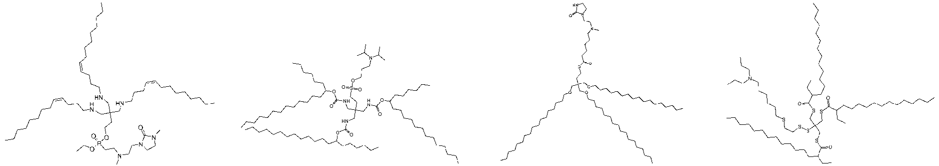

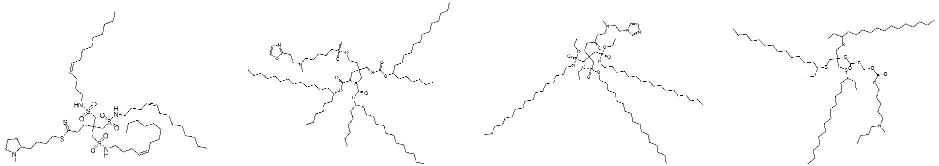

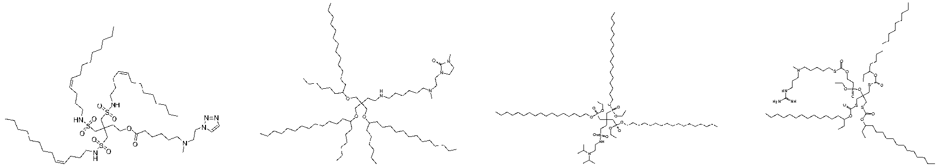

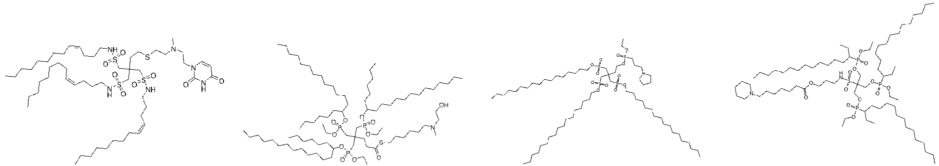

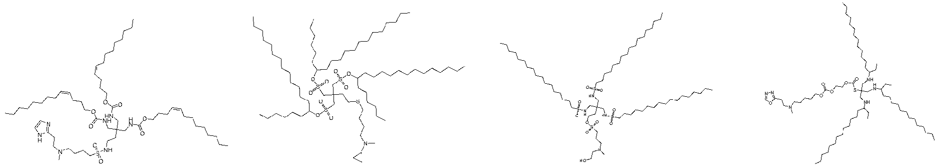

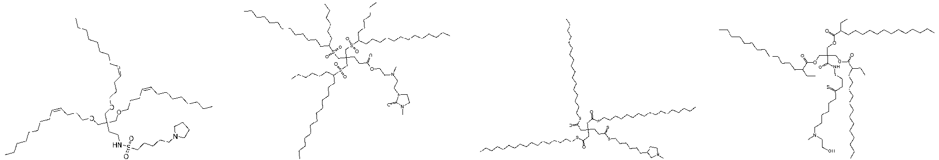

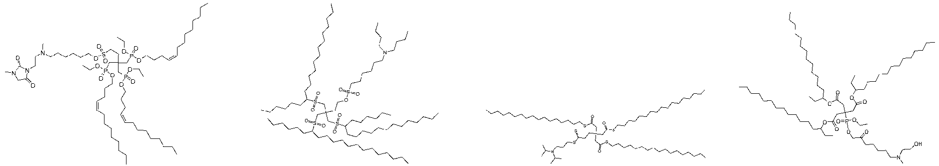

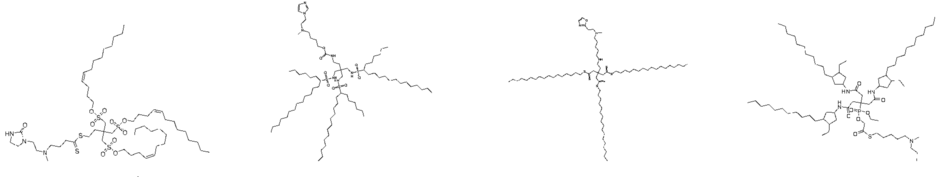

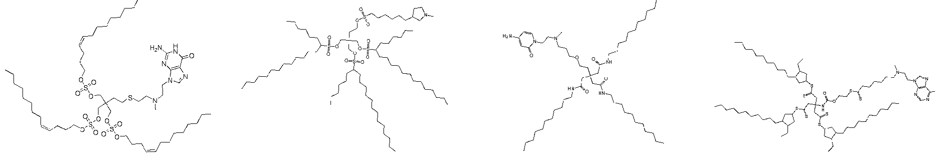

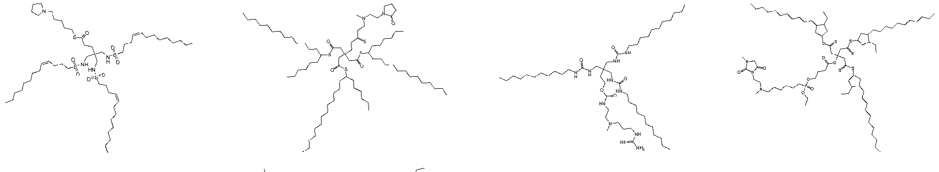

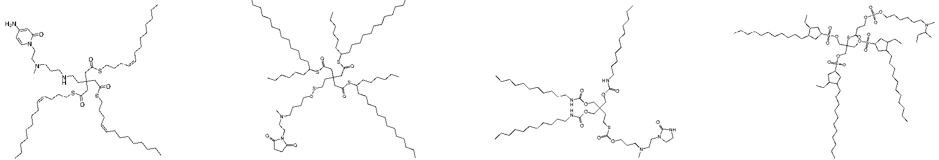

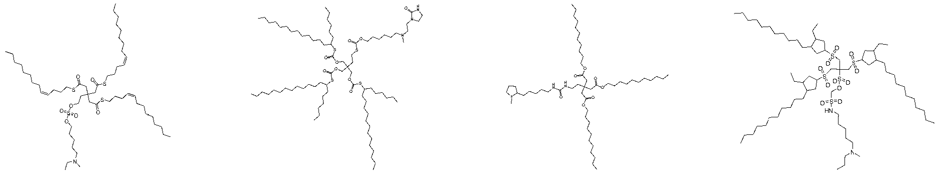

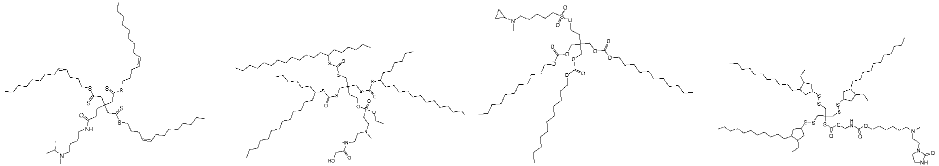

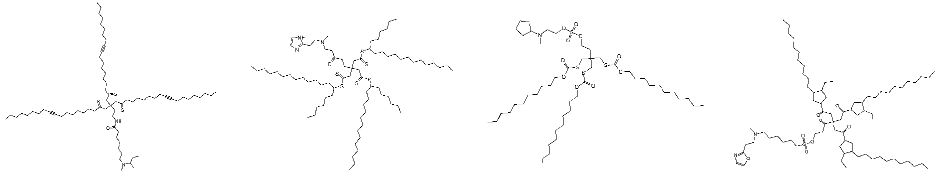

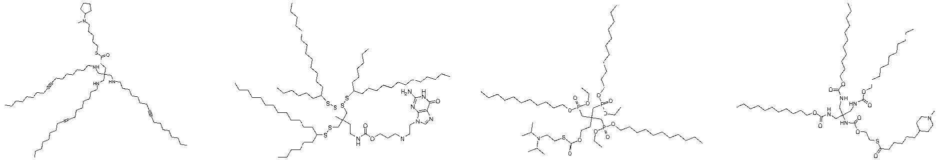

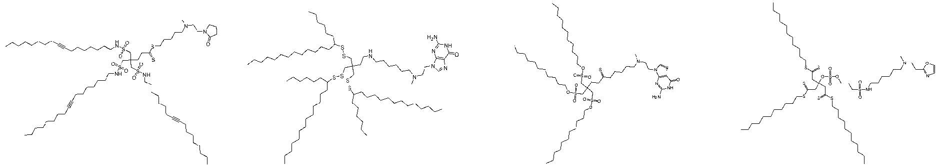

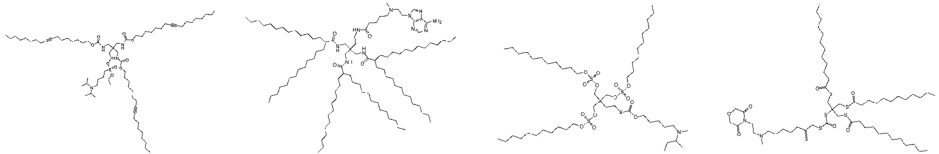

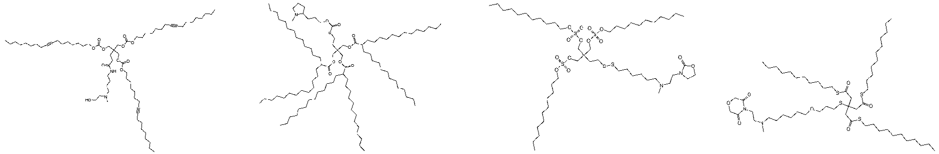

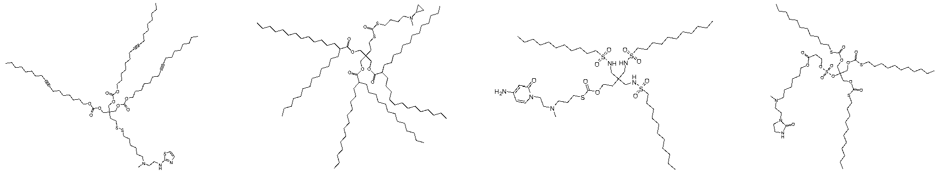

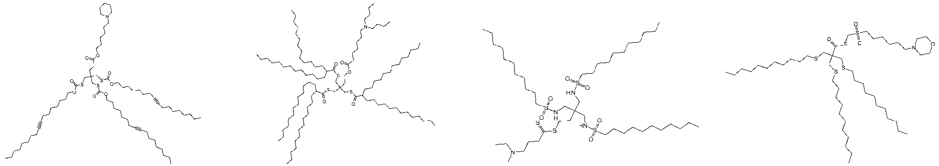

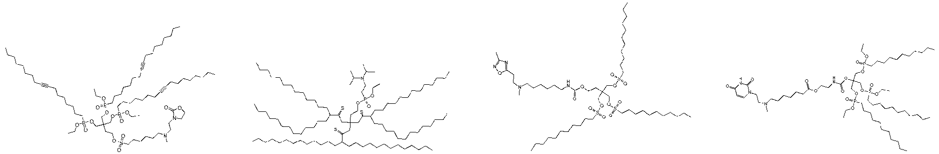

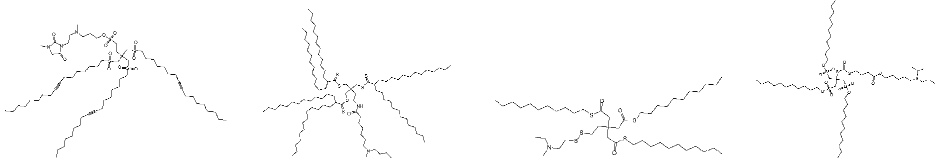

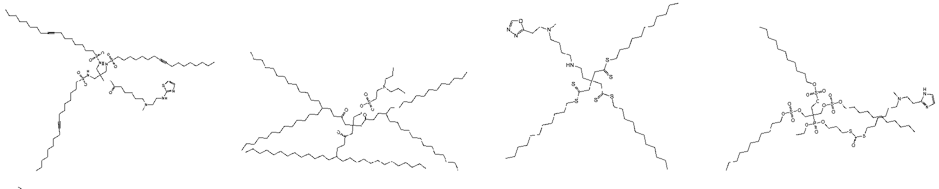

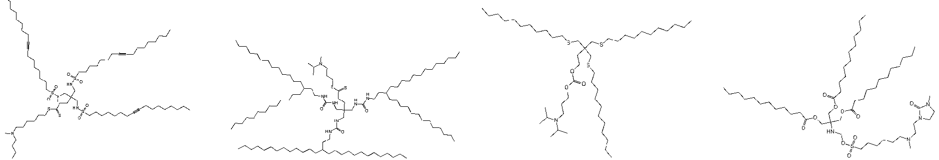

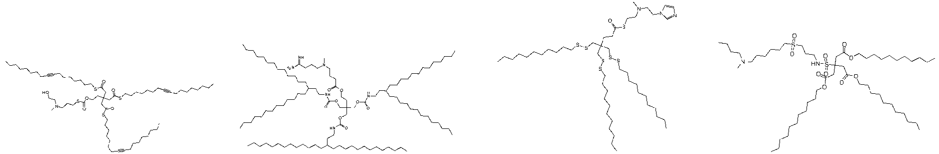

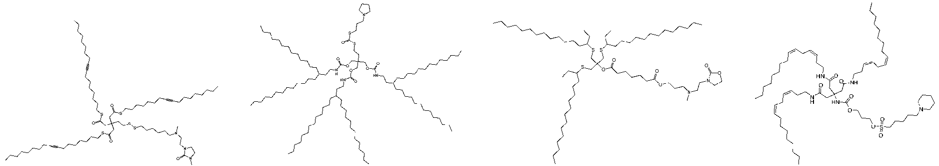

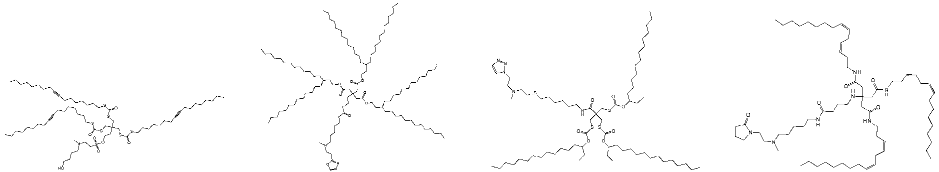

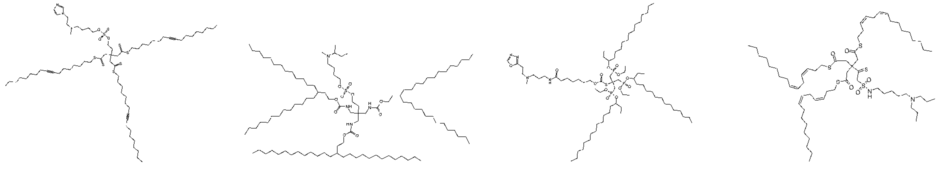

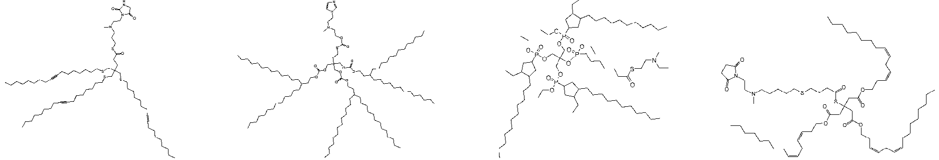

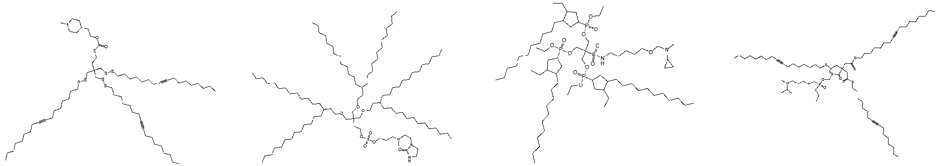

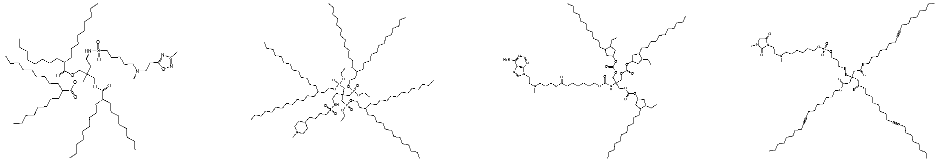

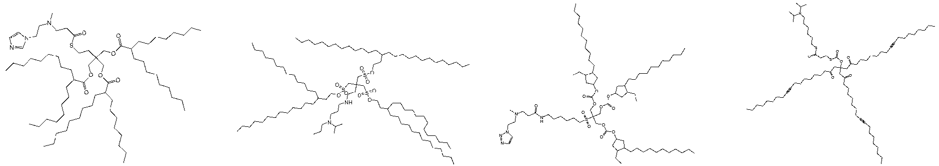

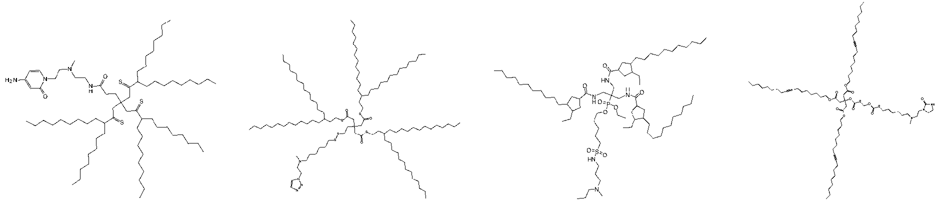

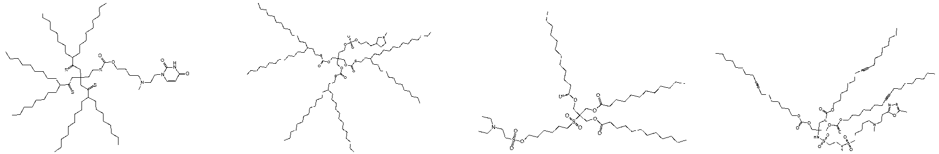

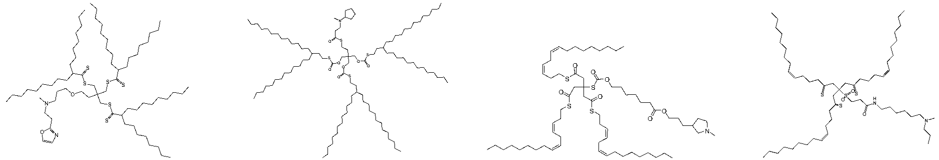

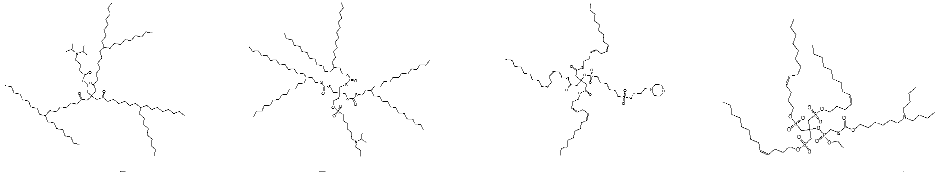

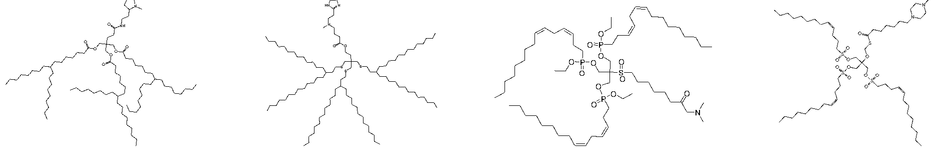

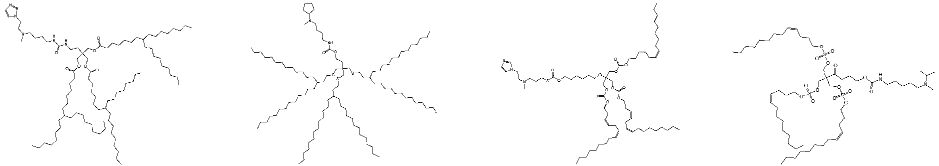

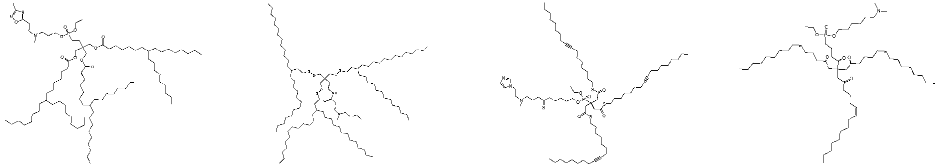

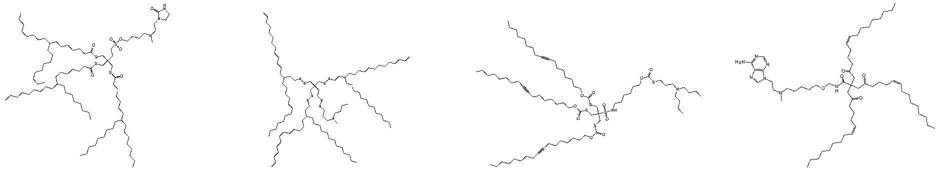

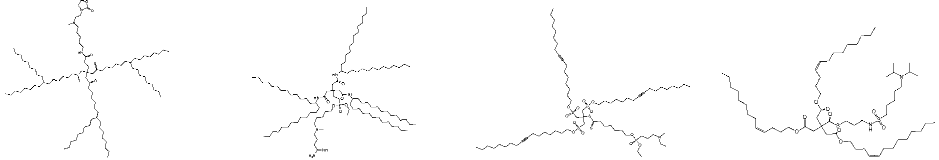

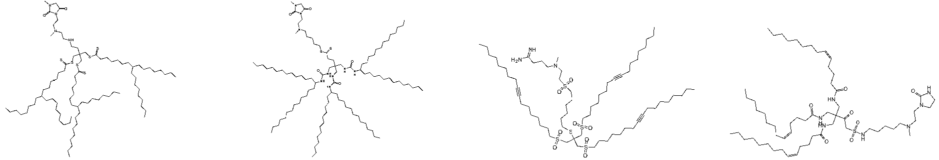

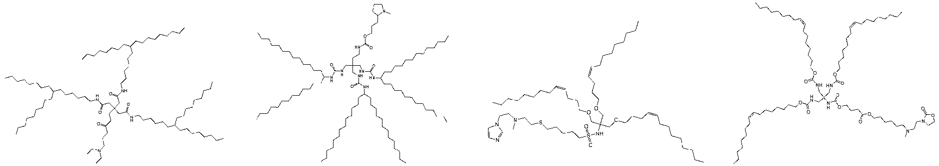

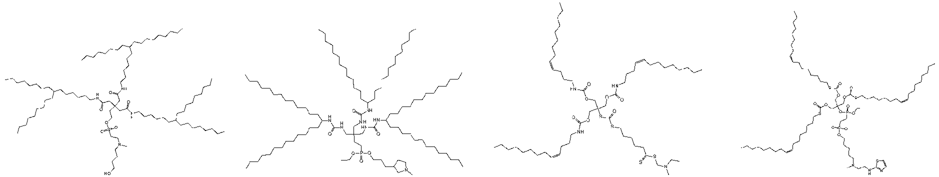

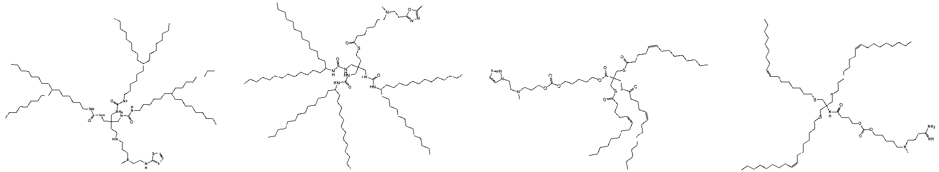

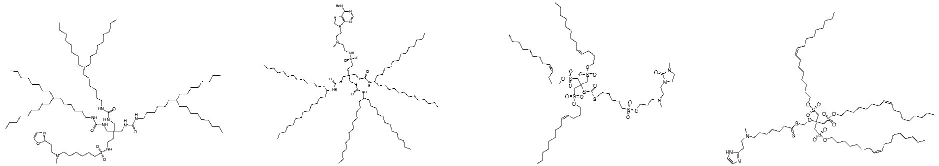

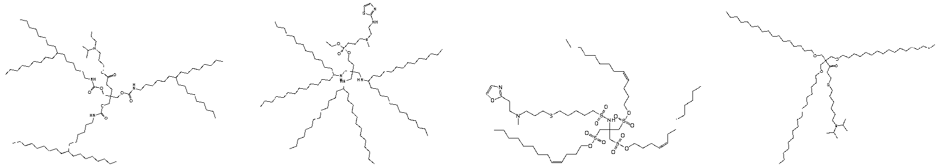

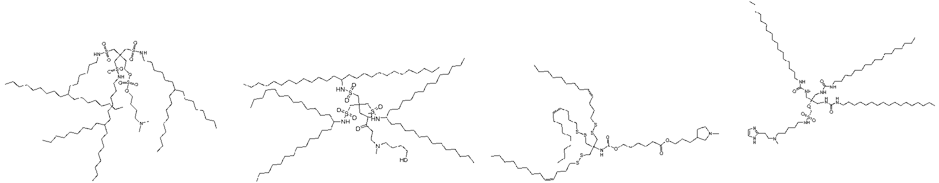

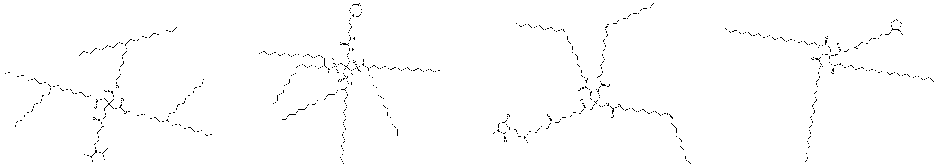

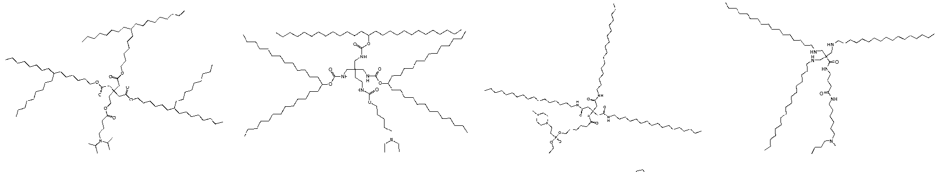

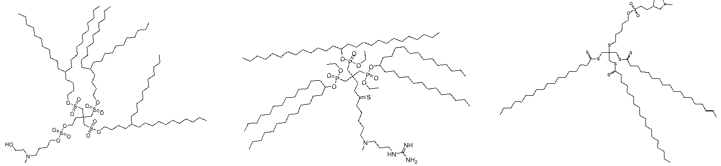

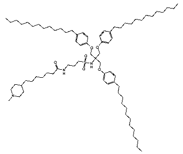

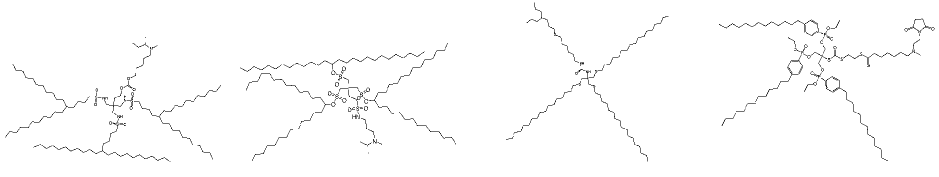

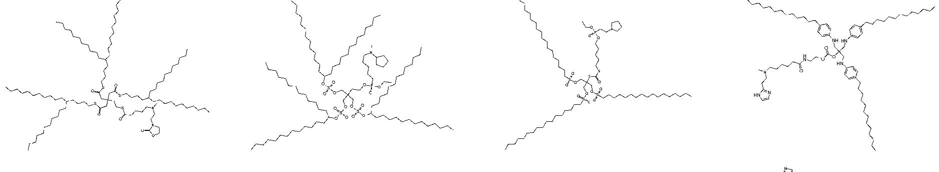

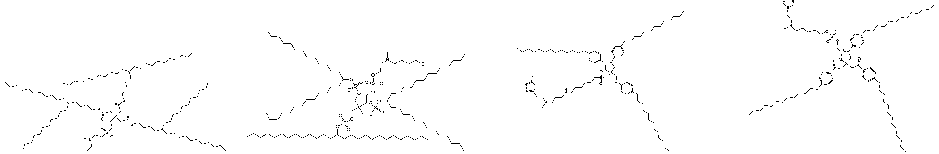

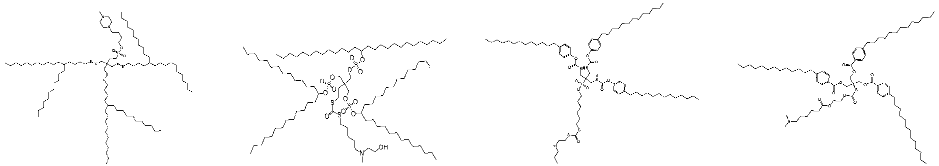

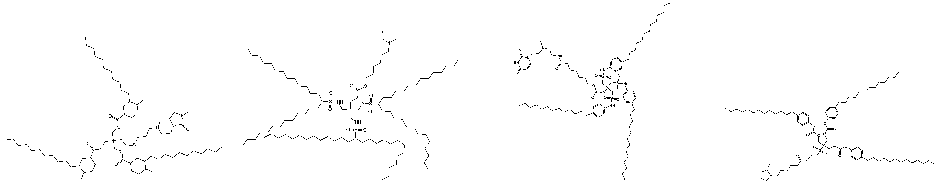

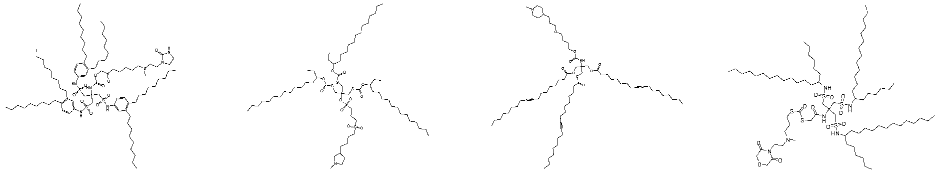

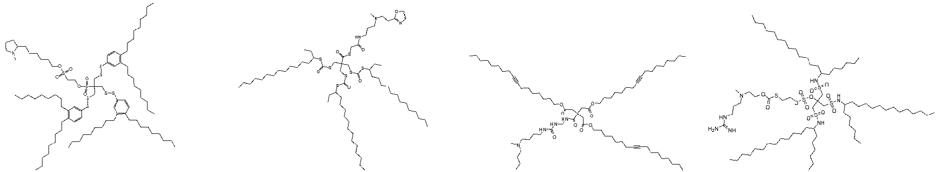

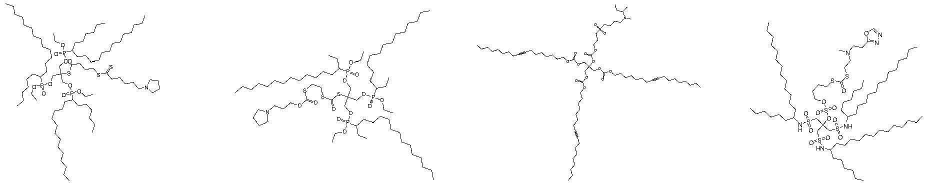

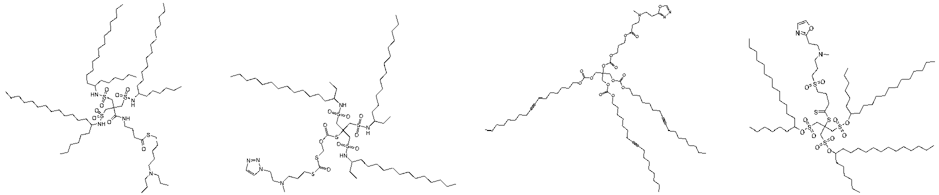

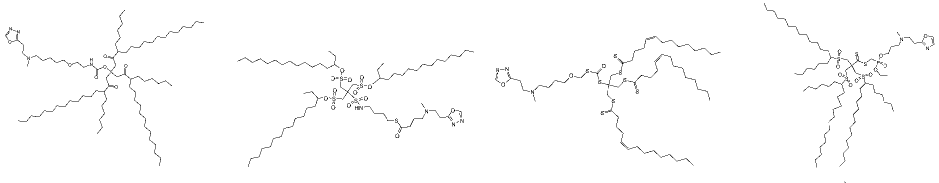

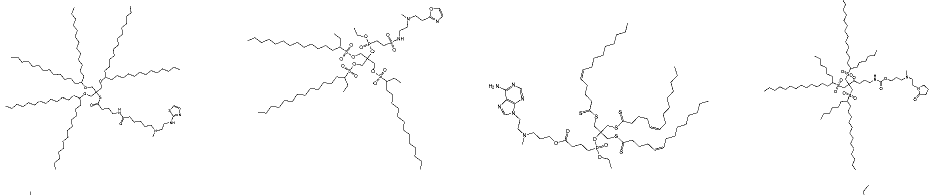

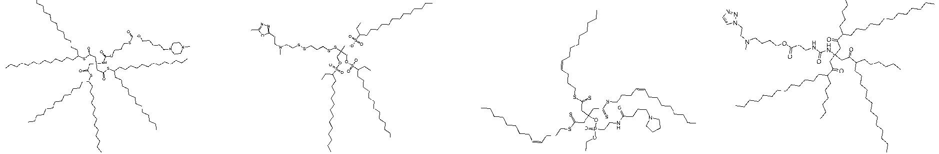

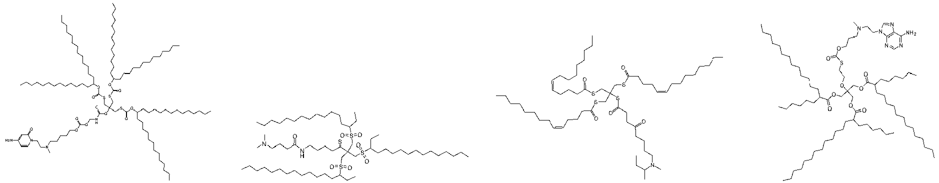

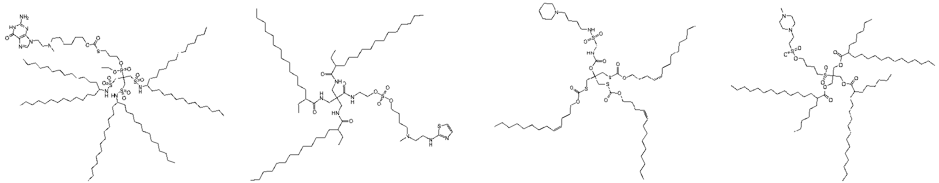

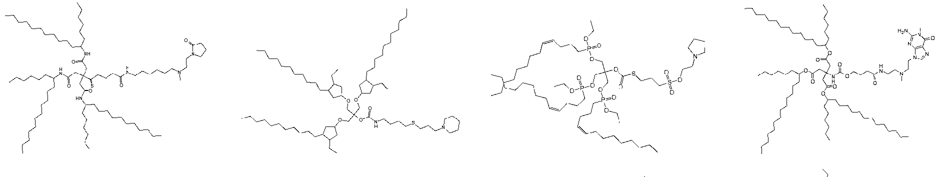

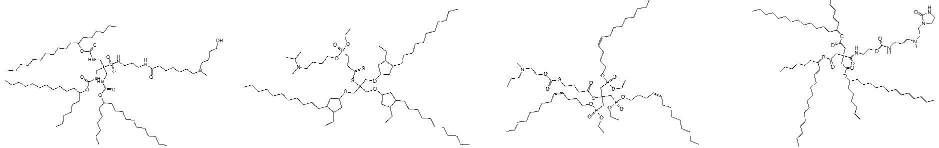

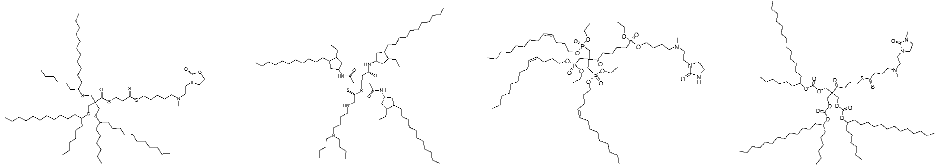

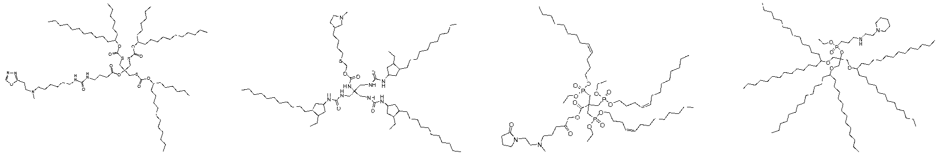

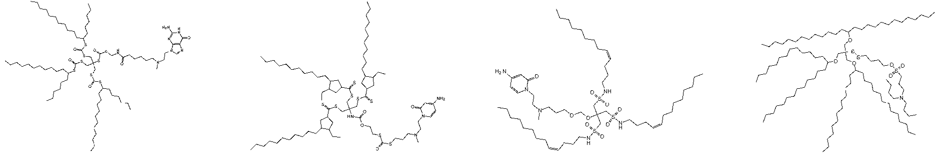

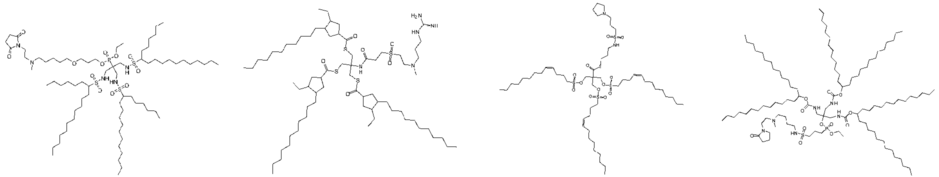

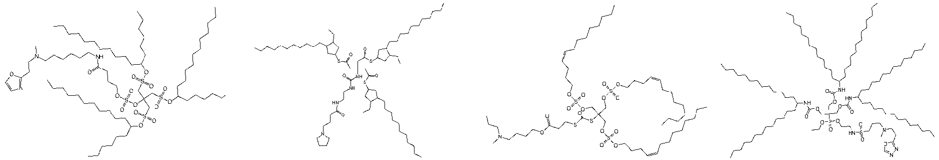

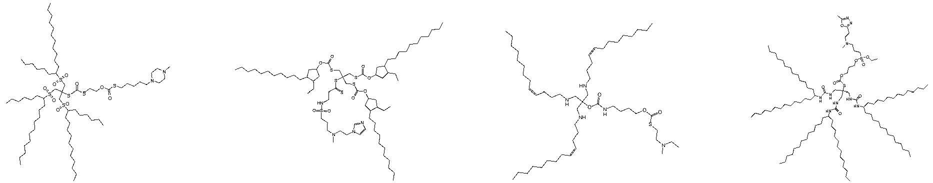

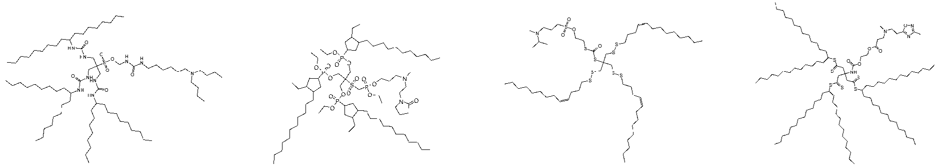

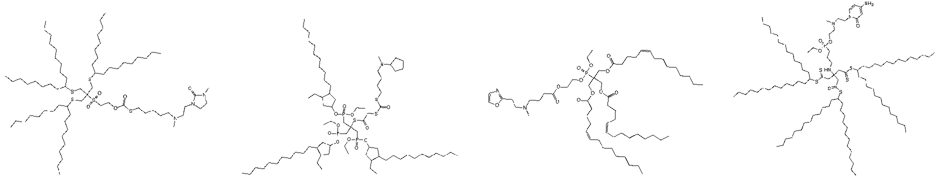

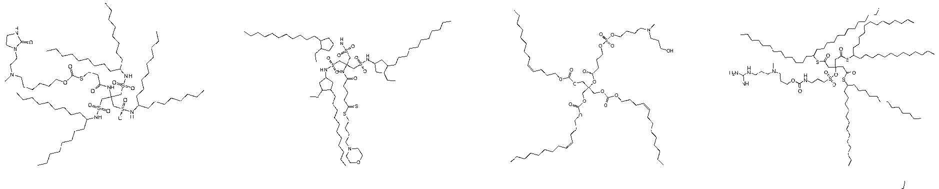

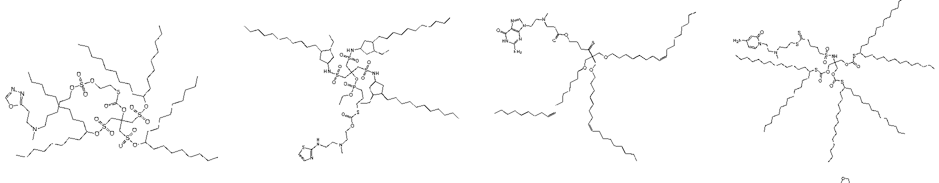

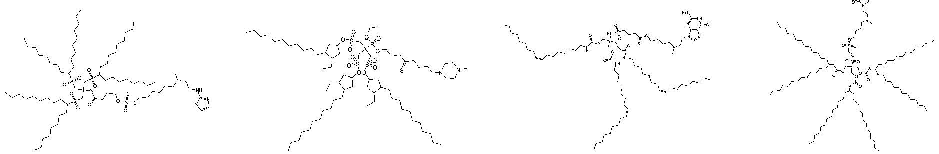

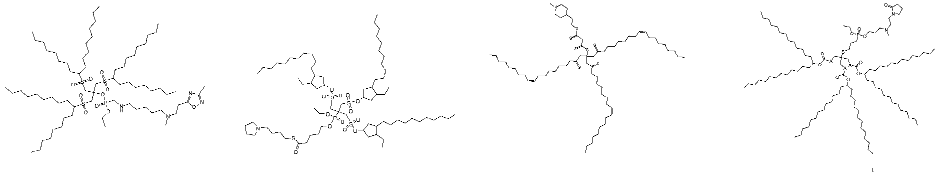

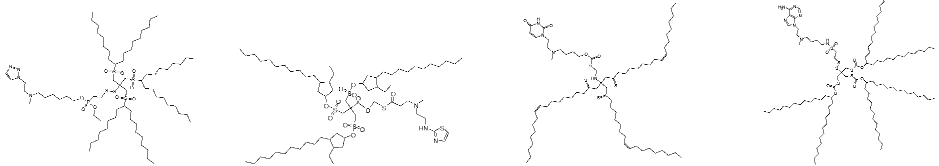

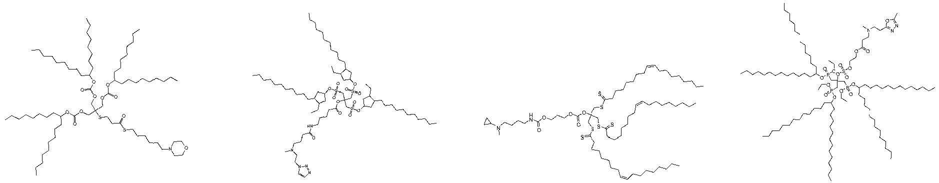

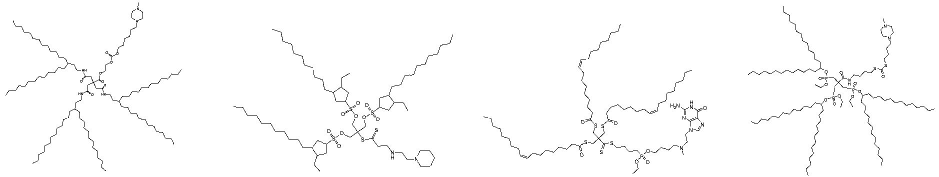

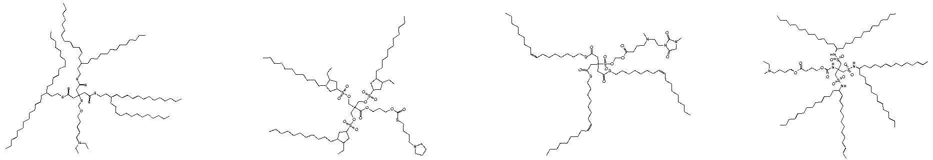

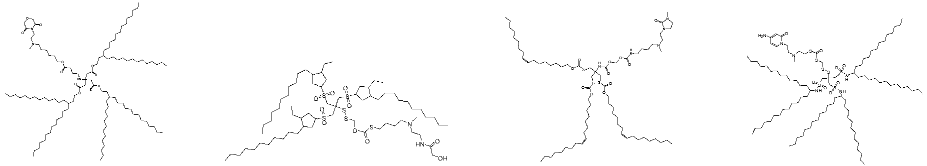

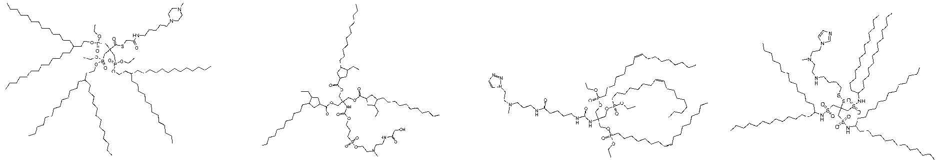

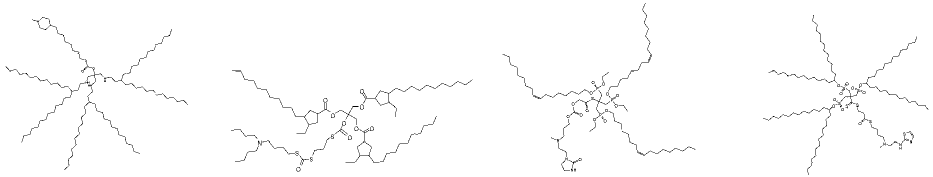

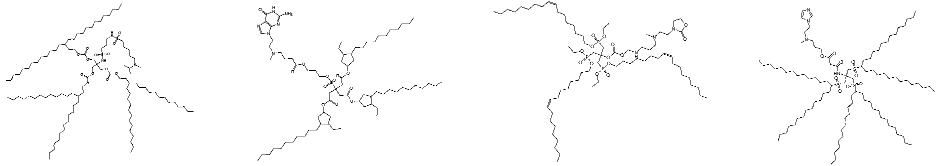

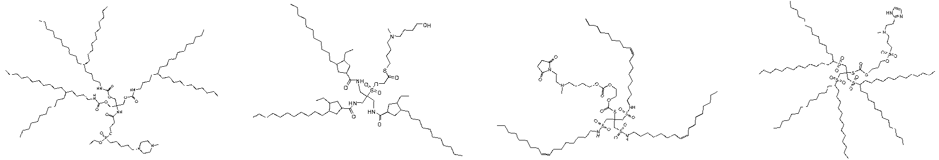

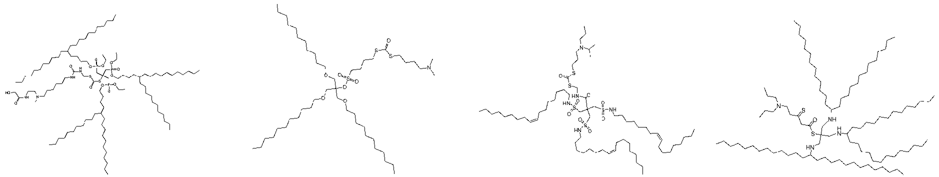

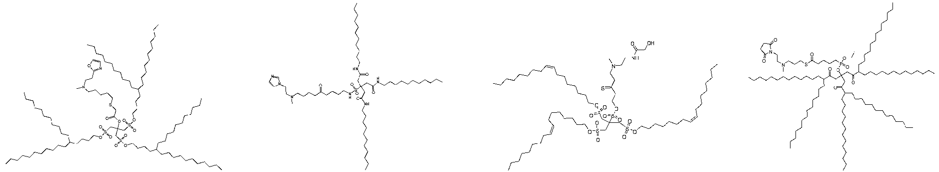

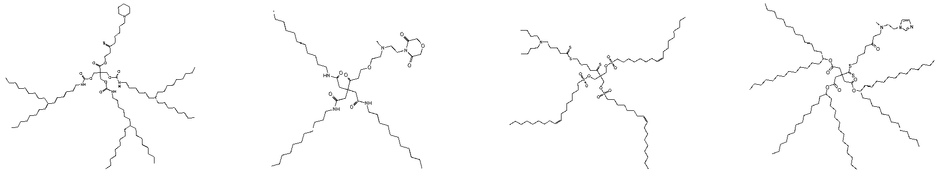

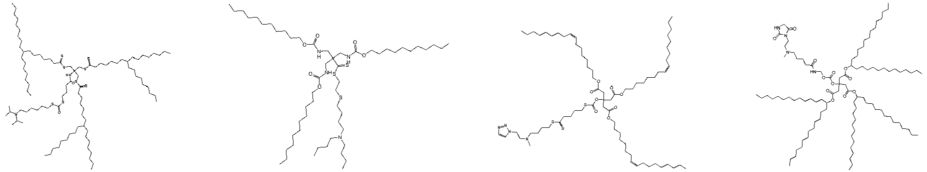

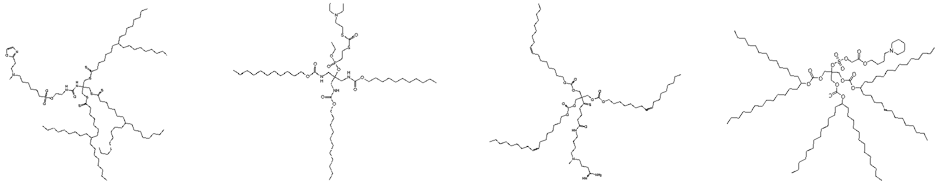

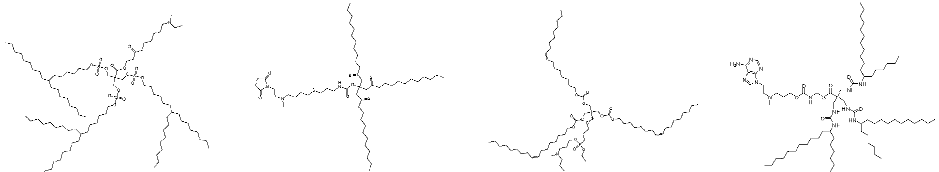

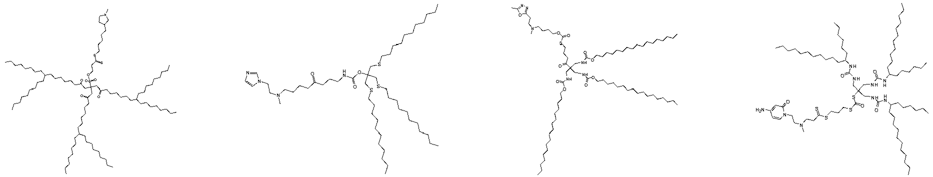

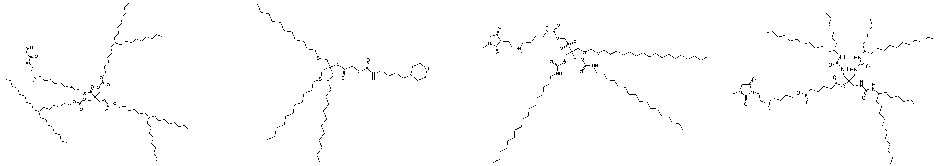

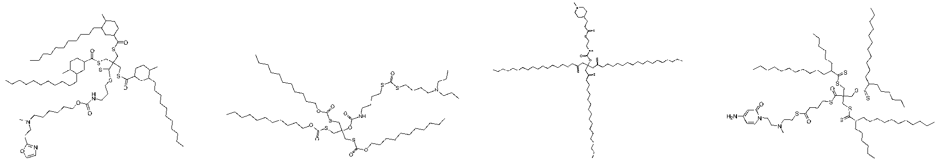

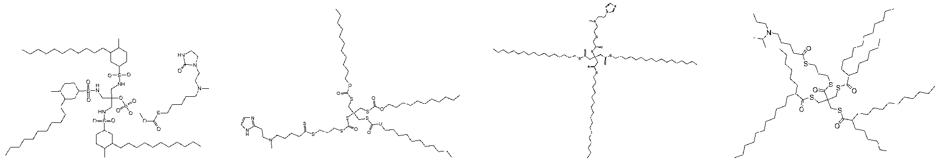

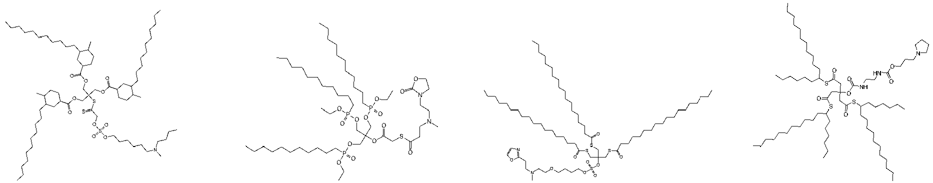

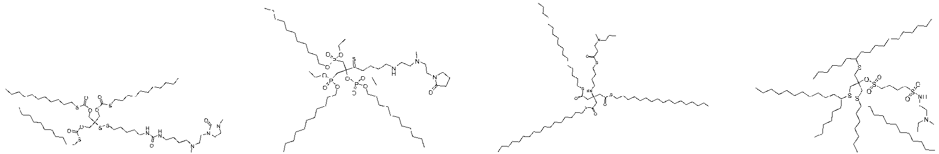

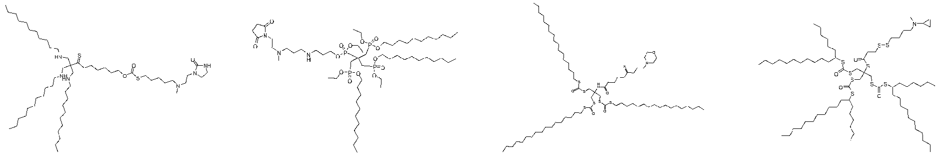

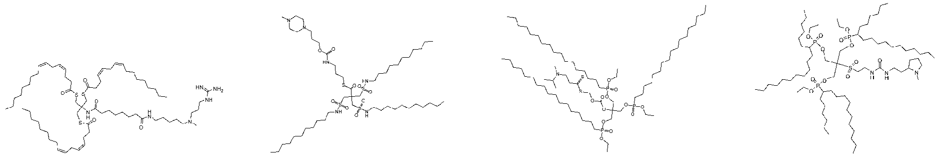

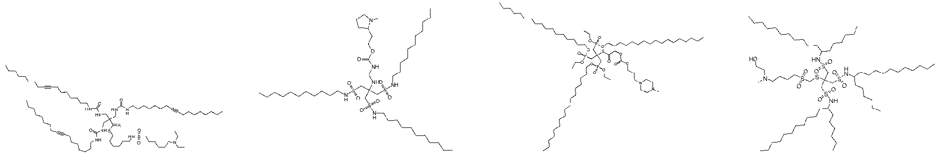

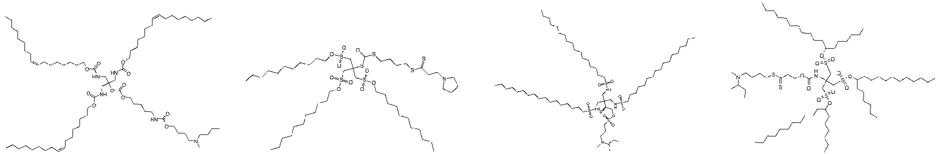

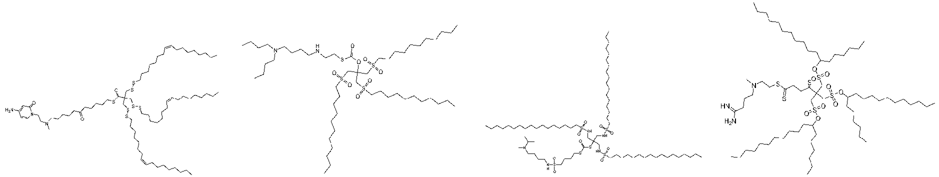

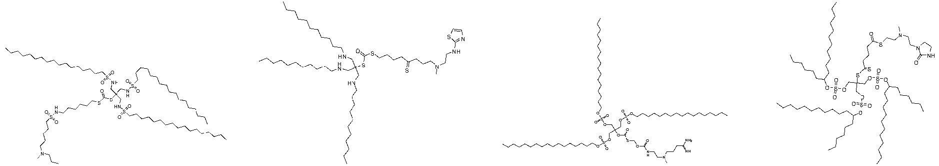

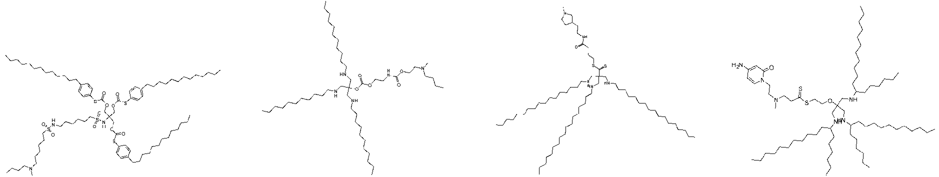

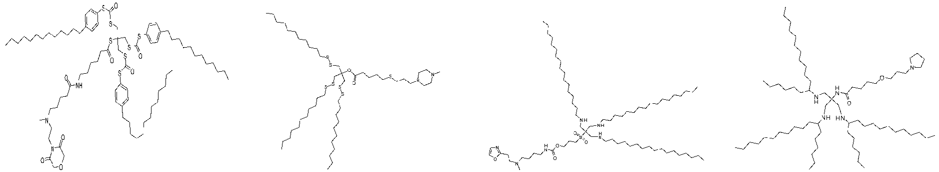

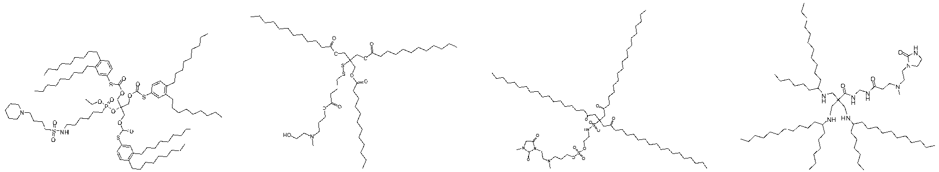

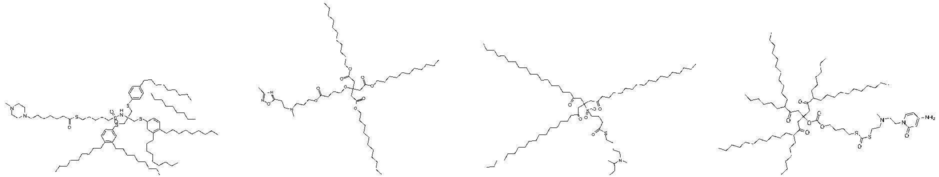

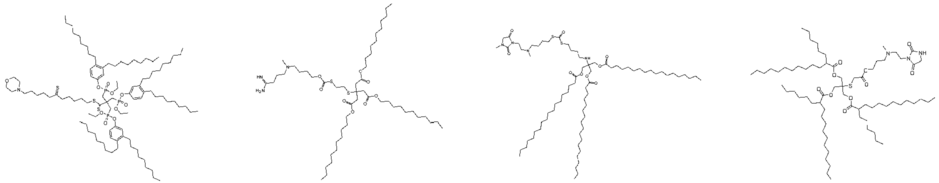

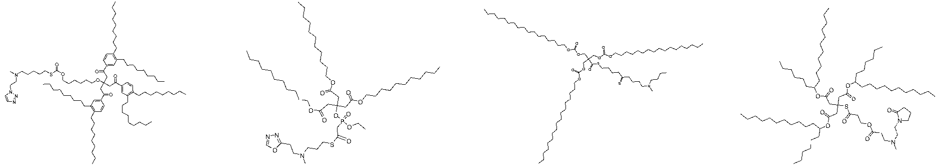

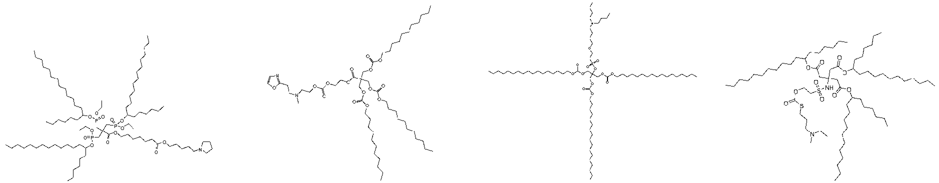

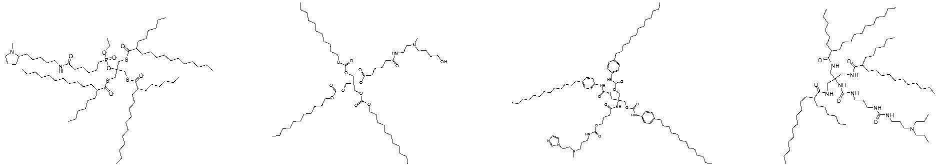

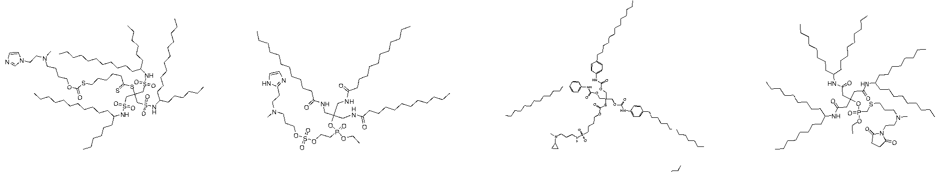

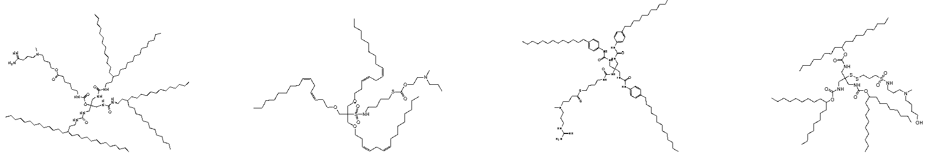

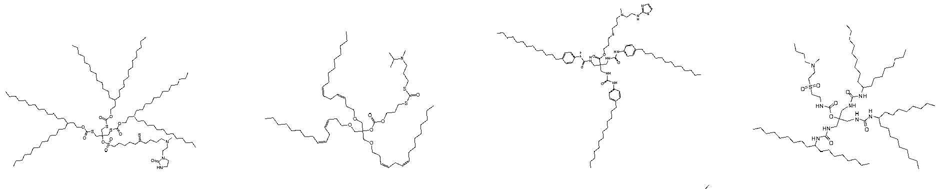

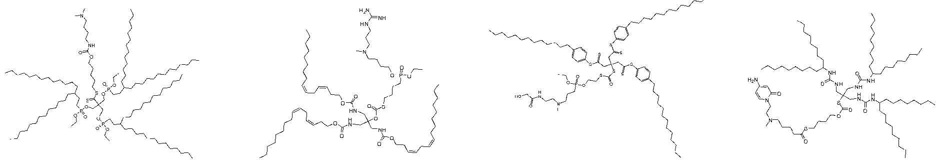

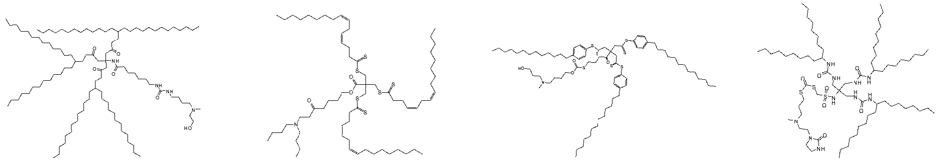

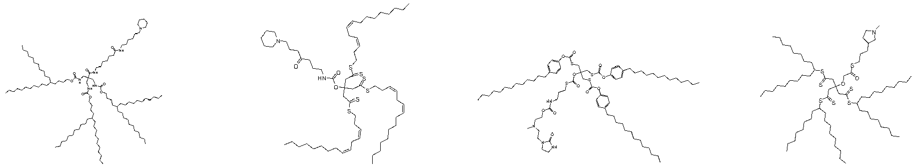

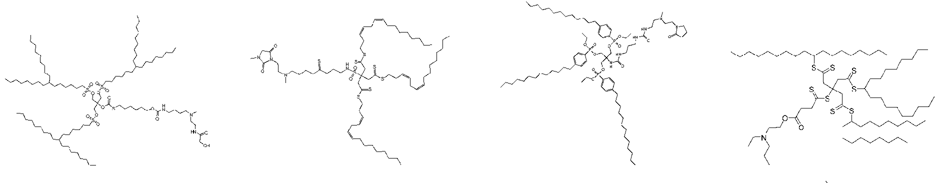

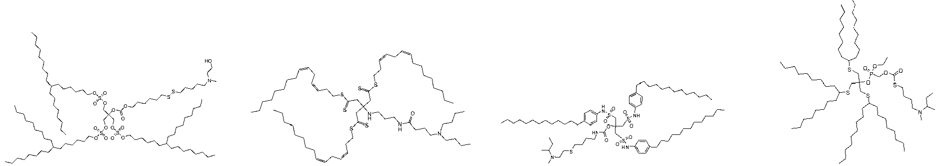

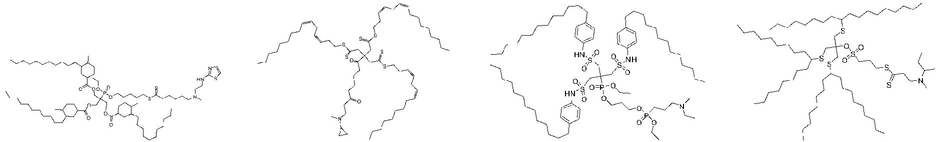

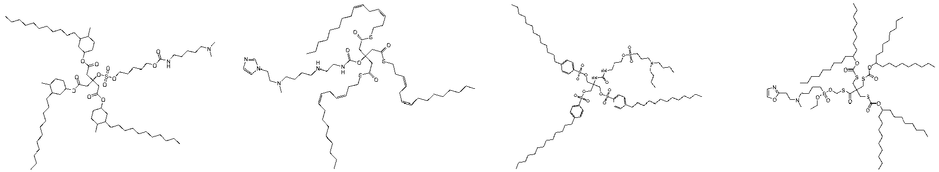

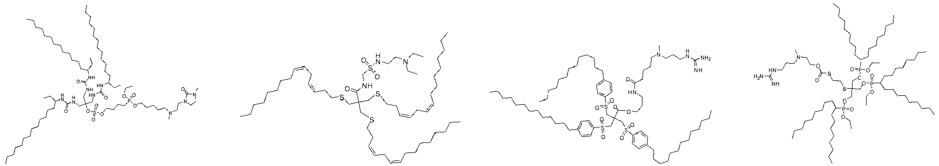

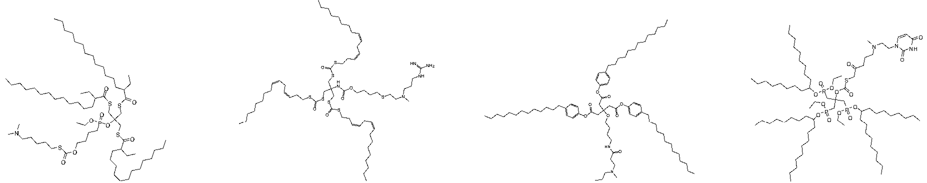

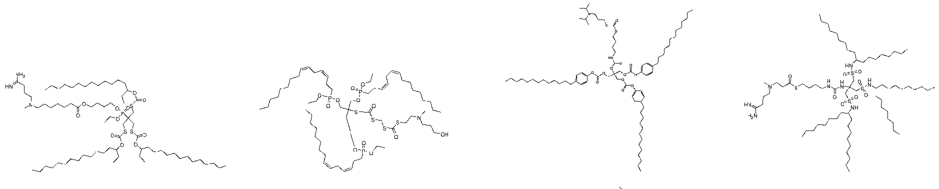

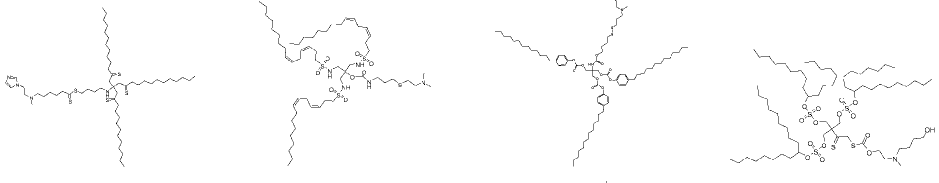

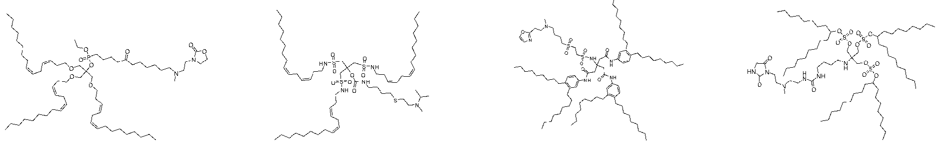

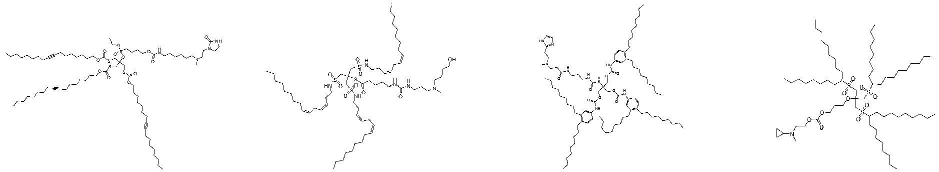

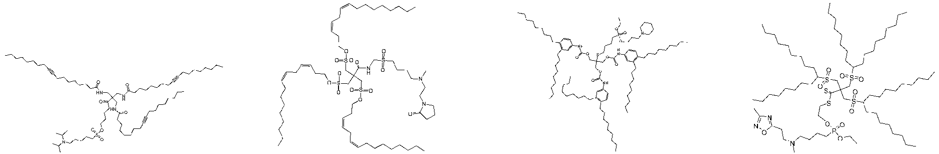

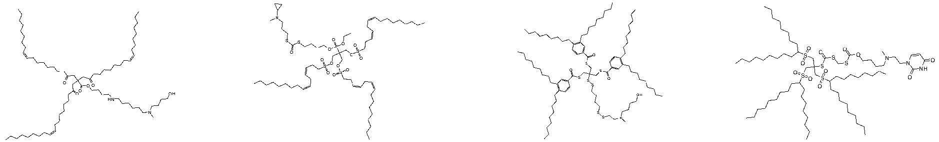

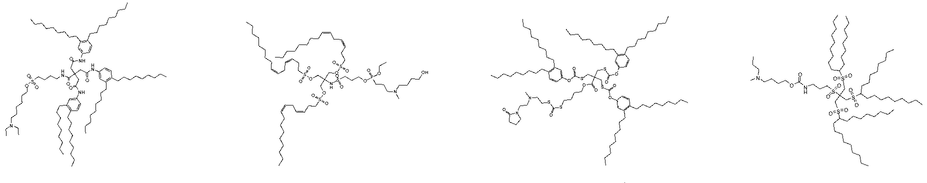

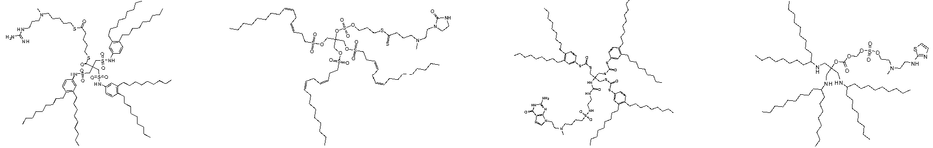

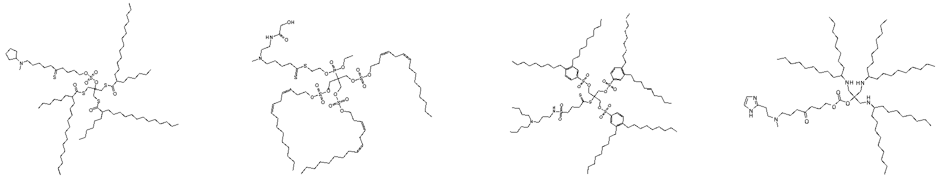

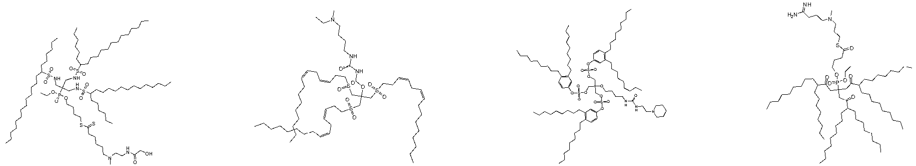

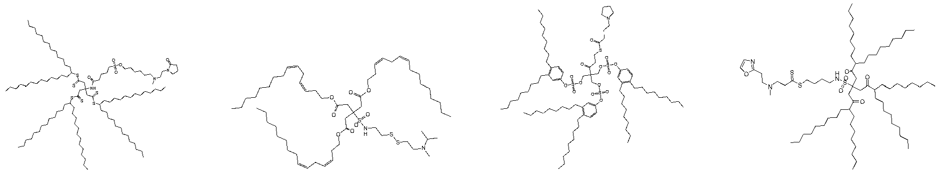

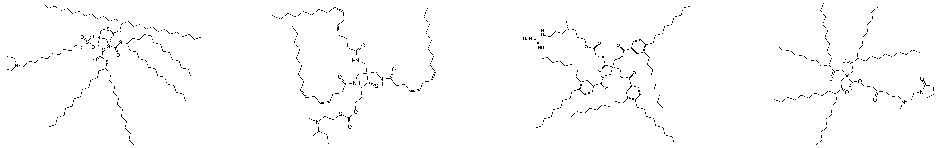

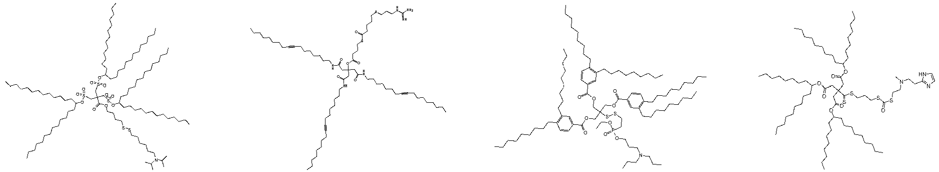

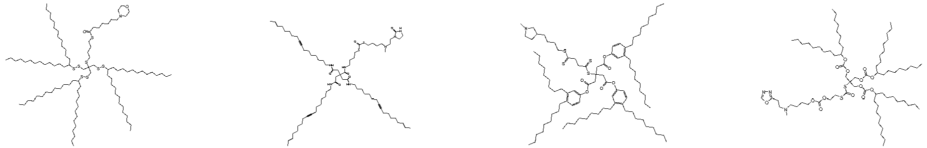

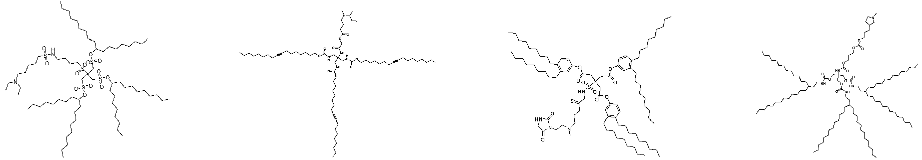

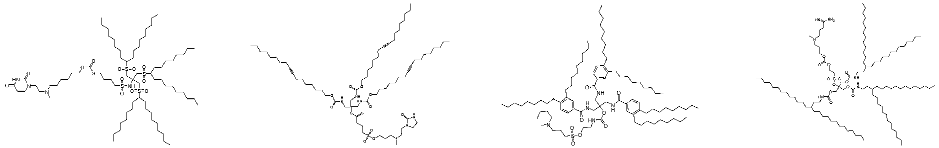

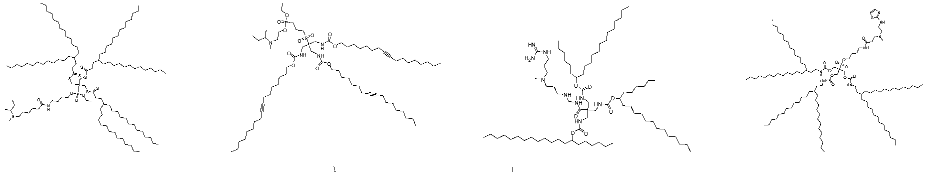

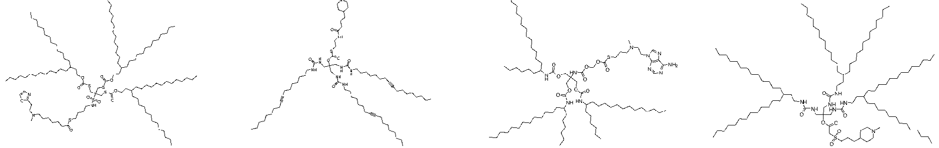

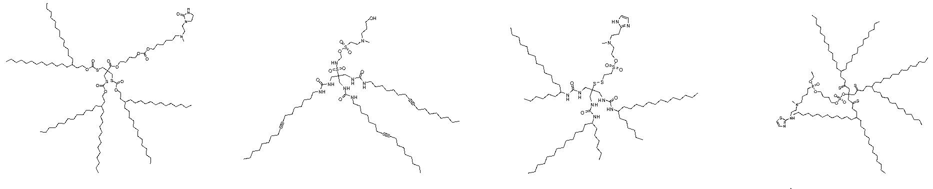

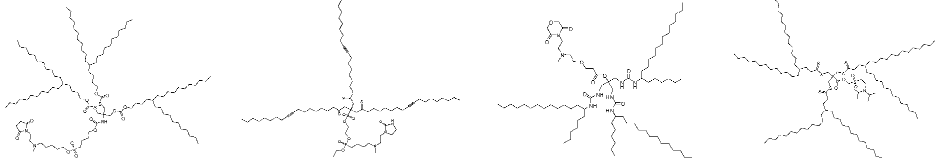

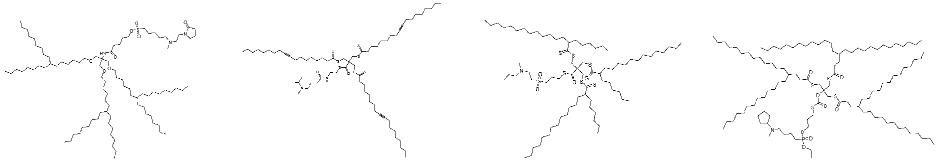

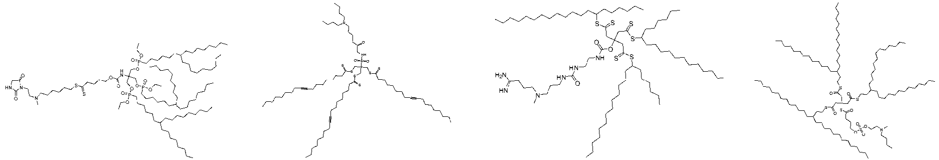

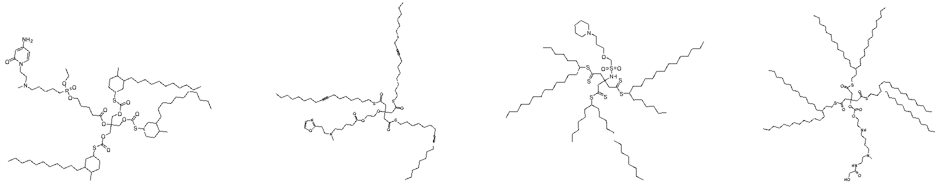

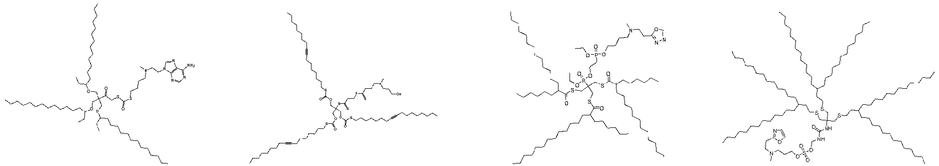

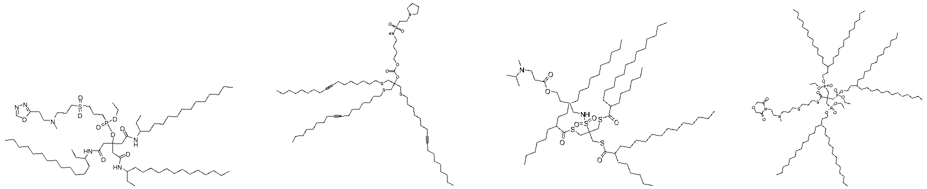

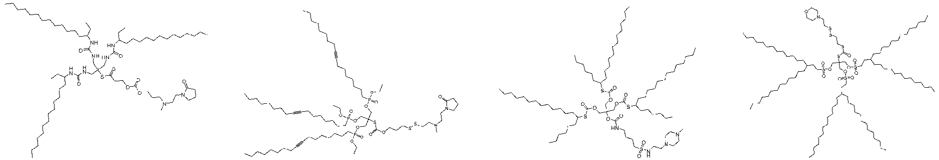

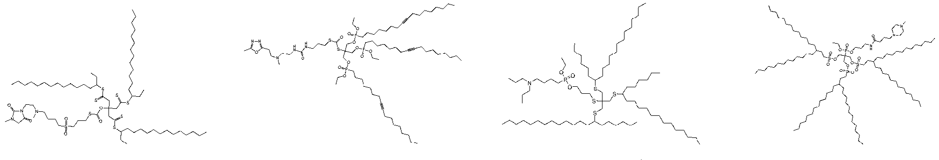

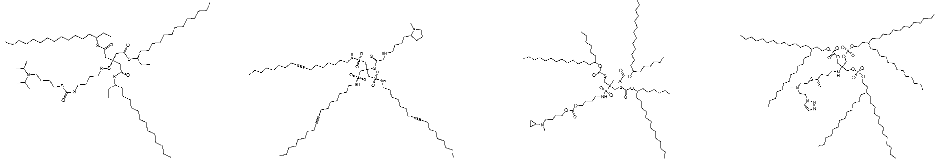

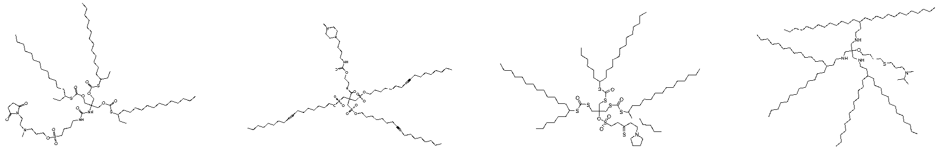

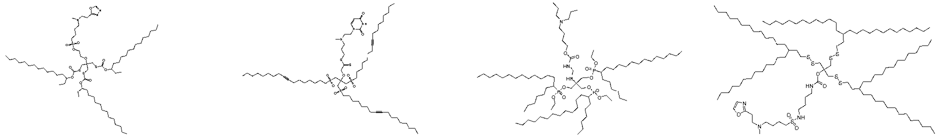

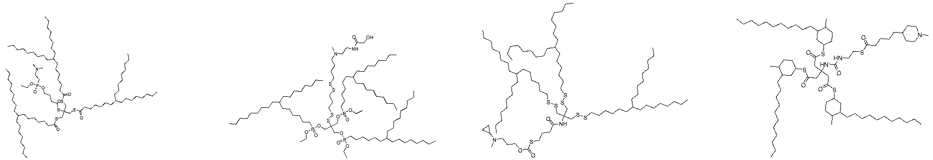

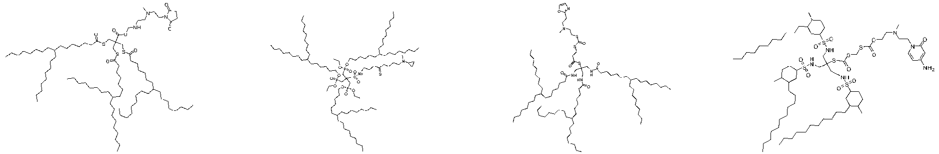

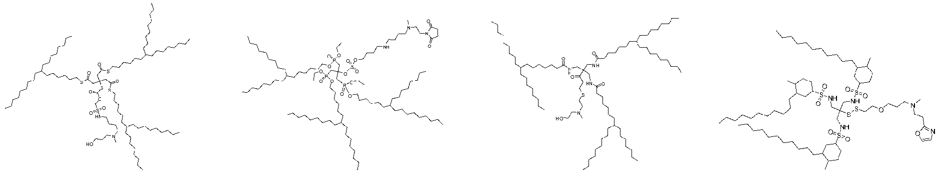

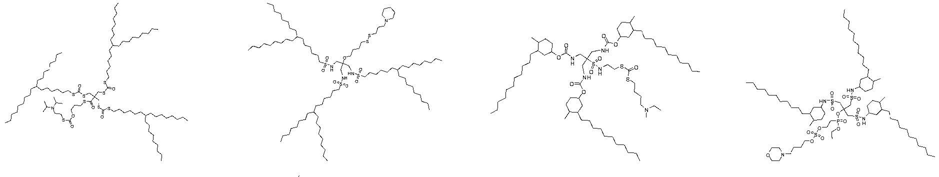

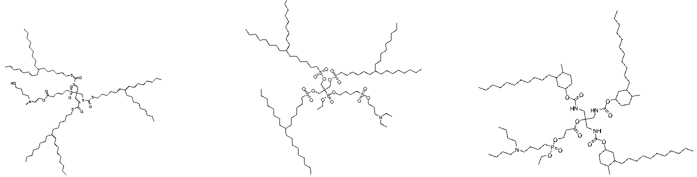

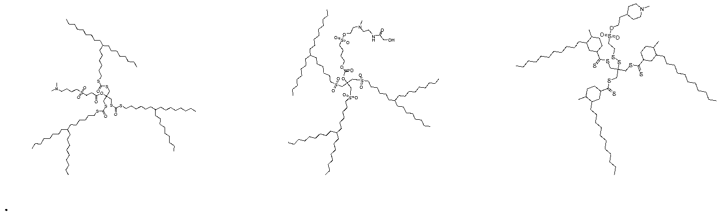

23. The lipid composition according to claim 1, wherein the auxiliary lipid is a mixture of a phospholipid and a steroid or a derivative thereof.

24. The lipid composition according to claim 23, wherein the phospholipid comprises at least one of 1,2-dilinoleoyl-sn-glycero-3-phosphocholine, 1,2-dimyristoyl-sn-glycero-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-phosphocholine, 1-palmitoyl-2-oleoyl-sn-glycero-3-phosphocholine, 1,2-di-O-octadecenyl-sn-glycero-3-phosphocholine, 1-oleoyl-2-cholesteryl-hemisuccinoyl-sn-glycero-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine, 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt, dipalmitoylphosphatidylglycerol, palmitoyloleoylphosphatidylethanolamine, distearoylphosphatidylethanolamine, dipalmitoylphosphatidylethanolamine, dimyristoylphosphoethanolamine, 1-stearoyl-2-oleoyl-phosphatidylethanolamine, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyloleoylphosphatidylcholine, lysophosphatidylcholine, and lysophosphatidylethanolamine.

25. The lipid composition according to claim 23, wherein the steroid or the derivative thereof comprises at least one of cholesterol, cholesterol stearate, coprostanol, sitosterol, ergosterol, campesterol, stigmasterol, brassicasterol, fucosterol, tomatine, ursolic acid, and α-tocopherol.

26. The lipid composition according to claim 1, wherein in the lipid composition, the amount of the ionizable lipid is 5-90 mol% of total lipids in formulation, and the amount of the auxiliary lipid is 10-95 mol% of total lipids in formulation.

27. The lipid composition according to claim 26, wherein a molar ratio of phospholipid to the steroid or derivative thereof in the auxiliary lipid is (5-80):(5-80).

28. The lipid composition according to any one of claims 1-27, wherein the lipid composition further comprise a PEG-conjugated lipid.

29. The lipid composition according to claim 28, wherein the PEG-conjugated lipid comprises at least one of PEG-modified phosphatidylethanolamine, PEG-modified phosphatidic acid, PEG-modified ceramide, PEG-modified dialkylamine, PEG-modified diacylglycerol, and PEG-modified dialkylglycerol.

30. The lipid composition according to claim 29, wherein the PEG-conjugated lipid comprises at least one of PEG-distearyloxypropyl, PEG-c-DOMG, PEG-DPPC, polyethylene glycol dimethacrylate, 1,2-dimyristoyl-rac-glycero-3-methoxypolyethylene glycol, dipalmitoylglycerol-polyethylene glycol, 1,2-distearoyl-rac-glycero-3-methoxypolyethylene glycol, 2-[(polyethylene glycol)-2000]-N,N-ditetradecylacetamide, dipalmitoylphosphatidylethanolamine-polyethylene glycol, distearoylphosphatidylethanolamine-polyethylene glycol, dilauroylphosphatidylethanolamine-polyethylene glycol, and dimyristoylphosphatidylethanolamine-polyethylene glycol lipids.

31. The lipid composition according to claim 28, wherein in the lipid composition, the amount of the ionizable lipid is 5-80 mol% of total lipids in formulation; the amount of the auxiliary lipid is 10-90 mol% of total lipids in formulation; and the amount of the PEG-conjugated lipid is 0.1-15 mol% of total lipids in formulation.

32. The lipid composition according to any one of claims 1-27, wherein the ionizable lipid further comprises at least one of a second ionizable lipid and a third ionizable lipid;
the second ionizable lipid is selected from a compound of formula (2), or a salt, a stereoisomer, or a tautomer thereof: 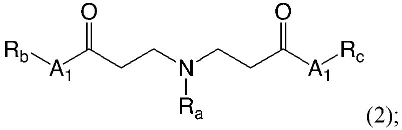
wherein A₁ is NH or O;
Rₐ is selected from a C₆-C₂₄ alkyl, a C₆-C₂₄ alkenyl, a C₆-C₂₄ cycloalkyl, C₆-C₂₄ alcohol, and C₆-C₂₄ short-chain polyoxyethylene; the C₆-C₂₄ alkyl, C₆-C₂₄ alkenyl, C₆-C₂₄ cycloalkyl, C₆-C₂₄ alcohol, and C₆-C₂₄ short-chain polyoxyethylene with a linear or branched structure; R_{b} and R_{c} are each independently selected from C₁-C₁₂ alkyl, C₂-C₁₂ alkenyl, C₁-C₁₂ alkynyl, C₃-C₁₂ cycloalkyl, C₆-C₁₂ aryl, C₁-C₁₂ alkyl alcohol, C₁-C₁₂ heterocyclyl, and alkylamine;
the alkylamine is 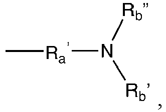 wherein the Rₐ' is C₁-C₁₂ alkyl; the R_{b}' and R_{b}" are each independently selected from H, C₁-C₆ alkylamine, 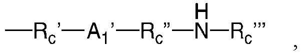 or 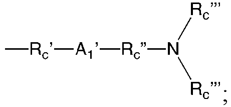 R_{c}" is selected from unsubstituted or amino-substituted C₁-C₆ alkyl; and R_{c}‴ is H, 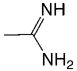 or -R_{c}'-A₁'-R_{c}"-NH₂;
provided that when A₁' is -CO-NH-, -NH-CO-, or -CO-O-, R_{c}' is C₁-C₆ alkyl; and when A₁' is -CO-, R_{c}' is absent; and
the third ionizable lipid is selected from at least one of the following compounds:

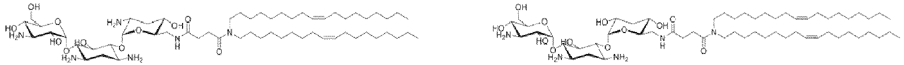

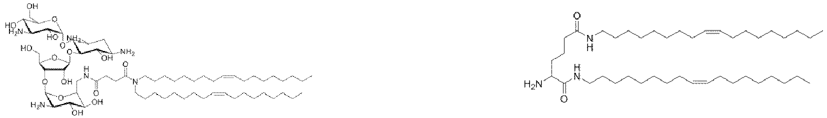

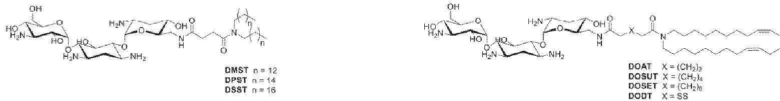

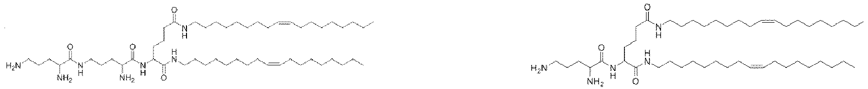

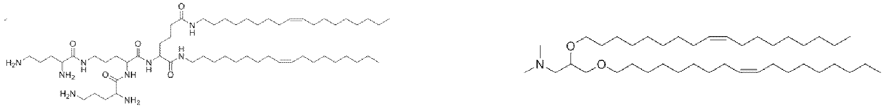

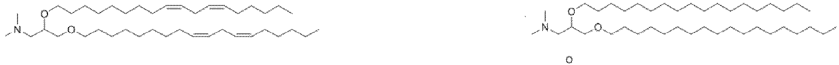

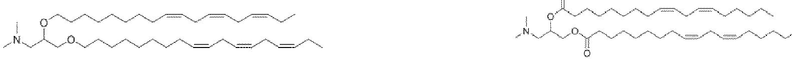

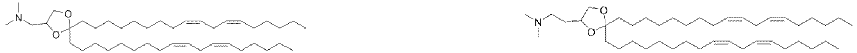

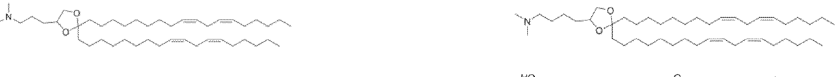

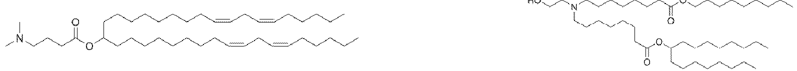

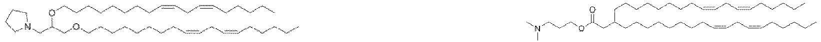

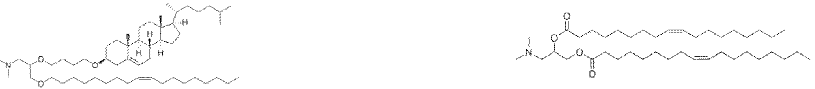

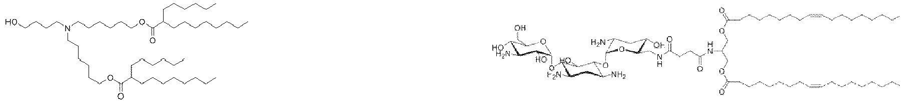

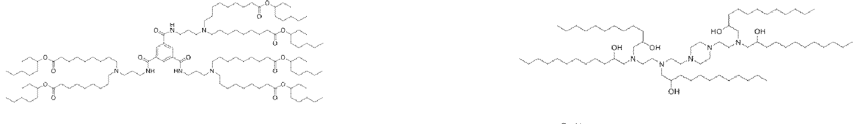

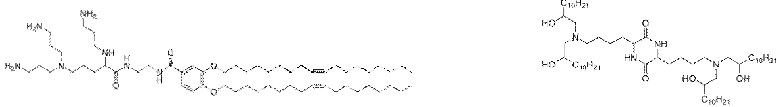

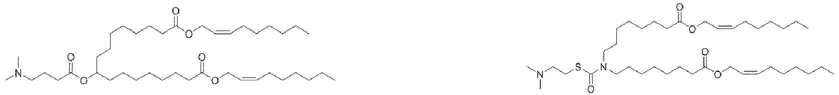

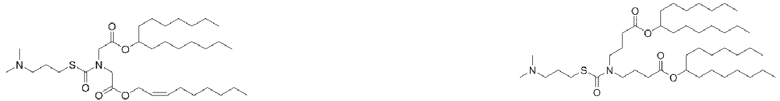

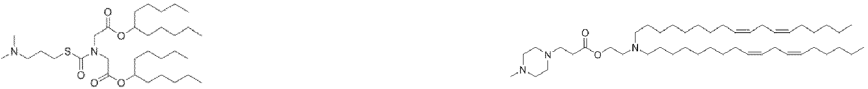

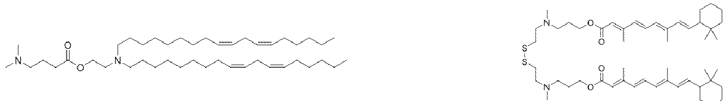

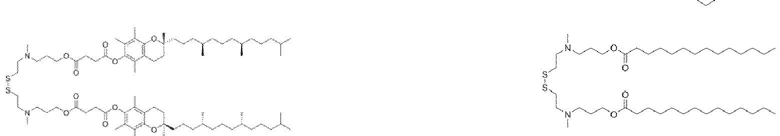

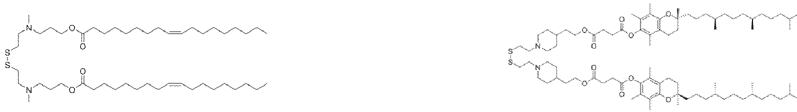

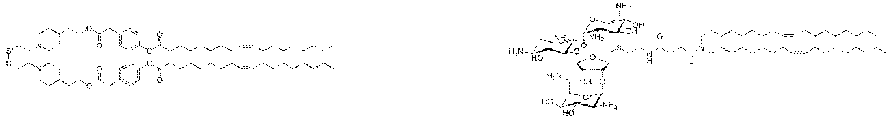

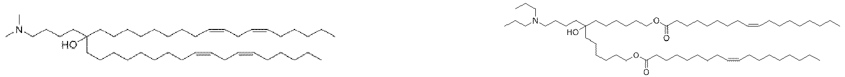

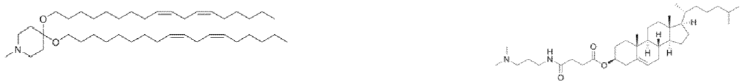

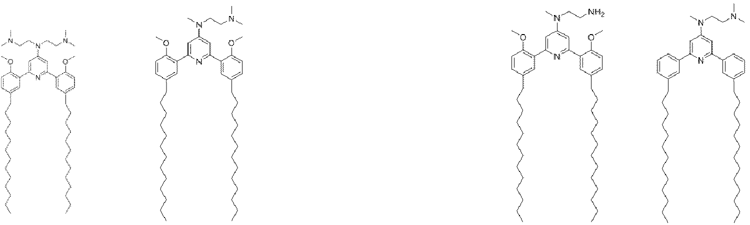

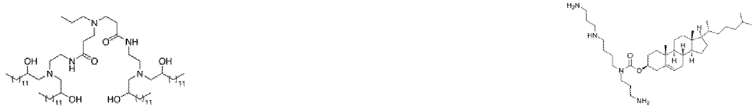

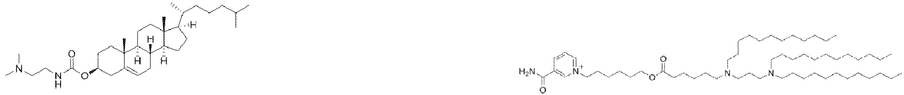

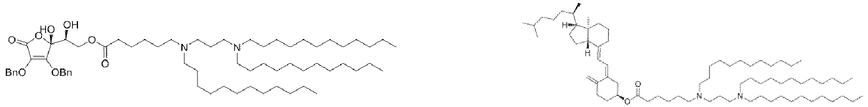

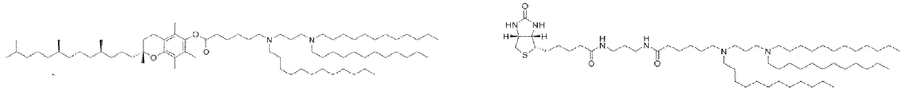

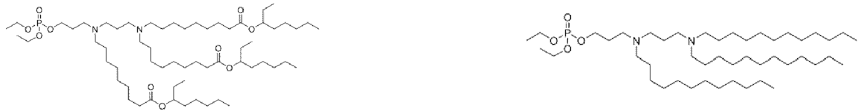

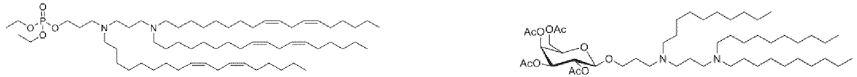

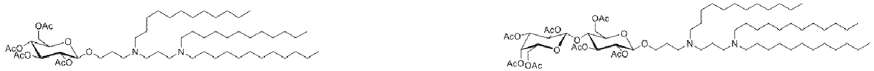

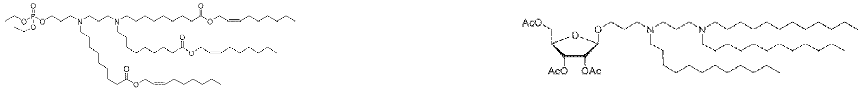

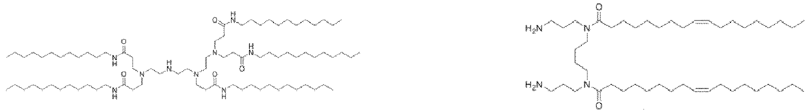

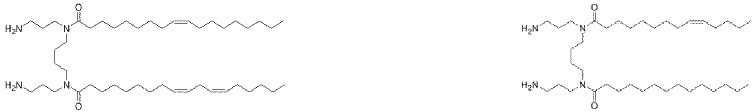

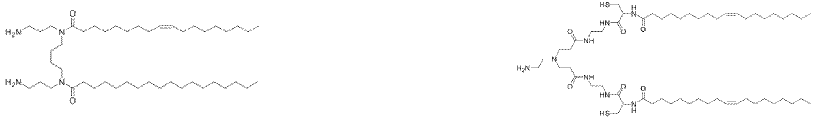

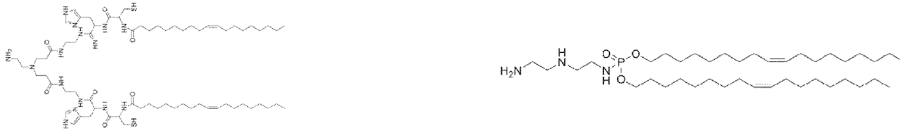

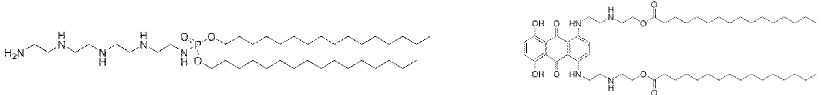

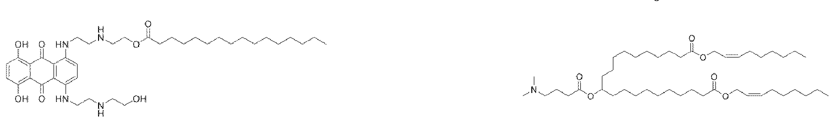

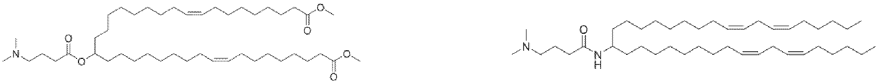

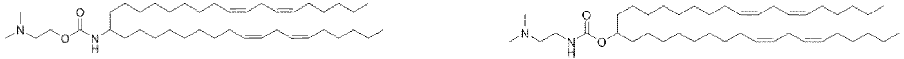

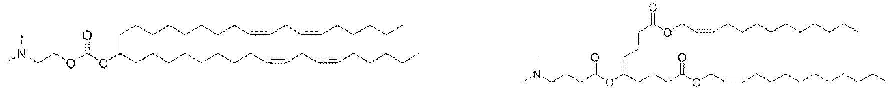

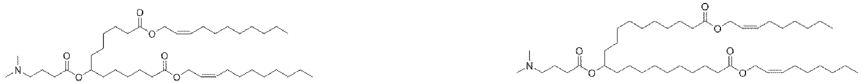

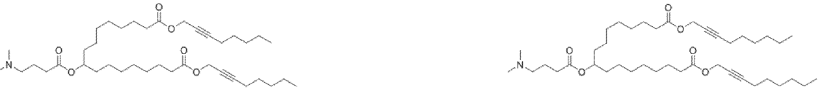

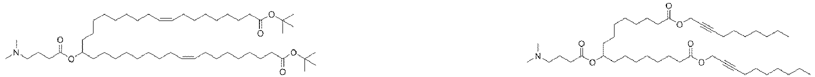

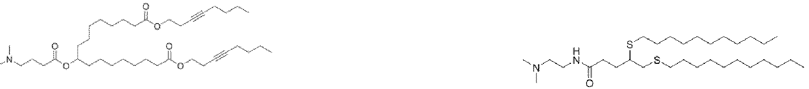

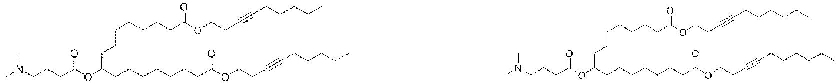

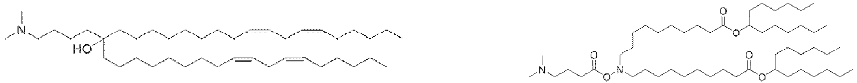

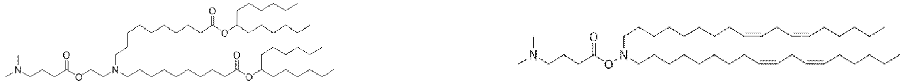

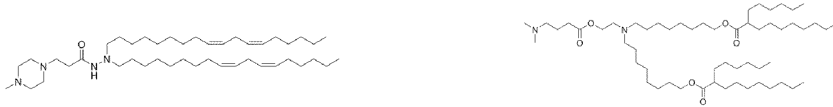

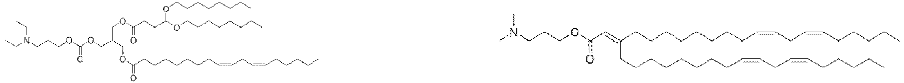

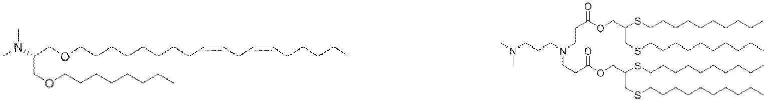

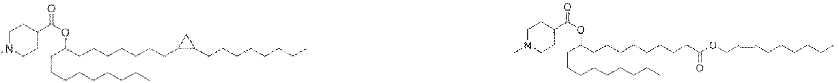

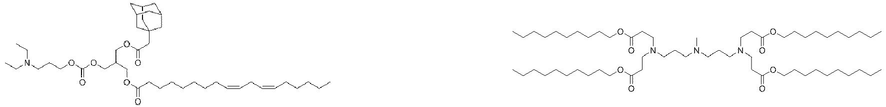

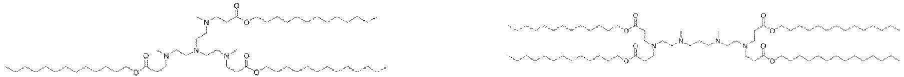

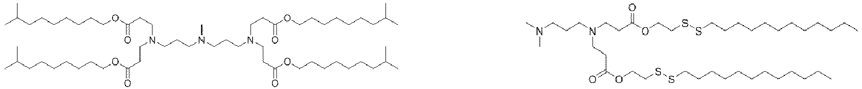

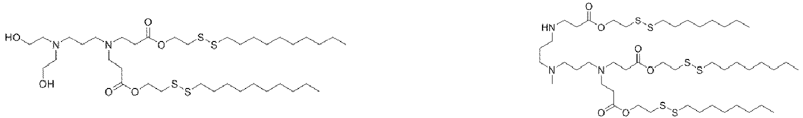

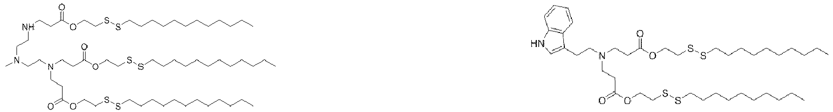

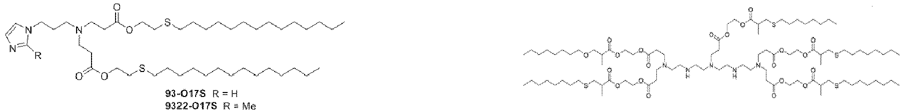

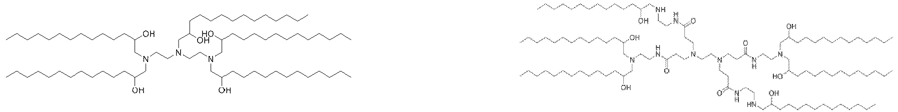

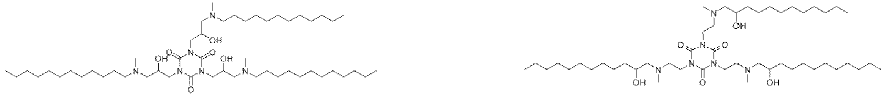

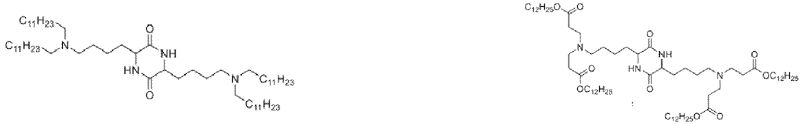

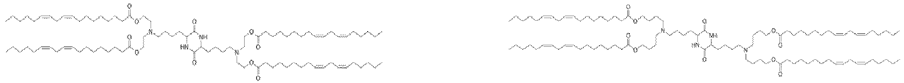

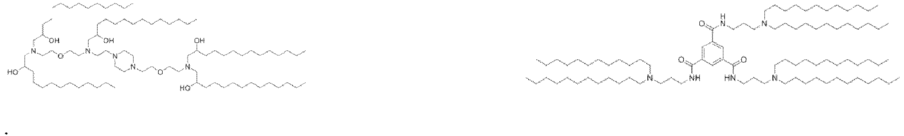

33. The lipid composition according to claim 32, wherein the compound of formula (2) is selected from at least one of the following compounds:

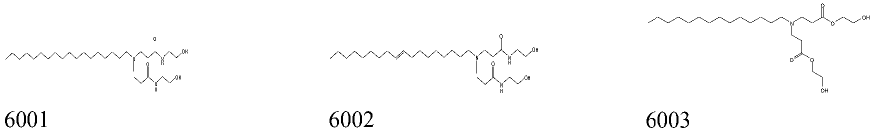

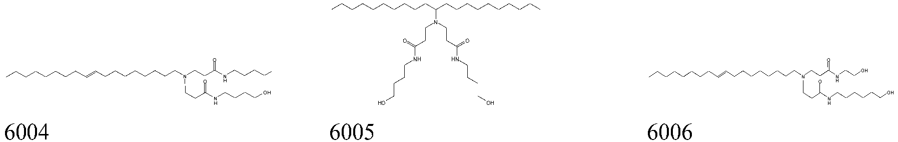

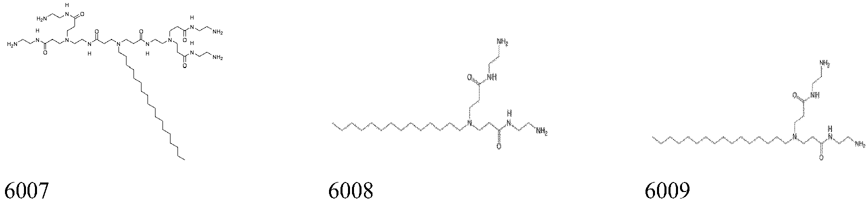

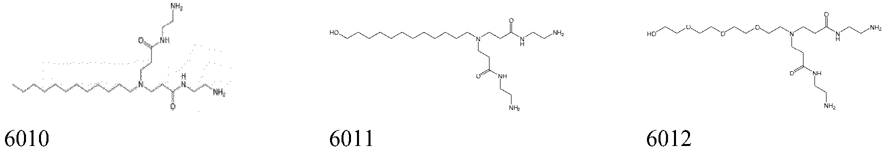

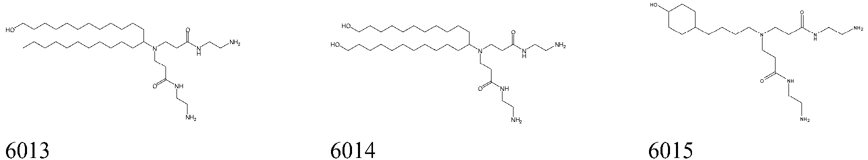

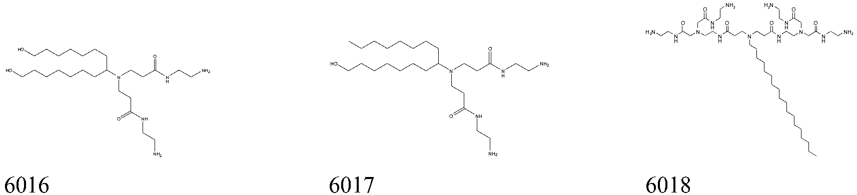

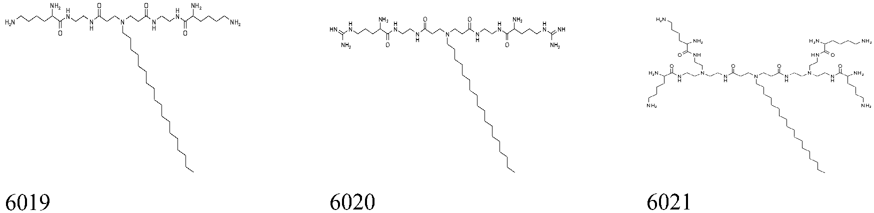

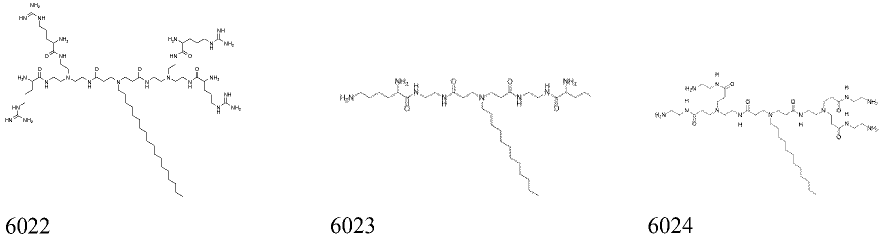

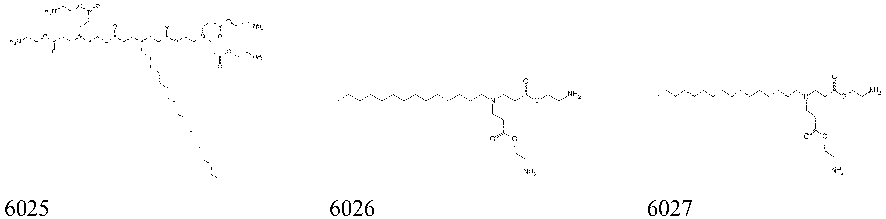

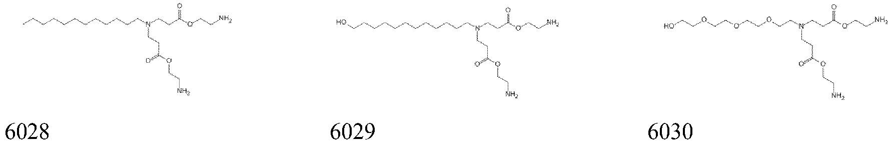

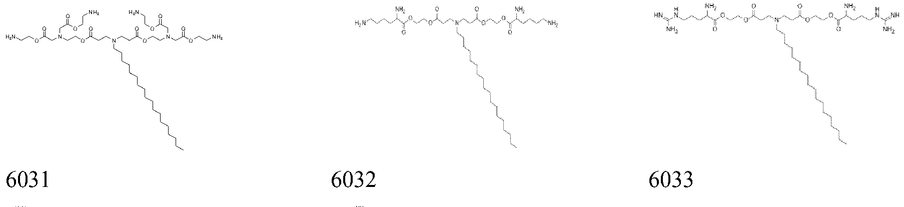

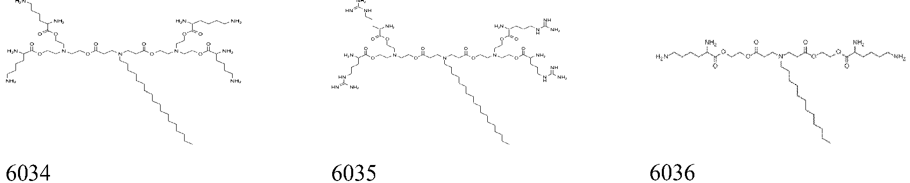

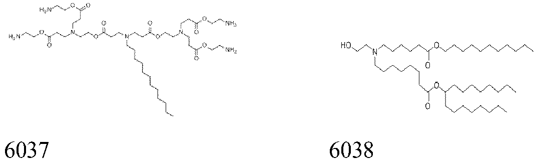

34. The lipid composition according to claim 32, wherein a molar ratio of the first ionizable lipid to the second ionizable lipid and/or the third ionizable lipid in the ionizable lipids is (5-90):(1-80).

35. The lipid composition according to any one of claims 1-27, wherein the lipid composition is in a form of nanoparticles.

36. The lipid composition according to claim 35, wherein the nanoparticles have a particle size of 20-400 nm and a polydispersity index of <0.5.

37. A preparation method for the lipid composition according to any one of claims 1-36, **characterized by** comprising the following steps:
1) dissolving the lipid compound in an organic solvent to form an organic phase;
2) using an aqueous solvent as an aqueous phase; and
3) mixing the organic phase and the aqueous phase, and performing ultrafiltration to obtain the lipid composition.

38. A method for delivering a pharmaceutically active ingredient, wherein the method uses the lipid composition according to any one of claims 1-36 as a carrier; and the pharmaceutically active ingredient comprises at least one of a macromolecular substance and a small molecular substance.
